(19) Europäisches Patentamt
European Patent Office
Office européen des brevets

(11) **EP 4 334 476 B1**

(12) **EUROPEAN PATENT SPECIFICATION**

(45) Date of publication and mention
of the grant of the patent:
**25.02.2026  Bulletin 2026/09**

(21) Application number: **22799768.1**

(22) Date of filing: **15.04.2022**

(51) International Patent Classification (IPC):
***C12Q 1/6886*** (2018.01)     ***G16B 20/20*** (2019.01)

(52) Cooperative Patent Classification (CPC):
**G16B 20/20; C12Q 1/6886;** C12Q 2600/156

(86) International application number:
**PCT/US2022/071759**

(87) International publication number:
**WO 2022/236221 (10.11.2022 Gazette 2022/45)**

(54) **METHODS AND SYSTEMS FOR ANALYZING NUCLEIC ACID MOLECULES**

VERFAHREN UND SYSTEME ZUR ANALYSE VON NUKLEINSÄUREMOLEKÜLEN

PROCÉDÉS ET SYSTÈMES POUR ANALYSER DES MOLÉCULES D'ACIDE NUCLÉIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.05.2021  US 202117308958
13.05.2021  US 202163188410 P
22.07.2021  US 202163224795 P**

(43) Date of publication of application:
**13.03.2024  Bulletin 2024/11**

(73) Proprietor: **The Board of Trustees of the Leland
Stanford
Junior University
Stanford, CA 94305-2038 (US)**

(72) Inventors:
• **CHABON, Jacob, J.**
**Arvada, CO 80004 (US)**
• **KURTZ, David, M.**
**San Carlos, CA 94070 (US)**
• **DIEHN, Maximilian**
**San Carlos, CA 94070 (US)**
• **ALIZADEH, Arash, Ash**
**San Mateo, CA 94402 (US)**

(74) Representative: **Patentanwaltskanzlei
Matschnig & Forsthuber OG
Biberstraße 22
Postfach 36
1010 Wien (AT)**

(56) References cited:
US-A1- 2015 376 700     US-A1- 2020 131 505
US-A1- 2020 131 505     US-A1- 2021 025 005

• GORELICK ALEXANDER N ET AL: "Phase and context shape the function of composite oncogenic mutations", NATURE,, vol. 582, no. 7810, 27 May 2020 (2020-05-27), pages 100 - 103, XP037154953, DOI: 10.1038/S41586-020-2315-8
• CASTEL STEPHANE E ET AL: "Modified penetrance of coding variants by cis-regulatory variation contributes to disease risk", NATURE GENETICS, NATURE PUBLISHING GROUP US, NEW YORK, vol. 50, no. 9, 20 August 2018 (2018-08-20), pages 1327 - 1334, XP036902794, ISSN: 1061-4036, [retrieved on 20180820], DOI: 10.1038/S41588-018-0192-Y
• FAN ZHANG ET AL: "Haplotype phasing of whole human genomes using bead-based barcode partitioning in a single tube", NATURE BIOTECHNOLOGY, vol. 35, no. 9, 26 June 2017 (2017-06-26), New York, pages 852 - 857, XP055584000, ISSN: 1087-0156, DOI: 10.1038/nbt.3897

**(Cont. next page)**

- **ILLUMIINA ET AL: "Local Run Manager TruSight Oncology 500 Analysis Module. Workflow Guide", 27 November 2019 (2019-11-27), XP055664458, Retrieved from the Internet <URL:https://jp.support.illumina.com/content/dam/illumina-support/documents/downloads/software/local-run-manager/local-run-manager-trusight-tumor-oncology-500-v2-workflow-guide-1000000099600.pdf> [retrieved on 20200203]**

Remarks:
    The complete document including Reference Table(s) and the Sequence Listing(s) can be downloaded from the EPO website

Remarks:
    The complete document including Reference Table(s) and the Sequence Listing(s) can be downloaded from the EPO website

**Description**

**CROSS-REFERENCE TO RELATED APPLICATIONS**

The instant application claims the benefit of U.S. Provisional Application No. 63/188,410, filed May 13, 2021, and U.S. Provisional Application No. 63/224,795, filed July 22, 2021, and U.S. Patent Application No. 17/308,958, filed May 5, 2021.

**SEQUENCE LISTING**

The instant application contains a Sequence Listing which has been submitted electronically in ASCII format. Said ASCII copy, created on November 3, 2020, is named 58626-702_601_SL.txt and is 307,199 bytes in size.

**GOVERNMENT RIGHTS**

This invention was made with Government support under CA233975, CA241076, and CA188298 awarded by the National Institutes of Health. The Government has certain rights in the invention.

**BACKGROUND**

Noninvasive blood tests that can detect somatic alterations (e.g., mutated nucleic acids) based on the analysis of cell-free nucleic acids (e.g., cell-free deoxyribonucleic acid (cfDNA) and cell-free ribonucleic acid (cfRNA)) are attractive candidates for cancer screening applications due to the relative ease of obtaining biological specimens (e.g., biological fluids). Circulating tumor nucleic acids (e.g., ctDNA or ctRNA; i.e., nucleic acids derived from cancerous cells) can be sensitive and specific biomarkers in numerous cancer subtypes. However, current methods for minimal residual disease (MRD) detection from ctDNA can be limited by one or more factors, such as low input DNA amounts and high background error rates.

Recent approaches have improved ctDNA MRD performance by tracking multiple somatic mutations with error-suppressed sequencing, resulting in detection limits as low as 4 parts in 100,000 from limited cfDNA input. Detection of residual disease during or after treatment is a powerful tool, with detectable MRD representing an adverse prognostic sign even during radiographic remission. However, current limits of detection may be insufficient to universally detect residual disease in patients destined for disease relapse or progression. This 'loss of detection' is exemplified in diffuse large B-cell lymphoma (DLBCL), where ctDNA detection after two cycles of curative-intent therapy is a strong prognostic marker. Despite this, almost one-third of patients experiencing disease progression do not have detectable ctDNA at this landmark, representing 'false-negative' tests. Similar false-negative rates in colon cancer and breast cancer have been observed. US 2020/131505 and the publication of Gorelick et al., Nature (2020) both disclose methods used for phasing.

**SUMMARY**

The present disclosure provides methods and systems for analyzing nucleic acids, such as cell-free nucleic acids (e.g., cfDNA, cfRNA) from a subject. Methods and systems of the present disclosure can utilize sequencing results derived from the subject to detect cancer-derived nucleic acids (e.g., ctDNA, ctRNA) for, e.g., disease diagnosis, disease monitoring, or determining treatments for the subject. Methods and systems of the present disclosure can exhibit enhanced sensitivity, specificity and/or reliability of detection of cancer-derived nucleic acids.

The present invention relates to methods for identifying sets of validated phased variants from a tumor sample of a subject, according to the appended claims.

Any passages or references in the present disclosure that relate to methods of treatment by therapy or surgery are only for illustrative purposes and better understanding of the claimed invention, but these methods of treatment by therapy or surgery are not part of the claimed invention.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a condition of the subject. In some embodiments, cellular DNA is used instead of cell-free DNA (e.g., for detection of leukemia or other hematological cancers).

In some embodiments of any one of the methods disclosed herein, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules.

In some embodiments, (b) further comprises identifying one or more insertions or deletions (indels) in the one or more cell-free nucleic acid molecules, and (c) further comprises determining the condition of the subject based at least in part on the identified one or more indels.

In some embodiments, the method further comprises determining the start position (i.e., the 5'-most nucleotide) and the end position (i.e., the 3'-most nucleotide) in a molecule. In some cases, tumor-derived nucleic acids, such as tumor-derived cfDNA molecules can have stereotyped start/end positions, which may reflect cleavage by tissue-specific nucleases. The start and end positions can be used-in connection with phased variants-to identify a condition of a subject.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a condition of the subject.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a condition of the subject.

In some embodiments of any one of the methods disclosed herein, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data.

In some embodiments of any one of the methods disclosed herein, each of the one or more cell-free nucleic acid molecules comprises a plurality of phased variants relative to a reference genomic sequence. In some embodiments of any one of the methods disclosed herein, a first phased variant of the plurality of phased variants and a second phased variant of the plurality of phased variants are separated by at least one nucleotide.

In some embodiments of any one of the methods disclosed herein, the processes (a) to (c) are performed by a computer system.

In some embodiments of any one of the methods disclosed herein, the sequencing data is generated based on nucleic acid amplification. In some embodiments of any one of the methods disclosed herein, the sequencing data is generated based on polymerase chain reaction. In some embodiments of any one of the methods disclosed herein, the sequencing data is generated based on amplicon sequencing.

In some embodiments of any one of the methods disclosed herein, the sequencing data is generated based on next-generation sequencing (NGS). Alternatively, in some embodiments of any one of the methods disclosed herein, the sequencing data is generated based on non-hybridization-based NGS.

In some embodiments of any one of the methods disclosed herein, the sequencing data is generated without use of molecular barcoding of at least a portion of the plurality of cell-free nucleic acid molecules. In some embodiments of any one of the methods disclosed herein, the sequencing data is obtained without use of sample barcoding of at least a portion of the plurality of cell-free nucleic acid molecules.

In some embodiments of any one of the methods disclosed herein, the sequencing data is obtained without in silico removal or suppression of (i) background error or (ii) sequencing error.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method of treating a condition of a subject (not encompassed by the wording of the claims, but useful for understanding the invention), the method comprising: (a) identifying the subject for treatment of the condition, wherein the subject has been determined to have the condition based on identification of one or more cell-free nucleic acid molecules from a plurality of cell-free nucleic acid molecules that is obtained or derived from the subject, wherein each of the one or more cell-free nucleic acid molecules identified comprises a plurality of phased variants relative to a reference genomic sequence that are separated by at least one nucleotide, and wherein a presence of the plurality of phased variants is indicative of the condition of the subject; and (b) subjecting the subject to the treatment based on the identification in (a).

In some embodiments, the subject has been determined to have the condition based at least in part on one or more insertions or deletions (indels) identified in the one or more cell-free nucleic acid molecules.

In one aspect, the present disclosure provides a method (not encompassed by the wording of the claims, but useful for understanding the invention) of monitoring a progress of a condition of a subject, the method comprising: (a) determining a first state of the condition of the subject based on identification of a first set of one or more cell-free nucleic acid molecules from a first plurality of cell-free nucleic acid molecules that is obtained or derived from the subject; (b) determining a second state of the condition of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining the progress of the condition based on the first state of the condition and the second state of the condition, wherein each of the one or more cell-free nucleic acid molecules comprises a plurality of phased variants relative to a reference genomic sequence that are separated by at least one nucleotide.

In some embodiments of any one of the methods disclosed herein, the progress of the condition is worsening of the condition.

In some embodiments of any one of the methods disclosed herein, the progress of the condition is at least a partial remission of the condition.

In some embodiments of any one of the methods disclosed herein, a presence of the plurality of phased variants is indicative of the first state or the second state of the condition of the subject.

In some embodiments of any one of the methods disclosed herein, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject.

In some embodiments of any one of the methods disclosed herein, the subject is subjected to a treatment for the condition (i) prior to obtaining the second plurality of cell-free nucleic acid molecules from the subject and (ii) subsequent to obtaining the first plurality of cell-free nucleic acid molecules from the subject.

In some embodiments of any one of the methods disclosed herein, the progress of the condition is indicative of minimal residual disease of the condition of the subject. In some embodiments of any one of the methods disclosed herein, the progress of the condition is indicative of tumor burden or cancer burden of the subject.

In some embodiments of any one of the methods disclosed herein, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the condition.

In some embodiments, the subject has been determined to have the condition based at least in part on one or more insertions or deletions (indels) identified in the one or more cell-free nucleic acid molecules.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a condition of the subject.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a condition of the subject.

In some embodiments of any one of the methods disclosed herein, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules.

In some embodiments of any one of the methods disclosed herein, a first phased variant of the plurality of phased variants and a second phased variant of the plurality of phased variants are separated by at least one nucleotide.

In some embodiments of any one of the methods disclosed herein, the activatable reporter agent is activated upon hybridization of the individual nucleic acid probe to the plurality of phased variants.

In some embodiments of any one of the methods disclosed herein, the activatable reporter agent is activated upon dehybridization of at least a portion of the individual nucleic acid probe that has been hybridized to the plurality of phased variants.

In some embodiments of any one of the methods disclosed herein, the method further comprises mixing (1) the set of nucleic acid probes and (2) the plurality of cell-free nucleic acid molecules.

In some embodiments of any one of the methods disclosed herein, the activatable reporter agent is a fluorophore.

In some embodiments of any one of the methods disclosed herein, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables.

In some embodiments of any one of the methods disclosed herein, the analyzing of the identified one or more cell-free nucleic acid molecules is not based on other cell-free nucleic acid molecules of the plurality of cell-free nucleic acid molecules that do not comprise the plurality of phased variants.

In some embodiments of any one of the methods disclosed herein, a number of the plurality of phased variants from the identified one or more cell-free nucleic acid molecules is indicative of the condition of the subject. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the condition of the subject.

In some embodiments of any one of the methods disclosed herein, a frequency of the plurality of phased variants in the identified one or more cell-free nucleic acid molecules is indicative of the condition of the subject. In some embodiments, the frequency is indicative of a diseased cell associated with the condition. In some embodiments, the condition is diffuse large B-cell lymphoma, and wherein the frequency is indicative of whether the one or more cell-free nucleic acid molecules are derived from germinal center B-cell (GCB) or activated B-cell (ABC).

In some embodiments of any one of the methods disclosed herein, genomic origin of the identified one or more cell-free nucleic acid molecules is indicative of the condition of the subject.

In some embodiments of any one of the methods disclosed herein, the first and second phased variants are separated by at least 2, at least 3, at least 4, at least 5, at least 6, at least 7, or at least 8 nucleotides. In some embodiments of any one of the methods disclosed herein, the first and second phased variants are separated by at most about 180, at most about 170, at most about 160, at most about 150, or at most about 140 nucleotides.

In some embodiments of any one of the methods disclosed herein, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV.

In some embodiments of any one of the methods disclosed herein, the plurality of phased variants comprises at least 3, at least 4, at least 5, at least 10, at least 15, at least 20, or at least 25 phased variants within the same cell-free nucleic acid molecule.

In some embodiments of any one of the methods disclosed herein, the one or more cell-free nucleic acid molecules identified comprises at least 2, at least 3, at least 4, at least 5, at least 10, at least 50, at least 100, at least 500, or at least 1,000 cell-free nucleic acid molecules.

In some embodiments of any one of the methods disclosed herein, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome.

In some embodiments of any one of the methods disclosed herein, the reference genomic sequence is derived from a sample of the subject.

In some embodiments of any one of the methods disclosed herein, the sample is a healthy sample. In some embodiments, the sample comprises a healthy cell. In some embodiments, the healthy cell comprises a healthy leukocyte.

In some embodiments of any one of the methods disclosed herein, the sample is a diseased sample. In some embodiments, the diseased sample comprises a diseased cell. In some embodiments, the diseased cell comprises a tumor cell. In some embodiments, the diseased sample comprises a solid tumor.

In some embodiments of any one of the methods disclosed herein, the set of nucleic acid probes is designed based on the plurality of phased variants that are identified by comparing (i) sequencing data from a solid tumor, lymphoma, or blood tumor of the subject and (ii) sequencing data from a healthy cell of the subject or a healthy cohort. In some embodiments, the healthy cell is from the subject. In some embodiments, the healthy cell is from the healthy cohort.

In some embodiments of any one of the methods disclosed herein, the set of nucleic acid probes are designed to hybridize to at least a portion of sequences of genomic loci associated with the condition. In some embodiments, the genomic loci associated with the condition are known to exhibit aberrant somatic hypermutation when the subject has the condition.

In some embodiments of any one of the methods disclosed herein, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3.

In some embodiments of any one of the methods disclosed herein, each nucleic acid probe of the set of nucleic acid probes has at least about 70%, at least about 80%, at least about 90% sequence identity, at least about 95% sequence identity, or about 100% sequence identity to a probe sequence selected from Table 6.

In some embodiments of any one of the methods disclosed herein, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6.

In some embodiments of any one of the methods disclosed herein, the method further comprises determining that the subject has the condition or determining a degree or status of the condition of the subject, based on the identified one or more cell-free nucleic acid molecules comprising the plurality of phased variants. In some embodiments, the method further comprises determining that the one or more cell-free nucleic acid molecules are derived from a sample associated with the condition, based on performing a statistical model analysis of the identified one or more cell-free nucleic acid molecules. In some embodiments, the statistical model analysis comprises a Monte Carlo statistical analysis.

In some embodiments of any one of the methods disclosed herein, the method further comprises monitoring a progress of the condition of the subject based on the identified one or more cell-free nucleic acid molecules.

In some embodiments of any one of the methods disclosed herein, the method further comprises performing a different procedure to confirm the condition of the subject. In some embodiments, the different procedure comprises a blood test, genetic test, medical imaging, physical exam, or tissue biopsy.

In some embodiments of any one of the methods disclosed herein, the method further comprises determining a treatment for the condition of the subject based on the identified one or more cell-free nucleic acid molecules.

In some embodiments of any one of the methods disclosed herein, the subject has been subjected to a treatment for the condition prior to (a).

In some embodiments of any one of the methods disclosed herein, the treatment comprises chemotherapy, radiotherapy, chemoradiotherapy, immunotherapy, adoptive cell therapy, hormone therapy, targeted drug therapy, surgery, transplant, transfusion, or medical surveillance.

In some embodiments of any one of the methods disclosed herein, the plurality of cell-free nucleic acid molecules comprise a plurality of cell-free deoxyribonucleic acid (DNA) molecules.

In some embodiments of any one of the methods disclosed herein, condition comprises a disease.

In some embodiments of any one of the methods disclosed herein, the plurality of cell-free nucleic acid molecules are derived from a bodily sample of the subject. In some embodiments, the bodily sample comprises plasma, serum, blood, cerebrospinal fluid, lymph fluid, saliva, urine, or stool.

In some embodiments of any one of the methods disclosed herein, the subject is a mammal. In some embodiments of any one of the methods disclosed herein, the subject is a human.

In some embodiments of any one of the methods disclosed herein, the condition comprises neoplasm, cancer, or tumor. In some embodiments, the condition comprises a solid tumor. In some embodiments, the condition comprises a lymphoma. In some embodiments, the condition comprises a B-cell lymphoma. In some embodiments, the condition comprises a sub-type of B-cell lymphoma selected from the group consisting of diffuse large B-cell lymphoma, follicular lymphoma, Burkitt lymphoma, and B-cell chronic lymphocytic leukemia. In some embodiments of any one of the methods disclosed herein, the condition comprises transplant rejection of or a chromosomal abnormality.

In some embodiments of any one of the methods disclosed herein, the plurality of phased variants have been previously identified as tumor-derived from sequencing a prior tumor sample or cell-free nucleic acid sample.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a composition (not encompassed by the wording of the claims, but useful for understanding the invention) comprising a bait set comprising a set of nucleic acid probes designed to capture cell-free DNA molecules derived from at least about 5% of genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the set of nucleic acid probes are designed to pull down cell-free DNA molecules derived from at least about 10%, at least about 20%, at least about 30%, at least about 40%, at least about 50%, at least about 60%, at least about 70%, at least about 80%, at least about 90%, or about 100% of the genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the set of nucleic acid probes are designed to capture the one or more cell-free DNA molecules derived from at most about 10%, at most about 20%, at most about 30%, at most about 40%, at most about 50%, at most about 60%, at most about 70%, at most about 80%, at most about 90%, or about 100% of the genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the bait set comprises at most 5, at most 10, at most 50, at most 100, at most 500, at most 1000, or at most 2000 nucleic acid probes.

In some embodiments of any of the compositions disclosed herein, an individual nucleic acid probe of the set of nucleic acid probes comprises a pull-down tag.

In some embodiments of any of the compositions disclosed herein, the pull-down tag comprises a nucleic acid barcode.

In some embodiments of any of the compositions disclosed herein, the pull-down tag comprises biotin.

In some embodiments of any of the compositions disclosed herein, each of the cell-free DNA molecules is between about 100 nucleotides and about 180 nucleotides in length.

In some embodiments of any of the compositions disclosed herein, the genomic regions are associated with a condition.

In some embodiments of any of the compositions disclosed herein, the genomic regions exhibit aberrant somatic hypermutation when a subject has the condition.

In some embodiments of any of the compositions disclosed herein, the condition comprises a B-cell lymphoma. In some embodiments, the condition comprises a sub-type of B-cell lymphoma selected from the group consisting of diffuse large B-cell lymphoma, follicular lymphoma, Burkitt lymphoma, and B-cell chronic lymphocytic leukemia.

In some embodiments of any of the compositions disclosed herein, the composition further comprises a plurality of cell-free DNA molecules obtained or derived from a subject.

In one aspect, the present disclosure provides a method (not encompassed by the wording of the claims, but useful for understanding the invention) to perform a clinical procedure on an individual, the method comprising: (a) obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; (b) identifying or having identified a plurality of variants in phase within the cell-free nucleic acid sequencing result; (c) determining or having determined, utilizing a statistical model and the identified phased variants, that the cell-free nucleic acid sequencing result contains nucleotides derived from a neoplasm; and (d) performing a clinical procedure on the individual to confirm the presence of the B-cell cancer, based upon determining that the cell-free nucleic acid sequencing result contains nucleic acid sequences likely derived from the B-cell cancer.

In some embodiments of any of the compositions disclosed herein, the biopsy is one of blood, serum, cerebrospinal fluid, lymph fluid, urine, or stool.

In some embodiments of any of the compositions disclosed herein, the genomic loci are selected from (i) the genomic regions identified in Table 1, (ii) the genomic regions identified in Table 3, or (iii) the genomic regions identified to have a plurality of phased variants in Table 3.

In some embodiments of any of the compositions disclosed herein, the sequences of the nucleic acid probes are selected from Table 6.

In some embodiments of any of the compositions disclosed herein, the clinical is procedure is a blood test, medical imaging, or a physical exam.

In some embodiments, the method further comprises identifying or having identified one or more insertions or deletions (indels) within the cell-free nucleic acid sequencing result, and determining or having determined, based least in part on the identified one or more indels, that the cell-free nucleic acid sequencing result contains the nucleotides derived from the neoplasm.

In one aspect, the present disclosure provides a method (not encompassed by the wording of the claims, but useful for understanding the invention) to treat an individual for a B-cell cancer, the method comprising: (a) obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; (b) identifying or having identified a plurality of variants in phase within the cell-free nucleic acid sequencing result; (c) determining or having determined, utilizing a statistical model and the identified phased variants, that the cell-free nucleic acid sequencing result contains nucleotides derived from a neoplasm; and (d) treating the individual to curtail the B-cell cancer, based upon determining that the cell-free nucleic acid sequencing result contains nucleic acid sequences derived from the B-cell cancer.

In some embodiments of any of the compositions disclosed herein, the biopsy is one of blood, serum, cerebrospinal fluid, lymph fluid, urine or stool.

In some embodiments of any of the compositions disclosed herein, the genomic loci are selected from (i) the genomic regions identified in Table 1, (ii) the genomic regions identified in Table 3, or (iii) the genomic regions identified to have a plurality of phased variants in Table 3.

In some embodiments of any of the compositions disclosed herein, the sequences of the nucleic acid probes are selected from Table 6.

In some embodiments of any of the compositions disclosed herein, the treatment is chemotherapy, radiotherapy, immunotherapy, hormone therapy, targeted drug therapy, or medical surveillance.

In some embodiments, the method further comprises identifying or having identified one or more insertions or deletions (indels) within the cell-free nucleic acid sequencing result, and determining or having determined, based least in part on the identified one or more indels, that the cell-free nucleic acid sequencing result contains the nucleotides derived from the neoplasm.

In one aspect, the present disclosure provides a method (not encompassed by the wording of the claims, but useful for understanding the invention) to detect cancerous minimal residual disease in an individual and to treat the individual for a cancer, the method comprising: (a) obtaining or having obtained a targeted sequencing result of a collection of cell-free nucleic acid molecules, wherein the collection of cell-free nucleic acid molecules are sourced from a liquid or waste biopsy of an individual, wherein the liquid or waste biopsy is sourced after a series of treatments in order to detect minimal residual disease, and wherein the targeting sequencing is performed utilizing nucleic acid probes to pull down sequences of genomic loci determined to contain a plurality of variants in phase, as determined by a prior sequencing result on a prior biopsy derived from the cancer; (b) identifying or having identified at least one set of the plurality of variants in phase within the cell-free nucleic acid sequencing result; and (c) treating the individual to curtail the cancer, based upon determining that the cell-free nucleic acid sequencing result contains nucleic acid sequences derived from the cancer.

In some embodiments of any of the compositions disclosed herein, the liquid or waste biopsy is one of blood, serum, cerebrospinal fluid, lymph fluid, urine or stool.

In some embodiments of any of the compositions disclosed herein, the treatment is chemotherapy, radiotherapy, immunotherapy, hormone therapy, targeted drug therapy, or medical surveillance.

In some embodiments, the method further comprises identifying or having identified one or more insertions or deletions (indels) within the cell-free nucleic acid sequencing result, and treating the individual to curtail the cancer, based least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises one or more insertions or deletions (indels) relative to a reference genomic sequence; and (c) analyzing, by the computer system, the one or more indels to determine a condition of the subject.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises one or more insertions or deletions (indels) relative to a reference genomic sequence; and (c) analyzing, by the computer system, the one or more insertions or deletions (indels) to determine a condition of the subject.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject; (b) processing the sequencing data to identify one or more cell-free nucleic acid molecules of the plurality of cell-free nucleic acid molecules with a limit of detection of less than about 1 out of 50,000 observations from the sequencing data, wherein each of the one or more cell-free nucleic acid molecules comprises one or more insertions or deletions (indels) relative to a reference genomic sequence; and (c) analyzing the identified one or more cell-free nucleic acid molecules to determine a condition of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, (a) to (c) are performed by a computer system. In some embodiments, the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error.

In one aspect, the present disclosure provides a method (not encompassed by the wording of the claims, but useful for understanding the invention) of treating a condition of a subject, the method comprising: (a) identifying the subject for treatment of the condition, wherein the subject has been determined to have the condition based on identification of one or more cell-free nucleic acid molecules from a plurality of cell-free nucleic acid molecules that is obtained or derived from the subject, wherein each of the one or more cell-free nucleic acid molecules comprises one or more insertions or deletions (indels) relative to a reference genomic sequence, and wherein a presence of the one or more indels is indicative of the condition of the subject; and (b) subjecting the subject to the treatment based on the identification in (a).

In one aspect, the present disclosure provides a method (not encompassed by the wording of the claims, but useful for understanding the invention) of monitoring a progress of a condition of a subject, the method comprising: (a) determining a first state of the condition of the subject based on identification of a first set of one or more cell-free nucleic acid molecules from a first plurality of cell-free nucleic acid molecules that is obtained or derived from the subject; (b) determining a second state of the condition of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining the progress of the condition based on the first state of the condition and the second state of the condition, wherein each of the one or more cell-free nucleic acid molecules comprises one or more insertions or deletions (indels) relative to a reference genomic sequence.

In some embodiments, the progress of the condition is worsening of the condition. In some embodiments, the progress of the condition is at least a partial remission of the condition. In some embodiments, a presence of the one or more indels is indicative of the first state or the second state of the condition of the subject. In some embodiments, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject. In some embodiments, the subject is subjected to a treatment for the condition (i) prior to obtaining the second plurality of cell-free nucleic acid molecules from the subject and (ii) subsequent to obtaining the first plurality of cell-free nucleic acid molecules from the subject. In some embodiments, the progress of the condition is indicative of minimal residual disease of the condition of the subject. In some embodiments, the progress of the condition is indicative of tumor burden or cancer burden of the subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the condition.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising one or more insertions or deletions (indels) relative to a reference genomic sequence, and wherein the individual nucleic acid probe comprises an activatable reporter agent, activation of the activatable reporter agent being selected from the group consisting of: (i) hybridization of the individual nucleic acid probe to the one or more indels and (ii) dehybridization of at least a portion of the individual nucleic acid probe that has been hybridized to the one or more indels; (b) detecting the activatable reporter agent that is activated, to identify one or more cell-free nucleic acid molecules of the plurality of cell-free nucleic acid molecules, wherein each of the one or more cell-free nucleic acid molecules comprises the one or more indels; and (c) analyzing the identified one or more cell-free nucleic acid molecules to determine a condition of the subject.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising one or more insertions or deletions (indels) relative to a reference genomic sequence, and wherein the individual nucleic acid probe comprises an activatable reporter agent, activation of the activatable reporter agent being selected from the group consisting of: (i) hybridization of the individual nucleic acid probe to the one or more indels and (ii) dehybridization of at least a portion of the individual nucleic acid probe that has been hybridized to the one or more indels; (b) detecting the activatable reporter agent that is activated, to identify one or more cell-free nucleic acid molecules of the plurality of cell-free nucleic acid molecules, wherein each of the one or more cell-free nucleic acid molecules comprises the one or more indels, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a condition of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, the activatable reporter agent is activated upon hybridization of the individual nucleic acid probe to the one or more indels. In some embodiments, the activatable reporter agent is activated upon dehybridization of at least a portion of the individual nucleic acid probe that has been hybridized to the one or more indels. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the one or more indels as different variables. In some embodiments, the analyzing of the identified one or more cell-free nucleic acid molecules is not based on other cell-free nucleic acid molecules of the plurality of cell-free nucleic acid molecules that do not comprise the one or more indels. In some embodiments, a number of the one or more indels from the identified one or more cell-free nucleic acid molecules is indicative of the condition of the subject. In some embodiments, a ratio of (i) the number of the one or more indels from the one or more cell-free nucleic acid molecules and (ii) a number of single nucleotide variants (SNVs) from the one or more cell-free nucleic acid molecules is indicative of the condition of the subject. In some embodiments, a frequency of the one or more indels in the identified one or more cell-free nucleic acid molecules is indicative of the condition of the subject. In some embodiments, the frequency is indicative of a diseased cell associated with the condition. In some embodiments, the condition is diffuse large B-cell lymphoma, and wherein the frequency is indicative of whether the one or more cell-free nucleic acid molecules are derived from germinal center B-cell (GCB) or activated B-cell (ABC). In some embodiments, genomic origin of the identified one or more cell-free nucleic acid molecules is indicative of the condition of the subject.

In some embodiments, the one or more indels comprises at least 3, at least 4, at least 5, at least 10, at least 15, at least 20, or at least 25 indels within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the subject. In some embodiments, the sample is a healthy sample. In some embodiments, the sample comprises a healthy cell. In some embodiments, the healthy cell comprises a healthy leukocyte. In some embodiments, the sample is a diseased sample. In some embodiments, the diseased sample comprises a diseased cell. In some embodiments, the diseased cell comprises a tumor cell. In some embodiments, the diseased sample comprises a solid tumor. In some embodiments, the set of nucleic acid probes is designed based on the one or more indels that are identified by comparing (i) sequencing data from a solid tumor, lymphoma, or blood tumor of the subject and (ii) sequencing data from a healthy cell of the subject or a healthy cohort. In some embodiments, the healthy cell is from the subject. In some embodiments, the healthy cell is from the healthy cohort. In some embodiments, the set of nucleic acid probes are designed to hybridize to at least a portion of sequences of genomic loci associated with the condition. In some embodiments, the genomic loci associated with the condition are known to exhibit aberrant somatic hypermutation when the subject has the condition.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, or (ii) the genomic regions identified in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6.

In some embodiments, the method further comprises determining that the subject has the condition or determining a degree or status of the condition of the subject, based on the identified one or more cell-free nucleic acid molecules comprising the one or more indels. In some embodiments, the method further comprises determining that the one or more cell-free nucleic acid molecules are derived from a sample associated with the condition, based on performing a statistical model analysis of the identified one or more cell-free nucleic acid molecules. In some embodiments, the statistical model analysis comprises a Monte Carlo statistical analysis. In some embodiments, the method further comprises monitoring a progress of the condition of the subject based on the identified one or more cell-free nucleic acid molecules. In some embodiments, the method further comprises performing a different procedure to confirm the condition of the subject. In some embodiments, the different procedure comprises a blood test, genetic test, medical imaging, physical exam, or tissue biopsy. In some embodiments, the method further comprises determining a treatment for the condition of the subject based on the identified one or more cell-free nucleic acid molecules. In some embodiments, the subject has been subjected to a treatment for the condition prior to (a). In some embodiments, the treatment comprises chemotherapy, radiotherapy, chemoradiotherapy, immunotherapy, adoptive cell therapy, hormone therapy, targeted drug therapy, surgery, transplant, transfusion, or medical surveillance. In some embodiments, the plurality of cell-free nucleic acid molecules comprises a plurality of cell-free deoxyribonucleic acid (DNA) molecules. In some embodiments, the condition comprises a disease. In some embodiments, the plurality of cell-free nucleic acid molecules is derived from a bodily sample of the subject. In some embodiments, the bodily sample comprises plasma, serum, blood, cerebrospinal fluid, lymph fluid, saliva, urine, or stool. In some embodiments, the subject is a mammal. In some embodiments, the subject is a human. In some embodiments, the condition comprises neoplasm, cancer, or tumor. In some embodiments, the condition comprises a solid tumor. In some embodiments, the condition comprises a lymphoma. In some embodiments, the condition comprises a B-cell lymphoma. In some embodiments, the condition comprises a sub-type of B-cell lymphoma selected from the group consisting of diffuse large B-cell lymphoma, follicular lymphoma, Burkitt lymphoma, and B-cell chronic lymphocytic leukemia. In some embodiments, the one or more indels have been previously identified as tumor-derived from sequencing a prior tumor sample or cell-free nucleic acid sample.

In one aspect, the present disclosure provides a method (not encompassed by the wording of the claims, but useful for understanding the invention) to perform a clinical procedure on an individual, the method comprising: obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; identifying or having identified one or more insertions or deletions (indels) within the cell-free nucleic acid sequencing result; determining or having determined, utilizing a statistical model and the identified one or more indels, that the cell-free nucleic acid sequencing result contains nucleotides derived from a neoplasm; and performing a clinical procedure on the individual to confirm the presence of the B-cell cancer, based upon determining that the cell-free nucleic acid sequencing result contains nucleic acid sequences likely derived from the B-cell cancer.

In some embodiments, the biopsy is one of blood, serum, cerebrospinal fluid, lymph fluid, urine, or stool. In some embodiments, the genomic loci are selected from (i) the genomic regions identified in Table 1, or (ii) the genomic regions identified in Table 3. In some embodiments, the sequences of the nucleic acid probes are selected from Table 6. In some embodiments, the clinical is procedure is a blood test, medical imaging, or a physical exam.

In one aspect, the present disclosure provides a method (not encompassed by the wording of the claims, but useful for understanding the invention) to treat an individual for a B-cell cancer, the method comprising: obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; identifying or having identified one or more insertions or deletions (indels) within the cell-free nucleic acid sequencing result; determining or having determined, utilizing a statistical model and the identified one or more indels, that the cell-free nucleic acid sequencing result contains nucleotides derived from a neoplasm; and treating the individual to curtail the B-cell cancer, based upon determining that the cell-free nucleic acid sequencing result contains nucleic acid sequences derived from the B-cell cancer.

In some embodiments, the biopsy is one of blood, serum, cerebrospinal fluid, lymph fluid, urine or stool. In some embodiments, the genomic loci are selected from (i) the genomic regions identified in Table 1, or (ii) the genomic regions identified in Table 3. In some embodiments, the sequences of the nucleic acid probes are selected from Table 6. In some embodiments, the treatment is chemotherapy, radiotherapy, immunotherapy, hormone therapy, targeted drug therapy, or medical surveillance.

In one aspect, the present disclosure provides a method (not encompassed by the wording of the claims, but useful for understanding the invention) to detect cancerous minimal residual disease in an individual and to treat the individual for a cancer, the method comprising: obtaining or having obtained a targeted sequencing result of a collection of cell-free nucleic acid molecules, wherein the collection of cell-free nucleic acid molecules are sourced from a liquid or waste biopsy of an individual, wherein the liquid or waste biopsy is sourced after a series of treatments in order to detect minimal residual disease, and wherein the targeting sequencing is performed utilizing nucleic acid probes to pull down sequences of genomic loci determined to contain one or more insertions or deletions (indels), as determined by a prior sequencing result on a prior biopsy derived from the cancer; identifying or having identified at least one set of the one or more indels within the cell-free nucleic acid sequencing result; and treating the individual to curtail the cancer, based upon determining that the cell-free nucleic acid sequencing result contains nucleic acid sequences derived from the cancer.

In some embodiments, the liquid or waste biopsy is one of blood, serum, cerebrospinal fluid, lymph fluid, urine or stool. In some embodiments, the treatment is chemotherapy, radiotherapy, immunotherapy, hormone therapy, targeted drug therapy, or medical surveillance.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject who has received an organ or tissue transplant; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a presence, an absence, or an extent of transplant rejection of the subject.

In some embodiments, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules. In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject who has received an organ or tissue transplant; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a presence, an absence, or an extent of transplant rejection of the subject.

In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject who has received an organ or tissue transplant; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a presence, an absence, or an extent of transplant rejection of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, each of the one or more cell-free nucleic acid molecules comprises a plurality of phased variants relative to a reference genomic sequence. In some embodiments, a first phased variant of the plurality of phased variants and a second phased variant of the plurality of phased variants are separated by at least one nucleotide. In some embodiments, (a) to (c) are performed by a computer system. In some embodiments, the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error. In some embodiments, (b) further comprises identifying one or more insertions or deletions (indels) in the one or more cell-free nucleic acid molecules, and wherein (c) further comprises determining the presence or the absence of the transplant rejection of the subject based at least in part on the identified one or more indels.

In one aspect, the present disclosure provides a method (not encompassed by the wording of the claims, but useful for understanding the invention) of treating a transplant rejection of a subject who has received an organ or tissue transplant, the method comprising: (a) identifying the subject for treatment of the transplant rejection, wherein the subject has been determined to have the transplant rejection based on identification of one or more cell-free nucleic acid molecules from a plurality of cell-free nucleic acid molecules that is obtained or derived from the subject, wherein each of the one or more cell-free nucleic acid molecules identified comprises a plurality of phased variants relative to a reference genomic sequence that are separated by at least one nucleotide, and wherein a presence of the plurality of phased variants is indicative of the transplant rejection of the subject; and (b) subjecting the subject to the treatment based on the identification in (a).

In some embodiments, the subject has been determined to have the transplant rejection based at least in part on one or more insertions or deletions (indels) identified in the one or more cell-free nucleic acid molecules.

In one aspect, the present disclosure provides a method (not encompassed by the wording of the claims, but useful for understanding the invention) of monitoring a subject who has received an organ or tissue transplant for a presence, an absence, or an extent of transplant rejection, the method comprising: (a) determining a first state of the presence, the absence, or the extent of transplant rejection of the subject based on identification of a first set of one or more cell-free nucleic acid molecules from a first plurality of cell-free nucleic acid molecules that is obtained or derived from the subject; (b) determining a second state of the presence, the absence, or the extent of transplant rejection of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining a transplant rejection status of the subject based on the first state and the second state, wherein each of the one or more cell-free nucleic acid molecules comprises a plurality of phased variants relative to a reference genomic sequence that are separated by at least one nucleotide.

In some embodiments, the transplant rejection status is at least a partial transplant rejection. In some embodiments, a presence of the plurality of phased variants is indicative of the first state or the second state. In some embodiments, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject. In some embodiments, the subject is subjected to a treatment for the transplant rejection (i) prior to obtaining the second plurality of cell-free nucleic acid molecules from the subject and (ii) subsequent to obtaining the first plurality of cell-free nucleic acid molecules from the subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the transplant rejection. In some embodiments, the subject has been determined to have the presence or the absence of the transplant rejection based at least in part on one or more insertions or deletions (indels) identified in the one or more cell-free nucleic acid molecules.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject who has received an organ or tissue transplant, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a presence, an absence, or an extent of transplant rejection of the subject.

In some embodiments, (b) further comprises identifying one or more insertions or deletions (indels) in the one or more cell-free nucleic acid molecules, and wherein (c) further comprises determining the presence or the absence of the transplant rejection of the subject based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject who has received an organ or tissue transplant, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a presence, an absence, or an extent of transplant rejection of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, a first phased variant of the plurality of phased variants and a second phased variant of the plurality of phased variants are separated by at least one nucleotide. In some embodiments, the activatable reporter agent is activated upon hybridization of the individual nucleic acid probe to the plurality of phased variants. In some embodiments, the activatable reporter agent is activated upon dehybridization of at least a portion of the individual nucleic acid probe that has been hybridized to the plurality of phased variants. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables. In some embodiments, the analyzing of the identified one or more cell-free nucleic acid molecules is not based on other cell-free nucleic acid molecules of the plurality of cell-free nucleic acid molecules that do not comprise the plurality of phased variants. In some embodiments, a number of the plurality of phased variants from the identified one or more cell-free nucleic acid molecules is indicative of the presence, the absence, or the extent of transplant rejection of the subject. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the presence, the absence, or the extent of transplant rejection of the subject. In some embodiments, a frequency of the plurality of phased variants in the identified one or more cell-free nucleic acid molecules is indicative of the presence or the absence of the transplant rejection of the subject. In some embodiments, the frequency is indicative of a diseased cell associated with the presence, the absence, or the extent of transplant rejection. In some embodiments, genomic origin of the identified one or more cell-free nucleic acid molecules is indicative of the presence or the absence of the transplant rejection of the subject. In some embodiments, the first and second phased variants are separated by at least 2, at least 3, at least 4, at least 5, at least 6, at least 7, or at least 8 nucleotides. In some embodiments, the first and second phased variants are separated by at most about 180, at most about 170, at most about 160, at most about 150, or at most about 140 nucleotides.

In some embodiments, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV. In some embodiments, the plurality of phased variants comprises at least 3, at least 4, at least 5, at least 10, at least 15, at least 20, or at least 25 phased variants within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the subject. In some embodiments, the sample is a healthy sample. In some embodiments, the sample comprises a healthy cell. In some embodiments, the healthy cell comprises a healthy leukocyte. In some embodiments, the sample is a diseased sample. In some embodiments, the diseased sample comprises a diseased cell. In some embodiments, the healthy cell is from the subject. In some embodiments, the healthy cell is from the healthy cohort. In some embodiments, the set of nucleic acid probes are designed to hybridize to at least a portion of sequences of genomic loci associated with the presence or the absence of the transplant rejection. In some embodiments, the genomic loci associated with the presence, the absence, or the extent of transplant rejection are known to exhibit aberrant somatic hypermutation when the subject has the transplant rejection.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6. In some embodiments, the method further comprises determining the presence or the absence of the transplant rejection or determining a degree or status thereof, based on the identified one or more cell-free nucleic acid molecules comprising the plurality of phased variants. In some embodiments, the method further comprises determining that the one or more cell-free nucleic acid molecules are derived from a sample associated with the presence or the absence of the transplant rejection, based on performing a statistical model analysis of the identified one or more cell-free nucleic acid molecules. In some embodiments, the statistical model analysis comprises a Monte Carlo statistical analysis. In some embodiments, the method further comprises monitoring a progress of the presence, the absence, or the extent of transplant rejection of the subject based on the identified one or more cell-free nucleic acid molecules. In some embodiments, the method further comprises performing a different procedure to confirm the presence, the absence, or the extent of transplant rejection of the subject. In some embodiments, the different procedure comprises a blood test, genetic test, medical imaging, physical exam, or tissue biopsy. In some embodiments, the method further comprises determining a treatment for the transplant rejection of the subject based on the identified one or more cell-free nucleic acid molecules. In some embodiments, the subject has been subjected to a treatment for the transplant rejection prior to (a). In some embodiments, the plurality of cell-free nucleic acid molecules comprises a plurality of cell-free deoxyribonucleic acid (DNA) molecules. In some embodiments, the plurality of cell-free nucleic acid molecules are derived from a bodily sample of the subject. In some embodiments, the bodily sample comprises plasma, serum, blood, cerebrospinal fluid, lymph fluid, saliva, urine, or stool. In some embodiments, the subject is a mammal. In some embodiments, the subject is a human. In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a pregnant subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a presence, an absence, or an elevated risk of a genetic abnormality of a fetus of the pregnant subject.

In some embodiments, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a pregnant subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a presence, an absence, or an elevated risk of a genetic abnormality of a fetus of the pregnant subject.

In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a pregnant subject; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a presence, an absence, or an elevated risk of a genetic abnormality of a fetus of the pregnant subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, each of the one or more cell-free nucleic acid molecules comprises a plurality of phased variants relative to a reference genomic sequence. In some embodiments, a first phased variant of the plurality of phased variants and a second phased variant of the plurality of phased variants are separated by at least one nucleotide. In some embodiments, (a) to (c) are performed by a computer system. In some embodiments, he method of any one of claims 309-313, wherein the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) of monitoring a pregnant subject for a presence, an absence, or an elevated risk of a genetic abnormality of a fetus of the pregnant subject, the method comprising: (a) determining a first state of the presence, the absence, or the elevated risk of the genetic abnormality of the fetus of the pregnant subject based on identification of a first set of one or more cell-free nucleic acid molecules from a first plurality of cell-free nucleic acid molecules that is obtained or derived from the pregnant subject; (b) determining a second state of the presence, the absence, or the elevated risk of the genetic abnormality of the fetus of the pregnant subject based on identification of a second set of one or more cell-free nucleic acid molecules from a second plurality of cell-free nucleic acid molecules that is obtained or derived from the pregnant subject, wherein the second plurality of cell-free nucleic acid molecules are obtained from the pregnant subject subsequent to obtaining the first plurality of cell-free nucleic acid molecules from the pregnant subject; and (c) determining the presence, the absence, or the elevated risk of the genetic abnormality of the fetus of the pregnant subject based on the first state and the second state, wherein each of the one or more cell-free nucleic acid molecules comprises a plurality of phased variants relative to a reference genomic sequence that are separated by at least one nucleotide.

In some embodiments, the transplant rejection status is at least a partial transplant rejection. In some embodiments, a presence of the plurality of phased variants is indicative of the first state or the second state. In some embodiments, the second plurality of cell-free nucleic acid molecules is obtained from the pregnant subject at least about 1 week, at least about 2 weeks, at least about 3 weeks, at least about 4 weeks, at least about 2 months, or at least about 3 months subsequent to obtaining the first plurality of cell-free nucleic acid molecules from the pregnant subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the genetic abnormality. In some embodiments, the fetus has been determined to have the presence, the absence, or the elevated risk of the genetic abnormality based at least in part on one or more insertions or deletions (indels) identified in the one or more cell-free nucleic acid molecules.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a pregnant subject, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a presence, an absence, or an elevated risk of a genetic abnormality of a fetus of the pregnant subject.

In some embodiments, (b) further comprises identifying one or more insertions or deletions (indels) in the one or more cell-free nucleic acid molecules, and wherein (c) further comprises determining the presence, the absence, or the elevated risk of the genetic abnormality based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a pregnant subject, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a presence, an absence, or an elevated risk of a genetic abnormality of a fetus of the pregnant subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, a first phased variant of the plurality of phased variants and a second phased variant of the plurality of phased variants are separated by at least one nucleotide. In some embodiments, the activatable reporter agent is activated upon hybridization of the individual nucleic acid probe to the plurality of phased variants. In some embodiments, the activatable reporter agent is activated upon dehybridization of at least a portion of the individual nucleic acid probe that has been hybridized to the plurality of phased variants. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables. In some embodiments, the analyzing of the identified one or more cell-free nucleic acid molecules is not based on other cell-free nucleic acid molecules of the plurality of cell-free nucleic acid molecules that do not comprise the plurality of phased variants. In some embodiments, a number of the plurality of phased variants from the identified one or more cell-free nucleic acid molecules is indicative of the genetic abnormality. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the genetic abnormality. In some embodiments, a frequency of the plurality of phased variants in the identified one or more cell-free nucleic acid molecules is indicative of the genetic abnormality. In some embodiments, genomic origin of the identified one or more cell-free nucleic acid molecules is indicative of the genetic abnormality. In some embodiments, the first and second phased variants are separated by at least 2, at least 3, at least 4, at least 5, at least 6, at least 7, or at least 8 nucleotides. In some embodiments, the first and second phased variants are separated by at most about 180, at most about 170, at most about 160, at most about 150, or at most about 140 nucleotides.

In some embodiments, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV. In some embodiments, the plurality of phased variants comprises at least 3, at least 4, at least 5, at least 10, at least 15, at least 20, or at least 25 phased variants within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the pregnant subject. In some embodiments, the sample is a healthy sample. In some embodiments, the sample comprises a healthy cell. In some embodiments, the sample is a diseased sample. In some embodiments, the diseased sample comprises a diseased cell. In some embodiments, the healthy cell is from the pregnant subject. In some embodiments, the healthy cell is from the healthy cohort. In some embodiments, the set of nucleic acid probes are designed to hybridize to at least a portion of sequences of genomic loci associated with the genetic abnormality.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6. In some embodiments, the method further comprises determining the presence, the absence, or the elevated risk of the genetic abnormality of the fetus of the pregnant subject, based on the identified one or more cell-free nucleic acid molecules comprising the plurality of phased variants. In some embodiments, the method further comprises determining that the one or more cell-free nucleic acid molecules are derived from a sample associated with the presence, the absence, or the elevated risk of the genetic abnormality of the fetus of the pregnant subject, based on performing a statistical model analysis of the identified one or more cell-free nucleic acid molecules. In some embodiments, the statistical model analysis comprises a Monte Carlo statistical analysis. In some embodiments, the method further comprises monitoring a progress of the presence, the absence, or the elevated risk of the genetic abnormality of the fetus of the pregnant subject based on the identified one or more cell-free nucleic acid molecules. In some embodiments, the method further comprises performing a different procedure to confirm the presence, the absence, or the elevated risk of the genetic abnormality of the fetus of the pregnant subject. In some embodiments, the different procedure comprises a blood test, genetic test, medical imaging, physical exam, or tissue biopsy. In some embodiments, the plurality of cell-free nucleic acid molecules comprise a plurality of cell-free deoxyribonucleic acid (DNA) molecules. In some embodiments, the plurality of cell-free nucleic acid molecules are derived from a bodily sample of the pregnant subject. In some embodiments, the bodily sample comprises plasma, serum, blood, cerebrospinal fluid, lymph fluid, saliva, urine, or stool. In some embodiments, the pregnant subject is a mammal. In some embodiments, the pregnant subject is a human. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels.

In one aspect, the present disclosure provides a method comprising adding a set of nucleic acid probes to a sample comprising a plurality of nucleic acid molecules that have been obtained or derived from a subject, wherein each nucleic acid probe of the set of nucleic acid probes is configured to hybridize to a target nucleic acid molecule comprising a plurality of phased variants such that the nucleic acid probe is complementary to at least a region of the target nucleic acid molecule that extends from a first phased variant of the plurality of phased variants to a second phased variant of the plurality of phased variants. (For clarity, the region includes both the first phased variant and the second phased variant.)

This method, and embodiments of it described herein, may involve the use of hybrid capture probes/baits, such as biotinylated oligonucleotides, that may be used in a hybrid capture enrichment step such that the hybrid capture probes bind to and preferentially capture nucleic acid molecules that contain phased variants. Such hybrid capture approaches may increase the capture sensitivity of circulating tumor DNA or circulating DNA from a transplanted organ. The hybrid capture probes can be synthesized to specifically target molecules containing phased variants by designing the hybrid capture probe to (1) contain a sequence that is complementary to the molecule that includes the phased variant (as opposed to the corresponding region of the reference genomic sequence) and (2) have a length that optimizes the nucleic acid binding kinetics/thermodynamics ($\Delta G$ or binding energy) such that the hybrid capture probe preferentially binds to a nucleic acid molecule that contains the phased variants of interest as compared to corresponding molecules without the phased variants. Such hybrid capture probes can lead to improved enrichment of relevant nucleic acid sequences, thereby requiring less sequencing as a result. For instance, in some cases (such as in assessing minimal residual disease, disease state, or state of transplant rejection), a cancerous sample or a sample from the transplanted organ may be obtained and sequenced to identify phased variants in such samples relative to a reference genomic sequence, such as a sequence from corresponding healthy cell(s) of the subject, and the hybrid capture probes can be designed to preferentially bind to nucleic acid sequences containing the phased variants identified from the cancerous and/or transplanted organ samples. In some circumstances, such hybrid capture probes can be used for single strand recovery of nucleic acid molecules that contain phased variants. The nucleic acid molecules captured by such probe sets can include DNA or RNA (e.g., single stranded RNA), such as cell-free DNA or cell-free DNA. Probes as described in this particular method can be used on combination with other methods described herein.

In some embodiments, each nucleic acid probe of the set of nucleic acid probes comprises a pull-down tag, such as biotin. In some embodiments, the method further comprises separation of target nucleic acid molecules that hybridize to the nucleic acid probes from nucleic acid molecules that do not hybridize to the nucleic acid probes to thereby capture target nucleic acid molecules. In some embodiments, the nucleic acid molecules are cell-free nucleic acid molecules. In some embodiments, the first phased variant is selected from the group consisting of a somatic single nucleotide variant, a somatic indel, a somatic translocation breakpoint, a somatic amplification or deletion breakpoint, a germline SNV, a germline indel, a germline translocation breakpoint, a germline amplification or deletion breakpoint, and a region of localized hypermutation, and the second phased variant is selected from the group consisting of a somatic single nucleotide variant, a somatic indel, a somatic translocation breakpoint, a somatic amplification or deletion breakpoint, a germline SNV, a germline indel, a germline translocation breakpoint, a germline amplification or deletion breakpoint, and a region of localized hypermutation. In some embodiments, the first phased variant of the plurality of phased variants and the second phased variant of the plurality of phased variants are separated by at least 1, 2, 3, 4, 5, 10, or 20 nucleotides. In some embodiments, each nucleic acid probe of the set of nucleic acid probes is either (1) less than 40 nucleotides, less than 30 nucleotides, or less than 20 nucleotides in length or (2) no more than 5 nucleotides, nor more than 10 nucleotides, no more than 20 nucleotides, or no more than 30 nucleotides longer than the distance between the first phased variant of the plurality of phased variants and the second phased variant of the plurality of phased variants, wherein the first phased variant and the second phased variant are the most separated phased variants (i.e., have the most number of intervening nucleotides) of the plurality of phased variants.

In some embodiments, the target nucleic acid molecule is a molecule that is derived from a pre-identified portion of a genome of a cancer cell or a transplanted cell from the subject that differs in sequence from a reference genomic sequence, wherein the preidentified portion of the genome is less than 200, less than 180, or less than 150 nucleotides in length. In some embodiments, each nucleic acid probe of the plurality of nucleic acid probes has a lower $\Delta G$ of binding to the target nucleic acid molecule than to a corresponding molecule that is identical in length and sequence to the target nucleic acid molecule except that the corresponding molecule has a sequence that corresponds with a reference genomic sequence. In some embodiments, the reference genomic sequence comprises a portion of either (1) a reference cohort, such as a portion of the hg19 human genome, hg18 genome, hg17 genome, hg16 genome, or hg38 genome or (2) a healthy sample from the subject. In some embodiments, the method involves the capture of the target nucleic acid derived from either the Watson strand or the Crick strand of a chromosome, but does not involve the capture of the corresponding complementary nucleic acid of the other strand. In some embodiments, the method comprises capture of at least 10, at least 100, at least 1000, or at least 10,000 target nucleic acid molecules. In some embodiments, the method further comprises sequencing the captured target nucleic acids to obtain sequencing data derived from the plurality of nucleic acid molecules. In some embodiments, the sequencing does not involve use of molecular barcodes. In some embodiments, the sequencing does not comprise duplex sequencing.

In one aspect, the present disclosure provides a method (not encompassed by the wording of the claims, but useful for understanding the invention) for determining a condition of a subject (e.g., assessing minimal residual disease, disease progression, or transplant rejection status), the method comprising obtaining, by a computer system, sequence information obtained by any method described herein involving the use of hybrid capture probes that are designed to bind preferentially to molecules that contain phased variants as compared to corresponding molecules that lack phased variants; processing, by the computer system, the sequencing data to identify one or more nucleic acid molecules of the plurality of nucleic acid molecules, wherein each of the one or more nucleic acid molecules comprises a plurality of phased variants relative to a reference genomic sequence; and analyzing, by the computer system, the identified one or more nucleic acid molecules to determine a condition of the subject. In some embodiments, such methods do not comprise duplex-mediated error suppression or barcode-mediated error suppression. Individuals may be treated (e.g., with anti-cancer agents, anti-rejection agents, or surgical procedures) based on the identification of a condition (e.g., state) of the subject.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a condition of the subject. In some embodiments, cellular DNA is used instead of cell-free DNA (e.g., for detection of leukemia or other hematological cancers).

In some embodiments of any one of the methods disclosed herein, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules.

In some embodiments, (b) further comprises identifying one or more insertions or deletions (indels) in the one or more cell-free nucleic acid molecules, and (c) further comprises determining the condition of the subject based at least in part on the identified one or more indels.

In some embodiments, the method further comprises determining the start position (i.e., the 5'-most nucleotide) and the end position (i.e., the 3'-most nucleotide) in a molecule. In some cases, tumor-derived nucleic acids, such as tumor-derived cfDNA molecules can have stereotyped start/end positions, which may reflect cleavage by tissue-specific nucleases. The start and end positions can be used-in connection with phased variants-to identify a condition of a subject.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a condition of the subject.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a condition of the subject.

In some embodiments of any one of the methods disclosed herein, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data.

In some embodiments of any one of the methods disclosed herein, each of the one or more cell-free nucleic acid molecules comprises a plurality of phased variants relative to a reference genomic sequence. In some embodiments of any one of the methods disclosed herein, a first phased variant of the plurality of phased variants and a second phased variant of the plurality of phased variants are separated by at least one nucleotide.

In some embodiments of any one of the methods disclosed herein, the processes (a) to (c) are performed by a computer system.

In some embodiments of any one of the methods disclosed herein, the sequencing data is generated based on nucleic acid amplification. In some embodiments of any one of the methods disclosed herein, the sequencing data is generated based on polymerase chain reaction. In some embodiments of any one of the methods disclosed herein, the sequencing data is generated based on amplicon sequencing.

In some embodiments of any one of the methods disclosed herein, the sequencing data is generated based on next-generation sequencing (NGS). Alternatively, in some embodiments of any one of the methods disclosed herein, the sequencing data is generated based on non-hybridization-based NGS.

In some embodiments of any one of the methods disclosed herein, the sequencing data is generated without use of molecular barcoding of at least a portion of the plurality of cell-free nucleic acid molecules. In some embodiments of any one of the methods disclosed herein, the sequencing data is obtained without use of sample barcoding of at least a portion of the plurality of cell-free nucleic acid molecules.

In some embodiments of any one of the methods disclosed herein, the sequencing data is obtained without in silico removal or suppression of (i) background error or (ii) sequencing error.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) of treating a condition of a subject, the method comprising: (a) identifying the subject for treatment of the condition, wherein the subject has been determined to have the condition based on identification of one or more cell-free nucleic acid molecules from a plurality of cell-free nucleic acid molecules that is obtained or derived from the subject, wherein each of the one or more cell-free nucleic acid molecules identified comprises a plurality of phased variants relative to a reference genomic sequence that are separated by at least one nucleotide, and wherein a presence of the plurality of phased variants is indicative of the condition of the subject; and (b) subjecting the subject to the treatment based on the identification in (a).

In some embodiments, the subject has been determined to have the condition based at least in part on one or more insertions or deletions (indels) identified in the one or more cell-free nucleic acid molecules.

In one aspect, the present disclosure provides a method (not encompassed by the wording of the claims, but useful for understanding the invention) of monitoring a progress of a condition of a subject, the method comprising: (a) determining a first state of the condition of the subject based on identification of a first set of one or more cell-free nucleic acid molecules from a first plurality of cell-free nucleic acid molecules that is obtained or derived from the subject; (b) determining a second state of the condition of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining the progress of the condition based on the first state of the condition and the second state of the condition, wherein each of the one or more cell-free nucleic acid molecules comprises a plurality of phased variants relative to a reference genomic sequence that are separated by at least one nucleotide.

In some embodiments of any one of the methods disclosed herein, the progress of the condition is worsening of the condition.

In some embodiments of any one of the methods disclosed herein, the progress of the condition is at least a partial remission of the condition.

In some embodiments of any one of the methods disclosed herein, a presence of the plurality of phased variants is indicative of the first state or the second state of the condition of the subject.

In some embodiments of any one of the methods disclosed herein, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject.

In some embodiments of any one of the methods disclosed herein, the subject is subjected to a treatment for the condition (i) prior to obtaining the second plurality of cell-free nucleic acid molecules from the subject and (ii) subsequent to obtaining the first plurality of cell-free nucleic acid molecules from the subject.

In some embodiments of any one of the methods disclosed herein, the progress of the condition is indicative of minimal residual disease of the condition of the subject. In some embodiments of any one of the methods disclosed herein, the progress of the condition is indicative of tumor burden or cancer burden of the subject.

In some embodiments of any one of the methods disclosed herein, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the condition.

In some embodiments, the subject has been determined to have the condition based at least in part on one or more insertions or deletions (indels) identified in the one or more cell-free nucleic acid molecules.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a condition of the subject.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a condition of the subject.

In some embodiments of any one of the methods disclosed herein, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules.

In some embodiments of any one of the methods disclosed herein, a first phased variant of the plurality of phased variants and a second phased variant of the plurality of phased variants are separated by at least one nucleotide.

In some embodiments of any one of the methods disclosed herein, the activatable reporter agent is activated upon hybridization of the individual nucleic acid probe to the plurality of phased variants.

In some embodiments of any one of the methods disclosed herein, the activatable reporter agent is activated upon dehybridization of at least a portion of the individual nucleic acid probe that has been hybridized to the plurality of phased variants.

In some embodiments of any one of the methods disclosed herein, the method further comprises mixing (1) the set of nucleic acid probes and (2) the plurality of cell-free nucleic acid molecules.

In some embodiments of any one of the methods disclosed herein, the activatable reporter agent is a fluorophore.

In some embodiments of any one of the methods disclosed herein, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables.

In some embodiments of any one of the methods disclosed herein, the analyzing of the identified one or more cell-free nucleic acid molecules is not based on other cell-free nucleic acid molecules of the plurality of cell-free nucleic acid molecules that do not comprise the plurality of phased variants.

In some embodiments of any one of the methods disclosed herein, a number of the plurality of phased variants from the identified one or more cell-free nucleic acid molecules is indicative of the condition of the subject. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the condition of the subject.

In some embodiments of any one of the methods disclosed herein, a frequency of the plurality of phased variants in the identified one or more cell-free nucleic acid molecules is indicative of the condition of the subject. In some embodiments, the frequency is indicative of a diseased cell associated with the condition. In some embodiments, the condition is diffuse large B-cell lymphoma, and wherein the frequency is indicative of whether the one or more cell-free nucleic acid molecules are derived from germinal center B-cell (GCB) or activated B-cell (ABC).

In some embodiments of any one of the methods disclosed herein, genomic origin of the identified one or more cell-free nucleic acid molecules is indicative of the condition of the subject.

In some embodiments of any one of the methods disclosed herein, the first and second phased variants are separated by at least 2, at least 3, at least 4, at least 5, at least 6, at least 7, or at least 8 nucleotides. In some embodiments of any one of the methods disclosed herein, the first and second phased variants are separated by at most about 180, at most about 170, at most about 160, at most about 150, or at most about 140 nucleotides.

In some embodiments of any one of the methods disclosed herein, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV.

In some embodiments of any one of the methods disclosed herein, the plurality of phased variants comprises at least 3, at least 4, at least 5, at least 10, at least 15, at least 20, or at least 25 phased variants within the same cell-free nucleic acid molecule.

In some embodiments of any one of the methods disclosed herein, the one or more cell-free nucleic acid molecules identified comprises at least 2, at least 3, at least 4, at least 5, at least 10, at least 50, at least 100, at least 500, or at least 1,000 cell-free nucleic acid molecules.

In some embodiments of any one of the methods disclosed herein, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome.

In some embodiments of any one of the methods disclosed herein, the reference genomic sequence is derived from a sample of the subject.

In some embodiments of any one of the methods disclosed herein, the sample is a healthy sample. In some embodiments, the sample comprises a healthy cell. In some embodiments, the healthy cell comprises a healthy leukocyte.

In some embodiments of any one of the methods disclosed herein, the sample is a diseased sample. In some embodiments, the diseased sample comprises a diseased cell. In some embodiments, the diseased cell comprises a tumor cell. In some embodiments, the diseased sample comprises a solid tumor.

In some embodiments of any one of the methods disclosed herein, the set of nucleic acid probes is designed based on the plurality of phased variants that are identified by comparing (i) sequencing data from a solid tumor, lymphoma, or blood tumor of the subject and (ii) sequencing data from a healthy cell of the subject or a healthy cohort. In some embodiments, the healthy cell is from the subject. In some embodiments, the healthy cell is from the healthy cohort.

In some embodiments of any one of the methods disclosed herein, the set of nucleic acid probes are designed to hybridize to at least a portion of sequences of genomic loci associated with the condition. In some embodiments, the genomic loci associated with the condition are known to exhibit aberrant somatic hypermutation when the subject has the condition.

In some embodiments of any one of the methods disclosed herein, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3.

In some embodiments of any one of the methods disclosed herein, each nucleic acid probe of the set of nucleic acid probes has at least about 70%, at least about 80%, at least about 90% sequence identity, at least about 95% sequence identity, or about 100% sequence identity to a probe sequence selected from Table 6.

In some embodiments of any one of the methods disclosed herein, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6.

In some embodiments of any one of the methods disclosed herein, the method further comprises determining that the subject has the condition or determining a degree or status of the condition of the subject, based on the identified one or more cell-free nucleic acid molecules comprising the plurality of phased variants. In some embodiments, the method further comprises determining that the one or more cell-free nucleic acid molecules are derived from a sample associated with the condition, based on performing a statistical model analysis of the identified one or more cell-free nucleic acid molecules. In some embodiments, the statistical model analysis comprises a Monte Carlo statistical analysis.

In some embodiments of any one of the methods disclosed herein, the method further comprises monitoring a progress of the condition of the subject based on the identified one or more cell-free nucleic acid molecules.

In some embodiments of any one of the methods disclosed herein, the method further comprises performing a different procedure to confirm the condition of the subject. In some embodiments, the different procedure comprises a blood test, genetic test, medical imaging, physical exam, or tissue biopsy.

In some embodiments of any one of the methods disclosed herein, the method further comprises determining a treatment for the condition of the subject based on the identified one or more cell-free nucleic acid molecules.

In some embodiments of any one of the methods disclosed herein, the subject has been subjected to a treatment for the condition prior to (a).

In some embodiments of any one of the methods disclosed herein, the treatment comprises chemotherapy, radiotherapy, chemoradiotherapy, immunotherapy, adoptive cell therapy, hormone therapy, targeted drug therapy, surgery, transplant, transfusion, or medical surveillance.

In some embodiments of any one of the methods disclosed herein, the plurality of cell-free nucleic acid molecules comprises a plurality of cell-free deoxyribonucleic acid (DNA) molecules.

In some embodiments of any one of the methods disclosed herein, condition comprises a disease.

In some embodiments of any one of the methods disclosed herein, the plurality of cell-free nucleic acid molecules are derived from a bodily sample of the subject. In some embodiments, the bodily sample comprises plasma, serum, blood, cerebrospinal fluid, lymph fluid, saliva, urine, or stool.

In some embodiments of any one of the methods disclosed herein, the subject is a mammal. In some embodiments of any one of the methods disclosed herein, the subject is a human.

In some embodiments of any one of the methods disclosed herein, the condition comprises neoplasm, cancer, or tumor. In some embodiments, the condition comprises a solid tumor. In some embodiments, the condition comprises a lymphoma. In some embodiments, the condition comprises a B-cell lymphoma. In some embodiments, the condition comprises a sub-type of B-cell lymphoma selected from the group consisting of diffuse large B-cell lymphoma, follicular lymphoma, Burkitt lymphoma, and B-cell chronic lymphocytic leukemia. In some embodiments of any one of the methods disclosed herein, the condition comprises transplant rejection of or a chromosomal abnormality.

In some embodiments of any one of the methods disclosed herein, the plurality of phased variants have been previously identified as tumor-derived from sequencing a prior tumor sample or cell-free nucleic acid sample.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a composition (not encompassed by the wording of the claims, but useful for understanding the invention) comprising a bait set comprising a set of nucleic acid probes designed to capture cell-free DNA molecules derived from at least about 5% of genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the set of nucleic acid probes are designed to pull down cell-free DNA molecules derived from at least about 10%, at least about 20%, at least about 30%, at least about 40%, at least about 50%, at least about 60%, at least about 70%, at least about 80%, at least about 90%, or about 100% of the genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the set of nucleic acid probes are designed to capture the one or more cell-free DNA molecules derived from at most about 10%, at most about 20%, at most about 30%, at most about 40%, at most about 50%, at most about 60%, at most about 70%, at most about 80%, at most about 90%, or about 100% of the genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the bait set comprises at most 5, at most 10, at most 50, at most 100, at most 500, at most 1000, or at most 2000 nucleic acid probes.

In some embodiments of any of the compositions disclosed herein, an individual nucleic acid probe of the set of nucleic acid probes comprises a pull-down tag.

In some embodiments of any of the compositions disclosed herein, the pull-down tag comprises a nucleic acid barcode.

In some embodiments of any of the compositions disclosed herein, the pull-down tag comprises biotin.

In some embodiments of any of the compositions disclosed herein, each of the cell-free DNA molecules is between about 100 nucleotides and about 180 nucleotides in length.

In some embodiments of any of the compositions disclosed herein, the genomic regions are associated with a condition.

In some embodiments of any of the compositions disclosed herein, the genomic regions exhibit aberrant somatic hypermutation when a subject has the condition.

In some embodiments of any of the compositions disclosed herein, the condition comprises a B-cell lymphoma. In some embodiments, the condition comprises a sub-type of B-cell lymphoma selected from the group consisting of diffuse large B-cell lymphoma, follicular lymphoma, Burkitt lymphoma, and B-cell chronic lymphocytic leukemia.

In some embodiments of any of the compositions disclosed herein, the composition further comprises a plurality of cell-free DNA molecules obtained or derived from a subject.

In one aspect, the present disclosure provides a method (not encompassed by the wording of the claims, but useful for understanding the invention) to perform a clinical procedure on an individual, the method comprising: (a) obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; (b) identifying or having identified a plurality of variants in phase within the cell-free nucleic acid sequencing result; (c) determining or having determined, utilizing a statistical model and the identified phased variants, that the cell-free nucleic acid sequencing result contains nucleotides derived from a neoplasm; and (d) performing a clinical procedure on the individual to confirm the presence of the B-cell cancer, based upon determining that the cell-free nucleic acid sequencing result contains nucleic acid sequences likely derived from the B-cell cancer.

In some embodiments of any of the compositions disclosed herein, the biopsy is one of blood, serum, cerebrospinal fluid, lymph fluid, urine, or stool.

In some embodiments of any of the compositions disclosed herein, the genomic loci are selected from (i) the genomic regions identified in Table 1, (ii) the genomic regions identified in Table 3, or (iii) the genomic regions identified to have a plurality of phased variants in Table 3.

In some embodiments of any of the compositions disclosed herein, the sequences of the nucleic acid probes are selected from Table 6.

In some embodiments of any of the compositions disclosed herein, the clinical is procedure is a blood test, medical imaging, or a physical exam.

In some embodiments, the method further comprises identifying or having identified one or more insertions or deletions (indels) within the cell-free nucleic acid sequencing result, and determining or having determined, based least in part on the identified one or more indels, that the cell-free nucleic acid sequencing result contains the nucleotides derived from the neoplasm.

In one aspect, the present disclosure provides a method (not encompassed by the wording of the claims, but useful for understanding the invention) to treat an individual for a B-cell cancer, the method comprising: (a) obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; (b) identifying or having identified a plurality of variants in phase within the cell-free nucleic acid sequencing result; (c) determining or having determined, utilizing a statistical model and the identified phased variants, that the cell-free nucleic acid sequencing result contains nucleotides derived from a neoplasm; and (d) treating the individual to curtail the B-cell cancer, based upon determining that the cell-free nucleic acid sequencing result contains nucleic acid sequences derived from the B-cell cancer.

In some embodiments of any of the compositions disclosed herein, the biopsy is one of blood, serum, cerebrospinal fluid, lymph fluid, urine or stool.

In some embodiments of any of the compositions disclosed herein, the genomic loci are selected from (i) the genomic regions identified in Table 1, (ii) the genomic regions identified in Table 3, or (iii) the genomic regions identified to have a plurality of phased variants in Table 3.

In some embodiments of any of the compositions disclosed herein, the sequences of the nucleic acid probes are selected from Table 6.

In some embodiments of any of the compositions disclosed herein, the treatment is chemotherapy, radiotherapy, immunotherapy, hormone therapy, targeted drug therapy, or medical surveillance.

In some embodiments, the method further comprises identifying or having identified one or more insertions or deletions (indels) within the cell-free nucleic acid sequencing result, and determining or having determined, based least in part on the identified one or more indels, that the cell-free nucleic acid sequencing result contains the nucleotides derived from the neoplasm.

In one aspect, the present disclosure provides a method (not encompassed by the wording of the claims, but useful for understanding the invention) to detect cancerous minimal residual disease in an individual and to treat the individual for a cancer, the method comprising: (a) obtaining or having obtained a targeted sequencing result of a collection of cell-free nucleic acid molecules, wherein the collection of cell-free nucleic acid molecules are sourced from a liquid or waste biopsy of an individual, wherein the liquid or waste biopsy is sourced after a series of treatments in order to detect minimal residual disease, and wherein the targeting sequencing is performed utilizing nucleic acid probes to pull down sequences of genomic loci determined to contain a plurality of variants in phase, as determined by a prior sequencing result on a prior biopsy derived from the cancer; (b) identifying or having identified at least one set of the plurality of variants in phase within the cell-free nucleic acid sequencing result; and (c) treating the individual to curtail the cancer, based upon determining that the cell-free nucleic acid sequencing result contains nucleic acid sequences derived from the cancer.

In some embodiments of any of the compositions disclosed herein, the liquid or waste biopsy is one of blood, serum, cerebrospinal fluid, lymph fluid, urine or stool.

In some embodiments of any of the compositions disclosed herein, the treatment is chemotherapy, radiotherapy, immunotherapy, hormone therapy, targeted drug therapy, or medical surveillance.

In some embodiments, the method further comprises identifying or having identified one or more insertions or deletions (indels) within the cell-free nucleic acid sequencing result, and treating the individual to curtail the cancer, based least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises one or more insertions or deletions (indels) relative to a reference genomic sequence; and (c) analyzing, by the computer system, the one or more indels to determine a condition of the subject.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises one or more insertions or deletions (indels) relative to a reference genomic sequence; and (c) analyzing, by the computer system, the one or more insertions or deletions (indels) to determine a condition of the subject.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject; (b) processing the sequencing data to identify one or more cell-free nucleic acid molecules of the plurality of cell-free nucleic acid molecules with a limit of detection of less than about 1 out of 50,000 observations from the sequencing data, wherein each of the one or more cell-free nucleic acid molecules comprises one or more insertions or deletions (indels) relative to a reference genomic sequence; and (c) analyzing the identified one or more cell-free nucleic acid molecules to determine a condition of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, (a) to (c) are performed by a computer system. In some embodiments, the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error.

In one aspect, the present disclosure provides a method (not encompassed by the wording of the claims, but useful for understanding the invention) of treating a condition of a subject, the method comprising: (a) identifying the subject for treatment of the condition, wherein the subject has been determined to have the condition based on identification of one or more cell-free nucleic acid molecules from a plurality of cell-free nucleic acid molecules that is obtained or derived from the subject, wherein each of the one or more cell-free nucleic acid molecules comprises one or more insertions or deletions (indels) relative to a reference genomic sequence, and wherein a presence of the one or more indels is indicative of the condition of the subject; and (b) subjecting the subject to the treatment based on the identification in (a).

In one aspect, the present disclosure provides a method (not encompassed by the wording of the claims, but useful for understanding the invention) of monitoring a progress of a condition of a subject, the method comprising: (a) determining a first state of the condition of the subject based on identification of a first set of one or more cell-free nucleic acid molecules from a first plurality of cell-free nucleic acid molecules that is obtained or derived from the subject; (b) determining a second state of the condition of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining the progress of the condition based on the first state of the condition and the second state of the condition, wherein each of the one or more cell-free nucleic acid molecules comprises one or more insertions or deletions (indels) relative to a reference genomic sequence.

In some embodiments, the progress of the condition is worsening of the condition. In some embodiments, the progress of the condition is at least a partial remission of the condition. In some embodiments, a presence of the one or more indels is indicative of the first state or the second state of the condition of the subject. In some embodiments, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject. In some embodiments, the subject is subjected to a treatment for the condition (i) prior to obtaining the second plurality of cell-free nucleic acid molecules from the subject and (ii) subsequent to obtaining the first plurality of cell-free nucleic acid molecules from the subject. In some embodiments, the progress of the condition is indicative of minimal residual disease of the condition of the subject. In some embodiments, the progress of the condition is indicative of tumor burden or cancer burden of the subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the condition.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising one or more insertions or deletions (indels) relative to a reference genomic sequence, and wherein the individual nucleic acid probe comprises an activatable reporter agent, activation of the activatable reporter agent being selected from the group consisting of: (i) hybridization of the individual nucleic acid probe to the one or more indels and (ii) dehybridization of at least a portion of the individual nucleic acid probe that has been hybridized to the one or more indels; (b) detecting the activatable reporter agent that is activated, to identify one or more cell-free nucleic acid molecules of the plurality of cell-free nucleic acid molecules, wherein each of the one or more cell-free nucleic acid molecules comprises the one or more indels; and (c) analyzing the identified one or more cell-free nucleic acid molecules to determine a condition of the subject.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising one or more insertions or deletions (indels) relative to a reference genomic sequence, and wherein the individual nucleic acid probe comprises an activatable reporter agent, activation of the activatable reporter agent being selected from the group consisting of: (i) hybridization of the individual nucleic acid probe to the one or more indels and (ii) dehybridization of at least a portion of the individual nucleic acid probe that has been hybridized to the one or more indels; (b) detecting the activatable reporter agent that is activated, to identify one or more cell-free nucleic acid molecules of the plurality of cell-free nucleic acid molecules, wherein each of the one or more cell-free nucleic acid molecules comprises the one or more indels, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a condition of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, the activatable reporter agent is activated upon hybridization of the individual nucleic acid probe to the one or more indels. In some embodiments, the activatable reporter agent is activated upon dehybridization of at least a portion of the individual nucleic acid probe that has been hybridized to the one or more indels. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the one or more indels as different variables. In some embodiments, the analyzing of the identified one or more cell-free nucleic acid molecules is not based on other cell-free nucleic acid molecules of the plurality of cell-free nucleic acid molecules that do not comprise the one or more indels. In some embodiments, a number of the one or more indels from the identified one or more cell-free nucleic acid molecules is indicative of the condition of the subject. In some embodiments, a ratio of (i) the number of the one or more indels from the one or more cell-free nucleic acid molecules and (ii) a number of single nucleotide variants (SNVs) from the one or more cell-free nucleic acid molecules is indicative of the condition of the subject. In some embodiments, a frequency of the one or more indels in the identified one or more cell-free nucleic acid molecules is indicative of the condition of the subject. In some embodiments, the frequency is indicative of a diseased cell associated with the condition. In some embodiments, the condition is diffuse large B-cell lymphoma, and wherein the frequency is indicative of whether the one or more cell-free nucleic acid molecules are derived from germinal center B-cell (GCB) or activated B-cell (ABC). In some embodiments, genomic origin of the identified one or more cell-free nucleic acid molecules is indicative of the condition of the subject.

In some embodiments, the one or more indels comprises at least 3, at least 4, at least 5, at least 10, at least 15, at least 20, or at least 25 indels within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the subject. In some embodiments, the sample is a healthy sample. In some embodiments, the sample comprises a healthy cell. In some embodiments, the healthy cell comprises a healthy leukocyte. In some embodiments, the sample is a diseased sample. In some embodiments, the diseased sample comprises a diseased cell. In some embodiments, the diseased cell comprises a tumor cell. In some embodiments, the diseased sample comprises a solid tumor. In some embodiments, the set of nucleic acid probes is designed based on the one or more indels that are identified by comparing (i) sequencing data from a solid tumor, lymphoma, or blood tumor of the subject and (ii) sequencing data from a healthy cell of the subject or a healthy cohort. In some embodiments, the healthy cell is from the subject. In some embodiments, the healthy cell is from the healthy cohort. In some embodiments, the set of nucleic acid probes are designed to hybridize to at least a portion of sequences of genomic loci associated with the condition. In some embodiments, the genomic loci associated with the condition are known to exhibit aberrant somatic hypermutation when the subject has the condition.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, or (ii) the genomic regions identified in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6.

In some embodiments, the method further comprises determining that the subject has the condition or determining a degree or status of the condition of the subject, based on the identified one or more cell-free nucleic acid molecules comprising the one or more indels. In some embodiments, the method further comprises determining that the one or more cell-free nucleic acid molecules are derived from a sample associated with the condition, based on performing a statistical model analysis of the identified one or more cell-free nucleic acid molecules. In some embodiments, the statistical model analysis comprises a Monte Carlo statistical analysis. In some embodiments, the method further comprises monitoring a progress of the condition of the subject based on the identified one or more cell-free nucleic acid molecules. In some embodiments, the method further comprises performing a different procedure to confirm the condition of the subject. In some embodiments, the different procedure comprises a blood test, genetic test, medical imaging, physical exam, or tissue biopsy. In some embodiments, the method further comprises determining a treatment for the condition of the subject based on the identified one or more cell-free nucleic acid molecules. In some embodiments, the subject has been subjected to a treatment for the condition prior to (a). In some embodiments, the treatment comprises chemotherapy, radiotherapy, chemoradiotherapy, immunotherapy, adoptive cell therapy, hormone therapy, targeted drug therapy, surgery, transplant, transfusion, or medical surveillance. In some embodiments, the plurality of cell-free nucleic acid molecules comprise a plurality of cell-free deoxyribonucleic acid (DNA) molecules. In some embodiments, the condition comprises a disease. In some embodiments, the plurality of cell-free nucleic acid molecules are derived from a bodily sample of the subject. In some embodiments, the bodily sample comprises plasma, serum, blood, cerebrospinal fluid, lymph fluid, saliva, urine, or stool. In some embodiments, the subject is a mammal. In some embodiments, the subject is a human. In some embodiments, the condition comprises neoplasm, cancer, or tumor. In some embodiments, the condition comprises a solid tumor. In some embodiments, the condition comprises a lymphoma. In some embodiments, the condition comprises a B-cell lymphoma. In some embodiments, the condition comprises a sub-type of B-cell lymphoma selected from the group consisting of diffuse large B-cell lymphoma, follicular lymphoma, Burkitt lymphoma, and B-cell chronic lymphocytic leukemia. In some embodiments, the one or more indels have been previously identified as tumor-derived from sequencing a prior tumor sample or cell-free nucleic acid sample.

In one aspect, the present disclosure provides a method (not encompassed by the wording of the claims, but useful for understanding the invention) to perform a clinical procedure on an individual, the method comprising: obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; identifying or having identified one or more insertions or deletions (indels) within the cell-free nucleic acid sequencing result; determining or having determined, utilizing a statistical model and the identified one or more indels, that the cell-free nucleic acid sequencing result contains nucleotides derived from a neoplasm; and performing a clinical procedure on the individual to confirm the presence of the B-cell cancer, based upon determining that the cell-free nucleic acid sequencing result contains nucleic acid sequences likely derived from the B-cell cancer.

In some embodiments, the biopsy is one of blood, serum, cerebrospinal fluid, lymph fluid, urine, or stool. In some embodiments, the genomic loci are selected from (i) the genomic regions identified in Table 1, or (ii) the genomic regions identified in Table 3. In some embodiments, the sequences of the nucleic acid probes are selected from Table 6. In some embodiments, the clinical is procedure is a blood test, medical imaging, or a physical exam.

In one aspect, the present disclosure provides a method (not encompassed by the wording of the claims, but useful for understanding the invention) to treat an individual for a B-cell cancer, the method comprising: obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; identifying or having identified one or more insertions or deletions (indels) within the cell-free nucleic acid sequencing result; determining or having determined, utilizing a statistical model and the identified one or more indels, that the cell-free nucleic acid sequencing result contains nucleotides derived from a neoplasm; and treating the individual to curtail the B-cell cancer, based upon determining that the cell-free nucleic acid sequencing result contains nucleic acid sequences derived from the B-cell cancer.

In some embodiments, the biopsy is one of blood, serum, cerebrospinal fluid, lymph fluid, urine or stool. In some embodiments, the genomic loci are selected from (i) the genomic regions identified in Table 1, or (ii) the genomic regions identified in Table 3. In some embodiments, the sequences of the nucleic acid probes are selected from Table 6. In some embodiments, the treatment is chemotherapy, radiotherapy, immunotherapy, hormone therapy, targeted drug therapy, or medical surveillance.

In one aspect, the present disclosure provides a method (not encompassed by the wording of the claims, but useful for understanding the invention) to detect cancerous minimal residual disease in an individual and to treat the individual for a cancer, the method comprising: obtaining or having obtained a targeted sequencing result of a collection of cell-free nucleic acid molecules, wherein the collection of cell-free nucleic acid molecules are sourced from a liquid or waste biopsy of an individual, wherein the liquid or waste biopsy is sourced after a series of treatments in order to detect minimal residual disease, and wherein the targeting sequencing is performed utilizing nucleic acid probes to pull down sequences of genomic loci determined to contain one or more insertions or deletions (indels), as determined by a prior sequencing result on a prior biopsy derived from the cancer; identifying or having identified at least one set of the one or more indels within the cell-free nucleic acid sequencing result; and treating the individual to curtail the cancer, based upon determining that the cell-free nucleic acid sequencing result contains nucleic acid sequences derived from the cancer.

In some embodiments, the liquid or waste biopsy is one of blood, serum, cerebrospinal fluid, lymph fluid, urine or stool. In some embodiments, the treatment is chemotherapy, radiotherapy, immunotherapy, hormone therapy, targeted drug therapy, or medical surveillance.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject who has received an organ or tissue transplant; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a presence, an absence, or an extent of transplant rejection of the subject.

In some embodiments, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules. In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject who has received an organ or tissue transplant; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a presence, an absence, or an extent of transplant rejection of the subject.

In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject who has received an organ or tissue transplant; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a presence, an absence, or an extent of transplant rejection of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, each of the one or more cell-free nucleic acid molecules comprises a plurality of phased variants relative to a reference genomic sequence. In some embodiments, a first phased variant of the plurality of phased variants and a second phased variant of the plurality of phased variants are separated by at least one nucleotide. In some embodiments, (a) to (c) are performed by a computer system. In some embodiments, the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error. In some embodiments, (b) further comprises identifying one or more insertions or deletions (indels) in the one or more cell-free nucleic acid molecules, and wherein (c) further comprises determining the presence or the absence of the transplant rejection of the subject based at least in part on the identified one or more indels.

In one aspect, the present disclosure provides a method (not encompassed by the wording of the claims, but useful for understanding the invention) of treating a transplant rejection of a subject who has received an organ or tissue transplant, the method comprising: (a) identifying the subject for treatment of the transplant rejection, wherein the subject has been determined to have the transplant rejection based on identification of one or more cell-free nucleic acid molecules from a plurality of cell-free nucleic acid molecules that is obtained or derived from the subject, wherein each of the one or more cell-free nucleic acid molecules identified comprises a plurality of phased variants relative to a reference genomic sequence that are separated by at least one nucleotide, and wherein a presence of the plurality of phased variants is indicative of the transplant rejection of the subject; and (b) subjecting the subject to the treatment based on the identification in (a).

In some embodiments, the subject has been determined to have the transplant rejection based at least in part on one or more insertions or deletions (indels) identified in the one or more cell-free nucleic acid molecules.

In one aspect, the present disclosure provides a method (not encompassed by the wording of the claims, but useful for understanding the invention) of monitoring a subject who has received an organ or tissue transplant for a presence, an absence, or an extent of transplant rejection, the method comprising: (a) determining a first state of the presence, the absence, or the extent of transplant rejection of the subject based on identification of a first set of one or more cell-free nucleic acid molecules from a first plurality of cell-free nucleic acid molecules that is obtained or derived from the subject; (b) determining a second state of the presence, the absence, or the extent of transplant rejection of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining a transplant rejection status of the subject based on the first state and the second state, wherein each of the one or more cell-free nucleic acid molecules comprises a plurality of phased variants relative to a reference genomic sequence that are separated by at least one nucleotide.

In some embodiments, the transplant rejection status is at least a partial transplant rejection. In some embodiments, a presence of the plurality of phased variants is indicative of the first state or the second state. In some embodiments, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject. In some embodiments, the subject is subjected to a treatment for the transplant rejection (i) prior to obtaining the second plurality of cell-free nucleic acid molecules from the subject and (ii) subsequent to obtaining the first plurality of cell-free nucleic acid molecules from the subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the transplant rejection. In some embodiments, the subject has been determined to have the presence or the absence of the transplant rejection based at least in part on one or more insertions or deletions (indels) identified in the one or more cell-free nucleic acid molecules.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject who has received an organ or tissue transplant, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a presence, an absence, or an extent of transplant rejection of the subject.

In some embodiments, (b) further comprises identifying one or more insertions or deletions (indels) in the one or more cell-free nucleic acid molecules, and wherein (c) further comprises determining the presence or the absence of the transplant rejection of the subject based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject who has received an organ or tissue transplant, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a presence, an absence, or an extent of transplant rejection of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, a first phased variant of the plurality of phased variants and a second phased variant of the plurality of phased variants are separated by at least one nucleotide. In some embodiments, the activatable reporter agent is activated upon hybridization of the individual nucleic acid probe to the plurality of phased variants. In some embodiments, the activatable reporter agent is activated upon dehybridization of at least a portion of the individual nucleic acid probe that has been hybridized to the plurality of phased variants. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables. In some embodiments, the analyzing of the identified one or more cell-free nucleic acid molecules is not based on other cell-free nucleic acid molecules of the plurality of cell-free nucleic acid molecules that do not comprise the plurality of phased variants. In some embodiments, a number of the plurality of phased variants from the identified one or more cell-free nucleic acid molecules is indicative of the presence, the absence, or the extent of transplant rejection of the subject. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the presence, the absence, or the extent of transplant rejection of the subject. In some embodiments, a frequency of the plurality of phased variants in the identified one or more cell-free nucleic acid molecules is indicative of the presence or the absence of the transplant rejection of the subject. In some embodiments, the frequency is indicative of a diseased cell associated with the presence, the absence, or the extent of transplant rejection. In some embodiments, genomic origin of the identified one or more cell-free nucleic acid molecules is indicative of the presence or the absence of the transplant rejection of the subject. In some embodiments, the first and second phased variants are separated by at least 2, at least 3, at least 4, at least 5, at least 6, at least 7, or at least 8 nucleotides. In some embodiments, the first and second phased variants are separated by at most about 180, at most about 170, at most about 160, at most about 150, or at most about 140 nucleotides.

In some embodiments, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV. In some embodiments, the plurality of phased variants comprises at least 3, at least 4, at least 5, at least 10, at least 15, at least 20, or at least 25 phased variants within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the subject. In some embodiments, the sample is a healthy sample. In some embodiments, the sample comprises a healthy cell. In some embodiments, the healthy cell comprises a healthy leukocyte. In some embodiments, the sample is a diseased sample. In some embodiments, the diseased sample comprises a diseased cell. In some embodiments, the healthy cell is from the subject. In some embodiments, the healthy cell is from the healthy cohort. In some embodiments, the set of nucleic acid probes are designed to hybridize to at least a portion of sequences of genomic loci associated with the presence or the absence of the transplant rejection. In some embodiments, the genomic loci associated with the presence, the absence, or the extent of transplant rejection are known to exhibit aberrant somatic hypermutation when the subject has the transplant rejection.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6. In some embodiments, the method further comprises determining the presence or the absence of the transplant rejection or determining a degree or status thereof, based on the identified one or more cell-free nucleic acid molecules comprising the plurality of phased variants. In some embodiments, the method further comprises determining that the one or more cell-free nucleic acid molecules are derived from a sample associated with the presence or the absence of the transplant rejection, based on performing a statistical model analysis of the identified one or more cell-free nucleic acid molecules. In some embodiments, the statistical model analysis comprises a Monte Carlo statistical analysis. In some embodiments, the method further comprises monitoring a progress of the presence, the absence, or the extent of transplant rejection of the subject based on the identified one or more cell-free nucleic acid molecules. In some embodiments, the method further comprises performing a different procedure to confirm the presence, the absence, or the extent of transplant rejection of the subject. In some embodiments, the different procedure comprises a blood test, genetic test, medical imaging, physical exam, or tissue biopsy. In some embodiments, the method further comprises determining a treatment for the transplant rejection of the subject based on the identified one or more cell-free nucleic acid molecules. In some embodiments, the subject has been subjected to a treatment for the transplant rejection prior to (a). In some embodiments, the plurality of cell-free nucleic acid molecules comprises a plurality of cell-free deoxyribonucleic acid (DNA) molecules. In some embodiments, the plurality of cell-free nucleic acid molecules are derived from a bodily sample of the subject. In some embodiments, the bodily sample comprises plasma, serum, blood, cerebrospinal fluid, lymph fluid, saliva, urine, or stool. In some embodiments, the subject is a mammal. In some embodiments, the subject is a human. In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method comprising: (a) obtaining, by a computer system, sequencing data derived from a plurality of cell-free nucleic acid molecules that is obtained or derived from a pregnant subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a presence, an absence, or an elevated risk of a genetic abnormality of a fetus of the pregnant subject.

In some embodiments, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a pregnant subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a presence, an absence, or an elevated risk of a genetic abnormality of a fetus of the pregnant subject.

In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a pregnant subject; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a presence, an absence, or an elevated risk of a genetic abnormality of a fetus of the pregnant subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, each of the one or more cell-free nucleic acid molecules comprises a plurality of phased variants relative to a reference genomic sequence. In some embodiments, a first phased variant of the plurality of phased variants and a second phased variant of the plurality of phased variants are separated by at least one nucleotide. In some embodiments, (a) to (c) are performed by a computer system. In some embodiments, he method of any one of claims 309-313, wherein the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) of monitoring a pregnant subject for a presence, an absence, or an elevated risk of a genetic abnormality of a fetus of the pregnant subject, the method comprising: (a) determining a first state of the presence, the absence, or the elevated risk of the genetic abnormality of the fetus of the pregnant subject based on identification of a first set of one or more cell-free nucleic acid molecules from a first plurality of cell-free nucleic acid molecules that is obtained or derived from the pregnant subject; (b) determining a second state of the presence, the absence, or the elevated risk of the genetic abnormality of the fetus of the pregnant subject based on identification of a second set of one or more cell-free nucleic acid molecules from a second plurality of cell-free nucleic acid molecules that is obtained or derived from the pregnant subject, wherein the second plurality of cell-free nucleic acid molecules are obtained from the pregnant subject subsequent to obtaining the first plurality of cell-free nucleic acid molecules from the pregnant subject; and (c) determining the presence, the absence, or the elevated risk of the genetic abnormality of the fetus of the pregnant subject based on the first state and the second state, wherein each of the one or more cell-free nucleic acid molecules comprises a plurality of phased variants relative to a reference genomic sequence that are separated by at least one nucleotide.

In some embodiments, the transplant rejection status is at least a partial transplant rejection. In some embodiments, a presence of the plurality of phased variants is indicative of the first state or the second state. In some embodiments, the second plurality of cell-free nucleic acid molecules is obtained from the pregnant subject at least about 1 week, at least about 2 weeks, at least about 3 weeks, at least about 4 weeks, at least about 2 months, or at least about 3 months subsequent to obtaining the first plurality of cell-free nucleic acid molecules from the pregnant subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the genetic abnormality. In some embodiments, the fetus has been determined to have the presence, the absence, or the elevated risk of the genetic abnormality based at least in part on one or more insertions or deletions (indels) identified in the one or more cell-free nucleic acid molecules.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a pregnant subject, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a presence, an absence, or an elevated risk of a genetic abnormality of a fetus of the pregnant subject.

In some embodiments, (b) further comprises identifying one or more insertions or deletions (indels) in the one or more cell-free nucleic acid molecules, and wherein (c) further comprises determining the presence, the absence, or the elevated risk of the genetic abnormality based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a pregnant subject, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a presence, an absence, or an elevated risk of a genetic abnormality of a fetus of the pregnant subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, a first phased variant of the plurality of phased variants and a second phased variant of the plurality of phased variants are separated by at least one nucleotide. In some embodiments, the activatable reporter agent is activated upon hybridization of the individual nucleic acid probe to the plurality of phased variants. In some embodiments, the activatable reporter agent is activated upon dehybridization of at least a portion of the individual nucleic acid probe that has been hybridized to the plurality of phased variants. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables. In some embodiments, the analyzing of the identified one or more cell-free nucleic acid molecules is not based on other cell-free nucleic acid molecules of the plurality of cell-free nucleic acid molecules that do not comprise the plurality of phased variants. In some embodiments, a number of the plurality of phased variants from the identified one or more cell-free nucleic acid molecules is indicative of the genetic abnormality. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the genetic abnormality. In some embodiments, a frequency of the plurality of phased variants in the identified one or more cell-free nucleic acid molecules is indicative of the genetic abnormality. In some embodiments, genomic origin of the identified one or more cell-free nucleic acid molecules is indicative of the genetic abnormality. In some embodiments, the first and second phased variants are separated by at least 2, at least 3, at least 4, at least 5, at least 6, at least 7, or at least 8 nucleotides. In some embodiments, the first and second phased variants are separated by at most about 180, at most about 170, at most about 160, at most about 150, or at most about 140 nucleotides.

In some embodiments, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV. In some embodiments, the plurality of phased variants comprises at least 3, at least 4, at least 5, at least 10, at least 15, at least 20, or at least 25 phased variants within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the pregnant subject. In some embodiments, the sample is a healthy sample. In some embodiments, the sample comprises a healthy cell. In some embodiments, the sample is a diseased sample. In some embodiments, the diseased sample comprises a diseased cell. In some embodiments, the healthy cell is from the pregnant subject. In some embodiments, the healthy cell is from the healthy cohort. In some embodiments, the set of nucleic acid probes are designed to hybridize to at least a portion of sequences of genomic loci associated with the genetic abnormality.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6. In some embodiments, the method further comprises determining the presence, the absence, or the elevated risk of the genetic abnormality of the fetus of the pregnant subject, based on the identified one or more cell-free nucleic acid molecules comprising the plurality of phased variants. In some embodiments, the method further comprises determining that the one or more cell-free nucleic acid molecules are derived from a sample associated with the presence, the absence, or the elevated risk of the genetic abnormality of the fetus of the pregnant subject, based on performing a statistical model analysis of the identified one or more cell-free nucleic acid molecules. In some embodiments, the statistical model analysis comprises a Monte Carlo statistical analysis. In some embodiments, the method further comprises monitoring a progress of the presence, the absence, or the elevated risk of the genetic abnormality of the fetus of the pregnant subject based on the identified one or more cell-free nucleic acid molecules. In some embodiments, the method further comprises performing a different procedure to confirm the presence, the absence, or the elevated risk of the genetic abnormality of the fetus of the pregnant subject. In some embodiments, the different procedure comprises a blood test, genetic test, medical imaging, physical exam, or tissue biopsy. In some embodiments, the plurality of cell-free nucleic acid molecules comprise a plurality of cell-free deoxyribonucleic acid (DNA) molecules. In some embodiments, the plurality of cell-free nucleic acid molecules are derived from a bodily sample of the pregnant subject. In some embodiments, the bodily sample comprises plasma, serum, blood, cerebrospinal fluid, lymph fluid, saliva, urine, or stool. In some embodiments, the pregnant subject is a mammal. In some embodiments, the pregnant subject is a human. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels.

In one aspect, the present disclosure provides a method (not encompassed by the wording of the claims, but useful for understanding the invention) comprising adding a set of nucleic acid probes to a sample comprising a plurality of nucleic acid molecules that have been obtained or derived from a subject, wherein each nucleic acid probe of the set of nucleic acid probes is configured to hybridize to a target nucleic acid molecule comprising a plurality of phased variants such that the nucleic acid probe is complementary to at least a region of the target nucleic acid molecule that extends from a first phased variant of the plurality of phased variants to a second phased variant of the plurality of phased variants. (For clarity, the region includes both the first phased variant and the second phased variant.)

This method, and embodiments of it described herein, may involve the use of hybrid capture probes/baits, such as biotinylated oligonucleotides, that may be used in a hybrid capture enrichment step such that the hybrid capture probes bind to and preferentially capture nucleic acid molecules that contain phased variants. Such hybrid capture approaches may increase the capture sensitivity of circulating tumor DNA or circulating DNA from a transplanted organ. The hybrid capture probes can be synthesized to specifically target molecules containing phased variants by designing the hybrid capture probe to (1) contain a sequence that is complementary to the molecule that includes the phased variant (as opposed to the corresponding region of the reference genomic sequence) and (2) have a length that optimizes the nucleic acid binding kinetics/thermodynamics ($\Delta G$ or binding energy) such that the hybrid capture probe preferentially binds to a nucleic acid molecule that contains the phased variants of interest as compared to corresponding molecules without the phased variants. Such hybrid capture probes can lead to improved enrichment of relevant nucleic acid sequences, thereby requiring less sequencing as a result. For instance, in some cases (such as in assessing minimal residual disease, disease state, or state of transplant rejection), a cancerous sample or a sample from the transplanted organ may be obtained and sequenced to identify phased variants in such samples relative to a reference genomic sequence, such as a sequence from corresponding healthy cell(s) of the subject, and the hybrid capture probes can be designed to preferentially bind to nucleic acid sequences containing the phased variants identified from the cancerous and/or transplanted organ samples. In some circumstances, such hybrid capture probes can be used for single strand recovery of nucleic acid molecules that contain phased variants. The nucleic acid molecules captured by such probe sets can include DNA or RNA (e.g., single stranded RNA), such as cell-free DNA or cell-free DNA. Probes as described in this particular method can be used on combination with other methods described herein.

In some embodiments, each nucleic acid probe of the set of nucleic acid probes comprises a pull-down tag, such as biotin. In some embodiments, the method further comprises separation of target nucleic acid molecules that hybridize to the nucleic acid probes from nucleic acid molecules that do not hybridize to the nucleic acid probes to thereby capture target nucleic acid molecules. In some embodiments, the nucleic acid molecules are cell-free nucleic acid molecules. In some embodiments, the first phased variant is selected from the group consisting of a somatic single nucleotide variant, a somatic indel, a somatic translocation breakpoint, a somatic amplification or deletion breakpoint, a germline SNV, a germline indel, a germline translocation breakpoint, a germline amplification or deletion breakpoint, and a region of localized hypermutation, and the second phased variant is selected from the group consisting of a somatic single nucleotide variant, a somatic indel, a somatic translocation breakpoint, a somatic amplification or deletion breakpoint, a germline SNV, a germline indel, a germline translocation breakpoint, a germline amplification or deletion breakpoint, and a region of localized hypermutation. In some embodiments, the first phased variant of the plurality of phased variants and the second phased variant of the plurality of phased variants are separated by at least 1, 2, 3, 4, 5, 10, or 20 nucleotides. In some embodiments, each nucleic acid probe of the set of nucleic acid probes is either (1) less than 40 nucleotides, less than 30 nucleotides, or less than 20 nucleotides in length or (2) no more than 5 nucleotides, nor more than 10 nucleotides, no more than 20 nucleotides, or no more than 30 nucleotides longer than the distance between the first phased variant of the plurality of phased variants and the second phased variant of the plurality of phased variants, wherein the first phased variant and the second phased variant are the most separated phased variants (i.e., have the most number of intervening nucleotides) of the plurality of phased variants.

In some embodiments, the target nucleic acid molecule is a molecule that is derived from a pre-identified portion of a genome of a cancer cell or a transplanted cell from the subject that differs in sequence from a reference genomic sequence, wherein the preidentified portion of the genome is less than 200, less than 180, or less than 150 nucleotides in length. In some embodiments, each nucleic acid probe of the plurality of nucleic acid probes has a lower $\Delta G$ of binding to the target nucleic acid molecule than to a corresponding molecule that is identical in length and sequence to the target nucleic acid molecule except that the corresponding molecule has a sequence that corresponds with a reference genomic sequence. In some embodiments, the reference genomic sequence comprises a portion of either (1) a reference cohort, such as a portion of the hg19 human genome, hg18 genome, hg17 genome, hg16 genome, or hg38 genome or (2) a healthy sample from the subject. In some embodiments, the method involves the capture of the target nucleic acid derived from either the Watson strand or the Crick strand of a chromosome, but does not involve the capture of the corresponding complementary nucleic acid of the other strand. In some embodiments, the method comprises capture of at least 10, at least 100, at least 1000, or at least 10,000 target nucleic acid molecules. In some embodiments, the method further comprises sequencing the captured target nucleic acids to obtain sequencing data derived from the plurality of nucleic acid molecules. In some embodiments, the sequencing does not involve use of molecular barcodes. In some embodiments, the sequencing does not comprise duplex sequencing.

In one aspect, the present disclosure provides a method (not encompassed by the wording of the claims, but useful for understanding the invention) for determining a condition of a subject (e.g., assessing minimal residual disease, disease progression, or transplant rejection status), the method comprising obtaining, by a computer system, sequence information obtained by any method described herein involving the use of hybrid capture probes that are designed to bind preferentially to molecules that contain phased variants as compared to corresponding molecules that lack phased variants; processing, by the computer system, the sequencing data to identify one or more nucleic acid molecules of the plurality of nucleic acid molecules, wherein each of the one or more nucleic acid molecules comprises a plurality of phased variants relative to a reference genomic sequence; and analyzing, by the computer system, the identified one or more nucleic acid molecules to determine a condition of the subject. In some embodiments, such methods do not comprise duplex-mediated error suppression or barcode-mediated error suppression. Individuals may be treated (e.g., with anti-cancer agents, anti-rejection agents, or surgical procedures) based on the identification of a condition (e.g., state) of the subject.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a condition of the subject.

In some embodiments of any one of the methods disclosed herein, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a condition of the subject.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a condition of the subject.

In some embodiments of any one of the methods disclosed herein, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data.

In some embodiments of any one of the methods disclosed herein, each of the one or more cell-free nucleic acid molecules comprises a plurality of phased variants relative to a reference genomic sequence. In some embodiments of any one of the methods disclosed herein, a first phased variant of the plurality of phased variants and a second phased variant of the plurality of phased variants are separated by at least one nucleotide.

In some embodiments of any one of the methods disclosed herein, the processes (a) to (c) are performed by a computer system.

In some embodiments of any one of the methods disclosed herein, the sequencing data is generated based on nucleic acid amplification. In some embodiments of any one of the methods disclosed herein, the sequencing data is generated based on polymerase chain reaction. In some embodiments of any one of the methods disclosed herein, the sequencing data is generated based on amplicon sequencing.

In some embodiments of any one of the methods disclosed herein, the sequencing data is generated based on next-generation sequencing (NGS). Alternatively, in some embodiments of any one of the methods disclosed herein, the sequencing data is generated based on non-hybridization-based NGS.

In some embodiments of any one of the methods disclosed herein, the sequencing data is generated without use of molecular barcoding of at least a portion of the plurality of cell-free nucleic acid molecules. In some embodiments of any one of the methods disclosed herein, the sequencing data is obtained without use of sample barcoding of at least a portion of the plurality of cell-free nucleic acid molecules.

In some embodiments of any one of the methods disclosed herein, the sequencing data is obtained without in silico removal or suppression of (i) background error or (ii) sequencing error.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) of treating a condition of a subject, the method comprising: (a) identifying the subject for treatment of the condition, wherein the subject has been determined to have the condition based on identification of one or more cell-free nucleic acid molecules from a plurality of cell-free nucleic acid molecules that is obtained or derived from the subject, wherein each of the one or more cell-free nucleic acid molecules identified comprises a plurality of phased variants relative to a reference genomic sequence that are separated by at least one nucleotide, and wherein a presence of the plurality of phased variants is indicative of the condition of the subject; and (b) subjecting the subject to the treatment based on the identification in (a).

In some embodiments, the subject has been determined to have the condition based at least in part on one or more insertions or deletions (indels) identified in the one or more cell-free nucleic acid molecules.

In one aspect, the present disclosure provides a method (not encompassed by the wording of the claims, but useful for understanding the invention) of monitoring a progress of a condition of a subject, the method comprising: (a) determining a first state of the condition of the subject based on identification of a first set of one or more cell-free nucleic acid molecules from a first plurality of cell-free nucleic acid molecules that is obtained or derived from the subject; (b) determining a second state of the condition of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining the progress of the condition based on the first state of the condition and the second state of the condition, wherein each of the one or more cell-free nucleic acid molecules comprises a plurality of phased variants relative to a reference genomic sequence that are separated by at least one nucleotide.

In some embodiments of any one of the methods disclosed herein, the progress of the condition is worsening of the condition.

In some embodiments of any one of the methods disclosed herein, the progress of the condition is at least a partial remission of the condition.

In some embodiments of any one of the methods disclosed herein, a presence of the plurality of phased variants is indicative of the first state or the second state of the condition of the subject.

In some embodiments of any one of the methods disclosed herein, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject.

In some embodiments of any one of the methods disclosed herein, the subject is subjected to a treatment for the condition (i) prior to obtaining the second plurality of cell-free nucleic acid molecules from the subject and (ii) subsequent to obtaining the first plurality of cell-free nucleic acid molecules from the subject.

In some embodiments of any one of the methods disclosed herein, the progress of the condition is indicative of minimal residual disease of the condition of the subject. In some embodiments of any one of the methods disclosed herein, the progress of the condition is indicative of tumor burden or cancer burden of the subject.

In some embodiments of any one of the methods disclosed herein, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the condition.

In some embodiments, the subject has been determined to have the condition based at least in part on one or more insertions or deletions (indels) identified in the one or more cell-free nucleic acid molecules.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a condition of the subject.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a condition of the subject.

In some embodiments of any one of the methods disclosed herein, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules.

In some embodiments of any one of the methods disclosed herein, a first phased variant of the plurality of phased variants and a second phased variant of the plurality of phased variants are separated by at least one nucleotide.

In some embodiments of any one of the methods disclosed herein, the activatable reporter agent is activated upon hybridization of the individual nucleic acid probe to the plurality of phased variants.

In some embodiments of any one of the methods disclosed herein, the activatable reporter agent is activated upon dehybridization of at least a portion of the individual nucleic acid probe that has been hybridized to the plurality of phased variants.

In some embodiments of any one of the methods disclosed herein, the method further comprises mixing (1) the set of nucleic acid probes and (2) the plurality of cell-free nucleic acid molecules.

In some embodiments of any one of the methods disclosed herein, the activatable reporter agent is a fluorophore.

In some embodiments of any one of the methods disclosed herein, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables.

In some embodiments of any one of the methods disclosed herein, the analyzing of the identified one or more cell-free nucleic acid molecules is not based on other cell-free nucleic acid molecules of the plurality of cell-free nucleic acid molecules that do not comprise the plurality of phased variants.

In some embodiments of any one of the methods disclosed herein, a number of the plurality of phased variants from the identified one or more cell-free nucleic acid molecules is indicative of the condition of the subject. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the condition of the subject.

In some embodiments of any one of the methods disclosed herein, a frequency of the plurality of phased variants in the identified one or more cell-free nucleic acid molecules is indicative of the condition of the subject. In some embodiments, the frequency is indicative of a diseased cell associated with the condition. In some embodiments, the condition is diffuse large B-cell lymphoma, and wherein the frequency is indicative of whether the one or more cell-free nucleic acid molecules are derived from germinal center B-cell (GCB) or activated B-cell (ABC).

In some embodiments of any one of the methods disclosed herein, genomic origin of the identified one or more cell-free nucleic acid molecules is indicative of the condition of the subject.

In some embodiments of any one of the methods disclosed herein, the first and second phased variants are separated by at least 2, at least 3, at least 4, at least 5, at least 6, at least 7, or at least 8 nucleotides. In some embodiments of any one of the methods disclosed herein, the first and second phased variants are separated by at most about 180, at most about 170, at most about 160, at most about 150, or at most about 140 nucleotides.

In some embodiments of any one of the methods disclosed herein, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV.

In some embodiments of any one of the methods disclosed herein, the plurality of phased variants comprises at least 3, at least 4, at least 5, at least 10, at least 15, at least 20, or at least 25 phased variants within the same cell-free nucleic acid molecule.

In some embodiments of any one of the methods disclosed herein, the one or more cell-free nucleic acid molecules identified comprises at least 2, at least 3, at least 4, at least 5, at least 10, at least 50, at least 100, at least 500, or at least 1,000 cell-free nucleic acid molecules.

In some embodiments of any one of the methods disclosed herein, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome.

In some embodiments of any one of the methods disclosed herein, the reference genomic sequence is derived from a sample of the subject.

In some embodiments of any one of the methods disclosed herein, the sample is a healthy sample. In some embodiments, the sample comprises a healthy cell. In some embodiments, the healthy cell comprises a healthy leukocyte.

In some embodiments of any one of the methods disclosed herein, the sample is a diseased sample. In some embodiments, the diseased sample comprises a diseased cell. In some embodiments, the diseased cell comprises a tumor cell. In some embodiments, the diseased sample comprises a solid tumor.

In some embodiments of any one of the methods disclosed herein, the set of nucleic acid probes is designed based on the plurality of phased variants that are identified by comparing (i) sequencing data from a solid tumor, lymphoma, or blood tumor of the subject and (ii) sequencing data from a healthy cell of the subject or a healthy cohort. In some embodiments, the healthy cell is from the subject. In some embodiments, the healthy cell is from the healthy cohort.

In some embodiments of any one of the methods disclosed herein, the set of nucleic acid probes are designed to hybridize to at least a portion of sequences of genomic loci associated with the condition. In some embodiments, the genomic loci associated with the condition are known to exhibit aberrant somatic hypermutation when the subject has the condition.

In some embodiments of any one of the methods disclosed herein, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3.

In some embodiments of any one of the methods disclosed herein, each nucleic acid probe of the set of nucleic acid probes has at least about 70%, at least about 80%, at least about 90% sequence identity, at least about 95% sequence identity, or about 100% sequence identity to a probe sequence selected from Table 6.

In some embodiments of any one of the methods disclosed herein, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6.

In some embodiments of any one of the methods disclosed herein, the method further comprises determining that the subject has the condition or determining a degree or status of the condition of the subject, based on the identified one or more cell-free nucleic acid molecules comprising the plurality of phased variants. In some embodiments, the method further comprises determining that the one or more cell-free nucleic acid molecules are derived from a sample associated with the condition, based on performing a statistical model analysis of the identified one or more cell-free nucleic acid molecules. In some embodiments, the statistical model analysis comprises a Monte Carlo statistical analysis.

In some embodiments of any one of the methods disclosed herein, the method further comprises monitoring a progress of the condition of the subject based on the identified one or more cell-free nucleic acid molecules.

In some embodiments of any one of the methods disclosed herein, the method further comprises performing a different procedure to confirm the condition of the subject. In some embodiments, the different procedure comprises a blood test, genetic test, medical imaging, physical exam, or tissue biopsy.

In some embodiments of any one of the methods disclosed herein, the method further comprises determining a treatment for the condition of the subject based on the identified one or more cell-free nucleic acid molecules.

In some embodiments of any one of the methods disclosed herein, the subject has been subjected to a treatment for the condition prior to (a).

In some embodiments of any one of the methods disclosed herein, the treatment comprises chemotherapy, radiotherapy, chemoradiotherapy, immunotherapy, adoptive cell therapy, hormone therapy, targeted drug therapy, surgery, transplant, transfusion, or medical surveillance.

In some embodiments of any one of the methods disclosed herein, the plurality of cell-free nucleic acid molecules comprises a plurality of cell-free deoxyribonucleic acid (DNA) molecules.

In some embodiments of any one of the methods disclosed herein, condition comprises a disease.

In some embodiments of any one of the methods disclosed herein, the plurality of cell-free nucleic acid molecules are derived from a bodily sample of the subject. In some embodiments, the bodily sample comprises plasma, serum, blood, cerebrospinal fluid, lymph fluid, saliva, urine, or stool.

In some embodiments of any one of the methods disclosed herein, the subject is a mammal. In some embodiments of any one of the methods disclosed herein, the subject is a human.

In some embodiments of any one of the methods disclosed herein, the condition comprises neoplasm, cancer, or tumor. In some embodiments, the condition comprises a solid tumor. In some embodiments, the condition comprises a lymphoma. In some embodiments, the condition comprises a B-cell lymphoma. In some embodiments, the condition comprises a sub-type of B-cell lymphoma selected from the group consisting of diffuse large B-cell lymphoma, follicular lymphoma, Burkitt lymphoma, and B-cell chronic lymphocytic leukemia. In some embodiments of any one of the methods disclosed herein, the condition comprises transplant rejection of or a chromosomal abnormality.

In some embodiments of any one of the methods disclosed herein, the plurality of phased variants have been previously identified as tumor-derived from sequencing a prior tumor sample or cell-free nucleic acid sample.

In some embodiments, (b) further comprises identifying one or more insertions or deletions (indels) in the one or more cell-free nucleic acid molecules, and (c) further comprises determining the condition of the subject based at least in part on the identified one or more indels.

In one aspect, the present disclosure provides a composition (not encompassed by the wording of the claims, but useful for understanding the invention) comprising a bait set comprising a set of nucleic acid probes designed to capture cell-free DNA molecules derived from at least about 5% of genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the set of nucleic acid probes are designed to pull down cell-free DNA molecules derived from at least about 10%, at least about 20%, at least about 30%, at least about 40%, at least about 50%, at least about 60%, at least about 70%, at least about 80%, at least about 90%, or about 100% of the genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the set of nucleic acid probes are designed to capture the one or more cell-free DNA molecules derived from at most about 10%, at most about 20%, at most about 30%, at most about 40%, at most about 50%, at most about 60%, at most about 70%, at most about 80%, at most about 90%, or about 100% of the genomic regions set forth in (i) the genomic regions identified in Table 1, (ii) the genomic regions identified in Table 3, or (iii) the genomic regions identified to have a plurality of phased variants in Table 3.

In some embodiments of any of the compositions disclosed herein, the bait set comprises at most 5, at most 10, at most 50, at most 100, at most 500, at most 1000, or at most 2000 nucleic acid probes.

In some embodiments of any of the compositions disclosed herein, an individual nucleic acid probe of the set of nucleic acid probes comprises a pull-down tag.

In some embodiments of any of the compositions disclosed herein, the pull-down tag comprises a nucleic acid barcode.

In some embodiments of any of the compositions disclosed herein, the pull-down tag comprises biotin.

In some embodiments of any of the compositions disclosed herein, each of the cell-free DNA molecules is between about 100 nucleotides and about 180 nucleotides in length.

In some embodiments of any of the compositions disclosed herein, the genomic regions are associated with a condition.

In some embodiments of any of the compositions disclosed herein, the genomic regions exhibit aberrant somatic hypermutation when a subject has the condition.

In some embodiments of any of the compositions disclosed herein, the condition comprises a B-cell lymphoma. In some embodiments, the condition comprises a sub-type of B-cell lymphoma selected from the group consisting of diffuse large B-cell lymphoma, follicular lymphoma, Burkitt lymphoma, and B-cell chronic lymphocytic leukemia.

In some embodiments of any of the compositions disclosed herein, the composition further comprises a plurality of cell-free DNA molecules obtained or derived from a subject.

In one aspect, the present disclosure provides a method (not encompassed by the wording of the claims, but useful for understanding the invention) to perform a clinical procedure on an individual, the method comprising: (a) obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; (b) identifying or having identified a plurality of variants in phase within the cell-free nucleic acid sequencing result; (c) determining or having determined, utilizing a statistical model and the identified phased variants, that the cell-free nucleic acid sequencing result contains nucleotides derived from a neoplasm; and (d) performing a clinical procedure on the individual to confirm the presence of the B-cell cancer, based upon determining that the cell-free nucleic acid sequencing result contains nucleic acid sequences likely derived from the B-cell cancer.

In some embodiments of any of the compositions disclosed herein, the biopsy is one of blood, serum, cerebrospinal fluid, lymph fluid, urine, or stool.

In some embodiments of any of the compositions disclosed herein, the genomic loci are selected from (i) the genomic regions identified in Table 1, (ii) the genomic regions identified in Table 3, or (iii) the genomic regions identified to have a plurality of phased variants in Table 3.

In some embodiments of any of the compositions disclosed herein, the sequences of the nucleic acid probes are selected from Table 6.

In some embodiments of any of the compositions disclosed herein, the clinical is procedure is a blood test, medical imaging, or a physical exam.

In some embodiments, the method further comprises identifying or having identified one or more insertions or deletions (indels) within the cell-free nucleic acid sequencing result, and determining or having determined, based least in part on the identified one or more indels, that the cell-free nucleic acid sequencing result contains the nucleotides derived from the neoplasm.

In one aspect, the present disclosure provides a method (not encompassed by the wording of the claims, but useful for understanding the invention) to treat an individual for a B-cell cancer, the method comprising: (a) obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; (b) identifying or having identified a plurality of variants in phase within the cell-free nucleic acid sequencing result; (c) determining or having determined, utilizing a statistical model and the identified phased variants, that the cell-free nucleic acid sequencing result contains nucleotides derived from a neoplasm; and (d) treating the individual to curtail the B-cell cancer, based upon determining that the cell-free nucleic acid sequencing result contains nucleic acid sequences derived from the B-cell cancer.

In some embodiments of any of the compositions disclosed herein, the biopsy is one of blood, serum, cerebrospinal fluid, lymph fluid, urine or stool.

In some embodiments of any of the compositions disclosed herein, the genomic loci are selected from (i) the genomic regions identified in Table 1, (ii) the genomic regions identified in Table 3, or (iii) the genomic regions identified to have a plurality of phased variants in Table 3.

In some embodiments of any of the compositions disclosed herein, the sequences of the nucleic acid probes are selected from Table 6.

In some embodiments of any of the compositions disclosed herein, the treatment is chemotherapy, radiotherapy, immunotherapy, hormone therapy, targeted drug therapy, or medical surveillance.

In some embodiments, the method further comprises identifying or having identified one or more insertions or deletions (indels) within the cell-free nucleic acid sequencing result, and determining or having determined, based least in part on the identified one or more indels, that the cell-free nucleic acid sequencing result contains the nucleotides derived from the neoplasm.

In one aspect, the present disclosure provides a method (not encompassed by the wording of the claims, but useful for understanding the invention) to detect cancerous minimal residual disease in an individual and to treat the individual for a cancer, the method comprising: (a) obtaining or having obtained a targeted sequencing result of a collection of cell-free nucleic acid molecules, wherein the collection of cell-free nucleic acid molecules are sourced from a liquid or waste biopsy of an individual, wherein the liquid or waste biopsy is sourced after a series of treatments in order to detect minimal residual disease, and wherein the targeting sequencing is performed utilizing nucleic acid probes to pull down sequences of genomic loci determined to contain a plurality of variants in phase, as determined by a prior sequencing result on a prior biopsy derived from the cancer; (b) identifying or having identified at least one set of the plurality of variants in phase within the cell-free nucleic acid sequencing result; and (c) treating the individual to curtail the cancer, based upon determining that the cell-free nucleic acid sequencing result contains nucleic acid sequences derived from the cancer.

In some embodiments of any of the compositions disclosed herein, the liquid or waste biopsy is one of blood, serum, cerebrospinal fluid, lymph fluid, urine or stool.

In some embodiments of any of the compositions disclosed herein, the treatment is chemotherapy, radiotherapy, immunotherapy, hormone therapy, targeted drug therapy, or medical surveillance.

In some embodiments, the method further comprises identifying or having identified one or more insertions or deletions (indels) within the cell-free nucleic acid sequencing result, and treating the individual to curtail the cancer, based least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises one or more insertions or deletions (indels) relative to a reference genomic sequence; and (c) analyzing, by the computer system, the one or more indels to determine a condition of the subject.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject; (b) processing, by the computer system, the sequencing data to identify one or more cell-free nucleic acid molecules of the plurality of cell-free nucleic acid molecules, wherein each of the one or more cell-free nucleic acid molecules comprises one or more insertions or deletions (indels) relative to a reference genomic sequence; and (c) analyzing, by the computer system, the one or more insertions or deletions (indels) to determine a condition of the subject.

**EP 4 334 476 B1**

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject; (b) processing the sequencing data to identify one or more cell-free nucleic acid molecules of the plurality of cell-free nucleic acid molecules with a limit of detection of less than about 1 out of 50,000 observations from the sequencing data, wherein each of the one or more cell-free nucleic acid molecules comprises one or more insertions or deletions (indels) relative to a reference genomic sequence; and (c) analyzing the identified one or more cell-free nucleic acid molecules to determine a condition of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, (a) to (c) are performed by a computer system. In some embodiments, the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error.

In one aspect, the present disclosure provides a method (not encompassed by the wording of the claims, but useful for understanding the invention) of treating a condition of a subject, the method comprising: (a) identifying the subject for treatment of the condition, wherein the subject has been determined to have the condition based on identification of one or more cell-free nucleic acid molecules from a plurality of cell-free nucleic acid molecules that is obtained or derived from the subject, wherein each of the one or more cell-free nucleic acid molecules comprises one or more insertions or deletions (indels) relative to a reference genomic sequence, and wherein a presence of the one or more indels is indicative of the condition of the subject; and (b) subjecting the subject to the treatment based on the identification in (a).

In one aspect, the present disclosure provides a method (not encompassed by the wording of the claims, but useful for understanding the invention) of monitoring a progress of a condition of a subject, the method comprising: (a) determining a first state of the condition of the subject based on identification of a first set of one or more cell-free nucleic acid molecules from a first plurality of cell-free nucleic acid molecules that is obtained or derived from the subject; (b) determining a second state of the condition of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining the progress of the condition based on the first state of the condition and the second state of the condition, wherein each of the one or more cell-free nucleic acid molecules comprises one or more insertions or deletions (indels) relative to a reference genomic sequence.

In some embodiments, the progress of the condition is worsening of the condition. In some embodiments, the progress of the condition is at least a partial remission of the condition. In some embodiments, a presence of the one or more indels is indicative of the first state or the second state of the condition of the subject. In some embodiments, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject. In some embodiments, the subject is subjected to a treatment for the condition (i) prior to obtaining the second plurality of cell-free nucleic acid molecules from the subject and (ii) subsequent to obtaining the first plurality of cell-free nucleic acid molecules from the subject. In some embodiments, the progress of the condition is indicative of minimal residual disease of the condition of the subject. In some embodiments, the progress of the condition is indicative of tumor burden or cancer burden of the subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the condition.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising one or more insertions or deletions (indels) relative to a reference genomic sequence, and wherein the individual nucleic acid probe comprises an activatable reporter agent, activation of the activatable reporter agent being selected from the group consisting of: (i) hybridization of the individual nucleic acid probe to the one or more indels and (ii) dehybridization of at least a portion of the individual nucleic acid probe that has been hybridized to the one or more indels;

(b) detecting the activatable reporter agent that is activated, to identify one or more cell-free nucleic acid molecules of the plurality of cell-free nucleic acid molecules, wherein each of the one or more cell-free nucleic acid molecules comprises the one or more indels; and (c) analyzing the identified one or more cell-free nucleic acid molecules to determine a condition of the subject.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject, wherein an individual nucleic acid probe of the set of nucleic acid probes is designed to hybridize to at least a portion of a target cell-free nucleic acid molecule comprising one or more insertions or deletions (indels) relative to a reference genomic sequence, and wherein the individual nucleic acid probe comprises an activatable reporter agent, activation of the activatable reporter agent being selected from the group consisting of: (i) hybridization of the individual nucleic acid probe to the one or more indels and (ii) dehybridization of at least a portion of the individual nucleic acid probe that has been hybridized to the one or more indels; (b) detecting the activatable reporter agent that is activated, to identify one or more cell-free nucleic acid molecules of the plurality of cell-free nucleic acid molecules, wherein each of the one or more cell-free nucleic acid molecules comprises the one or more indels, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a condition of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, the activatable reporter agent is activated upon hybridization of the individual nucleic acid probe to the one or more indels. In some embodiments, the activatable reporter agent is activated upon dehybridization of at least a portion of the individual nucleic acid probe that has been hybridized to the one or more indels. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the one or more indels as different variables. In some embodiments, the analyzing of the identified one or more cell-free nucleic acid molecules is not based on other cell-free nucleic acid molecules of the plurality of cell-free nucleic acid molecules that do not comprise the one or more indels. In some embodiments, a number of the one or more indels from the identified one or more cell-free nucleic acid molecules is indicative of the condition of the subject. In some embodiments, a ratio of (i) the number of the one or more indels from the one or more cell-free nucleic acid molecules and (ii) a number of single nucleotide variants (SNVs) from the one or more cell-free nucleic acid molecules is indicative of the condition of the subject. In some embodiments, a frequency of the one or more indels in the identified one or more cell-free nucleic acid molecules is indicative of the condition of the subject. In some embodiments, the frequency is indicative of a diseased cell associated with the condition. In some embodiments, the condition is diffuse large B-cell lymphoma, and wherein the frequency is indicative of whether the one or more cell-free nucleic acid molecules are derived from germinal center B-cell (GCB) or activated B-cell (ABC). In some embodiments, genomic origin of the identified one or more cell-free nucleic acid molecules is indicative of the condition of the subject.

In some embodiments, the one or more indels comprises at least 3, at least 4, at least 5, at least 10, at least 15, at least 20, or at least 25 indels within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the subject. In some embodiments, the sample is a healthy sample. In some embodiments, the sample comprises a healthy cell. In some embodiments, the healthy cell comprises a healthy leukocyte. In some embodiments, the sample is a diseased sample. In some embodiments, the diseased sample comprises a diseased cell. In some embodiments, the diseased cell comprises a tumor cell. In some embodiments, the diseased sample comprises a solid tumor. In some embodiments, the set of nucleic acid probes is designed based on the one or more indels that are identified by comparing (i) sequencing data from a solid tumor, lymphoma, or blood tumor of the subject and (ii) sequencing data from a healthy cell of the subject or a healthy cohort. In some embodiments, the healthy cell is from the subject. In some embodiments, the healthy cell is from the healthy cohort. In some embodiments, the set of nucleic acid probes are designed to hybridize to at least a portion of sequences of genomic loci associated with the condition. In some embodiments, the genomic loci associated with the condition are known to exhibit aberrant somatic hypermutation when the subject has the condition.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, or (ii) the genomic regions identified in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6.

In some embodiments, the method further comprises determining that the subject has the condition or determining a degree or status of the condition of the subject, based on the identified one or more cell-free nucleic acid molecules comprising the one or more indels. In some embodiments, the method further comprises determining that the one or more cell-free nucleic acid molecules are derived from a sample associated with the condition, based on performing a statistical model analysis of the identified one or more cell-free nucleic acid molecules. In some embodiments, the statistical model analysis comprises a Monte Carlo statistical analysis. In some embodiments, the method further comprises monitoring a progress of the condition of the subject based on the identified one or more cell-free nucleic acid molecules. In some embodiments, the method further comprises performing a different procedure to confirm the condition of the subject. In some embodiments, the different procedure comprises a blood test, genetic test, medical imaging, physical exam, or tissue biopsy. In some embodiments, the method further comprises determining a treatment for the condition of the subject based on the identified one or more cell-free nucleic acid molecules. In some embodiments, the subject has been subjected to a treatment for the condition prior to (a). In some embodiments, the treatment comprises chemotherapy, radiotherapy, chemoradiotherapy, immunotherapy, adoptive cell therapy, hormone therapy, targeted drug therapy, surgery, transplant, transfusion, or medical surveillance. In some embodiments, the plurality of cell-free nucleic acid molecules comprises a plurality of cell-free deoxyribonucleic acid (DNA) molecules. In some embodiments, the condition comprises a disease. In some embodiments, the plurality of cell-free nucleic acid molecules are derived from a bodily sample of the subject. In some embodiments, the bodily sample comprises plasma, serum, blood, cerebrospinal fluid, lymph fluid, saliva, urine, or stool. In some embodiments, the subject is a mammal. In some embodiments, the subject is a human. In some embodiments, the condition comprises neoplasm, cancer, or tumor. In some embodiments, the condition comprises a solid tumor. In some embodiments, the condition comprises a lymphoma. In some embodiments, the condition comprises a B-cell lymphoma. In some embodiments, the condition comprises a subtype of B-cell lymphoma selected from the group consisting of diffuse large B-cell lymphoma, follicular lymphoma, Burkitt lymphoma, and B-cell chronic lymphocytic leukemia. In some embodiments, the one or more indels have been previously identified as tumor-derived from sequencing a prior tumor sample or cell-free nucleic acid sample.

In one aspect, the present disclosure provides a method (not encompassed by the wording of the claims, but useful for understanding the invention) to perform a clinical procedure on an individual, the method comprising: obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; identifying or having identified one or more insertions or deletions (indels) within the cell-free nucleic acid sequencing result; determining or having determined, utilizing a statistical model and the identified one or more indels, that the cell-free nucleic acid sequencing result contains nucleotides derived from a neoplasm; and performing a clinical procedure on the individual to confirm the presence of the B-cell cancer, based upon determining that the cell-free nucleic acid sequencing result contains nucleic acid sequences likely derived from the B-cell cancer.

In some embodiments, the biopsy is one of blood, serum, cerebrospinal fluid, lymph fluid, urine, or stool. In some embodiments, the genomic loci are selected from (i) the genomic regions identified in Table 1, or (ii) the genomic regions identified in Table 3. In some embodiments, the sequences of the nucleic acid probes are selected from Table 6. In some embodiments, the clinical is procedure is a blood test, medical imaging, or a physical exam.

In one aspect, the present disclosure provides a method (not encompassed by the wording of the claims, but useful for understanding the invention) to treat an individual for a B-cell cancer, the method comprising: obtaining or having obtained a targeted sequencing result of a collection of cell-free nucleic acid molecules, wherein the collection of cell-free nucleic acid molecules are sourced from a liquid or waste biopsy of an individual, and wherein the targeting sequencing is performed utilizing nucleic acid probes to pull down sequences of genomic loci known to experience aberrant somatic hypermutation in a B-cell cancer; identifying or having identified one or more insertions or deletions (indels) within the cell-free nucleic acid sequencing result; determining or having determined, utilizing a statistical model and the identified one or more indels, that the cell-free nucleic acid sequencing result contains nucleotides derived from a neoplasm; and treating the individual to curtail the B-cell cancer, based upon determining that the cell-free nucleic acid sequencing result contains nucleic acid sequences derived from the B-cell cancer.

In some embodiments, the biopsy is one of blood, serum, cerebrospinal fluid, lymph fluid, urine or stool. In some embodiments, the genomic loci are selected from (i) the genomic regions identified in Table 1, or (ii) the genomic regions identified in Table 3. In some embodiments, the sequences of the nucleic acid probes are selected from Table 6. In some embodiments, the treatment is chemotherapy, radiotherapy, immunotherapy, hormone therapy, targeted drug therapy, or medical surveillance.

In one aspect, the present disclosure provides a method (not encompassed by the wording of the claims, but useful for understanding the invention) to detect cancerous minimal residual disease in an individual and to treat the individual for a cancer, the method comprising: obtaining or having obtained a targeted sequencing result of a collection of cell-free nucleic acid molecules, wherein the collection of cell-free nucleic acid molecules are sourced from a liquid or waste biopsy of an individual, wherein the liquid or waste biopsy is sourced after a series of treatments in order to detect minimal residual disease, and wherein the targeting sequencing is performed utilizing nucleic acid probes to pull down sequences of genomic loci determined to contain one or more insertions or deletions (indels), as determined by a prior sequencing result on a prior biopsy derived from the cancer; identifying or having identified at least one set of the one or more indels within the cell-free nucleic acid sequencing result; and treating the individual to curtail the cancer, based upon determining that the cell-free nucleic acid sequencing result contains nucleic acid sequences derived from the cancer.

In some embodiments, the liquid or waste biopsy is one of blood, serum, cerebrospinal fluid, lymph fluid, urine or stool. In some embodiments, the treatment is chemotherapy, radiotherapy, immunotherapy, hormone therapy, targeted drug therapy, or medical surveillance.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject who has received an organ or tissue transplant; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a presence, an absence, or an extent of transplant rejection of the subject.

In some embodiments, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules. In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a subject who has received an organ or tissue transplant; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a presence, an absence, or an extent of transplant rejection of the subject.

In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a subject who has received an organ or tissue transplant; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a presence, an absence, or an extent of transplant rejection of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, each of the one or more cell-free nucleic acid molecules comprises a plurality of phased variants relative to a reference genomic sequence. In some embodiments, a first phased variant of the plurality of phased variants and a second phased variant of the plurality of phased variants are separated by at least one nucleotide. In some embodiments, (a) to (c) are performed by a computer system. In some embodiments, the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error. In some embodiments, (b) further comprises identifying one or more insertions or deletions (indels) in the one or more cell-free nucleic acid molecules, and wherein (c) further comprises determining the presence or the absence of the transplant rejection of the subject based at least in part on the identified one or more indels.

In one aspect, the present disclosure provides a method (not encompassed by the wording of the claims, but useful for understanding the invention) of treating a transplant rejection of a subject who has received an organ or tissue transplant, the method comprising: (a) identifying the subject for treatment of the transplant rejection, wherein the subject has been determined to have the transplant rejection based on identification of one or more cell-free nucleic acid molecules from a plurality of cell-free nucleic acid molecules that is obtained or derived from the subject, wherein each of the one or more cell-free nucleic acid molecules identified comprises a plurality of phased variants relative to a reference genomic sequence that are separated by at least one nucleotide, and wherein a presence of the plurality of phased variants is indicative of the transplant rejection of the subject; and (b) subjecting the subject to the treatment based on the identification in (a).

In some embodiments, the subject has been determined to have the transplant rejection based at least in part on one or more insertions or deletions (indels) identified in the one or more cell-free nucleic acid molecules.

In one aspect, the present disclosure provides a method (not encompassed by the wording of the claims, but useful for understanding the invention) of monitoring a subject who has received an organ or tissue transplant for a presence, an absence, or an extent of transplant rejection, the method comprising: (a) determining a first state of the presence, the absence, or the extent of transplant rejection of the subject based on identification of a first set of one or more cell-free nucleic acid molecules from a first plurality of cell-free nucleic acid molecules that is obtained or derived from the subject; (b) determining a second state of the presence, the absence, or the extent of transplant rejection of the subject based on identification of a second set of one or more cell-free nucleic acid molecules from a second plurality of cell-free nucleic acid molecules that is obtained or derived from the subject, wherein the second plurality of cell-free nucleic acid molecules are obtained from the subject subsequent to obtaining the first plurality of cell-free nucleic acid molecules from the subject; and (c) determining a transplant rejection status of the subject based on the first state and the second state, wherein each of the one or more cell-free nucleic acid molecules comprises a plurality of phased variants relative to a reference genomic sequence that are separated by at least one nucleotide.

In some embodiments, the transplant rejection status is at least a partial transplant rejection. In some embodiments, a presence of the plurality of phased variants is indicative of the first state or the second state. In some embodiments, the second plurality of cell-free nucleic acid molecules is obtained from the subject at least about 1 week, at least about 2 weeks, at least about 3 weeks, at least about 4 weeks, at least about 2 months, or at least about 3 months subsequent to obtaining the first plurality of cell-free nucleic acid molecules from the subject. In some embodiments, the subject is subjected to a treatment for the transplant rejection (i) prior to obtaining the second plurality of cell-free nucleic acid molecules from the subject and (ii) subsequent to obtaining the first plurality of cell-free nucleic acid molecules from the subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the transplant rejection. In some embodiments, the subject has been determined to have the presence or the absence of the transplant rejection based at least in part on one or more insertions or deletions (indels) identified in the one or more cell-free nucleic acid molecules.

In one aspect, the present disclosure provides a method comprising: (a) providing a mixture comprising (1) a set of nucleic acid probes and (2) a plurality of cell-free nucleic acid molecules that is obtained or derived from a subject who has received an organ or tissue transplant, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a presence, an absence, or an extent of transplant rejection of the subject.

In some embodiments, (b) further comprises identifying one or more insertions or deletions (indels) in the one or more cell-free nucleic acid molecules, and wherein (c) further comprises determining the presence or the absence of the transplant rejection of the subject based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a subject who has received an organ or tissue transplant, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a presence, an absence, or an extent of transplant rejection of the subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, a first phased variant of the plurality of phased variants and a second phased variant of the plurality of phased variants are separated by at least one nucleotide. In some embodiments, the activatable reporter agent is activated upon hybridization of the individual nucleic acid probe to the plurality of phased variants. In some embodiments, the activatable reporter agent is activated upon dehybridization of at least a portion of the individual nucleic acid probe that has been hybridized to the plurality of phased variants. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables. In some embodiments, the analyzing of the identified one or more cell-free nucleic acid molecules is not based on other cell-free nucleic acid molecules of the plurality of cell-free nucleic acid molecules that do not comprise the plurality of phased variants. In some embodiments, a number of the plurality of phased variants from the identified one or more cell-free nucleic acid molecules is indicative of the presence, the absence, or the extent of transplant rejection of the subject. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the presence, the absence, or the extent of transplant rejection of the subject. In some embodiments, a frequency of the plurality of phased variants in the identified one or more cell-free nucleic acid molecules is indicative of the presence or the absence of the transplant rejection of the subject. In some embodiments, the frequency is indicative of a diseased cell associated with the presence, the absence, or the extent of transplant rejection. In some embodiments, genomic origin of the identified one or more cell-free nucleic acid molecules is indicative of the presence or the absence of the transplant rejection of the subject. In some embodiments, the first and second phased variants are separated by at least 2, at least 3, at least 4, at least 5, at least 6, at least 7, or at least 8 nucleotides. In some embodiments, the first and second phased variants are separated by at most about 180, at most about 170, at most about 160, at most about 150, or at most about 140 nucleotides.

In some embodiments, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV. In some embodiments, the plurality of phased variants comprises at least 3, at least 4, at least 5, at least 10, at least 15, at least 20, or at least 25 phased variants within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the subject. In some embodiments, the sample is a healthy sample. In some embodiments, the sample comprises a healthy cell. In some embodiments, the healthy cell comprises a healthy leukocyte. In some embodiments, the sample is a diseased sample. In some embodiments, the diseased sample comprises a diseased cell. In some embodiments, the healthy cell is from the subject. In some embodiments, the healthy cell is from the healthy cohort. In some embodiments, the set of nucleic acid probes are designed to hybridize to at least a portion of sequences of genomic loci associated with the presence or the absence of the transplant rejection. In some embodiments, the genomic loci associated with the presence, the absence, or the extent of transplant rejection are known to exhibit aberrant somatic hypermutation when the subject has the transplant rejection.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6. In some embodiments, the method further comprises determining the presence or the absence of the transplant rejection or determining a degree or status thereof, based on the identified one or more cell-free nucleic acid molecules comprising the plurality of phased variants. In some embodiments, the method further comprises determining that the one or more cell-free nucleic acid molecules are derived from a sample associated with the presence or the absence of the transplant rejection, based on performing a statistical model analysis of the identified one or more cell-free nucleic acid molecules. In some embodiments, the statistical model analysis comprises a Monte Carlo statistical analysis. In some embodiments, the method further comprises monitoring a progress of the presence, the absence, or the extent of transplant rejection of the subject based on the identified one or more cell-free nucleic acid molecules. In some embodiments, the method further comprises performing a different procedure to confirm the presence, the absence, or the extent of transplant rejection of the subject. In some embodiments, the different procedure comprises a blood test, genetic test, medical imaging, physical exam, or tissue biopsy. In some embodiments, the method further comprises determining a treatment for the transplant rejection of the subject based on the identified one or more cell-free nucleic acid molecules. In some embodiments, the subject has been subjected to a treatment for the transplant rejection prior to (a). In some embodiments, the plurality of cell-free nucleic acid molecules comprise a plurality of cell-free deoxyribonucleic acid (DNA) molecules. In some embodiments, the plurality of cell-free nucleic acid molecules are derived from a bodily sample of the subject. In some embodiments, the bodily sample comprises plasma, serum, blood, cerebrospinal fluid, lymph fluid, saliva, urine, or stool. In some embodiments, the subject is a mammal. In some embodiments, the subject is a human. In some embodiments, (b) further comprises identifying one or more insertions or deletions (indels) in the one or more cell-free nucleic acid molecules, and wherein (c) further comprises determining the presence, the absence, or the extent of transplant rejection of the subject based at least in part on the identified one or more indels.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a pregnant subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide; and (c) analyzing, by the computer system, the identified one or more cell-free nucleic acid molecules to determine a presence, an absence, or an elevated risk of a genetic abnormality of a fetus of the pregnant subject.

In some embodiments, the at least about 10% of the cell-free nucleic acid molecules comprise at least about 20%, at least about 30%, at least about 40%, at least about 50%, at least about 60%, at least about 70%, at least about 80%, at least about 90%, or about 100% of the one or more cell-free nucleic acid molecules. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) comprising: (a) obtaining, by a computer system, sequencing data derived from a plurality of cell-free nucleic acid molecules that is obtained or derived from a pregnant subject; (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide; and (c) analyzing, by the computer system, the identified one or more cell-free nucleic acid molecules to determine a presence, an absence, or an elevated risk of a genetic abnormality of a fetus of the pregnant subject.

In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) comprising: (a) obtaining sequencing data derived from a plurality of cell-free nucleic acid molecules that is obtained or derived from a pregnant subject; (b) processing the sequencing data to identify one or more cell-free nucleic acid molecules of the plurality of cell-free nucleic acid molecules with a limit of detection of less than about 1 out of 50,000 observations from the sequencing data; and (c) analyzing the identified one or more cell-free nucleic acid molecules to determine a presence, an absence, or an elevated risk of a genetic abnormality of a fetus of the pregnant subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 observations from the sequencing data. In some embodiments, each of the one or more cell-free nucleic acid molecules comprises a plurality of phased variants relative to a reference genomic sequence. In some embodiments, a first phased variant of the plurality of phased variants and a second phased variant of the plurality of phased variants are separated by at least one nucleotide. In some embodiments, (a) to (c) are performed by a computer system. In some embodiments, he method of any one of claims 309-313, wherein the sequencing data is generated based on nucleic acid amplification. In some embodiments, the sequencing data is generated based on polymerase chain reaction. In some embodiments, the sequencing data is generated based on amplicon sequencing. In some embodiments, the sequencing data is generated based on next-generation sequencing (NGS). In some embodiments, the sequencing data is generated based on non-hybridization-based NGS. In some embodiments, the sequencing data is generated without use of molecular barcoding of at least a portion of the plurality of cell-free nucleic acid molecules. In some embodiments, the sequencing data is obtained without use of sample barcoding of at least a portion of the plurality of cell-free nucleic acid molecules. In some embodiments, the sequencing data is obtained without in silico removal or suppression of (i) background error or (ii) sequencing error. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels. In some embodiments, the genetic abnormality is a chromosomal aneuploidy. In some embodiments, the chromosomal aneuploidy is in chromosome 13, 18, 21, X, or Y.

In one aspect, the present disclosure provides a method (not encompassed by the wording of the claims, but useful for understanding the invention) of monitoring a pregnant subject for a presence, an absence, or an elevated risk of a genetic abnormality of a fetus of the pregnant subject, the method comprising: (a) determining a first state of the presence, the absence, or the elevated risk of the genetic abnormality of the fetus of the pregnant subject based on identification of a first set of one or more cell-free nucleic acid molecules from a first plurality of cell-free nucleic acid molecules that is obtained or derived from the pregnant subject; (b) determining a second state of the presence, the absence, or the elevated risk of the genetic abnormality of the fetus of the pregnant subject based on identification of a second set of one or more cell-free nucleic acid molecules from a second plurality of cell-free nucleic acid molecules that is obtained or derived from the pregnant subject, wherein the second plurality of cell-free nucleic acid molecules are obtained from the pregnant subject subsequent to obtaining the first plurality of cell-free nucleic acid molecules from the pregnant subject; and (c) determining the presence, the absence, or the elevated risk of the genetic abnormality of the fetus of the pregnant subject based on the first state and the second state, wherein each of the one or more cell-free nucleic acid molecules comprises a plurality of phased variants relative to a reference genomic sequence that are separated by at least one nucleotide.

In some embodiments, the transplant rejection status is at least a partial transplant rejection. In some embodiments, a presence of the plurality of phased variants is indicative of the first state or the second state. In some embodiments, the second plurality of cell-free nucleic acid molecules is obtained from the pregnant subject at least about 1 week, at least about 2 weeks, at least about 3 weeks, at least about 4 weeks, at least about 2 months, or at least about 3 months subsequent to obtaining the first plurality of cell-free nucleic acid molecules from the pregnant subject. In some embodiments, the one or more cell-free nucleic acid molecules are captured from among the plurality of cell-free nucleic acid molecules with a set of nucleic acid probes, wherein the set of nucleic acid probes is configured to hybridize to at least a portion of cell-free nucleic acid molecules comprising one or more genomic regions associated with the genetic abnormality. In some embodiments, the fetus has been determined to have the presence, the absence, or the elevated risk of the genetic abnormality based at least in part on one or more insertions or deletions (indels) identified in the one or more cell-free nucleic acid molecules.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a pregnant subject, wherein an individual nucleic acid probe of the set of nucleic acid probes is designed to hybridize to at least a portion of a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants; and (c) analyzing the identified one or more cell-free nucleic acid molecules to determine a presence, an absence, or an elevated risk of a genetic abnormality of a fetus of the pregnant subject.

In some embodiments, (b) further comprises identifying one or more insertions or deletions (indels) in the one or more cell-free nucleic acid molecules, and wherein (c) further comprises determining the presence, the absence, or the elevated risk of the genetic abnormality based at least in part on the identified one or more indels.

In one aspect, the present disclosure provides a method (not encompassed by the wording of the claims, but useful for understanding the invention) comprising: (a) providing a mixture comprising (1) a set of nucleic acid probes and (2) a plurality of cell-free nucleic acid molecules that is obtained or derived from a pregnant subject, wherein an individual nucleic acid probe of the set of nucleic acid probes is designed to hybridize to at least a portion of a target cell-free nucleic acid molecule comprising a plurality of phased variants relative to a reference genomic sequence, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants; (b) detecting the activatable reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules; and (c) analyzing the identified one or more cell-free nucleic acid molecules to determine a presence, an absence, or an elevated risk of a genetic abnormality of a fetus of the pregnant subject.

In some embodiments, the limit of detection of the identification step is less than about 1 out of 100,000, less than about 1 out of 500,000, less than about 1 out of 1,000,000, less than about 1 out of 1,500,000, or less than about 1 out of 2,000,000 cell-free nucleic acid molecules of the plurality of cell-free nucleic acid molecules. In some embodiments, a first phased variant of the plurality of phased variants and a second phased variant of the plurality of phased variants are separated by at least one nucleotide. In some embodiments, the activatable reporter agent is activated upon hybridization of the individual nucleic acid probe to the plurality of phased variants. In some embodiments, the activatable reporter agent is activated upon dehybridization of at least a portion of the individual nucleic acid probe that has been hybridized to the plurality of phased variants. In some embodiments, the method further comprises mixing (1) the set of nucleic acid probes and (2) the plurality of cell-free nucleic acid molecules. In some embodiments, the activatable reporter agent is a fluorophore. In some embodiments, analyzing the identified one or more cell-free nucleic acid molecules comprises analyzing (i) the identified one or more cell-free nucleic acid molecules and (ii) other cell-free nucleic acid molecules of the plurality of cell-free nucleic acid molecules that do not comprise the plurality of phased variants as different variables. In some embodiments, the analyzing of the identified one or more cell-free nucleic acid molecules is not based on other cell-free nucleic acid molecules of the plurality of cell-free nucleic acid molecules that do not comprise the plurality of phased variants. In some embodiments, a number of the plurality of phased variants from the identified one or more cell-free nucleic acid molecules is indicative of the genetic abnormality. In some embodiments, a ratio of (i) the number of the plurality of phased variants from the one or more cell-free nucleic acid molecules and (ii) a number of single nucleotide variants (SNVs) from the one or more cell-free nucleic acid molecules is indicative of the genetic abnormality. In some embodiments, a frequency of the plurality of phased variants in the identified one or more cell-free nucleic acid molecules is indicative of the genetic abnormality. In some embodiments, genomic origin of the identified one or more cell-free nucleic acid molecules is indicative of the genetic abnormality. In some embodiments, the first and second phased variants are separated by at least 2, at least 3, at least 4, at least 5, at least 6, at least 7, or at least 8 nucleotides. In some embodiments, the first and second phased variants are separated by at most about 180, at most about 170, at most about 160, at most about 150, or at most about 140 nucleotides.

In some embodiments, at least about 10%, at least about 20%, at least about 30%, at least about 40%, or at least about 50% of the one or more cell-free nucleic acid molecules comprising a plurality of phased variants comprises a single nucleotide variant (SNV) that is at least 2 nucleotides away from an adjacent SNV. In some embodiments, the plurality of phased variants comprises at least 3, at least 4, at least 5, at least 10, at least 15, at least 20, or at least 25 phased variants within the same cell-free nucleic acid molecule. In some embodiments, the one or more cell-free nucleic acid molecules identified comprises at least 2, at least 3, at least 4, at least 5, at least 10, at least 50, at least 100, at least 500, or at least 1,000 cell-free nucleic acid molecules. In some embodiments, the reference genomic sequence is derived from a reference cohort. In some embodiments, the reference genomic sequence comprises a consensus sequence from the reference cohort. In some embodiments, the reference genomic sequence comprises at least a portion of hg19 human genome, hg18 genome, hg17 genome, hg16 genome, or hg38 genome. In some embodiments, the reference genomic sequence is derived from a sample of the pregnant subject. In some embodiments, the sample is a healthy sample. In some embodiments, the sample comprises a healthy cell. In some embodiments, the sample is a diseased sample. In some embodiments, the diseased sample comprises a diseased cell. In some embodiments, the healthy cell is from the pregnant subject. In some embodiments, the healthy cell is from the healthy cohort. In some embodiments, the set of nucleic acid probes are designed to hybridize to at least a portion of sequences of genomic loci associated with the genetic abnormality.

In some embodiments, the set of nucleic acid probes are designed to hybridize to at least about 5%, at least about 10%, at least about 20%, at least about 30%, at least about 40%, at least about 50%, at least about 60%, at least about 70%, at least about 80%, at least about 90%, or about 100% of (i) the genomic regions identified in Table 1, (ii) the genomic regions identified in Table 3, or (iii) the genomic regions identified to have a plurality of phased variants in Table 3. In some embodiments, each nucleic acid probe of the set of nucleic acid probes has at least about 70%, at least about 80%, at least about 90% sequence identity, at least about 95% sequence identity, or about 100% sequence identity to a probe sequence selected from Table 6. In some embodiments, the set of nucleic acid probes comprises at least about 5%, at least about 10%, at least about 20%, at least about 30%, at least about 40%, at least about 50%, at least about 60%, at least about 70%, at least about 80%, or at least about 90% of probe sequences in Table 6. In some embodiments, the method further comprises determining the presence, the absence, or the elevated risk of the genetic abnormality of the fetus of the pregnant subject, based on the identified one or more cell-free nucleic acid molecules comprising the plurality of phased variants. In some embodiments, the method further comprises determining that the one or more cell-free nucleic acid molecules are derived from a sample associated with the presence, the absence, or the elevated risk of the genetic abnormality of the fetus of the pregnant subject, based on performing a statistical model analysis of the identified one or more cell-free nucleic acid molecules. In some embodiments, the statistical model analysis comprises a Monte Carlo statistical analysis. In some embodiments, the method further comprises monitoring a progress of the presence, the absence, or the elevated risk of the genetic abnormality of the fetus of the pregnant subject based on the identified one or more cell-free nucleic acid molecules. In some embodiments, the method further comprises performing a different procedure to confirm the presence, the absence, or the elevated risk of the genetic abnormality of the fetus of the pregnant subject. In some embodiments, the different procedure comprises a blood test, genetic test, medical imaging, physical exam, or tissue biopsy. In some embodiments, the plurality of cell-free nucleic acid molecules comprise a plurality of cell-free deoxyribonucleic acid (DNA) molecules. In some embodiments, the plurality of cell-free nucleic acid molecules are derived from a bodily sample of the pregnant subject. In some embodiments, the bodily sample comprises plasma, serum, blood, cerebrospinal fluid, lymph fluid, saliva, urine, or stool. In some embodiments, the pregnant subject is a mammal. In some embodiments, the pregnant subject is a human. In some embodiments, (b) further comprises identifying one or more insertions or deletions (indels) in the one or more cell-free nucleic acid molecules, and wherein (c) further comprises determining the presence, the absence, or the elevated risk of the genetic abnormality of the fetus of the pregnant subject based at least in part on the identified one or more indels.

In one aspect, the present disclosure provides a computer program product (not encompassed by the wording of the claims, but useful for understanding the invention) comprising a non-transitory computer-readable medium having computer-executable code encoded therein, the computer-executable code adapted to be executed to implement any one of the methods disclosed herein.

In one aspect, the present disclosure provides a system (not encompassed by the wording of the claims, but useful for understanding the invention) comprising one or more computer processors and computer memory coupled thereto, wherein the computer memory comprises machine executable code that, upon execution by the one or more computer processors, implements any one of the methods disclosed herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## BRIEF DESCRIPTION OF THE DRAWINGS

Various features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

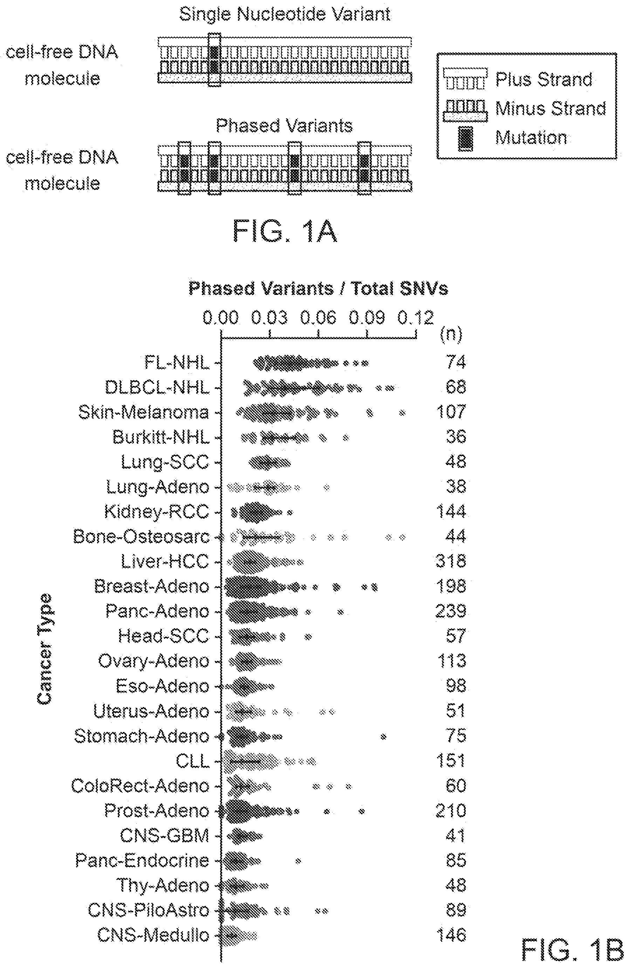

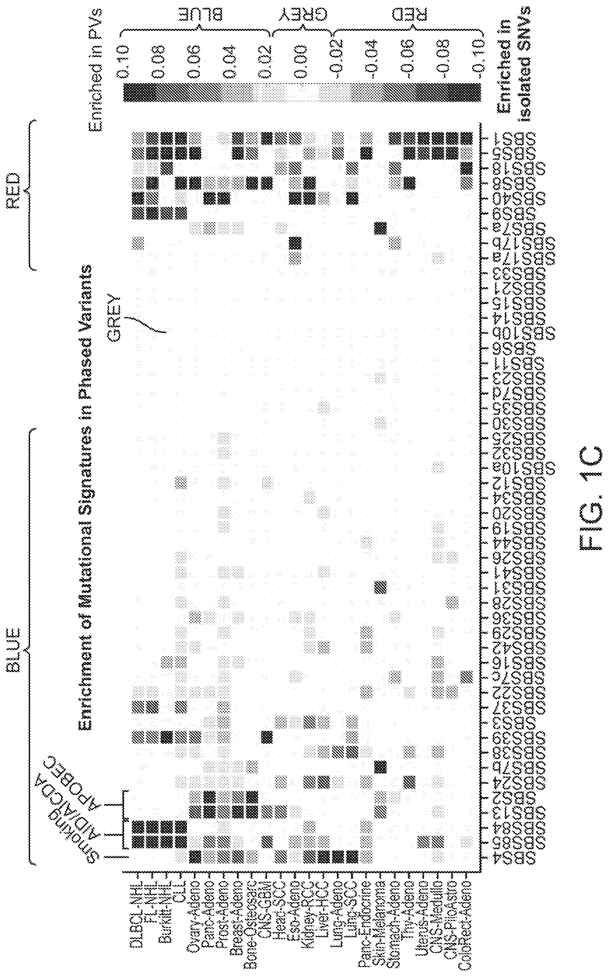

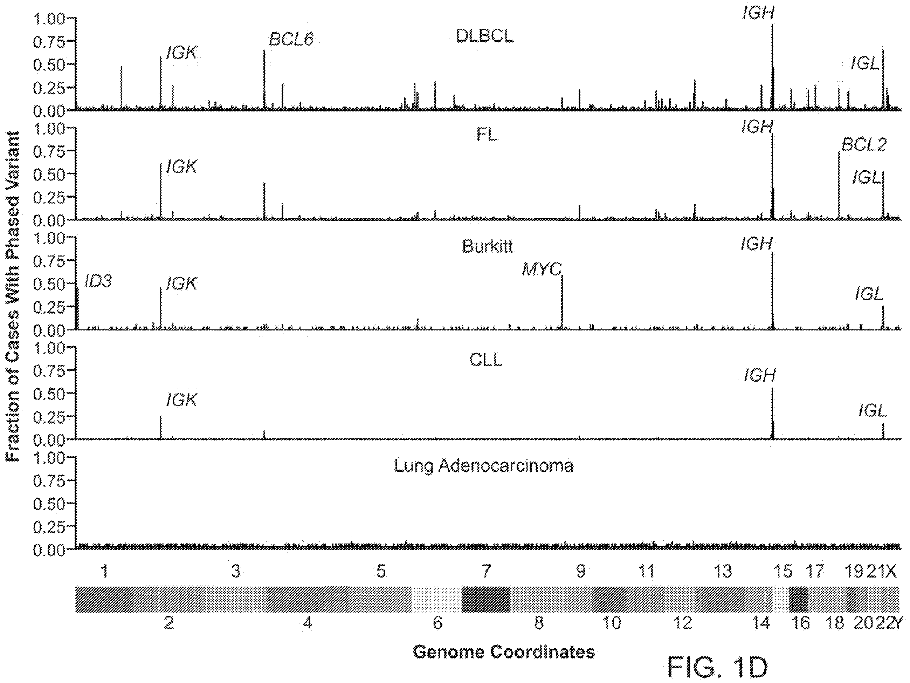

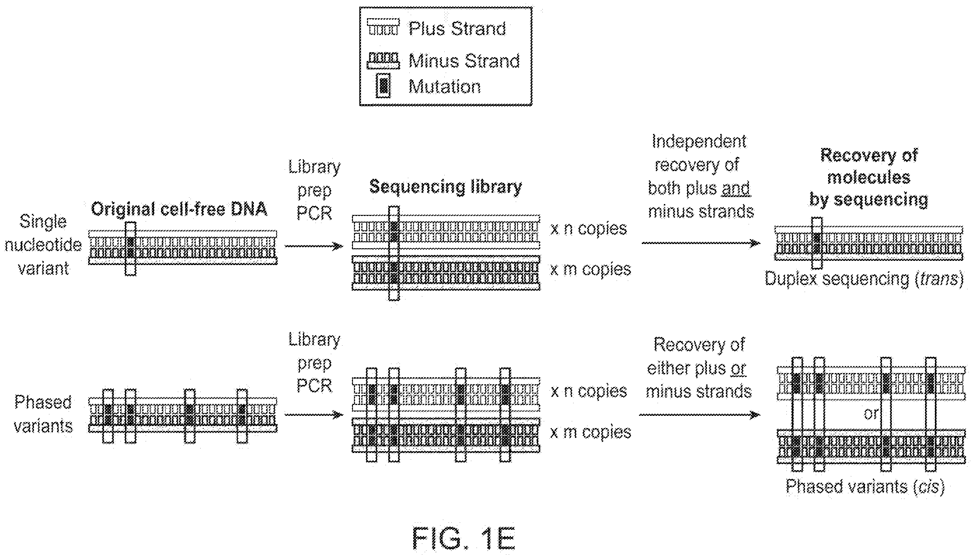

**FIGs. 1A-1E** illustrate discovery of phased variants and their mutational signatures via analysis of whole-genome sequencing data. **FIG. 1A.** is a cartoon depicting the difference between detection of a single nucleotide variant (SNV) (top) and multiple variants 'in-phase' (phased variants, PVs; bottom) on individual cell-free DNA molecules. In theory, detection of a PV is a more specific event than detection of an isolated SNV. While a phased variants are shown in this figure as SNVs, other changes relative to reference genomic sequence could also be considered as phased variants. **FIG. 1B.** is a scatter plot showing the distribution of the number of PVs from WGS data for 24 different histologies of cancer, normalized by the total number of SNVs. Bars show the median value and interquartile range. (FL-NHL, follicular lymphoma; DLBCL-NHL, diffuse large B-cell lymphoma; Burkitt-NHL, Burkitt lymphoma; Lung-SCC, squamous cell lung cancer; Lung-Adeno, lung adenocarcinoma; Kidney-RCC, renal cell carcinoma; Bone-Osteosarc, osteosarcoma; Liver-HCC, hepatocellular carcinoma; Breast-Adeno, breast adenocarcinoma; Panc-Adeno, pancreatic adenocarcinoma; Head-SCC, head and neck squamous cell carcinoma; Ovary-Adeno, ovarian adenocarcinoma; Eso-Adeno, esophageal adenocarcinoma; Uterus-Adeno, uterine adenocarcinoma; Stomach-Adeno, stomach adenocarcinoma; CLL, chronic lymphocytic leukemia; ColoRect-Adeno, colorectal adenocarcinoma; Prost-Adeno, prostate adenocarcinoma; CNS-GBM, glioblastoma multiforme; Panc-Endocrine, pancreatic neuroendocrine tumor; Thy-Adeno, thyroid adenocarcinoma; CNS-PiloAstro, piloastrocytoma; CNS-Medullo, medulloblastoma.) **FIG. 1C.** is a heatmap demonstrating the enrichment in single base substitution (SBS) mutational signatures for PVs versus single SNVs across multiple cancer types. Blue represents signatures which are enriched in PVs in specific histologies; darker gray represents signatures where un-phased, single SNVs are enriched; and red represents SNVs occurring in isolation. Only signatures which have a significant difference between PVs and unphased SNVs after correcting for multiple hypotheses are shown; other signatures are grey. Signatures associated with smoking, AID/AICDA, and APOBEC are indicated. **FIG. 1D.** demonstrate bar plots showing the distribution of PVs occurring in stereotyped regions across the genome in B-lymphoid malignancies and lung adenocarcinoma. In this plot, the genome was divided into 1000bp bins, and the fraction of samples of a given histology with a PV in each 1000bp bin was calculated. Only bins that have at least a 2 percent recurrence frequency in any cancer subtype are shown. Key genomic loci are also labeled. **FIG. 1E.** is a comparison of duplex sequencing to phased variant sequencing. A schema comparing error-suppressed sequencing by duplex sequencing vs. recovery of phased variants. In duplex sequencing, recovery of a single SNV observed on both strands of an original DNA double-helix (i.e., in trans) is required. This requires independent recovery of two molecules by sequencing as the plus and minus strands of the original DNA molecule go through library preparation and PCR independently. In contrast, recovery of PVs requires multiple SNVs observed on the same single strand of DNA (i.e., in cis). Thus, recovery of only the plus or the minus strand (rather than both) is sufficient for identification of PVs.

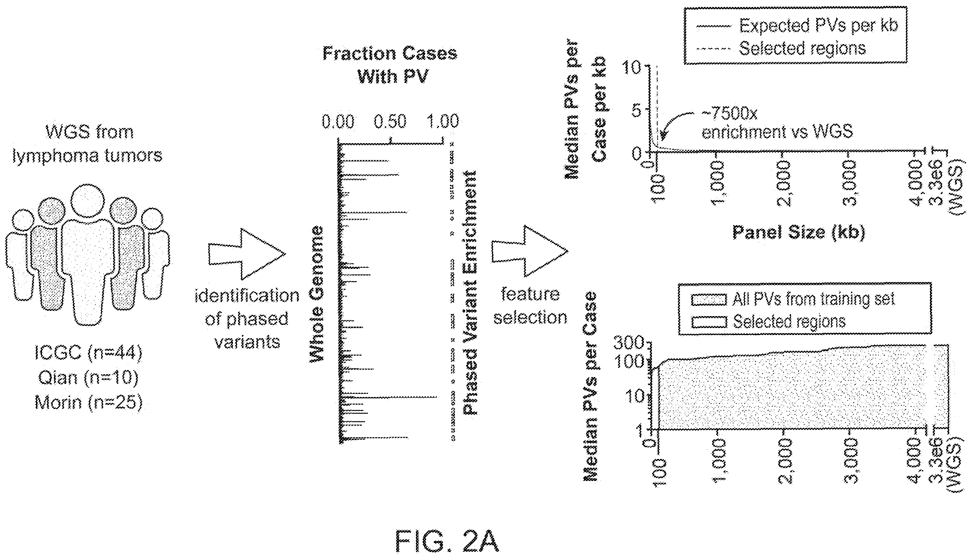

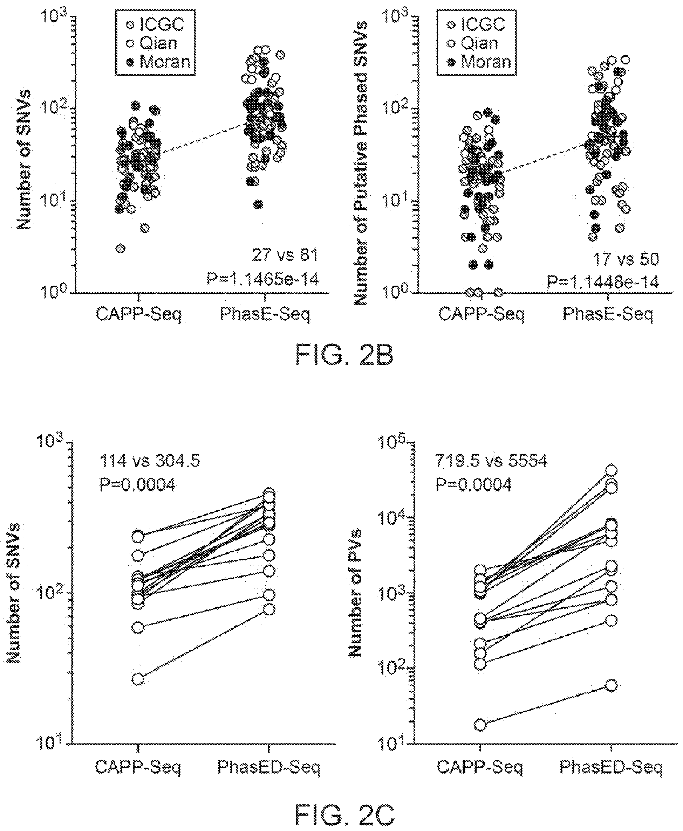

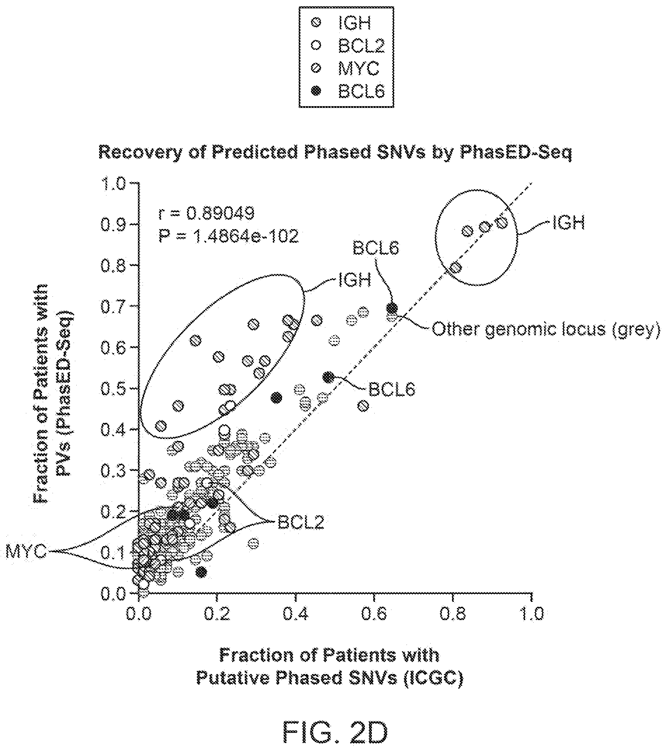

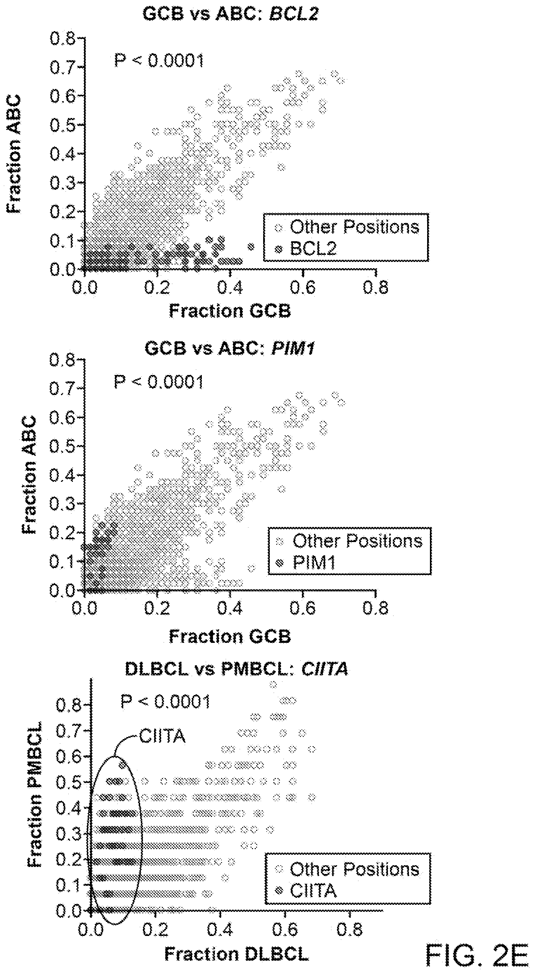

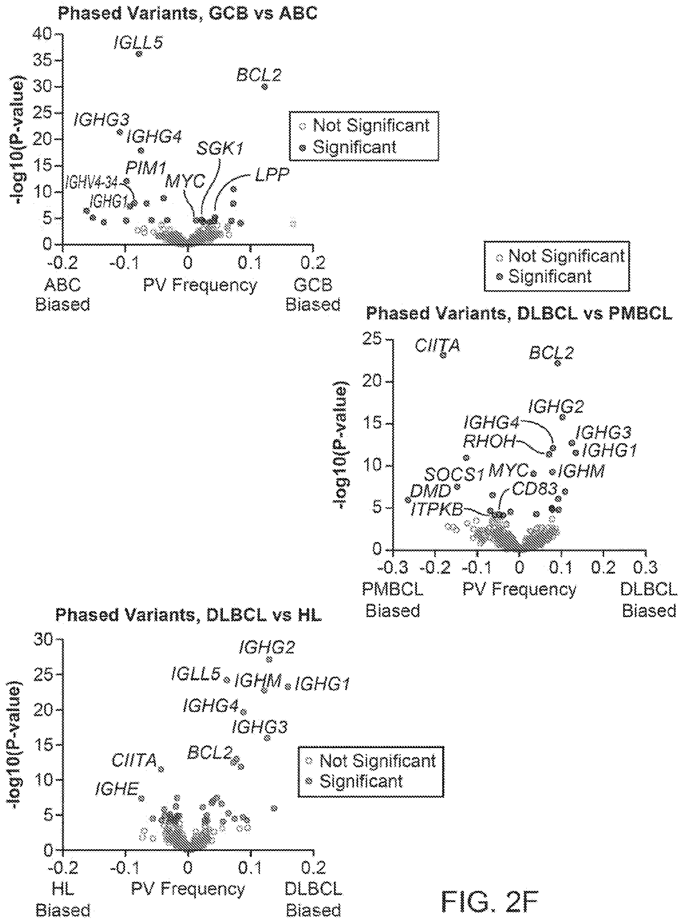

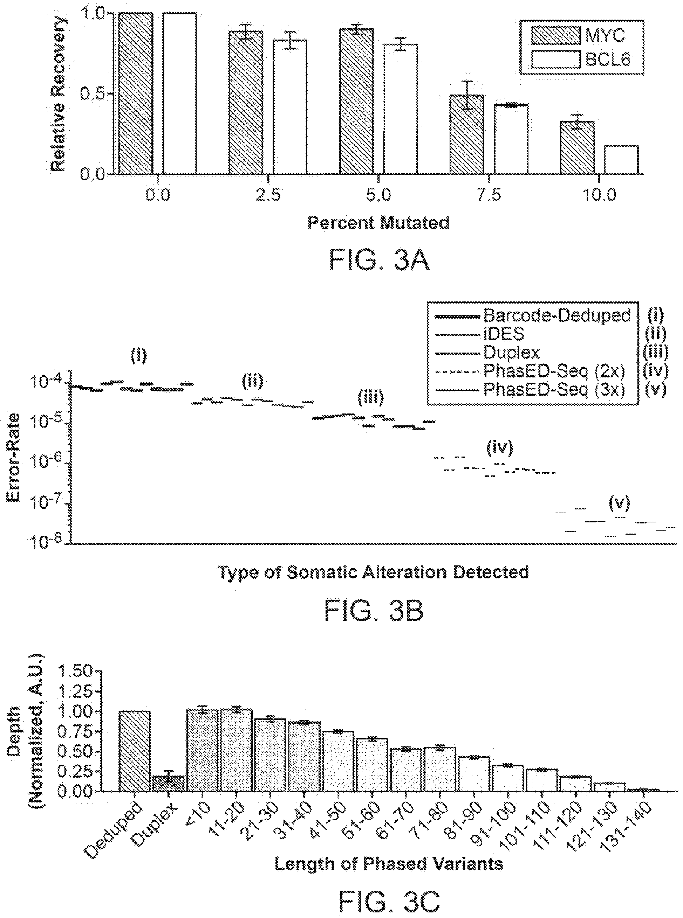

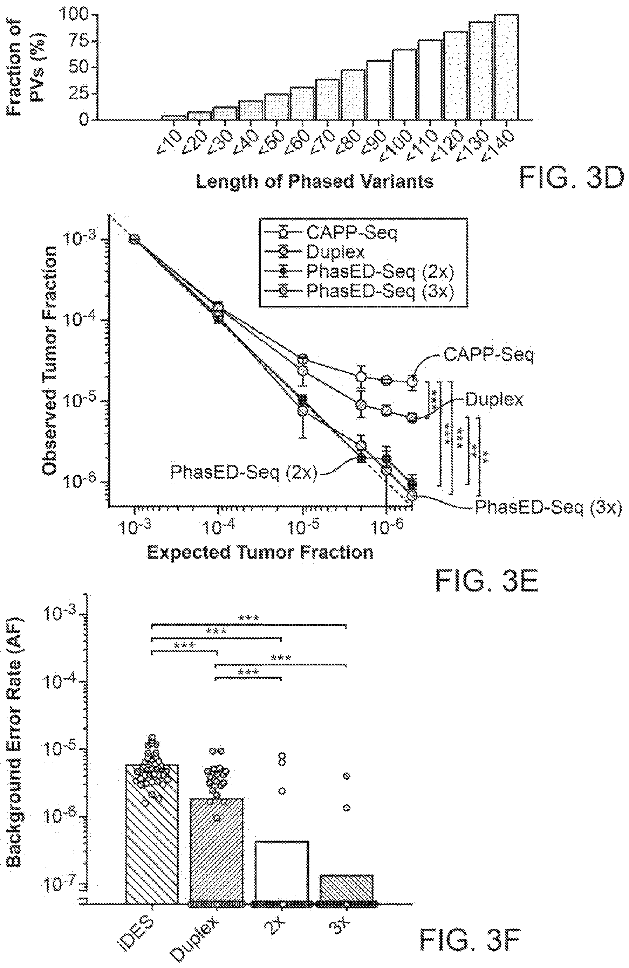

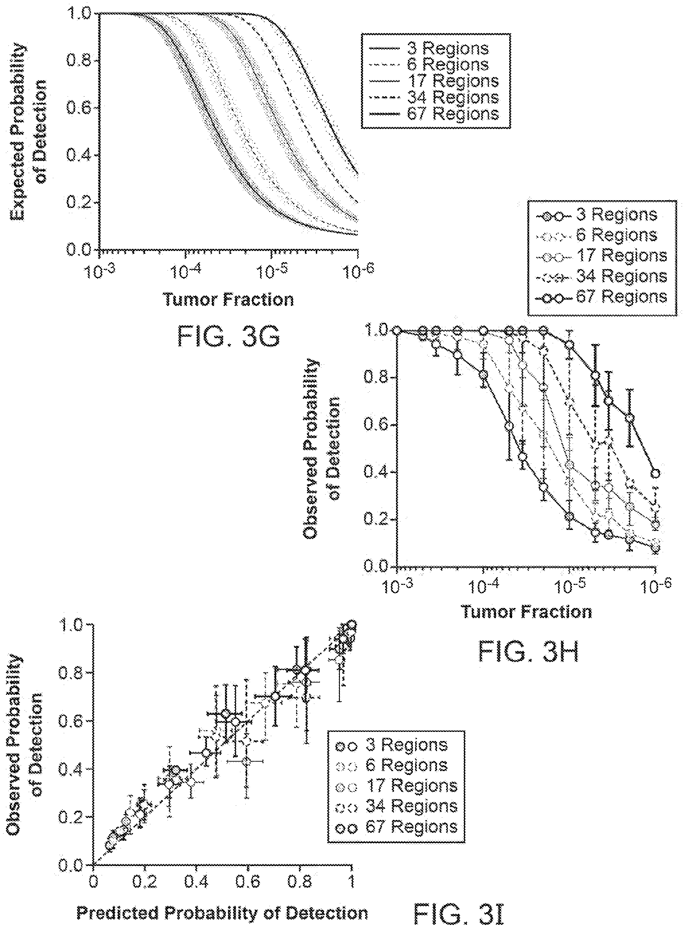

**FIGs. 2A-2F** illustrate design, validation, and application of phased variant enrichment sequencing. **FIG. 2A** is a schematic of the design for PhasED-Seq. WGS data from DLBCL tumor samples were aggregated (*left*), and areas of recurrent putative PVs were identified (*middle*). An assay capturing the genomic regions most recurrently containing PVs was then designed (*right*), resulting in an ~7500x enrichment in PVs compared to WGS. The *top right panel* shows the *in silico* expected number of PVs per case per kilobase of panel size (y-axis) for increasing panel sizes (x-axis). The dashed line shows the selected regions in the PhasED-Seq panel. The *bottom right panel* shows the total number of expected PVs per case (y-axis, assessed *in silico* from WGS data, for increasing panel sizes (y-axis). The dark area shows the selected regions in the PhasED-Seq panel. **FIG. 2B** illustrate two panels showing the yield of SNVs (left) and PVs (right) for sequencing tumor DNA and matched germline by a previously established lymphoma CAPP-Seq panel or PhasED-Seq; values are assessed in silico by limiting WGS to the targeted space of interest. PVs reported in the right panel include doublet, triplet, and quadruplet phased events. **FIG. 2C** shows the yield of SNVs (left) and PVs (right) from experimental sequencing of tumor and/or cell-free DNA from CAPP-Seq versus PhasED-Seq, similar to **FIG. 2B. FIG. 2D** is a scatterplot showing the frequency of PVs by genomic location (in 1000bp bins) for patients with DLBCL, identified either by WGS or identified by PhasED-Seq. PVs in *IGH, BCL2, MYC,* and *BCL6* are highlighted. **FIG. 2E** illustrate scatterplots comparing the frequency of PVs by genomic location (in 50bp bins) for patients with different types of lymphomas. The colored circles show the relative frequency of PVs in 50bp bins from a specific gene of interest; the other (gray) circles show the relative frequency of PVs in 50bp bins from the remainder of the PhasED-Seq sequencing panel. **FIG. 2F** illustrate volcano plots summarizing the difference in relative frequency of PVs in specific genetic loci between types of lymphoma, including ABC-DLBCL vs. GCB-DLBCL (dark Gray, left); PMBCL vs DLBCL (dark gray, middle); and HL vs. DLBCL (dark gray, right). The x-axis demonstrates the relative enrichment in PVs in a specific locus, while the y-axis demonstrates the statistical significance of this association. (**Example 10**). **FIGs. 3A-3I** illustrate technical performance of PhasED-Seq for disease detection. **FIG. 3A** illustrates bar plot showing the performance of hybrid capture sequencing for recovery of synthetic 150bp oligonucleotides from two loci (*MYC* and *BCL6*) with increasing degree of mutation / non-reference bases. Error bars represent the 95% confidence interval (n=3 replicates of each condition in distinct samples). **FIG. 3B** illustrates plot demonstrating the background error-rate (**Example 10**) for different types of error-suppression from 12 healthy control cell-free DNA samples sequenced on the PhasED-Seq panel. 'PhasED-Seq 2x' or 'doublets' represents detection of two mutations in-phase on the same DNA molecule; 'PhasED-Seq 3x' or 'triplets' represents detection of three mutations in-phase on the same DNA molecule. **FIG. 3C** illustrates bar plot showing the depth of unique molecular recovery (e.g., depth after barcode-mediated PCR duplicate removal) from sequencing data from 12 cell-free DNA samples for different types of error-suppression, including barcode deduplication, duplex sequencing, and recovery of PVs of increasing maximal distance between SNVs in-phase. **FIG. 3D** illustrates bar plot showing the cumulative fraction of PVs that have a maximal distance between SNVs less than the number of base-pairs shown on the x-axis. **FIG. 3E** illustrates a plot demonstrating the results of a limiting dilution series simulating cell-free DNA samples containing patient-specific tumor fractions of $1 \times 10^{-3}$ to $0.5 \times 10^{-6}$; cfDNA from 3 independent patients samples were used in each dilution. The same sequencing data was analyzed using a variety of error-suppression methods for recovery of expected tumor fractions, including iDES, duplex sequencing, and PhasED-Seq (both for recovery of doublet and triplet molecules). Points and error-bars represent the mean, minimum, and maximum across the three patient-specific tumor mutations considered. The difference between observed and expected tumor fractions for sample < 1:10,000 were compared via paired t-test. *, P<0.05, **, P<0.005, ***, P<0.0005. **FIG. 3F** illustrates plot demonstrating the background signal for detection of tumor-specific alleles in 12 unrelated, healthy cell-free DNA samples, and the healthy cfDNA sample used for limiting dilution series (n=13 total samples). In each sample, tumor-specific SNVs or PVs from the 3 patient samples utilized in the limiting dilution experiment shown in **FIG. 3E,** for a total of 39 assessments were assessed. Bars represent the arithmetic mean across all 39 assessments; statistical comparison performed by Wilcoxon rank-sum test. *, P<0.05, **, P<0.005, ***, P<0.0005. **FIG. 3G** illustrates plot showing the theoretical rate of detection for a sample with a given number of PV-containing regions, according to simple binomial sampling. This plot is produced by assuming a unique sequencing depth of 5000x (line), along with a varying number of independent 150bp PV-containing regions, from 3 regions (blue) to 67 regions (purple). Confidence envelopes consider depth from 4000-6000x; a 5% false-positive rate is also assumed. **FIG. 3H** illustrates plot showing the observed rate of detection (y-axis) for sample of a given true tumor fraction (x-axis), with varying numbers of PV-containing regions. For each number of tumor-reporter regions ranging from 3 to 67, this number of 150bp windows was randomly sampled from each of 3 patient-specific PV reporter lists 25 times and used to assess tumor-detection at each dilution. Filled-in points represent 'wet' dilution series experiments, while open points represent *in silico* dilution experiments. Points and error-bars represent the mean, minimum, and maximum across the three patient-specific PV reporter lists used in the original sampling. **FIG. 3I** illustrates scatter plot compares the predicted vs observed rate of detection for samples from the dilution series shown in panels **FIG. 3G** and **FIG. 3H.** Additional details of this experiment are provided in **Example 10.**

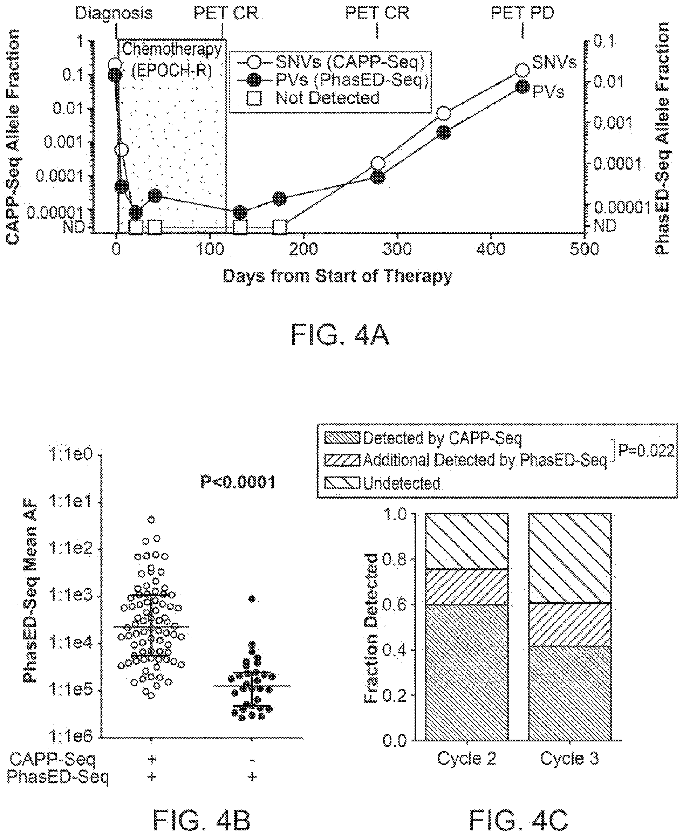

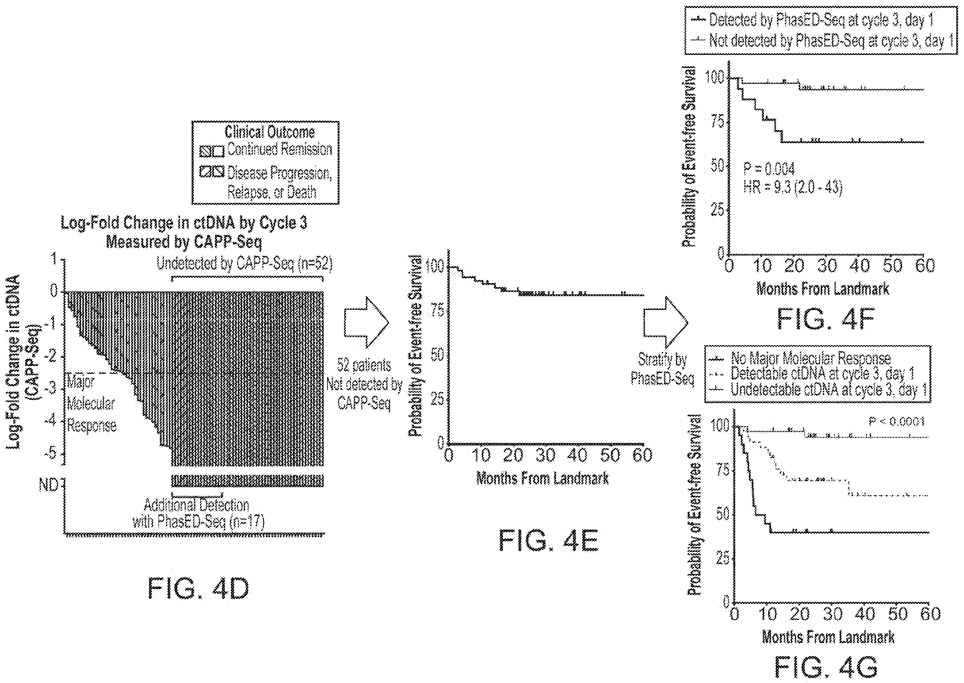

FIGs. 4A-4G illustrate clinical application of PhasED-Seq for ultra-sensitive disease detection and response monitoring in DLBCL. **FIG. 4A** illustrates plot showing ctDNA levels for a patient with DLBCL responding to, and subsequently relapsing after, first-line immuno-chemotherapy. Levels measured by CAPP-Seq are shown in darker gray circles while levels measured by PhasED-Seq are shown in lighter gray circles. Open circles represent undetectable levels by CAPP-Seq. **FIG. 4B** illustrates a univariate scatter plot showing the mean tumor allele fraction measured by PhasED-Seq for clinical samples at time-points of minimal disease (i.e., after 1 or 2 cycles of therapy). The plot is divided by samples detected vs undetected by standard CAPP-Seq; P-value from Wilcoxon rank-sum test. **FIG. 4C** illustrates bar plot showing the fraction of DLBCL patients who have detectable ctDNA by CAPP-Seq after 1 or 2 cycles of treatment (dark gray bars), as well as the fraction of additional patients with detectable disease when adding PhasED-Seq to standard CAPP-Seq (medium gray bars). P-value represents a Fisher's Exact Test for detection by CAPP-Seq alone versus the combination of PhasED-Seq and CAPP-Seq in 171 samples after 1 or 2 cycles of treatment. **FIG. 4D** illustrates a waterfall plot showing the change in ctDNA levels measured by CAPP-Seq after 2 cycles of first-line therapy in patients with DLBCL. Patients with undetectable ctDNA by CAPP-Seq are shown as "ND" ("not detected"), in darker colors. The colors of the bars also indicate the eventual clinical outcomes for these patients. **FIG. 4E** illustrates a Kaplan-Meier plot showing the event-free survival for 52 DLBCL patients with undetectable ctDNA measured by CAPP-Seq after 2 cycles. **FIG. 4F** illustrates a Kaplan-Meier plot showing the event-free survival of 52 patients shown in **FIG. 4E** (undetectable ctDNA by CAPP-Seq) stratified by ctDNA detection via PhasED-Seq at this same time-point (cycle 3, day 1). **FIG. 4G** illustrates a Kaplan-Meier plot showing the event-free survival for 89 patients with DLBCL stratified by ctDNA at cycle 3, day 1 separated into 3 strata - patients failing to achieve a major molecular response (dark gray), patients with a major molecular response who still have detectable ctDNA by PhasED-Seq and/or CAPP-Seq (light grey), and patients who have a stringent molecular remission (undetectable ctDNA by PhasED-Seq and CAPP-Seq; medium gray).

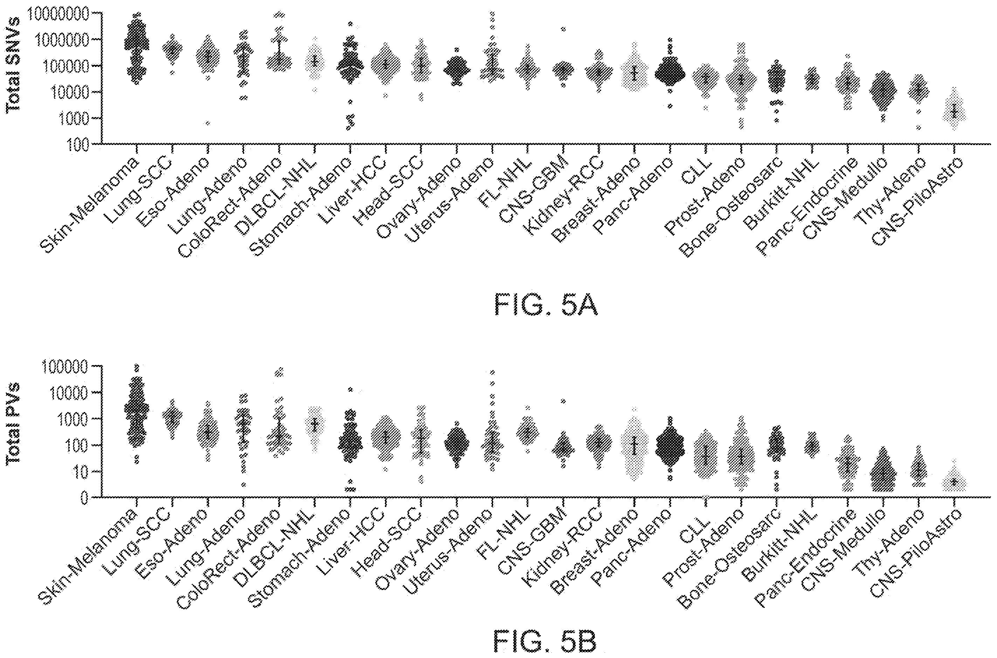

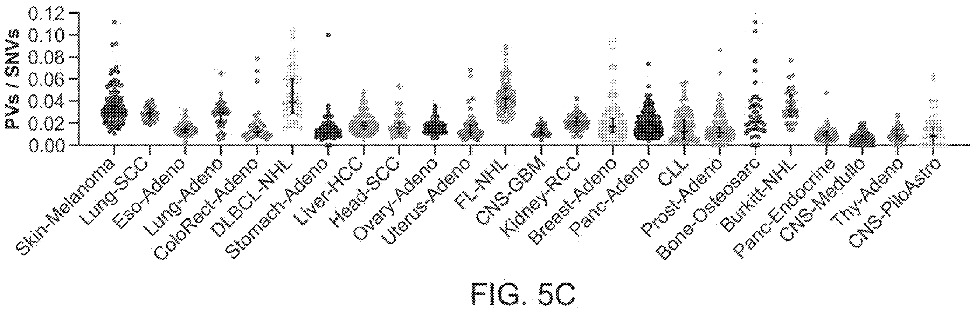

FIGs. 5A-5C illustrate enumeration of SNVs and PVs in diverse cancers from WGS. **FIG. 5A-C** illustrate Univariate scatter plots showing the number of SNVs (**FIG. 5A**), PVs (**FIG. 5B**), and PVs, controlling for total number of SNVs (**FIG. 5C**), from WGS data for 24 different histologies of cancer. Bars show the median value and interquartile range. (FL-NHL, follicular lymphoma; DLBCL-NHL, diffuse large B cell lymphoma; Burkitt-NHL, Burkitt lymphoma; Lung-SCC, squamous cell lung cancer; Lung-Adeno, lung adenocarcinoma; Kidney-RCC, renal cell carcinoma; Bone-Osteosarc, osteosarcoma; Liver-HCC, hepatocellular carcinoma; Breast-Adeno, breast adenocarcinoma; Panc-Adeno, pancreatic adenocarcinoma; Head-SCC, head and neck squamous cell carcinoma; Ovary-Adeno, ovarian adenocarcinoma; Eso-Adeno, esophageal adenocarcinoma; Uterus-Adeno, uterine adenocarcinoma; Stomach-Adeno, stomach adenocarcinoma; CLL, chronic lymphocytic leukemia; ColoRect-Adeno, colorectal adenocarcinoma; Prost-Adeno, prostate adenocarcinoma; CNS-GBM, glioblastoma multiforme; Panc-Endocrine, pancreatic neuroendocrine tumor; Thy-Adeno, thyroid adenocarcinoma; CNS-PiloAstro, piloastrocytoma; CNS-Medullo, medulloblastoma).

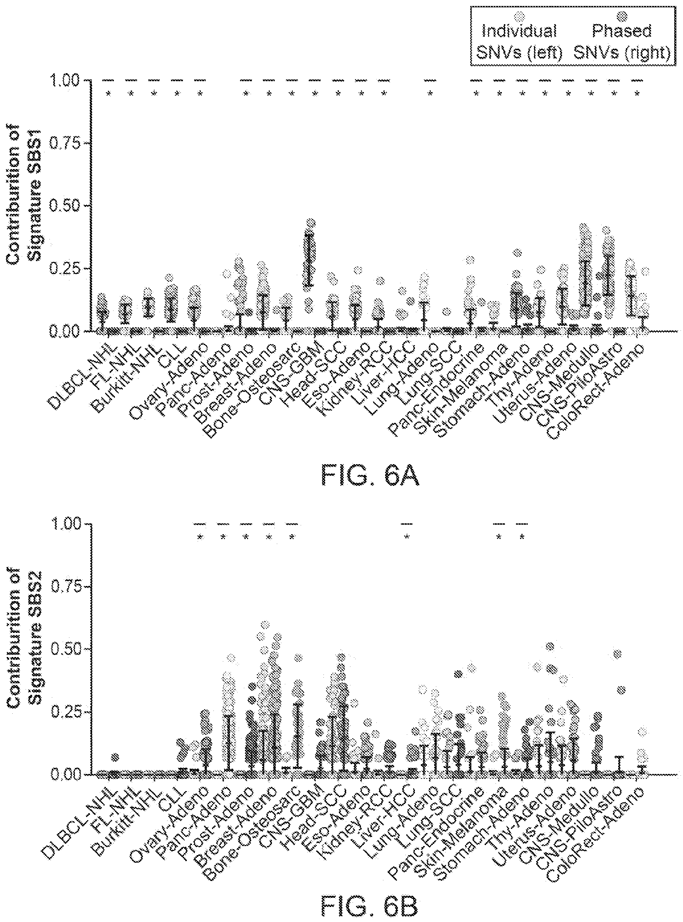

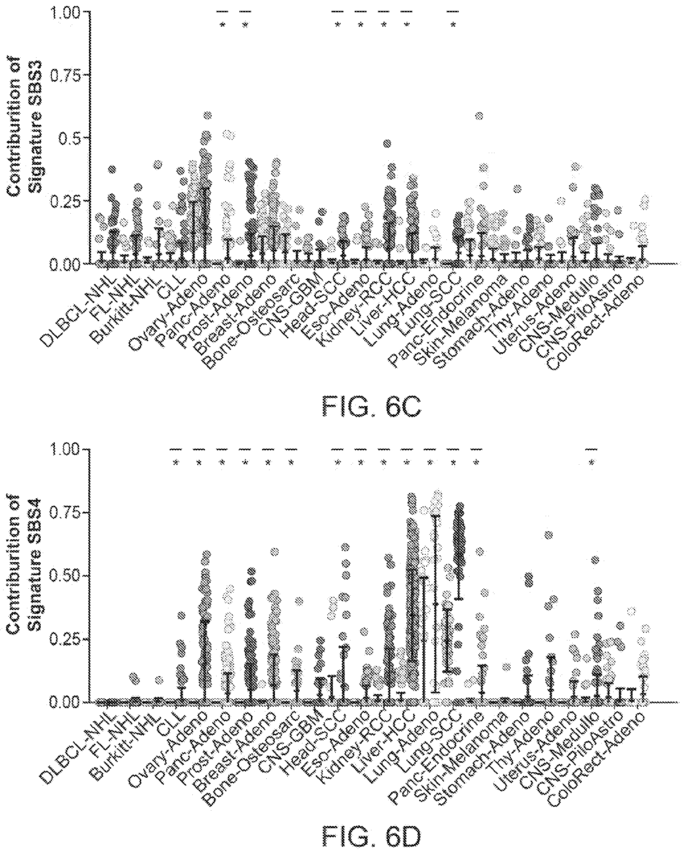

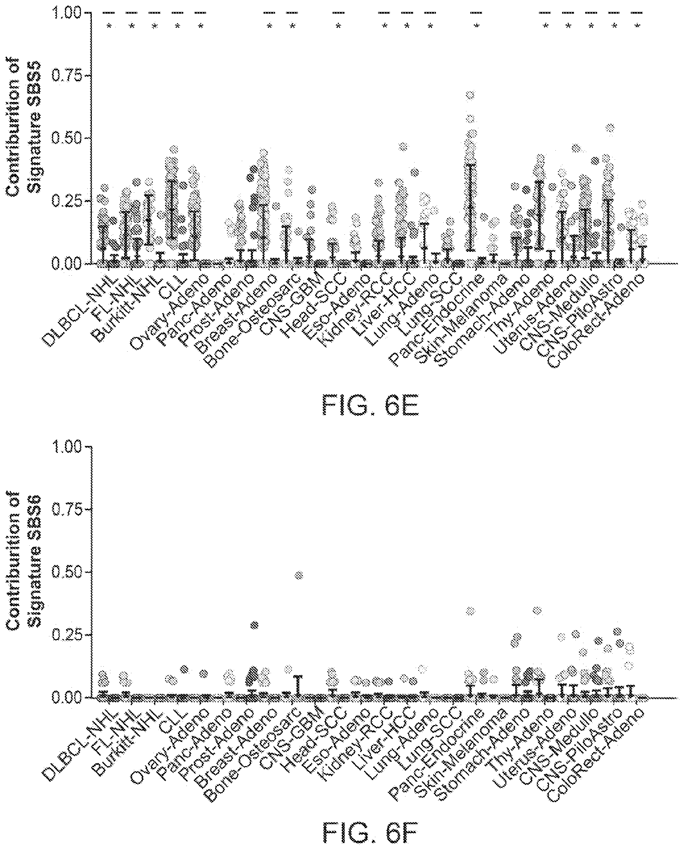

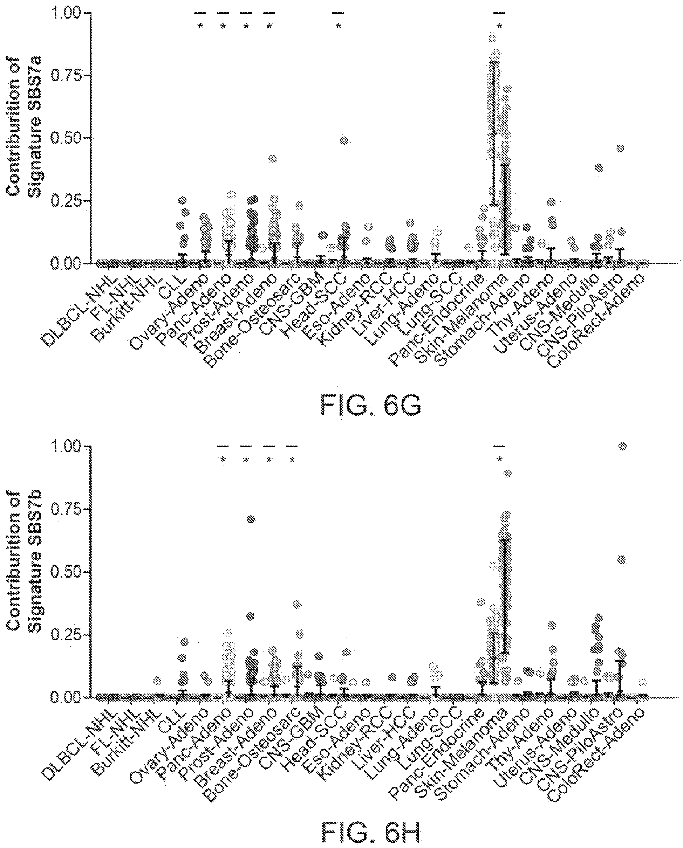

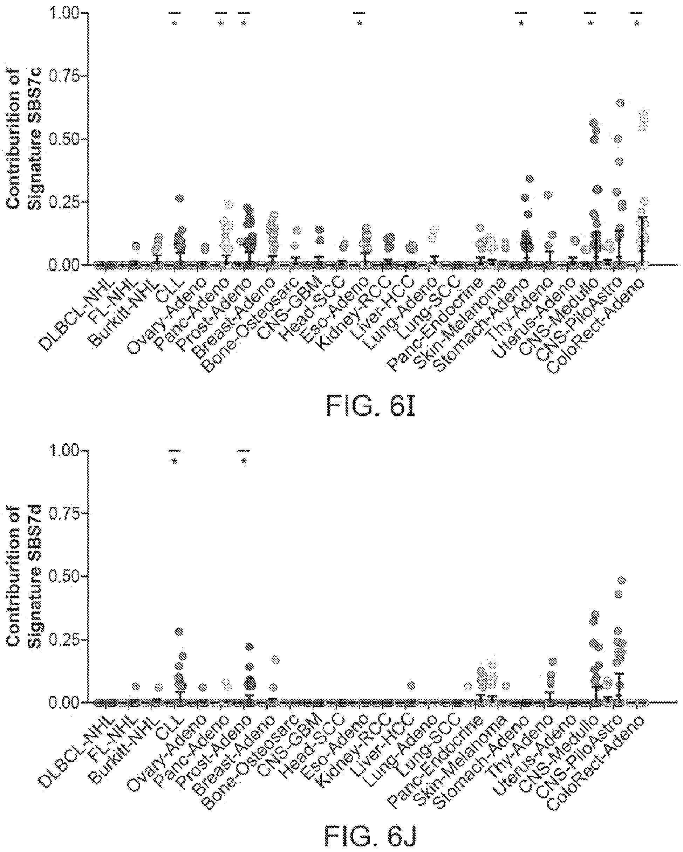

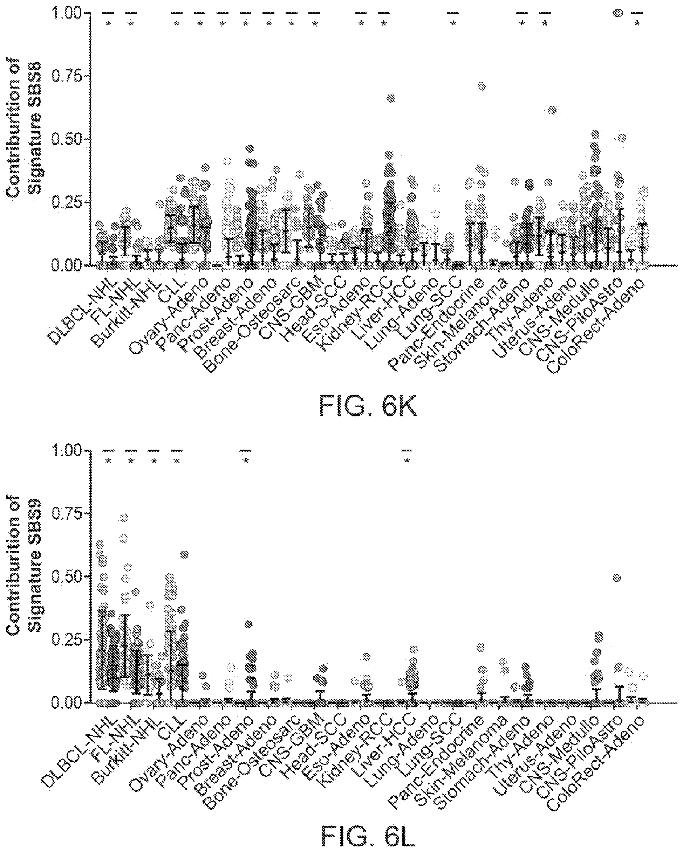

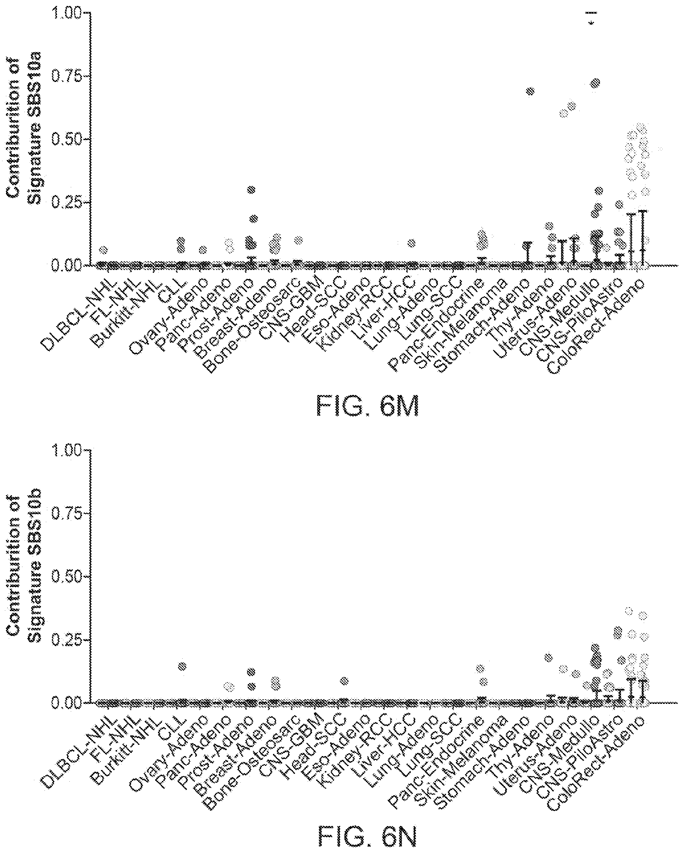

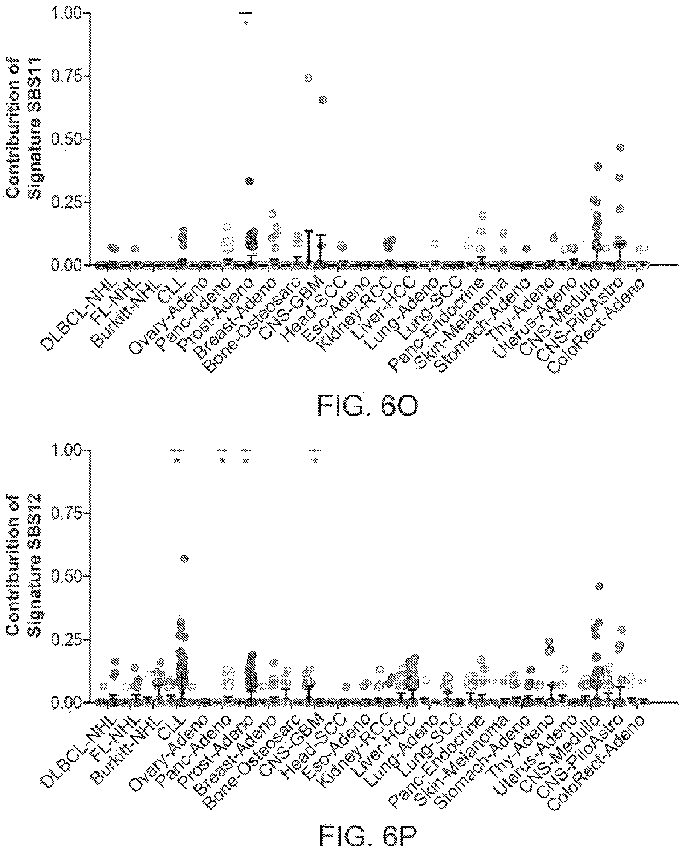

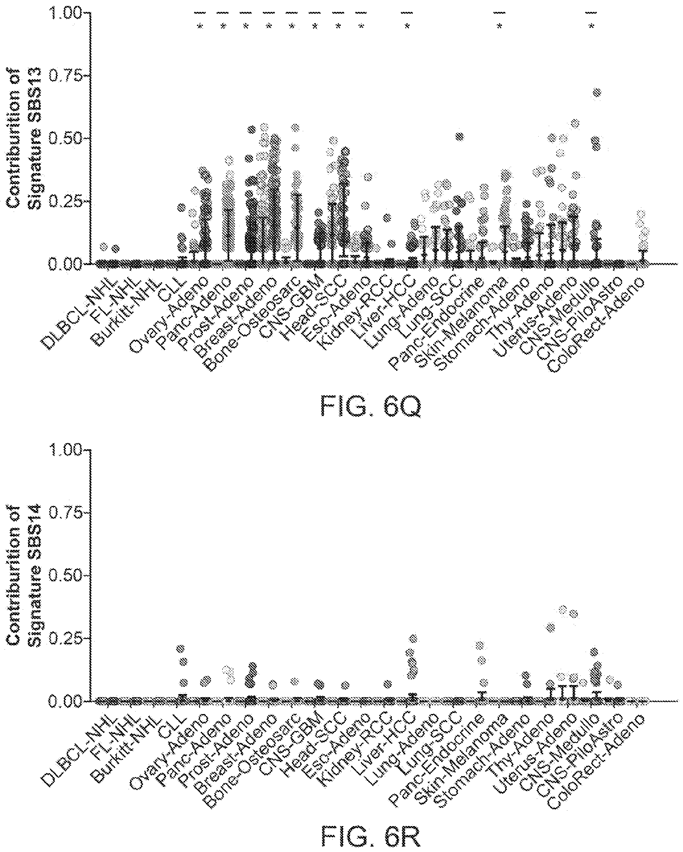

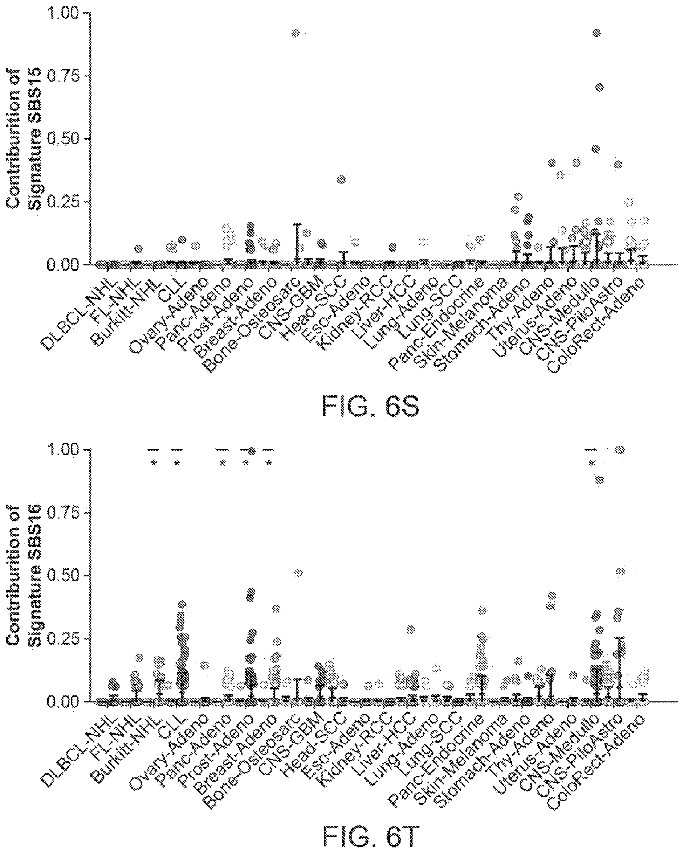

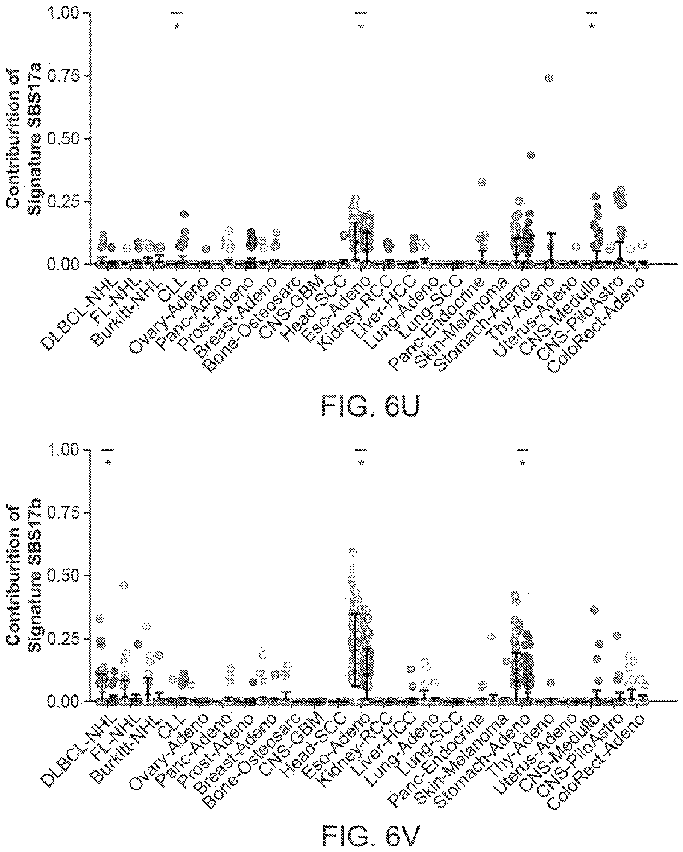

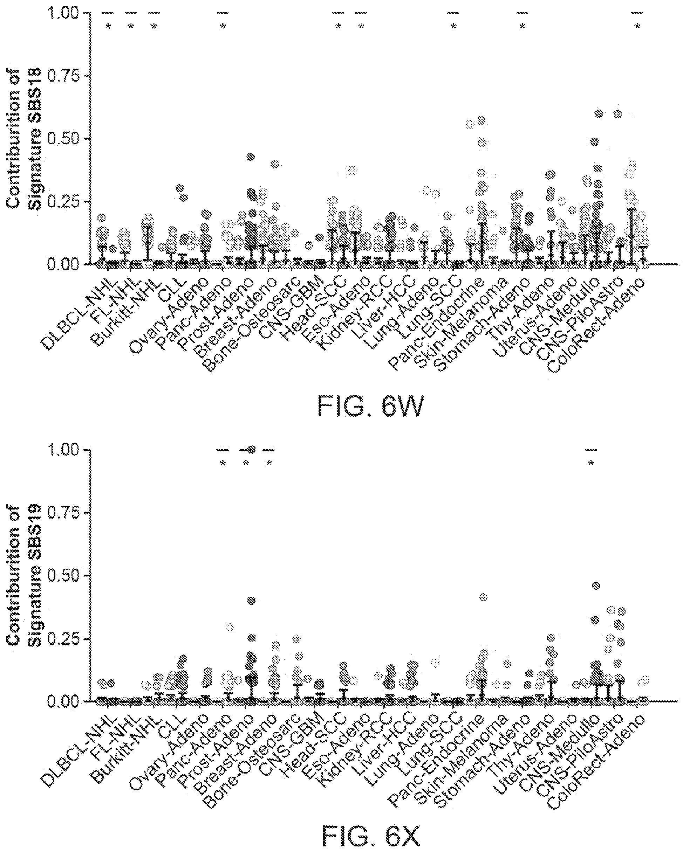

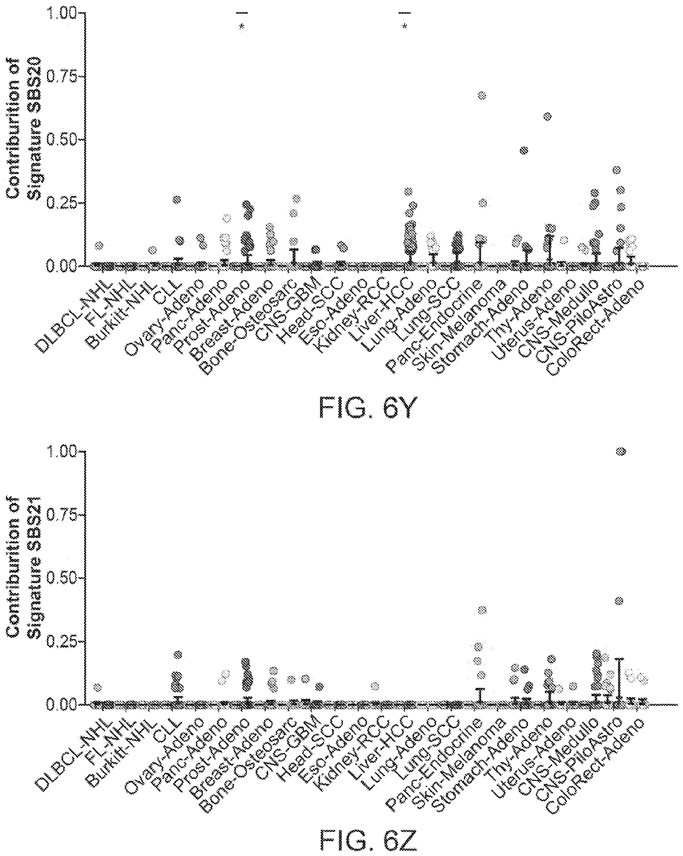

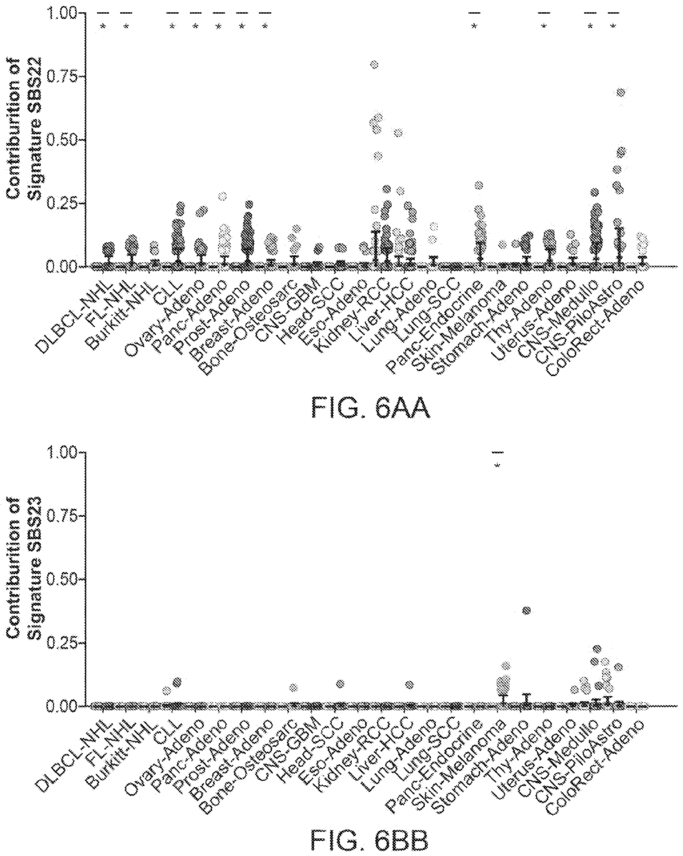

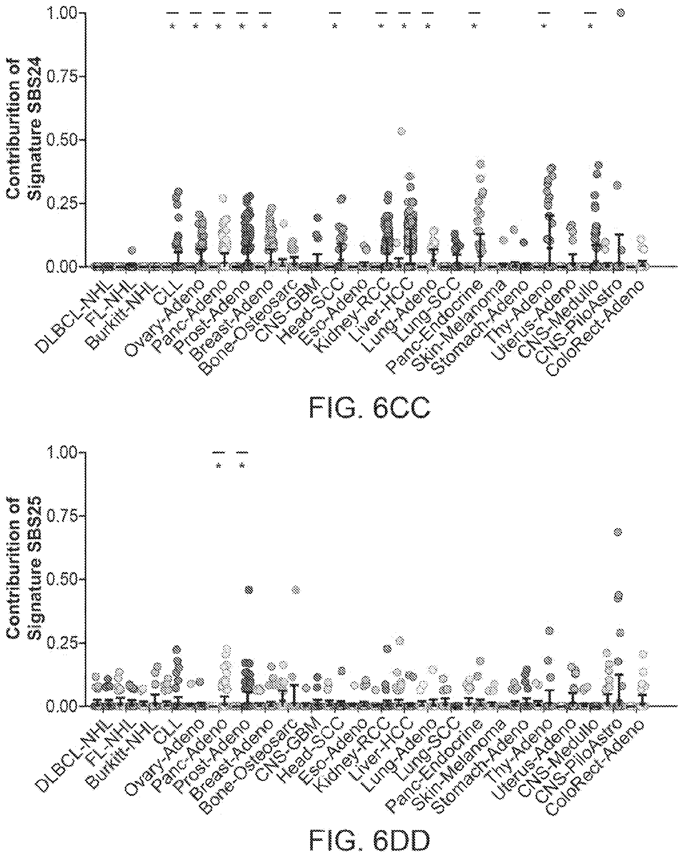

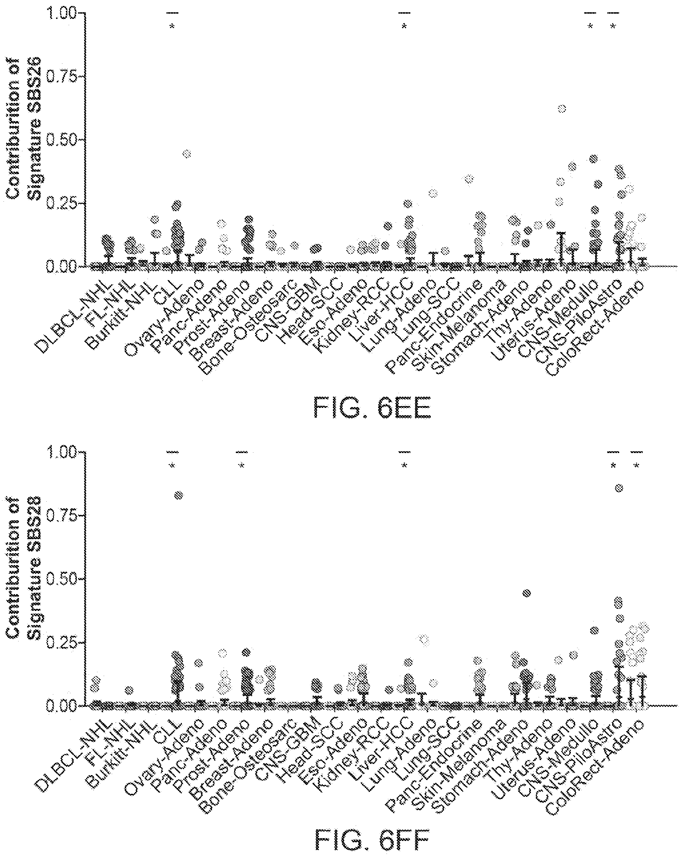

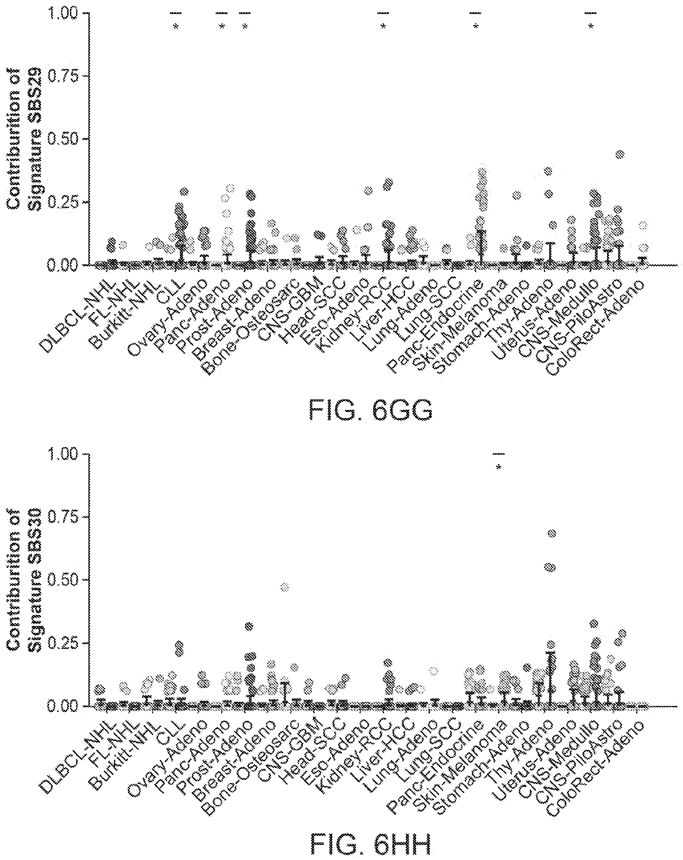

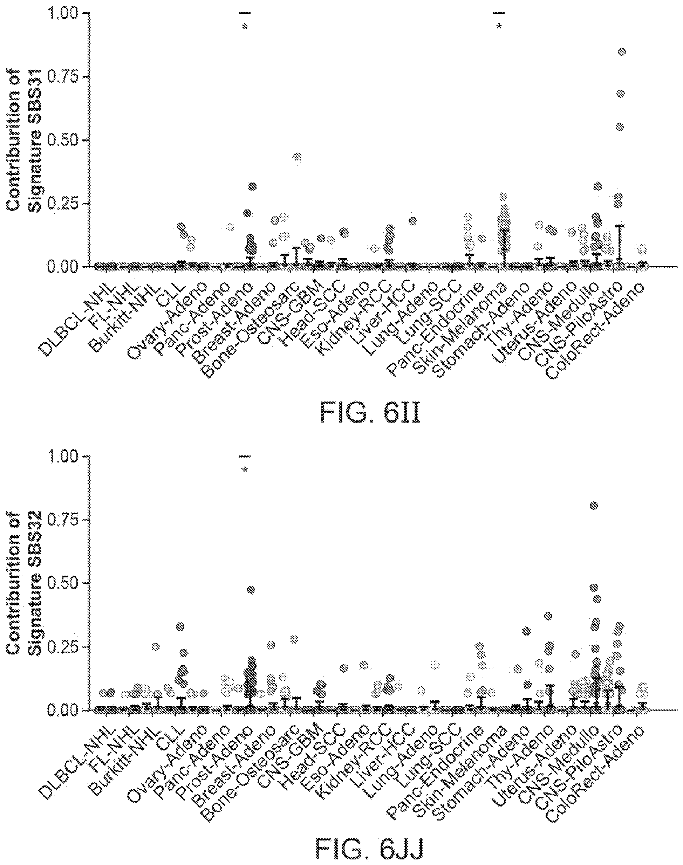

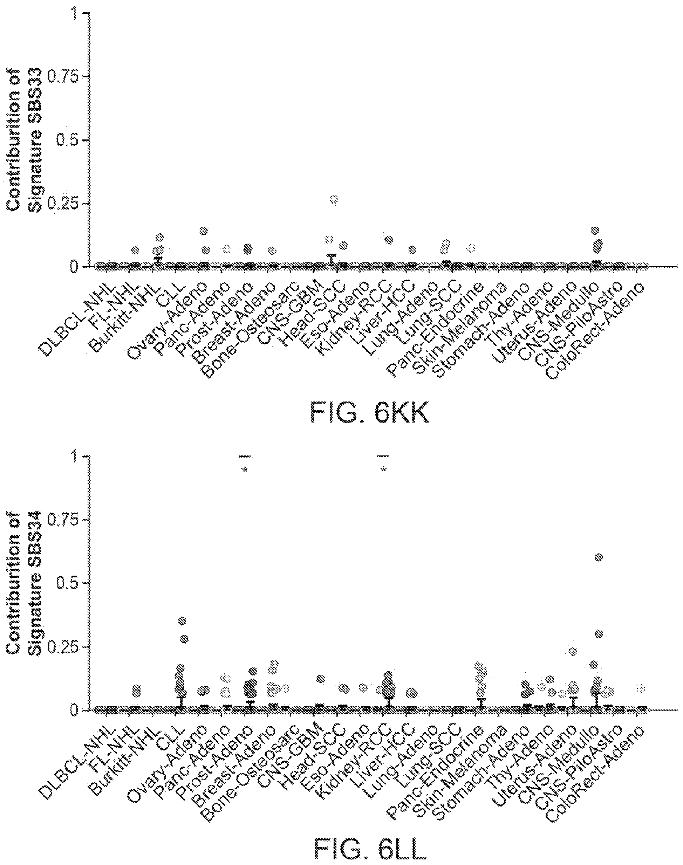

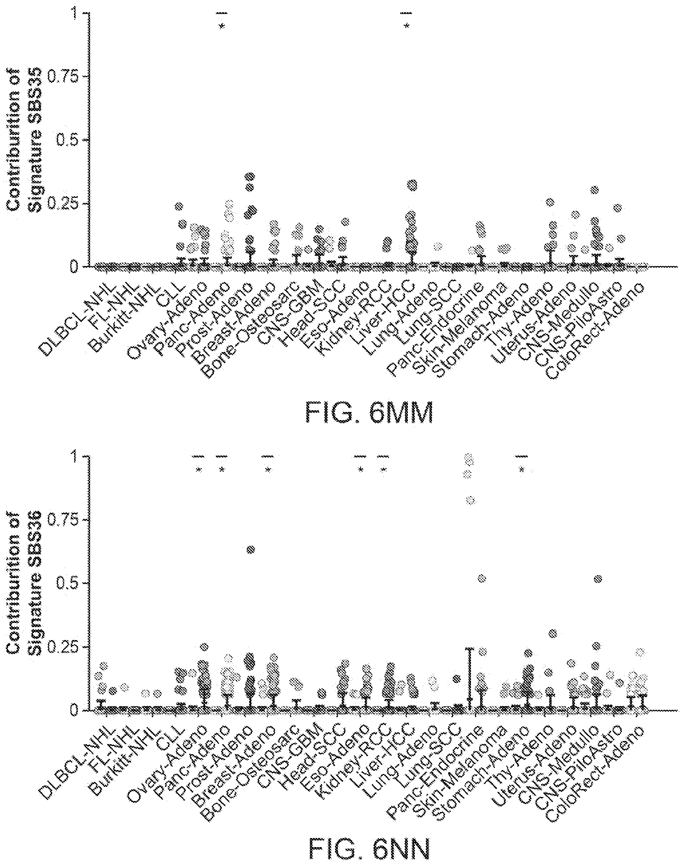

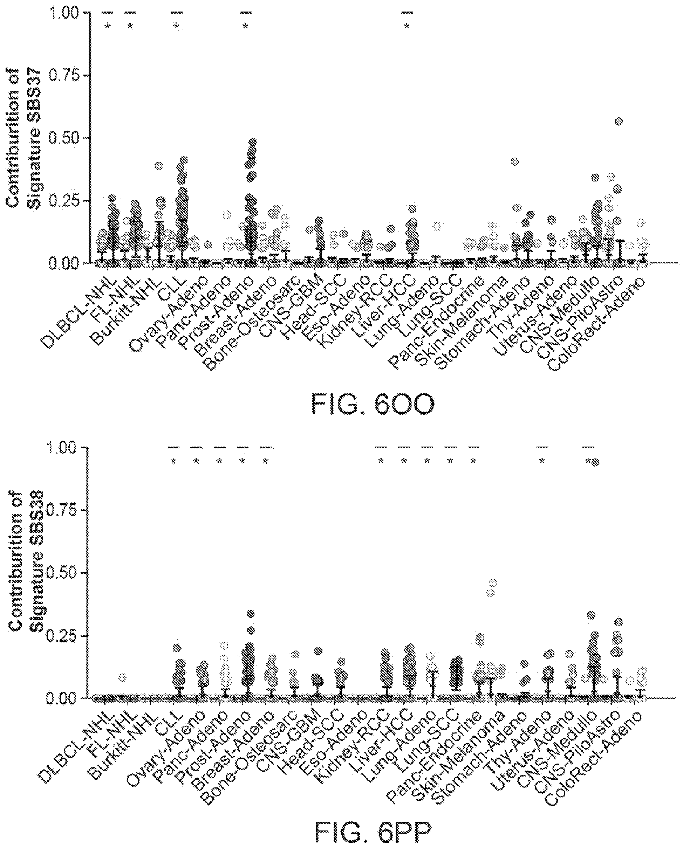

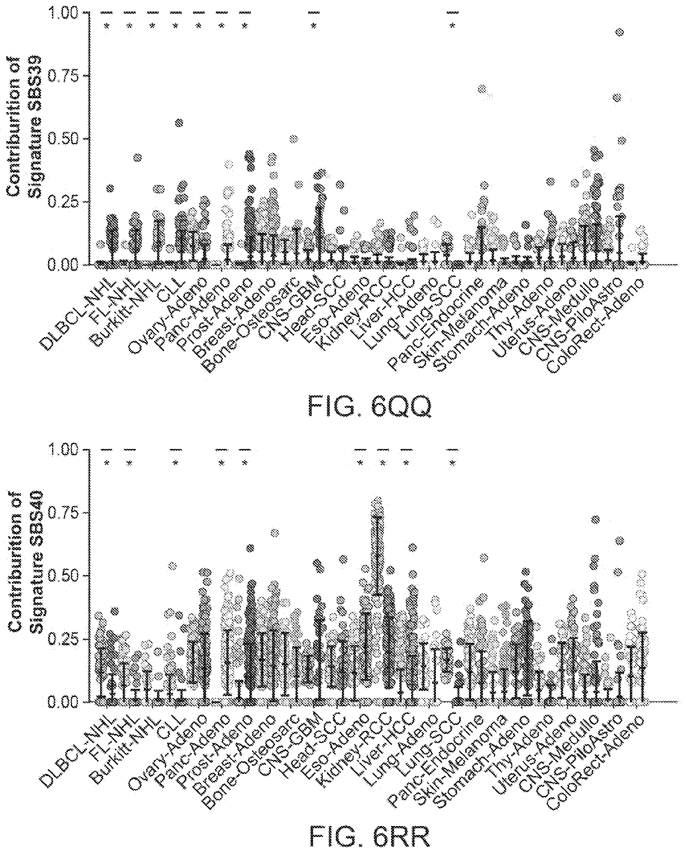

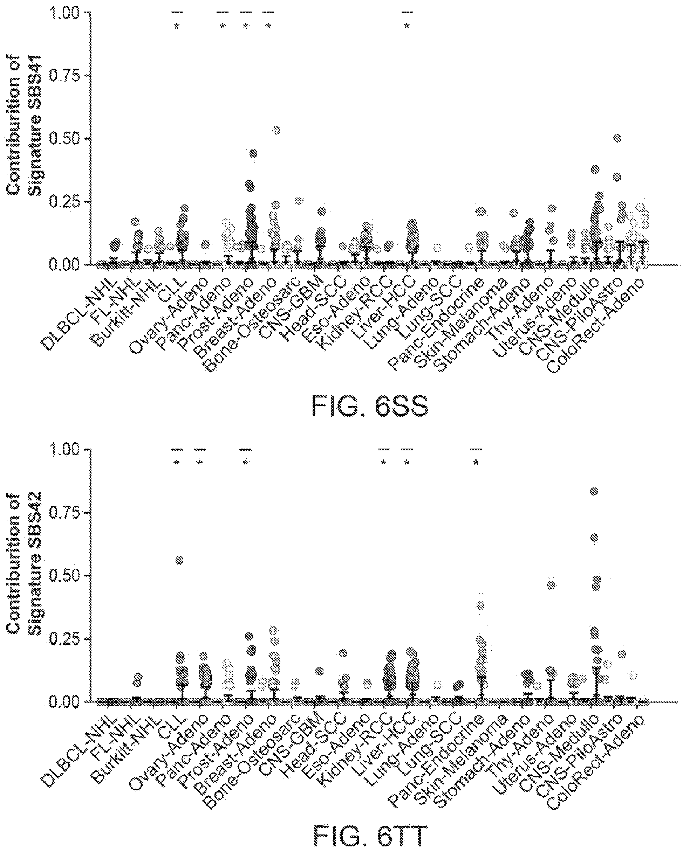

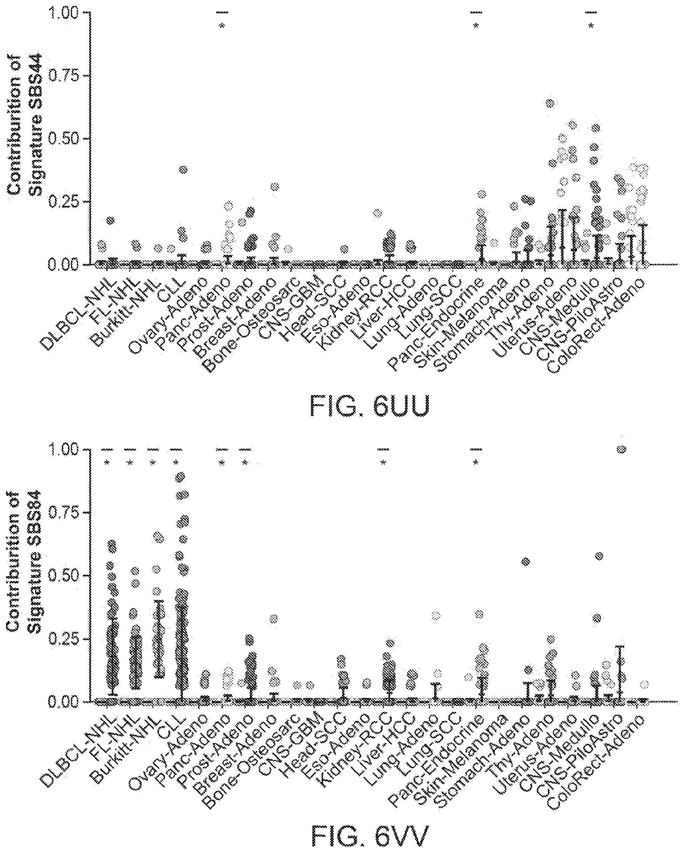

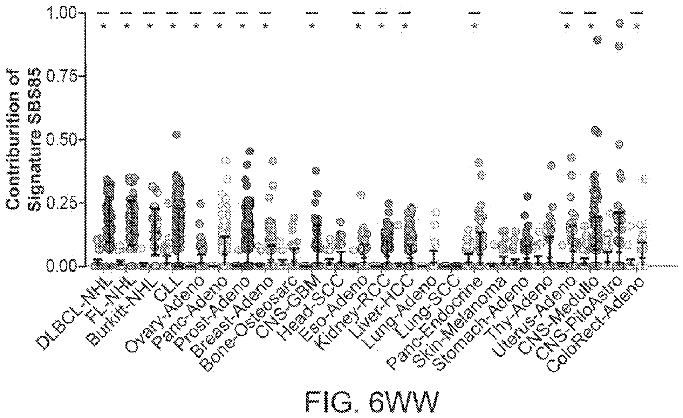

**FIGs. 6A-6WW** illustrate contribution of mutational signatures in phased and un-phased SNVs in WGS (**FIGs. 6A-6WW**.) Scatterplots showing the contribution of established single base substitution (SBS) mutational signatures to SNVs seen in PVs, shown in dark colors, and SNVs seen outside of possible phased relationships, shown in light colors, from WGS. This is presented for 49 SBS mutational signatures across 24 subtypes of cancer. Mutational signatures that show a significant difference in contribution between phased and un-phased SNVs after multiple hypothesis testing correction are indicated with a *. These figures represent the raw data summarized in FIG. 1C.

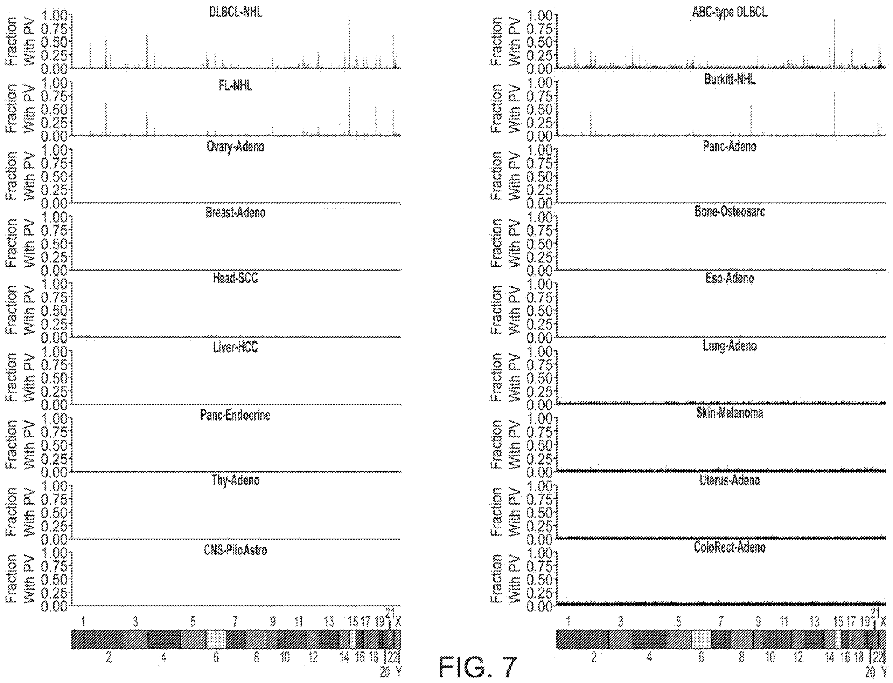

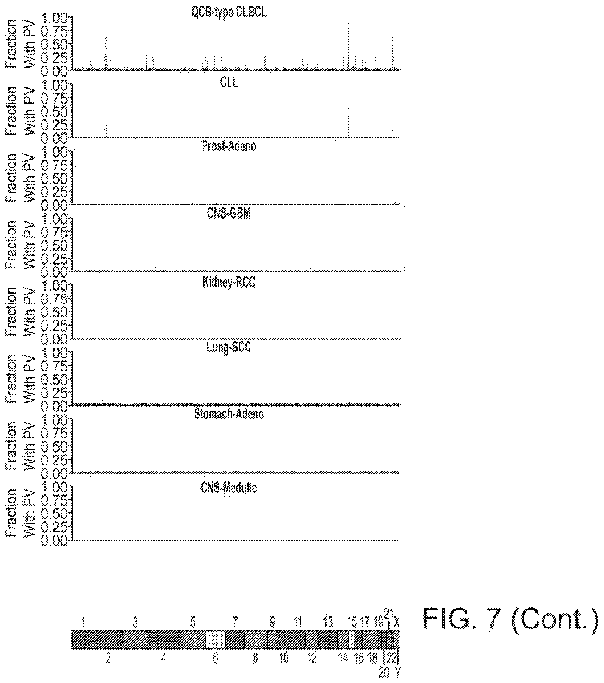

**FIG. 7** illustrates distribution of PVs in stereotyped regions across the genome. Bar plots show the distribution of PVs occurring in stereotyped regions across the genome of multiple cancer types. In this plot, the genome was divided into 1000bp bins, and the fraction of samples of a given histology with a PV in each 1000bp bin was calculated. Only bins that have at least a 2 percent recurrence frequency in any cancer subtype are shown. Histologies shown are as in **FIG. 1E;** activated B-cell (ABC) and germinal center B-cell (GCB) subtypes of DLBCL are also shown.

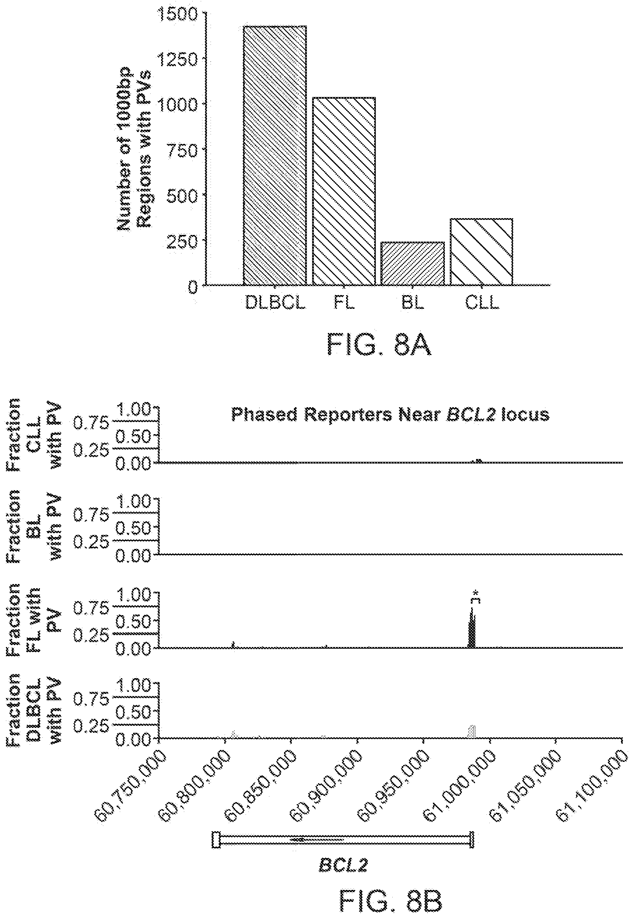

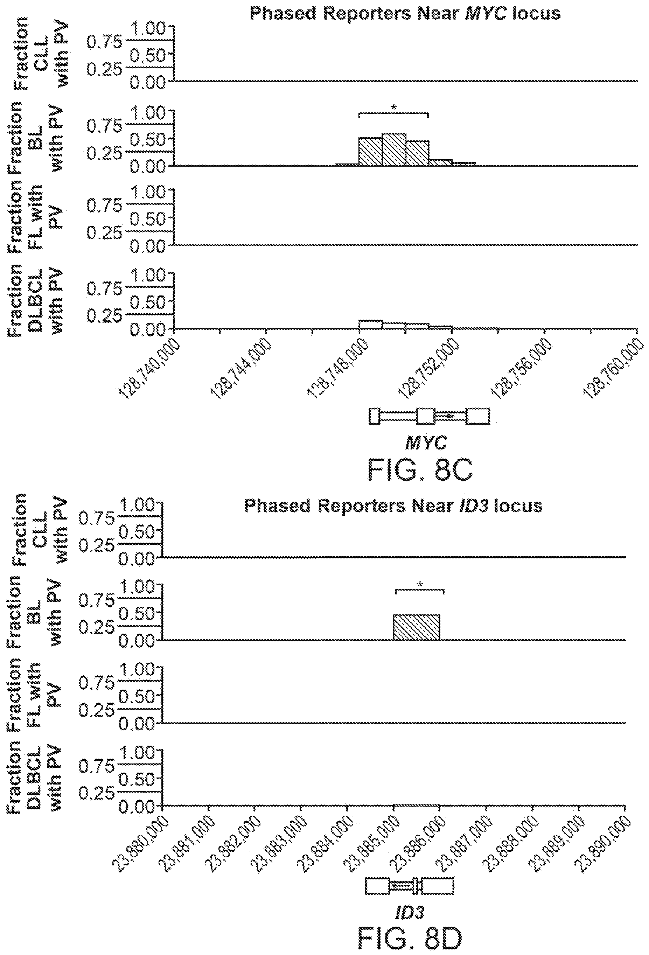

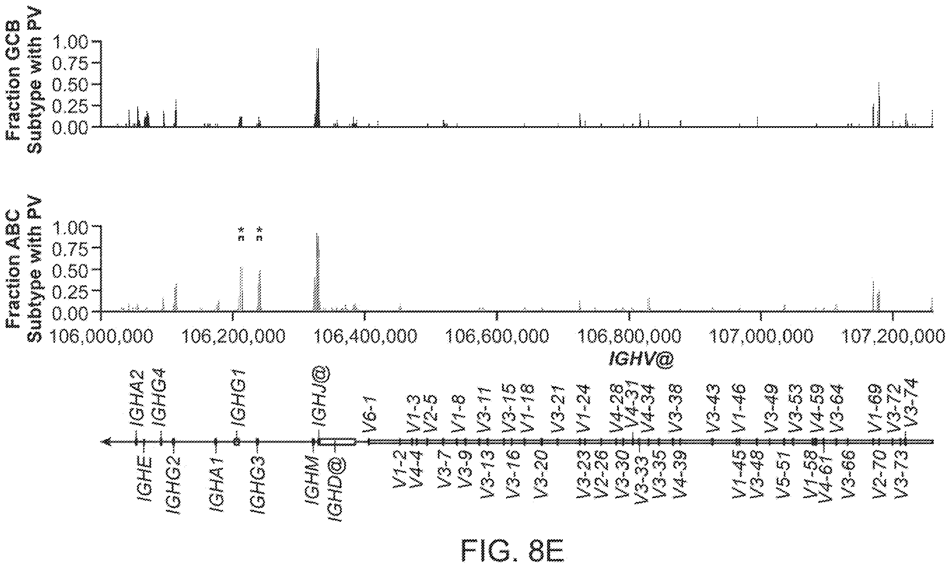

**FIGs. 8A-8E** illustrate quantity and genomic location of PVs from WGS in lymphoid malignancies. **FIG. 8A**. illustrates bar plot showing the number of independent 1000bp regions across the genome that recurrently contain PVs for DLBCL, FL, BL, and CLL (n=68, 74, 36, and 151 respectively). **FIG. 8B-D** illustrate plots showing the frequency of PVs for multiple lymphoid malignancies with relationships to specific genetic loci, including **FIG. 8B:** *BCL2,* **FIG. 8C:** *MYC,* and **FIG. 8D:** *ID3.* The location of the transcript for a given gene is shown below the plot in grey; exons are shown in darker gray. * indicates a region with significantly more PVs in a given cancer histology compared to all other histologies by Fisher's Exact Test (P<0.05). **FIG. 8E,** similar to **FIG. 8B-D,** these plots show the frequency of PVs across lymphoma subtypes. Here, it is shown the *IGH* locus, consisting of *IGHV, IGHD,* and *IGHJ* parts, for ABC and GCB subtype DLBCLs (n=25 and 25, respectively). Coding regions for Ig parts, including Ig-constant regions and V-genes, are shown. (DLBCL, diffuse large B-cell lymphoma; FL, follicular lymphoma; BL, Burkitt lymphoma, CLL, chronic lymphocytic leukemia).

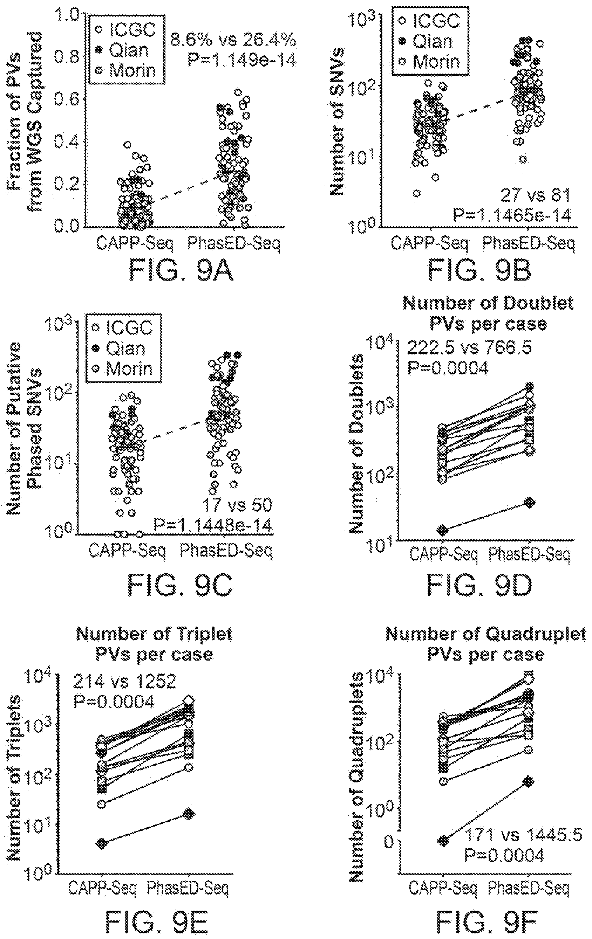

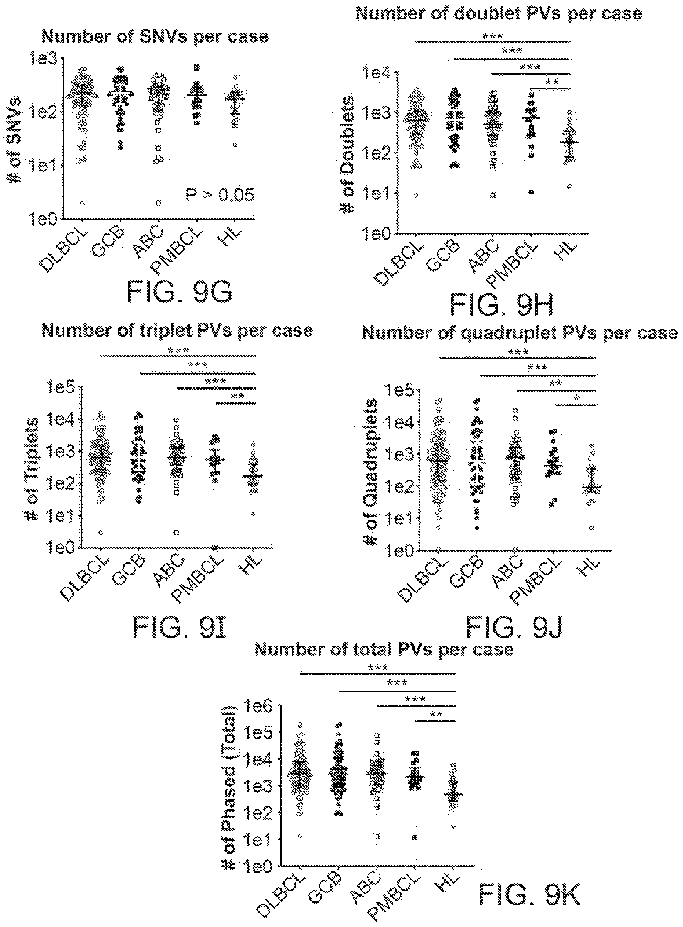

**FIGs. 9A-9K** illustrate performance of PhasED-Seq for recovery of PVs across lymphomas. **FIG. 9A** illustrates univariate scatter plot showing the fraction of all PVs across the genome identified by WGS (n=79) that were recovered by previously reported lymphoma CAPP-Seq panel[8] (left) compared to PhasED-Seq (right). **FIG. 9B** illustrates the expected yield of SNVs per case identified from WGS using a previously established lymphoma CAPP-Seq panel or the PhasED-Seq panel. **FIG. 9C** illustrates the expected yield of PVs per case identified from WGS using a previously established lymphoma CAPP-Seq panel or the PhasED-Seq panel. Data from three independent publicly available cohorts are shown in **FIGs. 9A-9C. FIGs. 9D-9F** illustrate plots showing the improvement in recovery of PVs by PhasED-Seq compared to CAPP-Seq in 16 patients sequenced by both assays. This includes improvement in d) two SNVs in phase (e.g., 2x or 'doublet PVs'), e) three SNVs in phase (3x or 'triplet PVs') and f) four SNVs in phase (e.g., 4x or 'quadruplet PVs'). **FIGs. 9G-9K.** illustrate panels showing the number of SNVs and PVs identified for patients with different types of lymphomas. These panels show the number of g) SNVs, h) doublet PVs, i) triplet PVs, j) quadruplet PVs, and k) all PVs. *, P<0.05; **, P<0.01, ***, P<0.001. (DLBCL, diffuse large B-cell lymphoma; GCB, germinal center B-cell like DLBCL; ABC, activated B-cell like DLBCL; PMBCL, primary mediastinal B-cell lymphoma; HL, Hodgkin lymphoma).

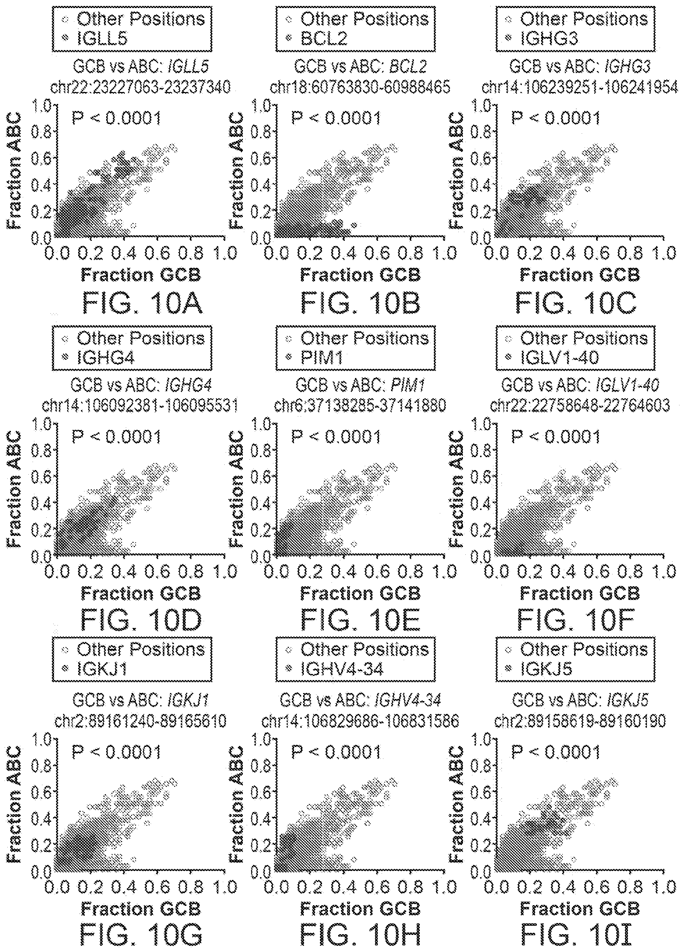

**FIGs. 10A-10Y** illustrate location-specific differences in PVs between ABC-DLBCL and GCB-DLBC (**FIGs. 10A-10Y**.) Similar to **FIG. 2D,** these scatterplots compare the frequency of PVs by genomic location (in 50bp bins) for patients with different types of lymphomas; in this figure, the difference between ABC-DLBCL and GCB-DLBCL is shown. The red circles show the relative frequency of PVs in 50bp bins from a specific gene of interest; the other (grey) circles show the relative frequency of PVs in 50bp bins from the remainder of the PhasED-Seq sequencing panel. Only genes with a statistically significant difference in PVs between ABC-DLBCL and GCB-DLBCL are shown. P-values represent a Wilcoxon rank-sum test of 50bp bins from a given gene against all other 50bp bins; see **Example 10.**

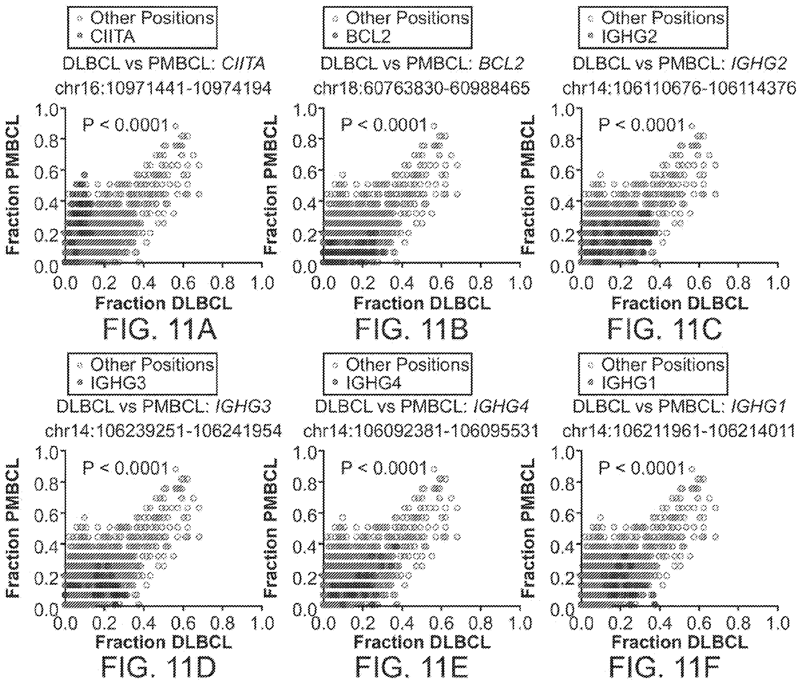

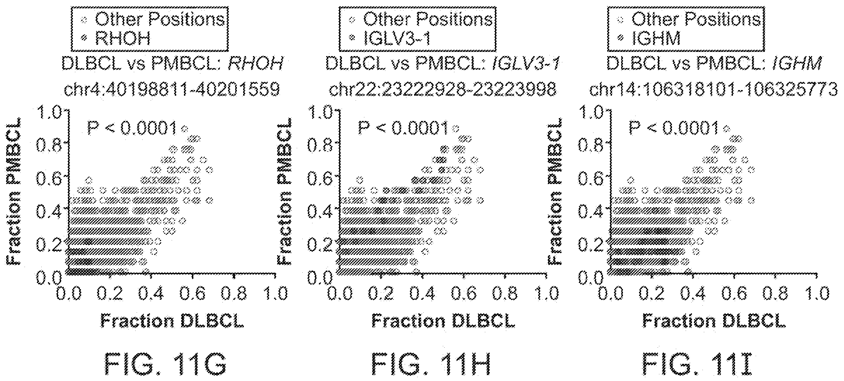

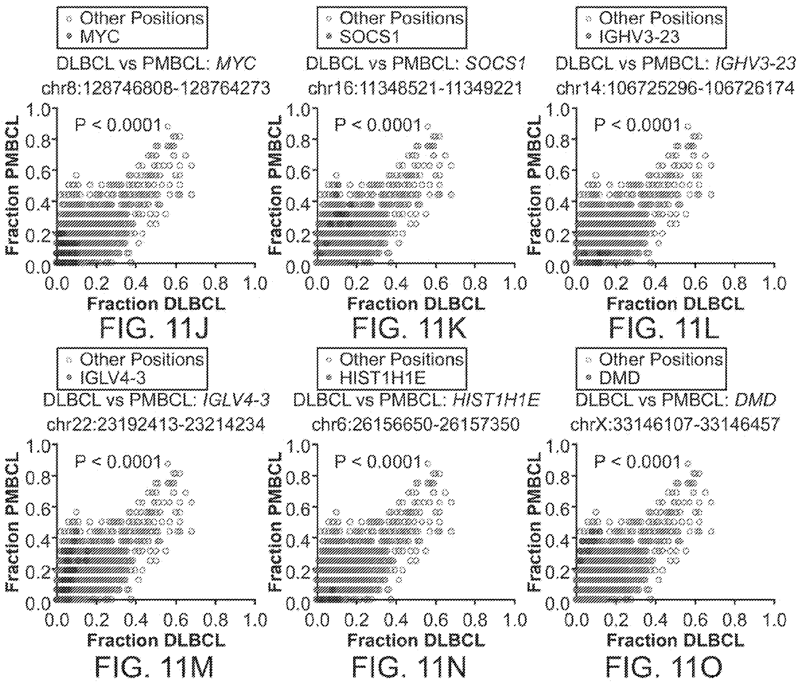

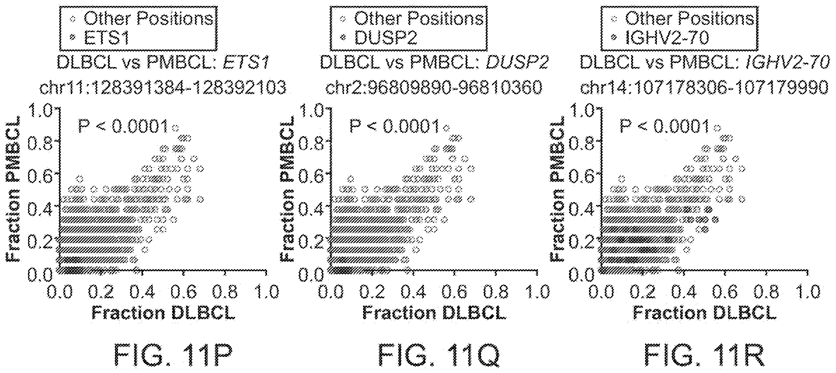

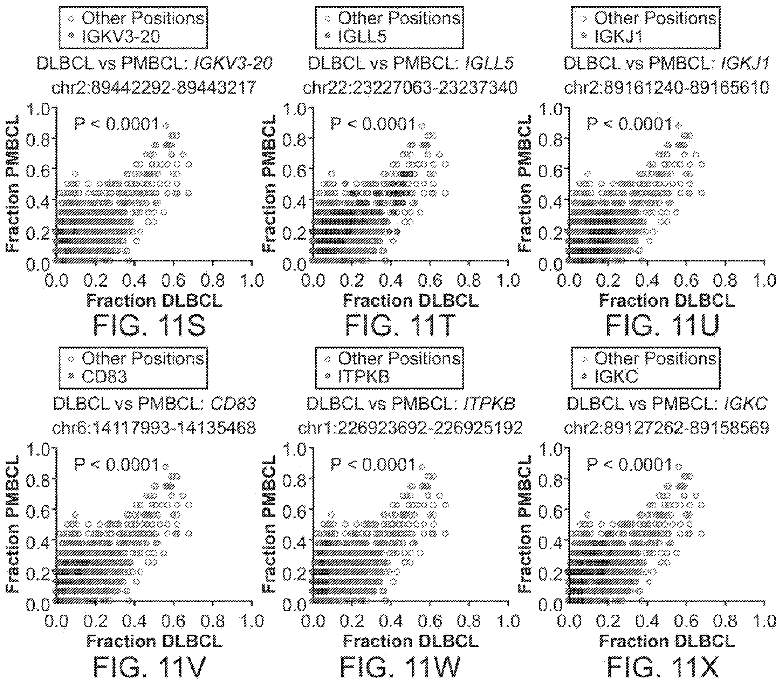

**FIGs. 11A-11X** illustrate Location-specific differences in PVs between DLBCL and PMBCL (**FIGs. 11A-11X**). Similar to **FIG. 2D,** these scatterplots compare the frequency of PVs by genomic location (in 50bp bins) for patients with different types of lymphomas; in this figure, the difference between DLBCL and PMBCL is shown. The blue circles show the relative frequency of PVs in 50bp bins from a specific gene of interest; the other (gray) circles show the relative frequency of PVs in 50bp bins from the remainder of the PhasED-Seq sequencing panel. Only genes with a statistically significant difference in PVs between DLBCL and PMBCL are shown. P-values represent a Wilcoxon rank-sum test of 50bp bins from a given gene against all other 50bp bins; see **Example 10.**

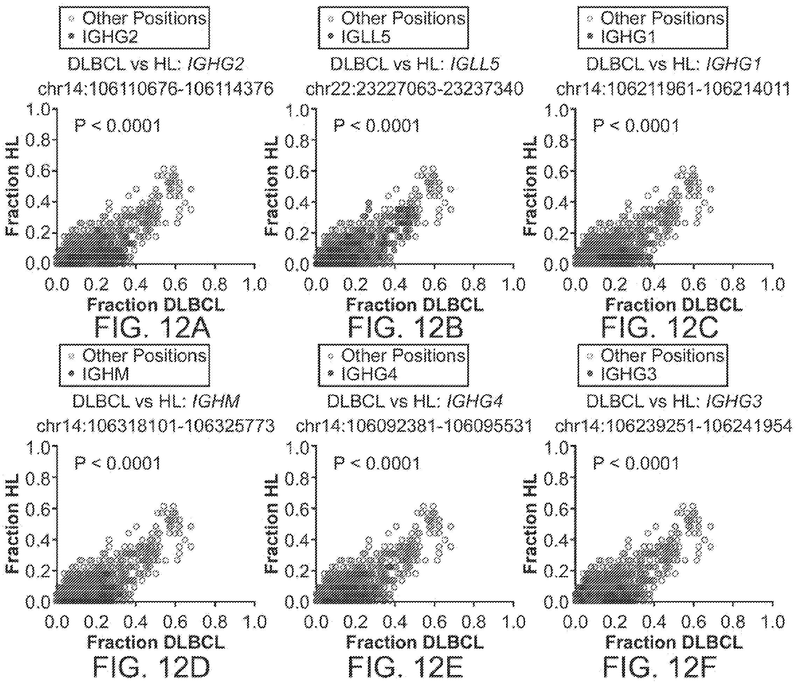

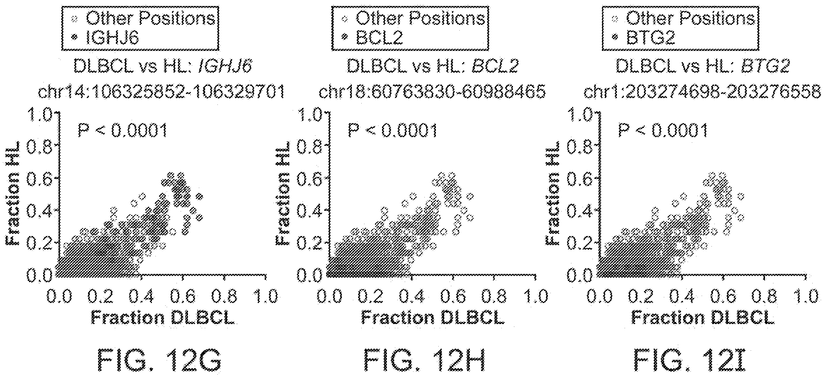

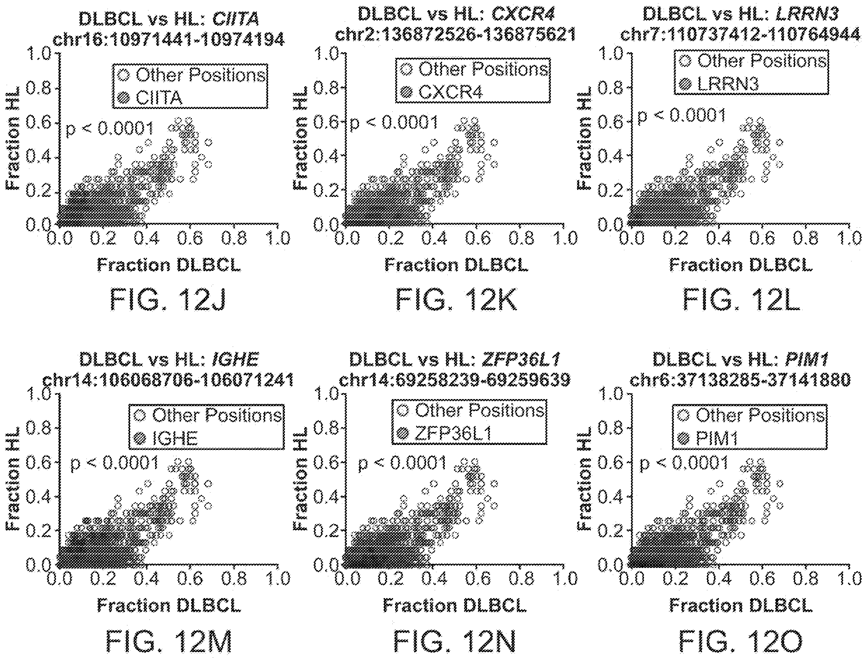

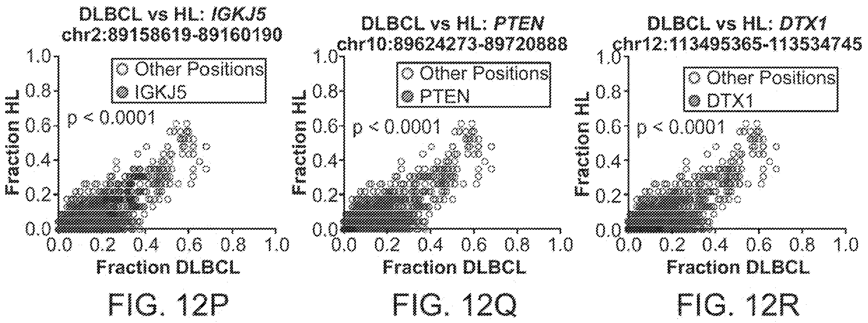

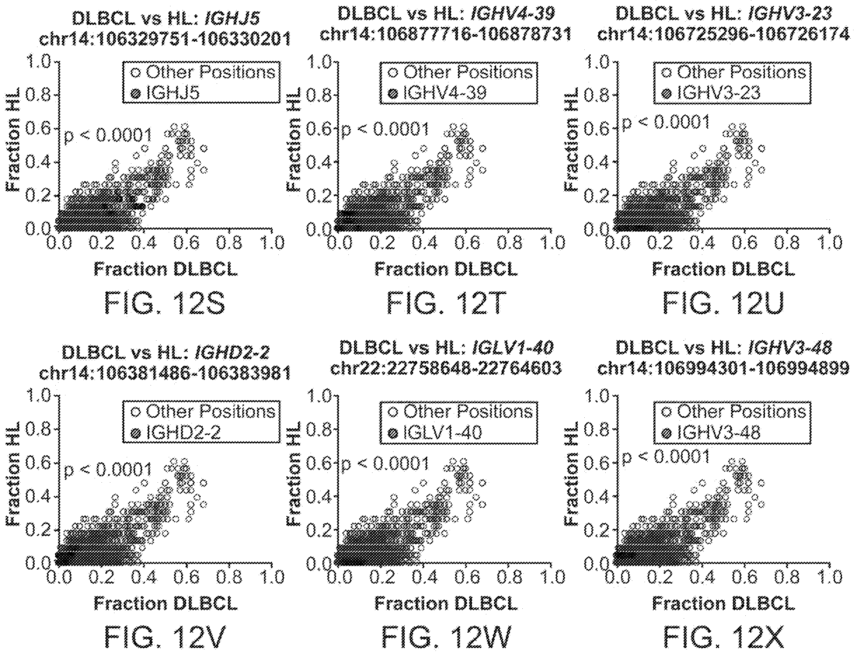

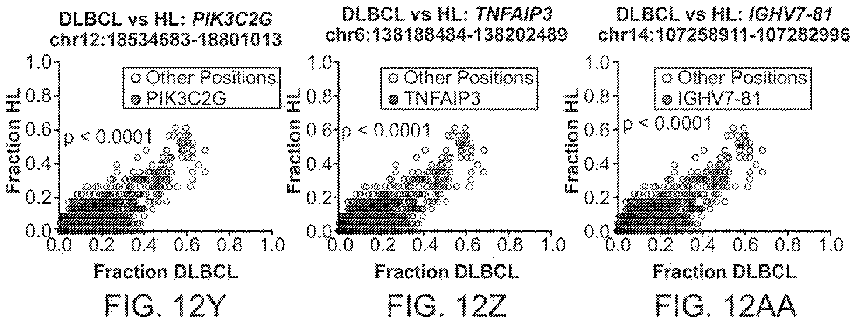

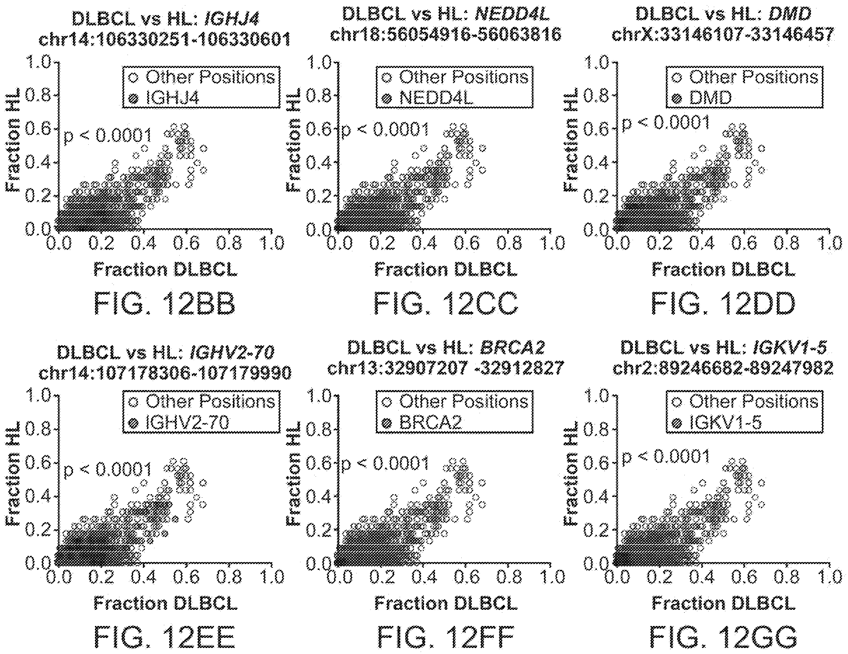

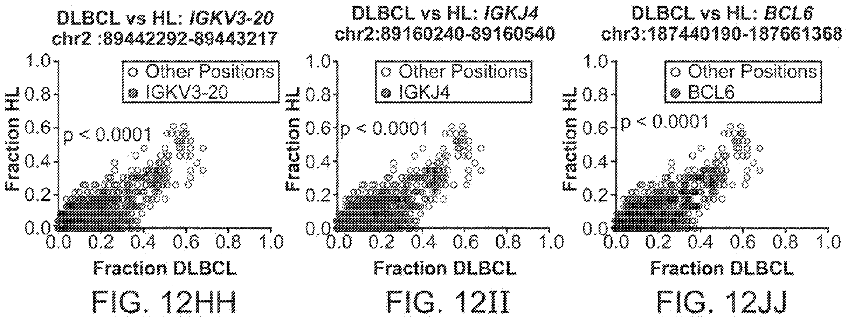

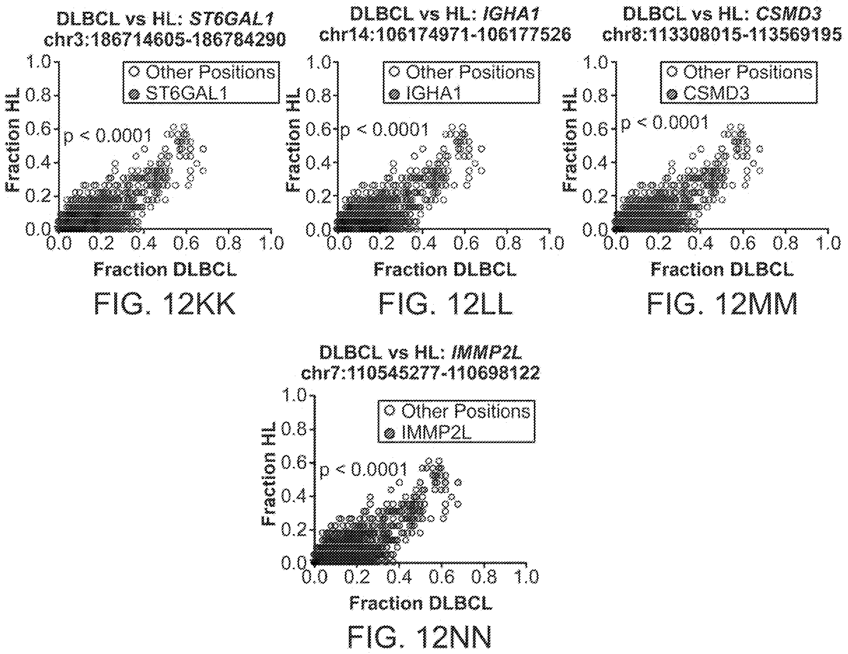

**FIGs. 12A-12NN** illustrate Location-specific differences in PVs between DLBCL and HL. Similar to **Fig. 2D,** scatterplots of **FIGs. 12A-12NN** compare the frequency of PVs by genomic location (in 50bp bins) for patients with different types of lymphomas; in this figure, the difference between DLBCL and HL is shown. The green circles show the relative frequency of PVs in 50bp bins from a specific gene of interest; the other (grey) circles show the relative frequency of PVs in 50bp bins from the remainder of the PhasED-Seq sequencing panel. Only genes with a statistically significant difference in PVs between DLBCL and HL are shown. P-values represent a Wilcoxon rank sum test of 50bp bins from a given gene against all other 50bp bins; see **Example 10.**

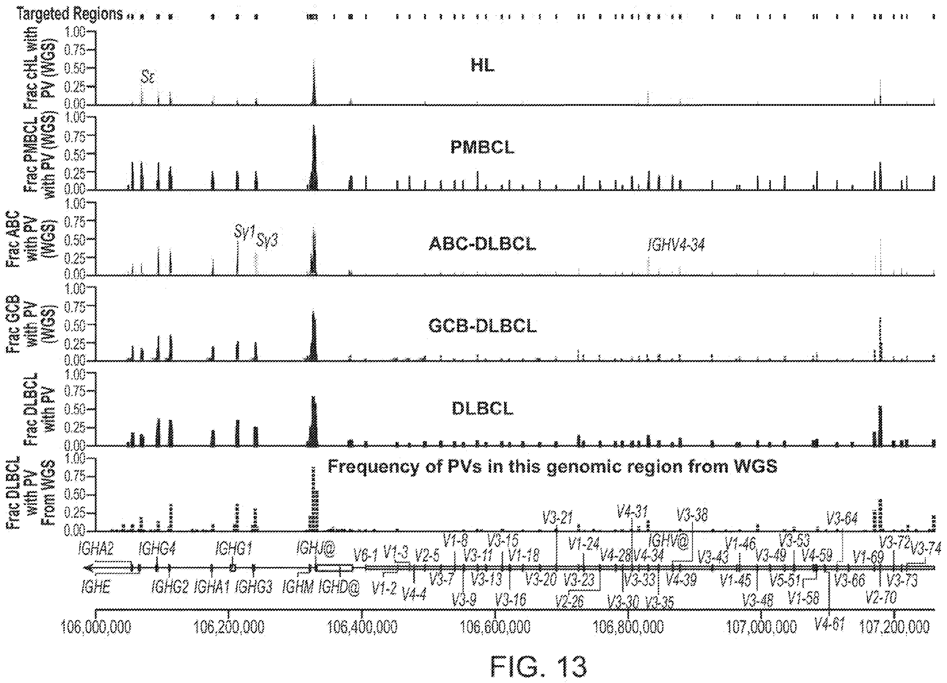

**FIG. 13** illustrates differences in PVs between lymphoma types in mutations in the *IGH* locus. This figure shows the frequency of PVs from PhasED-Seq across the @*IGH* locus for different types of B-cell lymphomas. The bottom track shows the structure of the @*IGH* locus and gene-parts, including Ig-constant genes and V-genes. The next (outlined) track shows the frequency of PVs in this genomic region from WGS data (ICGC cohort). The remainder of the tracks show the frequency of PVs from PhasED-Seq targeted sequencing data, including 1) DLBCL, GCB-DLBCL , ABC-DLBCL, PMBCL, and HL. The regions targeted by the PhasED-Seq panel are shown at the top. Selected immunoglobulin parts with PVs enriched in specific histologies are labeled (i.e., *IGHV4-34, Sε, Sγ3* and *Sγl*).

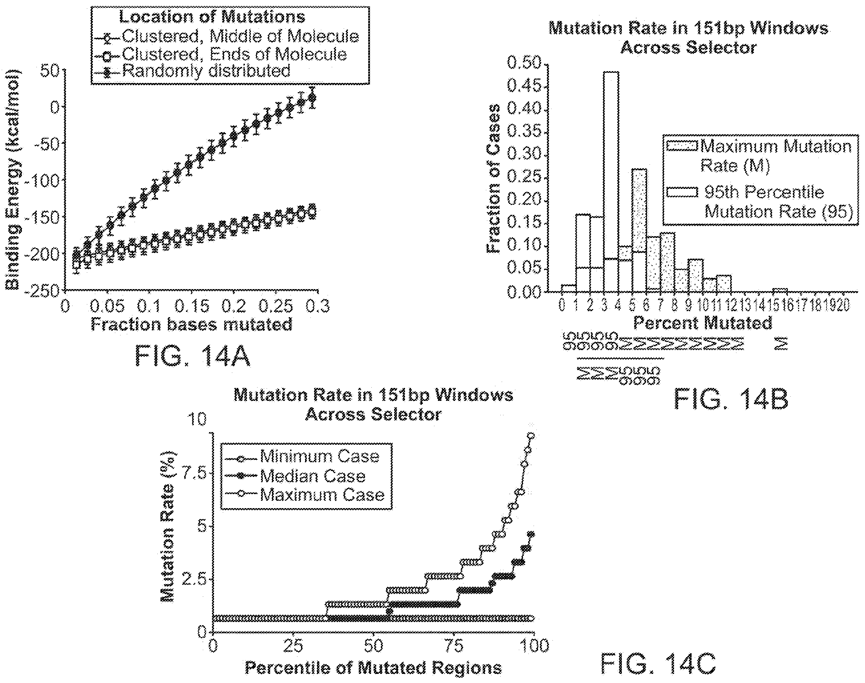

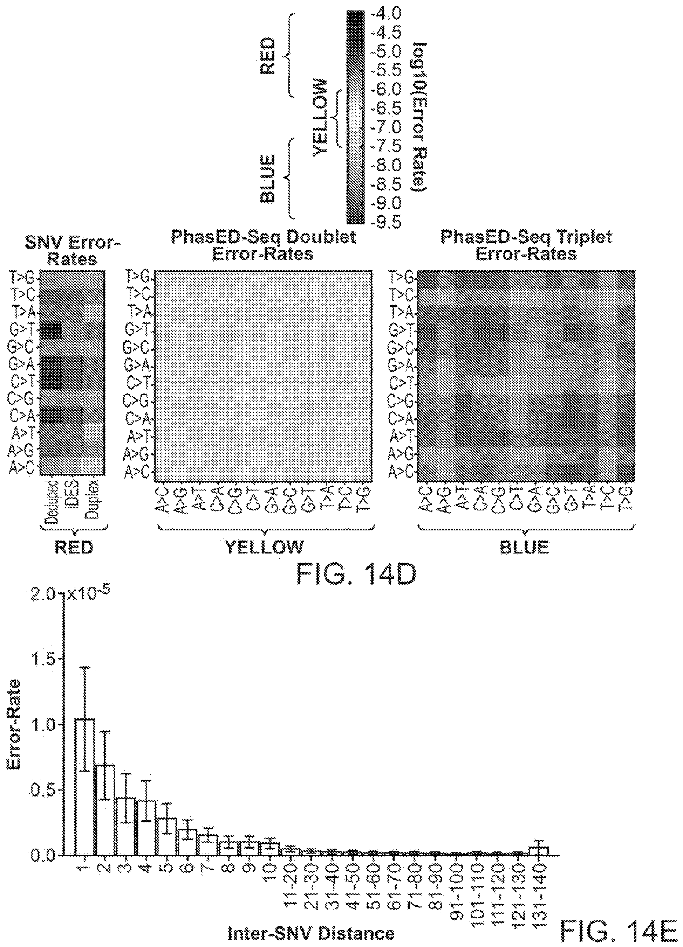

**FIGs. 14A-14E** illustrate Technical aspects of PhasED-Seq by hybrid-capture sequencing. **FIG. 14A** shows a plot of the theoretical energy of binding for typical 150-mers across the genome with increasing fraction of bases mutated from the reference genome. Mutations were spread throughout the 150-mer either clustered to one end of the sequence, clustered in the middle of the sequence, or randomly throughout the sequence. Point and error-bars represent the median and interquartile ranges from 10,000 *in silico* simulations. **FIG. 14B** illustrates a plot showing two histograms of summary metrics of the mutation rate of 151-bp windows across the PhasED-Seq panel across all patients in this study. The light gray histogram shows the maximum percent mutated in any 151-bp window for all patients in this study; the dark gray histogram shows the 95th percentile mutation rate across all mutated 151-bp windows. **FIG. 14C** is a plot showing the percentile of mutation rate across all mutated 151-bp windows across all patients in this study. **FIG. 14D** illustrates heatmaps showing the relative error rate (as log10(error rate)) for single SNVs (left, "RED"), doublet PVs (middle, "YELLOW"), and triplet PVs (right, "BLUE"). FIG. 14D demonstrates that analysis based on the plurality of phased variants (e.g., double or triplet PVs) yields a lower error rate than analysis based on single SNVs. In addition, FIG. 14D demonstrates that analysis using a higher number of phased variant sets (e.g., triplet PVs labeled as "BLUE") yields a lower error rate than analysis based on a lower number of phased variant sets (e.g., doublet PVs labeled as "YELLOW"). The error rate of single SNVs from sequencing with multiple error suppression methods is shown, including barcode deduplication, iDES, and duplex sequencing. Error rates are summarized by the type of mutation. In the case of triplet PVs, the x and y-axis of the heatmap represent the first and second type of base alteration in the PV; the third alteration is averaged over all 12 possible base changes. **FIG. 14E** illustrates a plot showing the error rate for doublet / 2x PVs as a function of the genomic distance between the component SNVs.

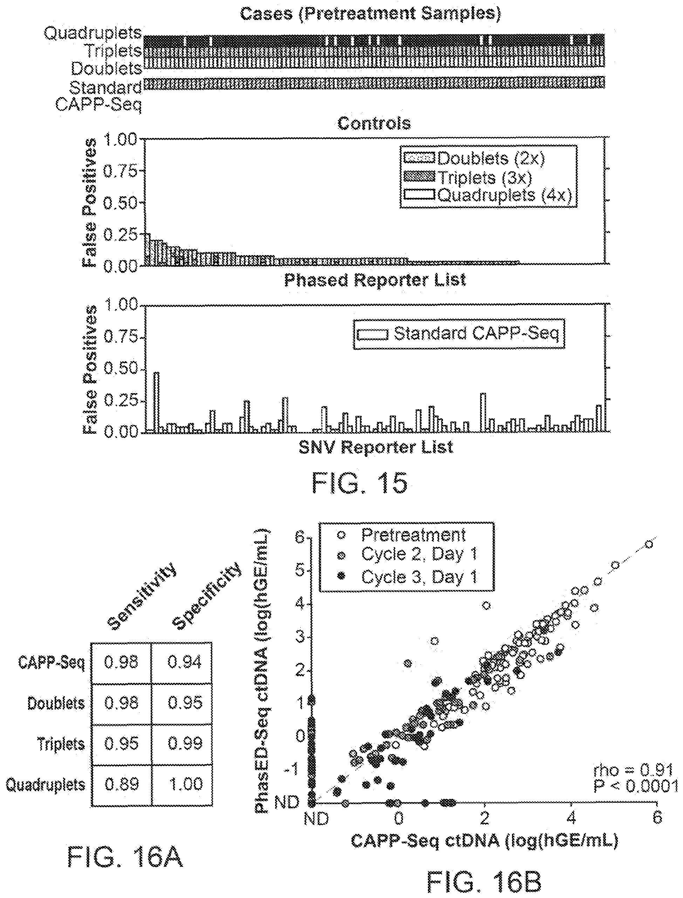

**FIGs. 15** and **16A-16B** illustrate comparison of ctDNA quantitation by PhasED-Seq to CAPP-Seq and clinical applications. **FIG. 15** illustrates the detection-rate of ctDNA from pretreatment samples across 107 patients with large-B cell lymphomas by standard CAPP-Seq (green), as well as PhasED-Seq using doublets (light blue), triplets (medium blue), and quadruplets (dark blue). The specificity of ctDNA detection is also shown. In the lower two plots, the false-detection rate in 40 withheld healthy control cfDNA samples is shown. The size of each bar in these two plots shows the detection-rate for patient-specific cfDNA mutations in these 40-withheld controls, across all 107 cases. **FIG. 16A** illustrates table summarizing the sensitivity and specificity for ctDNA detection in pretreatment samples by CAPP-Seq and PhasED-Seq using doublets, triplets, and quadruplets, shown in panel A. Sensitivity is calculated across all 107 cases, while specificity is calculated across the 40 withheld control samples, assessing for each of the 107 independent patient-specific mutation lists, for a total of 4280 independent tests. **FIG. 16B** illustrates a scatterplot showing the quantity of ctDNA (measured as log10(haploid genome equivalents/mL)) as measured by CAPP-Seq vs. PhasED-Seq in individual samples. Samples taken prior to cycle 1 of RCHOP therapy (i.e., pretreatment), prior to cycle 2, and prior to cycle 3, are shown in independent colors (blue, green, and red respectively; 278 total samples). Undetectable levels fall on the axes. Spearman correlation and P-value are shown.

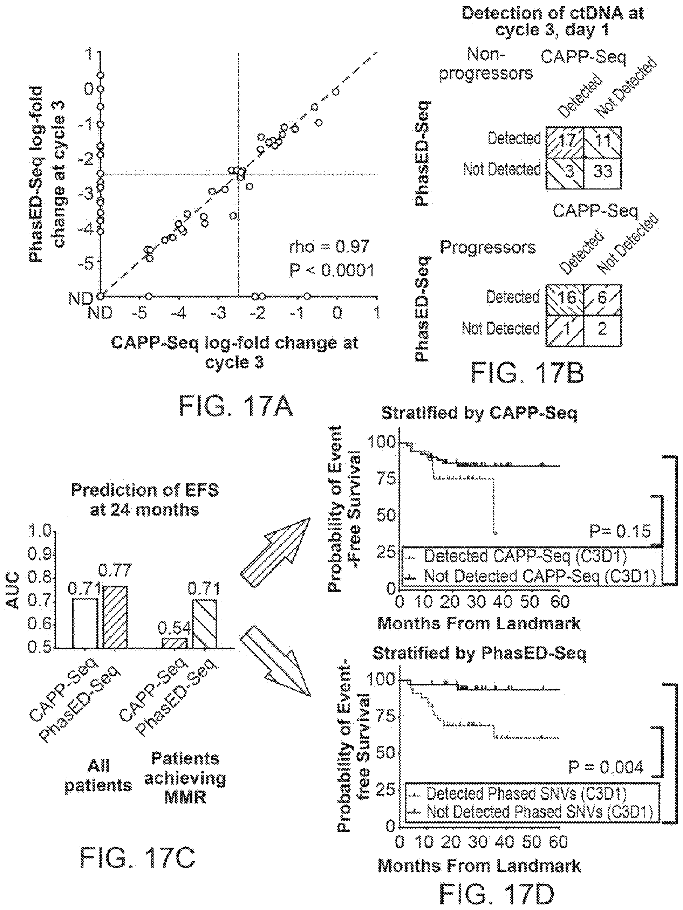

**FIGs. 17A-17D** illustrate detection of ctDNA after two cycles of systemic therapy. **FIG. 17A** illustrates a scatter plot showing the log-fold change in ctDNA after 2 cycles of therapy (i.e., the Major Molecular Response or MMR) measured by CAPP-Seq or PhasED-Seq for patients receiving RCHOP therapy. Dotted lines show the previously established threshold of a 2.5-log reduction in ctDNA for MMR. Undetectable samples fall on the axes; the correlation coefficient represents a Spearman rho for the 33 samples detected by both CAPP-Seq and PhasED-Seq. **FIG. 17B** illustrates 2 by 2 tables summarizing the detection rate of ctDNA samples after 2 cycles of therapy by PhasED-Seq vs CAPP-Seq. Patients with eventual disease progression are shown in bottom panel, while patients without eventual disease progression are shown in upper panel. **FIG. 17C** illustrates bar-plots showing the area under the receiver operator curve (AUC) for classification of patients for event-free survival at 24 months based on CAPP-Seq (light colors) or PhasED-Seq (dark colors) after 2 cycles of therapy. Classification of all patient (n=89, left) and only patients achieving a MMR (n=69, right) are both shown. **FIG. 17D** illustrates Kaplan-Meier plots showing the event-free survival of 69 patients achieving a MMR stratified by ctDNA detection with CAPP-Seq (top) or PhasED-Seq (bottom).

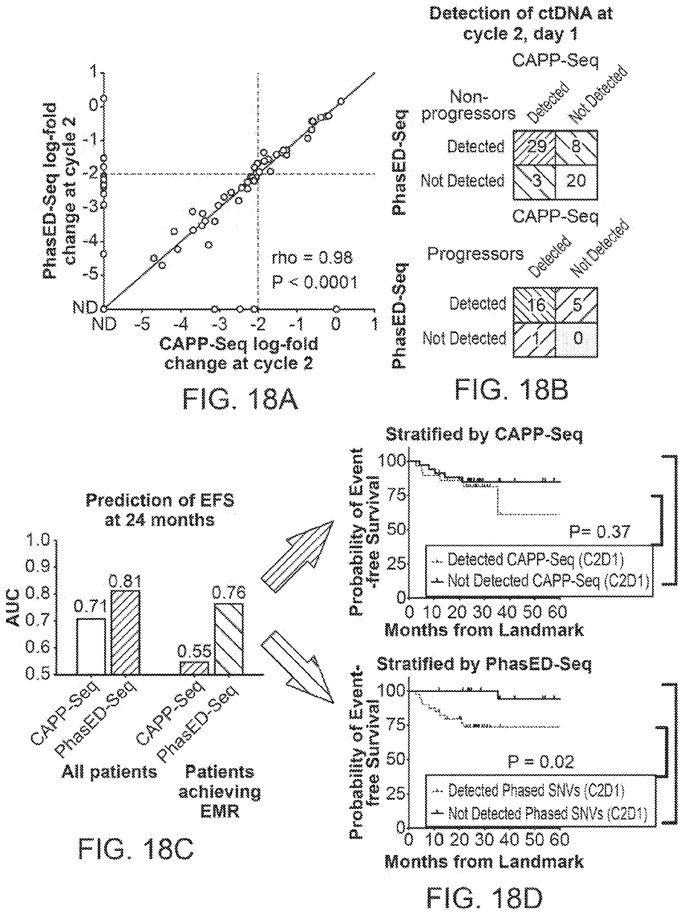

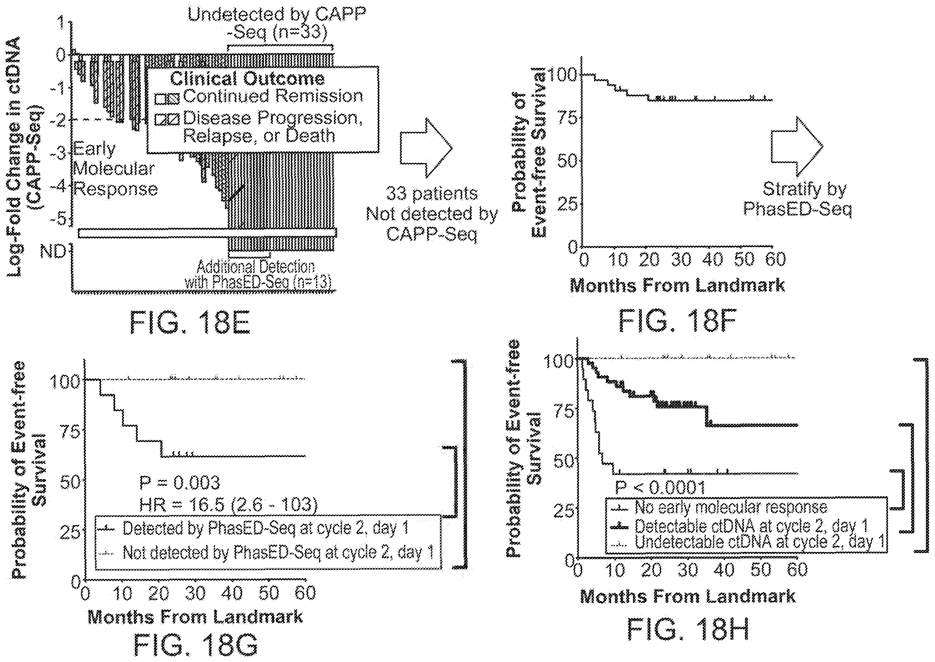

**FIGs. 18A-18H** illustrate detection of ctDNA after one cycle of systemic therapy. **FIG. 18A** illustrates scatterplot showing the log-fold change in ctDNA after 1 cycle of therapy (i.e., the Early Molecular Response or EMR) measured by CAPP-Seq or PhasED-Seq for patients receiving RCHOP therapy. Dotted lines show the previously established threshold of a 2-log reduction in ctDNA for EMR. Undetectable samples fall on the axes; the correlation coefficient represents a Spearman rho for the 45 samples detected by both CAPP-Seq and PhasED-Seq. **FIG. 18B** illustrates 2 by 2 tables summarizing the detection rate of ctDNA samples after 1 cycle of therapy by PhasED-Seq vs CAPP-Ceq. Patients with eventual disease progression are shown in red, while patients without eventual disease progression are shown in blue. **FIG. 18C** illustrates bar-plots showing the area under the receiver operator curve (AUC) for classification of patients for event-free survival at 24 months based on CAPP-Seq (light colors) or PhasED-Seq (dark colors) after 1 cycle of therapy. Classification of all patient (n=82, left) and only patients achieving an EMR (n=63, right) are both shown. **FIG. 18D** illustrates Kaplan-Meier plots showing the event-free survival of 63 patients achieving an EMR stratified by ctDNA detection with CAPP-Seq (top) or PhasED-Seq (bottom). **FIG. 18E** illustrates waterfall plot showing the change in ctDNA levels measured by CAPP-Seq after 1 cycle of first-line therapy in patients with DLBCL. Patients with undetectable ctDNA by CAPP-Seq are shown as "ND" ("not detected"), in darker colors. The colors of the bars also indicate the eventual clinical outcomes for these patients. **FIG. 18F** illustrates a Kaplan-Meier plot showing the event-free survival for 33 DLBCL patients with undetectable ctDNA measured by CAPP-Seq after 1 cycle of therapy. **FIG. 18G** illustrates a Kaplan-Meier plot showing the event-free survival of 33 patients shown in **FIG. 18F** (undetectable ctDNA by CAPP-Seq) stratified by ctDNA detection via PhasED-Seq at this same time-point (cycle 2, day 1). **FIG. 18H** illustrates a Kaplan-Meier plot showing the event-free survival for 82 patients with DLBCL stratified by ctDNA at cycle 2, day 1 separated into 3 strata - patients failing to achieve an early molecular response, patients with an early molecular response who still have detectable ctDNA by PhasED-Seq and/or CAPP-Seq, and patients who have a stringent molecular remission (undetectable ctDNA by PhasED-Seq and CAPP-Seq;).

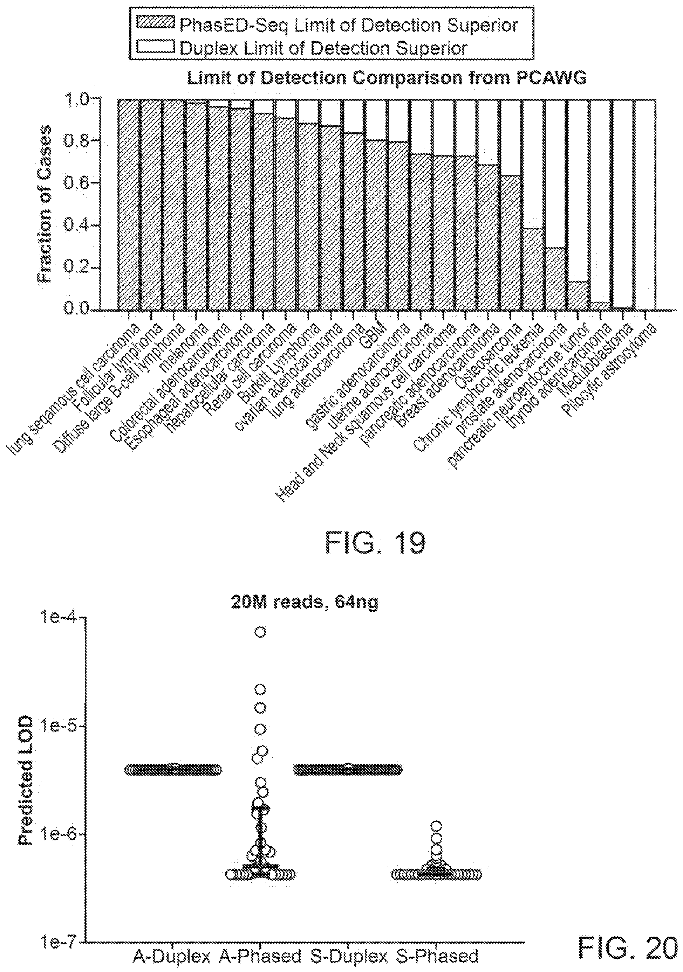

**FIG. 19** illustrates a fraction of patients where PhasED-Seq would achieve a lower LOD than duplex sequencing tracking SNVs based on PCAWG data (whole genome sequencing) from which the number of SNVs and phased variants (PVs) in different tumor types was quantified.

**FIG. 20** illustrates improved LODs achieved in lung cancers (adenocarcinoma, abbreviated 'A', and squamous cell carcinoma, abbreviated 'S'), compared to duplex sequencing of whole genome sequencing data.

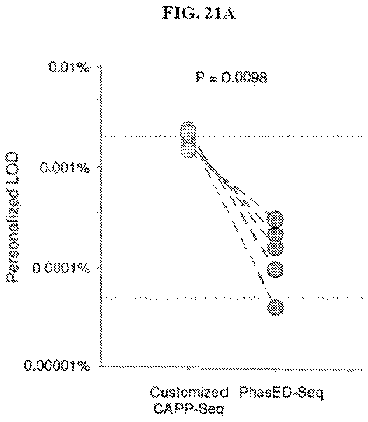

**FIG. 21A** illustrates empiric data from an experiment where WGS was performed on tumor tissue and custom panels were designed for 5 patients with solid tumors (5 lung cancers) to examine and compare the LODs of custom CAPP-Seq vs PhasED-Seq, showing a ~10X lower LOD using PhasED-Seq in 5/5 patients.

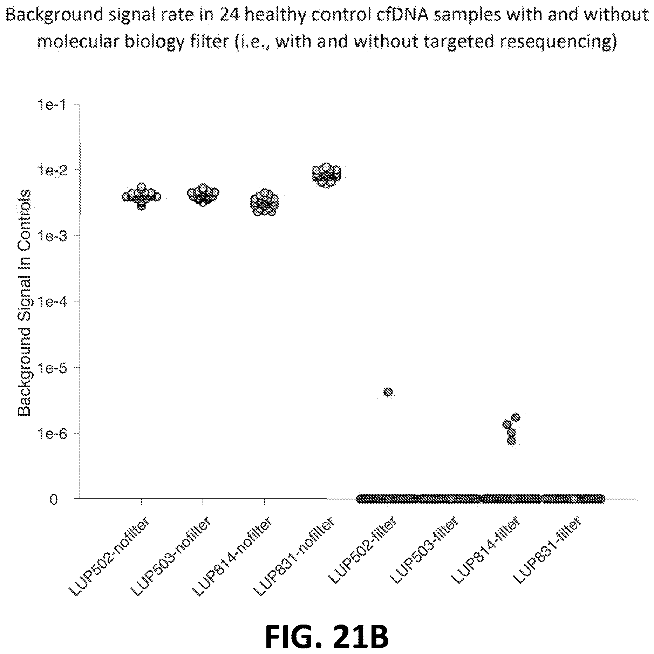

**FIG. 21B** shows the background signal for detecting patient and tumor-specific DNA in control cell-free DNA samples with and without a reanalysis involving targeted resequencing of the original patient's tumor and germline DNA.

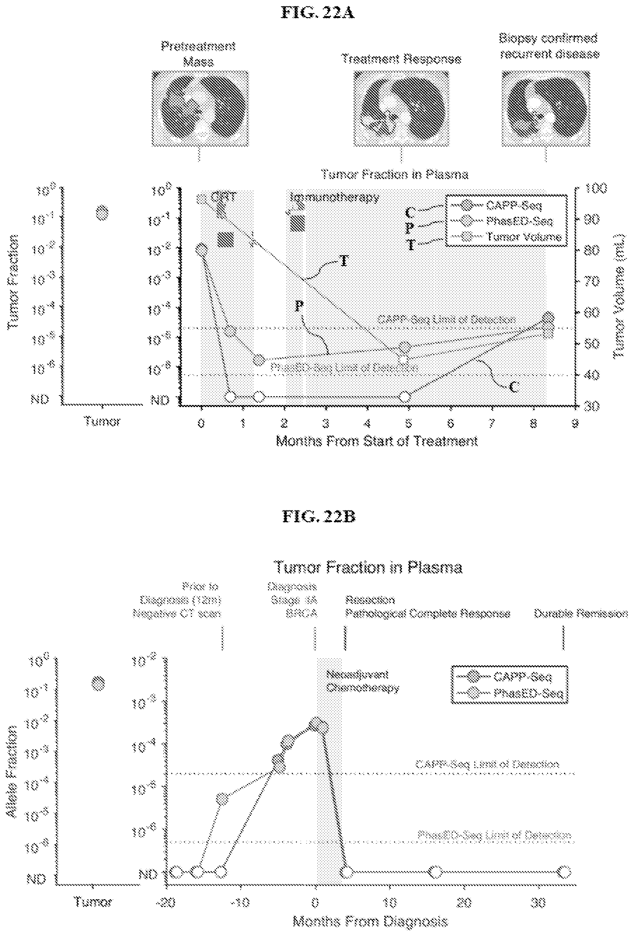

**FIG. 22A** illustrates proof of principle example patient vignette comparing using custom CAPP-Seq and PhasED-Seq for disease surveillance in lung cancer showing earlier detection of relapse using PhasED-Seq.

**FIG. 22B** illustrates proof of principle example patient vignette comparing using custom CAPP-Seq and PhasED-Seq for early detection of disease in breast cancer, showing earlier detection of disease with PhasED-Seq.

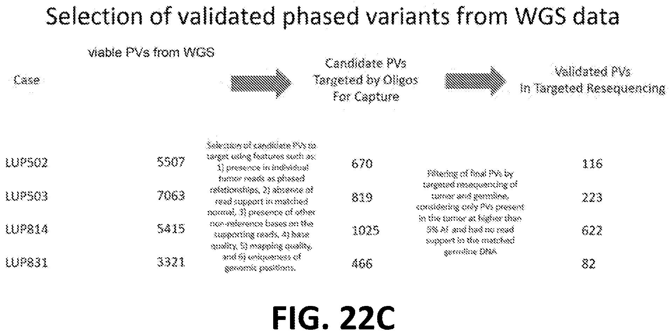

**FIG. 22C** shows a process for selection of validated phased variants from whole genome sequencing data.

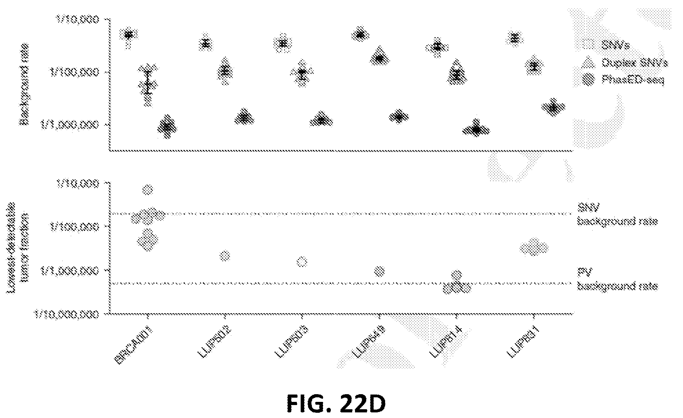

**FIG. 22D** shows the performance metrics of personalized PhasED-seq across six patients, including background signal and limit of detection. Top, the background rate of SNVs (squares), duplex SNVs (triangles) or PVs (circles); bars represent the median and IQR. Bottom, the lowest detectable tumor fraction for each sample. The background rate for SNVs is shown at $2 \times 10^{-5}$ and for PVs at $5 \times 10^{-7}$.

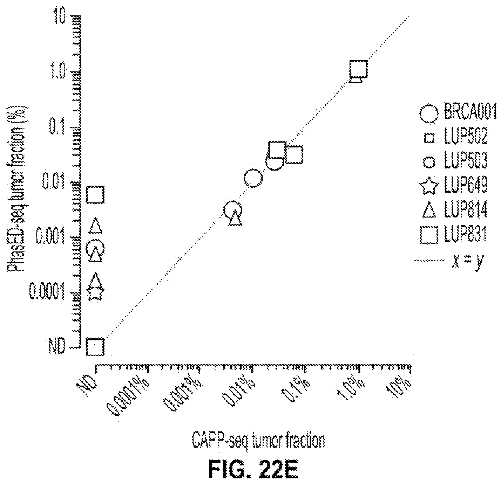

**FIG. 22E** shows a comparison between the recovered tumor fraction by CAPP-seq (x axis) and PhasED-seq (y axis) for all samples from the six patients with solid tumors.

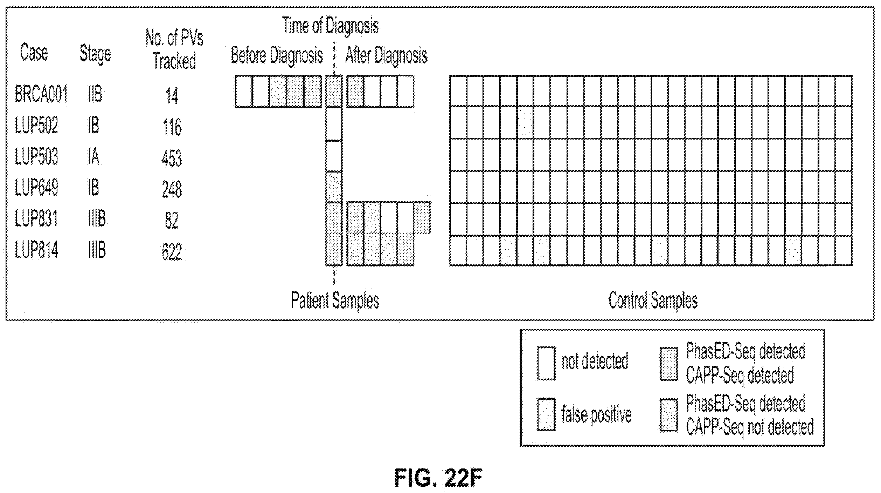

**FIG. 22F** shows detection of ctDNA for 6 cases of patients with solid tumors, including lung cancer (n = 5) and breast cancer (n = 1) using SNV-based detection (that is, CAPP-Seq) or PhasED-Seq with a personalized panel. Detection of ctDNA in patient plasma samples are shown in blue; samples detectable with PhasED-Seq but not SNV based approaches are in light blue. Specificity of the assay was assessed using 24 healthy control samples; detection of evidence of ctDNA by PhasED-Seq in these are shown on the right in pink across all 6 personalized panels, indicating 97% (139/144) specificity; CAPP-Seq on the same samples showed 95% (137/144) specificity.

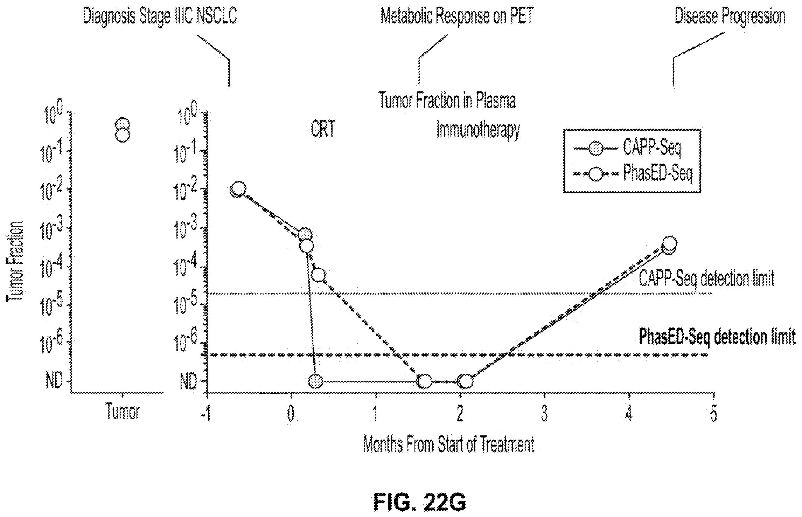

**FIG. 22G** shows the ctDNA profile of a patient with stage 3 lung adenocarcinoma (LUP831) undergoing combined chemo-radiotherapy (CRT) and immunotherapy, measured by both CAPP-Seq and PhasED-Seq. The left panel shows the measured tumor fraction in the tumor biopsy sample using both methods. The right panel shows the tumor fraction from plasma DNA, including a sample detected by PhasED-Seq that is undetected by CAPP-Seq. ND: not detected.

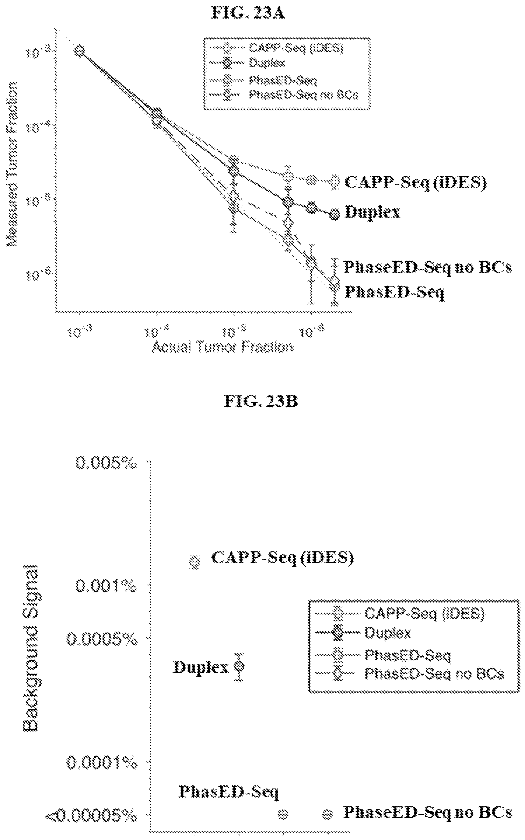

**FIGs. 23A-23B** illustrate that detection methods describe herein (e.g. method depicted yielding **FIG. 3E** and **FIG. 3F**) does not require barcode meditated error suppression.

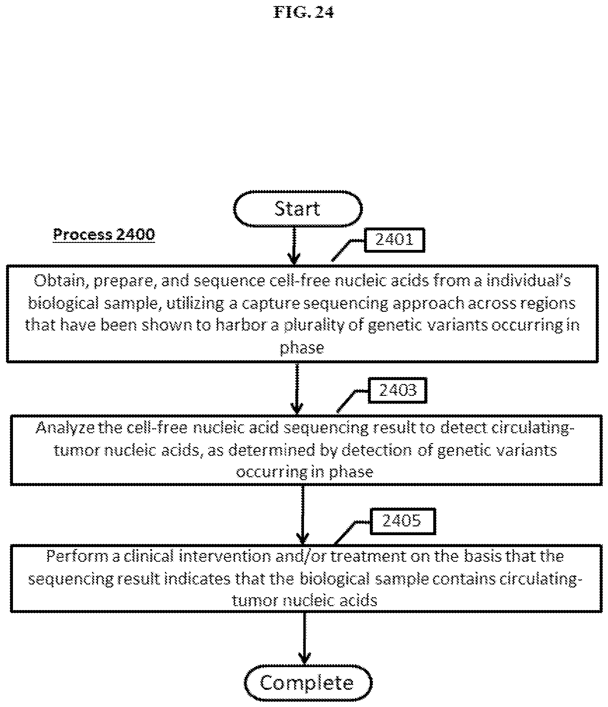

**FIG. 24** illustrates a flow diagram of a process to perform a clinical intervention and/or treatment on an individual based on detecting circulating-tumor nucleic acid sequences in a sequencing result in accordance with an embodiment.

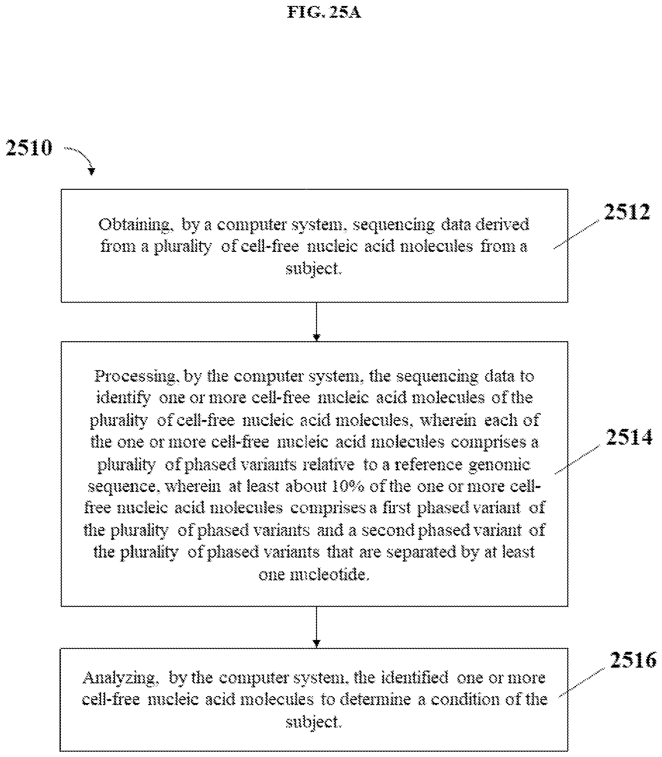

**FIGs. 25A-25C** show example flowcharts of methods for determining a condition of a subject based on one or more cell-free nucleic acid molecules comprising a plurality of variants.

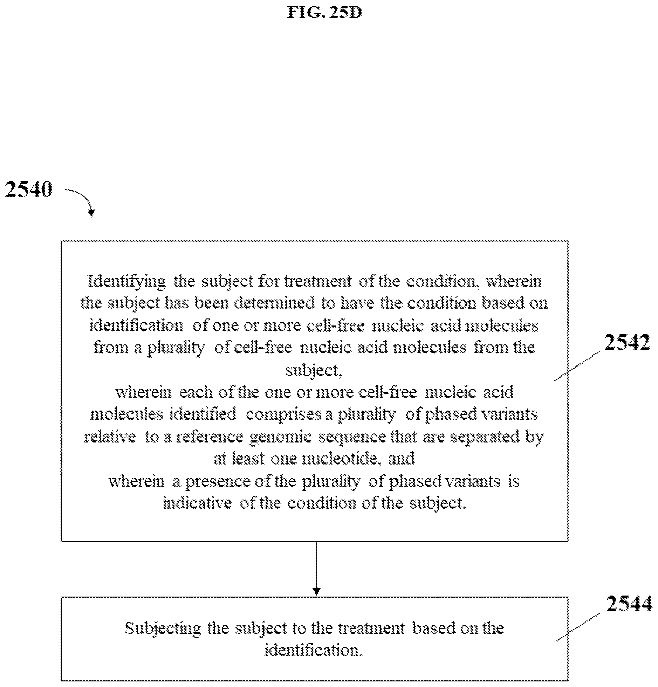

**FIG. 25D** shows an example flowchart of a method for treating a condition of a subject based on one or more cell-free nucleic acid molecules comprising a plurality of variants.

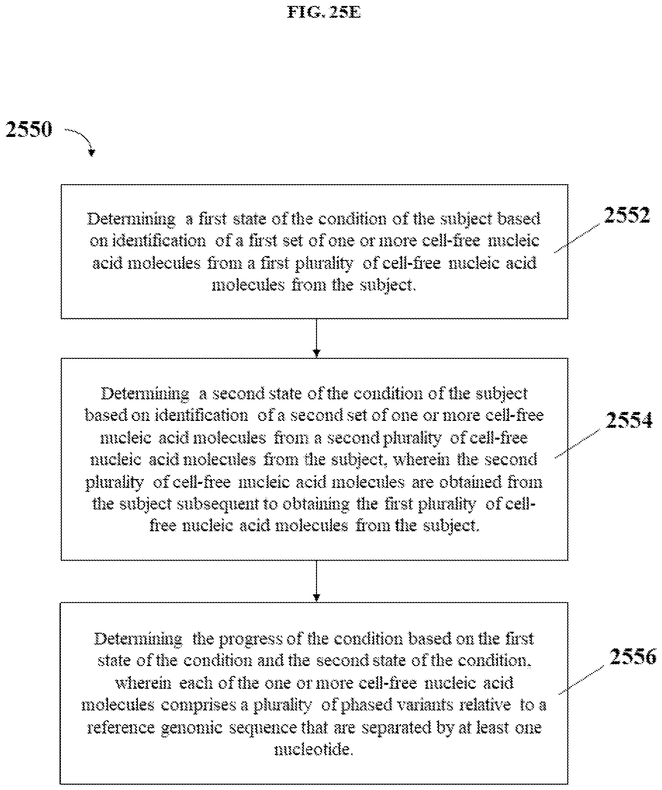

**FIG. 25E** shows an example flowchart of a method for determining a progress (e.g., progression or regression) of a condition of a subject based on one or more cell-free nucleic acid molecules comprising a plurality of variants.

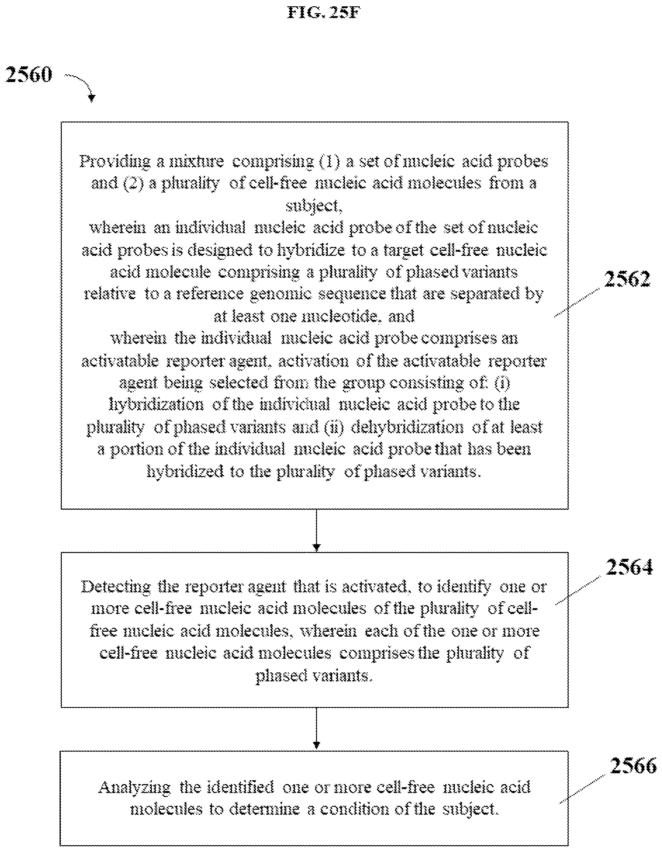

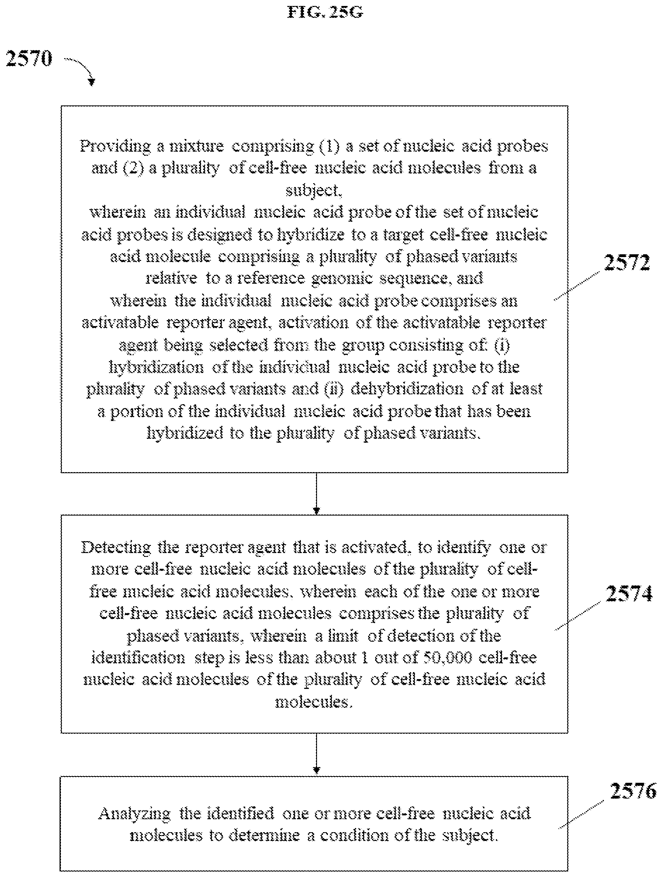

**FIGs. 25F** and **25G** show example flowcharts of methods for determining a condition of a subject based on one or more cell-free nucleic acid molecules comprising a plurality of variants.

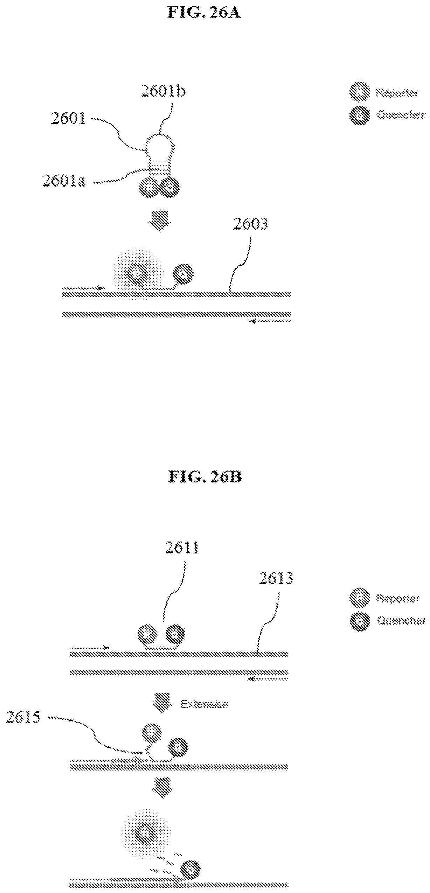

**FIGs. 26A** and **26B** schematically illustrate different fluorescent probes for identifying one or more cell-free nucleic acid molecules comprising a plurality of phased variants.

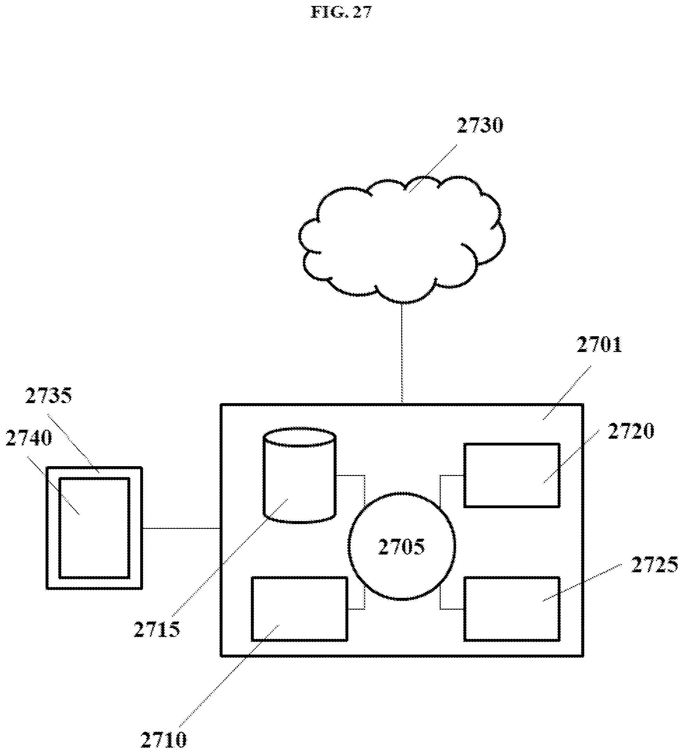

**FIG. 27** shows a computer system that is programmed or otherwise configured to implement methods provided herein.

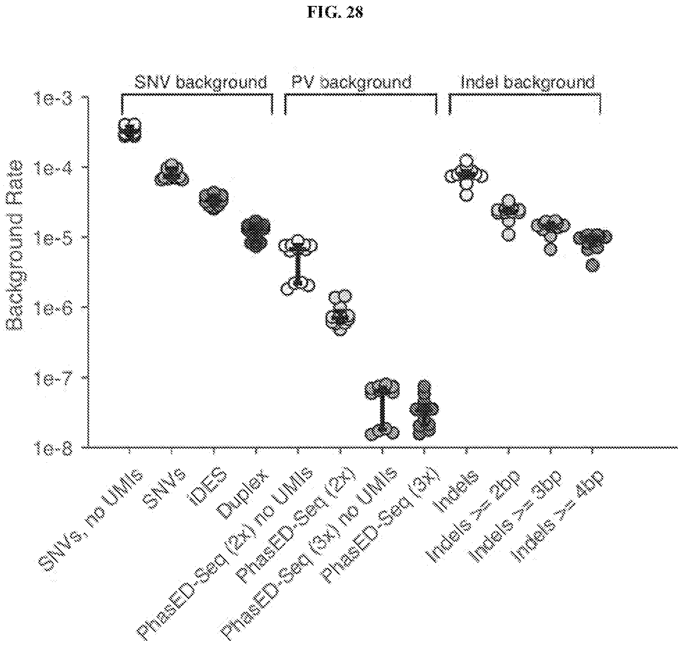

**FIG. 28** shows the low error rate of larger indels in comparison to duplex sequencing.

## DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

The term "about" or "approximately" generally means within an acceptable error range for the particular value, which may depend in part on how the value is measured or determined, e.g., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2- fold, of a value. Where particular values are described in the application and claims, unless otherwise stated, the term "about" meaning within an acceptable error range for the particular value may be assumed.

The terms "phased variants," "variants in phase," or "PV," as used interchangeably herein, generally refer to (1) two or more changes in nucleic acid sequence relative to a reference genomic sequence (e.g., SNVs, indel, translocation, amplification, etc.), or (2) one or more changes in nucleic acid sequence relative to a reference sequence and one or more changes in methylation status relative to a reference methylation status, so long as such changes occur within 170 bp of each other as determined by reference to the genomic reference sequence. Examples of changes in nucleic acid sequence relative to a reference genomic sequence (e.g., a sequence derived from one of more healthy cells or a consensus sequence from a cohort) can include: a somatic single nucleotide variant (SNV), a somatic indel, a somatic translocation breakpoint, a somatic amplification or deletion breakpoint (e.g., the boundary of a large genomic copy number alteration, such as a large-scale deletion or a large-scale amplification), a germline SNV, a germline indel, a germline translocation breakpoint, a germline amplification or deletion breakpoint, or a region of localized hypermutation (kataegis). In some cases, phased variants may occur in *cis* (i.e., on the same strand of a nucleic acid molecule) within a single molecule, such as a single cell-free nucleic acid molecule. In some cases, a cell-free nucleic acid molecule can be a cell-free deoxyribonucleic acid (cfDNA) molecule. In some cases, a cfDNA molecule can be derived from a diseased tissue, such as a tumor (e.g., a circulating tumor DNA (ctDNA) molecule). In some cases, the cell-free nucleic acid molecule can be a cell-free ribonucleic acid molecule. The term "phased variant" can refer to one of the plurality of variants that are required to occur in proximity to one another to constitute phased variants, while the term "set of phased variants," as used in the claims, can refer to the plurality of variants that together form phased variants (i.e., the variants that are within 170 bp of each other with respect to the reference genome, occurring on the same strand of DNA).

The term "biological sample" or "bodily sample," as used interchangeably herein, generally refers to a tissue or fluid sample derived from a subject. A biological sample can be directly obtained from the subject. Alternatively, a biological sample can be derived from the subject (e.g., by processing an initial biological sample obtained from the subject). The biological sample can be or can include one or more nucleic acid molecules, such as DNA or ribonucleic acid (RNA) molecules. The biological sample can be derived from any organ, tissue or biological fluid. A biological sample can comprise, for example, a bodily fluid or a solid tissue sample. An example of a solid tissue sample is a tumor sample, e.g., from a solid tumor biopsy. Non-limiting examples of bodily fluids include blood, serum, plasma, tumor cells, saliva, urine, cerebrospinal fluid, lymphatic fluid, prostatic fluid, seminal fluid, milk, sputum, stool, tears, and derivatives of these. In some cases, one or more cell-free nucleic acid molecules as disclosed herein can be derived from a biological sample.

The term "subject," as used herein, generally refers to any animal, mammal, or human. A subject can have, potentially have, or be suspected of having one or more conditions, such as a disease. In some cases, a condition of the subject can be cancer, a symptom(s) associated with cancer, or asymptomatic with respect to cancer or undiagnosed (e.g., not diagnosed for cancer). In some cases, the subject can have cancer, the subject can show a symptom(s) associated with cancer, the subject can be free from symptoms associated with cancer, or the subject may not be diagnosed with cancer. In some examples, the subject is a human.

The term "cell-free DNA" or "cfDNA," as used interchangeably herein, generally refers to DNA fragments circulating freely in a blood stream of a subject. Cell-free DNA fragments can have dinucleosomal protection (e.g., a fragment size of at least 240 base pairs ("bp")). These cfDNA fragments with dinucleosomal protection were likely not cut between the nucleosome, resulting in a longer fragment length (e.g., with a typical size distribution centered around 334 bp). Cell-free DNA fragments can have mononucleosomal protection (e.g., a fragment size of less than 240 base pairs ("bp")). These cfDNA fragments with mononucleosomal protection were likely cut between the nucleosome, resulting in a shorter fragment length (e.g., with a typical size distribution centered around 167 bp).

The term "sequencing data," as used herein, generally refers to "raw sequence reads" and/or "consensus sequences" of nucleic acids, such as cell-free nucleic acids or derivatives thereof. Raw sequence reads are the output of a DNA sequencer, and typically include redundant sequences of the same parent molecule, for example after amplification. "Consensus sequences" are sequences derived from redundant sequences of a parent molecule intended to represent the sequence of the original parent molecule. Consensus sequences can be produced by voting (wherein each majority nucleotide, e.g., the most commonly observed nucleotide at a given base position, among the sequences is the consensus nucleotide) or other approaches such as comparing to a reference genome. In some cases, consensus sequences can be produced by tagging original parent molecules with unique or non-unique molecular tags, which allow tracking of the progeny sequences (e.g., after amplification) by tracking of the tag and/or use of sequence read internal information.

The term "reference genomic sequence," as used herein, generally refers to a nucleotide sequence against which a subject's nucleotide sequences are compared.

The term "genomic region," as used herein, generally refers to any region (e.g., range of base pair locations) of a genome, e.g., an entire genome, a chromosome, a gene, or an exon. A genomic region can be a contiguous or a non-contiguous region. A "genetic locus" (or "locus") can be a portion or entirety of a genomic region (e.g., a gene, a portion of a gene, or a single nucleotide of a gene).

The term "likelihood," as used herein, generally refers to a probability, a relative probability, a presence or an absence, or a degree.

The term "liquid biopsy," as used herein, generally refers to a non-invasive or minimally invasive laboratory test or assay (e.g., of a biological sample or cell-free nucleic acids). The "liquid biopsy" assays can report detections or measurements (e.g., minor allele frequencies, gene expression, or protein expression) of one or more marker genes associated with a condition of a subject (e.g., cancer or tumor-associated marker genes).

## A. Introduction

Modifications (e.g., mutations) of genomic DNA can be manifested in a formation and/or progression of one or more conditions (e.g., a disease, such as cancer or tumor) of a subject. The present disclosure provides methods and systems for analyzing cell-free nucleic acid molecules, such as cfDNA, from a subject to determine the presence or absence of a condition of the subject, prognosis of a diagnosed condition of the subject, progress of the condition of the subject over time, therapeutic treatment of a diagnosed condition of the subject, or predicted treatment outcome for a condition of the subject.

Analysis of cell-free nucleic acids, such as cfDNA, have been developed with broad applications in, e.g., prenatal testing, organ or tissue transplantation, infectious disease, and oncology. In the context of detecting or monitoring a disease of a subject, such as cancer, circulating tumor DNA (ctDNA) can be a sensitive and specific biomarker in numerous cancer types. In some cases, ctDNA can be used to detect the presence of minimal residual disease (MRD) or tumor burden after treatment, such as chemotherapies or surgical resection of solid tumors. However, the limit of detection (LOD) for ctDNA analysis can be restricted by a number of factors including (i) low input DNA amounts from a typical blood collection and (ii) background error rates from sequencing.

In some cases, ctDNA-based cancer detection can be improved by tracking multiple somatic mutations with error-suppressed sequencing, e.g., with LOD of about 2 parts in 100,000 from cfDNA input while using off-the-shelf panels or personalized assays. However, in some cases, current LOD of ctDNA of interest can be insufficient to universally detect MRD in patients destined for disease relapse or progression. For example, such 'loss of detection' can be exemplified in diffuse large B-cell lymphoma (DLBCL). For DLBCL, interim ctDNA detection after only two cycles of curative-intent therapy can represent a major molecular response (MMR), and can be a strong prognostic marker for ultimate clinical outcomes. Despite this, nearly one-third of patients ultimately experiencing disease progression do not have detectable ctDNA at this interim landmark using available techniques (e.g., Cancer Personalized Profiling by Deep Sequencing (CAPP-Seq)), thus representing 'false-negative' measurements. Such high false-negative rates have also been observed in DLBCL patients by alternative methods, such as monitoring ctDNA through immunoglobulin gene rearrangements. Therefore, there exists a need for improved methods of ctDNA-based cancer detection with greater sensitivity.

Somatic variants detected on both of the complementary strands of parental DNA duplexes can be used to lower the LOD of ctDNA detection, thereby advantageously increasing the sensitivity of ctDNA detection. Such 'duplex sequencing' can reduce background error profile due to the requirement of two concordant events for detection of a single nucleotide variant (SNV). However, the duplex sequencing approach alone can be limited by inefficient recovery of DNA duplexes as recovery of both original strands can occur in a minority of all recovered molecules. Thus, duplex sequencing may be suboptimal and inefficient for real-world ctDNA detection with limited amount of starting sample, where input DNA from practical blood volumes (e.g., between about 4,000 to about 8,000 genomes per standard 10 milliliter (mL) blood collection tube) is limited and maximal recovery of genomes is essential.

Thus, there remains a significant unmet need for detection and analysis of ctDNA with low LOD (e.g., thereby yielding high sensitivity) for determining, for example, presence or absence of a disease of a subject, prognosis of the disease, treatment for the disease, and/or predicted outcome of the treatment.

**B. Methods and systems for determining or monitoring a condition**

The present disclosure describes methods and systems for detecting and analyzing cell free nucleic acids with a plurality of phased variants as a characteristic of a condition of a subject. In some aspects, the cell-free nucleic acid molecules can comprise cfDNA molecules, such as ctDNA molecules. The methods and systems disclosed herein can utilize sequencing data derived from a plurality of cell-free nucleic acid molecules of the subject to identify a subset of the plurality of cell-free nucleic acid molecules having the plurality of phased variants, thereby to determine the condition of the subject. The methods and systems disclosed herein can directly detect and, in some cases, pull down (or capture) such subset of the plurality of cell-free nucleic acid molecules that exhibit the plurality of phased variants, thereby to determine the condition of the subject with or without sequencing. The methods and systems disclosed herein can reduce background error rate often involved during detection and analysis of cell-free nucleic acid molecules, such as cfDNA.

In some aspects, methods and systems for cell-free nucleic acid sequencing and detection of cancer are provided. In some embodiments, cell-free nucleic acids (e.g., cfDNA or cfRNA) can be extracted from a liquid biopsy of an individual and prepared for sequencing. Sequencing results of the cell-free nucleic acids can be analyzed to detect somatic variants in phase (i.e., phased variants, as disclosed herein) as an indication of circulating-tumor nucleic acid (ctDNA or ctRNA) sequences (i.e., sequences that derived or are originated from nucleic acids of a cancer cell). Accordingly, in some cases, cancer can be detected in the individual by extracting a liquid biopsy from the individual and sequencing the cell-free nucleic acids derived from that liquid biopsy to detect circulating-tumor nucleic acid sequences, and the presence of circulating-tumor nucleic acid sequences can indicate that the individual has a cancer (e.g., a specific type of cancer). In some cases, a clinical intervention and/or treatment can be determined and/or performed on the individual based on the detection of the cancer.

As disclosed herein, a presence of somatic variants in phase can be a strong indication that the nucleic acids containing such phased variants are derived from a bodily sample with a condition, such as a cancerous cell (or alternatively, that the nucleic acids are from derived from a bodily sample obtained or derived from a subject with a condition, such as cancer). Detection of phased somatic variants can enhance the signal-to-noise ratio of cell-free nucleic acid detection methods (e.g., by reducing or eliminating spurious "noise" signals) as it may be unlikely that phased mutations would occur within a small genetic window that is approximately the size of a typical cell-free nucleic acid molecule (e.g., about 170 bp or less).

In some aspects, a number of genomic regions can be used as hotspots for detection of phased variants, especially in various cancers, e.g., lymphomas. In some cases, enzymes (e.g., AID, Apobec3a) can stereotypically mutagenize DNA in specific genes and locations, leading to development of particular cancers. Accordingly, cell-free nucleic acids derived from such hotspot genomic regions can be captured or targeted (e.g., with or without deep sequencing) for cancer detection and/or monitoring. Alternatively, capture or targeted sequencing can be performed on regions in which phased variants have been previously detected from a cancerous source (e.g., tumor) of a particular individual in order to detect cancer in that individual.

In some aspects, capture sequencing on cell-free nucleic acids can be performed as a screening diagnostic (e.g., in subjects that have not been previously diagnosed and/or previously suspected or having a condition, such as cancer). In some cases, a screening diagnostic can be developed and used to detect circulating-tumor nucleic acids for cancers that have stereotypical regions of phased variants. In some cases, capture sequencing on cell-free nucleic acids is performed as a diagnostic to detect MRD or tumor burden to determine if a particular disease is present during or after treatment. In some cases, capture sequencing on cell-free nucleic acids can be performed as a diagnostic to determine progress (e.g., progression or regression) of a treatment.

In some aspects, cell-free nucleic acid sequencing results can be analyzed to detect whether phased somatic single nucleotide variants (SNVs) or other mutations or variants (e.g., indels) exist within the cell-free nucleic acid sample. In some cases, the presence of particular somatic SNVs or other variants can be indicative of circulating-tumor nucleic acid sequences, and thus indicative of a tumor present in the subject. In some cases, a minimum of two variants can be detected in phase on a cell-free nucleic acid molecule. In some cases, a minimum of three variants can be detected in phase on a cell-free nucleic acid molecule. In some cases, a minimum of four variants can be detected in phase on a cell-free nucleic acid molecule. In some cases, a minimum of five or more variants can be detected in phase on a cell-free nucleic acid molecule. In some cases, the greater number of phased variants detected on a cell-free nucleic acid molecule, the greater the likelihood that the cell-free nucleic acid molecule is derived from cancer, as opposed to detecting an innocuous sequence of somatic variants that arise from molecular preparation of the sequence library or random biological errors. Accordingly, the likelihood of false-positive detection can decrease with detection of more variants in phase within a molecule (e.g., thereby increasing specificity of detection).

In some aspects, a cell-free nucleic acid sequencing result can be analyzed to detect whether an insertion or deletion of one or more nucleobases (i.e., indel) exist within the cell-free nucleic acid sample, e.g., relative to a reference genomic sequence. Without wishing to be bound by theory, in some cases, presence of indels in a cell-free nucleic acid molecule (e.g., cfDNA) can be indicative of a condition of a subject, e.g., a disease such as cancer. In some cases, a genetic variation as a result of an indel can be treated as a variant or mutation, and thus two indels can be treated as two phased variants, as disclosed herein. In some examples, within a cell-free nucleic acid molecule, a first genetic variation from a first indel (a first phase variant) and a second genetic variation from a second indel (a second phase variant) can be separated from each other by at least 1 nucleotide.

Within a single cell-free nucleic acid molecule (e.g., a single cfDNA molecule), as disclosed herein, a first phased variant can be a SNV and a second phased variant can be a part of a different small nucleotide polymorphism, e.g., another SNV or a part of a multi-nucleotide variant (MNV). A multi-nucleotide variant can be a cluster of two or more (e.g., at least 2, 3, 4, 5, or more) adjacent variants existing within the same stand of nucleic acid molecule. In some cases, the first phased variant and the second phased variant can be parts of the same MNV within the single cell-free nucleic acid molecule. In some cases, the first phased variant and the second phased variant can be from two different MNVs within the single cell-free nucleic acid molecule.

In some aspects, a statistical method can be utilized to calculate the likelihood that detected phased variants are from a cancer and not random or artificial (e.g., from sample prep or sequencing error). In some cases, a Monte Carlo sampling method can be utilized to determine the likelihood that detected phased variants are from a cancer and not random or artificial.

Aspects of the present disclosure provide identification or detection of cell-free nucleic acids (e.g., cfDNA molecule) with a plurality of phased variants, e.g., from a liquid biopsy of a subject. In some cases, a first phased variant of the plurality of phased variants and a second phased variant of the plurality of phased variants can be directly adjacent to each other (e.g., neighboring SNVs). In some cases, a first phased variant of the plurality of phased variants and a second phased variant of the plurality of phased variants can be separated by at least one nucleotide. The spacing between the first phased variant and the second phased variant can be limited by the length of the cell-free nucleic acid molecule.

Within a single cell-free nucleic acid molecule (e.g., a single cfDNA molecule), as disclosed herein, a first phased variant and a second phased variant can be separated from each other by at least or up to about 1 nucleotide, at least or up to about 2 nucleotides, at least or up to about 3 nucleotides, at least or up to about 4 nucleotides, at least or up to about 5 nucleotides, at least or up to about 6 nucleotides, at least or up to about 7 nucleotides, at least or up to about 8 nucleotides, at least or up to about 9 nucleotides, at least or up to about 10 nucleotides, at least or up to about 11 nucleotides, at least or up to about 12 nucleotides, at least or up to about 13 nucleotides, at least or up to about 14 nucleotides, at least or up to about 15 nucleotides, at least or up to about 20 nucleotides, at least or up to about 25 nucleotides, at least or up to about 30 nucleotides, at least or up to about 35 nucleotides, at least or up to about 40 nucleotides, at least or up to about 45 nucleotides, at least or up to about 50 nucleotides, at least or up to about 60 nucleotides, at least or up to about 70 nucleotides, at least or up to about 80 nucleotides, at least or up to about 90 nucleotides, at least or up to about 100 nucleotides, at least or up to about 110 nucleotides, at least or up to about 120 nucleotides, at least or up to about 130 nucleotides, at least or up to about 140 nucleotides, at least or up to about 150 nucleotides, at least or up to about 160 nucleotides, at least or up to about 170 nucleotides, or at least or up to about 180 nucleotides. Alternatively, or in addition to, within a single cell-free nucleic acid molecule, a first phased variant and a second phased variant may not or need not be separated by one or more nucleotides and thus can be directly adjacent to one another.

A single cell-free nucleic acid molecule (e.g., a single cfDNA molecule), as disclosed herein, can comprise at least or up to about 2 phased variants, at least or up to about 3 phased variants, at least or up to about 4 phased variants, at least or up to about 5 phased variants, at least or up to about 6 phased variants, at least or up to about 7 phased variants, at least or up to about 8 phased variants, at least or up to about 9 phased variants, at least or up to about 10 phased variants, at least or up to about 12 phased variants, at least or up to about 12 phased variants, at least or up to about 13 phased variants, at least or up to about 14 phased variants, at least or up to about 15 phased variants, at least or up to about 20 phased variants, or at least or up to about 25 phased variants within the same molecule.

From a plurality of cell-free nucleic acid molecules obtained (e.g., from a liquid biopsy of a subject), two or more (e.g., 10 or more, 1,000 or more, 10,000 or more) cell-free nucleic acid molecules can be identified to have an average of at least or up to about 2 phased variants, at least or up to about 3 phased variants, at least or up to about 4 phased variants, at least or up to about 5 phased variants, at least or up to about 6 phased variants, at least or up to about 7 phased variants, at least or up to about 8 phased variants, at least or up to about 9 phased variants, at least or up to about 10 phased variants, at least or up to about 12 phased variants, at least or up to about 12 phased variants, at least or up to about 13 phased variants, at least or up to about 14 phased variants, at least or up to about 15 phased variants, at least or up to about 20 phased variants, or at least or up to about 25 phased variants per each cell-free nucleic acid molecule identified to comprise a plurality of phased variants.

In some cases, a plurality of cell-free nucleic acid molecules (e.g., cfDNA molecules) can be obtained from a biological sample of a subject (e.g., solid tumor or liquid biopsy). Out of the plurality of cell-free nucleic acid molecules, at least or up to 1, at least or up to 2, at least or up to 3, at least or up to 4, at least or up to 5, at least or up to 6, at least or up to 7, at least or up to 8, at least or up to 9, at least or up to 10, at least or up to 15, at least or up to 20, at least or up to 25, at least or up to 30, at least or up to 35, at least or up to 40, at least or up to 45, at least or up to 50, at least or up to 60, at least or up to 70, at least or up to 80, at least or up to 90, at least or up to 100, at least or up to 150, at least or up to 200, at least or up to 300, at least or up to 400, at least or up to 500, at least or up to 600, at least or up to 700, at least or up to 800, at least or up to 900, at least or up to 1,000, at least or up to 5,000, at least or up to, 10,000, at least or up to 50,000, or at least or up to 100,000 cell-free nucleic acid molecules can be identified, such that each identified cell-free nucleic acid molecule comprises the plurality of phased variants, as disclosed herein.

In some cases, a plurality of cell-free nucleic acid molecules (e.g., cfDNA molecules) can be obtained from a biological sample of a subject (e.g., solid tumor or liquid biopsy). Out of the plurality of cell-free nucleic acid molecules, at least or up to 1, at least or up to 2, at least or up to 3, at least or up to 4, at least or up to 5, at least or up to 6, at least or up to 7, at least or up to 8, at least or up to 9, at least or up to 10, at least or up to 15, at least or up to 20, at least or up to 25, at least or up to 30, at least or up to 35, at least or up to 40, at least or up to 45, at least or up to 50, at least or up to 60, at least or up to 70, at least or up to 80, at least or up to 90, at least or up to 100, at least or up to 150, at least or up to 200, at least or up to 300, at least or up to 400, at least or up to 500, at least or up to 600, at least or up to 700, at least or up to 800, at least or up to 900, or at least or up to 1,000 cell-free nucleic acid molecules can be identified from a target genomic region (e.g., a target genomic locus), such that each identified cell-free nucleic acid molecule comprises the plurality of phased variants, as disclosed herein.

**FIGs. 1A** and **1E** schematically illustrate examples of (i) a cfDNA molecule comprising a SNV and (ii) another cfDNA molecule comprising a plurality of phased variants. Each variant identified within the cfDNA can indicate a presence of one more genetic mutations in the cell that the cfNDA is originated from. In alternative embodiments, one or more of the phased variants may be an insertion or deletion (indel) or other genomic alteration instead of an SNV.

In one aspect, the present disclosure provides a method for determining a condition of a subject, as shown by flowchart 2510 in **FIG. 25A.** The method can comprise (a) obtaining, by a computer system, sequencing data derived from a plurality of cell-free nucleic acid molecules that is obtained or derived from the subject (process 2512). The method can further comprise (b) processing, by the computer system, the sequencing data to identify one or more cell-free nucleic acid molecules of the plurality of cell-free nucleic acid molecules, wherein each of the one or more cell-free nucleic acid molecules identified comprises a plurality of phased variants relative to a reference genomic sequence (process 2514). In some cases, at least a portion of the one or more cell-free nucleic acid molecules can comprise a first phased variant of the plurality of phased variants and a second phased variant of the plurality of phased variants that are separated by at least one nucleotide, as disclosed herein. The method can optionally comprise (c) analyzing, by the computer system, at least a portion of the identified one or more cell-free nucleic acid molecules to determine the condition of the subject (process 2516).

In some cases, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, at least or up to about 50%, at least or up to about 60%, at least or up to about 70%, at least or up to about 80%, at least or up to about 90%, at least or up to about 95%, at least or up to about 99%, or about 100% of the one or more cell-free nucleic acid molecules can comprise a first phased variant of the plurality of phased variants and a second phased variant of the plurality of phased variants that are separated by at least one nucleotide, as disclosed herein. In some examples, a plurality of phased variants within a single cfDNA molecule can comprise (i) a first plurality of phased variants that are separated by at least one nucleotide from one another and (ii) a second plurality of phased variants that are adjacent to one another (e.g., two phased variants within a MNV). In some examples, a plurality of phased variants within a single cfDNA molecule can consist of phased variants that are separate by at least one nucleotide from one another.

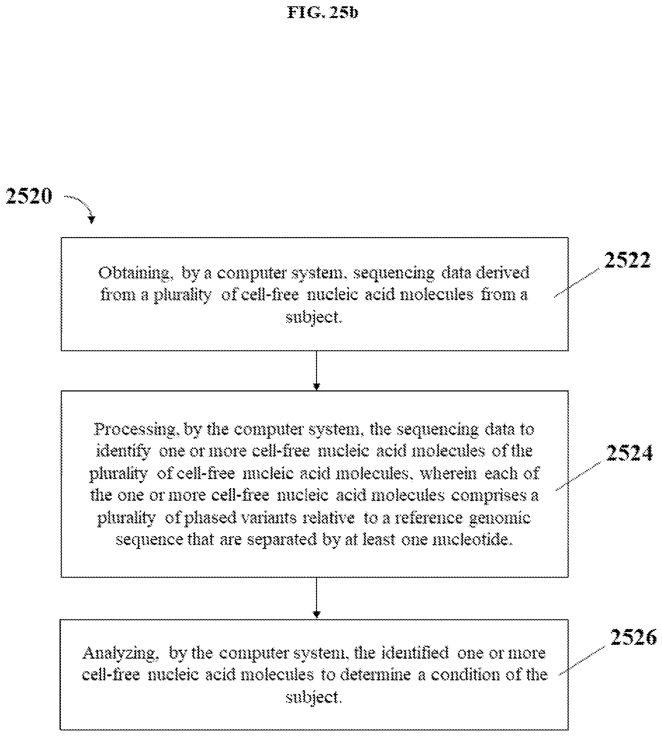

In one aspect, the present disclosure provides a method for determining a condition of the subject, as shown by flowchart 2520 in **FIG. 25B.** The method can comprise (a) obtaining, by a computer system, sequencing data derived from a plurality of cell-free nucleic acid molecules that is obtained or derived from a subject (process 2522). The method can further comprise (b) processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence (process 2524). In some cases, a first phased variant of the plurality of phased variant and a second phased variant of the plurality of phased variant can be separated by at least one nucleotide, as disclosed herein. The method can optionally comprise (c) analyzing, by the computer system, at least a portion of the identified one or more cell-free nucleic acid molecules to determine the condition of the subject (process 2526).

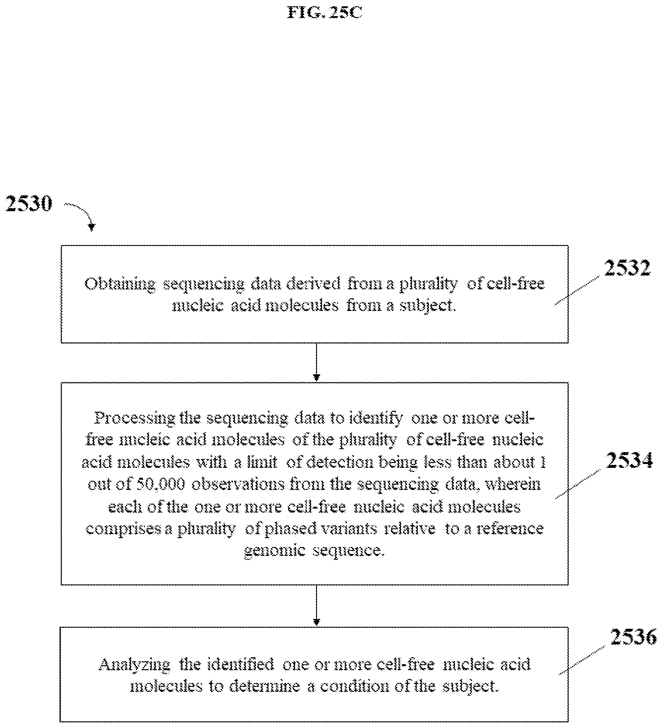

In one aspect, the present disclosure provides a method for determining a condition of a subject, as shown by flowchart 2530 in **FIG. 25C.** The method can comprise (a) obtaining sequencing data derived from a plurality of cell-free nucleic acid molecules that is obtained or derived from the subject (process 2532). The method can further comprise (b) processing the sequencing data to identify one or more cell-free nucleic acid molecules of the plurality of cell-free nucleic acid molecules with a LOD being less than about 1 out of 50,000 observations (or cell-free nucleic acid molecules) from the sequencing data (process 2534). In some cases, each of the one or more cell-free nucleic acid molecules comprises a plurality of phased variants relative to a reference genomic sequence. The method can optionally comprise (c) analyzing at least a portion of the identified one or more cell-free nucleic acid molecules to determine the condition of the subject (process 2536).

In some cases, the LOD of the operation of identifying the one or more cell-free nucleic acid molecules, as disclosed herein, can be less than about 1 out of 60,000, less than 1 out of 70,000, less than 10 out of 80,000, less than 1 out of 90,000, less than 1 out of 100,000, less than 1 out of 150,000, less than 1 out of 200,000, less than 1 out of 300,000, less than 1 out of 400,000, less than 1 out of 500,000, less than 1 out of 600,000, less than 1 out of 700,000, less than 1 out of 800,000, less than 1 out of 900,000, less than 1 out of 1,000,000, less than 1 out of 1,000,000, less than 1 out of 1,100,000, less than 1 out of 1,200,000, less than 1 out of 1,300,000, less than 1 out of 1,400,000, less than 1 out of 1,500,000, or less than 1 out of 2,000,000 observations from the sequencing data.

In some cases, at least one cell-free nucleic acid molecule of the identified one or more cell-free nucleic acid molecules can comprise a first phased variant of the plurality of phased variants and a second phased variant of the plurality of phased variants that are separated by at least one nucleotide, as disclosed herein.

In some cases, one or more of the operations (a) through (c) of the subject method can be performed by a computer system. In an example, all of the operations (a) through (c) of the subject method can be performed by the computer system.

The sequencing data, as disclosed herein, can be obtained from one or more sequencing methods. A sequencing method can be a first-generation sequencing method (e.g., Maxam-Gilbert sequencing, Sanger sequencing). A sequencing method can be a high-throughput sequencing method, such as next-generation sequencing (NGS) (e.g., sequencing by synthesis). A high-throughput sequencing method can sequence simultaneously (or substantially simultaneously) at least about 10,000, at least about 100,000, at least about 1 million, at least about 10 million, at least about 100 million, at least about 1 billion, or more polynucleotide molecules (e.g., cell-free nucleic acid molecules or derivatives thereof). NGS can be any generation number of sequencing technologies (e.g., second-generation sequencing technologies, third-generation sequencing technologies, fourth-generation sequencing technologies, etc.). Non-limiting examples of high-throughput sequencing methods include massively parallel signature sequencing, polony sequencing, pyrosequencing, sequencing-by-synthesis, combinatorial probe anchor synthesis (cPAS), sequencing-by-ligation (e.g., sequencing by oligonucleotide ligation and detection (SOLiD) sequencing), semiconductor sequencing (e.g., Ion Torrent semiconductor sequencing), DNA nanoball sequencing, and single-molecule sequencing, sequencing-by-hybridization.

In some embodiments of any one of the methods disclosed herein, the sequencing data can be obtained based on any of the disclosed sequencing methods that utilizes nucleic acid amplification (e.g., polymerase chain reaction (PCR)). Non-limiting examples of such sequencing methods can include 454 pyrosequencing, polony sequencing, and SoLiD sequencing. In some cases, amplicons (e.g., derivatives of the plurality of cell-free nucleic acid molecules that is obtained or derived from the subject, as disclosed herein) that correspond to a genomic region of interest (e.g., a genomic region associated with a disease) can be generated by PCR, optionally pooled, and subsequently sequenced to generating sequencing data. In some examples, because the regions of interest are amplified into amplicons by PCR before being sequenced, the nucleic acid sample is already enriched for the region of interest, and thus any additional pooling (e.g., hybridization) may not and need not be needed prior to sequencing (e.g., non-hybridization based NGS). Alternatively, pooling via hybridization can further be performed for additional enrichment prior to sequencing. Alternatively, the sequencing data can be obtained without generating PCR copies, e.g., via cPAS sequencing.

A number of embodiments utilize capture hybridization techniques to perform targeted sequencing. When performing sequencing on cell-free nucleic acids, in order to enhance resolution on particular genomic loci, library products can be captured by hybridization prior to sequencing. Capture hybridization can be particularly useful when trying to detect rare and/or somatic phased variants from a sample at particular genomic loci. In some situations, detection of rare and/or somatic phased variants is indicative of the source of nucleic acids, including nucleic acids derived from a cancer source. Accordingly, capture hybridization is a tool that can enhance detection of circulating-tumor nucleic acids within cell-free nucleic acids.

Various types of cancers repeatedly experience aberrant somatic hypermutation in particular genomic loci. For instance, the enzyme activation-induced deaminase induces aberrant somatic hypermutation in B-cells, which leads to various B-cell lymphomas, including (but not limited to) diffuse large B-cell lymphoma (DLBCL), follicular lymphoma (FL), Burkitt lymphoma (BL), and B-cell chronic lymphocytic leukemia (CLL). Accordingly, in numerous embodiments, probes are designed to pull down (or capture) genomic loci known to experience aberrant somatic hypermutation in a lymphoma. **FIG. 1D** and **Table 1** describe a number of regions that experience aberrant somatic hypermutation in DLBCL, FL, BL and CLL. Provided in **Table 6** is list of nucleic acid probes that can be utilized to pull down (or capture) genomic loci to detect aberrant somatic hypermutation in B-cell cancers.

Capture sequencing can also be performed utilizing personalized nucleic acid probes designed to detect the existence of an individual's cancer. An individual having a cancer can have their cancer biopsied and sequenced to detect somatic phased variants that have accumulated in the cancer. Based on the sequencing result, in accordance with a number of embodiments, nucleic acid probes are designed and synthesized capable of pulling down the genomic loci inclusive of the positions of where the phased variants. These personalized designed and synthesized nucleic acid probes can be utilized to detect circulating-tumor nucleic acids from a liquid biopsy of that individual. Accordingly, the personalized nucleic acid probes can be useful for determining treatment response and/or detecting MRD after treatment.

In some embodiments of any one of the methods disclosed herein, the sequencing data can be obtained based on any sequencing method that utilizes adapters. Nucleic acid samples (e.g., the plurality of cell-free nucleic acid molecules from the subject, as disclosed herein) can be conjugated with one or more adapters (or adapter sequences) for recognizing (e.g., via hybridization) of the sample or any derivatives thereof (e.g., amplicons). In some examples, the nucleic acid samples can be tagged with a molecular barcode, e.g., such that each cell-free nucleic acid molecule of the plurality of cell-free nucleic acid molecules can have a unique barcode. Alternatively, or in addition to, the nucleic acid samples can be tagged with a sample barcode, e.g., such that the plurality of cell-free nucleic acid molecules from the subject (e.g., a plurality of cell-free nucleic acid molecules obtained from a specific bodily tissue of the subject) can have the same barcode.

In alternative embodiments, the methods of identifying one or more cell-free nucleic acid molecules comprising the plurality of phased variants, as disclosed herein, can be performed without molecular barcoding, without sample barcoding, or without molecular barcoding and sample barcoding, at least in part due to high specificity and low LOD achieved by relying on identifying the phased variants as opposed to, e.g., a single SNV.

In some embodiments of any one of the methods disclosed herein, the sequencing data can be obtained and analyzed without in silico removal or suppression of (i) background error and/or (ii) sequencing error, at least in part due to high specificity and low LOD achieved by relying on identifying the phased variants as opposed to, e.g., a single SNV or indel.

In some embodiments of any one of the methods disclosed herein, using the plurality of variants as a condition to identify target cell-free nucleic acid molecules with specific mutations of interest without in silico methods of error suppression can yield a background error-rate that is lower than that of (i) barcode-deduplication, (ii) integrated digital error suppression, or (iii) duplex sequencing by at least about 5-fold, at least about 10-fold, at least about 20-fold, at least about 30-fold, at least about 40-fold, at least about 50-fold, at least about 60-fold, at least about 70-fold, at least about 80-fold, at least about 90-fold, at least about 100-fold, at least about 200-fold, at least about 400-fold, at least about 600-fold, at least about 800-fold, or at least about 1,000-fold. This approach may advantageously increase signal-to-noise ratio (thereby increasing sensitivity and/or specificity) of identifying target cell-free nucleic acid molecules with specific mutations of interest.

In some embodiments of any one of the methods disclosed herein, increasing a minimum number of phased variants (e.g., increasing from at least two phased variants to at least three phased variants) per cell-free nucleic acid molecule required as a condition to identify target cell-free nucleic acid molecules with specific mutations of interest can reduce the background error-rate by at least about 5-fold, at least about 10-fold, at least about 20-fold, at least about 30-fold, at least about 40-fold, at least about 50-fold, at least about 60-fold, at least about 70-fold, at least about 80-fold, at least about 90-fold, or at least about 100-fold. This approach may advantageously increase signal-to-noise ratio (thereby increasing sensitivity and/or specificity) of identifying target cell-free nucleic acid molecules with specific mutations of interest.

In one aspect, the present disclosure provides a method of treating a condition of a subject, as shown in flowchart 2540 in **FIG. 25D.** The method can comprise (a) identifying the subject for treatment of the condition, wherein the subject has been determined to have the condition based on identification of one or more cell-free nucleic acid molecules from a plurality of cell-free nucleic acid molecules that is obtained or derived from the subject (Process 2542). Each of the identified one or more cell-free nucleic acid molecules can comprise a plurality of phased variants relative to a reference genomic sequence. At least a portion (e.g., partial or all) of the plurality of phased variants can be separated by at least one nucleotide, such that a first phased variant of the plurality of phased variants and a second phased variant of the plurality of phased variants are separated by at least one nucleotide, as disclosed herein. In some cases, a presence of the plurality of phased variants is indicative of the condition (e.g., a disease, such as cancer) of the subject. The method can further comprise (b) subjecting the subject to the treatment based on the step (a) (process 2544). Examples of such treatment of the condition of the subject are disclosed elsewhere in the present disclosure.

In one aspect, the present disclosure provides a method of monitoring a progress (e.g., progression or regression) of a condition of a subject, as shown in flowchart 2550 in **FIG. 25E.** The method can comprise (a) determining a first state of the condition of the subject based on identification of a first set of one or more cell-free nucleic acid molecules from a first plurality of cell-free nucleic acid molecules that is obtained or derived from the subject (process 2552). The method can further comprise (b) determining a second state of the condition of the subject based on identification of a second set of one or more cell-free nucleic acid molecules from a second plurality of cell-free nucleic acid molecules that is obtained or derived from the subject (process 2554). The second plurality of cell-free nucleic acid molecules can be obtained from the subject subsequent to obtaining the first plurality of cell-free nucleic acid molecules from the subject. The method can optionally comprise (c) determining the progress (e.g., progression or regression) of the condition based at least in part on the first state of the condition and the second state of the condition (process 2556). In some cases, each of the one or more cell-free nucleic acid molecules identified (e.g., each of the first set of one or more cell-free nucleic acid molecules identified, each of the second set of one or more cell-free nucleic acid molecules identified) can comprise a plurality of phased variants relative to a reference genomic sequence. At least a portion (e.g., partial or all) of the one or more cell-free nucleic acid molecules identified can be separated by at least one nucleotide, as disclosed herein. In some cases, presence of the plurality of phased variants can be indicative of a state of the condition of the subject.

In some cases, the first plurality of cell-free nucleic acid molecules from the subject can be obtained (e.g., via blood biopsy) and analyzed to determine (e.g., diagnose) a first state of the condition (e.g., a disease, such as cancer) of the subject. The first plurality of cell-free nucleic acid molecules can be analyzed via any of the methods disclosed herein (e.g., with or without sequencing) to identify the first set of one or more cell-free nucleic acid molecules comprising the plurality of phased variants, and the presence or characteristics of the first set of one or more cell-free nucleic acid molecules can be used to determine the first state of the condition (e.g., an initial diagnosis) of the subject. Based on the determined first state of the condition, the subject can be subjected to one or more treatments (e.g., chemotherapy) as disclosed herein. Subsequent to the one or more treatments, he second plurality of cell-free nucleic acid molecules can be obtained from the subject.

In some cases, the subject can be subjected to at least or up to about 1 treatment, at least or up to about 2 treatments, at least or up to about 3 treatments, at least or up to about 4 treatments, at least or up to about 5 treatments, at least or up to about 6 treatments, at least or up to about 7 treatments, at least or up to about 8 treatments, at least or up to about 9 treatments, or at least or up to about 10 treatments based on the determined first state of the condition. In some cases, the subject can be subjected to a plurality of treatments based on the determined first state of the condition, and a first treatment of the plurality of treatments and a second treatment of the plurality of treatments can be separated by at least or up to about 1 day, at least or up to about 7 days, at least or up to about 2 weeks, at least or up to about 3 weeks, at least or up to about 4 weeks, at least or up to about 2 months, at least or up to about 3 months, at least or up to about 4 months, at least or up to about 5 months, at least or up to about 6 months, at least or up to about 12 months, at least or up to about 2 years, at least or up to about 3 years, at least or up to about 4 years, at least or up to about 5 years, or at least or up to about 10 years. The plurality of treatments for the subject can be the same. Alternatively, the plurality of treatments can be different by drug type (e.g., different chemotherapeutic drugs), drug dosage (e.g., increasing dosage, decreasing dosage), presence or absence of a co-therapeutic agent (e.g., chemotherapy and immunotherapy), modes of administration (e.g., intravenous vs oral administrations), frequency of administration (e.g., daily, weekly, monthly), etc.

In some cases, the subject may not and need not be treated for the condition between determination of the first state of the condition and determination of the second state of the condition. For example, without any intervening treatment, the second plurality of cell-free nucleic acid molecules may be contained (e.g., via liquid biopsy) from the subject to confirm whether the subject still exhibits indications of the first state of the condition.

In some cases, the second plurality of cell-free nucleic acid molecules from the subject can be obtained (e.g., via blood biopsy) at least or up to about 1 day, at least or up to about 7 days, at least or up to about 2 weeks, at least or up to about 3 weeks, at least or up to about 4 weeks, at least or up to about 2 months, at least or up to about 3 months, at least or up to about 4 months, at least or up to about 5 months, at least or up to about 6 months, at least or up to about 12 months, at least or up to about 2 years, at least or up to about 3 years, at least or up to about 4 years, at least or up to about 5 years, or at least or up to about 10 years after obtaining the first plurality of cell-free nucleic acid molecules from the subject.

In some cases, at least or up to about 2, at least or up to about 3, at least or up to about 4, at least or up to about 5, at least or up to about 6, at least or up to about 7, at least or up to about 8, at least or up to about 9, or at least or up to about 10 different samples comprising a plurality of nucleic acid molecules (e.g., at least the first plurality of cell-free nucleic acid molecules and the second plurality of cell-free nucleic acid molecules) can be obtained over time (e.g., once every month for 6 months, once every two months for a year, once every three months for a year, once every 6 months for one or more years, etc.) to monitor the progress of the condition of the subject, as disclosed herein.

In some cases, the step of determining the progress of the condition based on the first state of the condition and the second state of the condition can comprise comparing one or more characteristics of the first state and the second state of the condition, such as, for example, (i) a total number of cell-free nucleic acid molecules identified to comprise the plurality of phased variants in each state (e.g., per equal weight or volume of the biological sample of origin, per equal number of initial cell-free nucleic acid molecules analyzed, etc.), (ii) an average number of the plurality of phased variants per each cell-free nucleic acid molecule identified to comprise a plurality of phased variants (i.e., two or more phased variants), or (iii) a number of cell-free nucleic acid molecules identified to comprise the plurality of phased variants divided by a total number of cell-free nucleic acid molecules that comprise a mutation that overlaps with some of the plurality of phased variants (i.e., phased variant allele frequency). Based on such comparison, MRD of the condition (e.g., cancer or tumor) of the subject can be determined. For example, tumor burden or cancer burden of the subject can be determined based on such comparison.

In some cases, the progress of the condition can be progression or worsening of the condition. In an example, the worsening of the condition can comprise developing of a cancer from an earlier stage to a later stage, such as from stage I cancer to stage III cancer. In another example, the worsening of the condition can comprise increasing size (e.g., volume) of a solid tumor. Yet in a different example, the worsening of the condition can comprise cancer metastasis from once location to another location within the subject's body.

In some examples, (i) a total number of cell-free nucleic acid molecules identified to comprise the plurality of phased variants from the second state of the condition of the subject can be higher than (ii) a total number of cell-free nucleic acid molecules identified to comprise the plurality of phased variants from the first state of the condition of the subject by at least or up to about 0.1-fold, at least or up to about 0.2-fold, at least or up to about 0.3-fold, at least or up to about 0.4-fold, at least or up to about 0.5-fold, at least or up to about 0.6-fold, at least or up to about 0.7-fold, at least or up to about 0.8-fold, at least or up to about 0.9-fold, at least or up to about 1-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 30-fold, at least or up to about 40-fold, at least or up to about 50-fold, at least or up to about 60-fold, at least or up to about 70-fold, at least or up to about 80-fold, at least or up to about 90-fold, at least or up to about 100-fold, at least or up to about 200-fold, at least or up to about 300-fold, at least or up to about 400-fold, or at least or up to about 500-fold.

In some examples, (i) an average number of the plurality of phased variants per each cell-free nucleic acid molecule identified to comprise a plurality of phased variants from the second state of the condition of the subject can be higher than (ii) an average number of the plurality of phased variants per each cell-free nucleic acid molecule identified to comprise a plurality of phased variants from the first state of the condition of the subject by at least or up to about 0.1-fold, at least or up to about 0.2-fold, at least or up to about 0.3-fold, at least or up to about 0.4-fold, at least or up to about 0.5-fold, at least or up to about 0.6-fold, at least or up to about 0.7-fold, at least or up to about 0.8-fold, at least or up to about 0.9-fold, at least or up to about 1-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 30-fold, at least or up to about 40-fold, at least or up to about 50-fold, at least or up to about 60-fold, at least or up to about 70-fold, at least or up to about 80-fold, at least or up to about 90-fold, at least or up to about 100-fold, at least or up to about 200-fold, at least or up to about 300-fold, at least or up to about 400-fold, or at least or up to about 500-fold.

In some cases, the progress of the condition can be regression or at least a partial remission of the condition. In an example, the at least the partial remission of the condition can comprise downstaging of a cancer from a later stage to an earlier stage, such as from stage IV cancer to stage II cancer. Alternatively, the at least the partial remission of the condition can be full remission from cancer. In another example, the at least the partial remission of the condition can comprise decreasing size (e.g., volume) of a solid tumor.

In some examples, (i) a total number of cell-free nucleic acid molecules identified to comprise the plurality of phased variants from the second state of the condition of the subject can be lower than (ii) a total number of cell-free nucleic acid molecules identified to comprise the plurality of phased variants from the first state of the condition of the subject by at least or up to about 0.1-fold, at least or up to about 0.2-fold, at least or up to about 0.3-fold, at least or up to about 0.4-fold, at least or up to about 0.5-fold, at least or up to about 0.6-fold, at least or up to about 0.7-fold, at least or up to about 0.8-fold, at least or up to about 0.9-fold, at least or up to about 1-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 30-fold, at least or up to about 40-fold, at least or up to about 50-fold, at least or up to about 60-fold, at least or up to about 70-fold, at least or up to about 80-fold, at least or up to about 90-fold, at least or up to about 100-fold, at least or up to about 200-fold, at least or up to about 300-fold, at least or up to about 400-fold, or at least or up to about 500-fold.

In some examples, (i) an average number of the plurality of phased variants per each cell-free nucleic acid molecule identified to comprise a plurality of phased variants from the second state of the condition of the subject can be lower than (ii) an average number of the plurality of phased variants per each cell-free nucleic acid molecule identified to comprise a plurality of phased variants from the first state of the condition of the subject by at least or up to about 0.1-fold, at least or up to about 0.2-fold, at least or up to about 0.3-fold, at least or up to about 0.4-fold, at least or up to about 0.5-fold, at least or up to about 0.6-fold, at least or up to about 0.7-fold, at least or up to about 0.8-fold, at least or up to about 0.9-fold, at least or up to about 1-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 30-fold, at least or up to about 40-fold, at least or up to about 50-fold, at least or up to about 60-fold, at least or up to about 70-fold, at least or up to about 80-fold, at least or up to about 90-fold, at least or up to about 100-fold, at least or up to about 200-fold, at least or up to about 300-fold, at least or up to about 400-fold, or at least or up to about 500-fold.

In some cases, the progress of the condition can remain substantially the same between the two states of the condition of the subject. In some examples, (i) a total number of cell-free nucleic acid molecules identified to comprise the plurality of phased variants from the second state of the condition of the subject can be about the same as (ii) a total number of cell-free nucleic acid molecules identified to comprise the plurality of phased variants from the first state of the condition of the subject. In some examples, (i) an average number of the plurality of phased variants per each cell-free nucleic acid molecule identified to comprise a plurality of phased variants from the second state of the condition of the subject can about the same as (ii) an average number of the plurality of phased variants per each cell-free nucleic acid molecule identified to comprise a plurality of phased variants from the first state of the condition of the subject.

In some embodiments of any one of the methods disclosed herein, the one or more cell-free nucleic acid molecules comprising the plurality of phased variants can be identified from the plurality of cell-free nucleic acid molecules by one or more sequencing methods. Alternatively, or in addition to, the one or more cell-free nucleic acid molecules comprising the plurality of phased variants can be identified by being pulled down from (or captured from among) the plurality of cell-free nucleic acid molecules with a set of nucleic acid probes. The pull down (or capture) method via the set of nucleic acid probes can be sufficient to identify the one or more cell-free nucleic acid molecules of interest without sequencing. In some cases, the set of nucleic acid probes can be configured to hybridize to at least a portion of cell-free nucleic acid (e.g., cfDNA) molecules from one or more genomic regions associated with the condition of the subject. As such, a presence of one or more cell-free nucleic acid molecules that have been pulled down by the set of nucleic acid probes can be an indication that the one or more cell-free nucleic acid molecules are derived from the condition (e.g., ctDNA or ctRNA). Additional details of the set of nucleic probes are disclosed elsewhere the present disclosure.

In some embodiments of any one of the methods disclosed herein, based the sequencing data derived from the plurality of cell-free nucleic acid molecules (e.g., cfDNA) that is obtained or derived from the subject, (i) the one or more cell-free nucleic acid molecules identified to comprise the plurality of phased variants can be separated, in silico, from (ii) one or more other cell-free nucleic acid molecules that are not identified to comprise the plurality of phased variants (or one or more other cell-free nucleic acid molecules that do not comprise the plurality of phased variants). In some cases, the method can further comprise generating an additional data comprising sequencing information of only (i) the one or more cell-free nucleic acid molecules identified to comprise the plurality of phased variants. In some cases, the method can further comprise generating a different data comprising sequencing information of only (ii) the one or more other cell-free nucleic acid molecules that are not identified to comprise the plurality of phased variants (or the one or more other cell-free nucleic acid molecules that do not comprise the plurality of phased variants).

In one aspect, the present disclosure provides a method for determining a condition of the subject, as shown by flowchart 2560 in **FIG. 25F.** The method can comprise (a) providing a mixture comprising (1) a set of nucleic acid probes and (2) a plurality of cell-free nucleic acid molecules obtained or derived from the subject (process 2562). In some cases, an individual nucleic acid probe of the set of nucleic acid probes can be designed to hybridize to a target cell-free nucleic acid molecule comprising a plurality of phased variants relative to a reference genomic sequence that are separated by at least one nucleotide. As such, a first phased variant of the plurality of phased variants and a second phased variant of the plurality of phased variants can be separated by at least one nucleotide, as disclosed herein. In some cases, the individual nucleic acid probe can comprise an activatable reporter agent. The activatable reporter agent can be activated by either one of (i) hybridization of the individual nucleic acid probe to the plurality of phased variants and (ii) dehybridization of at least a portion of the individual nucleic acid probe that has been hybridized to the plurality of phased variants. The method can further comprise (b) detecting the reporter agent that is activated, to identify one or more cell-free nucleic acid molecules of the plurality of cell-free nucleic acid molecules (process 2564). Each of the one or more cell-free nucleic acid molecules can comprise the plurality of phased variants. The method can optionally comprise (c) analyzing at least a portion of the identified one or more cell-free nucleic acid molecules to determine the condition of the subject (process 2566).

In one aspect, the present disclosure provides a method for determining a condition of the subject, as shown by flowchart 2570 in **FIG. 25G.** The method can comprise (a) providing a mixture comprising (1) a set of nucleic acid probes and (2) a plurality of cell-free nucleic acid molecules obtained or derived from the subject (process 2572). In some cases, an individual nucleic acid probe of the set of nucleic acid probes can be designed to hybridize to a target cell-free nucleic acid molecule comprising a plurality of phased variants relative to a reference genomic sequence. In some cases, the individual nucleic acid probe can comprise an activatable reporter agent. The activatable reporter agent can be activated by either one of (i) hybridization of the individual nucleic acid probe to the plurality of phased variants and (ii) dehybridization of at least a portion of the individual nucleic acid probe that has been hybridized to the plurality of phased variants. The method can further comprise (b) detecting the reporter agent that is activated, to identify one or more cell-free nucleic acid molecules of the plurality of cell-free nucleic acid molecules (process 2574). Each of the one or more cell-free nucleic acid molecules can comprise the plurality of phased variants, and a LOD of the identification step can be less than about 1 out of 50,000 cell-free nucleic acid molecules of the plurality of cell-free nucleic acid molecules, as disclosed herein. The method can optionally comprise (c) analyzing at least a portion of the identified one or more cell-free nucleic acid molecules to determine the condition of the subject (process 2576).

In some cases, a first phased variant of the plurality of phased variants and a second phased variant of the plurality of phased variants are separated by at least one nucleotide, as disclosed herein.

In some cases, the LOD of the step of identifying the one or more cell-free nucleic acid molecules, as disclosed herein, can be less than about 1 out of 60,000, less than 1 out of 70,000, less than 10 out of 80,000, less than 1 out of 90,000, less than 1 out of 100,000, less than 1 out of 150,000, less than 1 out of 200,000, less than 1 out of 300,000, less than 1 out of 400,000, less than 1 out of 500,000, less than 1 out of 600,000, less than 1 out of 700,000, less than 1 out of 800,000, less than 1 out of 900,000, less than 1 out of 1,000,000, less than 1 out of 1,000,000, less than 1 out of 1,100,000, less than 1 out of 1,200,000, less than 1 out of 1,300,000, less than 1 out of 1,400,000, less than 1 out of 1,500,000, less than 1 out of 2,000,000, less than 1 out of 2,500,000, less than 1 out of 3,000,000, less than 1 out of 4,000,000, or less than 1 out of 5,000,000 cell-free nucleic acid molecules of the plurality of cell-free nucleic acid molecules. Generally, a detection method with a lower LOD has a greater sensitivity of such detection.

In some embodiments of any one of the methods disclosed herein, the method can further comprise mixing (1) the set of nucleic acid probes and (2) the plurality of cell-free nucleic acid molecules.

In some embodiments of any one of the methods disclosed herein, the activatable reporter agent of a nucleic acid probe can be activated upon hybridization of the individual nucleic acid probe to the plurality of phased variants. Non-limiting examples of such nucleic acid probe can include a molecular beacon, eclipse probe, amplifluor probe, scorpions PCR primer, and light upon extension fluorogenic PCR primer (LUX primer).

For example, the nucleic acid probe can be a molecular beacon, as shown in **FIG. 26A.** The molecular beacon can be fluorescently labeled (e.g., dye-labeled) oligonucleotide probe that comprises complementarity to a target cell-free nucleic acid molecule 2603 in a region that comprises the plurality of phased variants. The molecular beacon can have a length between about 25 nucleotides to about 50 nucleotides. The molecular beacon can also be designed to be partially self-complimentary, such that it form a hairpin structure with a stem 2601a and a loop 2601b. The 5' and 3' ends of the molecular beacon probe can have complementary sequences (e.g., about 5-6 nucleotides) that form the stem structure 2601a. The loop portion 2601b of the hairpin can be designed to specifically hybridize to a portion (e.g., about 15-30 nucleotides) of the target sequence comprising two or more phased variants. The hairpin can be designed to hybridize to a portion that comprises at least 2, 3, 4, 5, or more phased variants. A fluorescent reporter molecule can be attached to the 5' end of the molecular beacon probe, and a quencher that quenches fluorescence of the fluorescent reporter can be attached to the 3' end of the molecular beacon probe. Formation of the hairpin therefore can bring the fluorescent reporter and quencher together, such that no fluorescence is emitted. However, during annealing operation of amplification reaction of the plurality of cell-free nucleic acid molecules that is obtained or derived from the subject, the loop portion of the molecular beacon can bind to its target sequence, causing the stem to denature. Thus, the reporter and quencher can be separated, abolishing quenching, and the fluorescent reporter is activated and detectable. Because fluorescence of the fluorescent reporter is emitted from the molecular beacon probe only when the probe is bound to the target sequence, the amount or level of fluorescence detected can be proportional to the amount of target in the reaction (e.g., (i) a total number of cell-free nucleic acid molecules identified to comprise the plurality of phased variants in each state or (ii) an average number of the plurality of phased variants per each cell-free nucleic acid molecule identified to comprise a plurality of phased variants, as disclosed herein).

In some embodiments of any one of the methods disclosed herein, the activatable reporter agent can be activated upon dehybridization of at least a portion of the individual nucleic acid probe that has been hybridized to the plurality of phased variants. In other words, once the individual nucleic acid probe is hybridized to target cell-free nucleic acid molecule's portion that comprises the plurality of phased variants, dehybridization of at least a portion of the individual nucleic acid prob and the target cell-free nucleic acid can activate the activatable reporter agent. Non-limiting examples of such nucleic acid probe can include a hydrolysis probe (e.g., TaqMan prob), dual hybridization probes, and QZyme PCR primer.

For example, the nucleic acid probe can be a hydrolysis probe, as shown in **FIG. 26B.** The hydrolysis probe 2611 can be a fluorescently labeled oligonucleotide probe that can specifically hybridize to a portion (e.g., between about 10 and about 25 nucleotides) of the target cell-free nucleic acid molecule 2613, wherein the hybridized portion comprises two or more phased variants. The hydrolysis probe 2611 can be labeled with a fluorescent reporter at the 5' end and a quencher at the 3' end. When the hydrolysis probe is intact (e.g., not cleaved), the fluorescence of the reporter is quenched due to its proximity to the quencher **(FIG. 26B).** During annealing operation of amplification reaction of the plurality of cell-free nucleic acid molecules obtained or derived from the subject, 5' → 3' exonuclease activity of certain thermostable polymerases (e.g., Taq or Tth) The amplification reaction of the plurality of cell-free nucleic acid molecules obtained or derived from the subject can include a combined annealing/extension operation during which the hydrolysis probe hybridizes to the target cell-free nucleic acid molecule, and the dsDNA-specific 5' → 3' exonuclease activity of a thermostable polymerase (e.g., Taq or Tth) cleaves off the fluorescent reporter from the hydrolysis probe. As a result, the fluorescent reporter is separated from the quencher, resulting in a fluorescence signal that is proportional to the amount of target in the sample (e.g., (i) a total number of cell-free nucleic acid molecules identified to comprise the plurality of phased variants in each state or (ii) an average number of the plurality of phased variants per each cell-free nucleic acid molecule identified to comprise a plurality of phased variants, as disclosed herein).

In some embodiments of any one of the methods disclosed herein, the reporter agent can comprise a fluorescent reporter. Non-limiting examples of a fluorescent reporter include fluorescein amidite (FAM, 2-[3-(dimethylamino)-6-dimethyliminio-xanthen-9-yl]benzoate TAMRA, (2E)-2-[(2E,4E)-5-(2-tert-butyl-9-ethyl-6,8,8-trimethyl-pyrano[3,2-g]quinolin-1-ium-4-yl)penta-2,4-dienylidene]-1-(6-hydroxy-6-oxo-hexyl)-3,3-dimethyl-indoline-5-sulfonate Dy 750, 6-carboxy-2',4,4',5',7,7'-hexachlorofluorescein, 4,5,6,7- Tetrachlorofluorescein TET™, sulforhodamine 101 acid chloride succinimidyl ester Texas Red-X, ALEXA Dyes, Bodipy Dyes, cyanine Dyes, Rhodamine 123 (hydrochloride), Well RED Dyes, MAX, and TEX 613. In some cases, the reporter agent further comprises a quencher, as disclosed herein. Non-limiting examples of a quencher can include Black Hole Quencher, Iowa Black Quencher, and 4-dimethylaminoazobenzene-4'-sulfonyl chloride (DABCYL).

In some embodiments of any one of the methods disclosed herein, any PCR reaction utilizing the set of nucleic acid probes can be performed using real-time PCR (qPCR). Alternatively, the PCR reaction utilizing the set of nucleic acid probes can be performed using digital PCR (dPCR).

Provided in **FIG. 24** is an example flowchart of a process to perform a clinical intervention and/or treatment based on detecting circulating-tumor nucleic acids in an individual's biological sample. In several embodiments, detection of circulating-tumor nucleic acids is determined by the detection of somatic variants in phase in a cell-free nucleic acid sample. In many embodiments, detection of circulating-tumor nucleic acids indicates cancer is present, and thus appropriate clinical intervention and/or treatment can be performed.

Referring to **FIG. 24,** process 2400 can begin with obtaining, preparing, and sequencing (2401) cell-free nucleic acids obtained from a non-invasive biopsy (e.g., liquid or waste biopsy), utilizing a capture sequencing approach across regions shown to harbor a plurality of genetic mutations or variants occurring in phase. In several embodiments, cfDNA and/or cfRNA is extracted from plasma, blood, lymph, saliva, urine, stool, and/or other appropriate bodily fluid. Cell-free nucleic acids can be isolated and purified by any appropriate means. In some embodiments, column purification is utilized (e.g., QIAamp Circulating Nucleic Acid Kit from Qiagen, Hilden, Germany). In some embodiments, isolated RNA fragments can be converted into complementary DNA for further downstream analysis.

In some embodiments, a biopsy (e.g., a liquid biopsy) is extracted prior to any indication of cancer. In some embodiments, a biopsy is extracted to provide an early screen in order to detect a cancer. In some embodiments, a biopsy is extracted to detect if residual cancer exists after a treatment. In some embodiments, a biopsy is extracted during treatment to determine whether the treatment is providing the desired response. Screening of any particular cancer can be performed. In some embodiments, screening is performed to detect a cancer that develops somatic phased variants in stereotypical regions in the genome, such as (for example) lymphoma. In some embodiments, screening is performed to detect a cancer in which somatic phased variants were discovered utilizing a prior extracted cancer biopsy.

In some embodiments, a biopsy is extracted from an individual with a determined risk of developing cancer, such as those with a familial history of the disorder or have determined risk factors (e.g., exposure to carcinogens). In many embodiments, a biopsy is extracted from any individual within the general population. In some embodiments, a biopsy is extracted from individuals within a particular age group with higher risk of cancer, such as, for example, aging individuals above the age of 50. In some embodiments, a biopsy is extracted from an individual diagnosed with and treated for a cancer.

In some embodiments, extracted cell-free nucleic acids are prepared for sequencing. Accordingly, cell-free nucleic acids are converted into a molecular library for sequencing. In some embodiments, adapters and/or primers are attached onto cell-free nucleic acids to facilitate sequencing. In some embodiments, targeted sequencing of particular genomic loci is to be performed, and thus particular sequences corresponding to the particular loci are captured via hybridization prior to sequencing (e.g., capture sequencing). In some embodiments, capture sequencing is performed utilizing a set of probes that pull down (or capture) regions that have been discovered to commonly harbor phased variants for a particular cancer (e.g., lymphoma). In some embodiments, capture sequencing is performed utilizing a set of probes that pull down (or capture) regions that have been discovered to harbor phased variants as determined prior by sequencing a biopsy of the cancer. More detailed discussion of capture sequencing and probes is provided in the section entitled "Capture Sequencing."

In some embodiments, any appropriate sequencing technique can be utilized that can detect phased variants indicative of circulating-tumor nucleic acids. Sequencing techniques include (but are not limited to) 454 sequencing, Illumina sequencing, SOLiD sequencing, Ion Torrent sequencing, single-read sequencing, paired-end sequencing, etc.

Process 2400 analyzes (2403) the cell-free nucleic acid sequencing result to detect circulating-tumor nucleic acid sequences, as determined by detection of somatic variants occurring in phase. Because cancers are actively growing and expanding, neoplastic cells are often releasing biomolecules (especially nucleic acids) into the vasculature, lymph, and/or waste systems. In addition, due to biophysical constraints in their local environment, neoplastic cells are often rupturing, releasing their inner cell contents into the vasculature, lymph, and/or waste systems. Accordingly, it is possible to detect distal primary tumors and/or metastases from a liquid or waste biopsy.

Detection of circulating-tumor nucleic acid sequences indicates that a cancer is present in the individual being examined. Accordingly, based on detection of circulating-tumor nucleic acids, a clinical intervention and/or treatment may be performed (2405). In a number of embodiments, a clinical procedure is performed, such as (for example) a blood test, genetic test, medical imaging, physical exam, a tumor biopsy, or any combination thereof. In several embodiments, diagnostics are preformed to determine the particular stage of cancer. In a number of embodiments, a treatment is performed, such as (for example) chemotherapy, radiotherapy, chemoradiotherapy, immunotherapy, hormone therapy, targeted drug therapy, surgery, transplant, transfusion, medical surveillance, or any combination thereof. In some embodiments, an individual is assessed and/or treated by medical professional, such as a doctor, physician, physician's assistant, nurse practitioner, nurse, caretaker, dietician, or similar.

Various embodiments of the present disclosure are directed towards utilizing detection of cancer to perform clinical interventions. In a number of embodiments, an individual has a liquid or waste biopsy screened and processed by methods described herein to indicate that the individual has cancer and thus an intervention is to be performed. Clinical interventions include clinical procedures and treatments. Clinical procedures include (but are not limited to) blood tests, genetic test, medical imaging, physical exams, and tumor biopsies. Treatments include (but are not limited to) chemotherapy, radiotherapy, chemoradiotherapy, immunotherapy, hormone therapy, targeted drug therapy, surgery, transplant, transfusion, and medical surveillance. In several embodiments, diagnostics are performed to determine the particular stage of cancer. In some embodiments, an individual is assessed and/or treated by medical professional, such as a doctor, physician, physician's assistant, nurse practitioner, nurse, caretaker, dietician, or similar.

In several embodiments as described herein a cancer can be detected utilizing a sequencing result of cell-free nucleic acids derived from blood, serum, cerebrospinal fluid, lymph fluid, urine or stool. In many embodiments, cancer is detected when a sequencing result has one or more somatic variants present in phase within a short genetic window, such as the length of a cell-free molecule (e.g., about 170 bp). In numerous embodiments, a statistical method is utilized to determine whether the presence of phased variants is derived from a cancerous source (as opposed to molecular artifact or other biological source). Various embodiments utilize a Monte Carlo sampling method as the statistical method to determine whether a sequencing result of cell-free nucleic acids includes sequences of circulating-tumor nucleic acids based on a score as determined by the presence of phased variants. Accordingly, in a number of embodiments, cell-free nucleic acids are extracted, processed, and sequenced, and the sequencing result is analyzed to detect cancer. This process is especially useful in a clinical setting to provide a diagnostic scan.

An exemplary procedure for a diagnostic scan of an individual for a B-cell cancer is as follows:

 (a) extract liquid or waste biopsy from individual,
 (b) prepare and perform targeted sequencing of cell-free nucleic acids from biopsy utilizing nucleic acid probes specific for the B-cell cancer,
 (c) detect phased variants in a sequencing result that are indicative of circulating-tumor nucleic acid sequences, and
 (d) perform clinical intervention based on detection of circulating-tumor nucleic acid sequences.

An exemplary procedure for a personalized diagnostic scan of an individual for a cancer that has been previously sequenced to detect phased variants in particular genomic loci is as follows:

 (a) design and synthesize nucleic acid probes for genomic loci that include the positions of the detected phased variants,
 (b) extract liquid or waste biopsy from individual,
 (c) prepare and perform targeted sequencing of cell-free nucleic acids from biopsy utilizing the designed and synthesized nucleic acid probes,
 (d) detect phased variants in a sequencing result that are indicative of circulating-tumor nucleic acid sequences, and
 (e) perform clinical intervention based on detection of circulating-tumor nucleic acid sequences.

In some embodiments of any one of the methods disclosed herein, at least a portion of the identified one or more cell-free nucleic acid molecules comprising the plurality of phased variants can be further analyzed for determining the condition of the subject. In such analysis, (i) the identified one or more cell-free nucleic acid molecules and (ii) other cell-free nucleic acid molecules of the plurality of cell-free nucleic acid molecules that do not comprise the plurality of phased variants can be analyzed as different variables. In some cases, a ratio of (i) a number the identified one or more cell-free nucleic acid molecules and (ii) a number of the other cell-free nucleic acid molecules of the plurality of cell-free nucleic acid molecules that do not comprise the plurality of phased variants can be used a factor to determine the condition of the subject. In some cases, comparison of (i) a position(s) of the identified one or more cell-free nucleic acid molecules relative to the reference genomic sequence and (ii) a position(s) of the other cell-free nucleic acid molecules of the plurality of cell-free nucleic acid molecules that do not comprise the plurality of phased variants relative to the reference genomic sequence can be used a factor to determine the condition of the subject.

Alternatively, in some cases, the analysis of the identified one or more cell-free nucleic acid molecules comprising the plurality of phased variants for determining the condition of the subject may not and need not be based on the other cell-free nucleic acid molecules of the plurality of cell-free nucleic acid molecules that do not comprise the plurality of phased variants. As disclosed herein, non-limiting examples of information or characteristics of the one or more cell-free nucleic acid molecules comprising the plurality of phased variants can include (i) a total number of such cell-free nucleic acid molecules and (ii) an average number of the plurality of phased variations per each nucleic acid molecule in the population of identified cell-free nucleic acid molecules.

Thus, in some embodiments of any one of the methods disclosed herein, a number of the plurality of phased variants from the one or more cell-free nucleic acid molecules that have been identified to have the plurality of phased variants can be indicative of the condition of the subject. In some cases, a ratio of (i) the number of the plurality of phased variants from the one or more cell-free nucleic acid molecules and (ii) a number of single nucleotide variants from the one or more cell-free nucleic acid molecules can be indicative of the condition of the subject. For instance, a particular condition (e.g., follicular lymphoma) can exhibit a signature ratio that is different than that of another condition (e.g., breast cancer). In some examples, for cancer or solid tumor, the ratio as disclosed herein can be between about 0.01 and about 0.20. In some examples, for cancer or solid tumor, the ratio as disclosed herein can be about 0.01, about 0.02, about 0.03, about 0.04, about 0.05, about 0.06, about 0.07, about 0.08, about 0.09, about 0.10, about 0.11, about 0.12, about 0.13, about 0.14, about 0.15, about 0.16, about 0.17, about 0.18, about 0.19, or about 0.20. In some examples, for cancer or solid tumor, the ratio as disclosed herein can be at least or up to about 0.01, at least or up to about 0.02, at least or up to about 0.03, at least or up to about 0.04, at least or up to about 0.05, at least or up to about 0.06, at least or up to about 0.07, at least or up to about 0.08, at least or up to about 0.09, at least or up to about 0.10, at least or up to about 0.11, at least or up to about 0.12, at least or up to about 0.13, at least or up to about 0.14, at least or up to about 0.15, at least or up to about 0.16, at least or up to about 0.17, at least or up to about 0.18, at least or up to about 0.19, or at least or up to about 0.20.

In some embodiments of any one of the methods disclosed herein, a frequency of the plurality of phased variants in the one or more cell-free nucleic acid molecules that have been identified can be indicative of the condition of the subject. In some cases, based on the sequencing data disclosed herein, an average frequency of the plurality of phased variant per a predetermined bin length (e.g., a bin of about 50 base pairs) within each of the identified cell-free nucleic acid molecule can be indicative of the condition of the subject. In some cases, based on the sequencing data disclosed herein, an average frequency of the plurality of phased variant per a predetermined bin length (e.g., a bin of about 50 base pairs) within each of the identified cell-free nucleic acid molecule that is associated with a particular gene (e.g., BCL2, PIM1) can be indicative of the condition of the subject. The size of the bin can be about 30, about 40, about 50, about 60, about 70, or about 80.

In some examples, a first condition (e.g., Hodgkin lymphoma or HL) can exhibit a first average frequency and a second condition (e.g., DLBCL) can exhibit a different average frequency, thereby allowing identification and/or determination of whether the subject has or is suspected of having a particular condition. In some examples, a first sub-type of a disease can exhibit a first average frequency and a second sub-type of the same disease can exhibit a different average frequency, thereby allowing identification and/or determination of whether the subject has or is suspected of having a particular sub-type of the disease. For example, the subject can have DLBCL, and one or more cell-free nucleic acid molecules derived from germinal center B-cell (GCB) DLBCL or activated B-cell (ABC) DLBCL can have different average frequency of the plurality of phased variant per a predetermined bin length, as disclosed herein.

In some example, a condition of the subject may have a predetermined number of phased variants spanning predetermined genomic loci (i.e., a predetermined frequency of phased variants). When the predetermined frequency of phased variants match a frequency of the plurality of phased variants in the one or more cell-free nucleic acid molecules that have been identified from a plurality of cell-free nucleic acid molecules from the subject, it may indicate that the subject has such condition.

In some embodiments of any one of the methods disclosed herein, the one or more cell-free nucleic acid molecules identified to comprise the plurality of phased variants can be analyzed to determine their genomic origin (e.g., which gene locus they are from). The genomic origin of the one or more cell-free nucleic acid molecules that have been identified can be indicative of the condition of the subject, as different disease can have the plurality of phased variants in different signature genes. For example, a subject can have GCB DLBCL, and one or more cell-free nucleic acid molecules originated from GCBs of the subject can have the phased variants prevalent in BCL2 gene, while one or more cell-free nucleic acid molecules originated from ABCs of the same subject may not comprise as many phased variants in the BCL2 gene as those from GCBs. On the other hand, a subject can have ABC DLBCL, and one or more cell-free nucleic acid molecules originated from ABCs of the subject can have the phased variants prevalent in PIM1 gene, while one or more cell-free nucleic acid molecules originated from GCBs of the same subject may not comprise as many phased variants in the PIM1 gene as those from ABCs.

In some embodiments of any one of the methods disclosed herein, at least or up to about 10%, at least or up to about 15%, at least or up to about 20%, at least or up to about 25%, at least or up to about 30%, at least or up to about 35%, at least or up to about 40%, at least or up to about 45%, at least or up to about 50%, at least or up to about 55%, at least or up to about 60%, at least or up to about 65%, at least or up to about 70%, at least or up to about 75%, at least or up to about 80%, at least or up to about 85%, at least or up to about 90%, at least or up to about 95%, at least or up to about 99%, or about 100% of the one or more cell-free nucleic acid molecules comprising the plurality of phased variants can comprise a single nucleotide variant (SNV) that is at least 2 nucleotides away from an adjacent SNV.

In some embodiments of any one of the methods disclosed herein, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, or at least or up to about 50% of the one or more cell-free nucleic acid molecules comprising the plurality of phased variants can comprise a single nucleotide variant (SNV) that is at least 3 nucleotides away from an adjacent SNV.

In some embodiments of any one of the methods disclosed herein, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, or at least or up to about 50% of the one or more cell-free nucleic acid molecules comprising the plurality of phased variants can comprise a single nucleotide variant (SNV) that is at least 4 nucleotides away from an adjacent SNV.

In some embodiments of any one of the methods disclosed herein, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, or at least or up to about 50% of the one or more cell-free nucleic acid molecules comprising the plurality of phased variants can comprise a single nucleotide variant (SNV) that is at least 5 nucleotides away from an adjacent SNV.

In some embodiments of any one of the methods disclosed herein, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, or at least or up to about 50% of the one or more cell-free nucleic acid molecules comprising the plurality of phased variants can comprise a single nucleotide variant (SNV) that is at least 6 nucleotides away from an adjacent SNV.

In some embodiments of any one of the methods disclosed herein, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, or at least or up to about 50% of the one or more cell-free nucleic acid molecules comprising the plurality of phased variants can comprise a single nucleotide variant (SNV) that is at least 7 nucleotides away from an adjacent SNV.

In some embodiments of any one of the methods disclosed herein, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, or at least or up to about 50% of the one or more cell-free nucleic acid molecules comprising the plurality of phased variants can comprise a single nucleotide variant (SNV) that is at least 8 nucleotides away from an adjacent SNV.

In some embodiments of any one of the methods disclosed herein, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, or at least or up to about 50% of the one or more cell-free nucleic acid molecules comprising the plurality of phased variants can comprise a single nucleotide variant (SNV) that is at least 9 nucleotides away from an adjacent SNV.

In some embodiments of any one of the methods disclosed herein, at least or up to about 5%, at least or up to about 10%, at least or up to about 15%, at least or up to about 20%, at least or up to about 25%, at least or up to about 30%, at least or up to about 35%, at least or up to about 40%, at least or up to about 45%, or at least or up to about 50% of the one or more cell-free nucleic acid molecules comprising the plurality of phased variants can comprise a single nucleotide variant (SNV) that is at least 10 nucleotides away from an adjacent SNV.

**C. Reference genomic sequence**

In some embodiments of any one of the methods disclosed herein, the reference genomic sequence can be at least a portion of a nucleic acid sequence database (i.e., a reference genome), which database is assembled from genetic data and intended to represent the genome of a reference cohort. In some cases, a reference cohort can be a collection of individuals from a specific or varying genotype, haplotype, demographics, sex, nationality, age, ethnicity, relatives, physical condition (e.g., healthy or having been diagnosed to have the same or different condition, such as a specific type of cancer), or other groupings. A reference genomic sequence as disclosed herein can be a mosaic (or a consensus sequence) of the genomes of two or more individuals. The reference genomic sequence can comprise at least a portion of a publicly available reference genome or a private reference genome. Non-limiting examples of a human reference genome include hgl9, hg18, hg17, hg16, and hg38.

In some examples, the reference genomic sequence can comprise at least or up to about 500 nucleobases, at least or up to about 1 kilobase (kb), at least or up to about 2 kb, at least or up to about 3 kb, at least or up to about 4 kb, at least or up to about 5 kb, at least or up to about 6 kb, at least or up to about 7 kb, at least or up to about 8 kb, at least or up to about 9 kb, at least or up to about 10 kb, at least or up to about 20 kb, at least or up to about 30 kb, at least or up to about 40 kb, at least or up to about 50 kb, at least or up to about 60 kb, at least or up to about 70 kb, at least or up to about 80 kb, at least or up to about 90 kb, at least or up to about 100 kb, at least or up to about 200 kb, at least or up to about 300 kb, at least or up to about 400 kb, at least or up to about 500 kb, at least or up to about 600 kb, at least or up to about 700 kb, at least or up to about 800 kb, at least or up to about 900 kb, at least or up to about 1,000 kb, at least or up to about 2,000 kb, at least or up to about 3,000 kb, at least or up to about 4,000 kb, at least or up to about 5,000 kb, at least or up to about 6,000 kb, at least or up to about 7,000 kb, at least or up to about 8,000 kb, at least or up to about 9,000 kb, at least or up to about 10,000 kb, at least or up to about 20,000 kb, at least or up to about 30,000 kb, at least or up to about 40,000 kb, at least or up to about 50,000 kb, at least or up to about 60,000 kb, at least or up to about 70,000 kb, at least or up to about 80,000 kb, at least or up to about 90,000 kb, or at least or up to about 100,000 kb.

In some cases, the reference genomic sequence can be whole reference genome or a portion (e.g., a portion relevant to the condition of interest) of the genome. For example, the reference genomic sequence can consist of at least 1, 2, 3, 4, 5, or more genes that experience aberrant somatic hypermutation under certain types of cancer. In some cases, the reference genomic sequence can be a whole chromosomal sequence, or a fragment thereof. In some cases, the reference genomic sequence can comprise two or more (e.g., at least 2, 3, 4, 5, or more) different portions of the reference genome that are not adjacent to one another (e.g., within the same chromosome or from different chromosomes).

In some embodiments of any one of the methods disclosed herein, the reference genomic sequence can be at least a portion of a reference genome of a selected individual, such as a healthy individual or the subject of any of the methods as disclosed herein.

In some cases, the reference genomic sequence can be derived from an individual who is not the subject (e.g., a healthy control individual). Alternatively, in some cases, the reference genomic sequence can be derived from a sample of the subject. In some examples, the sample can be a healthy sample of the subject. The healthy sample of the subject can be any subject cell that is healthy, e.g., a healthy leukocyte. By comparing sequencing data of the plurality of cell-free nucleic acid molecules (e.g., cfDNA molecules) of the subject against at least a portion of the genomic sequence of a healthy cell of the same subject, one or more cell-free nucleic acid molecules that comprise the plurality of phased variants can be identified and analyzed, as disclosed herein. In some examples, the sample can be a diseased sample of the subject, such as a diseased cell (e.g., a tumor cell) or a solid tumor. The reference genomic sequence can be obtained from sequencing at least a portion of a diseased cell of the subject or from sequencing a plurality of cell-free nucleic acid molecules obtained from the solid tumor of the subject. Once the subject is diagnosed to have a particular condition (e.g., a disease), the reference genomic sequence of the subject that comprises the plurality of phased variants can be used to determine whether the subject still exhibits the same phased variants at future time points. In this context, any new phased variants identified between the "diseased" reference genomic sequence of the subject and new cell-free nucleic acid molecules obtained or derived from the subject can indicate a reduced degree of aberrant somatic hypermutation in particular genomic loci (e.g., at least a partial remission).

In various embodiments, diagnostic scans can be performed for any neoplasm type, including (but not limited to) acute lymphoblastic leukemia (ALL), acute myeloid leukemia (AML), anal cancer, astrocytomas, basal cell carcinoma, bile duct cancer, bladder cancer, breast cancer, Burkitt's lymphoma, cervical cancer, chronic lymphocytic leukemia (CLL) chronic myelogenous leukemia (CML), chronic myeloproliferative neoplasms, colorectal cancer, diffuse large B-cell lymphoma, endometrial cancer, ependymoma, esophageal cancer, esthesioneuroblastoma, Ewing sarcoma, fallopian tube cancer, follicular lymphoma, gallbladder cancer, gastric cancer, gastrointestinal carcinoid tumor, hairy cell leukemia, hepatocellular cancer, Hodgkin lymphoma, hypopharyngeal cancer, Kaposi sarcoma, Kidney cancer, Langerhans cell histiocytosis, laryngeal cancer, leukemia, liver cancer, lung cancer, lymphoma, melanoma, Merkel cell cancer, mesothelioma, mouth cancer, neuroblastoma, non-Hodgkin lymphoma, non-small cell lung cancer, osteosarcoma, ovarian cancer, pancreatic cancer, pancreatic neuroendocrine tumors, pharyngeal cancer, pituitary tumor, prostate cancer, rectal cancer, renal cell cancer, retinoblastoma, skin cancer, small cell lung cancer, small intestine cancer, squamous neck cancer, T-cell lymphoma, testicular cancer, thymoma, thyroid cancer, uterine cancer, vaginal cancer, and vascular tumors.

In a number of embodiments, a diagnostic scan is utilized to provide an early detection of cancer. In some embodiments, a diagnostic scan detects cancer in individuals having stage I, II, or III cancer. In some embodiments, a diagnostic scan is utilized to detect MRD or tumor burden. In some embodiments, a diagnostic scan is utilized to determine progress (e.g., progression or regression) of treatment. Based on the diagnostic scan, a clinical procedure and/or treatment may be performed.

## D. Nucleic acid probes

In some embodiments of any one of the methods disclosed herein, the set of nucleic acid probes can be designed based on the any of the subject reference genomic sequences of the present disclosure. In some cases, the set of nucleic acid probes can be designed based on the plurality of phased variants that have been identified by comparing (i) sequencing data from a solid tumor of the subject and (ii) sequencing data from a healthy cell of the subject or a healthy cohort, as disclosed herein. The set of nucleic acid probes can be designed based on the plurality of phased variants that have been identified by comparing (i) sequencing data from a solid tumor of the subject and (ii) sequencing data from a healthy cell of the subject. The set of nucleic acid probes can be designed based on the plurality of phased variants that have been identified by comparing (i) sequencing data from a solid tumor of the subject and (ii) sequencing data from a healthy cell of a healthy cohort.

In some embodiments of any one of the methods disclosed herein, the set of nucleic acid probes are designed to hybridize to sequences of genomic loci associated with the condition. As disclosed herein, the genomic loci associated with the condition can be determined to experience or exhibit aberrant somatic hypermutation when the subject has the condition. Alternatively, the set of nucleic acid probes are designed to hybridize to sequences of stereotyped regions.

In some embodiments of any one of the methods disclosed herein, the set of nucleic acid probes can be designed to hybridize to at least about 5%, at least about 10%, at least about 20%, at least about 30%, at least about 40%, at least about 50%, at least about 60%, at least about 70%, at least about 80%, at least about 90%, at least about 95%, at least about 99%, or about 100% of the genomic regions identified in Table 1.

In some embodiments of any one of the methods disclosed herein, the set of nucleic acid probes can be designed to hybridize to at least a portion of cell-free nucleic acid (e.g., cfDNA) molecules derived from at least about 5%, at least about 10%, at least about 20%, at least about 30%, at least about 40%, at least about 50%, at least about 60%, at least about 70%, at least about 80%, at least about 90%, at least about 95%, at least about 99%, or about 100% of the genomic regions identified in Table 1.

In some embodiments of any one of the methods disclosed herein, each nucleic acid probe of the set of nucleic acid probes can have at least about 50%, at least about 55%, at least about 60%, at least about 65%, at least about 70%, at least about 75%, at least about 80%, at least about 85%, at least about 90% sequence identity, at least about 95% sequence identity, at least about 99%, or about 100% sequence identity to a probe sequence selected from Table 6.

In some embodiments of any one of the methods disclosed herein, the set of nucleic acid probes can comprise at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or about 100% of probe sequences in Table 6.

In some embodiments of any one of the methods disclosed herein, the set of nucleic acid probes can be designed to cover one or more target genomic regions comprising at least or up to about 500 nucleobases, at least or up to about 1 kilobase (kb), at least or up to about 2 kb, at least or up to about 3 kb, at least or up to about 4 kb, at least or up to about 5 kb, at least or up to about 6 kb, at least or up to about 7 kb, at least or up to about 8 kb, at least or up to about 9 kb, at least or up to about 10 kb, at least or up to about 20 kb, at least or up to about 30 kb, at least or up to about 40 kb, at least or up to about 50 kb, at least or up to about 60 kb, at least or up to about 70 kb, at least or up to about 80 kb, at least or up to about 90 kb, at least or up to about 100 kb, at least or up to about 200 kb, at least or up to about 300 kb, at least or up to about 400 kb, or at least or up to about 500 kb.

In some embodiments of any one of the methods disclosed herein, a target genomic region (e.g., a target genomic locus) of the one or more target genomic regions can comprise at most about 200 nucleobases, at most about 300 nucleobases, 400 nucleobases, at most about 500 nucleobases, at most about 600 nucleobases, at most about 700 nucleobases, at most about 800 nucleobases, at most about 900 nucleobases, at most about 1 kb, at most about 2 kb, at most about 3 kb, at most about 4 kb, at most about 5 kb, at most about 6 kb, at most about 7 kb, at most about 8 kb, at most about 9 kb, at most about 10 kb, at most about 11 kb, at most about 12 kb, at most about 13 kb, at most about 14 kb, at most about 15 kb, at most about 16 kb, at most about 17 kb, at most about 18 kb, at most about 19 kb, at most about 20 kb, at most about 25 kb, at most about 30 kb, at most about 35 kb, at most about 40 kb, at most about 45 kb, at most about 50 kb, or at most about 100 kb.

In some embodiments of any one of the methods disclosed herein, the set of nucleic acid probes can comprise at least or up to about 10, at least or up to about 20, at least or up to about 30, at least or up to about 40, at least or up to about 50, at least or up to about 60, at least or up to about 70, at least or up to about 80, at least or up to about 90, at least or up to about 100, at least or up to about 200, at least or up to about 300, at least or up to about 400, at least or up to about 500, at least or up to about 600, at least or up to about 700, at least or up to about 800, at least or up to about 900, at least or up to about 1,000, at least or up to about 2,000, at least or up to about 3,000, at least or up to about 4,000, or at least or up to about 5,000 different nucleic acid probes designed to hybridize to different target nucleic acid sequences.

In some embodiments of any one of the methods disclosed herein, the set of nucleic acid probes can have a length of at least or up to about 50, at least or up to about 55, at least or up to about 60, at least or up to about 65, at least or up to about 70, at least or up to about 75, at least or up to about 80, at least or up to about 85, at least or up to about 90, at least or up to about 95, or at least or up to about 100 nucleotides.

In one aspect, the present disclosure provides a composition comprising a bait set comprising any one of the set of nucleic acid probes disclosed herein. The composition comprising such bait set can be used for any of the methods disclosed herein. In some cases, the set of nucleic acid probes can be designed to pull down (or capture) cfDNA molecules. In some cases, the set of nucleic acid probes can be designed to pull down (or capture) cfRNA molecules.

In some embodiments, the bait set can comprise a set of nucleic acid probes designed to pull down cell-free nucleic acid (e.g., cfDNA) molecules derived from genomic regions set forth in Table 1. The set of nucleic acid probes can be designed to pull down cell-free nucleic acid molecules derived from at least or up to about 1%, at least or up to about 2%, at least or up to about 3%, at least or up to about 4%, at least or up to about 5%, at least or up to about 6%, at least or up to about 7%, at least or up to about 8%, at least or up to about 9%, at least or up to about 10%, at least or up to about 15%, at least or up to about 20%, at least or up to about 25%, at least or up to about 30%, at least or up to about 35%, at least or up to about 40%, at least or up to about 45%, at least or up to about 50%, at least or up to about 55%, at least or up to about 60%, at least or up to about 65%, at least or up to about 70%, at least or up to about 75%, at least or up to about 80%, at least or up to about 85%, at least or up to about 90%, at least or up to about 95%, at least or up to about 99%, or about 100% of the genomic regions set forth in Table 1. In some cases, the set of nucleic acid probes can be designed to pull down cfDNA molecules. In some cases, the set of nucleic acid probes can be designed to pull down cfRNA molecules.

In some embodiments of any one of the compositions disclosed herein, an individual nucleic acid probe (or each nucleic acid probe) of the set of nucleic acid probes can comprise a pull-down tag. The pull-down tag can be used to enrich a sample (e.g., a sample comprising the plurality of nucleic acid molecules obtained or derived from the subject) for a specific subset (e.g., for cell-free nucleic acid molecules comprising the plurality of phased variants as disclosed herein).

In some cases, pull-down tag can comprise a nucleic acid barcode (e.g., on either or both sides of the nucleic acid probe). By utilizing beads or substrates comprising nucleic acid sequences having complementarity to the nucleic acid barcode, the nucleic acid barcode can be used to pull-down and enrich for any nucleic acid probe that is hybridized to a target cell-free nucleic acid molecule. Alternatively, or in addition to, the nucleic acid barcode can be used to identify the target cell-free nucleic acid molecule from any sequencing data (e.g., sequencing by amplification) obtained by using any of the set of nucleic acid probes disclosed herein.

In some cases, the pull-down tag can comprise an affinity target moiety that can be specifically recognized and bound by an affinity binding moiety. The affinity binding moiety specifically can bind the affinity target moiety to form an affinity pair. In some cases, by utilizing beads or substrates comprising the affinity binding moiety, the affinity target moiety can be used to pull-down and enrich for any nucleic acid probe that is hybridized to a target cell-free nucleic acid molecule. Alternatively, the pull-down tag can comprise the affinity binding moiety, while the beads/substrates can comprise the affinity target moiety. Non-limiting examples of the affinity pair can include biotin/avidin, antibody/antigen, biotin/streptavidin, metal/chelator, ligand/receptor, nucleic acid and binding protein, and complementary nucleic acids. In an example, the pull-down tag can comprise biotin.

In some embodiments of any one of the compositions disclosed herein, a length of a target cell-free nucleic acid (e.g., cfDNA) molecule that is to be pulled down by any subject nucleic acid probe can be about 100 nucleotides to about 200 nucleotides. The length of the target cell-free nucleic acid molecule can be at least about 100 nucleotides. The length of the target cell-free nucleic acid molecule can be at most about 200 nucleotides. The length of the target cell-free nucleic acid molecule can be about 100 nucleotides to about 110 nucleotides, about 100 nucleotides to about 120 nucleotides, about 100 nucleotides to about 130 nucleotides, about 100 nucleotides to about 140 nucleotides, about 100 nucleotides to about 150 nucleotides, about 100 nucleotides to about 160 nucleotides, about 100 nucleotides to about 170 nucleotides, about 100 nucleotides to about 180 nucleotides, about 100 nucleotides to about 190 nucleotides, about 100 nucleotides to about 200 nucleotides, about 110 nucleotides to about 120 nucleotides, about 110 nucleotides to about 130 nucleotides, about 110 nucleotides to about 140 nucleotides, about 110 nucleotides to about 150 nucleotides, about 110 nucleotides to about 160 nucleotides, about 110 nucleotides to about 170 nucleotides, about 110 nucleotides to about 180 nucleotides, about 110 nucleotides to about 190 nucleotides, about 110 nucleotides to about 200 nucleotides, about 120 nucleotides to about 130 nucleotides, about 120 nucleotides to about 140 nucleotides, about 120 nucleotides to about 150 nucleotides, about 120 nucleotides to about 160 nucleotides, about 120 nucleotides to about 170 nucleotides, about 120 nucleotides to about 180 nucleotides, about 120 nucleotides to about 190 nucleotides, about 120 nucleotides to about 200 nucleotides, about 130 nucleotides to about 140 nucleotides, about 130 nucleotides to about 150 nucleotides, about 130 nucleotides to about 160 nucleotides, about 130 nucleotides to about 170 nucleotides, about 130 nucleotides to about 180 nucleotides, about 130 nucleotides to about 190 nucleotides, about 130 nucleotides to about 200 nucleotides, about 140 nucleotides to about 150 nucleotides, about 140 nucleotides to about 160 nucleotides, about 140 nucleotides to about 170 nucleotides, about 140 nucleotides to about 180 nucleotides, about 140 nucleotides to about 190 nucleotides, about 140 nucleotides to about 200 nucleotides, about 150 nucleotides to about 160 nucleotides, about 150 nucleotides to about 170 nucleotides, about 150 nucleotides to about 180 nucleotides, about 150 nucleotides to about 190 nucleotides, about 150 nucleotides to about 200 nucleotides, about 160 nucleotides to about 170 nucleotides, about 160 nucleotides to about 180 nucleotides, about 160 nucleotides to about 190 nucleotides, about 160 nucleotides to about 200 nucleotides, about 170 nucleotides to about 180 nucleotides, about 170 nucleotides to about 190 nucleotides, about 170 nucleotides to about 200 nucleotides, about 180 nucleotides to about 190 nucleotides, about 180 nucleotides to about 200 nucleotides, or about 190 nucleotides to about 200 nucleotides. The length of the target cell-free nucleic acid molecule can be about 100 nucleotides, about 110 nucleotides, about 120 nucleotides, about 130 nucleotides, about 140 nucleotides, about 150 nucleotides, about 160 nucleotides, about 170 nucleotides, about 180 nucleotides, about 190 nucleotides, or about 200 nucleotides. In some examples, the length of the target cell-free nucleic acid molecule can range between about 100 nucleotides and about 180 nucleotides.

In some embodiments of any one of the compositions disclosed herein, the genomic regions can be associated with a condition. The genomic regions can be determined to exhibit aberrant somatic hypermutation when a subject has the condition. For example, the condition can comprise B-cell lymphoma or a sub-type thereof, such as diffuse large B-cell lymphoma, follicular lymphoma, Burkitt lymphoma, and B-cell chronic lymphocytic leukemia. Additional details of the condition are provided below.

In some embodiments of any one of the compositions disclosed herein, the composition further comprises the plurality of cell-free nucleic acid (e.g., cfDNA) molecules obtained or derived from the subject.

## E. Diagnostic or therapeutic applications

A number of embodiments are directed towards performing a diagnostic scan on cell-free nucleic acids of an individual and then based on results of the scan indicating cancer, performing further clinical procedures and/or treating the individual. In accordance with various embodiments, numerous types of neoplasms can be detected.

In some embodiments of any one of the methods disclosed herein, the method can comprise determining that the subject has the condition or determining a degree or status of the condition of the subject, based on the one or more cell-free nucleic acid molecules comprising the plurality of phased variants. In some cases, the method can further comprise determining that the one or more cell-free nucleic acid molecules (each identified to comprise a plurality of phased variants) are derived from a sample associated with the condition (e.g., cancer), based on a statistical model analysis (i.e., molecular analysis). For example, the method can comprise using one or more algorithms (e.g., Monte Carlos simulation) to determine a first probability of a cell-free nucleic acid identified to have a plurality of phased variants being associated with or originated from a first condition (e.g., 80%) and a second probability of the same cell-free nucleic acid being associated with or originated from a second condition (or from a healthy cell) (e.g., 20%). In some cases, the method can comprise determining a likelihood or probability that the subject has one or more conditions based on analysis of the one or more cell-free nucleic acid molecules each identified to comprise a plurality of phased variants (i.e., macro- or global analysis). For example, the method can comprise using one or more algorithms (e.g., comprising one or more mathematical models as disclosed herein, such as binomial sampling) to analyze a plurality of cell-free nucleic acid molecules each identified to comprise a plurality of phased variants, thereby to determine a first probability of the subject having a first condition (e.g., 80%) and a second probability of the subject having a second condition (or being healthy) (e.g., 20%).

The statistical model analysis as disclosed herein can be an approximate solution by a numerical approximation such as a binomial model, a ternary model, a Monte Carlo simulation, or a finite difference method. In an example, the statistical model analysis as used herein can be a Monte Carlo statistical analysis. In another example, the statistical model analysis as used herein can be a binomial or ternary model analysis.

In some embodiments of any one of the methods disclosed herein, the method can comprise monitoring a progress of the condition of the subject based on the one or more cell-free nucleic acid molecules identified, such that each of the identified cell-free nucleic acid molecule comprises a plurality of phased variants. In some cases, the progress of the condition can be worsening of the condition, as described in the present disclosure (e.g., developing from stage I cancer to stage III cancer). In some cases, the progress of the condition can be at least a partial remission of the condition, as described in the present disclosure (e.g., downstaging from stage IV cancer to stage II cancer). Alternatively, in some cases, the progress of the condition can remain substantially the same between two different time points, as described in the present disclosure. In an example, the method can comprise determining likelihoods or probabilities of different progresses of the condition of the subject. For example, the method can comprise using one or more algorithms (e.g., comprising one or more mathematical models as disclosed herein, such as binomial sampling) to determine a first probability of the subject's condition being worse than before (e.g., 20%), a second probability of at least partial remission of the condition (e.g., 70%), and a third probability that the subject's condition is the same as before (e.g., 10%).

In some embodiments of any one of the methods disclosed herein, the method can comprise comprising performing a different procedure (e.g., follow-up diagnostic procedures) to confirm the condition of the subject, which condition has been determined and/or monitored progress thereof, as provided in the present disclosure. Non-limiting examples of a different procedure can include physical exam, medical imaging, genetic test, mammography, endoscopy, stool sampling, pap test, alpha-fetoprotein blood test, CA-125 test, prostate-specific antigen (PSA) test, biopsy extraction, bone marrow aspiration, and tumor marker detection tests. Medical imaging includes (but is not limited to) X-ray, magnetic resonance imaging (MRI), computed tomography (CT), ultrasound, and positron emission tomography (PET). Endoscopy includes (but is not limited to) bronchoscopy, colonoscopy, colposcopy, cystoscopy, esophagoscopy, gastroscopy, laparoscopy, neuroendoscopy, proctoscopy, and sigmoidoscopy.

In some embodiments of any one of the methods disclosed herein, the method can comprise determining a treatment for the condition of the subject based on the one or more cell-free nucleic acid molecules identified, each identified cell-free nucleic acid molecule comprising a plurality of phased variants. In some cases, the treatment can be determined based on (i) the determined condition of the subject and/or (ii) the determined progress of the condition of the subject. In addition, the treatment can be determined based on one or more additional factors of the following: sex, nationality, age, ethnicity, and other physical conditions of the subject. In some examples, the treatment can be determined based on one or more features of the plurality of phased variants of the identified cell-free nucleic acid molecules, as disclosed herein.

In some embodiments of any one of the methods disclosed herein, the subject may not have been subjected to any treatment for the condition, e.g., the subject may not have been diagnosed with the condition (e.g., a lymphoma). In some embodiments of any one of the methods disclosed herein, the subject may been subjected to a treatment for the condition prior to any subject method of the present disclosure. In some cases, the methods disclosed herein can be performed to monitor progress of the condition that the subject has been diagnosed with, thereby to (i) determine efficacy of the previous treatment and (ii) assess whether to keep the treatment, modify the treatment, or cancel the treatment in favor of a new treatment.

In some embodiments of any one of the methods disclosed herein, non-limiting examples of a treatment (e.g., prior treatment, new treatment to be determined based on the methods of the present disclosure, etc.) can include chemotherapy, radiotherapy, chemoradiotherapy, immunotherapy, adoptive cell therapy (e.g., chimeric antigen receptor (CAR) T cell therapy, CAR NK cell therapy, modified T cell receptor (TCR) T cell therapy, etc.) hormone therapy, targeted drug therapy, surgery, transplant, transfusion, or medical surveillance.

In some embodiments of any one of the methods disclosed herein, the condition can comprise a disease. In some embodiments of any one of the methods disclosed herein, the condition can comprise neoplasm, cancer, or tumor. In an example, the condition can comprise a solid tumor. In another example, the condition can comprise a lymphoma, such as B-cell lymphoma (BCL). Non-limiting examples of BCL can include diffuse large B-cell lymphoma (DLBCL), follicular lymphoma (FL), Burkitt lymphoma (BL), B-cell chronic lymphocytic leukemia (CLL), Marginal zone B-cell lymphoma (MZL), and Mantle cell lymphoma (MCL).

As disclosed herein, a treatment for a condition of subject can comprise administering the subject with one or more therapeutic agents. The one or more therapeutic drugs can be administered to the subject by one or more of the following: orally, intraperitoneally, intravenously, intraarterially, transdermally, intramuscularly, liposomally, via local delivery by catheter or stent, subcutaneously, intraadiposally, and intrathecally.

Non-limiting examples of the therapeutic drugs can include cytotoxic agents, chemotherapeutic agents, growth inhibitory agents, agents used in radiation therapy, anti-angiogenesis agents, apoptotic agents, anti-tubulin agents, and other agents to treat cancer, for example, anti-CD20 antibodies, anti-PD1 antibodies (e.g., Pembrolizumab) platelet derived growth factor inhibitors (e.g., GLEEVEC™ (imatinib mesylate)), a COX-2 inhibitor (e.g., celecoxib), interferons, cytokines, antagonists (e.g., neutralizing antibodies) that bind to one or more of the following targets PDGFR-$\beta$, BlyS, APRIL, BCMA receptor(s), TRAIL/Apo2, other bioactive and organic chemical agents, and the like.

Non-limiting examples of a cytotoxic agent can include radioactive isotopes (e.g., At211, I131, I125, Y90, Re186, Re188, Sm153, Bi212, P32, and radioactive isotopes of Lu), chemotherapeutic agents, e.g., methotrexate, adriamycin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin.

Non-limiting examples of a chemotherapeutic agent can include alkylating agents such as thiotepa and CYTOXAN® cyclophosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethiylenethiophosphoramide and trimethylolmelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cyclophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics; dynemicin, including dynemicin A; an espiramicina; as well as neocarzinostatin chromophore and related chromoprotein enediyne antibiotic chromophores), aclacinomycins, actinomycin, anthramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as folinic acid; aceglatone; aldophosphamide glycoside; aminolevulinic acid; eniluracil; amsacrine; bestrabucil; bisantrene; edatraxate; defofamine; demecolcine; diaziquone; eflomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verrucarin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, for example taxanes including TAXOL® paclitaxel (Bristol-Myers Squibb Oncology, Princeton, N.J.), ABRAXANE™ Cremophor-free, albumin-engineered nanoparticle formulation of paclitaxel (American Pharmaceutical Partners, Schaumberg, Ill.), and TAXOTERE® docetaxel (Rhône-Poulenc Rorer, Antony, France); chlorambucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovorin.

Examples of a chemotherapeutic agent can also include "anti-hormonal agents" or "endocrine therapeutics" that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), EVISTA® raloxifene, droloxifene, 4-hydroxytamoxifen, trioxifene, keoxifene, LY117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD) leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGFR); vaccines such as THERATOPE® vaccine and gene therapy vaccines, for example, ALLOVECTIN® vaccine, LEUVECTIN® vaccine, and VAXID® vaccine; LURTOTECAN® topoisomerase 1 inhibitor; ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

Examples of a chemotherapeutic agent can also include antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG®, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth). Additional humanized monoclonal antibodies with therapeutic potential as agents in combination with the compounds described herein include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, feMzumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories) which is a recombinant exclusively human-sequence, full-length IgG1λ antibody genetically modified to recognize interleukin-12 p40 protein.

Examples of a chemotherapeutic agent can also include "tyrosine kinase inhibitors" such as an EGFR-targeting agent (e.g., small molecule, antibody, etc.); small molecule HER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-1 inhibitors such as antisense agent ISIS-5132 available from ISIS Pharmaceuticals which inhibit Raf-1 signaling; non-HER targeted TK inhibitors such as imatinib mesylate (GLEEVEC®, available from Glaxo SmithKline); multi-targeted tyrosine kinase inhibitors such as sunitinib (SUTENT®, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Warner-Lamber); antisense molecules (e.g., those that bind to HER-encoding nucleic acid); quinoxalines (U.S. Pat. No. 5,804,396); tryphostins (U.S. Pat. No. 5,804,396); ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVEC®); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-1C11 (Imclone); and rapamycin (sirolimus, RAPAMUNE®).

Examples of a chemotherapeutic agent can also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Examples of a chemotherapeutic agent can also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone-17-butyrate, clobetasol-17-propionate, fluocortolone caproate, fluocortolone pivalate and fluprednidene acetate: immune selective anti-inflammatory peptides (ImSAIDs) such as phenylalanine-glutamine-glycine (FEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, tumor necrosis factor alpha (TNFα) blockers such as etanercept (ENBREL®), infliximab (REMICADE®), adalimumab (HUMIRA®), certolizumab pegol (CIMZIA®), golimumab (SIMPONI®), Interleukin 1 (IL-1) blockers such as anakinra (KINERET®), T-cell costimulation blockers such as abatacept (ORENCIA®), Interleukin 6 (IL-6) blockers such as tocilizumab (ACTEMERA®); Interleukin 13 (IL-13) blockers such as lebrikizumab; Interferon alpha (IFN) blockers such as rontalizumab; beta 7 integrin blockers such as rhuMAb Beta7; IgE pathway blockers such as Anti-M1 prime; Secreted homotrimeric LTa3 and membrane bound heterotrimer LTa/β2 blockers such as Anti-lymphotoxin alpha (LTa); miscellaneous investigational agents such as thioplatin, PS-341, phenylbutyrate, ET-18-OCH3, or famesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; delta-9-tetrahydrocannabinol (dronabinol, MARINOL®); beta-lapachone; lapachol; colchicines; betulinic acid; acetylcamptothecin, scopolectin, and 9-aminocamptothecin); podophyllotoxin; tegafur (UFTORAL®); bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); and epidermal growth factor receptor (EGF-R); vaccines such as THERATOPE® vaccine; perifosine, COX-2 inhibitor (e.g., celecoxib or etoricoxib), proteosome inhibitor (e.g., PS341); CCI-779; tipifarnib (R11577); orafenib, ABT510; Bcl-2 inhibitor such as oblimersen sodium (GENASENSE®); pixantrone; famesyltransferase inhibitors such as lonafamib (SCH 6636, SARASAR™); and pharmaceutically acceptable salts, acids or derivatives of any of the above; as well as combinations of two or more of the above.

In accordance with many embodiments, once a diagnosis of cancer is indicated, a number of treatments can be performed, including (but not limited to) surgery, resection, chemotherapy, radiation therapy, immunotherapy, targeted therapy, hormone therapy, stem cell transplant, and blood transfusion. In some embodiments, an anti-cancer and/or chemotherapeutic agent is administered, including (but not limited to) alkylating agents, platinum agents, taxanes, vinca agents, anti-estrogen drugs, aromatase inhibitors, ovarian suppression agents, endocrine/hormonal agents, bisphophonate therapy agents and targeted biological therapy agents. Medications include (but are not limited to) cyclophosphamide, fluorouracil (or 5-fluorouracil or 5-FU), methotrexate, thiotepa, carboplatin, cisplatin, taxanes, paclitaxel, protein-bound paclitaxel, docetaxel, vinorelbine, tamoxifen, raloxifene, toremifene, fulvestrant, gemcitabine, irinotecan, ixabepilone, temozolomide, topotecan, vincristine, vinblastine, eribulin, mutamycin, capecitabine, capecitabine, anastrozole, exemestane, letrozole, leuprolide, abarelix, buserelin, goserelin, megestrol acetate, risedronate, pamidronate, ibandronate, alendronate, zoledronate, tykerb, daunorubicin, doxorubicin, epirubicin, idarubicin, valrubicin mitoxantrone, bevacizumab, cetuximab, ipilimumab, ado-trastuzumab emtansine, afatinib, aldesleukin, alectinib, alemtuzumab, atezolizumab, avelumab, axtinib, belimumab, belinostat, bevacizumab, blinatumomab, bortezomib, bosutinib, brentuximab vedotin, brigatinib, cabozantinib, canakinumab, carfilzomib, certinib, cetuximab, cobimetinib, crizotinib, dabrafenib, daratumumab, dasatinib, denosumab, dinutuximab, durvalumab, elotuzumab, enasidenib, erlotinib, everolimus, gefitinib, ibritumomab tiuxetan, ibrutinib, idelalisib, imatinib, ipilimumab, ixazomib, lapatinib, lenvatinib, midostaurin, necitumumab, neratinib, nilotinib, niraparib, nivolumab, obinutuzumab, ofatumumab, olaparib, olaratumab, osimertinib, palbociclib, panitumumab, panobinostat, pembrolizumab, pertuzumab, ponatinib, ramucirumab, regorafenib, ribociclib, rituximab, romidepsin, rucaparib, ruxolitinib, siltuximab, sipuleucel-T, sonidegib, sorafenib, temsi rolimus, tocilizumab, tofacitinib, tositumomab, trametinib, trastuzumab, vandetanib, vemurafenib, venetoclax, vismodegib, vorinostat, and ziv-aflibercept. In accordance with various embodiments, an individual may be treated, by a single medication or a combination of medications described herein. A common treatment combination is cyclophosphamide, methotrexate, and 5-fluorouracil (CMF).

In some embodiments of any one of the methods disclosed herein, any of the cell-free nucleic acid molecules (e.g., cfDNA, cfRNA) can be derived from a cell. For example, a cell sample or tissue sample may be obtained from a subject and processed to remove all cells from the sample, thereby producing cell-free nucleic acid molecules derived from the sample.

In some embodiments of any one of the methods disclosed herein, a reference genomic sequence can be derived from a cell of an individual. The individual can be a healthy control or the subject who is being subjected to the methods disclosed herein for determining or monitoring progress of a condition.

A cell can be a healthy cell. Alternatively, a cell can be a diseased cell. A diseased cell can have altered metabolic, gene expression, and/or morphologic features. A diseased cell can be a cancer cell, a diabetic cell, and an apoptotic cell. A diseased cell can be a cell from a diseased subject. Exemplary diseases can include blood disorders, cancers, metabolic disorders, eye disorders, organ disorders, musculoskeletal disorders, cardiac disease, and the like.

A cell can be a mammalian cell or derived from a mammalian cell. A cell can be a rodent cell or derived from a rodent cell. A cell can be a human cell or derived from a human cell. A cell can be a prokaryotic cell or derived from a prokaryotic cell. A cell can be a bacterial cell or can be derived from a bacterial cell. A cell can be an archaeal cell or derived from an archaeal cell. A cell can be a eukaryotic cell or derived from a eukaryotic cell. A cell can be a pluripotent stem cell. A cell can be a plant cell or derived from a plant cell. A cell can be an animal cell or derived from an animal cell. A cell can be an invertebrate cell or derived from an invertebrate cell. A cell can be a vertebrate cell or derived from a vertebrate cell. A cell can be a microbe cell or derived from a microbe cell. A cell can be a fungi cell or derived from a fungi cell. A cell can be from a specific organ or tissue.

Non-limiting examples of a cell(s) can include lymphoid cells, such as B cell, T cell (Cytotoxic T cell, Natural Killer T cell, Regulatory T cell, T helper cell), Natural killer cell, cytokine induced killer (CIK) cells; myeloid cells, such as granulocytes (Basophil granulocyte, Eosinophil granulocyte, Neutrophil granulocyte/Hypersegmented neutrophil), Monocyte/Macrophage, Red blood cell (Reticulocyte), Mast cell, Thrombocyte/Megakaryocyte, Dendritic cell; cells from the endocrine system, including thyroid (Thyroid epithelial cell, Parafollicular cell), parathyroid (Parathyroid chief cell, Oxyphil cell), adrenal (Chromaffin cell), pineal (Pinealocyte) cells; cells of the nervous system, including glial cells (Astrocyte, Microglia), Magnocellular neurosecretory cell, Stellate cell, Boettcher cell, and pituitary (Gonadotrope, Corticotrope, Thyrotrope, Somatotrope, Lactotroph); cells of the Respiratory system, including Pneumocyte (Type I pneumocyte, Type II pneumocyte), Clara cell, Goblet cell, Dust cell; cells of the circulatory system, including Myocardiocyte, Pericyte; cells of the digestive system, including stomach (Gastric chief cell, Parietal cell), Goblet cell, Paneth cell, G cells, D cells, ECL cells, I cells, K cells, S cells; enteroendocrine cells, including enterochromaffm cell, APUD cell, liver (Hepatocyte, Kupffer cell), Cartilage/bone/muscle; bone cells, including Osteoblast, Osteocyte, Osteoclast, teeth (Cementoblast, Ameloblast); cartilage cells, including Chondroblast, Chondrocyte; skin cells, including Trichocyte, Keratinocyte, Melanocyte (Nevus cell); muscle cells, including Myocyte; urinary system cells, including Podocyte, Juxtaglomerular cell, Intraglomerular mesangial cell/Extraglomerular mesangial cell, Kidney proximal tubule brush border cell, Macula densa cell; reproductive system cells, including Spermatozoon, Sertoli cell, Leydig cell, Ovum; and other cells, including Adipocyte, Fibroblast, Tendon cell, Epidermal keratinocyte (differentiating epidermal cell), Epidermal basal cell (stem cell), Keratinocyte of fingernails and toenails, Nail bed basal cell (stem cell), Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, Hair matrix cell (stem cell), Wet stratified barrier epithelial cells, Surface epithelial cell of stratified squamous epithelium of cornea, tongue, oral cavity, esophagus, anal canal, distal urethra and vagina, basal cell (stem cell) of epithelia of cornea, tongue, oral cavity, esophagus, anal canal, distal urethra and vagina, Urinary epithelium cell (lining urinary bladder and urinary ducts), Exocrine secretory epithelial cells, Salivary gland mucous cell (polysaccharide-rich secretion), Salivary gland serous cell (glycoprotein enzyme-rich secretion), Von Ebner's gland cell in tongue (washes taste buds), Mammary gland cell (milk secretion), Lacrimal gland cell (tear secretion), Ceruminous gland cell in ear (wax secretion), Eccrine sweat gland dark cell (glycoprotein secretion), Eccrine sweat gland clear cell (small molecule secretion). Apocrine sweat gland cell (odoriferous secretion, sex-hormone sensitive), Gland of Moll cell in eyelid (specialized sweat gland), Sebaceous gland cell (lipid-rich sebum secretion), Bowman's gland cell in nose (washes olfactory epithelium), Brunner's gland cell in duodenum (enzymes and alkaline mucus), Seminal vesicle cell (secretes seminal fluid components, including fructose for swimming sperm), Prostate gland cell (secretes seminal fluid components), Bulbourethral gland cell (mucus secretion), Bartholin's gland cell (vaginal lubricant secretion), Gland of Littre cell (mucus secretion), Uterus endometrium cell (carbohydrate secretion), Isolated goblet cell of respiratory and digestive tracts (mucus secretion), Stomach lining mucous cell (mucus secretion), Gastric gland zymogenic cell (pepsinogen secretion), Gastric gland oxyntic cell (hydrochloric acid secretion), Pancreatic acinar cell (bicarbonate and digestive enzyme secretion), Paneth cell of small intestine (lysozyme secretion), Type II pneumocyte of lung (surfactant secretion), Clara cell of lung, Hormone secreting cells, Anterior pituitary cells, Somatotropes, Lactotropes, Thyrotropes, Gonadotropes, Corticotropes, Intermediate pituitary cell, Magnocellular neurosecretory cells, Gut and respiratory tract cells, Thyroid gland cells, thyroid epithelial cell, parafollicular cell, Parathyroid gland cells, Parathyroid chief cell, Oxyphil cell, Adrenal gland cells, chromaffin cells, Ley dig cell of testes, Theca interna cell of ovarian follicle, Corpus luteum cell of ruptured ovarian follicle, Granulosa lutein cells, Theca lutein cells, Juxtaglomerular cell (renin secretion), Macula densa cell of kidney, Metabolism and storage cells, Barrier function cells (Lung, Gut, Exocrine Glands and Urogenital Tract), Kidney, Type I pneumocyte (lining air space of lung), Pancreatic duct cell (centroacinar cell), Nonstriated duct cell (of sweat gland, salivary gland, mammary gland, etc.), Duct cell (of seminal vesicle, prostate gland, etc.), Epithelial cells lining closed internal body cavities, Ciliated cells with propulsive function, Extracellular matrix secretion cells, Contractile cells; Skeletal muscle cells, stem cell, Heart muscle cells, Blood and immune system cells, Erythrocyte (red blood cell), Megakaryocyte (platelet precursor), Monocyte, Connective tissue macrophage (various types), Epidermal Langerhans cell, Osteoclast (in bone), Dendritic cell (in lymphoid tissues), Microglial cell (in central nervous system), Neutrophil granulocyte, Eosinophil granulocyte, Basophil granulocyte, Mast cell, Helper T cell, Suppressor T cell, Cytotoxic T cell, Natural Killer T cell, B cell, Natural killer cell, Reticulocyte, Stem cells and committed progenitors for the blood and immune system (various types), Pluripotent stem cells, Totipotent stem cells, Induced pluripotent stem cells, adult stem cells, Sensory transducer cells, Autonomic neuron cells, Sense organ and peripheral neuron supporting cells, Central nervous system neurons and glial cells, Lens cells, Pigment cells, Melanocyte, Retinal pigmented epithelial cell, Germ cells, Oogonium/Oocyte, Spermatid, Spermatocyte, Spermatogonium cell (stem cell for spermatocyte), Spermatozoon, Nurse cells, Ovarian follicle cell, Sertoli cell (in testis), Thymus epithelial cell, Interstitial cells, and Interstitial kidney cells.

In some embodiments of any one of the methods disclosed herein, the condition can be a cancer or tumor. Non-limiting examples of such condition can include Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia, Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloblastoma, Medulloepithelioma, Melanoma, Melanoma, Meningioma, Merkel Cell Carcinoma, Mesothelioma, Mesothelioma, Metastatic Squamous Neck Cancer with Occult Primary, Metastatic urothelial carcinoma, Mixed Mullerian tumor, Monocytic leukemia, Mouth Cancer, Mucinous tumor, Multiple Endocrine Neoplasia Syndrome, Multiple Myeloma, Multiple myeloma, Mycosis Fungoides, Mycosis fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neuroblastoma, Neurofibroma, Neuroma, Nodular melanoma, Non-Hodgkin Lymphoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral Cancer, Oral cancer, Oropharyngeal Cancer, Osteosarcoma, Osteosarcoma, Ovarian Cancer, Ovarian cancer, Ovarian Epithelial Cancer, Ovarian Germ Cell Tumor, Ovarian Low Malignant Potential Tumor, Paget's disease of the breast, Pancoast tumor, Pancreatic Cancer, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sezary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenstrom's macroglobulinemia, Warthin's tumor, and Wilms' tumor.

In accordance with various embodiments, numerous types of neoplasms can be detected, including (but not limited to) acute lymphoblastic leukemia (ALL), acute myeloid leukemia (AML), anal cancer, astrocytomas, basal cell carcinoma, bile duct cancer, bladder cancer, breast cancer, Burkitt's lymphoma, cervical cancer, chronic lymphocytic leukemia (CLL) chronic myelogenous leukemia (CML), chronic myeloproliferative neoplasms, colorectal cancer, diffuse large B-cell lymphoma, endometrial cancer, ependymoma, esophageal cancer, esthesioneuroblastoma, Ewing sarcoma, fallopian tube cancer, follicular lymphoma, gallbladder cancer, gastric cancer, gastrointestinal carcinoid tumor, hairy cell leukemia, hepatocellular cancer, Hodgkin lymphoma, hypopharyngeal cancer, Kaposi sarcoma, Kidney cancer, Langerhans cell histiocytosis, laryngeal cancer, leukemia, liver cancer, lung cancer, lymphoma, melanoma, Merkel cell cancer, mesothelioma, mouth cancer, neuroblastoma, non-Hodgkin lymphoma, non-small cell lung cancer, osteosarcoma, ovarian cancer, pancreatic cancer, pancreatic neuroendocrine tumors, pharyngeal cancer, pituitary tumor, prostate cancer, rectal cancer, renal cell cancer, retinoblastoma, skin cancer, small cell lung cancer, small intestine cancer, squamous neck cancer, T-cell lymphoma, testicular cancer, thymoma, thyroid cancer, uterine cancer, vaginal cancer, and vascular tumors.

Many embodiments are directed to diagnostic or companion diagnostic scans performed during cancer treatment of an individual. When performing diagnostic scans during treatment, the ability of agent to treat the cancer growth can be monitored. Most anti-cancer therapeutic agents result in death and necrosis of neoplastic cells, which should release higher amounts nucleic acids from these cells into the samples being tested. Accordingly, the level of circulating-tumor nucleic acids can be monitored over time, as the level should increase during early treatments and begin to decrease as the number of cancerous cells are decreased. In some embodiments, treatments are adjusted based on the treatment effect on cancer cells. For instance, if the treatment isn't cytotoxic to neoplastic cells, a dosage amount may be increased or an agent with higher cytotoxicity can be administered. In the alternative, if cytotoxicity of cancer cells is good but unwanted side effects are high, a dosage amount can be decreased or an agent with less side effects can be administered.

Various embodiments are also directed to diagnostic scans performed after treatment of an individual to detect residual disease and/or recurrence of cancer. If a diagnostic scan indicates residual and/or recurrence of cancer, further diagnostic tests and/or treatments may be performed as described herein. If the cancer and/or individual is susceptible to recurrence, diagnostic scans can be performed frequently to monitor any potential relapse.


**F. Computer systems**


In one aspect, the present disclosure provides a computer program product comprising a non-transitory computer-readable medium having computer-executable code encoded therein, the computer-executable code adapted to be executed to implement any one of the preceding methods.

The present disclosure provides computer systems that are programmed to implement methods of the disclosure. The system can, in some cases, include components such as a processor, an input module for inputting sequencing data or data derived therefrom, a computer-readable medium containing instructions that, when executed by the processor, perform an algorithm on the input regarding one or more cell-free nucleic acids molecules, and an output module providing one or more indicia associated with the condition.

FIG. 27 shows a computer system 2701 that is programmed or otherwise configured to implement partial or all of the methods disclosed herein. The computer system 2701 can regulate various aspects of the present disclosure, such as, for example, (i) identify, from sequencing data derived from a plurality of cell-free nucleic acid molecules, one or more cell-free nucleic acid molecules comprising the plurality of phased variants, (ii) analyze any of the identified cell-free nucleic acid molecules, (iii) determine a condition of the subject based at least in part on the identified cell-free nucleic acid molecules, (iv) monitor a progress of the condition of the subject based at least in part on the identified cell-free nucleic acid molecules, (v) identify the subject based at least in part on the identified cell-free nucleic acid molecules, or (vi) determine an appropriate treatment of the condition of the subject based at least in part on the identified cell-free nucleic acid molecules. The computer system 2701 can be an electronic device of a user or a computer system that is remotely located with respect to the electronic device. The electronic device can be a mobile electronic device.

The computer system 2701 includes a central processing unit (CPU, also "processor" and "computer processor" herein) 2705, which can be a single core or multi core processor, or a plurality of processors for parallel processing. The computer system 2701 also includes memory or memory location 2710 (e.g., random-access memory, read-only memory, flash memory), electronic storage unit 2715 (e.g., hard disk), communication interface 2720 (e.g., network adapter) for communicating with one or more other systems, and peripheral devices 2725, such as cache, other memory, data storage and/or electronic display adapters. The memory 2710, storage unit 2715, interface 2720 and peripheral devices 2725 are in communication with the CPU 2705 through a communication bus (solid lines), such as a motherboard. The storage unit 2715 can be a data storage unit (or data repository) for storing data. The computer system 2701 can be operatively coupled to a computer network ("network") 2730 with the aid of the communication interface 2720. The network 2730 can be the Internet, an internet and/or extranet, or an intranet and/or extranet that is in communication with the Internet. The network 2730 in some cases is a telecommunication and/or data network. The network 2730 can include one or more computer servers, which can enable distributed computing, such as cloud computing. The network 2730, in some cases with the aid of the computer system 2701, can implement a peer-to-peer network, which may enable devices coupled to the computer system 2701 to behave as a client or a server.

The CPU 2705 can execute a sequence of machine-readable instructions, which can be embodied in a program or software. The instructions may be stored in a memory location, such as the memory 2710. The instructions can be directed to the CPU 2705, which can subsequently program or otherwise configure the CPU 2705 to implement methods of the present disclosure. Examples of operations performed by the CPU 2705 can include fetch, decode, execute, and writeback.

The CPU 2705 can be part of a circuit, such as an integrated circuit. One or more other components of the system 2701 can be included in the circuit. In some cases, the circuit is an application specific integrated circuit (ASIC).

The storage unit 2715 can store files, such as drivers, libraries and saved programs. The storage unit 2715 can store user data, e.g., user preferences and user programs. The computer system 2701 in some cases can include one or more additional data storage units that are external to the computer system 2701, such as located on a remote server that is in communication with the computer system 2701 through an intranet or the Internet.

The computer system 2701 can communicate with one or more remote computer systems through the network 2730. For instance, the computer system 2701 can communicate with a remote computer system of a user. Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 2701 via the network 2730.

Methods as described herein can be implemented by way of machine (e.g., computer processor) executable code stored on an electronic storage location of the computer system 2701, such as, for example, on the memory 2710 or electronic storage unit 2715. The machine executable or machine readable code can be provided in the form of software. During use, the code can be executed by the processor 2705. In some cases, the code can be retrieved from the storage unit 2715 and stored on the memory 2710 for ready access by the processor 2705. In some situations, the electronic storage unit 2715 can be precluded, and machine-executable instructions are stored on memory 2710.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 2701, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 2701 can include or be in communication with an electronic display 2735 that comprises a user interface (UI) 2740 for providing, for example, (i) analysis of any of the identified cell-free nucleic acid molecules, (ii) a determined condition of the subject based at least in part on the identified cell-free nucleic acid molecules, (iii) a determined progress of the condition of the subject based at least in part on the identified cell-free nucleic acid molecules, (iv) the identified subject suspected of having the condition based at least in part on the identified cell-free nucleic acid molecules, or (v) a determined treatment of the condition of the subject based at least in part on the identified cell-free nucleic acid molecules. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 2705. The algorithm can, for example, (i) identify, from sequencing data derived from a plurality of cell-free nucleic acid molecules, one or more cell-free nucleic acid molecules comprising the plurality of phased variants, (ii) analyze any of the identified cell-free nucleic acid molecules, (iii) determine a condition of the subject based at least in part on the identified cell-free nucleic acid molecules, (iv) monitor a progress of the condition of the subject based at least in part on the identified cell-free nucleic acid molecules, (v) identify the subject based at least in part on the identified cell-free nucleic acid molecules, or (vi) determine an appropriate treatment of the condition of the subject based at least in part on the identified cell-free nucleic acid molecules.

## EXAMPLES

The following illustrative examples are representative of embodiments of the stimulation, systems, and methods described herein and are not meant to be limiting in any way.

### Example 1: Genomic Distribution of Phased Variants

Described is an alternative to duplex sequencing for reducing the background error rate that involves detection of 'phased variants' (PVs), where two or more mutations occur in cis (i.e., on the same strand of DNA **FIG. 1A** and **FIG. 1E).** Similar to duplex sequencing, this method provides lower error profiles due to the concordant detection of two separate non-reference events in individual molecules. However, unlike duplex sequencing, both events occur on the same sequencing read-pair, thereby increasing the efficiency of genome recovery. Phased mutations are present in diverse cancer types, but occur in stereotyped portions of the genome in B-cell malignancies, likely due to on-target and aberrant somatic hypermutation (aSHM) driven by activation-induced deaminase (AID). The most common regions of aSHM in B-cell non-Hodgkin lymphomas (NHL) are identified. Described herein is phased variant Enrichment and Detection Sequencing (PhasED-Seq), a novel method to detect ctDNA through phased variants to tumor fractions on the order of parts per million. Described herein is demonstration that PhasED-Seq can meaningfully improve detection of ctDNA in clinical samples both during therapy and prior to disease relapse.

To identify malignancies where PVs may potentially improve disease detection, the frequency of PVs across cancer types were assessed. Publicly available whole-genome sequencing data was analyzed to identify sets of variants occurring at a distance of <170bp apart, which represents the typical length of a single cfDNA fragment consisting of a single core nucleosome and associated linker. The frequency of these 'putative phased variants," **(Example 10)** controlling for the total number of SNVs, from 2538 tumors across 24 cancer histologies including solid tumors and hematological malignancies **(FIG. 1B, FIG. 5**, and **Table 1)** was identified and summarized. PVs were most significantly enriched in two B-cell lymphomas (DLBCL and follicular lymphoma, FL, P<0.05 vs all other histologies), a group of diseases with hypermutation driven by AID/AICDA.

### Example 2: Mutational Mechanisms Underlying PVs

To investigate the origin of PVs, the single base substitution (SBS) mutational signatures contributing to SNVs occurring within 170bp of another SNV, and SNVs occurring in isolation (e.g., not having another SNV within 170 bp) (Example 10) were compared. As expected, PVs were highly enriched in several mutational signatures associated with clustered mutations. Signatures of clustered mutations associated with activity of AID (SBS84 and SBS85) were significantly enriched in PVs from B-cell lymphomas and CLL, while signatures associated with activity of APOBEC3B (SBS2 and SBS13) - another mechanism of kataegis hypermutation - were significantly enriched in PVs from multiple solid cancer histologies, including ovarian, pancreatic, prostate, and breast adenocarcinomas **(FIG. 1C** and **FIGs. 6A-6WW).** Signatures of clustered mutations associated with activity of AID (SBS84 and SBS85) were enriched in PVs found in lymphomas and CLL, while signatures associated with activity of APOBEC3B (SBS2 and SBS13) were significantly enriched in breast cancer **(FIG. 1C** and **FIGs. 6A-6WW).** PVs from multiple tumor types were also associated with SBS4, a signature associated with tobacco use. Furthermore, among PVs across multiple tumor histologies, it was observed that novel enrichments in several other signatures without clearly associated mechanisms (e.g., SBS24, SBS37, SBS38, and SBS39). In contrast, aging-associated mutational signatures such as SBS1 and SBS5 were significantly enriched in isolated SNVs.

### Example 3: PVs Occur in Stereotyped Genomic Regions in Lymphoid Cancers

To assess the genomic distribution of putative PVs, these events were first binned into 1-kb regions to visualize their frequency across tumor types. It was observed that a strikingly stereotyped distribution of PVs in individual lymphoid neoplasms (e.g., DLBCL, FL, Burkitt lymphoma (BL), and chronic lymphocytic leukemia (CLL); **FIG. 1D** and **FIG. 7).** In contrast, non-lymphoid cancers generally did not exhibit substantial recurrence of clustered PVs in stereotyped regions. This lack of stereotype in the position of PVs was true even when considering melanomas and lung cancers, diseases with frequent PVs.

Notably, the majority of hypermutated regions were shared between all three lymphoma subtypes, with the highest densities seen in known targets of aSHM including BCL2, BCL6, and MYC, as well as the immunoglobulin (Ig) loci encoding the heavy and light chains IGH, IGK, and IGL **(Table 2).** Strikingly, certain regions within Ig loci were densely mutated in nearly all lymphoma patients as well as in patients with CLL **(FIG. 1D).** Among lymphoma subtypes, DLBCL tumors harbored the most 1-kb regions recurrently containing PVs **(FIG. 8A),** consistent with the highest number of recurrently mutated genes being observed in this tumor type. In total, 1639 unique 1-kb regions recurrently containing PVs in B-lymphoid malignancies were identified. Among these lymphoma-associated 1-kb regions, nearly one-third fell into genomic areas previously associated with physiological or aberrant SHM in B-cells. Specifically, 19% (315/1639) were located in Ig regions, while 13% (218/1639) were in portions of 68 previously identified targets of aSHM **(Table 2).** While most PVs fell into noncoding regions of the genome, additional recurrently affected loci not previously described as targets of aSHM, including XBP1, LPP, and AICDA, among others, were also identified.

The distribution of PVs within each lymphoid malignancy correlated with oncogenic features associated with the distinct pathophysiology of the corresponding disease. For example, cases of FL - where more than 90% of tumors harbor oncogenic BCL2 fusions - were significantly more likely to contain phased variants in BCL2 than other lymphoid malignancies **(FIG. 1D** and **FIG. 8B).** Similarly, significantly more Burkitt lymphomas (BL) harbored PVs in MYC and ID3, two driver genes strongly associated with the BL pathogenesis, than other lymphoid malignancies **(FIG. 1D** and **FIGs. 8C-8D).** DLBCL molecular subtypes associated with distinct cell-of-origin also demonstrated distinct distributions of PVs **(Table 2).** Specifically, while germinal center B-cell like (GCB) and activated B-cell like (ABC) DLBCLs harbored similar frequencies of PVs overall (median 798 vs 516, P = 0.37), significant enrichment for PVs in the telomeric IGH class-switch regions (S$\gamma$1, and S$\gamma$3) in ABC-DLBCLs, consistent with previous reports41 **(FIG. 8E),** was found. Conversely, GCB-DLBCLs harbored more phased haplotypes in centromeric IGH class switch regions (S$\alpha$2 and S$\epsilon$) and in BCL2.

### Example 4: Design and Validation of PhasED-Seq Panel for Lymphoma

To validate these PV-rich regions and assess their utility for disease detection from ctDNA, a sequencing panel targeting putative PVs identified within WGS from three independent cohorts of patients with DLBCL, as well as in patients with CLL **(FIG. 2A** and **Example 10)** was designed. This final Phased variant Enrichment and Detection Sequencing (PhasED-Seq) panel targeted ~115kb of genomic space focused on PVs, along with an additional ~200kb targeting genes that are recurrently mutated in B-NHLs **(Table 3)**. While the 115kb of space dedicated to PV-capture targets only 0.0035% of the human genome, it captures 26% of phased variants observed in mature B-cell neoplasms profiled by WGS **(FIG 9A),** thus yielding a ~7500-fold PV enrichment by PhasED-Seq over WGS.

Expected SNV and PV recovery was compared to previously reported CAPP-Seq selector designed to maximize SNVs per patient in B-cell lymphomas **(FIG. 9A-C).** When considering diverse B-NHLs with available WGS data, PhasED-Seq recovered 3.0x more SNVs (81 vs. 27) and 2.9x more PVs (50 vs. 17) in the median case than previous CAPP-Seq panel. This observation highlights the importance of including non-coding portions of the genome for maximal mutation recovery. To validate these yield improvements experimentally, 16 pretreatment tumor or plasma DNA samples from patients with DLBCL **(Table 4)** were profiled. Both CAPP-Seq and PhasED-Seq panels were applied to each specimen in parallel and then sequenced them to high unique molecular depths **(FIG. 2B).** Compared to the expected enrichment established from WGS, similar improvements in yield of SNVs by PhasED-Seq compared to CAPP-Seq (2.7x; median 304.5 vs. 114) were observed. However, when enumerating PVs observed in individual sequenced DNA fragments, an improvement in favor of PhasED-Seq beyond the expected improvement from WGS (7.7x; median 5554 vs 719.5 PVs/case) was found. This improvement is potentially due to either 1) the higher sequencing depth in targeted sequencing which leads to improvement in rare allele detection, or 2) enumeration of higher order PVs in targeted sequencing with PhasED-Seq or CAPP-Seq, which was not accounted for in the WGS design (i.e., >2 SNVs per fragment; **FIGs. 9D-9F).** Furthermore, across 1-kb windows in the panel, robust correlation between the frequency of putative PVs in WGS data and PVs from targeted sequencing by PhasED-Seq across 101 DLBCL samples **(FIG. 2C)** was observed, further validating the frequency and distribution of PVs in B-cell malignancies.

### Example 5: Differences in Phased Variants between Lymphoma Subtypes

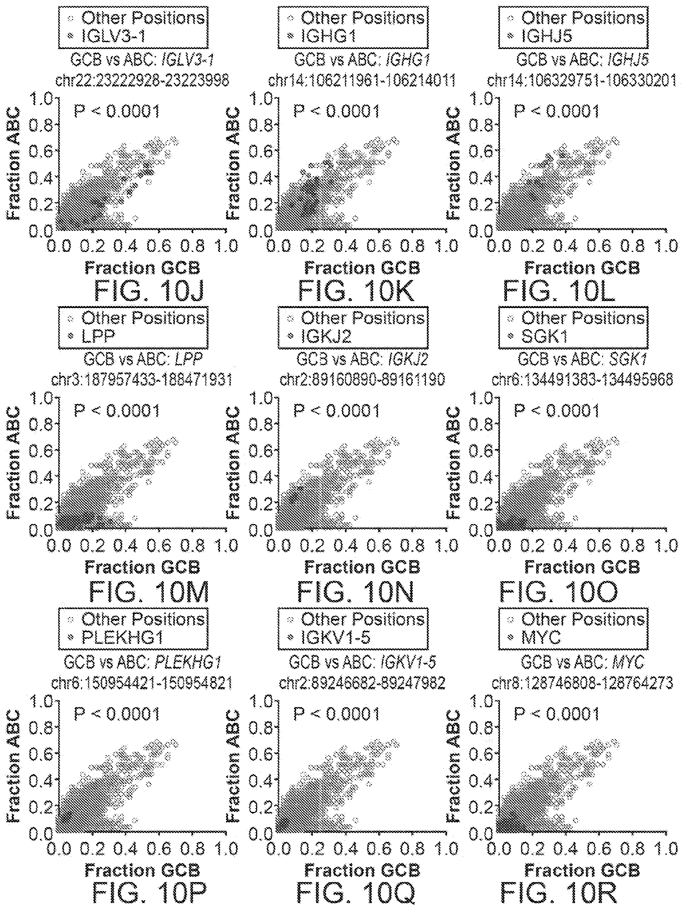

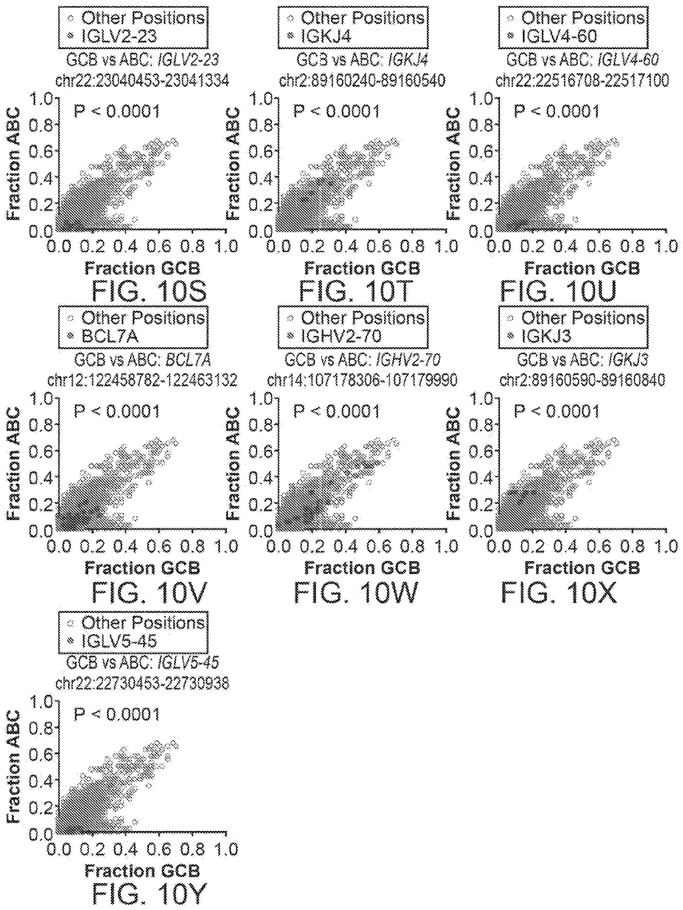

Having validated the PhasED-Seq panel, the biological differences in PVs between various B-cell malignancies, including DLBCL (n=101), primary mediastinal B-cell lymphoma (PMBCL) (n=16), and classical Hodgkin lymphoma (cHL) (n=23) were examined. The number of SNVs identified per case was not significantly different between lymphoma subtypes **(FIGs. 9G-9K).** However, when considering mutational haplotypes, cHL had a significantly lower burden of PVs than either DLBCL or PMBCL. In addition to this quantitative disparity, differences in the genomic locations of PVs between different B-cell lymphoma subtypes were also observed **(FIGs. 2D-2E** and **FIGs. 10-12).** This included previously established biological associations in DLBCL subtypes, including more frequent PVs in BCL2 in GCB-type than ABC-type DLBCL, with the opposite association seen for PIM1. More frequent PVs in CIITA in PMBCL compared with DLBCL, a gene in which breakpoints are common in PMBCL, was also observed. Relative enrichments were also observed throughout the IGH locus, with more frequent PVs seen in $S\gamma3$ and $S\gamma1$ regions in ABC-DLBCL (compared with GCB-DLBCL) and interestingly, more frequent PVs in the $S\varepsilon$ locus in cHL compared with DLBCL **(FIG. 2E** and **FIG. 13).** In total, after correcting for testing multiple hypotheses, significant relative enrichments in 25 genetic loci between ABC- and GCB-DLBCL, 24 between DLBCL and PMBCL, and 40 between DLBCL and cHL were found **(FIG. 10-12).**

## Example 6: Recovery of Phased Variants through PhasED-Seq

To facilitate detection of ctDNA using PVs, efficient recovery of DNA molecules is desired. Hybrid-capture sequencing is potentially sensitive to DNA mismatches, with increasing mutations decreasing hybridization efficiency. Indeed, AID hotspots can contain a 5-10% local mutation rate, with even higher rates in certain regions of IGH. To empirically assess the effect of mutation rate on capture efficiency, DNA hybridization of 150-mers with varying mutation rates in silico was simulated. As expected, predicted binding energy decreased with an increasing number of mutations **(FIG 14A).** Notably, randomly distributed mutations had a greater effect on binding energy than clustered mutations. To assess the effect of this decreased binding affinity, 150-mer DNA oligonucleotides with 0 to 10% difference from the reference sequence in MYC and BCL6, two loci that are targets of aSHM were synthesized. To assess the worst-case scenario for hybridization, non-reference bases were randomly distributed rather than in clusters **(Example 10).** An equimolar mixture of these oligonucleotides were then captured with PhasED-Seq panel. Concordant with the in silico predictions, increased mutational rates resulted in decreased capture efficiency **(FIG. 3A).** Molecules with a 5% mutation rate were captured with 85% efficiency relative to fully-wildtype counterparts, while molecules with 10% mutation were captured with only 27% relative efficiency. To assess the prevalence of this degree of mutation in human tumors, the distribution of variants in panel in 140 patients with B-cell lymphomas, calculating the fraction of mutated bases in overlapping 151-bp windows **(Example 10)** was examined. Only 7% (10/140) of patients had any 151-bp window exceeding 10% mutation rate **(FIG. 14B-C).** Indeed, in the experiment with synthetic oligonucleotides, a 5% mutation rate was recovered nearly as efficiently as the wild-type sequence. In over half of all cases considered, no locus had >5% mutation rate at any window, while in all cases >90% of windows had <5% mutations. Overall, these observations indicate that the majority of phased mutations are recoverable by efficient hybrid capture, despite hybridization biases.

## Example 7: Error Profile and Limit of Detection for Phased Variant Sequencing

Previous methods for highly error-suppressed sequencing applied to cfDNA have utilized either a combination of molecular and in silico methods for error suppression (e.g., integrated digital error suppression, iDES) or duplex molecular recovery. However, each of these has limitations, either for detecting events at ultra-low tumor fractions or for efficient recovery of original DNA molecules, which are important considerations for cfDNA analysis where input DNA is limited. The error profile and recovery of input genomes from plasma cfDNA samples form 12 heathy adults by PhasED-Seq were compared with both iDES-CAPP-Seq and duplex sequencing. While iDES-enhanced CAPP-Seq had a lower background error profile than barcode-deduplication alone, duplex sequencing offered the lowest background error rate for non-reference single nucleotide substitutions **(FIG. 3B,** $3.3 \times 10^{-5}$ vs. $1.2 \times 10^{-5}$, P<0.0001). However, the rate of phased errors - e.g., multiple non-reference bases occurring on the same sequencing fragment - was significantly lower than the rate of single errors in either iDES-enhanced CAPP-Seq or duplex sequencing data. This was true for the incidence of both two (2x or 'doublet' PVs) or three (3x or 'triplet' PVs) substitutions on the same DNA molecule **(FIG. 3B,** $8.0 \times 10^{-7}$ and $3.4 \times 10^{-8}$ respectively, P<0.0001). Phased errors containing C to T or T to C transition substitutions were more common than other types of PVs **(FIG. 14D).** Notably, the rate doublet PVs errors in cfDNA was also correlated with distance between positions, with the highest PV error-rate consisting of neighboring SNVs (e.g., DNVs) and decreasing error rate with increasing distance between constituent variants **(FIG. 14E).** When considering unique molecular depth, duplex sequencing recovered only 19% of all unique cfDNA fragments **(FIG. 3C).** In contrast, the unique depth of PVs within a genomic distance of <20bp was nearly identical to the depth of individual positions (e.g., molecules covering individual SNVs). Similarly, PVs up to 80bps in size had depth greater than 50% of the median unique molecular depth for a sample. Importantly, almost half (48%) of all PVs were within 80bp of each other, demonstrating their utility for disease detection from input-limited cfDNA samples **(FIG. 3D).**

To quantitatively compare the performance of PhasED-Seq to alternative methods for ctDNA detection, limiting dilutions of ctDNA from 3 lymphoma patients into healthy control cfDNA were generated, resulting in expected tumor fractions between 0.1% and 0.00005% (1 part in 2,000,000; **(Example 10).** The expected tumor fraction was compared to the estimated tumor content in each of these dilutions using PhasED-Seq to track tumor-derived PVs, as well as to error-suppressed detection methods depending on individual SNVs (e.g. iDES-enhanced CAPP-Seq or duplex sequencing; **FIG. 3E**). All methods performed equally well down to tumor fractions of 0.01% (1 part in 10,000). However, below this level (e.g., 0.001%, 0.0002%, 0.0001%, and 0.00005%), both PhasED-Seq and duplex sequencing significantly outperformed iDES-enhanced CAPP-Seq (P<0.0001 for duplex, '2x' PhasED-Seq, and '3x' PhasED-Seq; **FIG. 3E**). In addition, when compared to duplex-sequencing, tracking either 2 or 3 variants in-phase (e.g., 2x and 3x PhasED-Seq) more accurately identified expected tumor content, with superior linearity down to 1 part in 2,000,000 (P = 0.005 for duplex vs 2x PhasED-Seq, P=0.002 for 3x PhasED-Seq) **(Example 10)**. Specificity of PVs by looking for evidence of tumor-derived SNVs or PVs in cfDNA samples from 12 unrelated healthy control subjects and the healthy control used for the limiting dilution was assessed. Here again, both 2x- or 3x-PhasED-Seq showed significantly lower background signal levels than did CAPP-Seq and duplex sequencing **(FIG. 3F)**. This lower error rate and background from PVs improves the detection limit for ctDNA disease detection. In some instances, the method of sequencing-based cfDNA assays described herein (e.g. the method depicted in **FIG. 3E** and **FIG. 3F)** does not require molecular barcodes to achieve exquisite error-suppression and low limits of detection. Signal assessed by the method without barcode used limiting dilution series from 1 : 1,000 to 5 : 10,000,000, and 'blank' controls **(FIGs. 23A-23B)**.

This dilution series was used to assess the limit of detection for a given number of PVs **(FIGs. 3G-3I)**. When considering a set of PVs within 150 base pair (bp) regions, the probability of detection for a given sample may be accurately modelled by binomial sampling, considering both the depth of sequencing and the number of 150bp regions with PVs **(Example 10)**.

## Example 8: Improvements in Detection of Low-Burden Minimal Residual Disease

To test the utility of the lower LOD afforded by PhasED-Seq for detection of ultra-low burden MRD from cfDNA, Serial cell-free DNA samples were sequenced from a patient undergoing front-line therapy for DLBCL **(FIG. 4A)**. Using CAPP-Seq, this patient had undetectable ctDNA after only one cycle of therapy, with multiple subsequent samples during and after treatment also remaining undetectable. This patient had subsequent re-emergence of detectable ctDNA >250 days after the start of therapy, with eventual clinical and radiographic disease progression 5 months later, indicating falsely negative serial measurements with CAPP-Seq. Strikingly, all four of the plasma samples that were undetectable by CAPP-Seq during and after treatment had detectable ctDNA levels by PhasED-Seq, with mean allelic fractions as low as 6 parts in 1,000,000. This increased sensitivity improved the lead-time of disease detection by ctDNA compared to radiographic surveillance from 5 with CAPP-Seq to 10 months with PhasED-Seq.

Next, the performance of PhasED-Seq ctDNA detection in a cohort of 107 patients with large B-cell lymphomas and blood samples available after 1 or 2 cycles of standard immuno-chemotherapy was next assessed. Importantly, ctDNA levels measured by PhasED-Seq were highly correlated with those measured by CAPP-Seq. In total, 443 tumor, germ-line, and cell-free DNA samples, including ctDNA prior to therapy (n=107) and after 1 or 2 cycles of treatment (n=82 and 89), were assessed. Prior to therapy, patient-specific PVs were detectable by PhaseED-Seq in 98% of samples, with 95% specificity in ctDNA from healthy controls **(FIGs. 15** and **16A)**. Importantly, ctDNA levels measured by PhasED-Seq were highly correlated with those measured by CAPP-Seq, considering both pretreatment and post treatment samples (Spearman rho=0.91, **FIG. 16B)**. Next, quantitative levels of ctDNA measured by PhasED-Seq and CAPP-Seq from cfDNA samples after initiation of therapy were compared. In total, 72% (78/108) of samples with detectable ctDNA by PhasED-Seq after 1 or 2 cycles were also detected by conventional CAPP-Seq **(FIG. 4B)**. Among 108 samples detected by PhasED-Seq, disease burden was significantly lower for those with undetectable (28%) vs. detectable (72%) ctDNA levels using conventional CAPP-Seq, with a >10x difference in median ctDNA levels (tumor fraction $2.2 \times 10^{-4}$ vs $1.2 \times 10^{-5}$, P<0.001, **FIG. 4B).** In total, an additional 16% (13/82) of samples after 1 cycle of therapy and 19% (17/89) of samples after 2 cycles of therapy had detectable ctDNA when comparing PhasED-Seq with CAPP-Seq **(FIG. 4C)**.

ctDNA molecular response criteria was previously described for DLBCL patients using CAPP-Seq, including Major Molecular Response (MMR), defined as a 2.5-log reduction in ctDNA after 2 cycles of therapy22. While MMR at this time-point is prognostic for outcomes, many patients have undetectable ctDNA by CAPP-Seq at this landmark **(FIGs. 4D-4E)**. Importantly, even in patients with undetectable ctDNA by CAPP-Seq, detection of occult ultra-low ctDNA levels by PhasED-Seq was prognostic for outcomes including event-free and overall survival **(FIG. 4D)**. Indeed, in the 89 patients with a sample available from this time-point, 58% (52/89) had undetectable ctDNA by CAPP-Seq at their interim MMR assessment, after completing 2 of 6 planned cycles of therapy. Using PhasED-Seq, 33% (17/52) of samples not detected by CAPP-Seq had evidence of ctDNA as evidenced by PVs, with levels as low as ~3:1,000,000 **(FIGs. 17A-17D)** - these 17 cases additionally detected by PhasED-Seq represent potential false negative tests by CAPP-Seq. Similar results were seen at the Early Molecular Response (EMR) time-point (i.e., after 1 cycle of therapy, **FIGs. 18A-18H)**.

While detection of ctDNA in DLBCL after 1 or 2 cycles of therapy is a known adverse prognostic marker outcome for patients with undetectable ctDNA at these time-points are heterogeneous **(FIG. 4E** and **FIG. 18F)**. Importantly, even in patients with undetectable ctDNA by CAPP-Seq after 1 or 2 cycles of therapy, detection of ultra-low ctDNA levels by PhasED-Seq was strongly prognostic for outcomes including event-free survival **(FIG. 4F, FIG. 17C-D, FIG. 18C-D,** and **FIG. 18G).** When combining detection by PhasED-Seq with previously described MMR threshold, patients could be stratified into three groups - patients not achieving MMR, patients achieving MMR but with persistent ctDNA, and patients with undetectable ctDNA **(FIG. 4G).** Interestingly, while patients not achieving MMR were at especially high risk for early events despite additional planned first line therapy (e.g., within the first year of treatment), patients with persistent low levels of ctDNA appeared to have a higher risk of later relapse or progression events. In contrast, patients with undetectable ctDNA after 2 cycles of therapy by PhasED-Seq had overwhelmingly favorable outcomes, with 95% being event-free and 97% overall survival at 5 years. Similar results were seen at the EMR time-point after 1 cycle of therapy **(FIG. 18H).**

### Example 9: Exemplary embodiments of mutation detection using next generation sequencing (NGS) when the mutation is not a single base substation, but rather a pair of mutations

In many instances, a limitation of cfDNA tracking may be the limitation on the number of molecules available for detection. Additionally, there are multiple potential limitations on tracking tumor molecules from cell-free DNA, including not only the sequencing error profile, but also the number of molecules available for detection. The number of molecules available for detection - here termed the number of "evaluable fragments" - can be thought of as both a function of the number of recovered unique genomes (e.g., unique depth of sequencing) and the number of somatic mutations being tracked. More specifically, the number of evaluable fragments is equal to: $EF = d * n$.

Where $d$ = the unique molecular depth considered and $n$ = the number of somatic alterations tracked. For the typical cell-free DNA samples, less than 10,000 unique genomes are often recovered (d), requiring any sensitive method to track multiple alterations (n). Furthermore, as stated above, the major limitation for duplex sequencing is difficulty recovering sufficient unique molecular depth (d); thus, from a typical plasma sample with duplex depth of ~1,500x, even if following 100 somatic alterations, there are only 150,000 evaluable fragments. Thus, in this scenario, sensitivity is limited by the number of molecules available for detection. In contrast, other methods such as iDES-enhanced CAPP-Seq consider all molecules recovered. Here, as many as 5,000-6,000x unique haploid genomes can be recovered. Therefore, the number of evaluable fragments, tracking the same 100 somatic alterations, may be 500,000-600,000x. However, the error profile of single-stranded sequencing, even with error suppression, allows detection to levels of at best 1 part in 50,000. Therefore, methods aiming to improve on the detection limits for ctDNA must overcome both the error-profile of sequencing and the recovery of sufficient evaluable fragments to utilize said lower error-profiles.

To remedy this apparent deficiency, the method of PhasED-Seq, as described in the instant disclosure, allows for lymphoid malignancies and was applicable to other cancer histologies, (e.g., using a "personalized" approach). For a personalized approach, customized hybrid-capture oligonucleotides (or primers for PCR amplicons) were used to capture personalized somatic mutations identified from whole exome or genome sequencing. The PCAWG dataset assessed for SNVs occurring within 170bp of each other in genomic space was re-analyzed. It was found that in 14 of 24 cancer histologies considered, the median case contained >100 possible phased variants, including in several solid tumors such as Melanoma (median 2072), lung squamous cell carcinoma (1268), lung adenocarcinoma (644.5), and colorectal adenocarcinoma (216.5).

Next, the expected limit of detection in all cases in the PCAWG dataset using either duplex sequencing or PhasED-Seq was assessed. Again, the limit of detection was defined by the expected number of evaluable fragments, and thus depends on both the number of variants tracked and the expected depth of sequencing. Utilizing the data from optimized hybrid capture conditions, a model to predict the expected deduplicated (single-stranded) and duplex (double-stranded) molecular depth with a given DNA input and number of sequencing reads was constructed. Using this, along with the number of SNVs or possible PVs from the PCAWG dataset, for each case, which method would lead to a greater number of evaluable fragments, and therefore a superior limit of detection was assessed. The results of this exercise, assuming 64 nanograms (ng) of total cfDNA input and a total of 20 million sequencing reads are shown in **FIG. 19.** Notably, in the majority of cancer types (18/24 histologies), PhasED-Seq had a lower limit of detection than duplex sequencing. This importantly included not only B-cell lymphomas, but common solid tumors, including lung squamous cell carcinoma and adenocarcinoma, colorectal adenocarcinoma, esophageal and gastric adenocarcinoma, and breast adenocarcinoma, among others. Indeed, taking lung cancers as a specific example, an almost 10-fold lower limit of detection was found for the median squamous cell and adenocarcinoma lung cancer case using PhasED-Seq compared to duplex sequencing **(FIG. 20).** Both PhasED-Seq and duplex sequencing using a personalized approach had a lower limit of detection than non-personalized approaches (e.g., iDES-enhanced CAPP-Seq).

To further confirm the applicability of phased variants and PhasED-Seq in diverse solid tumors, WGS (~20-30x depth) was performed on paired tumor and normal DNA to identify PVs from five solid tumor patients predicted to have low ctDNA burden prior to treatment (lung cancer (n=5), along with one patient having breast cancer (n=1)). Sequencing reads were aligned to hg19 and deduplicated with samtools markdup. In accordance with GATK practices, tumor and normal deduplicated BAM files were processed with GATK IndelRealigner and BaseRecalibrator before variant calling, using default parameters (GATK v3.8-1-0-gf15c1c3ef) (Van der Auwera, G. A. et al. From FastQ data to high-confidence variant calls: the Genome Analysis Toolkit best practices pipeline. Curr. Protoc. Bioinformatics 43, 11.10.1-11.10.33 (2013)). Variant calling was performed using three methods: VarScan2 (v2.3.9) (Koboldt, D. C. et al. VarScan 2: somatic mutation and copy number alteration discovery in cancer by exome sequencing. Genome Res. 22, 568-576 (2012)), Mutect (v1.1.7) (Cibulskis, K. et al. Sensitive detection of somatic point mutations in impure and heterogeneous cancer samples. Nat. Biotechnol. 31, 213-219 (2013)), and Strelka2 (v2.9.1) (Kim, S. et al. Strelka2: fast and accurate calling of germline and somatic variants. Nat. Methods 15, 591 594 (2018)). Mutect and VarScan2 VCF files were annotated by annovar (v2018Apr16), and Strelka VCF files were annotated by Oncotator (v1.9.8.0). Variants called by each method were combined and filtered according to the following criteria: (1) pass caller-intrinsic quality filters (for example, base quality, orientation bias and germline risk); (2) depth $\geq 30\times$; (3) AF $\geq 5\%$; and (4) variant identified by $\geq 2$ variant callers. SNVs passing all filters were then assessed for possible phased relationships-any pair of SNVs $\leq 170$ bp from its nearest neighbor was considered a viable PV. ("Viable PVs" are defined in this example as PVs initially identified by WGS.) We also genotyped PVs directly from WGS reads, considering any viable PVs with at least two supporting reads, $10\times$ depth and 5% tumor fraction. Viable PVs were then assessed and prioritized for tumor specificity, considering the (1) presence in individual tumor reads as phased relationships, (2) absence of read support in matched normal, (3) presence of other non-reference bases on the supporting reads, (4) base quality, (5) mapping quality and (6) uniqueness of genomic positions. Based on these metrics, candidate PVs were then selected for targeted resequencing below. ("Candidate PVs" are defined as the subset of viable PVs selected for targeted resequencing and validation). As used in this example, "putative PVs" can refer to either or both or viable PVs or candidate PVs.

After identifying candidate PVs from each of these six tumors, we designed 120-bp biotinylated hybrid-capture oligonucleotides targeting the regions of interest (Integrated DNA Technologies). We then performed hybrid capture resequencing of the tumor-normal pairs to high unique molecular depth (~1000-3000$\times$ deduplicated depth) to create a validated list of PVs for tumor monitoring **(FIG. 22C)**. The numbers shown in each column of FIG. 22C represent the number of regions of less than 170 bp in length that include a plurality of phased variants. A PV was considered to be validated if it was present in the tumor at higher than 5% AF and had no read support in the matched germline DNA.

We applied the above personalized hybrid-capture panels targeting PVs to plasma samples from each of these six participants, sequencing to high unique molecular depth (~1000 to 10,000$\times$ deduplicated depth). We also sequenced 24 control healthy ctDNA samples with each panel to assess specificity.

Tumor fraction was defined as the number of reads containing an a priori defined PV over the total number of reads covering a PV position. Most samples had been assessed for ctDNA content using SNV-based CAPP-seq approaches previously, providing comparison to PhasED-seq. The results of these experiments are shown in **FIGS. 22D-22G.**

Considering the five lung cancer cases, the PhasED-Seq approach achieved a ~10-fold improvement in analytical sensitivity, achieving a median LOD of 0.00018% compared to 0.0019% using customized CAPP-Seq **(FIG. 21A and FIG. 21B).**

To demonstrate the clinical significance of this improved limit of detection for ctDNA from PhasED-Seq in solid tumors, serial plasma samples from a patient with stage 3 adenocarcinoma of the lung treated with chemoradiotherapy with curative intent (LUP814) were analyzed using both CAPP-Seq and PhasED-Seq. As outlined above, both CAPP-Seq and PhasED-Seq quantified a similar level of ctDNA prior to therapy (~1% tumor fraction). However, 3 subsequent samples after beginning therapy had undetectable ctDNA by standard CAPP-Seq, including samples during and after chemoradiation and during adjuvant immunotherapy with Durvalumab. Despite the lack of detectable disease by CAPP-Seq, the patient had biopsy-confirmed recurrent disease after an initial radiographic response. However, when analyzing these same samples with PhasED-Seq, molecular residual disease in 3/3 (100%) of samples was detected, with mean tumor fraction as low as 0.00016% (1.6 parts per million). Furthermore, the trend in ctDNA quantitation mirrored the patient's disease course, with an initial response to chemoradiotherapy but disease progression during immunotherapy. Importantly, this patient's disease remained detectable at all timepoints, with detectable disease at the completion of chemoradiotherapy 8 months prior to the patient's biopsy-confirmed disease progression **(FIG. 22).**

**Example 10: Methods of phased variant enrichment for enhanced disease detection from cell-free DNA**

**10(a): Whole-genome sequencing analysis**

*10(a)(1): Whole-Genome Sequencing Data Putative Phased Variant Identification*

Whole-genome sequencing data were obtained from two sources. Data for lymphoid malignancies (diffuse large B-cell lymphoma, DLBCL; follicular lymphoma, FL; Burkitt lymphoma, BL; chronic lymphocytic leukemia, CLL) were downloaded from the International Cancer Genome Consortium (ICGC) data portal on May 7th, 2018. Data from all other histologies were part of the pan-Cancer analysis of whole genomes (PCAWG) and downloaded on November 11th, 2019.

Only cancer histologies with at least 35 available cases were considered; details of the dataset considered are provided in **Table 1.** All samples had somatic mutations called from WGS using matched tumor and normal genotyping. Queries were limited to base substitutions obtained from WGS (single, double, triple, and oligo nucleotide variants; SNVs, DNVs, TNVs, and ONVs). Having thus identified the cases and variants of interest, the number of putative phased variants (PVs) in each tumor was next identified. To function as a PV on a single cell-free DNA (cfDNA) molecule, two variants, such as two single nucleotide variants (SNVs) generally must occur within a genomic distance less than the length of a typical cfDNA molecule (~170bp). Therefore, putative PVs were defined as two variants occurring on the same chromosome within a genomic distance of < 170bp. DNVs, TNVs, and ONVs were considered as the set of their respective component SNVs. The number of SNVs as well as the identity of putative PVs for each case are detailed in **Table 1.** The raw number of SNVs and putative PVs, as well as the number of putative PVs controlling for the number of SNVs, is shown in **FIG. 5A-C.**

### 10(a)(2): Mutational Signatures of Phased Variants from WGS

To assess the mutational processes associated with phased and non-phased mutations across different cancer types/subtypes, the mutational signatures of single base substitutions (SBS) were enumerated for each WGS case described above using the R package 'deconstructSigs'. The list of SNVs for each patient was first divided into two groups: 1) SNVs contained within a possible PV; that is, with an adjacent or 'nearest neighbor' SNV <170bp away, and 2) isolated SNVs (i.e., non-phased), defined as those occurring ≥170bp in distance from the closest adjacent SNV. 'DeconstructSigs' was then applied using the 49 SBS signatures described in COSMIC (excluding signatures linked to possible sequencing artefacts) to assess the contribution of each SBS signature to both candidate phased SNVs and un-phased SNVs for each patient. To compare the contribution of each SBS signature to phased and isolated SNVs, a Wilcoxon signed rank test was performed to compare the relative contribution of each SBS signature between these two categories for each cancer type **(FIGs. 6A-6WW).** To account for multiple hypotheses, Bonferroni's correction was applied, by considering any SBS signature that differed in contribution to phased vs. un-phased SNVs to be significant if the Wilcoxon signed rank test resulted in a P-value of <0.05 / 49 or 0.001. The distributions of these comparisons, along with significance testing, are depicted in **FIGs. 6A-6WW.** A summary of this analysis is also shown in **FIG. 1C** using a heat-map display, where the 'heat' represents the difference between the mean contribution of the SBS signature to phased variants to the mean contribution to isolated/un-phased variants.

### 10(a)(3): Genomic Distribution of Phased Variants from WGS

The recurrence frequency for PVs was assessed in each cancer type across the genome within each tumor type. Specifically, the human genome (build GRCh37 / hg19) was first divided into 1-kb bins (3,095,689 total bins); then, for each sample, the number of PVs (as defined above) contained in each 1-kb bin was counted. For this analysis, any PV with at least one of its constituent SNVs falling within the 1-kb bin of interest was included. The fraction of patients whose tumors harbored a PV for each cancer type within each genomic bin was then calculated. To identify 1-kb bins recurrently harboring PVs across patients, the fraction of patients containing PVs in each 1-kb bin vs. genomic coordinates **(FIG. 1D and FIG. 7)** was plotted; for this analysis, only bins where at least 2% of samples contained a PV in at least one cancer subtype were plotted.

### 10(a)(4): Identification of Recurrent 1-kb Bins with Phased Variants

To identify 1-kb bins that recurrently contain PVs in B-lymphoid malignancies, WGS data was utilized from the following diseases: DLBCL, FL, BL, and CLL. Any 1-kb bin where >1 sample from these tumor types was considered to recurrently contain PVs from B-lymphoid malignancies. The genomic coordinates of 1-kb bins containing recurrent PVs in lymphoid malignancies are enumerated in **Table 2,** and are plotted in **FIG. 8A.**

### 10(b): Design of PhasED-Seq panel for B-lymphoid malignancies

### 10(b)(1): Identification of recurrent PVs from WGS data at higher resolution

Given the prevalence of recurrent putative PVs from WGS data in B-cell malignancies, a targeted sequencing approach was designed for their hybridization-mediated capture - Phased variant Enrichment Sequencing (PhasED-Seq) - to enrich these specific PV events from tumor or cell-free DNA. In addition to the ICGC data described above, WGS data was also utilized from other sources in this design, including both B-cell NHLs as well as CLL.

Previous experience with targeted sequencing from cfDNA in NHLs was also examined. Pairs of SNVs occurring at a distance of <170bp apart in each B-cell tumor sample were identified. Then, genomic "windows" that contained PVs was identified as follows: for each chromosome, the PVs were sorted by genomic coordinates relative to reference genome. Then, the lowest (i.e., left-most) position was identified for any PV in any patient; this defined the left-hand (5') coordinate seeding a desired window of interest, to be captured from the genome. This window was then extended by growing its 3' end to capture successive PVs until a gap of ≥340bp was reached, with 340-bp chosen as capturing two successive chromatosomal sized fragments of ~170-bp. When such a gap was reached, a new window was started, and this iterative process of adding neighboring PVs was repeated again until the next gap of ≥340bp was reached. This resulted in a BED file of genomic windows containing all possible PVs from all samples considered. Finally, each window was additionally padded by 50bp on each side, to enable efficient capture from flanking sequences in rare scenarios when repetitive or poorly mapping intervening sequences might preclude their direct targeting for enrichment.

Having identified the regions of interest containing putative PVs, each window was then into 170bp segments (e.g., the approximate size of a chromatosomal ctDNA molecule). Then, the number of cases containing a PV was enumerated in each case. For each 170bp region, the region in final sequencing panel design was included if one or more of the following criteria was met: 1) at least one patient contained a PV in the 170bp region in 3 of 5 independent data-sets, 2) at least one patient contained a PV in the region in 2 of 5 independent data-sets if one dataset was prior CAPP-Seq experience, or 3) at least one patient contained a PV in the region in 2 of 5 independent data-sets, with a total of at least 3 patients containing a PV in the region. This resulted in 691 'tiles', with each tile representing a 170bp genomic region. These tiles, along with an additional ~200kb of genomic space targeting driver genes recurrently mutated in B-NHL, were combined into a unified targeted sequencing panel as previously described for both tumor and ctDNA genotyping using NimbleDesign (Roche NimbleGen). The final coordinates of this panel are provided in **Table 3.**

*10(b)(2): Comparison of PhasED-Seq and CAPP-Seq Performance in PV Yield*

To evaluate the performance of PhasED-Seq for capturing both SNVs and PVs compared to previously reported CAPP-Seq selector for B-cell lymphomas, the predicted number of both SNVs and PVs that may be recovered with each panel by limiting WGS *in silico* to the capture targets of each approach **(FIG. 9A-C)** was quantified. The predicted number of variants was then compared using the Wilcoxon signed rank test. Both CAPP-Seq and PhasED-Seq were also performed on 16 samples from patients with DLBCL. In these samples, tumor or plasma DNA, along with matched germline DNA, was sequenced. The resulting number of variants were again compared by the Wilcoxon signed rank text **(FIG. 2B,** and **FIGs. 9D-9E).** The sequencing depth for the samples included in this analysis are provided in **Tables 4.**

## 10(c): Identification of phased variants from targeted sequencing data

### 10(c)(1): Patient enrollment and clinical sample collection

Patients with B-cell lymphomas undergoing front-line therapy were enrolled on this study from six centers across North America and Europe, including Stanford University, MD Anderson Cancer Center, the National Cancer Institute, University of Eastern Piedmont (Italy), Essen University Hospital (Germany), and CHU Dijon (France). In total, 343 cell-free DNA, 73 tumor, and 183 germ-line samples from 183 patients were included in this study. All patient samples were collected with written informed consent for research use and were approved by the corresponding Institutional Review Boards in accordance with the Declaration of Helsinki. Cell-free, tumor, and germ-line DNA were isolated as previously described. All radiographic imaging was performed as part of standard clinical care.

### 10(c)(2): Library preparation and sequencing

To generate sequencing libraries and targeted sequencing data, CAPP-Seq was applied as previously described. Briefly, cell-free, tumor, and germ-line DNA were used to construct sequencing libraries through end repair, A-tailing, and adapter ligation following the KAPA Hyper Prep Kit manufacturer's instructions with ligation performed overnight at 4°C. CAPP-Seq adapters with unique molecular identifiers (UMIDs) were used for barcoding of unique DNA duplexes and subsequent deduplication of sequencing read pairs. Hybrid capture was then performed (SeqCap EZ Choice; NimbleGen) using the PhasED-Seq panel described above. Affinity capture was performed according to the manufacturer's protocol, with all 47 °C hybridizations conducted on an Eppendorf thermal cycler. Following enrichment, libraries were sequenced using an Illumina HiSeq4000 instrument with 2x150bp paired-end (PE) reads.

### 10(c)(3): Pre-processing and alignment

FASTQ files were de-multiplexed and UMIDs were extracted using a custom pipeline as previously described. Following demultiplexing, reads were aligned to the human genome (build GRCh37 / hg19) using BWA ALN. Molecular barcode-mediated error suppression and background polishing (i.e., integrated digital error suppression; iDES) were then performed as previously described.

*10(c)(4): Identification of phased variants and allelic quantitation*

After generating UMID error-suppressed alignment files (e.g., BAM files), PVs were identified from each sample as follows. First, matched germ-line sequencing of uninvolved peripheral blood mononuclear cells (PBMCs) was performed to identify patient-specific constitutional single nucleotide polymorphisms (SNPs). These were defined as non-reference positions with a variant allele fraction (VAF) above 40% with a depth of at least 10, or a VAF of above 0.25% with a depth of at least 100. Next, PVs were identified from read-level data for a sample of interest. Following UMID-mediated error suppression, each individual paired-end (PE) read and identified all non-reference positions were using 'samtools calmd'. PE data was used rather than single reads to identify variants occurring on the same template DNA molecule, which may subsequently fall into either read 1 or read 2. Any read-pair containing $\geq$2 non-reference positions was considered to represent a possible somatic PV. For reads with >2 non-reference positions, each permutation of size $\geq$2 was considered independently: i.e., if 4 non-reference positions were identified in a read-pair, all combinations of 2 SNVs (i.e., 'doublet' phased variants) and all combinations of 3 SNVs (i.e., 'triplet' phased variants) were independently considered. PVs containing putative germ-line SNPs were also removed as follows: if in a given n-mer (i.e., *n* SNVs in phase on a given molecule) $\geq$*n*-1 of the component variants were identified as germ-line SNPs, the PV was redacted. This filtering strategy ensures that for any remaining PV, at least 2 of the component SNVs were not seen in the germ-line, as relevant for both sensitivity and specificity.

Putative somatic PVs were filtered using a heuristic blacklisting approach in considering sequencing data from 170 germ-line DNA samples serving as controls. In each of these samples, PVs were identified on read-pairs as described above, but without filtering for matched germ-line. Any PV that occurred in one or greater paired-end read, in one or more of these control samples, was included in the blacklist and removed from patient-specific somatic PV lists.

To calculate the VAF of each PV, a numerator representing the number of DNA molecules containing a PV of interest was calculated over a denominator representing the total number of DNA molecules that covered the genomic region of interest. That is, the numerator is simply the total number of deduplicated read-pairs that contain a given PV while the denominator is the number of read-pairs that span the genomic locus of a given PV.

*10(c)(5): Genotyping phased variants from pretreatment samples*

The above strategy resulted in a list of PVs of $\geq$1 read-depth in each sample. To identify PVs serving as tumor-specific somatic reporters for disease monitoring, for each case a 'best genotyping' specimen - either DNA from a tumor tissue biopsy (preferred), or pretreatment cell-free DNA was identified. After identifying all possible PVs in the 'best genotyping sample', the list for specificity was further filtered as follows. For any n-mer PV set, if $\geq$*n*-1 of the constituent SNVs were present as germ-line SNPs in the 170 control samples described above, the PV was removed. Furthermore, only PVs that meet the following criteria were considered: 1) AF > 1%; 2) depth of the PV locus of $\geq$100 read-pairs, and 3) at least one component SNV must be in the on-target space. Finally, 4) any PV meeting these criteria was assessed for read-support in a cohort of 12 healthy control ctDNA samples. If any read-support was present in >1 of these 12 samples, the PV was removed. For genotyping from cell-free DNA samples identified as low tumor fraction by SNVs (i.e., < 1% mean AF across all SNVs), the AF threshold for determining PVs was relaxed to >0.2%. This filtering resulted in the PV lists used for disease monitoring and MRD detection.

*10(c)(6): Determination of tumor fraction in a sample from phased variants*

For evaluation of a sample for minimal residual disease (MRD) detection with prior knowledge of the tumor genotype, the presence of any PV identified in the best pretreatment genotyping sample in the MRD sample of interest can be assessed. Given a list of k possible tumor-derived PVs observed in the best genotyping sample, all read-pairs covering at least 1 of the *k* possible PVs were determined. This value, d, can be thought of as the aggregated 'informative depth' across all PVs spanned by cfDNA molecules in a PhasED-Seq experiment. It was then assessed how many of these d read-pairs actually contained 1 or more of the k possible PVs - this value, x, represents the number of tumor-derived molecules containing somatic PVs in a given sample. The number of tumor-derived molecules containing PVs divided by the informative depth - x/d - is therefore the phased-variant tumor fraction (PVAF) in a given sample. For detection of MRD in each sample, PVAF was calculated independently for doublet, triplet, and quadruplet PVs.

*10(c)(7): Monte Carlo simulation for empirical significance of PV detection within a specimen*

To assess the statistical significance of the detection of tumor-derived PVs in any sample, an empiric significance testing approach was implemented. A test statistic *f* was first defined as follows - from a given list of *k* possible tumor-derived PVs observed in the best genotyping sample, the arithmetic mean of allele fractions was calculated across all *k* PVs (allele fraction defined as the number of read-pairs containing an individual PV ($x_i$) over the number of read-pairs spanning the PV positions ($d_i$)):

$$f = \frac{\sum_{i=1}^{k} \frac{x_i}{d_i}}{k} \qquad (1)$$

to assess the hypothesis that f is not significantly different from the background error-rate of similar PVs assessed from the same sample. A Monte Carlo approach was used to develop a null distribution and perform statistical testing as follows:

1. Given a set of $k$ PVs, $\{pv_1 \dots pv_i \dots pv_k\}$, an 'alternate' list of PVs, $\{pv'_1 \dots pv'_i \dots pv'_k\}$, was generated such that for each alternate PV had the same type of base change and distance between SNVs as the test PV. For example, if a doublet PV, chr14:106329929 C>T and chr14:106329977 G>A, was identified in the genotyping sample and searched for an alternate two positions at the same genomic distance (here, 48bp) with reference bases C and G, and assessed for read-pairs with the same types of base changes (i.e., C>T and G>A), using the heuristic search scheme below.

2. For each tumor $pv_i$ in the set of $k$, 50 such alternates were identified. This was performed with a random search algorithm to scan the genomic space and identify alternates. To find these 50 alternates, a random position on the same chromosome as the test $pv_i$ was identified and then searched for the same types of reference bases at the same genomic distance as described above. Synteny of observed/alternate PVs was used to control for regional variation in SHM/aSHM as well as copy number variation, as potential confounders of the null distribution. Alternate positions that were identified as a germ-line SNP, defined as having AF > 5%, were excluded.

3. After identifying 50 such alternates for each $pv_i$, 10,000 random permutations of 1 alternate were generated for each of the k original PVs and calculated the phased-variant fraction f' for these alternate lists in the sample of interest being evaluated for presence of MRD, as described above.

4. An empiric P-value was calculated, defined as the fraction of times the true phased-variant fraction f is observed to be less than or equal to the alternate f' across the 10,000 random PV lists as an empirical measure of significance of MRD significance in the blood sample of interest.

While this resulting comparison is a measure of the significance for PV detection of tumor-reporter list compared to the empirically defined background PV error-rate *within* the sample of interest, its relationship to specificity of detection *across* cases and control samples was also evaluated, as described below.

### 10(c)(8): Assessment of specificity of PhasED-Seq

To determine the specificity of disease and MRD detection through PhasED-Seq, patient-specific PVs from 107 patients with DLBCL were first identified using pretreatment tumor or plasma DNA along with paired germ-line samples. 40 independent plasma DNA samples were then assessed from healthy individuals for presence of these patient-specific PVs, using the Monte Carlo approach outlined above. A threshold for P-values was empirically determined from Monte Carlo such that 95% specificity was achieved for disease detection from doublet, triplet, and quadruplet PVs. The P-value threshold yielding ≥95% specificity for each size of PV was as follows: < 0.041 for doublets, <1 for triplets, and <1 for quadruplets. The results of this specificity in control ctDNA analysis is shown in **FIGs. 15 and 16A and 16B.**

### 10(c)(9): Calculation of error rates

To assess the error profile of both isolated SNVs and PVs, the non-reference base observation rate of each type of variant was examined across all reads. For isolated SNVs, the error-rate for each possible base change $e_{n1>n1'}$ was calculated as the fraction of on-target bases with reference allele $n1$ that are mutated to alternate allele $n1'$, when considering all possible base-changes of the reference allele. Positions with a non-reference allele rate exceeding 5% were classified as probable germ-line events, and excluded from the error-rate analysis. A global error rate, defined as the rate of mutation from the hg19 reference allele to any alternate allele, was also calculated.

For phased variants, a similar calculation was performed. For the error-rate of a given type of phased variant composed of k constituent base-changes $\{e_{n1>n1'} \dots e_{nk>nk'}\}$, the error-rate was calculated by determining both the number of instances of the type of base change (i.e., the numerator), as well as the number of possible instances for the base change (i.e., the denominator). To calculate the numerator, N, the number of occurrences of the PV of interest over all read-pairs was counted in a given sample. For example, to calculate the error-rate of C>T and G>A phased doublets, the number of read-pairs that include *both* a reference C mutated to a T as well as a reference G mutated to an A was first counted.

To calculate the denominator, D, the number of possible instances of this type of phased variant was also calculated; this was performed first for each read-pair i, and then summed over all read pairs. A PV with k components can be summarized as having certain set of reference bases $P_A$, $p_C$, $P_G$, $p_T$, where $p_N$ is the number of each reference base in the PV. Similarly, a given read pair contains a certain set of reference bases $b_A$, $b_C$, $b_G$, $b_T$, where $b_N$ is the number of each reference base in the read pair. Therefore, for each read pair in a given sample, the number of possible occurrences of PV type of interest can be calculated combinatorially as:

$$D_i = \binom{b_A}{p_A}\binom{b_C}{p_C}\binom{b_G}{p_G}\binom{b_T}{p_T} \tag{2}$$

For example, consider a read-pair with 40 reference As, 50 reference Cs, 45 reference Gs, and 35 reference Ts. The number of positions for a C>T and G>A PV is:

$$D_i = \binom{40}{0}\binom{50}{1}\binom{45}{1}\binom{35}{0} = 2250 \tag{3}$$

The aggregated denominator, D, for error rate calculation is then simply the sum of this value over all read pairs. The error rate for this type of PV is then simply $N/D$.

**10(d): Differences in phased variants between lymphoma subtypes**

To compare the distribution of phased variants in different types of lymphomas, tumor-specific PVs were identified in 101 DLBCL, 16 PMBCL, and 23 cHL patients via sequencing of tumor biopsy specimens and/or pre-treatment cell-free DNA and paired germ-line specimens. After identifying these tumor-specific PVs, their distribution was the assessed across the targeted sequencing panel. The panel was first divided into 50bp bins; for each patient, it was then determined if each patient had evidence of a PV within the 50bp bin, defined as having at least one component of the PV within the bin. The nearest gene to each 50bp bin was further determined, based on GENCODEv19 annotation of the reference genome.

To assess how the distribution of PVs between subtypes of lymphoma varies at the level of specific genes, the distribution of PVs was examined across the 50bp bins spanning each gene (or nearest gene). For example, consider a given gene with n such 50bp bins represented in targeted sequencing panel. For each bin, it was first determined the fraction of patients, $f$, in each type of lymphoma with a PV falling within the 50bp bin - i.e., determining $\{f_{type1,1}, ... f_{type1,n}\}$ and $\{f_{type2,1}, ... f_{type2,n}\}$. Then, any two histologies were then compared for the fraction of cases harboring PVs in the set of 50bp bins assigned to each gene. These comparisons are depicted for individual genes on gene-specific plots in **FIG. 2D** and **FIGs. 10-12.**

The enrichment in PVs was statistically compared in a specific lymphoma type or subtype vs. another by calculating the difference in the fraction of patients which contain a PV in each 50bp bin across all bins assigned to a gene (i.e., overlapping a given gene or with a given nearest gene). Specifically, for any comparison between two lymphoma types ($type_1$ and $type_2$), this set of differences in PV-rate was first identified between histologies $\{f_{type1,1} - f_{type2,1}, ... f_{type1,n} - f_{type2,n}\}$. This set of gene-specific differences in frequency of PVs was the compared between types of lymphoma against the distribution of all other 50bp bins in the sequencing panel by the Wilcoxon rank sum test. For this test, the set of $n$ 50bp bins assigned to a given gene was compared to all other 50bp bins (i.e., 6755 - n, since there are 6755 50bp bins in sequencing panel). This P-value, along with the mean difference in fraction of patients with a PV in each bin for each gene between histologies, is depicted as a volcano plot in **FIG. 2E.** To account for the global difference in rate of PVs between different histologies, the mean difference in fraction of patients with a PV between histologies was centered on 0 by subtracting the mean difference across all genes.

**10(e): Hybridization Bias**

To assess the effect of mutations on hybridization efficiency, the affinity of mutated molecules to wildtype capture baits *in silico* was first estimated by considering DNA fragments harboring 0-30% mutations across the entire fragment. For each mutation condition across this range, 10,000 regions were first randomly sampled, each 150bp in length, from across the whole genome. These 150-mers were then mutated *in silico* to simulate the desired mutation rate in 3 different ways: 1) mutating 'clustered' or contiguous bases starting from the ends of a sequence, 2) mutating clustered bases started from the middle of the sequence, or 3) mutating bases selected at random positions throughout the sequence. The *energy.c* package was then used to calculate the theoretical binding energy (kcal/mol) between the mutated and wild-type sequences, in relying on a nearest-neighbor model employing established thermodynamic parameters **(FIG. 14A).**

This *in silico* experiment was then replicated by testing the effects of same mutation rates *in vitro.* Specifically, oligonucleotides (IDT) were synthesized and annealed to form DNA duplexes harboring 0-10% mutations at defined positions relative to the human reference genome sequence. These synthetic DNA molecules were then captured together at equimolar concentrations and quantified the relative capture efficiency of mutated duplexes compared to the wild-type, unmutated species **(FIG. 3A)**. Two sets of oligonucleotide sequences were selected from coding regions of *BCL6* and *MYC* to capture AID-mediated aberrant somatic hypermutations associated with each gene **(Table 5)**; the preserved mappability of the mutated species was ensured by BWA ALN. These synthetic oligonucleotide duplexes were then subjected to library preparation, then captured and sequenced using PhasED-Seq, performed in triplicate using distinct samples. This allowed assessment of the relative efficiency of hybrid capture and molecular recovery as directly compared to wildtype molecules identical to the reference genome.

**10(f): Assessment of limit of detection with limiting dilution series**

To empirically define the analytical sensitivity of PhasED-Seq, a limited dilution series of cell-free DNA from 3 patients that were spiked into healthy control cell-free DNA at defined concentrations was utilized. The dilution series contained samples with an expected mean tumor fraction of 0.1%, 0.01%, 0.001%, 0.0002%, 0.0001%, and 0.00005% or ranging from 1 part in 1,000 to 1 part in 2,000,000. The sequencing characteristics and ctDNA quantification via CAPP-Seq, duplex sequencing, and PhasED-Seq are provided. To compare the performance of each method, the difference was calculated, $\delta$, between the observed and expected tumor fraction for each patient i at each dilution concentration $j$:

$$\delta_{i,j} = \widehat{tumorfrac}_{i,j} - tumorfrac_{i,j} \qquad (4)$$

This value was calculated for patients $i = \{1,2,3\}$ and concentrations $j = \{0.001\%, 0.0002\%, 0.0001\%, 0.00005\%\}$ for each ctDNA detection method (CAPP-Seq, duplex, doublet PhasED-Seq, and triplet PhasED-Seq). The performance of each method was then compared to each other by paired t-test across this set of patients and concentrations.

**10(g): Model to predict the probability of detection for a given set of phased variants**

To build a mathematical model to predict the probability of detection for a given sample of interest, it began with the common assumption that ctDNA detection can be considered a random process based on binomial sampling. However, unlike SNVs occurring at large genomic distances apart from one another, detection of PVs can be highly interdependent, especially when PVs are degenerate (i.e., when two PVs share component SNVs) or occur in close proximity. To account for this, only PVs occurring >150bp apart from each other was considered as independent 'tumor reporters'. The number of 'tumor reporters' to allow for disease detection in a given sample can thus be determined as follows. The PhasED-Seq panel was broken apart into 150bp bins. Each PV in a given patient's reporter list was then turned into a BED coordinate, consisting of the start position (defined as the left-most component SNV) and end position (defined as the right-most component SNV). For each PV, the 150bp bin from the PhasED-Seq selector panel containing the PV was determined; if a PV spanned two or more 150bp bins, it was assigned to both bins. The number of independent tumor reporters was then defined as the number of separate 150bp bins containing a tumor-specific PV.

A mathematical model was then developed comparing the expected probability of detection for a given sample at a given tumor fraction with a given number of independent tumor reporters (e.g., 150 bp bins). With a given number of tumor reporters r, at a given tumor fraction *f*, with a given sequencing depth *d*, the probability of detecting 1 or more cell-free DNA molecule containing a tumor-specific PV containing can be defined as:

$$Pr(detection) = 1 - Pr(nondetection) \qquad (5)$$

$$= 1 - \binom{d * r}{0} f^0 (1 - f)^{d*r} \qquad (6)$$

based on simple binomial sampling. However, as ctDNA detection method was trained to have a 5% false positive rate, this false positive rate term was added to the model as well:

$$Pr(detection) = 1 - Pr(nondetection) + 0.05 * Pr(nondetection) \qquad (7)$$

$$Pr(detection) = 1 - 0.95 * Pr(nondetection) \qquad (8)$$

$$= 1 - 0.95 * \binom{d * r}{0} f^0 (1 - f)^{d * r} \qquad (9)$$

**FIG. 3G** shows the results of this model for a range of tumor reporters r from 3 to 67 at depth d of 5000. The confidence envelope on this plot shows solutions for a range of depth d from 4000 to 6000.

To empirically validate this model assessing the probability of disease detection, samples from limiting dilution series were utilized. In this dilution series, 3 patient ctDNA samples, each containing patient-specific PVs, were spiked into healthy control cfDNA. For each list of patient specific PVs, 25 random subsamplings of the 150bp bins containing patient-specific PVs were performed to generate reporter lists containing variable numbers of tumor-specific reporters. A maximum bin number of 67 was selected to allow sampling from all 3 patient-specific PV lists, followed by scaling down the number of bins by 2x or 3x per operation. This resulted in reporter lists containing patient-specific PVs from 3, 6, 17, 34, or 67 independent 150bp bins. Disease detection was then assessed using each of these patient-specific PV lists of increasing size in each of 'wet' limiting dilution samples from 1:1,000 to 1:1,000,000 **(FIG. 3H,** closed circles). *In silico* mixtures was further created using sequencing reads from limiting dilution samples with varying expected tumor-content, and again assessed for the probability of disease detection using patient-specific subsampled PV reporter lists of varying lengths (open circles). For this experiment, both the 'wet' and *'in-silico'* dilution bam files were down-sampled to achieve a depth of ~4000-6000x to correspond with modeled depth. The final mean and standard deviation of depth across all down-sampled bam files was 4214x $\pm$ 789. The probability of detection was summarized across all tests at a given expected tumor fraction, for a given patient-specific PV list. For each given dilution, multiple independently sampled sets of reads were considered to allow superior estimation of the true probability of detection. Specifically, the following number of replicates at each dilution indicated was considered in **Table 7**.

Table 7. Replicates at each dilution for predicting the probability of detection for a given set of phased variants.

| Dilution | Replicates | Number of Tests (Replicates * 25) | Wet or *In silico* |
|---|---|---|---|
| 1 : 1,000 | 1 | 25 | Wet |
| 5 : 10,000 | 3 | 75 | *In silico* |
| 3.5 : 10,000 | 3 | 75 | *In silico* |
| 2 : 10,000 | 3 | 75 | *In silico* |
| 1 : 10,000 | 3 | 75 | Wet |
| 5 : 100,000 | 3 | 75 | *In silico* |
| 3.5 : 100,000 | 3 | 75 | *In silico* |
| 2 : 100,000 | 3 | 75 | *In silico* |
| 1 : 100,000 | 3 | 75 | Wet |
| 5 : 1,000,000 | 8 | 200 | *In silico* |
| 3.5 : 1,000,000 | 8 | 200 | *In silico* |
| 2 : 1,000,000 | 8 | 200 | Wet |
| 1 : 1,000,000 | 8 | 200 | Wet |

The total number of tests, for each patient-specific PV list, is therefore the number of randomly subsampled PV lists (e.g., 25) times the number of independently downsampled bam files; this number is provided in the table above. In **FIG. 3H,** the points and error-bars represent the mean, minimum, and maximum across all three patients. The concordance between the predicted probability of disease detection from theoretical mathematical model and wet and *in silico* samples validating this model, is shown in **FIG. 3I.**

### 10(h): Statistical Analyses & Software Availability

All P-values reported in this manuscript are 2-sided unless otherwise noted. Comparisons of matched samples and populations were performed using the Wilcoxon signed rank test; comparisons of samples drawn from unrelated populations were performed using the Wilcoxon rank-sum test. Comparisons of paired samples were performed by paired t-test. Survival probabilities were estimated using the Kaplan-Meier method; survival of groups of patients based on ctDNA levels were compared using the log-rank test. Other statistical tests are noted in the manuscript text where utilized. All analyses were performed with the use of MATLAB, version 2018b, R Statistical Software version 3.4.1, and GraphPad Prism, version 8.0.2. The contribution of known mutational processes to phased and isolated SNVs from WGS was assessed with the deconstruct Sigs R package using the COSMIC signature set (v2) as described. Calculation of AUC accounting for survival and censorship was performed using the R 'survivalROC' package version 1.0.3 with default settings. An executable version of the PhasED-Seq software, developed in C++17, is available at phasedseq(dot) stanford(dot)edu.

**Example 11**

Using methods and systems of the present disclosure, cell-free nucleic acid molecules may be analyzed to detect insertions and deletions (indels) contained therein, and the detected indels may be applied toward various applications (e.g., determining a presence or absence of a condition in a subject, such as a neoplasm of the subject, a cancer of the subject, a transplant rejection of the subject, or a chromosomal abnormality of a fetus of the subject; and determining whether cell-free nucleic acid molecules are tumor-derived).

For example, using methods and systems of the present disclosure, cell-free nucleic acid molecules may be analyzed from a subject who has received an organ or tissue transplant to detect phased variants and/or insertions and deletions (indels) contained therein, and the detected PVs and/or indels may be applied toward various applications (e.g., determining a presence or absence of a transplant rejection of a subject.

As another example, using methods and systems of the present disclosure, cell-free nucleic acid molecules may be analyzed from a pregnant subject to detect phased variants and/or insertions and deletions (indels) contained therein, and the detected PVs and/or indels may be applied toward various applications (e.g., determining a presence, an absence, or an elevated risk of a genetic abnormality of a fetus of the pregnant subject).

While indels share some factors in common with phased variants (e.g., they contain multiple non-reference bases), indels may also differ from phased variants in various ways (e.g., biological differences, where a biological indel can occur with a single DNA replication error, while a PV may require two separate errors; and technical errors related to mapping, in which an indel may require one mismatch and/or non-templated event, while a phased variant may require two or more such mismatches and/or non-templated events).

In some embodiments, the indels alone that are detected in cell-free nucleic acid molecules may be applied toward various applications by leveraging their low background or error rates (e.g., determining a presence or absence of a condition in a subject, such as a neoplasm or cancer; and determining whether cell-free nucleic acid molecules are tumor-derived). In some embodiments, the detected indels in combination with detected phased variants in cell-free nucleic acid molecules may be applied toward various applications (e.g., determining a presence or absence of a condition in a subject, such as a neoplasm or cancer; and determining whether cell-free nucleic acid molecules are tumor-derived).

A set of 12 healthy ctDNA samples used to assess the error or background rate in iDES-enhanced CAPP-Seq, duplex sequencing, and PhasED-Seq, was analyzed to assess for the error-rate of indels as well. This analysis was performed on the same sequencing data, making the error-rates comparable. The error or background rate was defined for each of these types of alterations as follows. The SNV background rate was defined as the number of non-reference bases over the total number of bases, as described herein. The indel background rate was defined as the total number of indels observed after mapping over the total number of bases, as described herein. The PV background rate was defined as the total number of combinations of non-reference PVs over the total number of possible PVs for a given size, as described herein.

All events occurring at greater than 5% allele fraction were considered to be germline and were not included here. In addition to the observed background in SNVs and PVs reported, **FIG. 28** shows the background rate of indels of all sizes, greater or equal to 2 base pairs, greater or equal to 3 bps, and greater or equal to 4bps, and across this set of 12 healthy control ctDNA samples.

As **FIG. 28** demonstrates, the error profile of indels improves when only larger indels are considered. Interestingly, the background rate for indels of length 1 bp or larger was observed to be similar to the background rate for SNVs without in silico error suppression (8.0E-5 vs. 8.0E-5, respectively). However, longer indels (e.g., specifically those greater than or equal to 4bp long) had a lower background rate, comparable with the background rate of SNVs from duplex sequencing (8.9E-6 vs 1.2E-5). However, the background rate of both doublet and triplet PVs was observed to be lower than that of both the duplex and larger indels (background rate of 8.0E-7 and 3.5E-8 respectively for doublet and triplet PVs). Notably, this lower background for PVs was true even without the use of UMIs or molecular barcodes.

This lower background rate for PVs is likely biological in origin. As discussed herein, there is substantial potential for true biological background in SNVs or indels, which may be greater than for PVs, as each of the SNVs or indels may only require one somatic mutational event, while PVs may require at least two somatic events. Nevertheless, the background rate for PVs supports its utility for improving the limit of detection for low-level tumor burden from cell-free DNA. However, in cases with low numbers of PVs, tracking longer indels (e.g., greater than or equal to 3 bp in length) may provide an alternative source of low error-rate tumor-reporters to enable ultra-sensitive tumor monitoring. Therefore, indel monitoring may be leveraged as a complementary or alternative approach to the detection and analysis of PVs in cell-free DNA.

## Example 12

Using methods and systems of the present disclosure, cell-free nucleic acid molecules may be analyzed from a subject who has received an organ or tissue transplant to detect phased variants and/or insertions and deletions (indels) contained therein, and the detected PVs and/or indels may be applied toward various applications (e.g., determining a presence or absence of a transplant rejection of a subject). In some embodiments, the subject has received a transplant of an organ (e.g., heart, kidney, liver, lung, pancreas, stomach and intestine), a tissue (e.g., cornea, bone, tendon, skin, pancreas islets, heart valves, nerves and veins), cells (e.g., bone marrow and stem cells), or a limb (e.g., a hand, an arm, a foot).

In some embodiments, upon identifying a subject as having a transplant rejection, the method may further comprise treating the subject for the transplant rejection. In some embodiments, the treatment comprises an immunosuppressive drug, an anti-body based treatment, a blood transfer, a marrow transplant, a gene therapy, a transplant removal, and/or a re-transplant procedure. In some embodiments, the immunosuppressive drug comprises a corticosteroid (e.g., prednisolone, hydrocortisone), a calcineurin inhibitor (e.g., ciclosporin, tacrolimus), an antiproliferative (e.g., azathioprine, mycophenolic acid), or an mTOR inhibitor (e.g., sirolimus, everolimus). In some embodiments, the antibody-based treatment comprises a monoclonal anti-IL-2R$\alpha$ receptor antibody (e.g., basiliximab, daclizumab), a polyclonal anti-T-cell antibody (e.g., anti-thymocyte globulin (ATG), anti-lymphocyte globulin (ALG)), or a monoclonal anti-CD20 antibody (e.g., rituximab).

In some embodiments, the subject may be monitored over time (e.g., by analyzing cell-free nucleic acid molecules to detect PVs and/or indels at a plurality of different time points) to assess the transplant rejection status of the subject and/or to determine a progression of the transplant rejection status of the subject.

In some embodiments, the detected PVs and/or indels of a subject may be compared to those of a first subject cohort having transplant rejection and/or a second subject cohort not having transplant rejection.

## Example 13

Using methods and systems of the present disclosure, cell-free nucleic acid molecules may be analyzed from a pregnant subject to detect phased variants and/or insertions and deletions (indels) contained therein, and the detected PVs and/or indels may be applied toward various applications (e.g., determining a presence, an absence, or an elevated risk of a genetic abnormality of a fetus of the pregnant subject).

In some embodiments, upon identifying the fetus of the pregnant subject as having a genetic abnormality, the method may further comprise treating the subject or conducting follow-up clinical procedures (e.g., an invasive or non-invasive diagnostic procedure) for the pregnant subject.

In some embodiments, the detected PVs and/or indels of a subject may be compared to those of a first subject cohort having a fetus with a genetic abnormality and/or a second subject cohort not having a fetus with a genetic abnormality.

In some embodiments, the genetic abnormality is a chromosomal aneuploidy. In some embodiments, the chromosomal aneuploidy is in chromosome 13, 18, 21, X, or Y.

## Example 14

Additional details of the tables described throughout the present disclosure are provided herein:

TABLE 1: 1000bp regions of interest throughout the genome containing putative phased variants (PV) in various lymphoid neoplasms. Only regions containing > 1 subject with a PV are shown. Coordinates are in hg19. Regions from genes that were previously identified as targets of activation-induced deaminase (AID) are labeled. Regions that contain PVs in > 5% of subjects in any histology (BL, CLL, DLBCL, FL) are also labeled. BL, Burkitt lymphoma; CLL, chronic lymphocytic leukemia; DLBCL, diffuse large B-cell lymphoma; FL, follcicular lymphoma.

TABLE 2: 1000bp regions of interest throughout the genome containing putative phased variants (PV) in the ABC and GCB subtypes of DLBCL. Only regions containing > 1 subject with a PV are shown. Coordinates are in hg19. Regions from genes that were previously identified as targets of AID are labeled. ABC, activated B-cell subtype; GCB, germinal center B-cell subtype.

TABLE 3: Regions used for the PhasED-Seq capture reagent described in this paper focused on lymphoid malignancies. Coordinates are in hg19. The closest gene and the reason for inclusion (Phased Variants vs general DLBCL genotyping) is also shown.

TABLE 4: Enrichment of PVs at genetic loci throughout the PhasED-Seq targeted sequencing panel for different types of B-cell lymphomas (DLBCL including ABC and GCB subtypes, PMBCL, and cHL). The PhasED-Seq selector was binned into 50bp bins in hg19 coordinates, and each bin was labelled by gene or nearest gene. The mean of the fraction of cases of a given histology with a PV across all 50bp bins is shown. Significance was determined by rank-sum (Mann-Whitney U) test of 50bp bins for a given gene against the remainder of the sequencing panel. Uncorrected P-values are shown; multiple-hypothesis testing correction was performed by Bonferroni method. DLBCL, diffuse large B-cell lymphoma; PMBCL, primary mediastinal B-cell lymphoma; cHL, classical Hodgkin lymphoma; ABC, activated B-cell DLBCL; GCB, germinal center B-cell DLBCL.

**TABLE 5:** Sequences of oligonucleotides synthesized to assess hybridization and molecular recovery bias with increasing mutational burden (SEQ ID NOs. 1331-1358).

**TABLE 6:** Nucleic acid probes for Capture Sequencing of B-cell Cancers (SEQ ID NOs. 0001-1330).

Table 1

| # | Chromosome | Region Start | Region End | BL | CLL | DLBCL | FL | ClosestGene | Fisher_p_DLBCL_vs_FL | Fisher_p_DLBCL_vs_BL | Fisher_p_DLBCL_vs_CLL | Previously Identified | over5PctInAny Histology |
|---|---|---|---|---|---|---|---|---|---|---|---|---|---|
| 1 | chr1 | 756000 | 757000 | 0.028 | 0.000 | 0.015 | 0.000 | AL669831.1 | 0.47887 | 1.00000 | 0.29694 | 0 | 0 |
| 2 | chr1 | 1963000 | 1964000 | 0.028 | 0.000 | 0.015 | 0.000 | GABRD | 0.47887 | 1.00000 | 0.29694 | 0 | 0 |
| 3 | chr1 | 2052000 | 2053000 | 0.028 | 0.000 | 0.000 | 0.014 | PRKCZ | 1.00000 | 0.34615 | 1.00000 | 0 | 0 |
| 4 | chr1 | 3789000 | 3790000 | 0.000 | 0.000 | 0.029 | 0.000 | DFFB | 0.22755 | 0.54294 | 0.08726 | 0 | 0 |
| 5 | chr1 | 6613000 | 6614000 | 0.000 | 0.000 | 0.044 | 0.014 | NOL9 | 0.34948 | 0.54966 | 0.02537 | 1 | 0 |
| 6 | chr1 | 6614000 | 6615000 | 0.000 | 0.000 | 0.088 | 0.027 | NOL9 | 0.15270 | 0.09031 | 0.00058 | 1 | 1 |
| 7 | chr1 | 6661000 | 6662000 | 0.000 | 0.000 | 0.029 | 0.014 | KLHL21 | 0.60686 | 0.54294 | 0.08726 | 0 | 0 |
| 8 | chr1 | 6662000 | 6663000 | 0.000 | 0.000 | 0.044 | 0.014 | KLHL21 | 0.34948 | 0.54966 | 0.02537 | 0 | 0 |
| 9 | chr1 | 9129000 | 9130000 | 0.000 | 0.000 | 0.044 | 0.000 | SLC2A5 | 0.10727 | 0.54966 | 0.02537 | 0 | 0 |
| 10 | chr1 | 10894000 | 10895000 | 0.028 | 0.000 | 0.000 | 0.014 | C1orf127 | 1.00000 | 0.34615 | 1.00000 | 0 | 0 |
| 11 | chr1 | 17019000 | 17020000 | 0.028 | 0.000 | 0.000 | 0.014 | AL137798.1 | 1.00000 | 0.34615 | 1.00000 | 0 | 0 |
| 12 | chr1 | 17231000 | 17232000 | 0.000 | 0.000 | 0.015 | 0.014 | CROCC | 1.00000 | 1.00000 | 0.29694 | 0 | 0 |
| 13 | chr1 | 19935000 | 19936000 | 0.000 | 0.000 | 0.029 | 0.000 | MINOS1-NBL1 | 0.22755 | 0.54294 | 0.08726 | 0 | 0 |
| 14 | chr1 | 21091000 | 21092000 | 0.000 | 0.000 | 0.015 | 0.014 | HP1BP3 | 1.00000 | 1.00000 | 0.29694 | 0 | 0 |
| 15 | chr1 | 23885000 | 23886000 | 0.444 | 0.000 | 0.015 | 0.000 | ID3 | 0.47887 | 0.00000 | 0.29694 | 1 | 1 |
| 16 | chr1 | 28408000 | 28409000 | 0.000 | 0.000 | 0.029 | 0.000 | EYA3 | 0.22755 | 0.54294 | 0.08726 | 0 | 0 |
| 17 | chr1 | 32373000 | 32374000 | 0.000 | 0.000 | 0.029 | 0.000 | PTP4A2 | 0.22755 | 0.54294 | 0.08726 | 0 | 0 |
| 18 | chr1 | 36722000 | 36723000 | 0.000 | 0.012 | 0.015 | 0.000 | THRAP3 | 0.47887 | 1.00000 | 1.00000 | 0 | 0 |
| 19 | chr1 | 46576000 | 46577000 | 0.000 | 0.000 | 0.015 | 0.014 | PIK3R3 | 1.00000 | 1.00000 | 0.29694 | 0 | 0 |
| 20 | chr1 | 51965000 | 51966000 | 0.000 | 0.006 | 0.015 | 0.000 | EPS15 | 0.47887 | 1.00000 | 0.50663 | 0 | 0 |
| 21 | chr1 | 51978000 | 51979000 | 0.000 | 0.000 | 0.029 | 0.000 | EPS15 | 0.22755 | 0.54294 | 0.08726 | 0 | 0 |
| 22 | chr1 | 51983000 | 51984000 | 0.000 | 0.006 | 0.029 | 0.000 | EPS15 | 0.22755 | 0.54294 | 0.21104 | 0 | 0 |
| 23 | chr1 | 72393000 | 72394000 | 0.000 | 0.000 | 0.015 | 0.014 | NEGR1 | 1.00000 | 1.00000 | 0.29694 | 0 | 0 |
| 24 | chr1 | 73719000 | 73720000 | 0.000 | 0.000 | 0.029 | 0.000 | LRRIQ3 | 0.22755 | 0.54294 | 0.08726 | 0 | 0 |
| 25 | chr1 | 77315000 | 77316000 | 0.028 | 0.006 | 0.000 | 0.000 | ST6GALNAC5 | 1.00000 | 0.34615 | 1.00000 | 0 | 0 |
| 26 | chr1 | 81306000 | 81307000 | 0.000 | 0.000 | 0.015 | 0.014 | LPHN2 | 1.00000 | 1.00000 | 0.29694 | 0 | 0 |
| 27 | chr1 | 81527000 | 81528000 | 0.000 | 0.000 | 0.029 | 0.000 | LPHN2 | 0.22755 | 0.54294 | 0.08726 | 0 | 0 |
| 28 | chr1 | 82009000 | 82010000 | 0.028 | 0.000 | 0.015 | 0.000 | LPHN2 | 0.47887 | 1.00000 | 0.29694 | 0 | 0 |
| 29 | chr1 | 84106000 | 84107000 | 0.000 | 0.006 | 0.015 | 0.000 | TTLL7 | 0.47887 | 1.00000 | 0.50663 | 0 | 0 |
| 30 | chr1 | 87524000 | 87525000 | 0.000 | 0.000 | 0.015 | 0.000 | HS2ST1;HS2ST1LOC339524; | 0.47887 | 1.00000 | 0.50663 | 0 | 0 |
| 31 | chr1 | 94551000 | 94552000 | 0.000 | 0.000 | 0.029 | 0.000 | ABCA4 | 0.22755 | 0.54294 | 0.08726 | 0 | 0 |
| 32 | chr1 | 94552000 | 94553000 | 0.000 | 0.000 | 0.029 | 0.000 | ABCA4 | 0.22755 | 0.54294 | 0.08726 | 0 | 0 |
| 33 | chr1 | 103696000 | 103697000 | 0.000 | 0.000 | 0.000 | 0.027 | COL11A1 | 0.49735 | 1.00000 | 1.00000 | 0 | 0 |
| 34 | chr1 | 116979000 | 116980000 | 0.000 | 0.000 | 0.044 | 0.041 | ATP1A1 | 1.00000 | 0.54966 | 0.02537 | 0 | 0 |
| 35 | chr1 | 149784000 | 149785000 | 0.000 | 0.000 | 0.015 | 0.014 | HIST2H3D | 1.00000 | 1.00000 | 0.29694 | 1 | 0 |
| 36 | chr1 | 149821000 | 149822000 | 0.000 | 0.000 | 0.044 | 0.000 | HIST2H2AA4 | 0.10727 | 0.54966 | 0.02537 | 1 | 0 |
| 37 | chr1 | 149857000 | 149858000 | 0.000 | 0.000 | 0.015 | 0.014 | HIST2H2BE | 1.00000 | 1.00000 | 0.29694 | 1 | 0 |
| 38 | chr1 | 149858000 | 149859000 | 0.000 | 0.000 | 0.059 | 0.000 | HIST2H2AC;HIST2H2BE; | 0.05016 | 0.29551 | 0.00730 | 0 | 1 |
| 39 | chr1 | 160616000 | 160617000 | 0.000 | 0.000 | 0.015 | 0.014 | SLAMF1 | 1.00000 | 1.00000 | 0.29694 | 0 | 0 |
| 40 | chr1 | 162711000 | 162712000 | 0.000 | 0.000 | 0.015 | 0.014 | DDR2 | 1.00000 | 1.00000 | 0.29694 | 0 | 0 |
| 41 | chr1 | 163684000 | 163685000 | 0.000 | 0.000 | 0.015 | 0.014 | NUF2 | 1.00000 | 1.00000 | 0.29694 | 0 | 0 |
| 42 | chr1 | 167598000 | 167599000 | 0.000 | 0.000 | 0.044 | 0.014 | RCSD1 | 0.34948 | 0.54966 | 0.02537 | 0 | 0 |
| 43 | chr1 | 167599000 | 167600000 | 0.000 | 0.000 | 0.029 | 0.014 | RCSD1 | 0.60686 | 0.54294 | 0.08726 | 0 | 0 |
| 44 | chr1 | 167600000 | 167601000 | 0.000 | 0.000 | 0.044 | 0.000 | RCSD1 | 0.10727 | 0.54966 | 0.02537 | 0 | 0 |
| 45 | chr1 | 174333000 | 174334000 | 0.000 | 0.000 | 0.015 | 0.014 | RABGAP1L | 1.00000 | 1.00000 | 0.29694 | 0 | 0 |
| 46 | chr1 | 187263000 | 187264000 | 0.000 | 0.000 | 0.044 | 0.000 | PLA2G4A | 0.10727 | 0.54966 | 0.02537 | 0 | 0 |

| | | | | | | | | | | | | | |
|---|---|---|---|---|---|---|---|---|---|---|---|---|---|
| 47 | chr1 | 187283000 | 187284000 | 0.000 | 0.000 | 0.029 | 0.000 | PLA2G4A | 0.22755 | 0.54294 | 0.08726 | 0 | 0 |
| 48 | chr1 | 187892000 | 187893000 | 0.028 | 0.000 | 0.015 | 0.000 | PLA2G4A | 0.47887 | 1.00000 | 0.29694 | 0 | 0 |
| 49 | chr1 | 195282000 | 195283000 | 0.000 | 0.000 | 0.015 | 0.014 | KCNT2 | 1.00000 | 1.00000 | 0.29694 | 0 | 0 |
| 50 | chr1 | 198591000 | 198592000 | 0.000 | 0.000 | 0.029 | 0.000 | PTPRC | 0.22755 | 0.54294 | 0.08726 | 0 | 0 |
| 51 | chr1 | 198608000 | 198609000 | 0.000 | 0.000 | 0.029 | 0.000 | PTPRC | 0.22755 | 0.54294 | 0.08726 | 0 | 0 |
| 52 | chr1 | 198609000 | 198610000 | 0.000 | 0.000 | 0.029 | 0.000 | PTPRC | 0.22755 | 0.54294 | 0.08726 | 0 | 0 |
| 53 | chr1 | 202004000 | 202005000 | 0.028 | 0.000 | 0.029 | 0.000 | ELF3 | 0.22755 | 1.00000 | 0.08726 | 0 | 0 |
| 54 | chr1 | 203273000 | 203274000 | 0.000 | 0.000 | 0.029 | 0.000 | BTG2 | 0.22755 | 0.54294 | 0.08726 | 1 | 0 |
| 55 | chr1 | 203274000 | 203275000 | 0.000 | 0.000 | 0.176 | 0.014 | BTG2 | 0.00078 | 0.00730 | 0.00000 | 1 | 1 |
| 56 | chr1 | 203275000 | 203276000 | 0.028 | 0.006 | 0.471 | 0.081 | BTG2 | 0.00000 | 0.00000 | 0.00000 | 1 | 1 |
| 57 | chr1 | 203276000 | 203277000 | 0.028 | 0.000 | 0.059 | 0.000 | BTG2 | 0.05016 | 0.65667 | 0.00730 | 1 | 1 |
| 58 | chr1 | 205780000 | 205781000 | 0.000 | 0.000 | 0.000 | 0.027 | SLC41A1 | 0.49735 | 1.00000 | 1.00000 | 0 | 0 |
| 59 | chr1 | 205781000 | 205782000 | 0.000 | 0.000 | 0.000 | 0.027 | SLC41A1 | 0.49735 | 1.00000 | 1.00000 | 0 | 0 |
| 60 | chr1 | 206283000 | 206284000 | 0.000 | 0.000 | 0.015 | 0.014 | CTSE | 1.00000 | 1.00000 | 0.29694 | 0 | 0 |
| 61 | chr1 | 206286000 | 206287000 | 0.000 | 0.000 | 0.029 | 0.014 | CTSE | 0.60686 | 0.54294 | 0.08726 | 0 | 0 |
| 62 | chr1 | 217044000 | 217045000 | 0.000 | 0.000 | 0.029 | 0.000 | ESRRG | 0.22755 | 0.54294 | 0.08726 | 0 | 0 |
| 63 | chr1 | 226924000 | 226925000 | 0.000 | 0.000 | 0.029 | 0.000 | ITPKB | 0.22755 | 0.54294 | 0.08726 | 1 | 0 |
| 64 | chr1 | 226925000 | 226926000 | 0.000 | 0.000 | 0.044 | 0.000 | ITPKB | 0.10727 | 0.54966 | 0.02537 | 1 | 0 |
| 65 | chr1 | 226926000 | 226927000 | 0.000 | 0.000 | 0.029 | 0.000 | ITPKB | 0.22755 | 0.54294 | 0.08726 | 1 | 0 |
| 66 | chr1 | 229974000 | 229975000 | 0.028 | 0.000 | 0.015 | 0.027 | URB2 | 1.00000 | 1.00000 | 0.29694 | 0 | 0 |
| 67 | chr1 | 235131000 | 235132000 | 0.000 | 0.000 | 0.000 | 0.027 | TOMM20 | 0.49735 | 1.00000 | 1.00000 | 0 | 0 |
| 68 | chr1 | 235141000 | 235142000 | 0.000 | 0.000 | 0.015 | 0.014 | TOMM20 | 1.00000 | 1.00000 | 0.29694 | 0 | 0 |
| 69 | chr1 | 238787000 | 238788000 | 0.000 | 0.000 | 0.029 | 0.000 | MTRNR2L11 | 0.22755 | 0.54294 | 0.08726 | 0 | 0 |
| 70 | chr1 | 248088000 | 248089000 | 0.028 | 0.000 | 0.015 | 0.000 | OR2T8 | 0.47887 | 1.00000 | 0.29694 | 0 | 0 |
| 71 | chr2 | 630000 | 631000 | 0.000 | 0.000 | 0.000 | 0.027 | TMEM18 | 0.49735 | 1.00000 | 1.00000 | 0 | 0 |
| 72 | chr2 | 1484000 | 1485000 | 0.000 | 0.000 | 0.000 | 0.027 | TPO | 0.49735 | 1.00000 | 1.00000 | 0 | 0 |
| 73 | chr2 | 7991000 | 7992000 | 0.056 | 0.000 | 0.000 | 0.000 | RNF144A | 1.00000 | 0.11763 | 1.00000 | 0 | 1 |
| 74 | chr2 | 12173000 | 12174000 | 0.000 | 0.000 | 0.044 | 0.000 | LPIN1 | 0.10727 | 0.54966 | 0.02537 | 0 | 0 |
| 75 | chr2 | 12175000 | 12176000 | 0.000 | 0.000 | 0.029 | 0.000 | LPIN1 | 0.22755 | 0.54294 | 0.08726 | 0 | 0 |
| 76 | chr2 | 12249000 | 12250000 | 0.000 | 0.000 | 0.029 | 0.000 | LPIN1 | 0.22755 | 0.54294 | 0.08726 | 0 | 0 |
| 77 | chr2 | 14113000 | 14114000 | 0.000 | 0.000 | 0.000 | 0.027 | FAM84A | 0.49735 | 1.00000 | 1.00000 | 0 | 0 |
| 78 | chr2 | 17577000 | 17578000 | 0.000 | 0.000 | 0.015 | 0.014 | RAD51AP2 | 1.00000 | 1.00000 | 0.29694 | 0 | 0 |
| 79 | chr2 | 19253000 | 19254000 | 0.000 | 0.000 | 0.029 | 0.000 | OSR1 | 0.22755 | 0.54294 | 0.08726 | 0 | 0 |
| 80 | chr2 | 24802000 | 24803000 | 0.000 | 0.000 | 0.029 | 0.000 | NCOA1 | 0.22755 | 0.54294 | 0.08726 | 0 | 0 |
| 81 | chr2 | 31478000 | 31479000 | 0.000 | 0.000 | 0.015 | 0.014 | EHD3 | 1.00000 | 1.00000 | 0.29694 | 0 | 0 |
| 82 | chr2 | 41728000 | 41729000 | 0.000 | 0.000 | 0.015 | 0.014 | C2orf91 | 1.00000 | 1.00000 | 0.29694 | 0 | 0 |
| 83 | chr2 | 45404000 | 45405000 | 0.000 | 0.000 | 0.000 | 0.027 | SIX2 | 0.49735 | 1.00000 | 1.00000 | 0 | 0 |
| 84 | chr2 | 47923000 | 47924000 | 0.000 | 0.000 | 0.015 | 0.014 | MSH6 | 1.00000 | 1.00000 | 0.29694 | 0 | 0 |
| 85 | chr2 | 47944000 | 47945000 | 0.000 | 0.000 | 0.029 | 0.000 | MSH6 | 0.22755 | 0.54294 | 0.08726 | 0 | 0 |
| 86 | chr2 | 51360000 | 51361000 | 0.000 | 0.000 | 0.015 | 0.014 | NRXN1 | 1.00000 | 1.00000 | 0.29694 | 0 | 0 |
| 87 | chr2 | 51655000 | 51656000 | 0.000 | 0.000 | 0.000 | 0.027 | NRXN1 | 0.49735 | 1.00000 | 1.00000 | 0 | 0 |
| 88 | chr2 | 56565000 | 56566000 | 0.000 | 0.000 | 0.029 | 0.000 | CCDC85A | 0.22755 | 0.54294 | 0.08726 | 0 | 0 |
| 89 | chr2 | 57800000 | 57801000 | 0.000 | 0.000 | 0.015 | 0.014 | VRK2 | 1.00000 | 1.00000 | 0.29694 | 0 | 0 |
| 90 | chr2 | 60779000 | 60780000 | 0.000 | 0.000 | 0.029 | 0.027 | BCL11A | 1.00000 | 0.54294 | 0.08726 | 0 | 0 |
| 91 | chr2 | 60780000 | 60781000 | 0.000 | 0.000 | 0.029 | 0.000 | BCL11A | 0.22755 | 0.54294 | 0.08726 | 0 | 0 |
| 92 | chr2 | 63802000 | 63803000 | 0.000 | 0.000 | 0.000 | 0.027 | WDPCP | 0.49735 | 1.00000 | 1.00000 | 0 | 0 |
| 93 | chr2 | 63827000 | 63828000 | 0.000 | 0.000 | 0.015 | 0.014 | MDH1 | 1.00000 | 1.00000 | 0.29694 | 0 | 0 |
| 94 | chr2 | 64319000 | 64320000 | 0.000 | 0.000 | 0.044 | 0.000 | PELI1 | 0.10727 | 0.54966 | 0.02537 | 0 | 0 |
| 95 | chr2 | 65593000 | 65594000 | 0.000 | 0.000 | 0.044 | 0.054 | SPRED2 | 1.00000 | 0.54966 | 0.02537 | 1 | 1 |
| 96 | chr2 | 67002000 | 67003000 | 0.028 | 0.000 | 0.029 | 0.000 | MEIS1 | 0.22755 | 1.00000 | 0.08726 | 0 | 0 |
| 97 | chr2 | 70315000 | 70316000 | 0.083 | 0.000 | 0.000 | 0.000 | PCBP1 | 1.00000 | 0.03921 | 1.00000 | 0 | 1 |

| # | chr | start | end | v1 | v2 | v3 | v4 | gene | p1 | p2 | p3 | a | b |
|---|---|---|---|---|---|---|---|---|---|---|---|---|---|
| 98 | chr2 | 79502000 | 79503000 | 0.028 | 0.000 | 0.015 | 0.000 | REG3A | 0.47887 | 1.00000 | 0.29694 | 0 | 0 |
| 99 | chr2 | 79644000 | 79645000 | 0.000 | 0.000 | 0.000 | 0.027 | CTNNA2 | 0.49735 | 1.00000 | 1.00000 | 0 | 0 |
| 100 | chr2 | 81818000 | 81819000 | 0.000 | 0.000 | 0.000 | 0.027 | CTNNA2 | 0.49735 | 1.00000 | 1.00000 | 0 | 0 |
| 101 | chr2 | 82310000 | 82311000 | 0.028 | 0.000 | 0.015 | 0.000 | CTNNA2 | 0.47887 | 1.00000 | 0.29694 | 0 | 0 |
| 102 | chr2 | 82948000 | 82949000 | 0.000 | 0.000 | 0.029 | 0.000 | SUCLG1 | 0.22755 | 0.54294 | 0.08726 | 0 | 0 |
| 103 | chr2 | 85335000 | 85336000 | 0.000 | 0.000 | 0.000 | 0.027 | TCF7L1 | 0.49735 | 1.00000 | 1.00000 | 0 | 0 |
| 104 | chr2 | 88905000 | 88906000 | 0.000 | 0.000 | 0.059 | 0.000 | EIF2AK3 | 0.05016 | 0.29551 | 0.00730 | 0 | 1 |
| 105 | chr2 | 88906000 | 88907000 | 0.000 | 0.006 | 0.074 | 0.014 | EIF2AK3 | 0.10420 | 0.16101 | 0.00953 | 0 | 1 |
| 106 | chr2 | 88907000 | 88908000 | 0.000 | 0.000 | 0.059 | 0.000 | EIF2AK3 | 0.05016 | 0.29551 | 0.00730 | 0 | 1 |
| 107 | chr2 | 89052000 | 89053000 | 0.000 | 0.006 | 0.015 | 0.000 | RPIA | 0.47887 | 1.00000 | 0.50663 | 0 | 0 |
| 108 | chr2 | 89065000 | 89066000 | 0.000 | 0.000 | 0.015 | 0.027 | RPIA | 1.00000 | 1.00000 | 0.29694 | 0 | 0 |
| 109 | chr2 | 89066000 | 89067000 | 0.000 | 0.000 | 0.015 | 0.014 | RPIA | 1.00000 | 1.00000 | 0.29694 | 0 | 0 |
| 110 | chr2 | 89095000 | 89096000 | 0.000 | 0.000 | 0.015 | 0.014 | RPIA | 1.00000 | 1.00000 | 0.29694 | 0 | 0 |
| 111 | chr2 | 89127000 | 89128000 | 0.000 | 0.006 | 0.147 | 0.041 | IGKC | 0.03985 | 0.01404 | 0.00003 | 0 | 1 |
| 112 | chr2 | 89128000 | 89129000 | 0.028 | 0.006 | 0.176 | 0.041 | IGKC | 0.01224 | 0.03142 | 0.00000 | 0 | 1 |
| 113 | chr2 | 89129000 | 89130000 | 0.000 | 0.000 | 0.044 | 0.041 | IGKC | 1.00000 | 0.54966 | 0.02537 | 0 | 0 |
| 114 | chr2 | 89130000 | 89131000 | 0.000 | 0.000 | 0.044 | 0.000 | IGKC | 0.10727 | 0.54966 | 0.02537 | 0 | 0 |
| 115 | chr2 | 89131000 | 89132000 | 0.000 | 0.000 | 0.029 | 0.000 | IGKC | 0.22755 | 0.54294 | 0.08726 | 0 | 0 |
| 116 | chr2 | 89132000 | 89133000 | 0.000 | 0.006 | 0.015 | 0.014 | IGKC | 1.00000 | 1.00000 | 0.50663 | 0 | 0 |
| 117 | chr2 | 89133000 | 89134000 | 0.000 | 0.000 | 0.029 | 0.041 | IGKC | 1.00000 | 0.54294 | 0.08726 | 0 | 0 |
| 118 | chr2 | 89137000 | 89138000 | 0.000 | 0.000 | 0.044 | 0.014 | IGKC | 0.34948 | 0.54966 | 0.02537 | 0 | 0 |
| 119 | chr2 | 89138000 | 89139000 | 0.000 | 0.000 | 0.015 | 0.014 | IGKC | 1.00000 | 1.00000 | 0.29694 | 0 | 0 |
| 120 | chr2 | 89139000 | 89140000 | 0.000 | 0.000 | 0.044 | 0.014 | IGKC | 0.34948 | 0.54966 | 0.02537 | 0 | 0 |
| 121 | chr2 | 89140000 | 89141000 | 0.000 | 0.000 | 0.088 | 0.054 | IGKC | 0.52007 | 0.09031 | 0.00058 | 0 | 1 |
| 122 | chr2 | 89141000 | 89142000 | 0.000 | 0.006 | 0.103 | 0.027 | IGKC | 0.08710 | 0.09269 | 0.00099 | 0 | 1 |
| 123 | chr2 | 89142000 | 89143000 | 0.000 | 0.000 | 0.088 | 0.000 | IGKC | 0.01070 | 0.09031 | 0.00058 | 0 | 1 |
| 124 | chr2 | 89143000 | 89144000 | 0.000 | 0.000 | 0.029 | 0.000 | IGKC | 0.22755 | 0.54294 | 0.08726 | 0 | 0 |
| 125 | chr2 | 89144000 | 89145000 | 0.000 | 0.000 | 0.015 | 0.014 | IGKC | 1.00000 | 1.00000 | 0.29694 | 0 | 0 |
| 126 | chr2 | 89145000 | 89146000 | 0.000 | 0.000 | 0.029 | 0.014 | IGKC | 0.60686 | 0.54294 | 0.08726 | 0 | 0 |
| 127 | chr2 | 89146000 | 89147000 | 0.000 | 0.000 | 0.029 | 0.014 | IGKC | 0.60686 | 0.54294 | 0.08726 | 0 | 0 |
| 128 | chr2 | 89153000 | 89154000 | 0.000 | 0.000 | 0.029 | 0.000 | IGKC | 0.22755 | 0.54294 | 0.08726 | 0 | 0 |
| 129 | chr2 | 89155000 | 89156000 | 0.000 | 0.000 | 0.059 | 0.014 | IGKC | 0.19371 | 0.29551 | 0.00730 | 0 | 1 |
| 130 | chr2 | 89156000 | 89157000 | 0.000 | 0.000 | 0.103 | 0.014 | IGKC | 0.02808 | 0.09269 | 0.00016 | 0 | 1 |
| 131 | chr2 | 89157000 | 89158000 | 0.000 | 0.000 | 0.250 | 0.149 | IGKC | 0.14439 | 0.00048 | 0.00000 | 0 | 1 |
| 132 | chr2 | 89158000 | 89159000 | 0.028 | 0.019 | 0.426 | 0.270 | IGKC | 0.05462 | 0.00001 | 0.00000 | 0 | 1 |
| 133 | chr2 | 89159000 | 89160000 | 0.222 | 0.180 | 0.574 | 0.473 | IGKJ5 | 0.24418 | 0.00083 | 0.00000 | 0 | 1 |
| 134 | chr2 | 89160000 | 89161000 | 0.444 | 0.242 | 0.500 | 0.608 | IGKJ3;IGKJ4;IGKJ5; | 0.23729 | 0.68125 | 0.00019 | 0 | 1 |
| 135 | chr2 | 89161000 | 89162000 | 0.222 | 0.081 | 0.265 | 0.405 | IGKJ1;IGKJ2; | 0.10957 | 0.81234 | 0.00049 | 0 | 1 |
| 136 | chr2 | 89162000 | 89163000 | 0.056 | 0.012 | 0.221 | 0.108 | IGKJ1 | 0.10913 | 0.04835 | 0.00000 | 0 | 1 |
| 137 | chr2 | 89163000 | 89164000 | 0.000 | 0.068 | 0.235 | 0.176 | IGKJ1 | 0.41068 | 0.00098 | 0.00117 | 0 | 1 |
| 138 | chr2 | 89164000 | 89165000 | 0.028 | 0.137 | 0.294 | 0.216 | IGKJ1 | 0.33637 | 0.00075 | 0.00821 | 0 | 1 |
| 139 | chr2 | 89165000 | 89166000 | 0.083 | 0.143 | 0.279 | 0.216 | IGKJ1 | 0.43812 | 0.02316 | 0.02379 | 0 | 1 |
| 140 | chr2 | 89166000 | 89167000 | 0.028 | 0.012 | 0.044 | 0.027 | IGKJ1 | 0.67043 | 1.00000 | 0.15671 | 0 | 0 |
| 141 | chr2 | 89169000 | 89170000 | 0.000 | 0.000 | 0.015 | 0.014 | IGKJ1 | 1.00000 | 1.00000 | 0.29694 | 0 | 0 |
| 142 | chr2 | 89184000 | 89185000 | 0.000 | 0.006 | 0.015 | 0.054 | IGKV4-1 | 0.36833 | 1.00000 | 0.50663 | 0 | 1 |
| 143 | chr2 | 89185000 | 89186000 | 0.028 | 0.056 | 0.162 | 0.135 | IGKV4-1 | 0.81354 | 0.05349 | 0.01836 | 0 | 1 |
| 144 | chr2 | 89196000 | 89197000 | 0.000 | 0.000 | 0.059 | 0.014 | IGKV5-2 | 0.19371 | 0.29551 | 0.00730 | 0 | 1 |
| 145 | chr2 | 89197000 | 89198000 | 0.000 | 0.000 | 0.000 | 0.027 | IGKV5-2 | 0.49735 | 1.00000 | 1.00000 | 0 | 0 |
| 146 | chr2 | 89214000 | 89215000 | 0.000 | 0.012 | 0.000 | 0.000 | IGKV5-2 | 1.00000 | 1.00000 | 1.00000 | 0 | 0 |
| 147 | chr2 | 89246000 | 89247000 | 0.000 | 0.031 | 0.029 | 0.027 | IGKV1-5 | 1.00000 | 0.54294 | 1.00000 | 0 | 0 |
| 148 | chr2 | 89247000 | 89248000 | 0.028 | 0.019 | 0.118 | 0.054 | IGKV1-5 | 0.23086 | 0.15803 | 0.00321 | 0 | 1 |

| | | | | | | | | | | | | | |
|---|---|---|---|---|---|---|---|---|---|---|---|---|---|
| 149 | chr2 | 89248000 | 89249000 | 0.028 | 0.000 | 0.044 | 0.000 | IGKV1-5 | 0.10727 | 1.00000 | 0.02537 | 0 | 0 |
| 150 | chr2 | 89266000 | 89267000 | 0.000 | 0.000 | 0.015 | 0.014 | IGKV1-6 | 1.00000 | 1.00000 | 0.29694 | 0 | 0 |
| 151 | chr2 | 89291000 | 89292000 | 0.000 | 0.000 | 0.019 | 0.029 | IGKV1-8 | 0.22755 | 0.54294 | 0.63492 | 0 | 0 |
| 152 | chr2 | 89292000 | 89293000 | 0.000 | 0.025 | 0.044 | 0.000 | IGKV1-8 | 0.10727 | 0.54966 | 0.42650 | 0 | 0 |
| 153 | chr2 | 89326000 | 89327000 | 0.000 | 0.019 | 0.000 | 0.041 | IGKV3-11 | 0.24603 | 1.00000 | 0.55662 | 0 | 0 |
| 154 | chr2 | 89327000 | 89328000 | 0.000 | 0.012 | 0.015 | 0.027 | IGKV3-11 | 1.00000 | 1.00000 | 1.00000 | 0 | 0 |
| 155 | chr2 | 89442000 | 89443000 | 0.111 | 0.050 | 0.074 | 0.122 | IGKV3-20 | 0.40586 | 0.71556 | 0.53493 | 0 | 1 |
| 156 | chr2 | 89443000 | 89444000 | 0.000 | 0.000 | 0.015 | 0.041 | IGKV3-20 | 0.62100 | 1.00000 | 0.29694 | 0 | 0 |
| 157 | chr2 | 89476000 | 89477000 | 0.028 | 0.000 | 0.000 | 0.014 | IGKV2-24 | 1.00000 | 0.34615 | 1.00000 | 0 | 0 |
| 158 | chr2 | 89513000 | 89514000 | 0.000 | 0.000 | 0.029 | 0.000 | IGKV1-27 | 0.22755 | 0.54294 | 0.08726 | 0 | 0 |
| 159 | chr2 | 89521000 | 89522000 | 0.028 | 0.000 | 0.015 | 0.014 | IGKV2-28 | 1.00000 | 1.00000 | 0.29694 | 0 | 0 |
| 160 | chr2 | 89533000 | 89534000 | 0.028 | 0.000 | 0.044 | 0.014 | IGKV2-30 | 0.34948 | 1.00000 | 0.02537 | 0 | 0 |
| 161 | chr2 | 89534000 | 89535000 | 0.000 | 0.000 | 0.029 | 0.014 | IGKV2-30 | 0.60686 | 0.54294 | 0.08726 | 0 | 0 |
| 162 | chr2 | 89544000 | 89545000 | 0.028 | 0.012 | 0.059 | 0.014 | IGKV2-30 | 0.19371 | 0.65667 | 0.06548 | 0 | 1 |
| 163 | chr2 | 89545000 | 89546000 | 0.000 | 0.006 | 0.029 | 0.000 | IGKV2-30 | 0.22755 | 0.54294 | 0.21104 | 0 | 0 |
| 164 | chr2 | 90259000 | 90260000 | 0.000 | 0.000 | 0.015 | 0.014 | IGKV1D-8 | 1.00000 | 1.00000 | 0.29694 | 0 | 0 |
| 165 | chr2 | 90260000 | 90261000 | 0.000 | 0.000 | 0.059 | 0.014 | IGKV1D-8 | 0.19371 | 0.29551 | 0.00730 | 0 | 1 |
| 166 | chr2 | 96809000 | 96810000 | 0.000 | 0.000 | 0.044 | 0.000 | DUSP2 | 0.10727 | 0.54966 | 0.02537 | 1 | 0 |
| 167 | chr2 | 96810000 | 96811000 | 0.000 | 0.000 | 0.044 | 0.014 | DUSP2 | 0.34948 | 0.54966 | 0.02537 | 1 | 0 |
| 168 | chr2 | 96811000 | 96812000 | 0.000 | 0.000 | 0.029 | 0.000 | DUSP2 | 0.22755 | 0.54294 | 0.08726 | 1 | 0 |
| 169 | chr2 | 98611000 | 98612000 | 0.000 | 0.000 | 0.015 | 0.014 | TMEM131 | 1.00000 | 1.00000 | 0.29694 | 0 | 0 |
| 170 | chr2 | 100757000 | 100758000 | 0.000 | 0.000 | 0.029 | 0.027 | AFF3 | 1.00000 | 0.54294 | 0.08726 | 0 | 0 |
| 171 | chr2 | 100758000 | 100759000 | 0.000 | 0.000 | 0.044 | 0.014 | AFF3 | 0.34948 | 0.54966 | 0.02537 | 0 | 0 |
| 172 | chr2 | 106144000 | 106145000 | 0.000 | 0.000 | 0.029 | 0.000 | FHL2 | 0.22755 | 0.54294 | 0.08726 | 0 | 0 |
| 173 | chr2 | 111878000 | 111879000 | 0.000 | 0.000 | 0.029 | 0.014 | BCL2L11 | 0.60686 | 0.54294 | 0.08726 | 0 | 0 |
| 174 | chr2 | 111879000 | 111880000 | 0.000 | 0.000 | 0.044 | 0.014 | BCL2L11 | 0.34948 | 0.54966 | 0.02537 | 0 | 0 |
| 175 | chr2 | 112305000 | 112306000 | 0.000 | 0.000 | 0.015 | 0.014 | ANAPC1 | 1.00000 | 1.00000 | 0.29694 | 0 | 0 |
| 176 | chr2 | 116234000 | 116235000 | 0.000 | 0.000 | 0.015 | 0.014 | DPP10 | 1.00000 | 1.00000 | 0.29694 | 0 | 0 |
| 177 | chr2 | 116439000 | 116440000 | 0.028 | 0.000 | 0.000 | 0.014 | DPP10 | 1.00000 | 0.34615 | 1.00000 | 0 | 0 |
| 178 | chr2 | 124697000 | 124698000 | 0.028 | 0.000 | 0.015 | 0.000 | CNTNAP5 | 0.47887 | 1.00000 | 0.29694 | 0 | 0 |
| 179 | chr2 | 125235000 | 125236000 | 0.000 | 0.000 | 0.029 | 0.000 | CNTNAP5 | 0.22755 | 0.54294 | 0.08726 | 0 | 0 |
| 180 | chr2 | 127538000 | 127539000 | 0.028 | 0.000 | 0.015 | 0.000 | GYPC | 0.47887 | 1.00000 | 0.29694 | 0 | 0 |
| 181 | chr2 | 136874000 | 136875000 | 0.000 | 0.000 | 0.191 | 0.014 | CXCR4 | 0.00036 | 0.00372 | 0.00000 | 1 | 1 |
| 182 | chr2 | 136875000 | 136876000 | 0.083 | 0.019 | 0.265 | 0.081 | CXCR4 | 0.00626 | 0.03882 | 0.00000 | 1 | 1 |
| 183 | chr2 | 136996000 | 136997000 | 0.000 | 0.000 | 0.029 | 0.000 | CXCR4 | 0.22755 | 0.54294 | 0.08726 | 1 | 0 |
| 184 | chr2 | 137082000 | 137083000 | 0.000 | 0.000 | 0.015 | 0.014 | CXCR4 | 1.00000 | 1.00000 | 0.29694 | 1 | 0 |
| 185 | chr2 | 140951000 | 140952000 | 0.000 | 0.000 | 0.029 | 0.000 | LRP1B | 0.22755 | 0.54294 | 0.08726 | 0 | 0 |
| 186 | chr2 | 141335000 | 141336000 | 0.000 | 0.000 | 0.015 | 0.014 | LRP1B | 1.00000 | 1.00000 | 0.29694 | 0 | 0 |
| 187 | chr2 | 141770000 | 141771000 | 0.000 | 0.000 | 0.029 | 0.000 | LRP1B | 0.22755 | 0.54294 | 0.08726 | 0 | 0 |
| 188 | chr2 | 146445000 | 146446000 | 0.000 | 0.000 | 0.029 | 0.000 | ZEB2 | 0.22755 | 0.54294 | 0.08726 | 0 | 0 |
| 189 | chr2 | 146446000 | 146447000 | 0.000 | 0.000 | 0.029 | 0.014 | ZEB2 | 0.60686 | 0.54294 | 0.08726 | 0 | 0 |
| 190 | chr2 | 156443000 | 156444000 | 0.000 | 0.000 | 0.029 | 0.000 | KCNJ3 | 0.22755 | 0.54294 | 0.08726 | 0 | 0 |
| 191 | chr2 | 172590000 | 172591000 | 0.000 | 0.000 | 0.029 | 0.000 | DYNC1I2 | 0.22755 | 0.54294 | 0.08726 | 0 | 0 |
| 192 | chr2 | 176581000 | 176582000 | 0.028 | 0.000 | 0.000 | 0.014 | KIAA1715 | 1.00000 | 0.34615 | 1.00000 | 0 | 0 |
| 193 | chr2 | 179880000 | 179881000 | 0.000 | 0.000 | 0.015 | 0.014 | CCDC141 | 1.00000 | 1.00000 | 0.29694 | 0 | 0 |
| 194 | chr2 | 180358000 | 180359000 | 0.000 | 0.000 | 0.029 | 0.000 | ZNF385B | 0.22755 | 0.54294 | 0.08726 | 0 | 0 |
| 195 | chr2 | 189285000 | 189286000 | 0.000 | 0.000 | 0.015 | 0.014 | GULP1 | 1.00000 | 1.00000 | 0.29694 | 0 | 0 |
| 196 | chr2 | 189432000 | 189433000 | 0.028 | 0.000 | 0.000 | 0.014 | GULP1 | 1.00000 | 0.34615 | 1.00000 | 0 | 0 |
| 197 | chr2 | 194115000 | 194116000 | 0.000 | 0.000 | 0.015 | 0.014 | TMEFF2 | 1.00000 | 1.00000 | 0.29694 | 0 | 0 |
| 198 | chr2 | 197035000 | 197036000 | 0.000 | 0.000 | 0.044 | 0.014 | STK17B | 0.34948 | 0.54966 | 0.02537 | 0 | 0 |
| 199 | chr2 | 197041000 | 197042000 | 0.000 | 0.000 | 0.029 | 0.000 | STK17B | 0.22755 | 0.54294 | 0.08726 | 0 | 0 |

| | | | | | | | | | | | | | | |
|---|---|---|---|---|---|---|---|---|---|---|---|---|---|---|
| 200 | chr2 | 215999000 | 216600000 | 0.000 | 0.015 | 0.006 | 0.000 | 0.000 | ABCA12 | 0.47887 | 1.00000 | 0.50663 | 0 | 0 |
| 201 | chr2 | 216973000 | 216974000 | 0.014 | 0.000 | 0.000 | 0.000 | 0.028 | XRCC5 | 1.00000 | 0.34615 | 1.00000 | 0 | 0 |
| 202 | chr2 | 217247000 | 217248000 | 0.014 | 0.000 | 0.000 | 0.028 | 0.028 | 4-Mar-19 | 1.00000 | 0.34615 | 1.00000 | 0 | 0 |
| 203 | chr2 | 225386000 | 225387000 | 0.000 | 0.029 | 0.000 | 0.000 | 0.000 | CUL3 | 0.22755 | 0.54294 | 0.08726 | 0 | 0 |
| 204 | chr2 | 225524000 | 225525000 | 0.000 | 0.015 | 0.000 | 0.028 | 0.000 | CUL3 | 0.22755 | 0.54294 | 0.08726 | 0 | 0 |
| 205 | chr2 | 233478000 | 233479000 | 0.000 | 0.029 | 0.000 | 0.028 | 0.028 | EFHD1 | 0.47887 | 1.00000 | 0.29694 | 0 | 0 |
| 206 | chr2 | 233980000 | 233981000 | 0.027 | 0.000 | 0.000 | 0.028 | 0.028 | INPP5D | 0.22755 | 1.00000 | 0.08726 | 0 | 0 |
| 207 | chr2 | 240641000 | 240642000 | 0.027 | 0.000 | 0.000 | 0.000 | 0.000 | AC093802.1 | 0.49735 | 0.34615 | 1.00000 | 0 | 0 |
| 208 | chr2 | 241125000 | 241126000 | 0.027 | 0.000 | 0.000 | 0.000 | 0.000 | OTOS | 0.49735 | 1.00000 | 1.00000 | 0 | 0 |
| 209 | chr3 | 8759000 | 8760000 | 0.027 | 0.000 | 0.000 | 0.000 | 0.000 | CAV3 | 0.49735 | 1.00000 | 1.00000 | 1 | 0 |
| 210 | chr3 | 16407000 | 16408000 | 0.041 | 0.000 | 0.000 | 0.028 | 0.000 | RFTN1 | 0.49735 | 1.00000 | 1.00000 | 1 | 0 |
| 211 | chr3 | 16409000 | 16410000 | 0.000 | 0.044 | 0.000 | 0.000 | 0.000 | RFTN1 | 0.24603 | 0.34615 | 1.00000 | 1 | 0 |
| 212 | chr3 | 16419000 | 16420000 | 0.000 | 0.000 | 0.006 | 0.000 | 0.000 | RFTN1 | 0.10727 | 0.54966 | 0.07959 | 1 | 0 |
| 213 | chr3 | 16472000 | 16473000 | 0.014 | 0.015 | 0.000 | 0.000 | 0.000 | RFTN1 | 1.00000 | 1.00000 | 0.29694 | 1 | 0 |
| 214 | chr3 | 16495000 | 16496000 | 0.000 | 0.029 | 0.000 | 0.000 | 0.000 | RFTN1 | 0.22755 | 0.54294 | 0.08726 | 1 | 0 |
| 215 | chr3 | 16552000 | 16553000 | 0.014 | 0.029 | 0.012 | 0.000 | 0.000 | RFTN1 | 0.60686 | 0.54294 | 0.58408 | 1 | 0 |
| 216 | chr3 | 16554000 | 16555000 | 0.027 | 0.163 | 0.000 | 0.000 | 0.000 | RFTN1 | 0.68710 | 0.69269 | 0.00016 | 1 | 1 |
| 217 | chr3 | 16555000 | 16556000 | 0.000 | 0.029 | 0.000 | 0.000 | 0.000 | RFTN1 | 0.22755 | 0.54294 | 0.08726 | 1 | 0 |
| 218 | chr3 | 21658000 | 21659000 | 0.000 | 0.029 | 0.000 | 0.000 | 0.000 | ZNF385D | 0.22755 | 0.54294 | 0.08726 | 0 | 0 |
| 219 | chr3 | 25691000 | 25692000 | 0.000 | 0.029 | 0.000 | 0.000 | 0.000 | TOP2B | 0.22755 | 0.54294 | 0.08726 | 0 | 0 |
| 220 | chr3 | 31969000 | 31970000 | 0.000 | 0.044 | 0.000 | 0.000 | 0.000 | OSBPL10 | 0.22755 | 0.54294 | 0.08726 | 0 | 0 |
| 221 | chr3 | 31993000 | 31994000 | 0.000 | 0.044 | 0.000 | 0.000 | 0.000 | OSBPL10 | 0.10727 | 0.54966 | 0.02537 | 0 | 0 |
| 222 | chr3 | 32001000 | 32002000 | 0.000 | 0.044 | 0.000 | 0.000 | 0.000 | OSBPL10 | 0.10727 | 0.54966 | 0.02537 | 1 | 0 |
| 223 | chr3 | 32022000 | 32023000 | 0.014 | 0.088 | 0.000 | 0.000 | 0.000 | OSBPL10 | 0.05468 | 0.09031 | 0.60058 | 1 | 1 |
| 224 | chr3 | 32023000 | 32024000 | 0.000 | 0.029 | 0.000 | 0.000 | 0.000 | OSBPL10 | 0.22755 | 0.54294 | 0.08726 | 1 | 0 |
| 225 | chr3 | 50128000 | 50129000 | 0.000 | 0.022 | 0.000 | 0.000 | 0.000 | RBM5 | 0.22755 | 0.54294 | 0.08725 | 0 | 0 |
| 226 | chr3 | 54913000 | 54914000 | 0.000 | 0.015 | 0.006 | 0.000 | 0.028 | CACNA2D3 | 0.47887 | 1.00000 | 0.50663 | 0 | 0 |
| 227 | chr3 | 56074000 | 56075000 | 0.014 | 0.000 | 0.000 | 0.000 | 0.000 | ERC2 | 1.00000 | 0.34615 | 1.00000 | 0 | 0 |
| 228 | chr3 | 59577000 | 59578000 | 0.000 | 0.029 | 0.000 | 0.000 | 0.000 | FHIT | 0.22755 | 0.54294 | 0.08726 | 0 | 0 |
| 229 | chr3 | 60351000 | 60352000 | 0.000 | 0.044 | 0.000 | 0.000 | 0.000 | FHIT | 0.10727 | 0.54966 | 0.02537 | 0 | 0 |
| 230 | chr3 | 60356000 | 60357000 | 0.014 | 0.000 | 0.000 | 0.028 | 0.000 | FHIT | 1.00000 | 0.34615 | 1.00000 | 0 | 0 |
| 231 | chr3 | 60357000 | 60358000 | 0.014 | 0.015 | 0.000 | 0.000 | 0.000 | FHIT | 1.00000 | 1.00000 | 0.29694 | 0 | 0 |
| 232 | chr3 | 60358000 | 60359000 | 0.014 | 0.015 | 0.000 | 0.000 | 0.000 | FHIT | 1.00000 | 1.00000 | 0.29694 | 0 | 0 |
| 233 | chr3 | 60359000 | 60360000 | 0.000 | 0.029 | 0.000 | 0.000 | 0.000 | FHIT | 0.22755 | 0.54294 | 0.08726 | 0 | 0 |
| 234 | chr3 | 60389000 | 60390000 | 0.027 | 0.015 | 0.000 | 0.000 | 0.000 | FHIT | 1.00000 | 1.00000 | 0.29694 | 0 | 0 |
| 235 | chr3 | 60393000 | 60394000 | 0.000 | 0.029 | 0.000 | 0.000 | 0.000 | FHIT | 0.22755 | 0.54294 | 0.08726 | 0 | 0 |
| 236 | chr3 | 60395000 | 60396000 | 0.027 | 0.000 | 0.000 | 0.000 | 0.000 | FHIT | 0.49735 | 1.00000 | 1.00000 | 0 | 0 |
| 237 | chr3 | 60404000 | 60405000 | 0.000 | 0.029 | 0.000 | 0.000 | 0.000 | FHIT | 0.22755 | 0.54294 | 0.08726 | 0 | 0 |
| 238 | chr3 | 60436000 | 60437000 | 0.027 | 0.000 | 0.000 | 0.000 | 0.000 | FHIT | 0.49735 | 1.00000 | 1.00000 | 0 | 0 |
| 239 | chr3 | 60437000 | 60438000 | 0.000 | 0.029 | 0.000 | 0.000 | 0.000 | FHIT | 0.22755 | 0.54294 | 0.08726 | 0 | 0 |
| 240 | chr3 | 60477000 | 60478000 | 0.000 | 0.029 | 0.000 | 0.000 | 0.000 | FHIT | 0.22755 | 0.54294 | 0.08726 | 0 | 0 |
| 241 | chr3 | 60485000 | 60486000 | 0.014 | 0.015 | 0.000 | 0.000 | 0.000 | FHIT | 1.00000 | 1.00000 | 0.29694 | 0 | 0 |
| 242 | chr3 | 60515000 | 60516000 | 0.000 | 0.015 | 0.006 | 0.000 | 0.000 | FHIT | 1.00000 | 1.00000 | 0.50663 | 0 | 0 |
| 243 | chr3 | 60535000 | 60536000 | 0.014 | 0.015 | 0.000 | 0.000 | 0.000 | FHIT | 0.47887 | 1.00000 | 0.08726 | 0 | 0 |
| 244 | chr3 | 60602000 | 60603000 | 0.014 | 0.029 | 0.000 | 0.000 | 0.000 | FHIT | 0.60686 | 0.54294 | 0.08726 | 0 | 0 |
| 245 | chr3 | 60613000 | 60614000 | 0.014 | 0.029 | 0.000 | 0.000 | 0.000 | FHIT | 0.60686 | 0.54294 | 0.08726 | 0 | 0 |
| 246 | chr3 | 60614000 | 60615000 | 0.000 | 0.029 | 0.000 | 0.000 | 0.000 | FHIT | 0.22755 | 0.54294 | 1.00000 | 0 | 0 |
| 247 | chr3 | 60632000 | 60633000 | 0.027 | 0.000 | 0.000 | 0.000 | 0.000 | FHIT | 0.49735 | 1.00000 | 0.08726 | 0 | 0 |
| 248 | chr3 | 60635000 | 60636000 | 0.000 | 0.029 | 0.000 | 0.000 | 0.000 | FHIT | 0.22755 | 0.54294 | 0.08726 | 0 | 0 |
| 249 | chr3 | 60640000 | 60641000 | 0.027 | 0.000 | 0.000 | 0.000 | 0.000 | FHIT | 0.49735 | 1.00000 | 1.00000 | 0 | 0 |
| 250 | chr3 | 60647000 | 60648000 | 0.014 | 0.015 | 0.000 | 0.000 | 0.000 | FHIT | 1.00000 | 1.00000 | 0.29694 | 0 | 0 |

| | | | | | | | | | | | | | |
|---|---|---|---|---|---|---|---|---|---|---|---|---|---|
| 251 | chr3 | 60648000 | 60649000 | 0.000 | 0.000 | 0.015 | 0.014 | FHIT | 1.00000 | 1.00000 | 0.29694 | 0 | 0 |
| 252 | chr3 | 60652000 | 60653000 | 0.000 | 0.000 | 0.000 | 0.027 | FHIT | 0.49735 | 1.00000 | 1.00000 | 0 | 0 |
| 253 | chr3 | 60660000 | 60661000 | 0.000 | 0.000 | 0.029 | 0.014 | FHIT | 0.60686 | 0.54294 | 0.08726 | 0 | 0 |
| 254 | chr3 | 60665000 | 60666000 | 0.000 | 0.000 | 0.015 | 0.027 | FHIT | 1.00000 | 1.00000 | 0.29694 | 0 | 0 |
| 255 | chr3 | 60666000 | 60667000 | 0.000 | 0.000 | 0.015 | 0.014 | FHIT | 1.00000 | 1.00000 | 0.29694 | 0 | 0 |
| 256 | chr3 | 60671000 | 60672000 | 0.000 | 0.000 | 0.000 | 0.041 | FHIT | 0.24603 | 1.00000 | 1.00000 | 0 | 0 |
| 257 | chr3 | 60673000 | 60674000 | 0.000 | 0.000 | 0.044 | 0.000 | FHIT | 0.10727 | 0.54966 | 0.02537 | 0 | 0 |
| 258 | chr3 | 60675000 | 60676000 | 0.000 | 0.000 | 0.015 | 0.014 | FHIT | 1.00000 | 1.00000 | 0.29694 | 0 | 0 |
| 259 | chr3 | 60678000 | 60679000 | 0.000 | 0.000 | 0.044 | 0.000 | FHIT | 0.10727 | 0.54966 | 0.02537 | 0 | 0 |
| 260 | chr3 | 60683000 | 60684000 | 0.000 | 0.000 | 0.015 | 0.027 | FHIT | 1.00000 | 1.00000 | 0.29694 | 0 | 0 |
| 261 | chr3 | 60684000 | 60685000 | 0.000 | 0.000 | 0.015 | 0.041 | FHIT | 0.62100 | 1.00000 | 0.29694 | 0 | 0 |
| 262 | chr3 | 60688000 | 60689000 | 0.000 | 0.000 | 0.015 | 0.014 | FHIT | 1.00000 | 1.00000 | 0.29694 | 0 | 0 |
| 263 | chr3 | 60717000 | 60718000 | 0.000 | 0.000 | 0.000 | 0.027 | FHIT | 0.49735 | 1.00000 | 1.00000 | 0 | 0 |
| 264 | chr3 | 60740000 | 60741000 | 0.000 | 0.000 | 0.029 | 0.000 | FHIT | 0.22755 | 0.54294 | 0.08726 | 0 | 0 |
| 265 | chr3 | 60774000 | 60775000 | 0.000 | 0.000 | 0.029 | 0.000 | FHIT | 0.22755 | 0.54294 | 0.08726 | 0 | 0 |
| 266 | chr3 | 60792000 | 60793000 | 0.000 | 0.000 | 0.000 | 0.027 | FHIT | 0.49735 | 1.00000 | 1.00000 | 0 | 0 |
| 267 | chr3 | 60806000 | 60807000 | 0.028 | 0.000 | 0.000 | 0.014 | FHIT | 1.00000 | 0.34615 | 1.00000 | 0 | 0 |
| 268 | chr3 | 60812000 | 60813000 | 0.000 | 0.000 | 0.000 | 0.027 | FHIT | 0.49735 | 1.00000 | 1.00000 | 0 | 0 |
| 269 | chr3 | 60860000 | 60861000 | 0.000 | 0.000 | 0.000 | 0.027 | FHIT | 0.49735 | 1.00000 | 1.00000 | 0 | 0 |
| 270 | chr3 | 71551000 | 71552000 | 0.000 | 0.000 | 0.000 | 0.027 | EIF4E3 | 0.49735 | 1.00000 | 1.00000 | 0 | 0 |
| 271 | chr3 | 78274000 | 78275000 | 0.000 | 0.000 | 0.015 | 0.014 | ROBO1 | 1.00000 | 1.00000 | 0.29694 | 0 | 0 |
| 272 | chr3 | 80273000 | 80274000 | 0.000 | 0.006 | 0.015 | 0.000 | ROBO1 | 0.47887 | 1.00000 | 0.50663 | 0 | 0 |
| 273 | chr3 | 83094000 | 83095000 | 0.028 | 0.000 | 0.015 | 0.000 | GBE1 | 0.47887 | 1.00000 | 0.29694 | 0 | 0 |
| 274 | chr3 | 83924000 | 83925000 | 0.028 | 0.000 | 0.000 | 0.014 | CADM2 | 1.00000 | 0.34615 | 1.00000 | 0 | 0 |
| 275 | chr3 | 84293000 | 84294000 | 0.000 | 0.000 | 0.015 | 0.014 | CADM2 | 1.00000 | 1.00000 | 0.29694 | 0 | 0 |
| 276 | chr3 | 85260000 | 85261000 | 0.000 | 0.000 | 0.044 | 0.000 | CADM2 | 0.10727 | 0.54966 | 0.02537 | 0 | 0 |
| 277 | chr3 | 85261000 | 85262000 | 0.000 | 0.000 | 0.029 | 0.000 | CADM2 | 0.22755 | 0.54294 | 0.08726 | 0 | 0 |
| 278 | chr3 | 85799000 | 85800000 | 0.000 | 0.000 | 0.029 | 0.000 | CADM2 | 0.22755 | 0.54294 | 0.08726 | 0 | 0 |
| 279 | chr3 | 86226000 | 86227000 | 0.000 | 0.000 | 0.029 | 0.000 | CADM2 | 0.22755 | 0.54294 | 0.08726 | 0 | 0 |
| 280 | chr3 | 88146000 | 88147000 | 0.000 | 0.000 | 0.029 | 0.000 | CGGBP1 | 0.22755 | 0.54294 | 0.08726 | 0 | 0 |
| 281 | chr3 | 94709000 | 94710000 | 0.000 | 0.000 | 0.029 | 0.000 | NSUN3 | 0.22755 | 0.54294 | 0.08726 | 0 | 0 |
| 282 | chr3 | 95460000 | 95461000 | 0.028 | 0.000 | 0.015 | 0.000 | MTRNR2L12 | 0.47887 | 1.00000 | 0.29694 | 0 | 0 |
| 283 | chr3 | 95724000 | 95725000 | 0.000 | 0.000 | 0.029 | 0.000 | MTRNR2L12 | 0.22755 | 0.54294 | 0.08726 | 0 | 0 |
| 284 | chr3 | 101569000 | 101570000 | 0.028 | 0.000 | 0.015 | 0.000 | NFKBIZ | 0.47887 | 1.00000 | 0.29694 | 0 | 0 |
| 285 | chr3 | 111851000 | 111852000 | 0.000 | 0.000 | 0.044 | 0.000 | GCSAM | 0.10727 | 0.54966 | 0.02537 | 0 | 0 |
| 286 | chr3 | 111852000 | 111853000 | 0.000 | 0.000 | 0.059 | 0.000 | GCSAM | 0.05016 | 0.29551 | 0.00730 | 0 | 1 |
| 287 | chr3 | 122377000 | 122378000 | 0.028 | 0.000 | 0.044 | 0.000 | PARP14 | 0.10727 | 1.00000 | 0.02537 | 0 | 0 |
| 288 | chr3 | 150478000 | 150479000 | 0.000 | 0.000 | 0.029 | 0.000 | SIAH2 | 0.22755 | 0.54294 | 0.08726 | 0 | 0 |
| 289 | chr3 | 150479000 | 150480000 | 0.000 | 0.000 | 0.029 | 0.000 | SIAH2 | 0.22755 | 0.54294 | 0.08726 | 0 | 0 |
| 290 | chr3 | 150480000 | 150481000 | 0.000 | 0.000 | 0.015 | 0.014 | SIAH2 | 1.00000 | 1.00000 | 0.29694 | 0 | 0 |
| 291 | chr3 | 163237000 | 163238000 | 0.000 | 0.000 | 0.000 | 0.027 | SI | 0.49735 | 1.00000 | 1.00000 | 0 | 0 |
| 292 | chr3 | 163238000 | 163239000 | 0.000 | 0.000 | 0.029 | 0.000 | SI | 0.22755 | 0.54294 | 0.08726 | 0 | 0 |
| 293 | chr3 | 163615000 | 163616000 | 0.000 | 0.000 | 0.029 | 0.000 | SI | 0.22755 | 0.54294 | 0.08726 | 0 | 0 |
| 294 | chr3 | 183270000 | 183271000 | 0.000 | 0.000 | 0.029 | 0.000 | KLHL6 | 0.22755 | 0.54294 | 0.08726 | 0 | 0 |
| 295 | chr3 | 183271000 | 183272000 | 0.000 | 0.000 | 0.029 | 0.014 | KLHL6 | 0.60686 | 0.54294 | 0.08726 | 0 | 0 |
| 296 | chr3 | 183272000 | 183273000 | 0.000 | 0.000 | 0.029 | 0.014 | KLHL6 | 0.60686 | 0.54294 | 0.08726 | 0 | 0 |
| 297 | chr3 | 183273000 | 183274000 | 0.000 | 0.019 | 0.044 | 0.027 | KLHL6 | 0.67043 | 0.54966 | 0.36534 | 0 | 0 |
| 298 | chr3 | 186648000 | 186649000 | 0.000 | 0.000 | 0.044 | 0.014 | ADIPOQ | 0.34948 | 0.54966 | 0.02537 | 0 | 0 |
| 299 | chr3 | 186714000 | 186715000 | 0.000 | 0.000 | 0.132 | 0.027 | ST6GAL1 | 0.02624 | 0.02564 | 0.00009 | 1 | 1 |
| 300 | chr3 | 186715000 | 186716000 | 0.000 | 0.000 | 0.044 | 0.014 | ST6GAL1 | 0.34948 | 0.54966 | 0.02537 | 1 | 0 |
| 301 | chr3 | 186739000 | 186740000 | 0.000 | 0.006 | 0.074 | 0.014 | ST6GAL1 | 0.10420 | 0.16101 | 0.00953 | 1 | 1 |

| 302 | chr3 | 186740000 | 186741000 | 0.056 | 0.006 | 0.074 | 0.027 | ST6GAL1 | 0.25970 | 1.00000 | 0.00953 | 1 | 1 |
| 303 | chr3 | 186742000 | 186743000 | 0.000 | 0.000 | 0.029 | 0.000 | ST6GAL1 | 0.22755 | 0.54294 | 0.08726 | 1 | 0 |
| 304 | chr3 | 186783000 | 186784000 | 0.000 | 0.050 | 0.338 | 0.041 | ST6GAL1 | 0.00001 | 0.00001 | 0.00000 | 1 | 1 |
| 305 | chr3 | 186784000 | 186785000 | 0.000 | 0.025 | 0.044 | 0.000 | ST6GAL1 | 0.10727 | 0.54966 | 0.42650 | 1 | 0 |
| 306 | chr3 | 187458000 | 187459000 | 0.000 | 0.000 | 0.029 | 0.000 | BCL6 | 0.22755 | 0.54294 | 0.08726 | 1 | 0 |
| 307 | chr3 | 187459000 | 187460000 | 0.000 | 0.000 | 0.029 | 0.000 | BCL6 | 0.22755 | 0.54294 | 0.08726 | 1 | 0 |
| 308 | chr3 | 187460000 | 187461000 | 0.000 | 0.000 | 0.088 | 0.041 | BCL6 | 0.31126 | 0.09031 | 0.00058 | 1 | 1 |
| 309 | chr3 | 187461000 | 187462000 | 0.000 | 0.006 | 0.353 | 0.122 | BCL6 | 0.00137 | 0.00001 | 0.00000 | 1 | 1 |
| 310 | chr3 | 187462000 | 187463000 | 0.056 | 0.081 | 0.647 | 0.392 | BCL6 | 0.00266 | 0.00000 | 0.00000 | 1 | 1 |
| 311 | chr3 | 187463000 | 187464000 | 0.000 | 0.037 | 0.485 | 0.230 | BCL6 | 0.00164 | 0.00000 | 0.00000 | 1 | 1 |
| 312 | chr3 | 187464000 | 187465000 | 0.028 | 0.000 | 0.162 | 0.000 | BCL6 | 0.00019 | 0.05349 | 0.00000 | 1 | 1 |
| 313 | chr3 | 187468000 | 187469000 | 0.000 | 0.000 | 0.044 | 0.000 | BCL6 | 0.10727 | 0.54966 | 0.02537 | 1 | 0 |
| 314 | chr3 | 187635000 | 187636000 | 0.000 | 0.000 | 0.029 | 0.000 | BCL6 | 0.22755 | 0.54294 | 0.08726 | 1 | 0 |
| 315 | chr3 | 187636000 | 187637000 | 0.000 | 0.000 | 0.000 | 0.027 | BCL6 | 0.49735 | 1.00000 | 1.00000 | 1 | 0 |
| 316 | chr3 | 187653000 | 187654000 | 0.000 | 0.000 | 0.044 | 0.014 | BCL6 | 0.34948 | 0.54966 | 0.02537 | 1 | 0 |
| 317 | chr3 | 187658000 | 187659000 | 0.000 | 0.000 | 0.029 | 0.000 | BCL6 | 0.22755 | 0.54294 | 0.08726 | 1 | 0 |
| 318 | chr3 | 187660000 | 187661000 | 0.000 | 0.019 | 0.118 | 0.054 | BCL6 | 0.23086 | 0.04825 | 0.00321 | 1 | 1 |
| 319 | chr3 | 187661000 | 187662000 | 0.000 | 0.012 | 0.191 | 0.081 | BCL6 | 0.08249 | 0.00372 | 0.00000 | 1 | 1 |
| 320 | chr3 | 187664000 | 187665000 | 0.000 | 0.000 | 0.044 | 0.000 | BCL6 | 0.10727 | 0.54966 | 0.02537 | 1 | 0 |
| 321 | chr3 | 187686000 | 187687000 | 0.028 | 0.000 | 0.029 | 0.014 | AC022498.1 | 0.60686 | 1.00000 | 0.08726 | 0 | 0 |
| 322 | chr3 | 187687000 | 187688000 | 0.000 | 0.006 | 0.000 | 0.014 | AC022498.1 | 1.00000 | 1.00000 | 1.00000 | 0 | 0 |
| 323 | chr3 | 187693000 | 187694000 | 0.000 | 0.000 | 0.015 | 0.014 | AC022498.1 | 1.00000 | 1.00000 | 0.29694 | 0 | 0 |
| 324 | chr3 | 187696000 | 187697000 | 0.000 | 0.006 | 0.059 | 0.000 | AC022498.1 | 0.05016 | 0.29551 | 0.02818 | 0 | 0 |
| 325 | chr3 | 187697000 | 187698000 | 0.000 | 0.000 | 0.044 | 0.000 | AC022498.1 | 0.10727 | 0.54966 | 0.02537 | 0 | 0 |
| 326 | chr3 | 187803000 | 187804000 | 0.000 | 0.000 | 0.029 | 0.000 | AC022498.1 | 0.22755 | 0.54294 | 0.08726 | 0 | 0 |
| 327 | chr3 | 187806000 | 187807000 | 0.000 | 0.000 | 0.059 | 0.014 | AC022498.1 | 0.19371 | 0.29551 | 0.00730 | 0 | 1 |
| 328 | chr3 | 187957000 | 187958000 | 0.000 | 0.006 | 0.132 | 0.014 | AC022498.1 | 0.00701 | 0.02564 | 0.00009 | 0 | 1 |
| 329 | chr3 | 187958000 | 187959000 | 0.028 | 0.025 | 0.221 | 0.095 | AC022498.1 | 0.06156 | 0.00936 | 0.00000 | 0 | 1 |
| 330 | chr3 | 187959000 | 187960000 | 0.000 | 0.012 | 0.118 | 0.000 | AC022498.1 | 0.00220 | 0.04825 | 0.00116 | 0 | 1 |
| 331 | chr3 | 187960000 | 187961000 | 0.000 | 0.000 | 0.029 | 0.000 | AC022498.1 | 0.22755 | 0.54294 | 0.08726 | 0 | 0 |
| 332 | chr3 | 188222000 | 188223000 | 0.000 | 0.000 | 0.029 | 0.000 | LPP | 0.22755 | 0.54294 | 0.08726 | 0 | 0 |
| 333 | chr3 | 188298000 | 188299000 | 0.000 | 0.000 | 0.015 | 0.014 | LPP | 1.00000 | 1.00000 | 0.29694 | 0 | 0 |
| 334 | chr3 | 188299000 | 188300000 | 0.000 | 0.006 | 0.088 | 0.027 | LPP | 0.15270 | 0.09031 | 0.00311 | 0 | 1 |
| 335 | chr3 | 188471000 | 188472000 | 0.000 | 0.006 | 0.191 | 0.068 | LPP | 0.04150 | 0.00372 | 0.00000 | 0 | 1 |
| 336 | chr3 | 188472000 | 188473000 | 0.000 | 0.000 | 0.044 | 0.027 | LPP | 0.67043 | 0.54966 | 0.02537 | 0 | 0 |
| 337 | chr4 | 50000 | 51000 | 0.000 | 0.000 | 0.029 | 0.000 | ZNF595;ZNF718; | 0.22755 | 0.54294 | 0.08726 | 0 | 0 |
| 338 | chr4 | 51000 | 52000 | 0.000 | 0.000 | 0.044 | 0.014 | ZNF595;ZNF718; | 0.34948 | 0.54966 | 0.02537 | 0 | 0 |
| 339 | chr4 | 54000 | 55000 | 0.000 | 0.000 | 0.029 | 0.000 | ZNF595;ZNF718; | 0.22755 | 0.54294 | 0.08726 | 0 | 0 |
| 340 | chr4 | 290000 | 291000 | 0.056 | 0.000 | 0.000 | 0.000 | ZNF732 | 1.00000 | 0.11763 | 1.00000 | 0 | 1 |
| 341 | chr4 | 385000 | 386000 | 0.000 | 0.000 | 0.029 | 0.000 | ZNF141 | 0.22755 | 0.54294 | 0.08726 | 0 | 0 |
| 342 | chr4 | 550000 | 551000 | 0.000 | 0.000 | 0.000 | 0.027 | PIGG | 0.49735 | 1.00000 | 1.00000 | 0 | 0 |
| 343 | chr4 | 2707000 | 2708000 | 0.028 | 0.000 | 0.015 | 0.000 | FAM193A | 0.47887 | 1.00000 | 0.29694 | 0 | 0 |
| 344 | chr4 | 5206000 | 5207000 | 0.000 | 0.000 | 0.029 | 0.000 | STK32B | 0.22755 | 0.54294 | 0.08726 | 0 | 0 |
| 345 | chr4 | 25863000 | 25864000 | 0.000 | 0.000 | 0.059 | 0.014 | SEL1L3 | 0.19371 | 0.29551 | 0.00730 | 0 | 1 |
| 346 | chr4 | 25864000 | 25865000 | 0.000 | 0.006 | 0.044 | 0.027 | SEL1L3 | 0.67043 | 0.54966 | 0.07959 | 0 | 0 |
| 347 | chr4 | 25865000 | 25866000 | 0.000 | 0.000 | 0.074 | 0.027 | SEL1L3 | 0.25970 | 0.16101 | 0.00208 | 0 | 1 |
| 348 | chr4 | 29657000 | 29658000 | 0.000 | 0.000 | 0.015 | 0.014 | PCDH7 | 1.00000 | 1.00000 | 0.29694 | 0 | 0 |
| 349 | chr4 | 30356000 | 30357000 | 0.000 | 0.006 | 0.015 | 0.000 | PCDH7 | 0.47887 | 1.00000 | 0.50663 | 0 | 0 |
| 350 | chr4 | 33418000 | 33419000 | 0.000 | 0.000 | 0.029 | 0.000 | PCDH7 | 0.22755 | 0.54294 | 0.08726 | 0 | 0 |
| 351 | chr4 | 33449000 | 33450000 | 0.028 | 0.000 | 0.015 | 0.000 | PCDH7 | 0.47887 | 1.00000 | 0.29694 | 0 | 0 |
| 352 | chr4 | 39348000 | 39349000 | 0.000 | 0.000 | 0.015 | 0.014 | RFC1 | 1.00000 | 1.00000 | 0.29694 | 0 | 0 |

| 353 | chr4 | 39974000 | 39975000 | 0.000 | 0.000 | 0.000 | 0.027 | PDS5A | 0.49735 | 1.00000 | 1.00000 | 0 | 0 |
|---|---|---|---|---|---|---|---|---|---|---|---|---|---|
| 354 | chr4 | 40194000 | 40195000 | 0.000 | 0.000 | 0.044 | 0.027 | N4BP2 | 0.67043 | 0.54966 | 0.02537 | 0 | 0 |
| 355 | chr4 | 40195000 | 40196000 | 0.000 | 0.000 | 0.015 | 0.027 | N4BP2 | 1.00000 | 1.00000 | 0.29694 | 0 | 0 |
| 356 | chr4 | 40196000 | 40197000 | 0.000 | 0.000 | 0.074 | 0.014 | N4BP2 | 0.10420 | 0.16101 | 0.00208 | 0 | 1 |
| 357 | chr4 | 40197000 | 40198000 | 0.000 | 0.000 | 0.015 | 0.027 | N4BP2 | 1.00000 | 1.00000 | 0.29694 | 0 | 0 |
| 358 | chr4 | 40198000 | 40199000 | 0.000 | 0.000 | 0.088 | 0.041 | N4BP2 | 0.31126 | 0.09031 | 0.00058 | 0 | 1 |
| 359 | chr4 | 40199000 | 40200000 | 0.056 | 0.000 | 0.279 | 0.162 | N4BP2 | 0.10628 | 0.00895 | 0.00000 | 0 | 1 |
| 360 | chr4 | 40200000 | 40201000 | 0.000 | 0.006 | 0.118 | 0.041 | RHOH | 0.11795 | 0.04825 | 0.00030 | 1 | 1 |
| 361 | chr4 | 40201000 | 40202000 | 0.000 | 0.000 | 0.088 | 0.041 | RHOH | 0.31126 | 0.09031 | 0.00058 | 1 | 1 |
| 362 | chr4 | 40202000 | 40203000 | 0.000 | 0.000 | 0.029 | 0.014 | RHOH | 0.60686 | 0.54294 | 0.08726 | 1 | 0 |
| 363 | chr4 | 40204000 | 40205000 | 0.000 | 0.000 | 0.029 | 0.000 | RHOH | 0.22755 | 0.54294 | 0.08726 | 1 | 0 |
| 364 | chr4 | 45308000 | 45309000 | 0.000 | 0.000 | 0.029 | 0.000 | GNPDA2 | 0.22755 | 0.54294 | 0.08726 | 0 | 0 |
| 365 | chr4 | 46360000 | 46361000 | 0.000 | 0.000 | 0.015 | 0.014 | GABRA2 | 1.00000 | 1.00000 | 0.29694 | 0 | 0 |
| 366 | chr4 | 62375000 | 62376000 | 0.000 | 0.000 | 0.029 | 0.000 | LPHN3 | 0.22755 | 0.54294 | 0.08726 | 0 | 0 |
| 367 | chr4 | 62530000 | 62531000 | 0.000 | 0.000 | 0.029 | 0.000 | LPHN3 | 0.22755 | 0.54294 | 0.08726 | 0 | 0 |
| 368 | chr4 | 62911000 | 62912000 | 0.000 | 0.000 | 0.029 | 0.000 | LPHN3 | 0.22755 | 0.54294 | 0.08726 | 0 | 0 |
| 369 | chr4 | 63120000 | 63121000 | 0.000 | 0.000 | 0.029 | 0.000 | LPHN3 | 0.22755 | 0.54294 | 0.08726 | 0 | 0 |
| 370 | chr4 | 64015000 | 64016000 | 0.000 | 0.000 | 0.029 | 0.000 | LPHN3 | 0.22755 | 0.54294 | 0.08726 | 0 | 0 |
| 371 | chr4 | 65038000 | 65039000 | 0.000 | 0.000 | 0.015 | 0.014 | TECRL | 1.00000 | 1.00000 | 0.29694 | 0 | 0 |
| 372 | chr4 | 65165000 | 65166000 | 0.000 | 0.000 | 0.015 | 0.014 | TECRL | 1.00000 | 1.00000 | 0.29694 | 0 | 0 |
| 373 | chr4 | 65966000 | 65967000 | 0.000 | 0.006 | 0.000 | 0.014 | EPHA5 | 1.00000 | 1.00000 | 1.00000 | 0 | 0 |
| 374 | chr4 | 66827000 | 66828000 | 0.000 | 0.000 | 0.029 | 0.000 | EPHA5 | 0.22755 | 0.54294 | 0.08726 | 0 | 0 |
| 375 | chr4 | 71531000 | 71532000 | 0.000 | 0.000 | 0.015 | 0.041 | IGJ | 0.62100 | 1.00000 | 0.29694 | 0 | 0 |
| 376 | chr4 | 71532000 | 71533000 | 0.000 | 0.000 | 0.000 | 0.027 | IGJ | 0.49735 | 1.00000 | 1.00000 | 0 | 0 |
| 377 | chr4 | 74456000 | 74457000 | 0.000 | 0.000 | 0.029 | 0.000 | RASSF6 | 0.22755 | 0.54294 | 0.08726 | 0 | 0 |
| 378 | chr4 | 74483000 | 74484000 | 0.000 | 0.006 | 0.015 | 0.000 | RASSF6 | 0.47887 | 1.00000 | 0.50663 | 0 | 0 |
| 379 | chr4 | 74484000 | 74485000 | 0.000 | 0.000 | 0.044 | 0.000 | RASSF6 | 0.10727 | 0.54966 | 0.02537 | 0 | 0 |
| 380 | chr4 | 74485000 | 74486000 | 0.000 | 0.000 | 0.088 | 0.000 | RASSF6 | 0.01070 | 0.09031 | 0.00058 | 0 | 1 |
| 381 | chr4 | 91886000 | 91887000 | 0.000 | 0.000 | 0.015 | 0.014 | CCSER1 | 1.00000 | 1.00000 | 0.29694 | 0 | 0 |
| 382 | chr4 | 92787000 | 92788000 | 0.000 | 0.000 | 0.029 | 0.000 | CCSER1 | 0.22755 | 0.54294 | 0.08726 | 0 | 0 |
| 383 | chr4 | 113206000 | 113207000 | 0.000 | 0.000 | 0.029 | 0.000 | TIFA | 0.22755 | 0.54294 | 0.08726 | 0 | 0 |
| 384 | chr4 | 114466000 | 114467000 | 0.000 | 0.000 | 0.029 | 0.000 | CAMK2D | 0.22755 | 0.54294 | 0.08726 | 0 | 0 |
| 385 | chr4 | 114681000 | 114682000 | 0.000 | 0.000 | 0.044 | 0.000 | CAMK2D | 0.10727 | 0.54966 | 0.02537 | 0 | 0 |
| 386 | chr4 | 117928000 | 117929000 | 0.000 | 0.000 | 0.029 | 0.000 | TRAM1L1 | 0.22755 | 0.54294 | 0.08726 | 0 | 0 |
| 387 | chr4 | 123637000 | 123638000 | 0.000 | 0.000 | 0.000 | 0.027 | BBS12 | 0.49735 | 1.00000 | 1.00000 | 0 | 0 |
| 388 | chr4 | 125227000 | 125228000 | 0.000 | 0.000 | 0.015 | 0.014 | ANKRD50 | 1.00000 | 1.00000 | 0.29694 | 0 | 0 |
| 389 | chr4 | 127371000 | 127372000 | 0.000 | 0.000 | 0.029 | 0.000 | FAT4 | 0.22755 | 0.54294 | 0.08726 | 0 | 0 |
| 390 | chr4 | 133455000 | 133456000 | 0.000 | 0.000 | 0.000 | 0.027 | PCDH10 | 0.49735 | 1.00000 | 1.00000 | 0 | 0 |
| 391 | chr4 | 134538000 | 134539000 | 0.000 | 0.000 | 0.015 | 0.014 | PCDH10 | 1.00000 | 1.00000 | 0.29694 | 0 | 0 |
| 392 | chr4 | 134743000 | 134744000 | 0.000 | 0.000 | 0.029 | 0.000 | PABPC4L | 0.22755 | 0.54294 | 0.08726 | 0 | 0 |
| 393 | chr4 | 134867000 | 134868000 | 0.000 | 0.000 | 0.029 | 0.000 | PABPC4L | 0.22755 | 0.54294 | 0.08726 | 0 | 0 |
| 394 | chr4 | 134949000 | 134950000 | 0.000 | 0.000 | 0.029 | 0.000 | PABPC4L | 0.22755 | 0.54294 | 0.08726 | 0 | 0 |
| 395 | chr4 | 135064000 | 135065000 | 0.000 | 0.000 | 0.015 | 0.014 | PABPC4L | 1.00000 | 1.00000 | 0.29694 | 0 | 0 |
| 396 | chr4 | 135077000 | 135078000 | 0.000 | 0.000 | 0.029 | 0.000 | PABPC4L | 0.22755 | 0.54294 | 0.08726 | 0 | 0 |
| 397 | chr4 | 136799000 | 136800000 | 0.028 | 0.006 | 0.000 | 0.000 | PCDH18 | 1.00000 | 0.34615 | 1.00000 | 0 | 0 |
| 398 | chr4 | 136867000 | 136868000 | 0.000 | 0.000 | 0.015 | 0.014 | PCDH18 | 1.00000 | 1.00000 | 0.29694 | 0 | 0 |
| 399 | chr4 | 140236000 | 140237000 | 0.000 | 0.000 | 0.015 | 0.014 | NAA15 | 1.00000 | 1.00000 | 0.29694 | 0 | 0 |
| 400 | chr4 | 151723000 | 151724000 | 0.000 | 0.000 | 0.029 | 0.000 | LRBA | 0.22755 | 0.54294 | 0.08726 | 0 | 0 |
| 401 | chr4 | 151950000 | 151951000 | 0.000 | 0.000 | 0.000 | 0.027 | LRBA | 0.49735 | 1.00000 | 1.00000 | 0 | 0 |
| 402 | chr4 | 152125000 | 152126000 | 0.028 | 0.000 | 0.029 | 0.000 | SH3D19 | 0.22755 | 1.00000 | 0.08726 | 0 | 0 |
| 403 | chr4 | 157246000 | 157247000 | 0.000 | 0.000 | 0.015 | 0.014 | CTSO | 1.00000 | 1.00000 | 0.29694 | 0 | 0 |

| | | | | | | | | | | | | | |
|---|---|---|---|---|---|---|---|---|---|---|---|---|---|
| 404 | chr4 | 164532000 | 164533000 | 0.000 | 0.000 | 0.000 | 0.027 | 1-Mar-19 | 0.49735 | 1.00000 | 1.00000 | 0 | 0 |
| 405 | chr4 | 178732000 | 178733000 | 0.028 | 0.000 | 0.000 | 0.014 | AGA | 1.00000 | 0.34615 | 1.00000 | 0 | 0 |
| 406 | chr4 | 178885000 | 178886000 | 0.000 | 0.000 | 0.029 | 0.000 | AGA | 0.22755 | 0.54294 | 0.08726 | 0 | 0 |
| 407 | chr4 | 179898000 | 179899000 | 0.000 | 0.000 | 0.029 | 0.000 | AGA | 0.22755 | 0.54294 | 0.08726 | 0 | 0 |
| 408 | chr4 | 180885000 | 180886000 | 0.000 | 0.006 | 0.029 | 0.000 | TENM3 | 0.22755 | 0.54294 | 0.21104 | 0 | 0 |
| 409 | chr4 | 181554000 | 181555000 | 0.000 | 0.000 | 0.029 | 0.000 | TENM3 | 0.22755 | 0.54294 | 0.08726 | 0 | 0 |
| 410 | chr4 | 182122000 | 182123000 | 0.000 | 0.000 | 0.015 | 0.014 | TENM3 | 1.00000 | 1.00000 | 0.29694 | 0 | 0 |
| 411 | chr5 | 436000 | 437000 | 0.028 | 0.000 | 0.000 | 0.014 | AHRR | 1.00000 | 0.34615 | 1.00000 | 0 | 0 |
| 412 | chr5 | 3982000 | 3983000 | 0.000 | 0.000 | 0.029 | 0.000 | IRX1 | 0.22755 | 0.54294 | 0.08726 | 0 | 0 |
| 413 | chr5 | 17218000 | 17219000 | 0.000 | 0.000 | 0.029 | 0.000 | BASP1 | 0.22755 | 0.54294 | 0.08726 | 0 | 0 |
| 414 | chr5 | 17219000 | 17220000 | 0.000 | 0.000 | 0.029 | 0.000 | BASP1 | 0.22755 | 0.54294 | 0.08726 | 0 | 0 |
| 415 | chr5 | 18514000 | 18515000 | 0.028 | 0.000 | 0.000 | 0.014 | CDH18 | 1.00000 | 0.34615 | 1.00000 | 0 | 0 |
| 416 | chr5 | 22356000 | 22357000 | 0.000 | 0.000 | 0.029 | 0.000 | CDH12 | 0.22755 | 0.54294 | 0.08726 | 0 | 0 |
| 417 | chr5 | 22517000 | 22518000 | 0.000 | 0.000 | 0.015 | 0.014 | CDH12 | 1.00000 | 1.00000 | 0.29694 | 0 | 0 |
| 418 | chr5 | 24632000 | 24633000 | 0.000 | 0.000 | 0.029 | 0.000 | CDH10 | 0.22755 | 0.54294 | 0.08726 | 0 | 0 |
| 419 | chr5 | 25275000 | 25276000 | 0.000 | 0.000 | 0.015 | 0.014 | CDH10 | 1.00000 | 1.00000 | 0.29694 | 0 | 0 |
| 420 | chr5 | 25541000 | 25542000 | 0.000 | 0.000 | 0.029 | 0.000 | CDH10 | 0.22755 | 0.54294 | 0.08726 | 0 | 0 |
| 421 | chr5 | 26119000 | 26120000 | 0.000 | 0.000 | 0.015 | 0.014 | CDH9 | 1.00000 | 1.00000 | 0.29694 | 0 | 0 |
| 422 | chr5 | 26450000 | 26451000 | 0.000 | 0.000 | 0.029 | 0.000 | CDH9 | 0.22755 | 0.54294 | 0.08726 | 0 | 0 |
| 423 | chr5 | 29224000 | 29225000 | 0.000 | 0.000 | 0.029 | 0.000 | CDH6 | 0.22755 | 0.54294 | 0.08726 | 0 | 0 |
| 424 | chr5 | 29492000 | 29493000 | 0.000 | 0.000 | 0.029 | 0.000 | CDH6 | 0.22755 | 0.54294 | 0.08726 | 0 | 0 |
| 425 | chr5 | 29648000 | 29649000 | 0.000 | 0.000 | 0.029 | 0.000 | CDH6 | 0.22755 | 0.54294 | 0.08726 | 0 | 0 |
| 426 | chr5 | 51521000 | 51522000 | 0.000 | 0.000 | 0.044 | 0.014 | CTD-2203A3.1 | 0.34948 | 0.54966 | 0.02537 | 0 | 0 |
| 427 | chr5 | 83841000 | 83842000 | 0.000 | 0.000 | 0.029 | 0.000 | EDIL3 | 0.22755 | 0.54294 | 0.08726 | 0 | 0 |
| 428 | chr5 | 88177000 | 88178000 | 0.000 | 0.000 | 0.029 | 0.000 | MEF2C | 0.22755 | 0.54294 | 0.08726 | 0 | 0 |
| 429 | chr5 | 88178000 | 88179000 | 0.000 | 0.000 | 0.015 | 0.014 | MEF2C | 1.00000 | 1.00000 | 0.29694 | 0 | 0 |
| 430 | chr5 | 91417000 | 91418000 | 0.000 | 0.000 | 0.000 | 0.027 | ARRDC3 | 0.49735 | 1.00000 | 1.00000 | 0 | 0 |
| 431 | chr5 | 103678000 | 103679000 | 0.000 | 0.000 | 0.015 | 0.014 | NUDT12 | 1.00000 | 1.00000 | 0.29694 | 0 | 0 |
| 432 | chr5 | 123696000 | 123697000 | 0.000 | 0.000 | 0.000 | 0.027 | ZNF608 | 0.49735 | 1.00000 | 1.00000 | 1 | 0 |
| 433 | chr5 | 124079000 | 124080000 | 0.000 | 0.000 | 0.029 | 0.014 | ZNF608 | 0.60686 | 0.54294 | 0.08726 | 1 | 0 |
| 434 | chr5 | 124080000 | 124081000 | 0.000 | 0.000 | 0.029 | 0.014 | ZNF608 | 0.60686 | 0.54294 | 0.08726 | 1 | 0 |
| 435 | chr5 | 127594000 | 127595000 | 0.000 | 0.000 | 0.015 | 0.014 | FBN2 | 1.00000 | 1.00000 | 0.29694 | 0 | 0 |
| 436 | chr5 | 127875000 | 127876000 | 0.000 | 0.000 | 0.000 | 0.027 | FBN2 | 0.49735 | 1.00000 | 1.00000 | 0 | 0 |
| 437 | chr5 | 131825000 | 131826000 | 0.000 | 0.000 | 0.074 | 0.000 | IRF1 | 0.02326 | 0.16101 | 0.00208 | 0 | 1 |
| 438 | chr5 | 131826000 | 131827000 | 0.000 | 0.000 | 0.029 | 0.000 | IRF1 | 0.22755 | 0.54294 | 0.08726 | 0 | 0 |
| 439 | chr5 | 149791000 | 149792000 | 0.000 | 0.000 | 0.132 | 0.014 | CD74 | 0.00701 | 0.02564 | 0.00001 | 1 | 1 |
| 440 | chr5 | 149792000 | 149793000 | 0.000 | 0.000 | 0.015 | 0.014 | CD74 | 1.00000 | 1.00000 | 0.29694 | 1 | 0 |
| 441 | chr5 | 158380000 | 158381000 | 0.028 | 0.000 | 0.015 | 0.000 | EBF1 | 0.47887 | 1.00000 | 0.29694 | 0 | 0 |
| 442 | chr5 | 158479000 | 158480000 | 0.000 | 0.000 | 0.029 | 0.000 | EBF1 | 0.22755 | 0.54294 | 0.08726 | 0 | 0 |
| 443 | chr5 | 158526000 | 158527000 | 0.028 | 0.000 | 0.044 | 0.000 | EBF1 | 0.10727 | 1.00000 | 0.02537 | 0 | 0 |
| 444 | chr5 | 158527000 | 158528000 | 0.000 | 0.000 | 0.029 | 0.000 | EBF1 | 0.22755 | 0.54294 | 0.08726 | 0 | 0 |
| 445 | chr5 | 158528000 | 158529000 | 0.000 | 0.000 | 0.059 | 0.000 | EBF1 | 0.05016 | 0.29551 | 0.00730 | 0 | 1 |
| 446 | chr5 | 164247000 | 164248000 | 0.000 | 0.000 | 0.029 | 0.000 | MAT2B | 0.22755 | 0.54294 | 0.08726 | 0 | 0 |
| 447 | chr5 | 164441000 | 164442000 | 0.028 | 0.000 | 0.015 | 0.000 | MAT2B | 0.47887 | 1.00000 | 0.29694 | 0 | 0 |
| 448 | chr5 | 165932000 | 165933000 | 0.000 | 0.000 | 0.015 | 0.014 | TENM2 | 1.00000 | 1.00000 | 0.29694 | 0 | 0 |
| 449 | chr5 | 173300000 | 173301000 | 0.000 | 0.000 | 0.000 | 0.027 | CPEB4 | 0.49735 | 1.00000 | 1.00000 | 0 | 0 |
| 450 | chr5 | 179166000 | 179167000 | 0.000 | 0.000 | 0.000 | 0.027 | MAML1 | 1.00000 | 1.00000 | 0.29694 | 0 | 0 |
| 451 | chr5 | 180102000 | 180103000 | 0.000 | 0.000 | 0.015 | 0.014 | FLT4 | 1.00000 | 1.00000 | 0.29694 | 0 | 0 |
| 452 | chr6 | 392000 | 393000 | 0.000 | 0.000 | 0.074 | 0.000 | IRF4 | 0.02326 | 0.16101 | 0.00208 | 1 | 1 |
| 453 | chr6 | 393000 | 394000 | 0.000 | 0.000 | 0.074 | 0.000 | IRF4 | 0.02326 | 0.16101 | 0.00208 | 1 | 1 |
| 454 | chr6 | 14118000 | 14119000 | 0.000 | 0.000 | 0.279 | 0.041 | CD83 | 0.00011 | 0.00013 | 0.00000 | 1 | 1 |

| | | | | | | | | | | | | | | |
|---|---|---|---|---|---|---|---|---|---|---|---|---|---|---|
| 455 | chr6 | 14119000 | 14120000 | 0.000 | 0.000 | 0.044 | 0.027 | | CD83 | 0.67043 | 0.54966 | 0.02537 | 1 | 0 |
| 456 | chr6 | 18111000 | 18112000 | 0.028 | 0.000 | 0.044 | 0.000 | | NHLRC1 | 0.10727 | 1.00000 | 0.02537 | 0 | 0 |
| 457 | chr6 | 18387000 | 18388000 | 0.000 | 0.000 | 0.000 | 0.027 | | RNF144B | 0.49735 | 1.00000 | 1.00000 | 1 | 0 |
| 458 | chr6 | 18388000 | 18389000 | 0.000 | 0.000 | 0.000 | 0.027 | | RNF144B | 0.49735 | 1.00000 | 1.00000 | 1 | 0 |
| 459 | chr6 | 19573000 | 19574000 | 0.000 | 0.000 | 0.029 | 0.000 | | ID4 | 0.22755 | 0.54294 | 0.08726 | 0 | 0 |
| 460 | chr6 | 22873000 | 22874000 | 0.000 | 0.000 | 0.015 | 0.014 | | HDGFL1 | 1.00000 | 1.00000 | 0.29694 | 0 | 0 |
| 461 | chr6 | 26031000 | 26032000 | 0.000 | 0.000 | 0.000 | 0.027 | | HIST1H3B | 0.49735 | 1.00000 | 1.00000 | 1 | 0 |
| 462 | chr6 | 26032000 | 26033000 | 0.000 | 0.000 | 0.000 | 0.027 | | HIST1H3B | 0.49735 | 1.00000 | 1.00000 | 1 | 0 |
| 463 | chr6 | 26056000 | 26057000 | 0.000 | 0.000 | 0.059 | 0.027 | | HIST1H1C | 0.42627 | 0.29551 | 0.00730 | 1 | 1 |
| 464 | chr6 | 26123000 | 26124000 | 0.000 | 0.000 | 0.059 | 0.014 | | HIST1H2BC | 0.19371 | 0.29551 | 0.00730 | 1 | 1 |
| 465 | chr6 | 26124000 | 26125000 | 0.000 | 0.000 | 0.074 | 0.000 | | HIST1H2AC;HIST1H2BC; | 0.02326 | 0.16101 | 0.00208 | 0 | 1 |
| 466 | chr6 | 26125000 | 26126000 | 0.000 | 0.000 | 0.015 | 0.014 | | HIST1H2AC | 1.00000 | 1.00000 | 0.29694 | 1 | 0 |
| 467 | chr6 | 26156000 | 26157000 | 0.000 | 0.000 | 0.074 | 0.014 | | HIST1H1E | 0.10420 | 0.16101 | 0.00208 | 1 | 1 |
| 468 | chr6 | 26157000 | 26158000 | 0.000 | 0.000 | 0.029 | 0.014 | | HIST1H1E | 0.60686 | 0.54294 | 0.08726 | 1 | 0 |
| 469 | chr6 | 26216000 | 26217000 | 0.000 | 0.000 | 0.029 | 0.000 | | HIST1H2BG | 0.22755 | 0.54294 | 0.08726 | 1 | 0 |
| 470 | chr6 | 26234000 | 26235000 | 0.000 | 0.000 | 0.044 | 0.000 | | HIST1H1D | 0.10727 | 0.54966 | 0.02537 | 0 | 0 |
| 471 | chr6 | 27101000 | 27102000 | 0.000 | 0.000 | 0.029 | 0.000 | | HIST1H2AG | 0.22755 | 0.54294 | 0.08726 | 1 | 0 |
| 472 | chr6 | 27114000 | 27115000 | 0.000 | 0.000 | 0.059 | 0.014 | | HIST1H2AH;HIST1H2BK; | 0.19371 | 0.29551 | 0.00730 | 0 | 1 |
| 473 | chr6 | 27792000 | 27793000 | 0.000 | 0.000 | 0.044 | 0.014 | | HIST1H4J | 0.34948 | 0.54966 | 0.02537 | 0 | 0 |
| 474 | chr6 | 27833000 | 27834000 | 0.000 | 0.000 | 0.015 | 0.014 | | HIST1H2AL | 1.00000 | 1.00000 | 0.29694 | 1 | 0 |
| 475 | chr6 | 27860000 | 27861000 | 0.000 | 0.000 | 0.029 | 0.027 | | HIST1H2AM | 1.00000 | 0.54294 | 0.08726 | 1 | 0 |
| 476 | chr6 | 27861000 | 27862000 | 0.000 | 0.000 | 0.015 | 0.014 | | HIST1H2BO | 1.00000 | 1.00000 | 0.29694 | 1 | 0 |
| 477 | chr6 | 29778000 | 29779000 | 0.028 | 0.000 | 0.000 | 0.014 | | LOC554223 | 1.00000 | 0.34615 | 1.00000 | 0 | 0 |
| 478 | chr6 | 29780000 | 29781000 | 0.000 | 0.000 | 0.015 | 0.014 | | HLA-G | 1.00000 | 1.00000 | 0.29694 | 0 | 0 |
| 479 | chr6 | 29911000 | 29912000 | 0.000 | 0.000 | 0.044 | 0.000 | | HLA-A | 0.10727 | 0.54966 | 0.02537 | 0 | 0 |
| 480 | chr6 | 29927000 | 29928000 | 0.000 | 0.000 | 0.015 | 0.014 | | HLA-A | 1.00000 | 1.00000 | 0.29694 | 0 | 0 |
| 481 | chr6 | 31324000 | 31325000 | 0.000 | 0.000 | 0.029 | 0.014 | | HLA-B | 0.60686 | 0.54294 | 0.08726 | 1 | 0 |
| 482 | chr6 | 31325000 | 31326000 | 0.028 | 0.000 | 0.000 | 0.014 | | HLA-B | 1.00000 | 0.34615 | 1.00000 | 1 | 0 |
| 483 | chr6 | 31543000 | 31544000 | 0.000 | 0.000 | 0.029 | 0.000 | | TNF | 0.22755 | 0.54294 | 0.08726 | 1 | 0 |
| 484 | chr6 | 31549000 | 31550000 | 0.000 | 0.000 | 0.006 | 0.191 | 0.068 | LTB | 0.04150 | 0.00372 | 0.00000 | 1 | 1 |
| 485 | chr6 | 31550000 | 31551000 | 0.000 | 0.000 | 0.044 | 0.000 | | LTB | 0.10727 | 0.54966 | 0.02537 | 1 | 0 |
| 486 | chr6 | 32440000 | 32441000 | 0.000 | 0.000 | 0.044 | 0.027 | | HLA-DRA | 0.67043 | 0.54966 | 0.02537 | 0 | 0 |
| 487 | chr6 | 32451000 | 32452000 | 0.056 | 0.000 | 0.000 | 0.000 | | HLA-DRB5 | 1.00000 | 0.11763 | 1.00000 | 0 | 1 |
| 488 | chr6 | 32452000 | 32453000 | 0.028 | 0.000 | 0.015 | 0.000 | | HLA-DRB5 | 0.47887 | 1.00000 | 0.29694 | 0 | 0 |
| 489 | chr6 | 32455000 | 32456000 | 0.028 | 0.000 | 0.015 | 0.000 | | HLA-DRB5 | 0.47887 | 1.00000 | 0.29694 | 0 | 0 |
| 490 | chr6 | 32457000 | 32458000 | 0.000 | 0.000 | 0.000 | 0.027 | | HLA-DRB5 | 0.49735 | 1.00000 | 1.00000 | 0 | 0 |
| 491 | chr6 | 32498000 | 32499000 | 0.000 | 0.000 | 0.000 | 0.027 | | HLA-DRB5 | 0.49735 | 1.00000 | 1.00000 | 0 | 0 |
| 492 | chr6 | 32505000 | 32506000 | 0.000 | 0.000 | 0.029 | 0.014 | | HLA-DRB5 | 0.60686 | 0.54294 | 0.08726 | 0 | 0 |
| 493 | chr6 | 32511000 | 32512000 | 0.000 | 0.000 | 0.000 | 0.041 | | HLA-DRB5 | 0.24603 | 1.00000 | 1.00000 | 0 | 0 |
| 494 | chr6 | 32522000 | 32523000 | 0.028 | 0.000 | 0.015 | 0.027 | | HLA-DRB1 | 1.00000 | 1.00000 | 0.29694 | 0 | 0 |
| 495 | chr6 | 32525000 | 32526000 | 0.000 | 0.000 | 0.029 | 0.014 | | HLA-DRB1 | 0.60686 | 0.54294 | 0.08726 | 0 | 0 |
| 496 | chr6 | 32526000 | 32527000 | 0.000 | 0.000 | 0.000 | 0.041 | | HLA-DRB1 | 0.24603 | 1.00000 | 1.00000 | 0 | 0 |
| 497 | chr6 | 32527000 | 32528000 | 0.000 | 0.000 | 0.000 | 0.027 | | HLA-DRB1 | 0.49735 | 1.00000 | 1.00000 | 0 | 0 |
| 498 | chr6 | 32548000 | 32549000 | 0.000 | 0.000 | 0.029 | 0.014 | | HLA-DRB1 | 0.60686 | 0.54294 | 0.08726 | 0 | 0 |
| 499 | chr6 | 32552000 | 32553000 | 0.056 | 0.000 | 0.015 | 0.027 | | HLA-DRB1 | 1.00000 | 0.27446 | 0.29694 | 0 | 1 |
| 500 | chr6 | 32557000 | 32558000 | 0.028 | 0.000 | 0.000 | 0.041 | | HLA-DRB1 | 0.24603 | 0.34615 | 1.00000 | 0 | 0 |
| 501 | chr6 | 32609000 | 32610000 | 0.028 | 0.000 | 0.059 | 0.014 | | HLA-DQA1 | 0.19371 | 0.65667 | 0.00730 | 0 | 1 |
| 502 | chr6 | 32630000 | 32631000 | 0.000 | 0.000 | 0.015 | 0.014 | | HLA-DQB1 | 1.00000 | 1.00000 | 0.29694 | 0 | 0 |
| 503 | chr6 | 32632000 | 32633000 | 0.111 | 0.000 | 0.029 | 0.027 | | HLA-DQB1 | 1.00000 | 0.17874 | 0.08726 | 0 | 1 |

EP 4 334 476 B1

| | | | | | | | | | | | | | |
|---|---|---|---|---|---|---|---|---|---|---|---|---|---|
| 504 | chr6 | 32727000 | 32728000 | 0.056 | 0.000 | 0.015 | 0.000 | HLA-DQB2 | 0.47887 | 0.27446 | 0.29694 | 0 | 1 |
| 505 | chr6 | 32729000 | 32730000 | 0.056 | 0.000 | 0.029 | 0.014 | HLA-DQB2 | 0.60686 | 0.60763 | 0.08726 | 0 | 1 |
| 506 | chr6 | 33048000 | 33049000 | 0.000 | 0.000 | 0.015 | 0.014 | HLA-DPB1 | 1.00000 | 1.00000 | 0.29694 | 0 | 0 |
| 507 | chr6 | 34179000 | 34180000 | 0.000 | 0.000 | 0.029 | 0.000 | HMGA1 | 0.22755 | 0.54294 | 0.08726 | 0 | 0 |
| 508 | chr6 | 37138000 | 37139000 | 0.000 | 0.000 | 0.191 | 0.081 | PIM1 | 0.08249 | 0.00372 | 0.00000 | 1 | 1 |
| 509 | chr6 | 37139000 | 37140000 | 0.000 | 0.000 | 0.088 | 0.041 | PIM1 | 0.31126 | 0.09031 | 0.00058 | 1 | 1 |
| 510 | chr6 | 37140000 | 37141000 | 0.000 | 0.000 | 0.029 | 0.014 | PIM1 | 0.60686 | 0.54294 | 0.08726 | 1 | 0 |
| 511 | chr6 | 58001000 | 58002000 | 0.000 | 0.000 | 0.015 | 0.014 | PRIM2 | 1.00000 | 1.00000 | 0.29694 | 0 | 0 |
| 512 | chr6 | 67923000 | 67924000 | 0.000 | 0.000 | 0.015 | 0.014 | BAI3 | 1.00000 | 1.00000 | 0.29694 | 0 | 0 |
| 513 | chr6 | 72256000 | 72257000 | 0.000 | 0.000 | 0.029 | 0.000 | IMPG1 | 0.22755 | 0.54294 | 0.08726 | 0 | 0 |
| 514 | chr6 | 81437000 | 81438000 | 0.000 | 0.000 | 0.015 | 0.014 | BCKDHB | 1.00000 | 1.00000 | 0.29694 | 0 | 0 |
| 515 | chr6 | 88468000 | 88469000 | 0.000 | 0.000 | 0.015 | 0.014 | AKIRIN2 | 1.00000 | 1.00000 | 0.29694 | 0 | 0 |
| 516 | chr6 | 88630000 | 88631000 | 0.000 | 0.000 | 0.044 | 0.014 | SPACA1 | 0.34948 | 0.54966 | 0.02537 | 0 | 0 |
| 517 | chr6 | 88876000 | 88877000 | 0.028 | 0.000 | 0.015 | 0.000 | CNR1 | 0.47887 | 1.00000 | 0.29694 | 0 | 0 |
| 518 | chr6 | 89323000 | 89324000 | 0.000 | 0.000 | 0.029 | 0.014 | RNGTT | 0.60686 | 0.54294 | 0.08726 | 0 | 0 |
| 519 | chr6 | 89338000 | 89339000 | 0.000 | 0.000 | 0.029 | 0.000 | RNGTT | 0.22755 | 0.54294 | 0.08726 | 0 | 0 |
| 520 | chr6 | 89348000 | 89349000 | 0.000 | 0.000 | 0.044 | 0.000 | RNGTT | 0.10727 | 0.54966 | 0.02537 | 0 | 0 |
| 521 | chr6 | 89470000 | 89471000 | 0.000 | 0.000 | 0.029 | 0.000 | RNGTT | 0.22755 | 0.54294 | 0.08726 | 0 | 0 |
| 522 | chr6 | 89471000 | 89472000 | 0.000 | 0.000 | 0.029 | 0.000 | RNGTT | 0.22755 | 0.54294 | 0.08726 | 0 | 0 |
| 523 | chr6 | 90061000 | 90062000 | 0.000 | 0.000 | 0.059 | 0.000 | UBE2J1 | 0.05016 | 0.29551 | 0.00730 | 1 | 1 |
| 524 | chr6 | 90062000 | 90063000 | 0.000 | 0.000 | 0.029 | 0.000 | UBE2J1 | 0.22755 | 0.54294 | 0.08726 | 1 | 0 |
| 525 | chr6 | 90994000 | 90995000 | 0.000 | 0.000 | 0.029 | 0.014 | MAP3K7 | 0.60686 | 0.54294 | 0.08726 | 0 | 0 |
| 526 | chr6 | 91004000 | 91005000 | 0.000 | 0.000 | 0.059 | 0.014 | MAP3K7 | 0.19371 | 0.29551 | 0.00730 | 0 | 1 |
| 527 | chr6 | 91005000 | 91006000 | 0.000 | 0.019 | 0.294 | 0.095 | MAP3K7 | 0.00279 | 0.00011 | 0.00000 | 0 | 1 |
| 528 | chr6 | 91006000 | 91007000 | 0.000 | 0.006 | 0.118 | 0.027 | MAP3K7 | 0.04838 | 0.04825 | 0.00030 | 0 | 1 |
| 529 | chr6 | 91007000 | 91008000 | 0.000 | 0.012 | 0.029 | | MAP3K7 | 0.22755 | 0.54294 | 0.58408 | 0 | 0 |
| 530 | chr6 | 94822000 | 94823000 | 0.028 | 0.000 | 0.015 | 0.000 | EPHA7 | 0.47887 | 1.00000 | 0.29694 | 0 | 0 |
| 531 | chr6 | 107704000 | 107705000 | 0.028 | 0.000 | 0.000 | 0.014 | PDSS2 | 1.00000 | 0.34615 | 1.00000 | 0 | 0 |
| 532 | chr6 | 112885000 | 112886000 | 0.000 | 0.000 | 0.015 | 0.014 | RFPL4B | 1.00000 | 1.00000 | 0.29694 | 0 | 0 |
| 533 | chr6 | 118244000 | 118245000 | 0.000 | 0.000 | 0.015 | 0.014 | SLC35F1 | 1.00000 | 1.00000 | 0.29694 | 0 | 0 |
| 534 | chr6 | 121288000 | 121289000 | 0.000 | 0.000 | 0.000 | 0.027 | C6orf170 | 0.49735 | 1.00000 | 1.00000 | 0 | 0 |
| 535 | chr6 | 121489000 | 121490000 | 0.000 | 0.000 | 0.029 | 0.000 | C6orf170 | 0.22755 | 0.54294 | 0.08726 | 0 | 0 |
| 536 | chr6 | 123504000 | 123505000 | 0.000 | 0.006 | 0.015 | 0.000 | TRDN | 0.47887 | 1.00000 | 0.50663 | 0 | 0 |
| 537 | chr6 | 127313000 | 127314000 | 0.000 | 0.006 | 0.015 | 0.000 | RSPO3 | 0.47887 | 1.00000 | 0.50663 | 0 | 0 |
| 538 | chr6 | 133785000 | 133786000 | 0.000 | 0.000 | 0.029 | 0.000 | EYA4 | 0.22755 | 0.54294 | 0.08726 | 0 | 0 |
| 539 | chr6 | 134491000 | 134492000 | 0.000 | 0.000 | 0.029 | 0.000 | SGK1 | 0.22755 | 0.54294 | 0.08726 | 1 | 0 |
| 540 | chr6 | 134492000 | 134493000 | 0.000 | 0.000 | 0.044 | 0.014 | SGK1 | 0.34948 | 0.54966 | 0.02537 | 1 | 0 |
| 541 | chr6 | 134493000 | 134494000 | 0.000 | 0.000 | 0.029 | 0.000 | SGK1 | 0.22755 | 0.54294 | 0.08726 | 1 | 0 |
| 542 | chr6 | 134494000 | 134495000 | 0.000 | 0.000 | 0.029 | 0.000 | SGK1 | 0.22755 | 0.54294 | 0.08726 | 1 | 0 |
| 543 | chr6 | 134495000 | 134496000 | 0.000 | 0.000 | 0.162 | 0.041 | SGK1 | 0.02233 | 0.01471 | 0.00000 | 1 | 1 |
| 544 | chr6 | 134496000 | 134497000 | 0.000 | 0.000 | 0.029 | 0.000 | SGK1 | 0.22755 | 0.54294 | 0.08726 | 1 | 0 |
| 545 | chr6 | 142046000 | 142047000 | 0.000 | 0.000 | 0.059 | 0.000 | NMBR | 0.05016 | 0.29551 | 0.00730 | 0 | 1 |
| 546 | chr6 | 147860000 | 147861000 | 0.028 | 0.000 | 0.015 | 0.000 | SAMD5 | 0.47887 | 1.00000 | 0.29694 | 0 | 0 |
| 547 | chr6 | 150954000 | 150955000 | 0.000 | 0.000 | 0.044 | 0.014 | PLEKHG1 | 0.34948 | 0.54966 | 0.02537 | 0 | 0 |
| 548 | chr6 | 159238000 | 159239000 | 0.000 | 0.012 | 0.044 | 0.014 | EZR | 0.34948 | 0.54966 | 0.15671 | 0 | 0 |
| 549 | chr6 | 159239000 | 159240000 | 0.000 | 0.000 | 0.029 | 0.014 | EZR | 0.60686 | 0.54294 | 0.08726 | 0 | 0 |
| 550 | chr6 | 159240000 | 159241000 | 0.000 | 0.000 | 0.029 | 0.014 | EZR | 0.60686 | 0.54294 | 0.08726 | 0 | 0 |
| 551 | chr6 | 159464000 | 159465000 | 0.000 | 0.000 | 0.015 | 0.014 | TAGAP | 1.00000 | 1.00000 | 0.29694 | 0 | 0 |
| 552 | chr6 | 159465000 | 159466000 | 0.000 | 0.000 | 0.029 | 0.000 | TAGAP | 0.22755 | 0.54294 | 0.08726 | 0 | 0 |
| 553 | chr6 | 161265000 | 161266000 | 0.028 | 0.000 | 0.000 | 0.027 | PLG | 0.49735 | 0.34615 | 1.00000 | 0 | 0 |
| 554 | chr6 | 161833000 | 161834000 | 0.028 | 0.000 | 0.000 | 0.027 | PARK2 | 0.49735 | 0.34615 | 1.00000 | 0 | 0 |

| No. | chr | start | end | | | | | | gene | | | | | |
|---|---|---|---|---|---|---|---|---|---|---|---|---|---|---|
| 555 | chr6 | 162712000 | 162713000 | 0.000 | 0.000 | 0.000 | 0.029 | 0.000 | PARK2 | 0.22755 | 0.54294 | 0.08726 | 0 | 0 |
| 556 | chr6 | 164941000 | 164942000 | 0.000 | 0.000 | 0.000 | 0.029 | 0.000 | C6orf118 | 0.22755 | 0.54294 | 0.08726 | 0 | 0 |
| 557 | chr6 | 168813000 | 168814000 | 0.023 | 0.000 | 0.000 | 0.015 | 0.000 | SMOC2 | 0.47887 | 1.00000 | 0.29694 | 0 | 0 |
| 558 | chr7 | 1898000 | 1899000 | 0.000 | 0.000 | 0.000 | 0.029 | 0.000 | AC110781.3 | 0.22755 | 0.54294 | 0.08726 | 0 | 0 |
| 559 | chr7 | 1963000 | 1964000 | 0.028 | 0.000 | 0.000 | 0.015 | 0.014 | MAD1L1 | 1.00000 | 1.00000 | 0.29694 | 0 | 0 |
| 560 | chr7 | 2080000 | 2081000 | 0.000 | 0.000 | 0.000 | 0.015 | 0.014 | MAD1L1 | 1.00000 | 1.00000 | 0.29694 | 0 | 0 |
| 561 | chr7 | 5568000 | 5569000 | 0.000 | 0.000 | 0.000 | 0.059 | 0.014 | ACTB | 0.19371 | 0.29551 | 0.60730 | 1 | 1 |
| 562 | chr7 | 5569000 | 5570000 | 0.000 | 0.000 | 0.000 | 0.059 | 0.014 | ACTB | 0.19371 | 0.29551 | 0.60730 | 1 | 1 |
| 563 | chr7 | 5570000 | 5571000 | 0.000 | 0.000 | 0.000 | 0.015 | 0.027 | ACTB | 1.00000 | 1.00000 | 0.29694 | 0 | 0 |
| 564 | chr7 | 9933000 | 9934000 | 0.000 | 0.000 | 0.000 | 0.029 | 0.014 | NDUFA4 | 0.60686 | 0.54294 | 0.08726 | 0 | 0 |
| 565 | chr7 | 13017000 | 13018000 | 0.028 | 0.000 | 0.000 | 0.015 | 0.000 | ARL4A | 0.47887 | 1.00000 | 0.29694 | 0 | 0 |
| 566 | chr7 | 13346000 | 13347000 | 0.000 | 0.000 | 0.000 | 0.000 | 0.027 | ETV1 | 0.49735 | 1.00000 | 1.00000 | 0 | 0 |
| 567 | chr7 | 15459000 | 15460000 | 0.000 | 0.000 | 0.000 | 0.000 | 0.027 | AGMO | 0.49735 | 1.00000 | 1.00000 | 0 | 0 |
| 568 | chr7 | 16382000 | 16383000 | 0.000 | 0.000 | 0.000 | 0.015 | 0.014 | ISPD | 1.00000 | 1.00000 | 0.29694 | 0 | 0 |
| 569 | chr7 | 28600000 | 28601000 | 0.028 | 0.000 | 0.000 | 0.015 | 0.000 | CREB5 | 0.47887 | 1.00000 | 0.29694 | 0 | 0 |
| 570 | chr7 | 40846000 | 40847000 | 0.000 | 0.000 | 0.000 | 0.015 | 0.041 | C7orf10 | 0.62100 | 1.00000 | 0.29694 | 0 | 0 |
| 571 | chr7 | 50349000 | 50350000 | 0.000 | 0.000 | 0.000 | 0.059 | 0.014 | IKZF1 | 0.19371 | 0.29551 | 0.60730 | 1 | 1 |
| 572 | chr7 | 50350000 | 50351000 | 0.000 | 0.000 | 0.000 | 0.044 | 0.000 | IKZF1 | 0.10727 | 0.54966 | 0.02537 | 0 | 0 |
| 573 | chr7 | 53335000 | 53336000 | 0.028 | 0.000 | 0.000 | 0.000 | 0.027 | POM121L12 | 0.49735 | 1.00000 | 1.00000 | 0 | 0 |
| 574 | chr7 | 57713000 | 57714000 | 0.000 | 0.000 | 0.000 | 0.029 | 0.000 | ZNF716 | 0.22755 | 0.54294 | 0.29694 | 0 | 0 |
| 575 | chr7 | 62475000 | 62476000 | 0.000 | 0.000 | 0.000 | 0.015 | 0.027 | AC006455.1 | 1.00000 | 1.00000 | 1.00000 | 0 | 0 |
| 576 | chr7 | 70669000 | 70670000 | 0.000 | 0.000 | 0.000 | 0.015 | 0.000 | WBSCR17 | 0.22755 | 0.54294 | 0.08726 | 0 | 0 |
| 577 | chr7 | 71553000 | 71554000 | 0.000 | 0.000 | 0.000 | 0.015 | 0.014 | CALN1 | 1.00000 | 1.00000 | 0.29694 | 0 | 0 |
| 578 | chr7 | 79847000 | 79848000 | 0.000 | 0.000 | 0.000 | 0.015 | 0.014 | GNAI1 | 1.00000 | 1.00000 | 0.29694 | 0 | 0 |
| 579 | chr7 | 80694000 | 80695000 | 0.000 | 0.000 | 0.000 | 0.029 | 0.000 | AC004908.2 | 0.22755 | 0.54294 | 0.08726 | 0 | 0 |
| 580 | chr7 | 81556000 | 81557000 | 0.000 | 0.000 | 0.000 | 0.000 | 0.027 | CACNA2D1 | 0.49735 | 1.00000 | 1.00000 | 0 | 0 |
| 581 | chr7 | 84127000 | 84128000 | 0.028 | 0.000 | 0.000 | 0.015 | 0.000 | SEMA3A | 0.22755 | 1.00000 | 0.29694 | 0 | 0 |
| 582 | chr7 | 84247000 | 84248000 | 0.000 | 0.000 | 0.000 | 0.029 | 0.000 | SEMA3D | 0.22755 | 0.54294 | 0.08726 | 0 | 0 |
| 583 | chr7 | 84257000 | 84258000 | 0.028 | 0.000 | 0.000 | 0.015 | 0.000 | SEMA3D | 0.47887 | 1.00000 | 1.00000 | 0 | 0 |
| 584 | chr7 | 86914000 | 86915000 | 0.000 | 0.000 | 0.000 | 0.015 | 0.014 | CROT | 1.00000 | 1.00000 | 0.29694 | 0 | 0 |
| 585 | chr7 | 90356000 | 90357000 | 0.000 | 0.000 | 0.000 | 0.029 | 0.000 | CDK14 | 0.22755 | 0.54294 | 0.08726 | 0 | 0 |
| 586 | chr7 | 93304000 | 93305000 | 0.000 | 0.000 | 0.000 | 0.029 | 0.000 | CALCR | 0.22755 | 0.54294 | 0.08726 | 0 | 0 |
| 587 | chr7 | 93682000 | 93683000 | 0.000 | 0.000 | 0.000 | 0.015 | 0.014 | BET1 | 1.00000 | 1.00000 | 0.29694 | 0 | 0 |
| 588 | chr7 | 102644000 | 102645000 | 0.028 | 0.000 | 0.000 | 0.000 | 0.014 | FBXL13 | 1.00000 | 1.00000 | 0.29694 | 0 | 0 |
| 589 | chr7 | 105699000 | 105700000 | 0.000 | 0.000 | 0.000 | 0.015 | 0.027 | CDHR3 | 1.00000 | 0.34615 | 1.00000 | 0 | 0 |
| 590 | chr7 | 110521000 | 110522000 | 0.000 | 0.000 | 0.000 | 0.029 | 0.000 | IMMP2L | 0.22755 | 0.54294 | 0.08726 | 0 | 0 |
| 591 | chr7 | 110543000 | 110544000 | 0.000 | 0.000 | 0.000 | 0.029 | 0.000 | IMMP2L | 0.22755 | 0.54294 | 0.08726 | 0 | 0 |
| 592 | chr7 | 110545000 | 110546000 | 0.000 | 0.000 | 0.000 | 0.015 | 0.014 | IMMP2L | 1.00000 | 1.00000 | 0.29694 | 0 | 0 |
| 593 | chr7 | 110597000 | 110598000 | 0.000 | 0.000 | 0.000 | 0.015 | 0.014 | IMMP2L | 1.00000 | 1.00000 | 0.29694 | 0 | 0 |
| 594 | chr7 | 110601000 | 110602000 | 0.000 | 0.000 | 0.000 | 0.029 | 0.000 | IMMP2L | 0.22755 | 0.54294 | 0.08726 | 0 | 0 |
| 595 | chr7 | 110602000 | 110603000 | 0.000 | 0.000 | 0.000 | 0.029 | 0.000 | IMMP2L | 0.22755 | 0.54294 | 0.08726 | 0 | 0 |
| 596 | chr7 | 110609000 | 110610000 | 0.028 | 0.000 | 0.000 | 0.029 | 0.000 | IMMP2L | 0.10727 | 0.54294 | 0.08726 | 0 | 0 |
| 597 | chr7 | 110617000 | 110618000 | 0.000 | 0.000 | 0.000 | 0.029 | 0.000 | IMMP2L | 0.22755 | 0.54966 | 0.02537 | 0 | 0 |
| 598 | chr7 | 110618000 | 110619000 | 0.000 | 0.000 | 0.000 | 0.029 | 0.000 | IMMP2L | 0.22755 | 0.54966 | 0.08726 | 0 | 0 |
| 599 | chr7 | 110619000 | 110620000 | 0.000 | 0.000 | 0.000 | 0.029 | 0.000 | IMMP2L | 0.10727 | 0.54966 | 0.02537 | 0 | 0 |
| 600 | chr7 | 110621000 | 110622000 | 0.000 | 0.000 | 0.000 | 0.029 | 0.000 | IMMP2L | 0.22755 | 0.54294 | 0.08726 | 0 | 0 |
| 601 | chr7 | 110628000 | 110629000 | 0.000 | 0.000 | 0.000 | 0.029 | 0.000 | IMMP2L | 0.22755 | 0.54294 | 0.08726 | 0 | 0 |
| 602 | chr7 | 110629000 | 110630000 | 0.027 | 0.000 | 0.000 | 0.015 | 0.027 | IMMP2L | 0.22755 | 0.54294 | 0.08726 | 0 | 0 |
| 603 | chr7 | 110631000 | 110632000 | 0.000 | 0.000 | 0.000 | 0.044 | 0.000 | IMMP2L | 1.00000 | 1.00000 | 0.29694 | 0 | 0 |
| 604 | chr7 | 110632000 | 110633000 | 0.000 | 0.000 | 0.000 | 0.044 | 0.000 | IMMP2L | 0.10727 | 0.54966 | 0.02537 | 0 | 0 |
| 605 | chr7 | 110633000 | 110634000 | 0.000 | 0.000 | 0.000 | 0.029 | 0.014 | IMMP2L | 0.60686 | 0.54294 | 0.08726 | 0 | 0 |

| | | | | | | | | | | | | | |
|---|---|---|---|---|---|---|---|---|---|---|---|---|---|
| 606 | chr7 | 110636000 | 110637000 | 0.000 | 0.000 | 0.029 | 0.014 | IMMP2L | 0.60686 | 0.54294 | 0.68726 | 0 | 0 |
| 607 | chr7 | 110637000 | 110638000 | 0.000 | 0.000 | 0.029 | 0.014 | IMMP2L | 0.60686 | 0.54294 | 0.08726 | 0 | 0 |
| 608 | chr7 | 110638000 | 110639000 | 0.000 | 0.000 | 0.029 | 0.027 | IMMP2L | 1.00000 | 0.54294 | 0.08726 | 0 | 0 |
| 609 | chr7 | 110639000 | 110644000 | 0.000 | 0.000 | 0.044 | 0.000 | IMMP2L | 0.10727 | 0.54966 | 0.02537 | 0 | 0 |
| 610 | chr7 | 110644000 | 110650000 | 0.000 | 0.000 | 0.029 | 0.000 | IMMP2L | 0.22755 | 0.54294 | 0.08726 | 0 | 0 |
| 611 | chr7 | 110650000 | 110651000 | 0.000 | 0.000 | 0.029 | 0.000 | IMMP2L | 0.60686 | 0.54294 | 0.08726 | 0 | 0 |
| 612 | chr7 | 110651000 | 110652000 | 0.000 | 0.000 | 0.029 | 0.014 | IMMP2L | 0.49735 | 0.54294 | 0.08726 | 0 | 0 |
| 613 | chr7 | 110666000 | 110667000 | 0.006 | 0.000 | 0.000 | 0.027 | IMMP2L | 0.22755 | 1.00000 | 1.00000 | 0 | 0 |
| 614 | chr7 | 110671000 | 110672000 | 0.000 | 0.000 | 0.029 | 0.000 | IMMP2L | 0.60686 | 0.54294 | 0.58726 | 0 | 0 |
| 615 | chr7 | 110677000 | 110678000 | 0.000 | 0.000 | 0.029 | 0.014 | IMMP2L | 0.22755 | 0.54294 | 0.08726 | 0 | 0 |
| 616 | chr7 | 110679000 | 110680000 | 0.000 | 0.000 | 0.029 | 0.000 | IMMP2L | 0.60686 | 0.54294 | 0.08726 | 0 | 0 |
| 617 | chr7 | 110680000 | 110681000 | 0.000 | 0.000 | 0.074 | 0.000 | IMMP2L | 0.02326 | 0.16101 | 0.00208 | 1 | 0 |
| 618 | chr7 | 110685000 | 110686000 | 0.000 | 0.000 | 0.029 | 0.000 | LRRN3 | 0.22755 | 0.54294 | 0.08726 | 0 | 0 |
| 619 | chr7 | 110686000 | 110688000 | 0.028 | 0.000 | 0.044 | 0.027 | LRRN3 | 0.67043 | 1.00000 | 0.02537 | 0 | 0 |
| 620 | chr7 | 110688000 | 110689000 | 0.000 | 0.000 | 0.029 | 0.000 | LRRN3 | 0.22755 | 0.54294 | 0.08726 | 0 | 0 |
| 621 | chr7 | 110699000 | 110700000 | 0.000 | 0.000 | 0.059 | 0.000 | LRRN3 | 0.05016 | 0.29551 | 0.00730 | 1 | 0 |
| 622 | chr7 | 110700000 | 110701000 | 0.000 | 0.000 | 0.029 | 0.000 | LRRN3 | 0.22755 | 0.54294 | 0.08726 | 0 | 0 |
| 623 | chr7 | 110709000 | 110710000 | 0.000 | 0.000 | 0.029 | 0.000 | LRRN3 | 0.22755 | 0.54294 | 0.08726 | 0 | 0 |
| 624 | chr7 | 110711000 | 110712000 | 0.000 | 0.000 | 0.044 | 0.000 | LRRN3 | 0.10727 | 0.54966 | 0.02537 | 0 | 0 |
| 625 | chr7 | 110714000 | 110715000 | 0.000 | 0.000 | 0.015 | 0.014 | LRRN3 | 1.00000 | 1.00000 | 0.29694 | 0 | 0 |
| 626 | chr7 | 110727000 | 110728000 | 0.000 | 0.000 | 0.029 | 0.000 | LRRN3 | 0.22755 | 0.54294 | 0.08726 | 0 | 0 |
| 627 | chr7 | 110728000 | 110729000 | 0.000 | 0.000 | 0.015 | 0.014 | LRRN3 | 1.00000 | 1.00000 | 0.29694 | 0 | 0 |
| 628 | chr7 | 110729000 | 110730000 | 0.000 | 0.000 | 0.029 | 0.014 | LRRN3 | 0.60686 | 0.54294 | 0.08726 | 0 | 0 |
| 629 | chr7 | 110734000 | 110735000 | 0.000 | 0.000 | 0.015 | 0.014 | LRRN3 | 1.00000 | 1.00000 | 0.29694 | 0 | 0 |
| 630 | chr7 | 110737000 | 110738000 | 0.000 | 0.000 | 0.015 | 0.027 | LRRN3 | 1.00000 | 1.00000 | 0.29694 | 0 | 0 |
| 631 | chr7 | 110740000 | 110741000 | 0.000 | 0.000 | 0.029 | 0.027 | LRRN3 | 1.00000 | 0.54294 | 0.08726 | 0 | 0 |
| 632 | chr7 | 110744000 | 110745000 | 0.000 | 0.000 | 0.029 | 0.000 | LRRN3 | 0.22755 | 0.54294 | 0.08726 | 0 | 0 |
| 633 | chr7 | 110746000 | 110747000 | 0.000 | 0.000 | 0.029 | 0.000 | LRRN3 | 0.60686 | 0.54294 | 0.08726 | 0 | 0 |
| 634 | chr7 | 110748000 | 110749000 | 0.000 | 0.000 | 0.029 | 0.000 | LRRN3 | 0.22755 | 0.54294 | 0.08726 | 0 | 0 |
| 635 | chr7 | 110755000 | 110756000 | 0.000 | 0.000 | 0.044 | 0.000 | LRRN3 | 0.22755 | 0.54294 | 0.08726 | 0 | 0 |
| 636 | chr7 | 110764000 | 110765000 | 0.000 | 0.000 | 0.029 | 0.000 | LRRN3 | 0.10727 | 0.54966 | 0.02537 | 0 | 0 |
| 637 | chr7 | 110767000 | 110768000 | 0.000 | 0.000 | 0.029 | 0.000 | LRRN3 | 0.22755 | 0.54294 | 0.08726 | 0 | 0 |
| 638 | chr7 | 110769000 | 110770000 | 0.000 | 0.000 | 0.029 | 0.014 | LRRN3 | 0.60686 | 0.54966 | 0.08726 | 0 | 0 |
| 639 | chr7 | 110769000 | 110773000 | 0.028 | 0.000 | 0.044 | 0.000 | LRRN3 | 0.10727 | 0.54294 | 0.02537 | 0 | 0 |
| 640 | chr7 | 110771000 | 110772000 | 0.000 | 0.000 | 0.029 | 0.014 | LRRN3 | 0.60686 | 0.54966 | 0.08726 | 0 | 0 |
| 641 | chr7 | 110779000 | 110780000 | 0.000 | 0.000 | 0.015 | 0.000 | LRRN3 | 1.00000 | 1.00000 | 0.29694 | 0 | 0 |
| 642 | chr7 | 110780000 | 110781000 | 0.000 | 0.000 | 0.029 | 0.000 | LRRN3 | 0.22755 | 0.54294 | 0.08726 | 0 | 0 |
| 643 | chr7 | 110783000 | 110784000 | 0.000 | 0.000 | 0.044 | 0.000 | LRRN3 | 0.10727 | 0.54966 | 0.02537 | 0 | 0 |
| 644 | chr7 | 110785000 | 110786000 | 0.000 | 0.000 | 0.029 | 0.000 | LRRN3 | 0.22755 | 0.54294 | 0.08726 | 0 | 0 |
| 645 | chr7 | 110801000 | 110802000 | 0.000 | 0.000 | 0.015 | 0.027 | LRRN3 | 1.00000 | 1.00000 | 0.29694 | 0 | 0 |
| 646 | chr7 | 110803000 | 110803000 | 0.000 | 0.000 | 0.029 | 0.000 | LRRN3 | 0.60686 | 0.54294 | 0.08726 | 0 | 0 |
| 647 | chr7 | 110810000 | 110811000 | 0.028 | 0.000 | 0.044 | 0.000 | LRRN3 | 0.10727 | 0.54966 | 0.02537 | 0 | 0 |
| 648 | chr7 | 110816000 | 110817000 | 0.000 | 0.000 | 0.029 | 0.000 | LRRN3 | 0.22755 | 0.54294 | 0.08726 | 0 | 0 |
| 649 | chr7 | 110821000 | 110822000 | 0.000 | 0.000 | 0.029 | 0.000 | LRRN3 | 1.00000 | 0.54294 | 0.08726 | 0 | 0 |
| 650 | chr7 | 110824000 | 110825000 | 0.000 | 0.000 | 0.029 | 0.014 | LRRN3 | 0.10727 | 1.00000 | 0.29694 | 0 | 0 |
| 651 | chr7 | 110827000 | 110828000 | 0.000 | 0.000 | 0.015 | 0.000 | LRRN3 | 0.22755 | 0.54966 | 0.02537 | 0 | 0 |
| 652 | chr7 | 110836000 | 110857000 | 0.000 | 0.000 | 0.044 | 0.000 | LRRN3 | 1.00000 | 0.54294 | 0.08726 | 0 | 0 |
| 653 | chr7 | 110847000 | 110848000 | 0.000 | 0.000 | 0.029 | 0.014 | LRRN3 | 1.00000 | 0.34615 | 1.00000 | 0 | 0 |
| 654 | chr7 | 115567000 | 115568000 | 0.028 | 0.000 | 0.000 | 0.000 | DOCK4 | 1.00000 | 1.00000 | 1.00000 | 0 | 0 |
| 655 | chr7 | 119056000 | 119057000 | 0.000 | 0.000 | 0.015 | 0.014 | KCND2 | 1.00000 | 1.00000 | 0.29694 | 0 | 0 |
| 656 | chr7 | 121358000 | 121381000 | 0.000 | 0.000 | 0.006 | 0.014 | PTPRZ1 | 1.00000 | 1.00000 | 0.50663 | 0 | 0 |

EP 4 334 476 B1

| 657 | chr7 | 123887000 | 123888000 | 0.000 | 0.000 | 0.029 | 0.000 | TMEM229A | 0.22755 | 0.54294 | 0.08726 | 0 | 0 |
| 658 | chr7 | 125262000 | 125263000 | 0.000 | 0.000 | 0.015 | 0.014 | POT1 | 1.00000 | 1.00000 | 0.29694 | 0 | 0 |
| 659 | chr7 | 145723000 | 145724000 | 0.000 | 0.000 | 0.029 | 0.000 | CNTNAP2 | 0.22755 | 0.54294 | 0.08726 | 0 | 0 |
| 660 | chr7 | 148508000 | 148509000 | 0.000 | 0.000 | 0.000 | 0.041 | EZH2 | 0.24603 | 1.00000 | 1.00000 | 0 | 0 |
| 661 | chr7 | 155127000 | 155128000 | 0.000 | 0.000 | 0.000 | 0.027 | BLACE | 0.49735 | 1.00000 | 1.00000 | 0 | 0 |
| 662 | chr7 | 157162000 | 157163000 | 0.056 | 0.000 | 0.000 | 0.000 | DNAJB6 | 1.00000 | 0.11763 | 1.00000 | 0 | 1 |
| 663 | chr7 | 158684000 | 158685000 | 0.000 | 0.000 | 0.015 | 0.014 | WDR60 | 1.00000 | 1.00000 | 0.29694 | 0 | 0 |
| 664 | chr8 | 1646000 | 1647000 | 0.000 | 0.000 | 0.015 | 0.027 | DLGAP2 | 1.00000 | 1.00000 | 0.29694 | 0 | 0 |
| 665 | chr8 | 5558000 | 5559000 | 0.000 | 0.000 | 0.029 | 0.000 | MCPH1 | 0.22755 | 0.54294 | 0.08726 | 0 | 0 |
| 666 | chr8 | 5612000 | 5613000 | 0.000 | 0.000 | 0.029 | 0.027 | MCPH1 | 0.49735 | 1.00000 | 1.00000 | 0 | 0 |
| 667 | chr8 | 8602000 | 8603000 | 0.000 | 0.000 | 0.029 | 0.014 | MFHAS1 | 0.60686 | 0.54294 | 0.08726 | 0 | 0 |
| 668 | chr8 | 8706000 | 8707000 | 0.000 | 0.000 | 0.029 | 0.000 | MFHAS1 | 0.22755 | 0.54294 | 0.08726 | 0 | 0 |
| 669 | chr8 | 8717000 | 8718000 | 0.000 | 0.000 | 0.029 | 0.000 | MFHAS1 | 0.22755 | 0.54294 | 0.08726 | 0 | 0 |
| 670 | chr8 | 11352000 | 11353000 | 0.000 | 0.000 | 0.029 | 0.014 | BLK | 0.60686 | 0.54294 | 0.08726 | 0 | 0 |
| 671 | chr8 | 14080000 | 14081000 | 0.000 | 0.000 | 0.015 | 0.014 | SGCZ | 1.00000 | 1.00000 | 0.29694 | 0 | 0 |
| 672 | chr8 | 14796000 | 14797000 | 0.000 | 0.006 | 0.015 | 0.000 | SGCZ | 0.47887 | 1.00000 | 0.50663 | 0 | 0 |
| 673 | chr8 | 16090000 | 16091000 | 0.000 | 0.000 | 0.015 | 0.014 | MSR1 | 1.00000 | 1.00000 | 0.29694 | 0 | 0 |
| 674 | chr8 | 16187000 | 16188000 | 0.028 | 0.000 | 0.015 | 0.000 | MSR1 | 0.47887 | 1.00000 | 0.29694 | 0 | 0 |
| 675 | chr8 | 23101000 | 23102000 | 0.000 | 0.000 | 0.015 | 0.014 | CHMP7 | 1.00000 | 1.00000 | 0.29694 | 0 | 0 |
| 676 | chr8 | 24207000 | 24208000 | 0.000 | 0.000 | 0.029 | 0.000 | ADAM28 | 0.22755 | 0.54294 | 0.08726 | 0 | 0 |
| 677 | chr8 | 29155000 | 29156000 | 0.028 | 0.000 | 0.000 | 0.014 | KIF13B | 1.00000 | 0.34615 | 1.00000 | 0 | 0 |
| 678 | chr8 | 35657000 | 35658000 | 0.000 | 0.000 | 0.029 | 0.000 | AC012215.1 | 0.22755 | 0.54294 | 0.08726 | 0 | 0 |
| 679 | chr8 | 38759000 | 38760000 | 0.000 | 0.000 | 0.029 | 0.000 | PLEKHA2 | 0.22755 | 0.54294 | 0.08726 | 0 | 0 |
| 680 | chr8 | 54986000 | 54987000 | 0.000 | 0.000 | 0.029 | 0.000 | LYPLA1 | 0.22755 | 0.54294 | 0.08726 | 0 | 0 |
| 681 | chr8 | 60031000 | 60032000 | 0.000 | 0.000 | 0.015 | 0.014 | TOX | 1.00000 | 1.00000 | 0.29694 | 0 | 0 |
| 682 | chr8 | 67525000 | 67526000 | 0.000 | 0.000 | 0.015 | 0.014 | MYBL1 | 1.00000 | 1.00000 | 0.29694 | 0 | 0 |
| 683 | chr8 | 77105000 | 77106000 | 0.000 | 0.000 | 0.029 | 0.000 | ZFHX4 | 0.22755 | 0.54294 | 0.08726 | 0 | 0 |
| 684 | chr8 | 78400000 | 78401000 | 0.000 | 0.000 | 0.029 | 0.000 | PEX2 | 0.22755 | 0.54294 | 0.08726 | 0 | 0 |
| 685 | chr8 | 90322000 | 90323000 | 0.000 | 0.000 | 0.029 | 0.000 | RIPK2 | 0.22755 | 0.54294 | 0.08726 | 0 | 0 |
| 686 | chr8 | 93199000 | 93200000 | 0.000 | 0.000 | 0.029 | 0.000 | RUNX1T1 | 0.22755 | 0.54294 | 0.08726 | 0 | 0 |
| 687 | chr8 | 94618000 | 94619000 | 0.028 | 0.000 | 0.015 | 0.000 | FAM92A1 | 0.47887 | 1.00000 | 0.29694 | 0 | 0 |
| 688 | chr8 | 110586000 | 110587000 | 0.000 | 0.000 | 0.015 | 0.014 | SYBU | 1.00000 | 1.00000 | 0.29694 | 0 | 0 |
| 689 | chr8 | 126687000 | 126688000 | 0.028 | 0.000 | 0.015 | 0.014 | TRIB1 | 1.00000 | 1.00000 | 0.29694 | 0 | 0 |
| 690 | chr8 | 128748000 | 128749000 | 0.500 | 0.000 | 0.132 | 0.000 | MYC | 0.00099 | 0.00010 | 0.00001 | 1 | 1 |
| 691 | chr8 | 128749000 | 128750000 | 0.583 | 0.000 | 0.103 | 0.014 | MYC | 0.02808 | 0.00000 | 0.00016 | 1 | 1 |
| 692 | chr8 | 128750000 | 128751000 | 0.444 | 0.000 | 0.088 | 0.014 | MYC | 0.05468 | 0.00007 | 0.00058 | 1 | 1 |
| 693 | chr8 | 128751000 | 128752000 | 0.111 | 0.000 | 0.044 | 0.000 | MYC | 0.10727 | 0.23165 | 0.02537 | 1 | 1 |
| 694 | chr8 | 128752000 | 128753000 | 0.056 | 0.000 | 0.015 | 0.000 | MYC | 0.47887 | 0.27446 | 0.29694 | 1 | 1 |
| 695 | chr8 | 137918000 | 137919000 | 0.028 | 0.000 | 0.015 | 0.000 | FAM135B | 0.47887 | 1.00000 | 0.29694 | 0 | 0 |
| 696 | chr8 | 138274000 | 138275000 | 0.000 | 0.000 | 0.000 | 0.027 | FAM135B | 0.49735 | 1.00000 | 1.00000 | 0 | 0 |
| 697 | chr8 | 143183000 | 143184000 | 0.028 | 0.000 | 0.015 | 0.000 | TSNARE1 | 0.47887 | 1.00000 | 0.29694 | 0 | 0 |
| 698 | chr8 | 144123000 | 144124000 | 0.000 | 0.000 | 0.029 | 0.000 | C8orf31 | 0.22755 | 0.54294 | 0.08726 | 0 | 0 |
| 699 | chr9 | 6411000 | 6412000 | 0.000 | 0.000 | 0.029 | 0.000 | UHRF2 | 0.22755 | 0.54294 | 0.08726 | 0 | 0 |
| 700 | chr9 | 6413000 | 6414000 | 0.000 | 0.000 | 0.015 | 0.014 | UHRF2 | 1.00000 | 1.00000 | 0.29694 | 0 | 0 |
| 701 | chr9 | 6414000 | 6415000 | 0.000 | 0.000 | 0.029 | 0.014 | UHRF2 | 0.60686 | 0.54294 | 0.08726 | 0 | 0 |
| 702 | chr9 | 9928000 | 9929000 | 0.000 | 0.000 | 0.000 | 0.027 | PTPRD | 0.49735 | 1.00000 | 1.00000 | 0 | 0 |
| 703 | chr9 | 13965000 | 13966000 | 0.000 | 0.000 | 0.029 | 0.000 | NFIB | 0.22755 | 0.54294 | 0.08726 | 0 | 0 |
| 704 | chr9 | 22824000 | 22825000 | 0.000 | 0.000 | 0.029 | 0.000 | DMRTA1 | 0.22755 | 0.54294 | 0.08726 | 0 | 0 |
| 705 | chr9 | 25260000 | 25261000 | 0.000 | 0.000 | 0.029 | 0.000 | TUSC1 | 0.22755 | 0.54294 | 0.08726 | 0 | 0 |
| 706 | chr9 | 29890000 | 29891000 | 0.000 | 0.000 | 0.015 | 0.014 | LINGO2 | 1.00000 | 1.00000 | 0.29694 | 0 | 0 |
| 707 | chr9 | 30656000 | 30657000 | 0.000 | 0.000 | 0.015 | 0.014 | ACO1 | 1.00000 | 1.00000 | 0.29694 | 0 | 0 |

115

| | | | | | | | | | | | | | |
|---|---|---|---|---|---|---|---|---|---|---|---|---|---|
| 708 | chr9 | 37003000 | 37004000 | 0.000 | 0.006 | 0.015 | 0.000 | PAX5 | 0.47887 | 1.00000 | 0.50663 | 1 | 0 |
| 709 | chr9 | 37005000 | 37006000 | 0.000 | 0.000 | 0.015 | 0.014 | PAX5 | 1.00000 | 1.00000 | 0.29694 | 1 | 0 |
| 710 | chr9 | 37024000 | 37025000 | 0.000 | 0.000 | 0.044 | 0.027 | PAX5 | 0.67043 | 0.54966 | 0.02537 | 1 | 0 |
| 711 | chr9 | 37025000 | 37026000 | 0.000 | 0.000 | 0.132 | 0.054 | PAX5 | 0.14640 | 0.02564 | 0.00001 | 1 | 1 |
| 712 | chr9 | 37026000 | 37027000 | 0.000 | 0.006 | 0.221 | 0.108 | PAX5 | 0.10913 | 0.00107 | 0.00000 | 1 | 1 |
| 713 | chr9 | 37027000 | 37028000 | 0.000 | 0.000 | 0.029 | 0.014 | PAX5 | 0.60686 | 0.54294 | 0.08726 | 1 | 0 |
| 714 | chr9 | 37033000 | 37034000 | 0.000 | 0.000 | 0.044 | 0.014 | PAX5 | 0.34948 | 0.54966 | 0.02537 | 1 | 0 |
| 715 | chr9 | 37034000 | 37035000 | 0.000 | 0.000 | 0.074 | 0.041 | PAX5 | 0.47996 | 0.16101 | 0.00208 | 1 | 1 |
| 716 | chr9 | 37035000 | 37036000 | 0.000 | 0.000 | 0.015 | 0.014 | PAX5 | 1.00000 | 1.00000 | 0.29694 | 1 | 0 |
| 717 | chr9 | 37196000 | 37197000 | 0.000 | 0.000 | 0.029 | 0.014 | ZCCHC7 | 0.60686 | 0.54294 | 0.08726 | 0 | 0 |
| 718 | chr9 | 37197000 | 37198000 | 0.000 | 0.000 | 0.029 | 0.000 | ZCCHC7 | 0.22755 | 0.54294 | 0.08726 | 0 | 0 |
| 719 | chr9 | 37293000 | 37294000 | 0.000 | 0.000 | 0.029 | 0.027 | ZCCHC7 | 1.00000 | 0.54294 | 0.08726 | 0 | 0 |
| 720 | chr9 | 37294000 | 37295000 | 0.000 | 0.000 | 0.044 | 0.027 | ZCCHC7 | 0.67043 | 0.54966 | 0.02537 | 0 | 0 |
| 721 | chr9 | 37327000 | 37328000 | 0.000 | 0.000 | 0.015 | 0.014 | ZCCHC7 | 1.00000 | 1.00000 | 0.29694 | 0 | 0 |
| 722 | chr9 | 37336000 | 37337000 | 0.000 | 0.000 | 0.044 | 0.014 | ZCCHC7 | 0.34948 | 0.54966 | 0.02537 | 0 | 0 |
| 723 | chr9 | 37337000 | 37338000 | 0.000 | 0.012 | 0.015 | 0.041 | ZCCHC7 | 0.62100 | 1.00000 | 1.00000 | 0 | 0 |
| 724 | chr9 | 37338000 | 37339000 | 0.000 | 0.000 | 0.029 | 0.014 | ZCCHC7 | 0.60686 | 0.54294 | 0.08726 | 0 | 0 |
| 725 | chr9 | 37369000 | 37370000 | 0.000 | 0.000 | 0.029 | 0.000 | ZCCHC7 | 0.22755 | 0.54294 | 0.08726 | 0 | 0 |
| 726 | chr9 | 37371000 | 37372000 | 0.028 | 0.025 | 0.118 | 0.068 | ZCCHC7 | 0.38669 | 0.15803 | 0.00732 | 0 | 1 |
| 727 | chr9 | 37372000 | 37373000 | 0.000 | 0.000 | 0.015 | 0.014 | ZCCHC7 | 1.00000 | 1.00000 | 0.29694 | 0 | 0 |
| 728 | chr9 | 37383000 | 37384000 | 0.000 | 0.000 | 0.059 | 0.027 | ZCCHC7 | 0.42627 | 0.29551 | 0.00730 | 0 | 1 |
| 729 | chr9 | 37384000 | 37385000 | 0.000 | 0.000 | 0.059 | 0.054 | ZCCHC7 | 1.00000 | 0.29551 | 0.00730 | 0 | 1 |
| 730 | chr9 | 37385000 | 37386000 | 0.000 | 0.000 | 0.029 | 0.014 | ZCCHC7 | 0.60686 | 0.54294 | 0.08726 | 0 | 0 |
| 731 | chr9 | 37387000 | 37388000 | 0.000 | 0.000 | 0.059 | 0.014 | ZCCHC7 | 0.19371 | 0.29551 | 0.00730 | 0 | 1 |
| 732 | chr9 | 37397000 | 37398000 | 0.000 | 0.000 | 0.044 | 0.000 | GRHPR | 0.10727 | 0.54966 | 0.02537 | 0 | 0 |
| 733 | chr9 | 37398000 | 37399000 | 0.000 | 0.000 | 0.029 | 0.000 | GRHPR | 0.22755 | 0.54294 | 0.08726 | 0 | 0 |
| 734 | chr9 | 37399000 | 37400000 | 0.000 | 0.000 | 0.029 | 0.000 | GRHPR | 0.22755 | 0.54294 | 0.08726 | 0 | 0 |
| 735 | chr9 | 37402000 | 37403000 | 0.000 | 0.006 | 0.029 | 0.000 | GRHPR | 0.22755 | 0.54294 | 0.21104 | 0 | 0 |
| 736 | chr9 | 37406000 | 37407000 | 0.000 | 0.000 | 0.015 | 0.014 | GRHPR | 1.00000 | 1.00000 | 0.29694 | 0 | 0 |
| 737 | chr9 | 37407000 | 37408000 | 0.000 | 0.000 | 0.132 | 0.149 | GRHPR | 0.81382 | 0.02564 | 0.00001 | 0 | 1 |
| 738 | chr9 | 37408000 | 37409000 | 0.000 | 0.006 | 0.029 | 0.027 | GRHPR | 1.00000 | 0.54294 | 0.21104 | 0 | 0 |
| 739 | chr9 | 37410000 | 37411000 | 0.000 | 0.000 | 0.029 | 0.000 | GRHPR | 0.22755 | 0.54294 | 0.08726 | 0 | 0 |
| 740 | chr9 | 37424000 | 37425000 | 0.000 | 0.000 | 0.044 | 0.000 | GRHPR | 0.10727 | 0.54966 | 0.02537 | 0 | 0 |
| 741 | chr9 | 37425000 | 37426000 | 0.000 | 0.000 | 0.029 | 0.000 | GRHPR | 0.22755 | 0.54294 | 0.08726 | 0 | 0 |
| 742 | chr9 | 112811000 | 112812000 | 0.000 | 0.000 | 0.059 | 0.014 | AKAP2 | 0.19371 | 0.29551 | 0.00730 | 0 | 1 |
| 743 | chr9 | 117037000 | 117038000 | 0.056 | 0.000 | 0.000 | 0.014 | COL27A1 | 1.00000 | 0.11763 | 1.00000 | 0 | 1 |
| 744 | chr9 | 119779000 | 119780000 | 0.000 | 0.000 | 0.044 | 0.000 | ASTN2 | 0.10727 | 0.54966 | 0.02537 | 0 | 0 |
| 745 | chr9 | 126232000 | 126233000 | 0.056 | 0.000 | 0.000 | 0.000 | DENND1A | 1.00000 | 0.11763 | 1.00000 | 0 | 1 |
| 746 | chr9 | 130741000 | 130742000 | 0.000 | 0.000 | 0.059 | 0.000 | FAM102A | 0.05016 | 0.29551 | 0.00730 | 1 | 1 |
| 747 | chr9 | 130742000 | 130743000 | 0.000 | 0.000 | 0.059 | 0.027 | FAM102A | 0.42627 | 0.29551 | 0.00730 | 1 | 1 |
| 748 | chr9 | 132767000 | 132768000 | 0.000 | 0.000 | 0.015 | 0.014 | FNBP1 | 1.00000 | 1.00000 | 0.29694 | 0 | 0 |
| 749 | chr9 | 132785000 | 132786000 | 0.000 | 0.000 | 0.029 | 0.000 | FNBP1 | 0.22755 | 0.54294 | 0.08726 | 0 | 0 |
| 750 | chr9 | 132803000 | 132804000 | 0.000 | 0.000 | 0.015 | 0.014 | FNBP1 | 1.00000 | 1.00000 | 0.29694 | 0 | 0 |
| 751 | chr9 | 132804000 | 132805000 | 0.000 | 0.000 | 0.029 | 0.027 | FNBP1 | 1.00000 | 0.54294 | 0.08726 | 0 | 0 |
| 752 | chr9 | 134551000 | 134552000 | 0.000 | 0.000 | 0.029 | 0.000 | RAPGEF1 | 0.22755 | 0.54294 | 0.08726 | 0 | 0 |
| 753 | chr9 | 138874000 | 138875000 | 0.056 | 0.000 | 0.029 | 0.014 | UBAC1 | 0.60686 | 0.60763 | 0.08726 | 0 | 1 |
| 754 | chr10 | 3333000 | 3334000 | 0.000 | 0.000 | 0.000 | 0.027 | PITRM1 | 0.49735 | 1.00000 | 1.00000 | 0 | 0 |
| 755 | chr10 | 5707000 | 5708000 | 0.000 | 0.000 | 0.029 | 0.014 | ASB13 | 0.60686 | 0.54294 | 0.08726 | 0 | 0 |
| 756 | chr10 | 5728000 | 5729000 | 0.000 | 0.006 | 0.015 | 0.000 | ASB13 | 0.47887 | 1.00000 | 0.50663 | 0 | 0 |
| 757 | chr10 | 15393000 | 15394000 | 0.028 | 0.000 | 0.015 | 0.000 | FAM171A1 | 0.47887 | 1.00000 | 0.29694 | 0 | 0 |
| 758 | chr10 | 20796000 | 20797000 | 0.000 | 0.006 | 0.015 | 0.000 | PLXDC2 | 0.47887 | 1.00000 | 0.50663 | 0 | 0 |

| # | chr | start | end | | | | | gene | | | | | |
|---|---|---|---|---|---|---|---|---|---|---|---|---|---|
| 759 | chr10 | 35424000 | 35425000 | 0.000 | 0.000 | 0.029 | 0.000 | CREM | 0.22755 | 0.54294 | 0.68726 | 0 | 0 |
| 760 | chr10 | 56678000 | 56679000 | 0.000 | 0.000 | 0.000 | 0.027 | PCDH15 | 0.49735 | 1.00000 | 1.00000 | 0 | 0 |
| 761 | chr10 | 63440000 | 63441000 | 0.023 | 0.000 | 0.015 | 0.000 | C10orf107 | 0.47887 | 1.00000 | 0.29694 | 0 | 0 |
| 762 | chr10 | 63659000 | 63660000 | 0.000 | 0.000 | 0.044 | 0.014 | ARID5B | 0.34948 | 0.54966 | 0.02537 | 1 | 1 |
| 763 | chr10 | 63661000 | 63662000 | 0.000 | 0.000 | 0.059 | 0.014 | ARID5B | 0.19371 | 0.29551 | 0.00730 | 1 | 1 |
| 764 | chr10 | 63663000 | 63663000 | 0.000 | 0.000 | 0.029 | 0.000 | ARID5B | 0.60686 | 0.54294 | 0.08726 | 1 | 1 |
| 765 | chr10 | 63720000 | 63721000 | 0.000 | 0.000 | 0.029 | 0.027 | ARID5B | 0.22755 | 0.54294 | 0.08726 | 1 | 1 |
| 766 | chr10 | 63804000 | 63804000 | 0.000 | 0.000 | 0.000 | 0.014 | ARID5B | 0.49735 | 1.00000 | 1.00000 | 1 | 1 |
| 767 | chr10 | 63809000 | 63810000 | 0.000 | 0.000 | 0.015 | 0.027 | ARID5B | 1.00000 | 1.00000 | 0.29694 | 1 | 1 |
| 768 | chr10 | 63810000 | 63811000 | 0.000 | 0.000 | 0.000 | 0.000 | ARID5B | 0.49735 | 1.00000 | 1.00000 | 1 | 1 |
| 769 | chr10 | 67907000 | 67908000 | 0.000 | 0.000 | 0.015 | 0.027 | CTNNA3 | 0.47887 | 1.00000 | 0.50663 | 0 | 0 |
| 770 | chr10 | 68474000 | 68475000 | 0.000 | 0.000 | 0.000 | 0.000 | CTNNA3 | 0.49735 | 1.00000 | 0.08726 | 0 | 0 |
| 771 | chr10 | 98510000 | 98510000 | 0.028 | 0.000 | 0.029 | 0.000 | PIK3AP1 | 0.22755 | 0.54294 | 0.08726 | 0 | 0 |
| 772 | chr10 | 101384000 | 101385000 | 0.000 | 0.000 | 0.015 | 0.014 | SLC25A28 | 1.00000 | 1.00000 | 0.29694 | 0 | 0 |
| 773 | chr10 | 108276000 | 108277000 | 0.000 | 0.000 | 0.029 | 0.000 | SORCS1 | 0.22755 | 0.54294 | 0.08726 | 0 | 0 |
| 774 | chr10 | 113473000 | 113474000 | 0.028 | 0.000 | 0.015 | 0.000 | GPAM | 0.47887 | 1.00000 | 0.29694 | 0 | 0 |
| 775 | chr10 | 113636000 | 113637000 | 0.000 | 0.000 | 0.029 | 0.000 | GPAM | 0.22755 | 0.54294 | 0.08726 | 0 | 0 |
| 776 | chr10 | 116458000 | 116459000 | 0.000 | 0.000 | 0.044 | 0.000 | ABLIM1 | 0.10727 | 0.54966 | 0.02537 | 0 | 0 |
| 777 | chr10 | 121623000 | 121624000 | 0.000 | 0.000 | 0.029 | 0.027 | MCMBP | 0.22755 | 0.54294 | 0.08726 | 0 | 0 |
| 778 | chr10 | 132973000 | 132974000 | 0.028 | 0.000 | 0.015 | 0.000 | TCERG1L | 1.00000 | 1.00000 | 0.29694 | 0 | 0 |
| 779 | chr10 | 134326000 | 134326000 | 0.028 | 0.000 | 0.015 | 0.000 | IAPP5A | 0.47887 | 1.00000 | 0.29694 | 0 | 0 |
| 780 | chr11 | 871000 | 872000 | 0.028 | 0.000 | 0.029 | 0.000 | CHID1 | 0.22755 | 1.00000 | 0.08726 | 0 | 0 |
| 781 | chr11 | 1149000 | 1150000 | 0.028 | 0.000 | 0.015 | 0.000 | MUC5AC | 0.47887 | 1.00000 | 0.29694 | 0 | 0 |
| 782 | chr11 | 25065000 | 25066000 | 0.000 | 0.000 | 0.029 | 0.000 | LUZP2 | 0.22755 | 0.54294 | 0.08726 | 0 | 0 |
| 783 | chr11 | 25289000 | 25290000 | 0.000 | 0.000 | 0.029 | 0.000 | LUZP2 | 0.22755 | 0.54294 | 0.08726 | 0 | 0 |
| 784 | chr11 | 27216000 | 27217000 | 0.023 | 0.000 | 0.022 | 0.014 | BBOX1 | 0.60686 | 1.00000 | 0.08726 | 0 | 0 |
| 785 | chr11 | 28849000 | 28850000 | 0.000 | 0.000 | 0.000 | 0.027 | METTL15 | 0.49735 | 1.00000 | 1.00000 | 0 | 0 |
| 786 | chr11 | 29253000 | 29254000 | 0.000 | 0.000 | 0.029 | 0.000 | KCNA4 | 0.22755 | 0.54294 | 0.08726 | 0 | 0 |
| 787 | chr11 | 29900000 | 29901000 | 0.000 | 0.000 | 0.029 | 0.000 | KCNA4 | 0.22755 | 0.54294 | 0.08726 | 0 | 0 |
| 788 | chr11 | 40626000 | 40627000 | 0.000 | 0.000 | 0.029 | 0.000 | LRRC4C | 0.22755 | 0.54294 | 0.08726 | 0 | 0 |
| 789 | chr11 | 40845000 | 40846000 | 0.000 | 0.000 | 0.029 | 0.000 | LRRC4C | 0.22755 | 0.54294 | 0.08726 | 0 | 0 |
| 790 | chr11 | 40868000 | 40869000 | 0.000 | 0.000 | 0.029 | 0.000 | LRRC4C | 0.22755 | 0.54294 | 0.08726 | 0 | 0 |
| 791 | chr11 | 41066000 | 41067000 | 0.028 | 0.000 | 0.015 | 0.000 | LRRC4C | 0.22755 | 0.54294 | 0.08726 | 0 | 0 |
| 792 | chr11 | 41844000 | 41845000 | 0.028 | 0.000 | 0.029 | 0.014 | API5 | 0.47887 | 1.00000 | 1.00000 | 0 | 0 |
| 793 | chr11 | 57171000 | 57172000 | 0.000 | 0.000 | 0.029 | 0.000 | SLC43A3 | 0.60686 | 0.54294 | 1.00000 | 0 | 0 |
| 794 | chr11 | 60224000 | 60225000 | 0.000 | 0.000 | 0.074 | 0.027 | MS4A1 | 0.10420 | 0.16101 | 0.00208 | 1 | 1 |
| 795 | chr11 | 65190000 | 65191000 | 0.000 | 0.000 | 0.074 | 0.014 | FRMD8 | 0.25970 | 0.16101 | 0.00208 | 0 | 0 |
| 796 | chr11 | 65191000 | 65192000 | 0.000 | 0.000 | 0.103 | 0.014 | FRMD8 | 0.62808 | 0.09269 | 0.00016 | 0 | 0 |
| 797 | chr11 | 65266000 | 65267000 | 0.000 | 0.000 | 0.029 | 0.014 | SCYL1 | 0.60686 | 0.54294 | 0.08726 | 0 | 0 |
| 798 | chr11 | 65267000 | 65268000 | 0.000 | 0.000 | 0.103 | 0.000 | SCYL1 | 0.60488 | 0.09269 | 0.00016 | 0 | 0 |
| 799 | chr11 | 85963000 | 85964000 | 0.000 | 0.000 | 0.029 | 0.000 | EED | 0.22755 | 0.54294 | 0.08726 | 0 | 0 |
| 800 | chr11 | 92261000 | 92262000 | 0.028 | 0.000 | 0.029 | 0.000 | FAT3 | 0.49735 | 0.54294 | 0.08726 | 0 | 0 |
| 801 | chr11 | 102117000 | 102118000 | 0.000 | 0.000 | 0.000 | 0.027 | YAP1 | 0.16270 | 1.00000 | 1.00000 | 1 | 1 |
| 802 | chr11 | 102188000 | 102189000 | 0.000 | 0.000 | 0.206 | 0.108 | BIRC3 | 0.05016 | 0.00197 | 0.00208 | 0 | 0 |
| 803 | chr11 | 102189000 | 102190000 | 0.000 | 0.012 | 0.059 | 0.000 | BIRC3 | 0.29551 | 0.29551 | 0.00730 | 1 | 1 |
| 804 | chr11 | 107497000 | 107498000 | 0.028 | 0.000 | 0.015 | 0.014 | ELMOD1 | 0.47887 | 1.00000 | 1.00000 | 0 | 0 |
| 805 | chr11 | 108781000 | 108782000 | 0.000 | 0.000 | 0.015 | 0.014 | DDX10 | 1.00000 | 1.00000 | 0.29694 | 1 | 1 |
| 806 | chr11 | 108975000 | 108976000 | 0.000 | 0.000 | 0.015 | 0.000 | DDX10 | 1.00000 | 1.00000 | 0.29694 | 0 | 0 |
| 807 | chr11 | 109066000 | 109067000 | 0.028 | 0.000 | 0.029 | 0.014 | C11orf87 | 0.47887 | 1.00000 | 0.29694 | 1 | 1 |
| 808 | chr11 | 111248000 | 111249000 | 0.000 | 0.000 | 0.039 | 0.014 | POU2AF1 | 0.60686 | 0.54294 | 0.08726 | 1 | 1 |
| 809 | chr11 | 111249000 | 111250000 | 0.000 | 0.012 | 0.103 | 0.081 | POU2AF1 | 0.77363 | 0.09269 | 0.00337 | 1 | 1 |

| # | chr | start | end | | | | | gene | | | | | |
|---|---|---|---|---|---|---|---|---|---|---|---|---|---|
| 810 | chr1 | 115761000 | 115762000 | 0.028 | 0.000 | 0.015 | 0.041 | CADM1 | 0.62100 | 1.00000 | 0.29694 | 0 | 0 |
| 811 | chr1 | 118723000 | 118724000 | 0.000 | 0.000 | 0.029 | 0.000 | CXCR5 | 0.22755 | 0.54294 | 0.08726 | 0 | 0 |
| 812 | chr1 | 126496000 | 126497000 | 0.023 | 0.000 | 0.015 | 0.014 | KIRREL3 | 1.00000 | 1.00000 | 0.29694 | 1 | 0 |
| 813 | chr1 | 128390000 | 128391000 | 0.000 | 0.000 | 0.044 | 0.014 | ETS1 | 0.34948 | 0.54966 | 0.02537 | 1 | 0 |
| 814 | chr1 | 128391000 | 128392000 | 0.000 | 0.000 | 0.118 | 0.014 | ETS1 | 0.61415 | 0.04825 | 0.00004 | 1 | 1 |
| 815 | chr12 | 6554000 | 6555000 | 0.000 | 0.000 | 0.029 | 0.000 | CD27 | 0.22755 | 0.54294 | 0.08726 | 0 | 0 |
| 816 | chr12 | 8762000 | 8763000 | 0.000 | 0.000 | 0.015 | 0.014 | AICDA | 1.00000 | 1.00000 | 0.29694 | 0 | 0 |
| 817 | chr12 | 8763000 | 8764000 | 0.000 | 0.000 | 0.044 | 0.041 | AICDA | 1.00000 | 0.54966 | 0.02537 | 0 | 1 |
| 818 | chr12 | 8764000 | 8765000 | 0.000 | 0.000 | 0.029 | 0.068 | AICDA | 0.44431 | 0.54294 | 0.08726 | 0 | 0 |
| 819 | chr12 | 8765000 | 8766000 | 0.000 | 0.000 | 0.015 | 0.027 | AICDA | 1.00000 | 1.00000 | 0.29694 | 1 | 0 |
| 820 | chr12 | 9823000 | 9824000 | 0.000 | 0.000 | 0.015 | 0.014 | CLEC2D | 1.00000 | 1.00000 | 0.50663 | 0 | 0 |
| 821 | chr12 | 11710000 | 11711000 | 0.000 | 0.000 | 0.029 | 0.000 | ETV6 | 0.22755 | 0.54294 | 0.08726 | 0 | 0 |
| 822 | chr12 | 11803000 | 11804000 | 0.000 | 0.000 | 0.015 | 0.014 | ETV6 | 1.00000 | 1.00000 | 0.29694 | 1 | 0 |
| 823 | chr12 | 14923000 | 14924000 | 0.000 | 0.000 | 0.015 | 0.014 | HIST4H4 | 1.00000 | 1.00000 | 0.29694 | 1 | 0 |
| 824 | chr12 | 16717000 | 16718000 | 0.000 | 0.006 | 0.000 | 0.027 | LMO3 | 0.49735 | 1.00000 | 1.00000 | 0 | 0 |
| 825 | chr12 | 23805000 | 23806000 | 0.000 | 0.006 | 0.029 | 0.000 | SOX5 | 0.22755 | 0.54294 | 0.08726 | 0 | 0 |
| 826 | chr12 | 25149000 | 25150000 | 0.000 | 0.000 | 0.029 | 0.000 | C12orf77 | 0.22755 | 0.54294 | 0.08726 | 0 | 0 |
| 827 | chr12 | 25153000 | 25154000 | 0.000 | 0.000 | 0.015 | 0.014 | C12orf77 | 1.00000 | 1.00000 | 0.29694 | 0 | 0 |
| 828 | chr12 | 25174000 | 25175000 | 0.000 | 0.000 | 0.044 | 0.000 | C12orf77 | 0.10727 | 0.54966 | 0.02537 | 0 | 0 |
| 829 | chr12 | 25206000 | 25207000 | 0.000 | 0.006 | 0.015 | 0.000 | LRMP | 0.47887 | 1.00000 | 0.50663 | 0 | 0 |
| 830 | chr12 | 25206000 | 25207000 | 0.000 | 0.006 | 0.103 | 0.014 | LRMP | 0.02808 | 0.09269 | 0.00099 | 0 | 0 |
| 831 | chr12 | 25207000 | 25208000 | 0.000 | 0.006 | 0.118 | 0.014 | LRMP | 0.61415 | 0.04825 | 0.00030 | 1 | 0 |
| 832 | chr12 | 25208000 | 25209000 | 0.000 | 0.000 | 0.029 | 0.014 | LRMP | 0.60686 | 0.54294 | 0.08726 | 0 | 0 |
| 833 | chr12 | 25665000 | 25666000 | 0.028 | 0.000 | 0.015 | 0.000 | IFLTD1 | 0.47887 | 1.00000 | 0.29694 | 0 | 0 |
| 834 | chr12 | 38920000 | 38921000 | 0.000 | 0.000 | 0.029 | 0.000 | CPNE8 | 0.22755 | 0.54294 | 0.08726 | 0 | 0 |
| 835 | chr12 | 48027000 | 48028000 | 0.028 | 0.000 | 0.059 | 0.027 | RPAP3 | 0.42627 | 0.65667 | 0.00730 | 0 | 0 |
| 836 | chr12 | 57496000 | 57497000 | 0.000 | 0.000 | 0.015 | 0.014 | STAT6 | 1.00000 | 1.00000 | 0.29694 | 0 | 0 |
| 837 | chr12 | 69203000 | 69204000 | 0.000 | 0.006 | 0.015 | 0.000 | MDM2 | 0.47887 | 1.00000 | 0.50663 | 0 | 0 |
| 838 | chr12 | 76202000 | 76203000 | 0.000 | 0.006 | 0.000 | 0.027 | PHLDA1 | 0.49735 | 1.00000 | 1.00000 | 1 | 1 |
| 839 | chr12 | 79270000 | 79271000 | 0.000 | 0.000 | 0.029 | 0.027 | SYT1 | 1.00000 | 0.54294 | 0.08726 | 0 | 0 |
| 840 | chr12 | 82572000 | 82573000 | 0.000 | 0.000 | 0.015 | 0.014 | CCDC59 | 1.00000 | 1.00000 | 0.29694 | 0 | 0 |
| 841 | chr12 | 84837000 | 84838000 | 0.000 | 0.000 | 0.000 | 0.027 | SLC6A15 | 0.49735 | 1.00000 | 1.00000 | 1 | 0 |
| 842 | chr12 | 86114000 | 86115000 | 0.000 | 0.000 | 0.029 | 0.000 | RASSF9 | 0.22755 | 0.54294 | 0.08726 | 0 | 0 |
| 843 | chr12 | 86115000 | 86116000 | 0.000 | 0.000 | 0.029 | 0.000 | RASSF9 | 0.22755 | 0.54294 | 0.08726 | 0 | 0 |
| 844 | chr12 | 92539000 | 92540000 | 0.000 | 0.000 | 0.088 | 0.027 | BTG1 | 0.15270 | 0.09031 | 0.00058 | 0 | 0 |
| 845 | chr12 | 92539000 | 92540000 | 0.000 | 0.000 | 0.074 | 0.014 | BTG1 | 0.10420 | 0.16101 | 0.00208 | 0 | 0 |
| 846 | chr12 | 96030000 | 96031000 | 0.000 | 0.006 | 0.015 | 0.000 | NTN4 | 0.47887 | 1.00000 | 0.29694 | 1 | 1 |
| 847 | chr12 | 110171000 | 110172000 | 0.000 | 0.006 | 0.015 | 0.000 | FAM222A | 0.47887 | 1.00000 | 0.50663 | 0 | 0 |
| 848 | chr12 | 110980000 | 110981000 | 0.000 | 0.006 | 0.015 | 0.014 | PPTC7 | 1.00000 | 1.00000 | 0.29694 | 1 | 0 |
| 849 | chr12 | 113493000 | 113494000 | 0.000 | 0.000 | 0.059 | 0.000 | DTX1 | 0.35016 | 0.29551 | 0.00730 | 0 | 0 |
| 850 | chr12 | 113494000 | 113495000 | 0.000 | 0.000 | 0.176 | 0.041 | DTX1 | 0.01224 | 0.04730 | 0.00000 | 1 | 0 |
| 851 | chr12 | 113495000 | 113496000 | 0.000 | 0.000 | 0.162 | 0.068 | DTX1 | 0.11004 | 0.61471 | 0.00000 | 1 | 0 |
| 852 | chr12 | 113496000 | 113497000 | 0.000 | 0.000 | 0.132 | 0.054 | DTX1 | 0.14640 | 0.02564 | 0.00001 | 1 | 0 |
| 853 | chr12 | 113497000 | 113498000 | 0.000 | 0.000 | 0.074 | 0.000 | DTX1 | 0.02326 | 0.16101 | 0.00208 | 1 | 0 |
| 854 | chr12 | 113498000 | 113499000 | 0.000 | 0.000 | 0.029 | 0.000 | DTX1 | 0.22755 | 0.54294 | 0.08726 | 0 | 0 |
| 855 | chr12 | 113512000 | 113513000 | 0.000 | 0.006 | 0.029 | 0.027 | MED13L | 0.22755 | 0.54294 | 0.08726 | 0 | 0 |
| 856 | chr12 | 115966000 | 115967000 | 0.000 | 0.000 | 0.000 | 0.000 | WDR66 | 0.49735 | 1.00000 | 1.00000 | 1 | 0 |
| 857 | chr12 | 122432000 | 122433000 | 0.000 | 0.000 | 0.029 | 0.027 | WDR66 | 0.19371 | 0.29551 | 0.68726 | 0 | 0 |
| 858 | chr12 | 122433000 | 122434000 | 0.000 | 0.000 | 0.059 | 0.014 | WDR66 | 0.49735 | 0.29551 | 0.00730 | 0 | 0 |
| 859 | chr12 | 122447000 | 122448000 | 0.000 | 0.000 | 0.000 | 0.027 | WDR66 | 1.00000 | 1.00000 | 1.00000 | 1 | 1 |
| 860 | chr12 | 122458000 | 122459000 | 0.000 | 0.000 | 0.118 | 0.068 | BCL7A | 0.38669 | 0.04825 | 0.00030 | 0 | 1 |

118

| | | | | | | | | | | | | | |
|---|---|---|---|---|---|---|---|---|---|---|---|---|---|
| 861 | chr12 | 122459000 | 122460000 | 0.000 | 0.006 | 0.324 | 0.108 | BCL7A | 0.00197 | 0.00003 | 0.00000 | 1 | 1 |
| 862 | chr12 | 122460000 | 122461000 | 0.000 | 0.000 | 0.176 | 0.081 | BCL7A | 0.12879 | 0.00730 | 0.00000 | 1 | 1 |
| 863 | chr12 | 122461000 | 122462000 | 0.000 | 0.006 | 0.279 | 0.162 | BCL7A | 0.10628 | 0.00013 | 0.00000 | 1 | 1 |
| 864 | chr12 | 122462000 | 122463000 | 0.000 | 0.012 | 0.191 | 0.027 | BCL7A | 0.00186 | 0.00372 | 0.00000 | 1 | 1 |
| 865 | chr12 | 122463000 | 122464000 | 0.000 | 0.012 | 0.132 | 0.054 | BCL7A | 0.14640 | 0.02564 | 0.00038 | 1 | 1 |
| 866 | chr12 | 124054000 | 124055000 | 0.028 | 0.000 | 0.015 | 0.014 | TMED2 | 1.00000 | 1.00000 | 0.29694 | 0 | 0 |
| 867 | chr12 | 127965000 | 127966000 | 0.000 | 0.000 | 0.000 | 0.027 | TMEM132C | 0.49735 | 1.00000 | 1.00000 | 0 | 0 |
| 868 | chr12 | 131303000 | 131304000 | 0.056 | 0.000 | 0.015 | 0.014 | STX2 | 1.00000 | 0.27446 | 0.29694 | 0 | 1 |
| 869 | chr12 | 131649000 | 131650000 | 0.000 | 0.000 | 0.000 | 0.027 | GPR133 | 0.49735 | 1.00000 | 1.00000 | 0 | 0 |
| 870 | chr12 | 133306000 | 133307000 | 0.028 | 0.000 | 0.015 | 0.027 | ANKLE2 | 1.00000 | 1.00000 | 0.29694 | 0 | 0 |
| 871 | chr13 | 21913000 | 21914000 | 0.000 | 0.000 | 0.029 | 0.000 | ZDHHC20 | 0.22755 | 0.54294 | 0.08726 | 0 | 0 |
| 872 | chr13 | 32116000 | 32117000 | 0.028 | 0.000 | 0.015 | 0.000 | RXFP2 | 0.47887 | 1.00000 | 0.29694 | 0 | 0 |
| 873 | chr13 | 35498000 | 35499000 | 0.000 | 0.000 | 0.015 | 0.027 | NBEA | 1.00000 | 1.00000 | 0.29694 | 0 | 0 |
| 874 | chr13 | 38371000 | 38372000 | 0.028 | 0.000 | 0.015 | 0.000 | TRPC4 | 0.47887 | 1.00000 | 0.29694 | 0 | 0 |
| 875 | chr13 | 38630000 | 38631000 | 0.000 | 0.000 | 0.029 | 0.000 | TRPC4 | 0.22755 | 0.54294 | 0.08726 | 0 | 0 |
| 876 | chr13 | 41156000 | 41157000 | 0.000 | 0.000 | 0.029 | 0.000 | FOXO1 | 0.22755 | 0.54294 | 0.08726 | 1 | 0 |
| 877 | chr13 | 41240000 | 41241000 | 0.028 | 0.000 | 0.029 | 0.000 | FOXO1 | 0.22755 | 1.00000 | 0.08726 | 1 | 0 |
| 878 | chr13 | 46958000 | 46959000 | 0.000 | 0.000 | 0.029 | 0.000 | KIAA0226L | 0.22755 | 0.54294 | 0.08726 | 0 | 0 |
| 879 | chr13 | 46959000 | 46960000 | 0.000 | 0.000 | 0.029 | 0.000 | KIAA0226L | 0.22755 | 0.54294 | 0.08726 | 0 | 0 |
| 880 | chr13 | 46960000 | 46961000 | 0.000 | 0.000 | 0.088 | 0.027 | KIAA0226L | 0.15270 | 0.09031 | 0.00058 | 0 | 1 |
| 881 | chr13 | 46961000 | 46962000 | 0.000 | 0.000 | 0.015 | 0.014 | KIAA0226L | 1.00000 | 1.00000 | 0.29694 | 0 | 0 |
| 882 | chr13 | 46962000 | 46963000 | 0.000 | 0.000 | 0.015 | 0.014 | KIAA0226L | 1.00000 | 1.00000 | 0.29694 | 0 | 0 |
| 883 | chr13 | 55239000 | 55240000 | 0.000 | 0.000 | 0.029 | 0.000 | OLFM4 | 0.22755 | 0.54294 | 0.08726 | 0 | 0 |
| 884 | chr13 | 55386000 | 55387000 | 0.000 | 0.000 | 0.029 | 0.000 | OLFM4 | 0.22755 | 0.54294 | 0.08726 | 0 | 0 |
| 885 | chr13 | 55598000 | 55599000 | 0.000 | 0.000 | 0.029 | 0.000 | OLFM4 | 0.22755 | 0.54294 | 0.08726 | 0 | 0 |
| 886 | chr13 | 57222000 | 57223000 | 0.000 | 0.000 | 0.029 | 0.000 | PRR20A;PRR20DPRR20BPRR20E; | 0.22755 | 0.54294 | 0.08726 | 0 | 0 |
| 887 | chr13 | 61343000 | 61344000 | 0.028 | 0.000 | 0.015 | 0.000 | TDRD3 | 0.47887 | 1.00000 | 0.29694 | 0 | 0 |
| 888 | chr13 | 62830000 | 62831000 | 0.000 | 0.000 | 0.000 | 0.027 | PCDH20 | 0.49735 | 1.00000 | 1.00000 | 0 | 0 |
| 889 | chr13 | 63049000 | 63050000 | 0.000 | 0.000 | 0.029 | 0.000 | PCDH20 | 0.22755 | 0.54294 | 0.08726 | 0 | 0 |
| 890 | chr13 | 63157000 | 63158000 | 0.028 | 0.000 | 0.015 | 0.000 | AL445989.1 | 0.47887 | 1.00000 | 0.29694 | 0 | 0 |
| 891 | chr13 | 63214000 | 63215000 | 0.028 | 0.000 | 0.015 | 0.000 | AL445989.1 | 0.47887 | 1.00000 | 0.29694 | 0 | 0 |
| 892 | chr13 | 64802000 | 64803000 | 0.000 | 0.000 | 0.015 | 0.014 | AL445989.1 | 1.00000 | 1.00000 | 0.29694 | 0 | 0 |
| 893 | chr13 | 65637000 | 65638000 | 0.000 | 0.000 | 0.029 | 0.000 | PCDH9 | 0.22755 | 0.54294 | 0.08726 | 0 | 0 |
| 894 | chr13 | 68656000 | 68657000 | 0.000 | 0.000 | 0.000 | 0.027 | PCDH9 | 0.49735 | 1.00000 | 1.00000 | 0 | 0 |
| 895 | chr13 | 69418000 | 69419000 | 0.000 | 0.000 | 0.029 | 0.014 | KLHL1 | 0.60686 | 0.54294 | 0.08726 | 0 | 0 |
| 896 | chr13 | 70956000 | 70957000 | 0.000 | 0.012 | 0.015 | 0.000 | KLHL1 | 0.47887 | 1.00000 | 1.00000 | 0 | 0 |
| 897 | chr13 | 74542000 | 74543000 | 0.000 | 0.000 | 0.029 | 0.000 | KLF12 | 0.22755 | 0.54294 | 0.08726 | 0 | 0 |
| 898 | chr13 | 75983000 | 75984000 | 0.000 | 0.000 | 0.074 | 0.014 | TBC1D4 | 0.10420 | 0.16101 | 0.00208 | 0 | 1 |
| 899 | chr13 | 75984000 | 75985000 | 0.000 | 0.000 | 0.118 | 0.027 | TBC1D4 | 0.04838 | 0.04825 | 0.00004 | 0 | 1 |
| 900 | chr13 | 83450000 | 83451000 | 0.000 | 0.000 | 0.029 | 0.000 | SLITRK1 | 0.22755 | 0.54294 | 0.08726 | 0 | 0 |
| 901 | chr13 | 84641000 | 84642000 | 0.000 | 0.000 | 0.015 | 0.014 | SLITRK1 | 1.00000 | 1.00000 | 0.29694 | 0 | 0 |
| 902 | chr13 | 87793000 | 87794000 | 0.000 | 0.000 | 0.015 | 0.014 | SLITRK5 | 1.00000 | 1.00000 | 0.29694 | 0 | 0 |
| 903 | chr13 | 91480000 | 91481000 | 0.000 | 0.000 | 0.000 | 0.027 | GPC5 | 0.49735 | 1.00000 | 1.00000 | 0 | 0 |
| 904 | chr13 | 106081000 | 106082000 | 0.000 | 0.000 | 0.015 | 0.014 | DAOA | 1.00000 | 1.00000 | 0.29694 | 0 | 0 |
| 905 | chr13 | 114786000 | 114787000 | 0.000 | 0.000 | 0.015 | 0.027 | RASA3 | 1.00000 | 1.00000 | 0.29694 | 0 | 0 |
| 906 | chr13 | 114916000 | 114917000 | 0.028 | 0.000 | 0.000 | 0.014 | RASA3 | 1.00000 | 0.34615 | 1.00000 | 0 | 0 |
| 907 | chr14 | 22948000 | 22949000 | 0.000 | 0.000 | 0.029 | 0.000 | TRAJ56 | 0.22755 | 0.54294 | 0.08726 | 0 | 0 |
| 908 | chr14 | 22949000 | 22950000 | 0.000 | 0.000 | 0.044 | 0.000 | TRAJ56 | 0.10727 | 0.54966 | 0.02537 | 0 | 0 |
| 909 | chr14 | 22950000 | 22951000 | 0.000 | 0.000 | 0.029 | 0.000 | TRAJ54 | 0.22755 | 0.54294 | 0.08726 | 0 | 0 |
| 910 | chr14 | 22977000 | 22978000 | 0.000 | 0.000 | 0.015 | 0.014 | TRAJ33 | 1.00000 | 1.00000 | 0.29694 | 0 | 0 |

119

| | | | | | | | | | | | | | |
|---|---|---|---|---|---|---|---|---|---|---|---|---|---|
| 911 | chr14 | 27286000 | 27287000 | 0.000 | 0.000 | 0.029 | 0.000 | NOVA1 | 0.22755 | 0.54294 | 0.08726 | 0 | 0 |
| 912 | chr14 | 28645000 | 28646000 | 0.000 | 0.000 | 0.000 | 0.027 | FOXG1 | 0.49735 | 1.00000 | 1.00000 | 0 | 0 |
| 913 | chr14 | 49407000 | 49408000 | 0.000 | 0.000 | 0.000 | 0.041 | RPS29 | 0.24603 | 1.00000 | 1.00000 | 0 | 0 |
| 914 | chr14 | 50864000 | 50865000 | 0.000 | 0.000 | 0.029 | 0.000 | CDKL1 | 0.22755 | 0.54294 | 0.08726 | 0 | 0 |
| 915 | chr14 | 54812000 | 54813000 | 0.000 | 0.000 | 0.000 | 0.027 | CDKN3 | 0.49735 | 1.00000 | 1.00000 | 0 | 0 |
| 916 | chr14 | 55348000 | 55349000 | 0.000 | 0.000 | 0.029 | 0.000 | GCH1 | 0.22755 | 0.54294 | 0.08726 | 0 | 0 |
| 917 | chr14 | 59827000 | 59828000 | 0.000 | 0.000 | 0.029 | 0.000 | DAAM1 | 0.22755 | 0.54294 | 0.08726 | 0 | 0 |
| 918 | chr14 | 63143000 | 63144000 | 0.000 | 0.000 | 0.015 | 0.014 | KCNH5 | 1.00000 | 1.00000 | 0.29694 | 0 | 0 |
| 919 | chr14 | 64194000 | 64195000 | 0.000 | 0.000 | 0.015 | 0.014 | SGPP1 | 1.00000 | 1.00000 | 0.29694 | 0 | 0 |
| 920 | chr14 | 69258000 | 69259000 | 0.000 | 0.000 | 0.191 | 0.027 | ZFP36L1 | 0.00186 | 0.00372 | 0.00000 | 1 | 1 |
| 921 | chr14 | 69259000 | 69260000 | 0.000 | 0.012 | 0.265 | 0.068 | ZFP36L1 | 0.00244 | 0.00024 | 0.00000 | 1 | 1 |
| 922 | chr14 | 78418000 | 78419000 | 0.000 | 0.000 | 0.029 | 0.000 | ADCK1 | 0.22755 | 0.54294 | 0.08726 | 1 | 0 |
| 923 | chr14 | 81685000 | 81686000 | 0.028 | 0.000 | 0.015 | 0.000 | GTF2A1 | 0.47887 | 1.00000 | 0.29694 | 0 | 0 |
| 924 | chr14 | 84420000 | 84421000 | 0.000 | 0.006 | 0.015 | 0.000 | FLRT2 | 0.47887 | 1.00000 | 0.50663 | 0 | 0 |
| 925 | chr14 | 91883000 | 91884000 | 0.000 | 0.000 | 0.015 | 0.014 | CCDC88C | 1.00000 | 1.00000 | 0.29694 | 0 | 0 |
| 926 | chr14 | 94941000 | 94942000 | 0.000 | 0.006 | 0.029 | 0.014 | SERPINA9 | 0.60686 | 0.54294 | 0.21104 | 1 | 0 |
| 927 | chr14 | 94942000 | 94943000 | 0.000 | 0.000 | 0.118 | 0.041 | SERPINA9 | 0.01415 | 0.04825 | 0.00004 | 1 | 1 |
| 928 | chr14 | 96179000 | 96180000 | 0.028 | 0.037 | 0.132 | 0.108 | TCL1A | 0.79702 | 0.15881 | 0.01566 | 1 | 1 |
| 929 | chr14 | 96180000 | 96181000 | 0.028 | 0.025 | 0.088 | 0.054 | TCL1A | 0.52007 | 0.41714 | 0.06858 | 1 | 1 |
| 930 | chr14 | 101597000 | 101598000 | 0.000 | 0.000 | 0.000 | 0.027 | AL117190.3 | 0.49735 | 1.00000 | 1.00000 | 0 | 0 |
| 931 | chr14 | 102285000 | 102286000 | 0.000 | 0.000 | 0.015 | 0.014 | PPP2R5C | 1.00000 | 1.00000 | 0.29694 | 0 | 0 |
| 932 | chr14 | 105954000 | 105955000 | 0.000 | 0.000 | 0.044 | 0.014 | CRIP1 | 0.34948 | 0.54966 | 0.02537 | 0 | 0 |
| 933 | chr14 | 106031000 | 106032000 | 0.000 | 0.000 | 0.015 | 0.014 | IGHA2 | 1.00000 | 1.00000 | 0.29694 | 0 | 0 |
| 934 | chr14 | 106042000 | 106043000 | 0.000 | 0.019 | 0.103 | 0.041 | IGHA2 | 0.19468 | 0.09269 | 0.00855 | 0 | 1 |
| 935 | chr14 | 106048000 | 106049000 | 0.000 | 0.006 | 0.015 | 0.000 | IGHA2 | 0.47887 | 1.00000 | 0.50663 | 0 | 0 |
| 936 | chr14 | 106054000 | 106055000 | 0.000 | 0.000 | 0.029 | 0.014 | IGHA2 | 0.60686 | 0.54294 | 0.08726 | 0 | 0 |
| 937 | chr14 | 106055000 | 106056000 | 0.056 | 0.000 | 0.103 | 0.027 | IGHA2 | 0.08710 | 0.49207 | 0.00016 | 0 | 1 |
| 938 | chr14 | 106056000 | 106057000 | 0.056 | 0.006 | 0.074 | 0.027 | IGHA2 | 0.25970 | 1.00000 | 0.00953 | 0 | 1 |
| 939 | chr14 | 106057000 | 106058000 | 0.000 | 0.000 | 0.059 | 0.000 | IGHA2 | 0.05016 | 0.29551 | 0.00730 | 0 | 1 |
| 940 | chr14 | 106058000 | 106059000 | 0.000 | 0.000 | 0.029 | 0.000 | IGHA2 | 0.22755 | 0.54294 | 0.08726 | 0 | 0 |
| 941 | chr14 | 106066000 | 106067000 | 0.000 | 0.000 | 0.059 | 0.000 | IGHE | 0.05016 | 0.29551 | 0.00730 | 0 | 1 |
| 942 | chr14 | 106067000 | 106068000 | 0.000 | 0.000 | 0.044 | 0.014 | IGHE | 0.34948 | 0.54966 | 0.02537 | 0 | 0 |
| 943 | chr14 | 106068000 | 106069000 | 0.000 | 0.000 | 0.103 | 0.027 | IGHE | 0.08710 | 0.09269 | 0.00016 | 0 | 1 |
| 944 | chr14 | 106069000 | 106070000 | 0.000 | 0.006 | 0.206 | 0.216 | IGHE | 1.00000 | 0.00197 | 0.00000 | 0 | 1 |
| 945 | chr14 | 106070000 | 106071000 | 0.000 | 0.000 | 0.088 | 0.068 | IGHE | 0.75773 | 0.09031 | 0.00058 | 0 | 1 |
| 946 | chr14 | 106071000 | 106072000 | 0.000 | 0.000 | 0.074 | 0.068 | IGHE | 1.00000 | 0.16101 | 0.00208 | 0 | 1 |
| 947 | chr14 | 106072000 | 106073000 | 0.000 | 0.000 | 0.029 | 0.014 | IGHE | 0.60686 | 0.54294 | 0.08726 | 0 | 0 |
| 948 | chr14 | 106082000 | 106083000 | 0.000 | 0.000 | 0.015 | 0.027 | IGHG4 | 1.00000 | 1.00000 | 0.29694 | 0 | 0 |
| 949 | chr14 | 106092000 | 106093000 | 0.000 | 0.000 | 0.029 | 0.000 | IGHG4 | 0.22755 | 0.54294 | 0.08726 | 0 | 0 |
| 950 | chr14 | 106094000 | 106095000 | 0.000 | 0.006 | 0.147 | 0.027 | IGHG4 | 0.01393 | 0.01404 | 0.00003 | 0 | 1 |
| 951 | chr14 | 106095000 | 106096000 | 0.000 | 0.000 | 0.103 | 0.081 | IGHG4 | 0.77363 | 0.09269 | 0.00016 | 0 | 1 |
| 952 | chr14 | 106110000 | 106111000 | 0.000 | 0.000 | 0.074 | 0.014 | IGHG2 | 0.10420 | 0.16101 | 0.00208 | 0 | 1 |
| 953 | chr14 | 106111000 | 106112000 | 0.000 | 0.000 | 0.015 | 0.014 | IGHG2 | 1.00000 | 1.00000 | 0.29694 | 0 | 0 |
| 954 | chr14 | 106112000 | 106113000 | 0.000 | 0.056 | 0.294 | 0.257 | IGHG2 | 0.70749 | 0.00011 | 0.00000 | 0 | 1 |
| 955 | chr14 | 106113000 | 106114000 | 0.028 | 0.068 | 0.397 | 0.284 | IGHG2 | 0.16121 | 0.00002 | 0.00000 | 0 | 1 |
| 956 | chr14 | 106114000 | 106115000 | 0.000 | 0.000 | 0.279 | 0.122 | IGHG2 | 0.02111 | 0.00013 | 0.00000 | 0 | 1 |
| 957 | chr14 | 106146000 | 106147000 | 0.000 | 0.000 | 0.029 | 0.000 | IGHA1 | 0.22755 | 0.54294 | 0.08726 | 0 | 0 |
| 958 | chr14 | 106151000 | 106152000 | 0.000 | 0.006 | 0.015 | 0.014 | IGHA1 | 1.00000 | 1.00000 | 0.50663 | 0 | 0 |
| 959 | chr14 | 106152000 | 106153000 | 0.000 | 0.006 | 0.015 | 0.027 | IGHA1 | 1.00000 | 1.00000 | 0.50663 | 0 | 0 |
| 960 | chr14 | 106161000 | 106162000 | 0.000 | 0.000 | 0.015 | 0.014 | IGHA1 | 1.00000 | 1.00000 | 0.29694 | 0 | 0 |
| 961 | chr14 | 106173000 | 106174000 | 0.028 | 0.006 | 0.029 | 0.027 | IGHA1 | 1.00000 | 1.00000 | 0.21104 | 0 | 0 |

120

| No. | chr | start | end | | | | | Gene | | | | | |
|---|---|---|---|---|---|---|---|---|---|---|---|---|---|
| 962 | chr14 | 106174000 | 106175000 | 0.000 | 0.006 | 0.029 | 0.000 | IGHA1 | 0.22755 | 0.54294 | 0.21104 | 0 | 0 |
| 963 | chr14 | 106175000 | 106176000 | 0.028 | 0.006 | 0.059 | 0.014 | IGHA1 | 0.19371 | 0.65667 | 0.02818 | 0 | - |
| 964 | chr14 | 106176000 | 106177000 | 0.139 | 0.031 | 0.103 | 0.068 | IGHA1 | 0.55139 | 0.74810 | 0.04551 | 0 | - |
| 965 | chr14 | 106177000 | 106178000 | 0.000 | 0.019 | 0.059 | 0.027 | IGHA1 | 0.42627 | 0.29551 | 0.20027 | 0 | - |
| 966 | chr14 | 106178000 | 106179000 | 0.000 | 0.006 | 0.059 | 0.027 | IGHA1 | 0.08710 | 0.09269 | 0.02818 | 0 | - |
| 967 | chr14 | 106209000 | 106210000 | 0.000 | 0.006 | 0.103 | 0.054 | IGHG1 | 0.23486 | 0.04825 | 0.00016 | 0 | - |
| 968 | chr14 | 106210000 | 106211000 | 0.000 | 0.000 | 0.118 | 0.068 | IGHG1 | 0.38669 | 0.04825 | 0.00030 | 0 | - |
| 969 | chr14 | 106211000 | 106212000 | 0.000 | 0.056 | 0.118 | 0.149 | IGHG1 | 0.20587 | 0.00098 | 0.00004 | 0 | - |
| 970 | chr14 | 106212000 | 106213000 | 0.028 | 0.106 | 0.235 | 0.270 | IGHG1 | 0.71144 | 0.00070 | 0.00025 | 0 | - |
| 971 | chr14 | 106213000 | 106214000 | 0.056 | 0.068 | 0.309 | 0.216 | IGHG1 | 0.04243 | 0.00034 | 0.00035 | 0 | - |
| 972 | chr14 | 106214000 | 106215000 | 0.000 | 0.000 | 0.382 | 0.000 | IGHG1 | 0.00044 | 0.01404 | 0.00060 | 0 | - |
| 973 | chr14 | 106237000 | 106238000 | 0.000 | 0.000 | 0.147 | 0.027 | IGHG3 | 0.01070 | 0.09031 | 0.00058 | 0 | - |
| 974 | chr14 | 106238000 | 106239000 | 0.056 | 0.000 | 0.088 | 0.135 | IGHG3 | 0.00370 | 0.00730 | 0.00000 | 0 | - |
| 975 | chr14 | 106239000 | 106240000 | 0.028 | 0.062 | 0.176 | 0.230 | IGHG3 | 0.27339 | 0.04910 | 0.00000 | 0 | - |
| 976 | chr14 | 106240000 | 106241000 | 0.000 | 0.130 | 0.206 | 0.081 | IGHG3 | 0.25971 | 0.00034 | 0.00349 | 0 | - |
| 977 | chr14 | 106241000 | 106242000 | 0.000 | 0.025 | 0.324 | 0.014 | IGHG3 | 0.03144 | 0.00107 | 0.00136 | 0 | - |
| 978 | chr14 | 106242000 | 106243000 | 0.000 | 0.000 | 0.221 | 0.000 | IGHG3 | 0.34948 | 0.54966 | 0.00000 | 0 | - |
| 979 | chr14 | 106243000 | 106321000 | 0.056 | 0.000 | 0.044 | 0.054 | IGHG3 | 0.05016 | 0.29551 | 0.02537 | 0 | 0 |
| 980 | chr14 | 106321000 | 106322000 | 0.250 | 0.006 | 0.059 | 0.162 | IGHM | 0.00556 | 0.00107 | 0.00730 | 0 | - |
| 981 | chr14 | 106322000 | 106323000 | 0.694 | 0.062 | 0.221 | 0.284 | IGHM | 0.29797 | 0.02782 | 0.00000 | 0 | - |
| 982 | chr14 | 106323000 | 106324000 | 0.833 | 0.193 | 0.235 | 0.365 | IGHM | 0.44266 | 0.80827 | 0.00040 | 0 | - |
| 983 | chr14 | 106324000 | 106325000 | 0.333 | 0.335 | 0.221 | 0.838 | IGHM | 0.28848 | 0.00006 | 0.71834 | 0 | - |
| 984 | chr14 | 106325000 | 106326000 | 0.250 | 0.540 | 0.279 | 0.905 | IGHM | 1.00000 | 1.00000 | 0.44111 | 0 | - |
| 985 | chr14 | 106326000 | 106327000 | 0.694 | 0.335 | 0.838 | 0.739 | IGHJ6 | 0.76698 | 0.00000 | 0.00001 | 0 | - |
| 986 | chr14 | 106327000 | 106328000 | 0.694 | 0.248 | 0.926 | 0.932 | IGHJ6 | 0.32171 | 0.00000 | 0.00000 | 0 | - |
| 987 | chr14 | 106328000 | 106329000 | 0.028 | 0.441 | 0.809 | 0.649 | IGHJ6 | 0.38669 | 0.03086 | 0.00000 | 0 | - |
| 988 | chr14 | 106329000 | 106330000 | 0.028 | 0.298 | 0.882 | 0.027 | IGHJ6 | 0.39187 | 0.29080 | 0.00017 | 0 | - |
| 989 | chr14 | 106330000 | 106331000 | 0.000 | 0.012 | 0.574 | 0.000 | IGHJ6 | 0.67943 | 1.00000 | 0.15671 | 0 | 0 |
| 990 | chr14 | 106331000 | 106331000 | 0.028 | 0.006 | 0.044 | 0.000 | IGHJ3;IGHD4;IGHD5;IGHD7-27;IGHD1;IGHD2; | 1.00000 | 0.34615 | 1.00000 | 0 | 0 |
| 991 | chr14 | 106338000 | 106339000 | 0.028 | 0.006 | 0.000 | 0.000 | IGHD7-27 | 0.22755 | 0.54294 | 0.21104 | 0 | 0 |
| 992 | chr14 | 106350000 | 106351000 | 0.000 | 0.000 | 0.029 | 0.000 | IGHD4-23 | 0.22755 | 0.54294 | 0.08726 | 0 | 0 |
| 993 | chr14 | 106352000 | 106353000 | 0.000 | 0.006 | 0.029 | 0.000 | IGHD3-22 | 0.22755 | 0.54294 | 0.21104 | 0 | 0 |
| 994 | chr14 | 106353000 | 106354000 | 0.000 | 0.006 | 0.029 | 0.000 | IGHD2-21 | 0.47887 | 0.54966 | 0.50663 | 0 | 0 |
| 995 | chr14 | 106354000 | 106355000 | 0.000 | 0.006 | 0.015 | 0.000 | IGHD2-21 | 0.10727 | 0.65667 | 0.02537 | 0 | 0 |
| 996 | chr14 | 106355000 | 106356000 | 0.028 | 0.006 | 0.044 | 0.000 | IGHD2-21 | 0.05016 | 0.54294 | 0.00730 | 0 | 0 |
| 997 | chr14 | 106358000 | 106359000 | 0.000 | 0.006 | 0.059 | 0.000 | IGHD1-20;IGHD6-19; | 0.22755 | 0.34615 | 0.21104 | 0 | 0 |
| 998 | chr14 | 106362000 | 106363000 | 0.028 | 0.006 | 0.029 | 0.000 | IGHD5-18 | 1.00000 | 0.54294 | 1.00000 | 0 | 0 |
| 999 | chr14 | 106364000 | 106365000 | 0.000 | 0.006 | 0.000 | 0.000 | IGHD3-16 | 0.22755 | 0.54294 | 0.08726 | 0 | 0 |
| 1000 | chr14 | 106367000 | 106368000 | 0.000 | 0.012 | 0.029 | 0.000 | IGHD2-15 | 0.22755 | 0.54966 | 0.08726 | 0 | 0 |
| 1001 | chr14 | 106370000 | 106371000 | 0.000 | 0.012 | 0.029 | 0.014 | IGHD6-13 | 0.34948 | 0.54294 | 0.15671 | 0 | 0 |
| 1002 | chr14 | 106371000 | 106372000 | 0.000 | 0.006 | 0.044 | 0.014 | IGHD3-10;IGHD3-9; | 0.60686 | 0.54966 | 0.58408 | 0 | 0 |
| 1003 | chr14 | 106372000 | 106373000 | 0.000 | 0.019 | 0.029 | 0.029 | IGHD3-9 | 0.47887 | 1.00000 | 0.50663 | 0 | 0 |
| 1004 | chr14 | 106375000 | 106377000 | 0.000 | 0.012 | 0.029 | 0.000 | IGHD2-8 | 0.47887 | 1.00000 | 1.00000 | 0 | 0 |
| 1005 | chr14 | 106376000 | 106377000 | 0.000 | 0.031 | 0.015 | 0.000 | IGHD1-7 | 1.00000 | 1.00000 | 1.00000 | 0 | 0 |
| 1006 | chr14 | 106380000 | 106380000 | 0.000 | 0.031 | 0.015 | 0.000 | IGHD6-6 | 1.00000 | 1.00000 | 1.00000 | 0 | 0 |
| 1007 | chr14 | 106381000 | 106381000 | 0.000 | 0.037 | 0.015 | 0.000 | IGHD3-3 | 1.00000 | 1.00000 | 0.32529 | 0 | 0 |
| 1008 | chr14 | 106382000 | 106383000 | 0.014 | 0.012 | 0.000 | 0.014 | IGHD2-2 | 0.34948 | 0.34948 | 0.32529 | 0 | 0 |
| 1009 | chr14 | 106383000 | 106384000 | 0.014 | 0.044 | 0.044 | 0.014 | IGHD2-2 | 0.34948 | 0.54966 | 0.72719 | 0 | 0 |
| 1010 | chr14 | 106384000 | 106385000 | 0.014 | 0.044 | 0.044 | 0.014 | IGHD2-2 | 0.34948 | 0.54966 | 0.02537 | 0 | 0 |
| 1011 | chr14 | 106385000 | | 0.014 | 0.012 | 0.044 | 0.014 | IGHD1-1 | 0.34948 | 0.54966 | 0.15671 | 0 | 0 |

121

| 1012 | chr14 | 106385000 | 106386000 | 0.000 | 0.000 | 0.029 | 0.014 | IGHD1-1 | 0.60686 | 0.54294 | 0.08726 | 0 | 0 |
| 1013 | chr14 | 106387000 | 106388000 | 0.000 | 0.000 | 0.029 | 0.014 | KIAA0125 | 0.60686 | 0.54294 | 0.08726 | 0 | 0 |
| 1014 | chr14 | 106405000 | 106406000 | 0.000 | 0.006 | 0.015 | 0.014 | IGHV6-1 | 1.00000 | 1.00000 | 0.50663 | 0 | 0 |
| 1015 | chr14 | 106406000 | 106407000 | 0.000 | 0.006 | 0.015 | 0.014 | IGHV6-1 | 1.00000 | 1.00000 | 0.50663 | 0 | 0 |
| 1016 | chr14 | 106419000 | 106420000 | 0.000 | 0.006 | 0.015 | 0.000 | IGHV6-1 | 0.47887 | 1.00000 | 0.50663 | 0 | 0 |
| 1017 | chr14 | 106452000 | 106453000 | 0.000 | 0.006 | 0.029 | 0.000 | IGHV1-2 | 0.22755 | 0.54294 | 0.21104 | 0 | 0 |
| 1018 | chr14 | 106453000 | 106454000 | 0.000 | 0.006 | 0.044 | 0.000 | IGHV1-2 | 0.10727 | 0.54966 | 0.07959 | 0 | 0 |
| 1019 | chr14 | 106454000 | 106455000 | 0.000 | 0.000 | 0.029 | 0.000 | IGHV1-2 | 0.22755 | 0.54294 | 0.08726 | 0 | 0 |
| 1020 | chr14 | 106494000 | 106495000 | 0.000 | 0.019 | 0.000 | 0.014 | IGHV2-5 | 1.00000 | 1.00000 | 0.55662 | 0 | 0 |
| 1021 | chr14 | 106518000 | 106519000 | 0.028 | 0.037 | 0.000 | 0.054 | IGHV3-7 | 0.12104 | 0.34615 | 0.18288 | 0 | 1 |
| 1022 | chr14 | 106519000 | 106520000 | 0.000 | 0.012 | 0.000 | 0.027 | IGHV3-7 | 0.49735 | 1.00000 | 1.00000 | 0 | 0 |
| 1023 | chr14 | 106539000 | 106540000 | 0.000 | 0.031 | 0.015 | 0.000 | IGHV1-8 | 0.47887 | 1.00000 | 0.67240 | 0 | 0 |
| 1024 | chr14 | 106552000 | 106553000 | 0.000 | 0.006 | 0.029 | 0.014 | IGHV3-9 | 0.60686 | 0.54294 | 0.21104 | 0 | 0 |
| 1025 | chr14 | 106573000 | 106574000 | 0.000 | 0.019 | 0.029 | 0.068 | IGHV3-11 | 0.44431 | 0.54294 | 0.63492 | 0 | 1 |
| 1026 | chr14 | 106574000 | 106575000 | 0.000 | 0.006 | 0.029 | 0.041 | IGHV3-11 | 1.00000 | 0.54294 | 0.21104 | 0 | 0 |
| 1027 | chr14 | 106578000 | 106579000 | 0.000 | 0.000 | 0.015 | 0.027 | IGHV3-11 | 1.00000 | 1.00000 | 0.29694 | 0 | 0 |
| 1028 | chr14 | 106579000 | 106580000 | 0.000 | 0.000 | 0.015 | 0.027 | IGHV3-11 | 1.00000 | 1.00000 | 0.29694 | 0 | 0 |
| 1029 | chr14 | 106610000 | 106611000 | 0.056 | 0.012 | 0.029 | 0.000 | IGHV3-15 | 0.22755 | 0.60763 | 0.58408 | 0 | 1 |
| 1030 | chr14 | 106641000 | 106642000 | 0.000 | 0.019 | 0.015 | 0.000 | IGHV1-18 | 0.47887 | 1.00000 | 1.00000 | 0 | 0 |
| 1031 | chr14 | 106642000 | 106643000 | 0.000 | 0.012 | 0.015 | 0.000 | IGHV1-18 | 0.47887 | 1.00000 | 1.00000 | 0 | 0 |
| 1032 | chr14 | 106691000 | 106692000 | 0.000 | 0.012 | 0.029 | 0.027 | IGHV3-21 | 1.00000 | 0.54294 | 0.58408 | 0 | 0 |
| 1033 | chr14 | 106692000 | 106693000 | 0.000 | 0.006 | 0.015 | 0.041 | IGHV3-21 | 0.62100 | 1.00000 | 0.50663 | 0 | 0 |
| 1034 | chr14 | 106725000 | 106726000 | 0.083 | 0.068 | 0.103 | 0.135 | IGHV3-23 | 0.61250 | 1.00000 | 0.42238 | 0 | 1 |
| 1035 | chr14 | 106726000 | 106727000 | 0.028 | 0.019 | 0.088 | 0.095 | IGHV3-23 | 1.00000 | 0.41714 | 0.02173 | 0 | 1 |
| 1036 | chr14 | 106733000 | 106734000 | 0.028 | 0.006 | 0.015 | 0.027 | IGHV1-24 | 1.00000 | 1.00000 | 0.50663 | 0 | 0 |
| 1037 | chr14 | 106757000 | 106758000 | 0.056 | 0.000 | 0.015 | 0.000 | IGHV2-26 | 0.47887 | 0.27446 | 0.29694 | 0 | 1 |
| 1038 | chr14 | 106758000 | 106759000 | 0.056 | 0.000 | 0.000 | 0.000 | IGHV2-26 | 1.00000 | 0.11763 | 1.00000 | 0 | 1 |
| 1039 | chr14 | 106791000 | 106792000 | 0.056 | 0.006 | 0.015 | 0.000 | IGHV3-30 | 0.47887 | 0.27446 | 0.50663 | 0 | 1 |
| 1040 | chr14 | 106804000 | 106805000 | 0.000 | 0.006 | 0.029 | 0.000 | IGHV4-31 | 0.22755 | 0.54294 | 0.21104 | 0 | 0 |
| 1041 | chr14 | 106805000 | 106806000 | 0.000 | 0.006 | 0.044 | 0.014 | IGHV4-31 | 0.34948 | 0.54966 | 0.07959 | 0 | 0 |
| 1042 | chr14 | 106806000 | 106807000 | 0.000 | 0.006 | 0.015 | 0.000 | IGHV4-31 | 0.47887 | 1.00000 | 0.50663 | 0 | 0 |
| 1043 | chr14 | 106815000 | 106816000 | 0.000 | 0.012 | 0.044 | 0.027 | IGHV3-33 | 0.67043 | 0.54966 | 0.15671 | 0 | 0 |
| 1044 | chr14 | 106816000 | 106817000 | 0.000 | 0.000 | 0.074 | 0.014 | IGHV3-33 | 0.10420 | 0.16101 | 0.00953 | 0 | 0 |
| 1045 | chr14 | 106817000 | 106818000 | 0.000 | 0.000 | 0.029 | 0.000 | IGHV3-33 | 0.22755 | 0.54294 | 0.08726 | 0 | 0 |
| 1046 | chr14 | 106829000 | 106830000 | 0.167 | 0.050 | 0.162 | 0.135 | IGHV4-34 | 0.81354 | 1.00000 | 0.00804 | 0 | 1 |
| 1047 | chr14 | 106830000 | 106831000 | 0.028 | 0.043 | 0.118 | 0.135 | IGHV4-34 | 0.80514 | 0.15803 | 0.07447 | 0 | 1 |
| 1048 | chr14 | 106877000 | 106878000 | 0.056 | 0.006 | 0.015 | 0.041 | IGHV4-39 | 0.62100 | 0.27446 | 0.50663 | 0 | 1 |
| 1049 | chr14 | 106878000 | 106879000 | 0.028 | 0.012 | 0.044 | 0.041 | IGHV4-39 | 1.00000 | 1.00000 | 0.15671 | 0 | 0 |
| 1050 | chr14 | 106967000 | 106968000 | 0.056 | 0.000 | 0.015 | 0.000 | IGHV1-46 | 0.47887 | 0.27446 | 0.29694 | 0 | 1 |
| 1051 | chr14 | 106994000 | 106995000 | 0.028 | 0.012 | 0.088 | 0.122 | IGHV3-48 | 0.59201 | 0.41714 | 0.00949 | 0 | 1 |
| 1052 | chr14 | 106995000 | 106996000 | 0.000 | 0.000 | 0.029 | 0.027 | IGHV3-48 | 0.49735 | 1.00000 | 1.00000 | 0 | 0 |
| 1053 | chr14 | 107034000 | 107035000 | 0.028 | 0.000 | 0.000 | 0.014 | IGHV5-51 | 1.00000 | 0.34615 | 1.00000 | 0 | 0 |
| 1054 | chr14 | 107035000 | 107036000 | 0.000 | 0.006 | 0.029 | 0.014 | IGHV5-51 | 0.60686 | 0.54294 | 0.21104 | 0 | 0 |
| 1055 | chr14 | 107048000 | 107049000 | 0.028 | 0.006 | 0.000 | 0.000 | IGHV3-53 | 1.00000 | 0.34615 | 1.00000 | 0 | 0 |
| 1056 | chr14 | 107049000 | 107050000 | 0.000 | 0.012 | 0.044 | 0.027 | IGHV3-53 | 0.67043 | 0.54966 | 0.15671 | 0 | 0 |
| 1057 | chr14 | 107083000 | 107084000 | 0.000 | 0.006 | 0.044 | 0.054 | IGHV4-59 | 1.00000 | 0.54966 | 0.07959 | 0 | 1 |
| 1058 | chr14 | 107084000 | 107085000 | 0.000 | 0.006 | 0.029 | 0.027 | IGHV4-59 | 1.00000 | 0.54294 | 0.21104 | 0 | 0 |
| 1059 | chr14 | 107095000 | 107096000 | 0.000 | 0.006 | 0.015 | 0.000 | IGHV4-61 | 0.47887 | 1.00000 | 0.50663 | 0 | 0 |
| 1060 | chr14 | 107113000 | 107114000 | 0.000 | 0.000 | 0.029 | 0.000 | IGHV3-64 | 0.22755 | 0.54294 | 0.08726 | 0 | 0 |
| 1061 | chr14 | 107114000 | 107115000 | 0.000 | 0.000 | 0.029 | 0.000 | IGHV3-64 | 0.22755 | 0.54294 | 0.08726 | 0 | 0 |
| 1062 | chr14 | 107169000 | 107170000 | 0.056 | 0.068 | 0.206 | 0.041 | IGHV1-69 | 0.00346 | 0.04910 | 0.00442 | 0 | 1 |

| 1063 | chr14 | 107170000 | 107171000 | 0.028 | 0.075 | 0.294 | 0.095 | IGHV1-69 | 0.00279 | 0.00075 | 0.00004 | 0 | 1 |
|------|-------|-----------|-----------|-------|-------|-------|-------|----------|---------|---------|---------|---|---|
| 1064 | chr14 | 107176000 | 107177000 | 0.028 | 0.006 | 0.118 | 0.027 | IGHV2-70 | 0.04838 | 0.15803 | 0.00030 | 0 | 1 |
| 1065 | chr14 | 107177000 | 107178000 | 0.000 | 0.044 | 0.027 | | IGHV2-70 | 0.67043 | 0.54966 | 0.02537 | 0 | 0 |
| 1066 | chr14 | 107178000 | 107179000 | 0.056 | 0.161 | 0.456 | 0.284 | IGHV2-70 | 0.03781 | 0.00002 | 0.00001 | 0 | 1 |
| 1067 | chr14 | 107179000 | 107180000 | 0.056 | 0.180 | 0.382 | 0.338 | IGHV2-70.2 | 0.60350 | 0.00034 | 0.00206 | 0 | 1 |
| 1068 | chr14 | 107183000 | 107184000 | 0.000 | 0.006 | 0.029 | 0.000 | IGHV2-70 | 0.22755 | 0.54294 | 0.21104 | 0 | 0 |
| 1069 | chr14 | 107199000 | 107200000 | 0.000 | 0.012 | 0.015 | 0.000 | IGHV3-72 | 0.47887 | 1.00000 | 1.00000 | 0 | 0 |
| 1070 | chr14 | 107218000 | 107219000 | 0.028 | 0.012 | 0.015 | 0.000 | IGHV3-74 | 0.47887 | 1.00000 | 1.00000 | 0 | 0 |
| 1071 | chr14 | 107219000 | 107220000 | 0.000 | 0.012 | 0.074 | 0.027 | IGHV3-74 | 0.25970 | 0.16101 | 0.02559 | 0 | 1 |
| 1072 | chr14 | 107221000 | 107222000 | 0.000 | 0.000 | 0.059 | 0.000 | IGHV3-74 | 0.05016 | 0.29551 | 0.00730 | 0 | 1 |
| 1073 | chr14 | 107232000 | 107233000 | 0.000 | 0.000 | 0.029 | 0.000 | IGHV3-74 | 0.22755 | 0.54294 | 0.08726 | 0 | 0 |
| 1074 | chr14 | 107253000 | 107254000 | 0.000 | 0.000 | 0.044 | 0.014 | IGHV7-81 | 0.34948 | 0.54966 | 0.02537 | 0 | 0 |
| 1075 | chr14 | 107258000 | 107259000 | 0.000 | 0.000 | 0.015 | 0.014 | IGHV7-81 | 1.00000 | 1.00000 | 0.29694 | 0 | 0 |
| 1076 | chr14 | 107259000 | 107260000 | 0.000 | 0.025 | 0.235 | 0.027 | IGHV7-81 | 0.00021 | 0.00098 | 0.00000 | 0 | 1 |
| 1077 | chr15 | 45003000 | 45004000 | 0.000 | 0.000 | 0.044 | 0.000 | B2M | 0.10727 | 0.54966 | 0.02537 | 0 | 0 |
| 1078 | chr15 | 45007000 | 45008000 | 0.000 | 0.000 | 0.044 | 0.000 | B2M | 0.10727 | 0.54966 | 0.02537 | 0 | 0 |
| 1079 | chr15 | 45814000 | 45815000 | 0.000 | 0.000 | 0.015 | 0.014 | SLC30A4 | 1.00000 | 1.00000 | 0.29694 | 0 | 0 |
| 1080 | chr15 | 59664000 | 59665000 | 0.000 | 0.000 | 0.044 | 0.041 | MYO1E | 1.00000 | 0.54966 | 0.02537 | 0 | 0 |
| 1081 | chr15 | 65588000 | 65589000 | 0.028 | 0.000 | 0.000 | 0.014 | PARP16 | 1.00000 | 0.34615 | 1.00000 | 0 | 0 |
| 1082 | chr15 | 78332000 | 78333000 | 0.028 | 0.000 | 0.000 | 0.014 | TBC1D2B | 1.00000 | 0.34615 | 1.00000 | 0 | 0 |
| 1083 | chr15 | 83227000 | 83228000 | 0.000 | 0.000 | 0.029 | 0.000 | CPEB1 | 0.22755 | 0.54294 | 0.08726 | 0 | 0 |
| 1084 | chr15 | 86226000 | 86227000 | 0.000 | 0.000 | 0.044 | 0.000 | AKAP13 | 0.10727 | 0.54966 | 0.02537 | 0 | 0 |
| 1085 | chr15 | 86233000 | 86234000 | 0.000 | 0.000 | 0.029 | 0.014 | AKAP13 | 0.60686 | 0.54294 | 0.08726 | 0 | 0 |
| 1086 | chr15 | 86245000 | 86246000 | 0.000 | 0.000 | 0.059 | 0.000 | AKAP13 | 0.05016 | 0.29551 | 0.00730 | 0 | 1 |
| 1087 | chr16 | 368000 | 369000 | 0.000 | 0.000 | 0.015 | 0.014 | AXIN1 | 1.00000 | 1.00000 | 0.29694 | 0 | 0 |
| 1088 | chr16 | 3788000 | 3789000 | 0.000 | 0.000 | 0.015 | 0.014 | CREBBP | 1.00000 | 1.00000 | 0.29694 | 0 | 0 |
| 1089 | chr16 | 10971000 | 10972000 | 0.000 | 0.000 | 0.162 | 0.041 | CIITA | 0.02233 | 0.01471 | 0.00000 | 1 | 1 |
| 1090 | chr16 | 10972000 | 10973000 | 0.000 | 0.000 | 0.191 | 0.081 | CIITA | 0.08249 | 0.00372 | 0.00000 | 1 | 1 |
| 1091 | chr16 | 10973000 | 10974000 | 0.000 | 0.000 | 0.162 | 0.095 | CIITA | 0.31342 | 0.01471 | 0.00000 | 1 | 1 |
| 1092 | chr16 | 10974000 | 10975000 | 0.000 | 0.000 | 0.059 | 0.000 | CIITA | 0.05016 | 0.29551 | 0.00730 | 1 | 1 |
| 1093 | chr16 | 11348000 | 11349000 | 0.000 | 0.000 | 0.191 | 0.027 | SOCS1 | 0.00186 | 0.00372 | 0.00000 | 1 | 1 |
| 1094 | chr16 | 11349000 | 11350000 | 0.000 | 0.000 | 0.221 | 0.041 | SOCS1 | 0.00179 | 0.00107 | 0.00000 | 1 | 1 |
| 1095 | chr16 | 21167000 | 21168000 | 0.000 | 0.000 | 0.015 | 0.014 | DNAH3 | 1.00000 | 1.00000 | 0.29694 | 0 | 0 |
| 1096 | chr16 | 27325000 | 27326000 | 0.000 | 0.000 | 0.029 | 0.041 | CTD-3203P2.2 | 1.00000 | 0.54294 | 0.08726 | 0 | 0 |
| 1097 | chr16 | 27326000 | 27327000 | 0.000 | 0.000 | 0.088 | 0.041 | CTD-3203P2.2 | 0.31126 | 0.09031 | 0.00058 | 0 | 0 |
| 1098 | chr16 | 27327000 | 27328000 | 0.000 | 0.000 | 0.029 | 0.000 | IL4R | 0.22755 | 0.54294 | 0.08726 | 0 | 0 |
| 1099 | chr16 | 27414000 | 27415000 | 0.000 | 0.000 | 0.029 | 0.000 | IL21R | 0.22755 | 0.54294 | 0.08726 | 0 | 0 |
| 1100 | chr16 | 29248000 | 29249000 | 0.000 | 0.000 | 0.029 | 0.000 | 61E3.4 | 0.22755 | 0.54294 | 0.08726 | 0 | 0 |
| 1101 | chr16 | 31910000 | 31911000 | 0.000 | 0.000 | 0.015 | 0.014 | ZNF267 | 1.00000 | 1.00000 | 0.29694 | 0 | 0 |
| 1102 | chr16 | 46821000 | 46822000 | 0.000 | 0.000 | 0.015 | 0.014 | C16orf87 | 1.00000 | 1.00000 | 0.29694 | 0 | 0 |
| 1103 | chr16 | 50985000 | 50986000 | 0.000 | 0.000 | 0.015 | 0.014 | CYLD | 1.00000 | 1.00000 | 0.29694 | 0 | 0 |
| 1104 | chr16 | 64351000 | 64352000 | 0.000 | 0.000 | 0.029 | 0.014 | CDH11 | 0.60686 | 0.54294 | 0.08726 | 0 | 0 |
| 1105 | chr16 | 78398000 | 78399000 | 0.000 | 0.000 | 0.000 | 0.027 | WWOX | 0.49735 | 1.00000 | 1.00000 | 0 | 0 |
| 1106 | chr16 | 78615000 | 78616000 | 0.000 | 0.000 | 0.015 | 0.014 | WWOX | 1.00000 | 1.00000 | 0.29694 | 0 | 0 |
| 1107 | chr16 | 78753000 | 78754000 | 0.000 | 0.000 | 0.015 | 0.014 | WWOX | 1.00000 | 1.00000 | 0.29694 | 0 | 0 |
| 1108 | chr16 | 78811000 | 78812000 | 0.000 | 0.000 | 0.000 | 0.027 | WWOX | 0.49735 | 1.00000 | 1.00000 | 0 | 0 |
| 1109 | chr16 | 79988000 | 79989000 | 0.000 | 0.000 | 0.015 | 0.014 | MAF | 1.00000 | 1.00000 | 0.29694 | 0 | 0 |
| 1110 | chr16 | 81836000 | 81837000 | 0.000 | 0.000 | 0.029 | 0.000 | PLCG2 | 0.22755 | 0.54294 | 0.08726 | 0 | 0 |
| 1111 | chr16 | 85932000 | 85933000 | 0.000 | 0.000 | 0.059 | 0.027 | IRF8 | 0.42627 | 0.29551 | 0.00730 | 1 | 1 |
| 1112 | chr16 | 85933000 | 85934000 | 0.000 | 0.012 | 0.221 | 0.081 | IRF8 | 0.03144 | 0.00107 | 0.00000 | 1 | 1 |
| 1113 | chr16 | 85934000 | 85935000 | 0.000 | 0.006 | 0.015 | 0.027 | IRF8 | 1.00000 | 1.00000 | 0.50663 | 1 | 0 |

| | | | | | | | | | | | | | |
|---|---|---|---|---|---|---|---|---|---|---|---|---|---|
| 1114 | chr16 | 85936000 | 85937000 | 0.000 | 0.000 | 0.029 | 0.000 | IRF8 | 0.22755 | 0.54294 | 0.08726 | 1 | 0 |
| 1115 | chr16 | 88441000 | 88442000 | 0.000 | 0.000 | 0.015 | 0.014 | ZNF469 | 1.00000 | 1.00000 | 0.29694 | 0 | 0 |
| 1116 | chr17 | 3598000 | 3599000 | 0.000 | 0.000 | 0.029 | 0.014 | P2RX5;P2RX5-TAX1BP3P2RX5; | 0.60686 | 0.54294 | 0.08726 | 0 | 0 |
| 1117 | chr17 | 17286000 | 17287000 | 0.000 | 0.000 | 0.029 | 0.000 | SMCR9 | 0.22755 | 0.54294 | 0.08726 | 0 | 0 |
| 1118 | chr17 | 21194000 | 21195000 | 0.000 | 0.000 | 0.015 | 0.041 | MAP2K3 | 0.62100 | 1.00000 | 0.29694 | 0 | 0 |
| 1119 | chr17 | 29646000 | 29647000 | 0.000 | 0.000 | 0.029 | 0.014 | EVI2A | 0.60686 | 0.54294 | 0.08726 | 0 | 0 |
| 1120 | chr17 | 38020000 | 38021000 | 0.000 | 0.000 | 0.029 | 0.014 | IKZF3 | 0.60686 | 0.54294 | 0.08726 | 0 | 0 |
| 1121 | chr17 | 43662000 | 43663000 | 0.000 | 0.000 | 0.029 | 0.000 | PLEKHM1 | 0.22755 | 0.54294 | 0.08726 | 0 | 0 |
| 1122 | chr17 | 56408000 | 56409000 | 0.000 | 0.006 | 0.059 | 0.027 | BZRAP1 | 0.42627 | 0.29551 | 0.02818 | 0 | 0 |
| 1123 | chr17 | 56409000 | 56410000 | 0.000 | 0.000 | 0.265 | 0.027 | BZRAP1 | 0.00005 | 0.00024 | 0.00000 | 0 | 1 |
| 1124 | chr17 | 57916000 | 57917000 | 0.000 | 0.000 | 0.029 | 0.014 | VMP1 | 0.60686 | 0.54294 | 0.08726 | 1 | 0 |
| 1125 | chr17 | 57917000 | 57918000 | 0.000 | 0.000 | 0.029 | 0.000 | VMP1 | 0.22755 | 0.54294 | 0.08726 | 1 | 0 |
| 1126 | chr17 | 62007000 | 62008000 | 0.000 | 0.000 | 0.029 | 0.000 | CD79B | 0.22755 | 0.54294 | 0.08726 | 0 | 0 |
| 1127 | chr17 | 62008000 | 62009000 | 0.000 | 0.000 | 0.044 | 0.014 | CD79B | 0.34948 | 0.54966 | 0.02537 | 0 | 0 |
| 1128 | chr17 | 63067000 | 63068000 | 0.000 | 0.000 | 0.015 | 0.014 | GNA13 | 1.00000 | 1.00000 | 0.29694 | 0 | 0 |
| 1129 | chr17 | 65676000 | 65677000 | 0.000 | 0.000 | 0.029 | 0.000 | PITPNC1 | 0.22755 | 0.54294 | 0.08726 | 0 | 0 |
| 1130 | chr17 | 69365000 | 69366000 | 0.000 | 0.000 | 0.015 | 0.014 | AC007461.1 | 1.00000 | 1.00000 | 0.29694 | 0 | 0 |
| 1131 | chr17 | 70083000 | 70084000 | 0.028 | 0.000 | 0.000 | 0.014 | SOX9 | 1.00000 | 0.34615 | 1.00000 | 0 | 0 |
| 1132 | chr17 | 74733000 | 74734000 | 0.000 | 0.000 | 0.000 | 0.027 | SRSF2 | 0.49735 | 1.00000 | 1.00000 | 0 | 0 |
| 1133 | chr17 | 75447000 | 75448000 | 0.000 | 0.000 | 0.044 | 0.000 | 9-Sep-19 | 0.10727 | 0.54966 | 0.02537 | 0 | 0 |
| 1134 | chr17 | 75448000 | 75449000 | 0.000 | 0.000 | 0.044 | 0.000 | 9-Sep-19 | 0.10727 | 0.54966 | 0.02537 | 0 | 0 |
| 1135 | chr17 | 76775000 | 76776000 | 0.000 | 0.000 | 0.000 | 0.027 | CYTH1 | 0.49735 | 1.00000 | 1.00000 | 0 | 0 |
| 1136 | chr17 | 80928000 | 80929000 | 0.000 | 0.000 | 0.029 | 0.000 | B3GNTL1 | 0.22755 | 0.54294 | 0.08726 | 0 | 0 |
| 1137 | chr17 | 80976000 | 80977000 | 0.000 | 0.000 | 0.015 | 0.014 | B3GNTL1 | 1.00000 | 1.00000 | 0.29694 | 0 | 0 |
| 1138 | chr18 | 2709000 | 2710000 | 0.000 | 0.000 | 0.029 | 0.000 | SMCHD1 | 0.22755 | 0.54294 | 0.08726 | 0 | 0 |
| 1139 | chr18 | 3600000 | 3601000 | 0.000 | 0.000 | 0.015 | 0.014 | DLGAP1 | 1.00000 | 1.00000 | 0.29694 | 0 | 0 |
| 1140 | chr18 | 12062000 | 12063000 | 0.000 | 0.000 | 0.000 | 0.041 | ANKRD62 | 0.24603 | 1.00000 | 1.00000 | 0 | 0 |
| 1141 | chr18 | 27771000 | 27772000 | 0.000 | 0.000 | 0.029 | 0.000 | DSC3 | 0.22755 | 0.54294 | 0.08726 | 0 | 0 |
| 1142 | chr18 | 28066000 | 28067000 | 0.000 | 0.000 | 0.029 | 0.000 | DSC3 | 0.22755 | 0.54294 | 0.08726 | 0 | 0 |
| 1143 | chr18 | 30349000 | 30350000 | 0.000 | 0.000 | 0.000 | 0.027 | AC012123.1;KLHL14; | 0.49735 | 1.00000 | 1.00000 | 0 | 0 |
| 1144 | chr18 | 36806000 | 36807000 | 0.000 | 0.000 | 0.029 | 0.000 | CELF4 | 0.22755 | 0.54294 | 0.08726 | 0 | 0 |
| 1145 | chr18 | 37751000 | 37752000 | 0.000 | 0.000 | 0.015 | 0.014 | PIK3C3 | 1.00000 | 1.00000 | 0.29694 | 0 | 0 |
| 1146 | chr18 | 38672000 | 38673000 | 0.028 | 0.000 | 0.000 | 0.014 | PIK3C3 | 1.00000 | 0.34615 | 1.00000 | 0 | 0 |
| 1147 | chr18 | 42168000 | 42169000 | 0.028 | 0.000 | 0.000 | 0.014 | SETBP1 | 1.00000 | 0.34615 | 1.00000 | 0 | 0 |
| 1148 | chr18 | 51952000 | 51953000 | 0.000 | 0.000 | 0.029 | 0.000 | C18orf54 | 0.22755 | 0.54294 | 0.08726 | 0 | 0 |
| 1149 | chr18 | 52447000 | 52448000 | 0.000 | 0.000 | 0.015 | 0.014 | RAB27B | 1.00000 | 1.00000 | 0.29694 | 0 | 0 |
| 1150 | chr18 | 52988000 | 52989000 | 0.000 | 0.000 | 0.029 | 0.000 | TCF4 | 0.22755 | 0.54294 | 0.08726 | 0 | 0 |
| 1151 | chr18 | 54653000 | 54654000 | 0.000 | 0.000 | 0.000 | 0.027 | WDR7 | 0.49735 | 1.00000 | 1.00000 | 0 | 0 |
| 1152 | chr18 | 60794000 | 60795000 | 0.000 | 0.000 | 0.029 | 0.000 | BCL2 | 0.22755 | 0.54294 | 0.08726 | 1 | 0 |
| 1153 | chr18 | 60805000 | 60806000 | 0.000 | 0.000 | 0.074 | 0.081 | BCL2 | 1.00000 | 0.16101 | 0.00208 | 1 | 1 |
| 1154 | chr18 | 60806000 | 60807000 | 0.000 | 0.006 | 0.132 | 0.122 | BCL2 | 1.00000 | 0.02564 | 0.00009 | 1 | 1 |
| 1155 | chr18 | 60809000 | 60810000 | 0.000 | 0.000 | 0.059 | 0.027 | BCL2 | 0.42627 | 0.29551 | 0.00730 | 1 | 1 |
| 1156 | chr18 | 60821000 | 60822000 | 0.000 | 0.000 | 0.029 | 0.000 | BCL2 | 0.22755 | 0.54294 | 0.08726 | 1 | 0 |
| 1157 | chr18 | 60825000 | 60826000 | 0.000 | 0.000 | 0.044 | 0.027 | BCL2 | 0.67043 | 0.54966 | 0.02537 | 1 | 0 |
| 1158 | chr18 | 60826000 | 60827000 | 0.000 | 0.000 | 0.029 | 0.000 | BCL2 | 0.22755 | 0.54294 | 0.08726 | 1 | 0 |
| 1159 | chr18 | 60828000 | 60829000 | 0.000 | 0.000 | 0.015 | 0.027 | BCL2 | 1.00000 | 1.00000 | 0.29694 | 1 | 0 |
| 1160 | chr18 | 60873000 | 60874000 | 0.000 | 0.000 | 0.044 | 0.027 | BCL2 | 0.67043 | 0.54966 | 0.02537 | 1 | 0 |
| 1161 | chr18 | 60875000 | 60876000 | 0.000 | 0.000 | 0.044 | 0.027 | BCL2 | 0.67043 | 0.54966 | 0.02537 | 1 | 0 |
| 1162 | chr18 | 60876000 | 60877000 | 0.000 | 0.000 | 0.015 | 0.054 | BCL2 | 0.36833 | 1.00000 | 0.29694 | 1 | 0 |
| 1163 | chr18 | 60983000 | 60984000 | 0.000 | 0.006 | 0.059 | 0.068 | BCL2 | 1.00000 | 0.29551 | 0.02818 | 1 | 1 |

| | | | | | | | | | | | | | |
|---|---|---|---|---|---|---|---|---|---|---|---|---|---|
| 1164 | chr18 | 60984000 | 60985000 | 0.000 | 0.012 | 0.176 | 0.459 | BCL2 | 0.00034 | 0.00730 | 0.00001 | 1 | 1 |
| 1165 | chr18 | 60985000 | 60986000 | 0.000 | 0.000 | 0.221 | 0.635 | BCL2 | 0.00000 | 0.00107 | 0.00000 | 1 | 1 |
| 1166 | chr18 | 60986000 | 60987000 | 0.000 | 0.019 | 0.235 | 0.730 | BCL2 | 0.00000 | 0.00098 | 0.00000 | 1 | 1 |
| 1167 | chr18 | 60987000 | 60988000 | 0.000 | 0.019 | 0.191 | 0.500 | BCL2 | 0.00019 | 0.00372 | 0.00001 | 1 | 1 |
| 1168 | chr18 | 60988000 | 60989000 | 0.000 | 0.012 | 0.221 | 0.595 | BCL2 | 0.00001 | 0.00107 | 0.00000 | 1 | 1 |
| 1169 | chr18 | 61810000 | 61811000 | 0.000 | 0.000 | 0.015 | 0.014 | SERPINB8 | 1.00000 | 1.00000 | 0.29694 | 0 | 0 |
| 1170 | chr18 | 63080000 | 63081000 | 0.000 | 0.000 | 0.029 | 0.000 | CDH7 | 0.22755 | 0.54294 | 0.08726 | 0 | 0 |
| 1171 | chr18 | 63791000 | 63792000 | 0.028 | 0.000 | 0.015 | 0.000 | CDH7 | 0.47887 | 1.00000 | 0.29694 | 0 | 0 |
| 1172 | chr18 | 63875000 | 63876000 | 0.000 | 0.000 | 0.029 | 0.000 | CDH19 | 0.22755 | 0.54294 | 0.08726 | 0 | 0 |
| 1173 | chr18 | 64643000 | 64644000 | 0.000 | 0.000 | 0.029 | 0.000 | CDH19 | 0.22755 | 0.54294 | 0.08726 | 0 | 0 |
| 1174 | chr18 | 65863000 | 65864000 | 0.000 | 0.000 | 0.000 | 0.027 | TMX3 | 0.49735 | 1.00000 | 1.00000 | 0 | 0 |
| 1175 | chr18 | 66328000 | 66329000 | 0.000 | 0.000 | 0.015 | 0.014 | TMX3 | 1.00000 | 1.00000 | 0.29694 | 0 | 0 |
| 1176 | chr18 | 70462000 | 70463000 | 0.000 | 0.000 | 0.015 | 0.014 | NETO1 | 1.00000 | 1.00000 | 0.29694 | 0 | 0 |
| 1177 | chr18 | 73767000 | 73768000 | 0.000 | 0.000 | 0.015 | 0.014 | ZNF516 | 1.00000 | 1.00000 | 0.29694 | 0 | 0 |
| 1178 | chr18 | 76515000 | 76516000 | 0.000 | 0.000 | 0.029 | 0.014 | SALL3 | 0.60686 | 0.54294 | 0.08726 | 0 | 0 |
| 1179 | chr18 | 76724000 | 76725000 | 0.000 | 0.000 | 0.015 | 0.014 | SALL3 | 1.00000 | 1.00000 | 0.29694 | 0 | 0 |
| 1180 | chr18 | 76725000 | 76726000 | 0.000 | 0.000 | 0.015 | 0.014 | SALL3 | 1.00000 | 1.00000 | 0.29694 | 0 | 0 |
| 1181 | chr19 | 1612000 | 1613000 | 0.056 | 0.000 | 0.000 | 0.000 | TCF3 | 1.00000 | 0.11763 | 1.00000 | 0 | 1 |
| 1182 | chr19 | 2476000 | 2477000 | 0.000 | 0.000 | 0.029 | 0.000 | GADD45B | 0.22755 | 0.54294 | 0.08726 | 1 | 0 |
| 1183 | chr19 | 10304000 | 10305000 | 0.000 | 0.000 | 0.059 | 0.000 | DNMT1 | 0.05016 | 0.29551 | 0.00730 | 0 | 1 |
| 1184 | chr19 | 10305000 | 10306000 | 0.000 | 0.000 | 0.044 | 0.000 | DNMT1 | 0.10727 | 0.54966 | 0.02537 | 0 | 0 |
| 1185 | chr19 | 10335000 | 10336000 | 0.000 | 0.000 | 0.015 | 0.014 | S1PR2 | 1.00000 | 1.00000 | 0.29694 | 1 | 0 |
| 1186 | chr19 | 10340000 | 10341000 | 0.000 | 0.000 | 0.118 | 0.041 | S1PR2 | 0.11795 | 0.04825 | 0.00004 | 1 | 1 |
| 1187 | chr19 | 10341000 | 10342000 | 0.000 | 0.012 | 0.206 | 0.054 | S1PR2 | 0.01013 | 0.00197 | 0.00000 | 1 | 1 |
| 1188 | chr19 | 16030000 | 16031000 | 0.028 | 0.000 | 0.015 | 0.000 | CYP4F11 | 0.47887 | 1.00000 | 0.29694 | 0 | 0 |
| 1189 | chr19 | 16436000 | 16437000 | 0.000 | 0.000 | 0.029 | 0.014 | KLF2 | 0.60686 | 0.54294 | 0.08726 | 1 | 0 |
| 1190 | chr19 | 20889000 | 20890000 | 0.000 | 0.006 | 0.015 | 0.000 | ZNF626 | 0.47887 | 1.00000 | 0.50663 | 0 | 0 |
| 1191 | chr19 | 21073000 | 21074000 | 0.000 | 0.000 | 0.015 | 0.027 | ZNF85 | 1.00000 | 1.00000 | 0.29694 | 0 | 0 |
| 1192 | chr19 | 21092000 | 21093000 | 0.000 | 0.000 | 0.029 | 0.000 | ZNF85 | 0.22755 | 0.54294 | 0.08726 | 0 | 0 |
| 1193 | chr19 | 23841000 | 23842000 | 0.000 | 0.000 | 0.015 | 0.027 | ZNF675 | 1.00000 | 1.00000 | 0.29694 | 0 | 0 |
| 1194 | chr19 | 29256000 | 29257000 | 0.000 | 0.000 | 0.029 | 0.000 | UQCRFS1 | 0.22755 | 0.54294 | 0.08726 | 0 | 0 |
| 1195 | chr19 | 44183000 | 44184000 | 0.000 | 0.000 | 0.029 | 0.000 | PLAUR | 0.22755 | 0.54294 | 0.08726 | 0 | 0 |
| 1196 | chr19 | 50399000 | 50400000 | 0.000 | 0.000 | 0.029 | 0.000 | IL4I1 | 0.22755 | 0.54294 | 0.08726 | 0 | 0 |
| 1197 | chr19 | 53419000 | 53420000 | 0.028 | 0.000 | 0.015 | 0.014 | ZNF321P;ZNF816;ZNF816-ZNF321PZNF321PZNF816-ZNF321P; | 1.00000 | 1.00000 | 0.29694 | 0 | 0 |
| 1198 | chr20 | 15470000 | 15471000 | 0.028 | 0.006 | 0.000 | 0.000 | MACROD2 | 1.00000 | 0.34615 | 1.00000 | 0 | 0 |
| 1199 | chr20 | 23359000 | 23360000 | 0.056 | 0.000 | 0.000 | 0.000 | NAPB | 1.00000 | 0.11763 | 1.00000 | 0 | 1 |
| 1200 | chr20 | 23912000 | 23913000 | 0.000 | 0.000 | 0.000 | 0.027 | CST5 | 0.49735 | 1.00000 | 1.00000 | 0 | 0 |
| 1201 | chr20 | 46131000 | 46132000 | 0.000 | 0.000 | 0.059 | 0.014 | NCOA3 | 0.19371 | 0.29551 | 0.00730 | 1 | 1 |
| 1202 | chr20 | 49127000 | 49128000 | 0.000 | 0.000 | 0.029 | 0.014 | PTPN1 | 0.60686 | 0.54294 | 0.08726 | 0 | 0 |
| 1203 | chr20 | 49648000 | 49649000 | 0.000 | 0.000 | 0.029 | 0.000 | KCNG1 | 0.22755 | 0.54294 | 0.08726 | 0 | 0 |
| 1204 | chr20 | 61607000 | 61608000 | 0.000 | 0.000 | 0.000 | 0.027 | SLC17A9 | 0.49735 | 1.00000 | 1.00000 | 0 | 0 |
| 1205 | chr21 | 21597000 | 21598000 | 0.000 | 0.000 | 0.029 | 0.000 | NCAM2 | 0.22755 | 0.54294 | 0.08726 | 0 | 0 |
| 1206 | chr21 | 23458000 | 23459000 | 0.000 | 0.000 | 0.029 | 0.000 | NCAM2 | 0.22755 | 0.54294 | 0.08726 | 0 | 0 |
| 1207 | chr21 | 24998000 | 24999000 | 0.000 | 0.000 | 0.029 | 0.000 | MRPL39 | 0.22755 | 0.54294 | 0.08726 | 0 | 0 |
| 1208 | chr21 | 26935000 | 26936000 | 0.000 | 0.000 | 0.015 | 0.014 | MRPL39 | 1.00000 | 1.00000 | 0.29694 | 0 | 0 |
| 1209 | chr21 | 35779000 | 35780000 | 0.000 | 0.000 | 0.000 | 0.027 | SMIM11 | 0.49735 | 1.00000 | 1.00000 | 0 | 0 |
| 1210 | chr21 | 38779000 | 38780000 | 0.000 | 0.000 | 0.000 | 0.027 | DYRK1A | 0.49735 | 1.00000 | 1.00000 | 0 | 0 |
| 1211 | chr21 | 43254000 | 43255000 | 0.000 | 0.000 | 0.029 | 0.000 | PRDM15 | 0.22755 | 0.54294 | 0.08726 | 0 | 0 |

| | | | | | | | | | | | | | | |
|---|---|---|---|---|---|---|---|---|---|---|---|---|---|---|
| 1212 | chr21 | 44612000 | 44613000 | 0.000 | 0.000 | 0.000 | 0.027 | CRYAA | 0.49735 | 1.00000 | 1.00000 | 0 | 0 |
| 1213 | chr21 | 45382000 | 45383000 | 0.000 | 0.000 | 0.029 | 0.000 | AGPAT3 | 0.22755 | 0.54294 | 0.08726 | 0 | 0 |
| 1214 | chr21 | 46058000 | 46059000 | 0.000 | 0.000 | 0.015 | 0.027 | KRTAP10-10 | 1.00000 | 1.00000 | 0.29694 | 0 | 0 |
| 1215 | chr22 | 19050000 | 19051000 | 0.000 | 0.006 | 0.000 | 0.027 | DGCR2 | 0.49735 | 1.00000 | 1.00000 | 0 | 0 |
| 1216 | chr22 | 20212000 | 20213000 | 0.000 | 0.000 | 0.029 | 0.014 | RTN4R | 0.60686 | 0.54294 | 0.08726 | 0 | 0 |
| 1217 | chr22 | 20708000 | 20709000 | 0.000 | 0.000 | 0.000 | 0.000 | FAM230A | 0.22755 | 0.54294 | 0.08726 | 0 | 0 |
| 1218 | chr22 | 21994000 | 21995000 | 0.028 | 0.000 | 0.015 | 0.000 | SDF2L1 | 0.47887 | 1.00000 | 0.29694 | 0 | 0 |
| 1219 | chr22 | 22379000 | 22380000 | 0.000 | 0.000 | 0.029 | 0.027 | IGLV4-69 | 1.00000 | 0.54294 | 0.08725 | 0 | 1 |
| 1220 | chr22 | 22380000 | 22381000 | 0.000 | 0.012 | 0.044 | 0.068 | IGLV5-69 | 0.72064 | 0.54966 | 0.15671 | 0 | 0 |
| 1221 | chr22 | 22381000 | 22382000 | 0.000 | 0.012 | 0.015 | 0.027 | IGLV4-69 | 1.00000 | 1.00000 | 1.00000 | 0 | 1 |
| 1222 | chr22 | 22385000 | 22386000 | 0.028 | 0.031 | 0.029 | 0.068 | IGLV4-69 | 0.44431 | 1.00000 | 1.00000 | 0 | 0 |
| 1223 | chr22 | 22452000 | 22453000 | 0.000 | 0.012 | 0.015 | 0.014 | IGLV8-61 | 1.00000 | 1.00000 | 1.00000 | 0 | 0 |
| 1224 | chr22 | 22453000 | 22454000 | 0.000 | 0.012 | 0.015 | 0.014 | IGLV8-61 | 1.00000 | 1.00000 | 1.00000 | 0 | 0 |
| 1225 | chr22 | 22516000 | 22517000 | 0.000 | 0.025 | 0.015 | 0.054 | IGLV4-60 | 0.36833 | 1.00000 | 1.00000 | 0 | 1 |
| 1226 | chr22 | 22517000 | 22518000 | 0.000 | 0.019 | 0.000 | 0.014 | IGLV4-60 | 1.00000 | 1.00000 | 0.53662 | 0 | 0 |
| 1227 | chr22 | 22550000 | 22551000 | 0.056 | 0.006 | 0.044 | 0.054 | IGLV6-57 | 1.00000 | 1.00000 | 0.07959 | 0 | 1 |
| 1228 | chr22 | 22569000 | 22570000 | 0.000 | 0.006 | 0.015 | 0.014 | IGLV10-54 | 1.00000 | 1.00000 | 0.50663 | 0 | 0 |
| 1229 | chr22 | 22676000 | 22677000 | 0.028 | 0.000 | 0.015 | 0.000 | IGLV1-51 | 0.47887 | 0.11840 | 0.29694 | 0 | 0 |
| 1230 | chr22 | 22677000 | 22678000 | 0.083 | 0.012 | 0.015 | 0.014 | IGLV1-51 | 1.00000 | 1.00000 | 1.00000 | 0 | 0 |
| 1231 | chr22 | 22707000 | 22708000 | 0.028 | 0.006 | 0.044 | 0.014 | IGLV5-48 | 0.34948 | 1.00000 | 0.07959 | 0 | 0 |
| 1232 | chr22 | 22712000 | 22713000 | 0.083 | 0.012 | 0.088 | 0.041 | IGLV1-47 | 0.31126 | 1.00000 | 0.00949 | 0 | 0 |
| 1233 | chr22 | 22723000 | 22724000 | 0.000 | 0.006 | 0.015 | 0.027 | IGLV7-46 | 1.00000 | 1.00000 | 0.50663 | 0 | 1 |
| 1234 | chr22 | 22724000 | 22725000 | 0.028 | 0.012 | 0.088 | 0.041 | IGLV7-46 | 0.31126 | 0.41714 | 0.00949 | 0 | 0 |
| 1235 | chr22 | 22730000 | 22731000 | 0.000 | 0.006 | 0.059 | 0.054 | IGLV5-45 | 1.00000 | 0.29551 | 0.02818 | 0 | 0 |
| 1236 | chr22 | 22731000 | 22732000 | 0.000 | 0.006 | 0.029 | 0.000 | IGLV5-45 | 0.22755 | 0.54294 | 0.21104 | 0 | 0 |
| 1237 | chr22 | 22735000 | 22736000 | 0.028 | 0.037 | 0.059 | 0.068 | IGLV1-44 | 1.00000 | 0.65667 | 0.48849 | 0 | 0 |
| 1238 | chr22 | 22749000 | 22750000 | 0.000 | 0.006 | 0.059 | 0.027 | IGLV7-43 | 0.42627 | 0.29551 | 0.02818 | 0 | 0 |
| 1239 | chr22 | 22758000 | 22759000 | 0.028 | 0.006 | 0.029 | 0.014 | IGLV1-40 | 0.60686 | 1.00000 | 0.21104 | 0 | 0 |
| 1240 | chr22 | 22759000 | 22760000 | 0.056 | 0.006 | 0.044 | 0.027 | IGLV1-40 | 0.67043 | 1.00000 | 0.07959 | 0 | 0 |
| 1241 | chr22 | 22764000 | 22765000 | 0.111 | 0.006 | 0.044 | 0.068 | IGLV1-40 | 0.72064 | 0.23165 | 0.07959 | 0 | 0 |
| 1242 | chr22 | 23028000 | 23029000 | 0.000 | 0.006 | 0.015 | 0.000 | IGLV3-25 | 0.47887 | 1.00000 | 0.50663 | 0 | 0 |
| 1243 | chr22 | 23029000 | 23030000 | 0.028 | 0.062 | 0.132 | 0.108 | IGLV3-25 | 0.79792 | 0.15881 | 0.11274 | 0 | 0 |
| 1244 | chr22 | 23035000 | 23036000 | 0.000 | 0.000 | 0.015 | 0.014 | IGLV2-23 | 1.00000 | 1.00000 | 0.29694 | 0 | 0 |
| 1245 | chr22 | 23039000 | 23040000 | 0.000 | 0.000 | 0.000 | 0.027 | IGLV2-23 | 0.49735 | 0.49735 | 1.00000 | 0 | 0 |
| 1246 | chr22 | 23040000 | 23041000 | 0.000 | 0.043 | 0.103 | 0.054 | IGLV2-23 | 0.35266 | 0.66188 | 0.12716 | 0 | 0 |
| 1247 | chr22 | 23054000 | 23055000 | 0.028 | 0.006 | 0.044 | 0.000 | IGLV2-23 | 0.10727 | 1.00000 | 0.07959 | 0 | 0 |
| 1248 | chr22 | 23055000 | 23056000 | 0.000 | 0.056 | 0.059 | 0.014 | IGLV3-21 | 0.19371 | 0.54294 | 1.00000 | 0 | 1 |
| 1249 | chr22 | 23063000 | 23064000 | 0.000 | 0.000 | 0.074 | 0.041 | IGLV3-19 | 0.47996 | 0.16161 | 0.00208 | 0 | 0 |
| 1250 | chr22 | 23090000 | 23091000 | 0.000 | 0.000 | 0.059 | 0.041 | IGLV5-16 | 0.70990 | 0.29551 | 0.00730 | 0 | 0 |
| 1251 | chr22 | 23100000 | 23101000 | 0.028 | 0.019 | 0.044 | 0.054 | IGLV2-14 | 1.00000 | 0.54966 | 0.36534 | 0 | 0 |
| 1252 | chr22 | 23101000 | 23102000 | 0.000 | 0.031 | 0.074 | 0.081 | IGLV2-14 | 1.00000 | 0.66188 | 0.16714 | 0 | 1 |
| 1253 | chr22 | 23114000 | 23115000 | 0.000 | 0.000 | 0.015 | 0.027 | IGLV3-12 | 0.60686 | 1.00000 | 0.29694 | 0 | 0 |
| 1254 | chr22 | 23134000 | 23135000 | 0.000 | 0.000 | 0.029 | 0.014 | IGLV2-11 | 0.25970 | 0.16161 | 0.09269 | 0 | 0 |
| 1255 | chr22 | 23154000 | 23155000 | 0.000 | 0.019 | 0.074 | 0.027 | IGLV3-10 | 1.00000 | 1.00000 | 0.05242 | 0 | 1 |
| 1256 | chr22 | 23161000 | 23162000 | 0.000 | 0.006 | 0.000 | 0.014 | IGLV3-9 | 1.00000 | 1.00000 | 1.00000 | 0 | 0 |
| 1257 | chr22 | 23162000 | 23163000 | 0.000 | 0.012 | 0.000 | 0.014 | IGLV3-9 | 0.24603 | 0.69031 | 1.00000 | 0 | 1 |
| 1258 | chr22 | 23165000 | 23166000 | 0.000 | 0.012 | 0.088 | 0.041 | IGLV2-8 | 0.31126 | 1.00000 | 0.00311 | 0 | 0 |
| 1259 | chr22 | 23192000 | 23193000 | 0.000 | 0.006 | 0.015 | 0.041 | IGLV4-3 | 0.47887 | 0.47887 | 0.50663 | 0 | 0 |
| 1260 | chr22 | 23197000 | 23198000 | 0.000 | 0.006 | 0.147 | 0.000 | IGLV4-3 | 0.17231 | 0.17231 | 0.00108 | 0 | 0 |
| 1261 | chr22 | 23198000 | 23199000 | 0.000 | 0.025 | 0.221 | 0.068 | IGLV4-3 | 0.01424 | 0.01404 | 0.00002 | 0 | 0 |
| 1262 | chr22 | 23199000 | 23200000 | 0.000 | 0.031 | 0.221 | 0.068 | IGLV4-3 | 0.01424 | 0.00107 | 0.00002 | 0 | 1 |

| # | chr | start | end | v1 | v2 | v3 | v4 | gene | p1 | p2 | p3 | z1 | z2 | flag |
|---|---|---|---|---|---|---|---|---|---|---|---|---|---|---|
| 1263 | chr22 | 23203000 | 23204000 | 0.000 | 0.000 | 0.029 | 0.000 | IGLV4-3 | 0.22755 | 0.54294 | 0.68726 | 0 | 0 | 0 |
| 1264 | chr22 | 23204000 | 23205000 | 0.056 | 0.000 | 0.059 | 0.041 | IGLV4-3 | 0.70990 | 1.00000 | 0.60730 | 0 | 0 | 1 |
| 1265 | chr22 | 23205000 | 23206000 | 0.000 | 0.000 | 0.015 | 0.027 | IGLV4-3 | 1.00000 | 1.00000 | 0.29694 | 0 | 0 | 0 |
| 1266 | chr22 | 23207000 | 23208000 | 0.000 | 0.000 | 0.029 | 0.000 | IGLV4-3 | 0.22755 | 0.54294 | 0.08726 | 0 | 0 | 0 |
| 1267 | chr22 | 23209000 | 23210000 | 0.000 | 0.000 | 0.088 | 0.027 | IGLV4-3 | 0.27255 | 0.54294 | 0.08726 | 0 | 0 | 0 |
| 1268 | chr22 | 23213000 | 23214000 | 0.000 | 0.000 | 0.074 | 0.027 | IGLV4-3 | 0.15270 | 0.09931 | 0.00058 | 0 | 0 | 1 |
| 1269 | chr22 | 23214000 | 23215000 | 0.000 | 0.000 | 0.044 | 0.000 | IGLV4-3 | 0.25970 | 0.16101 | 0.00208 | 0 | 0 | 0 |
| 1270 | chr22 | 23219000 | 23220000 | 0.000 | 0.000 | 0.059 | 0.000 | IGLV3-1 | 0.10727 | 0.54966 | 0.02537 | 0 | 0 | 1 |
| 1271 | chr22 | 23220000 | 23221000 | 0.000 | 0.066 | 0.147 | 0.014 | IGLV3-1 | 0.65016 | 0.29551 | 0.60730 | 0 | 0 | 1 |
| 1272 | chr22 | 23222000 | 23223000 | 0.083 | 0.149 | 0.544 | 0.432 | IGLV3-1 | 0.00342 | 0.01404 | 0.00003 | 0 | 0 | 1 |
| 1273 | chr22 | 23223000 | 23224000 | 0.000 | 0.000 | 0.118 | 0.027 | IGLV3-1 | 0.23940 | 0.00000 | 0.00000 | 0 | 0 | 1 |
| 1274 | chr22 | 23224000 | 23225000 | 0.028 | 0.000 | 0.029 | 0.000 | IGLV3-1 | 0.04838 | 0.04825 | 0.00004 | 0 | 0 | 1 |
| 1275 | chr22 | 23226000 | 23227000 | 0.028 | 0.056 | 0.412 | 0.257 | IGLV3-1 | 0.22755 | 0.54294 | 0.08726 | 0 | 0 | 0 |
| 1276 | chr22 | 23227000 | 23228000 | 0.000 | 0.019 | 0.309 | 0.095 | IGLL5 | 0.67371 | 0.00001 | 0.00000 | 0 | 0 | 1 |
| 1277 | chr22 | 23228000 | 23229000 | 0.222 | 0.000 | 0.118 | 0.041 | IGLL5 | 0.00152 | 0.00070 | 0.00000 | 0 | 0 | 1 |
| 1278 | chr22 | 23229000 | 23230000 | 0.250 | 0.161 | 0.647 | 0.514 | IGLL5 | 0.11795 | 0.04825 | 0.00004 | 0 | 0 | 1 |
| 1279 | chr22 | 23230000 | 23231000 | 0.000 | 0.155 | 0.647 | 0.514 | IGLL5 | 0.12719 | 0.00007 | 0.00000 | 0 | 0 | 1 |
| 1280 | chr22 | 23231000 | 23232000 | 0.056 | 0.012 | 0.426 | 0.162 | IGLL5 | 0.12719 | 0.00017 | 0.00000 | 0 | 0 | 1 |
| 1281 | chr22 | 23232000 | 23233000 | 0.056 | 0.006 | 0.162 | 0.054 | IGLL5 | 0.00075 | 0.00000 | 0.00000 | 0 | 0 | 1 |
| 1282 | chr22 | 23233000 | 23234000 | 0.111 | 0.000 | 0.147 | 0.041 | IGLJ1 | 0.05410 | 0.01471 | 0.00001 | 0 | 0 | 1 |
| 1283 | chr22 | 23234000 | 23235000 | 0.083 | 0.031 | 0.176 | 0.068 | IGLJ1 | 0.03985 | 0.20979 | 0.00000 | 0 | 0 | 1 |
| 1284 | chr22 | 23235000 | 23236000 | 0.028 | 0.043 | 0.250 | 0.095 | IGLJ1;IGLL5 | 0.66843 | 0.13046 | 0.00035 | 0 | 0 | 1 |
| 1285 | chr22 | 23236000 | 23237000 | 0.023 | 0.006 | 0.103 | 0.054 | IGLJ1;IGLL5 | 0.02356 | 0.12484 | 0.00001 | 0 | 0 | 1 |
| 1286 | chr22 | 23237000 | 23238000 | 0.000 | 0.012 | 0.074 | 0.000 | IGLC1;IGLL5 | 0.35266 | 1.00000 | 0.00009 | 0 | 0 | 1 |
| 1287 | chr22 | 23241000 | 23242000 | 0.111 | 0.050 | 0.147 | 0.108 | IGLJ2 | 0.02326 | 0.66188 | 0.02559 | 0 | 0 | 0 |
| 1288 | chr22 | 23242000 | 23243000 | 0.000 | 0.000 | 0.029 | 0.000 | IGLC2 | 0.61516 | 0.69212 | 0.92792 | 0 | 0 | 0 |
| 1289 | chr22 | 23243000 | 23244000 | 0.000 | 0.012 | 0.015 | 0.014 | IGLC2 | 0.22755 | 0.54294 | 0.08726 | 0 | 0 | 0 |
| 1290 | chr22 | 23245000 | 23246000 | 0.000 | 0.099 | 0.088 | 0.122 | IGLC2 | 1.00000 | 1.00000 | 1.00000 | 0 | 0 | 0 |
| 1291 | chr22 | 23247000 | 23248000 | 0.111 | 0.012 | 0.015 | 0.027 | IGLJ3 | 0.59261 | 0.7481 | 1.00000 | 0 | 0 | 1 |
| 1292 | chr22 | 23248000 | 23249000 | 0.000 | 0.006 | 0.029 | 0.027 | IGLC3 | 1.00000 | 1.00000 | 1.00000 | 0 | 0 | 1 |
| 1293 | chr22 | 23249000 | 23250000 | 0.000 | 0.025 | 0.015 | 0.000 | IGLC3 | 1.00000 | 0.54294 | 0.21104 | 0 | 0 | 0 |
| 1294 | chr22 | 23260000 | 23261000 | 0.000 | 0.012 | 0.015 | 0.014 | IGLJ6 | 0.47887 | 1.00000 | 1.00000 | 0 | 0 | 1 |
| 1295 | chr22 | 23261000 | 23262000 | 0.000 | 0.006 | 0.044 | 0.014 | IGLJ6 | 1.00000 | 1.00000 | 1.00000 | 0 | 0 | 1 |
| 1296 | chr22 | 23263000 | 23264000 | 0.000 | 0.000 | 0.044 | 0.027 | IGLJ7 | 0.34948 | 0.54966 | 0.07959 | 0 | 0 | 1 |
| 1297 | chr22 | 23264000 | 23265000 | 0.000 | 0.000 | 0.044 | 0.000 | IGLC7 | 0.67043 | 0.54966 | 0.07959 | 0 | 0 | 1 |
| 1298 | chr22 | 23265000 | 23266000 | 0.000 | 0.000 | 0.029 | 0.014 | IGLC7 | 0.10727 | 0.54294 | 0.02537 | 0 | 0 | 1 |
| 1299 | chr22 | 23277000 | 23278000 | 0.000 | 0.006 | 0.059 | 0.014 | IGLC7 | 0.64686 | 0.29551 | 0.08726 | 0 | 0 | 0 |
| 1300 | chr22 | 23278000 | 23279000 | 0.000 | 0.000 | 0.029 | 0.027 | IGLC7 | 0.19371 | 0.54294 | 0.02818 | 0 | 0 | 0 |
| 1301 | chr22 | 23282000 | 23283000 | 0.000 | 0.006 | 0.029 | 0.027 | IGLC7 | 0.60686 | 0.01404 | 0.08726 | 0 | 0 | 0 |
| 1302 | chr22 | 23283000 | 23284000 | 0.028 | 0.000 | 0.147 | 0.000 | IGLC7 | 0.01393 | 0.54294 | 0.00003 | 0 | 0 | 1 |
| 1303 | chr22 | 23285000 | 23286000 | 0.000 | 0.000 | 0.029 | 0.060 | IGLC7 | 0.22755 | 1.00000 | 0.08726 | 0 | 0 | 0 |
| 1304 | chr22 | 23524000 | 23525000 | 0.000 | 0.000 | 0.015 | 0.015 | BCR | 0.62160 | 1.00000 | 0.29694 | 0 | 0 | 0 |
| 1305 | chr22 | 23525000 | 23526000 | 0.000 | 0.000 | 0.029 | 0.041 | BCR | 0.60686 | 0.54294 | 0.08726 | 0 | 0 | 0 |
| 1306 | chr22 | 27236000 | 27237000 | 0.028 | 0.000 | 0.029 | 0.000 | CRYBA4 | 0.22755 | 1.00000 | 0.02537 | 0 | 0 | 0 |
| 1307 | chr22 | 29195000 | 29196000 | 0.000 | 0.000 | 0.088 | 0.041 | XBP1 | 0.61070 | 0.09931 | 0.08726 | 0 | 0 | 1 |
| 1308 | chr22 | 29196000 | 29197000 | 0.000 | 0.000 | 0.059 | 0.041 | XBP1 | 0.70990 | 0.29551 | 0.62818 | 0 | 0 | 0 |
| 1309 | chr22 | 29826000 | 29827000 | 0.000 | 0.000 | 0.029 | 0.000 | DRG1 | 0.54294 | 1.00000 | 0.08726 | 0 | 0 | 1 |
| 1310 | chr22 | 32982000 | 32983000 | 0.028 | 0.000 | 0.015 | 0.000 | SYN3 | 0.47887 | 0.54294 | 0.29694 | 0 | 0 | 0 |
| 1311 | chr22 | 39853000 | 39854000 | 0.000 | 0.000 | 0.029 | 0.000 | TAB1 | 0.22755 | 0.54294 | 0.08726 | 0 | 0 | 1 |
| 1312 | chr22 | 39854000 | 39855000 | 0.000 | 0.000 | 0.029 | 0.000 | TAB1 | 0.22755 | 0.54294 | 0.08726 | 0 | 0 | 1 |
| 1313 | chr22 | 43360000 | 43361000 | 0.000 | 0.000 | 0.029 | 0.000 | PACSIN2 | 0.22755 | 0.54294 | 0.08726 | 0 | 0 | 0 |

127

| ID | chr | Pos1 | Pos2 | | | | | Gene | | | | | |
|---|---|---|---|---|---|---|---|---|---|---|---|---|---|
| 1314 | chr22 | 47186000 | 47187000 | 0.000 | 0.000 | 0.029 | 0.000 | TBC1D22A | 0.22755 | 0.54294 | 0.08726 | 0 | 0 |
| 1315 | chr22 | 47738000 | 47739000 | 0.000 | 0.000 | 0.000 | 0.027 | LL22NC03-75H12.2 | 0.49735 | 1.00000 | 1.00000 | 0 | 0 |
| 1316 | chr22 | 50356000 | 50337000 | 0.028 | 0.000 | 0.015 | 0.000 | CRELD2 | 0.47857 | 1.00000 | 0.29694 | 0 | 0 |
| 1317 | chrX | 228000 | 229000 | 0.000 | 0.000 | 0.000 | 0.027 | GTPBP6 | 0.49735 | 1.00000 | 1.00000 | 0 | 0 |
| 1318 | chrX | 1514000 | 1515000 | 0.000 | 0.000 | 0.015 | 0.014 | SLC25A6 | 1.00000 | 1.00000 | 0.29694 | 0 | 0 |
| 1319 | chrX | 1611000 | 1612000 | 0.000 | 0.000 | 0.029 | 0.000 | P2RY8 | 0.22755 | 0.54294 | 0.08726 | 1 | 1 |
| 1320 | chrX | 12993000 | 12994000 | 0.000 | 0.000 | 0.235 | 0.041 | TMSB4X | 0.00091 | 0.00098 | 0.00000 | 1 | 1 |
| 1321 | chrX | 12994000 | 12995000 | 0.000 | 0.000 | 0.221 | 0.027 | TMSB4X | 0.00045 | 0.00107 | 0.00000 | 1 | 0 |
| 1322 | chrX | 13419000 | 13420000 | 0.028 | 0.000 | 0.029 | 0.027 | ATXN3L | 1.00000 | 1.00000 | 0.08726 | 0 | 1 |
| 1323 | chrX | 27031000 | 27032000 | 0.000 | 0.000 | 0.059 | 0.000 | DCAF8L2 | 0.05016 | 0.29551 | 0.00730 | 1 | 0 |
| 1324 | chrX | 32315000 | 32316000 | 0.000 | 0.000 | 0.000 | 0.027 | DMD | 0.49735 | 1.00000 | 1.00000 | 1 | 0 |
| 1325 | chrX | 32317000 | 32318000 | 0.028 | 0.000 | 0.000 | 0.014 | DMD | 1.00000 | 0.34615 | 1.00000 | 1 | 0 |
| 1326 | chrX | 33144000 | 33145000 | 0.000 | 0.000 | 0.029 | 0.014 | DMD | 0.60686 | 0.54294 | 0.08726 | 1 | 0 |
| 1327 | chrX | 33145000 | 33146000 | 0.000 | 0.000 | 0.044 | 0.027 | DMD | 0.67043 | 0.54966 | 0.62537 | 1 | 0 |
| 1328 | chrX | 33146000 | 33147000 | 0.000 | 0.000 | 0.162 | 0.065 | DMD | 0.11404 | 0.61471 | 0.00000 | 1 | 1 |
| 1329 | chrX | 41366000 | 41367000 | 0.000 | 0.000 | 0.015 | 0.027 | CASK | 1.00000 | 1.00000 | 0.29694 | 0 | 0 |
| 1330 | chrX | 42802000 | 42803000 | 0.000 | 0.000 | 0.074 | 0.027 | MAOA | 0.25970 | 0.16101 | 0.00208 | 0 | 1 |
| 1331 | chrX | 48775000 | 48776000 | 0.000 | 0.000 | 0.044 | 0.014 | PIM2 | 0.34948 | 0.54966 | 0.02537 | 1 | 0 |
| 1332 | chrX | 48776000 | 48777000 | 0.000 | 0.000 | 0.029 | 0.014 | PIM2 | 0.60686 | 0.54294 | 0.08726 | 1 | 1 |
| 1333 | chrX | 64071000 | 64072000 | 0.028 | 0.000 | 0.059 | 0.014 | ZC4H2 | 0.19371 | 0.29551 | 0.00730 | 0 | 0 |
| 1334 | chrX | 67030000 | 67031000 | 0.000 | 0.000 | 0.015 | 0.000 | AR | 0.47887 | 1.00000 | 0.29694 | 0 | 0 |
| 1335 | chrX | 80258000 | 80259000 | 0.000 | 0.000 | 0.000 | 0.027 | HMGN5 | 0.49735 | 1.00000 | 1.00000 | 0 | 0 |
| 1336 | chrX | 81172000 | 81173000 | 0.000 | 0.000 | 0.015 | 0.027 | SH3BGRL | 1.00000 | 1.00000 | 0.29694 | 0 | 0 |
| 1337 | chrX | 87742000 | 87743000 | 0.000 | 0.000 | 0.029 | 0.000 | CPXCR1 | 0.22755 | 0.54294 | 0.08726 | 0 | 0 |
| 1338 | chrX | 87831000 | 87832000 | 0.000 | 0.000 | 0.015 | 0.027 | CPXCR1 | 0.49735 | 1.00000 | 1.00000 | 0 | 0 |
| 1339 | chrX | 88263000 | 88264000 | 0.000 | 0.000 | 0.000 | 0.027 | CPXCR1 | 0.49735 | 1.00000 | 1.00000 | 0 | 0 |
| 1340 | chrX | 88455000 | 88459000 | 0.000 | 0.000 | 0.029 | 0.000 | NAP1L3 | 0.22755 | 0.54294 | 0.08726 | 0 | 0 |
| 1341 | chrX | 92647000 | 92648000 | 0.000 | 0.000 | 0.000 | 0.027 | FAM133A | 0.49735 | 1.00000 | 0.29694 | 0 | 0 |
| 1342 | chrX | 93279000 | 93380000 | 0.000 | 0.000 | 0.015 | 0.014 | FAM133A | 1.00000 | 1.00000 | 0.29694 | 0 | 0 |
| 1343 | chrX | 94079000 | 94080000 | 0.000 | 0.000 | 0.015 | 0.014 | FAM133A | 1.00000 | 1.00000 | 0.29694 | 0 | 0 |
| 1344 | chrX | 104066000 | 104067000 | 0.000 | 0.000 | 0.015 | 0.014 | IL1RAPL2 | 1.00000 | 1.00000 | 0.29694 | 0 | 0 |
| 1345 | chrX | 104269000 | 104270000 | 0.000 | 0.000 | 0.015 | 0.014 | IL1RAPL2 | 1.00000 | 1.00000 | 1.00000 | 0 | 0 |
| 1346 | chrX | 106132000 | 106133000 | 0.000 | 0.000 | 0.000 | 0.027 | RIPPLY1 | 0.49735 | 1.00000 | 0.50663 | 0 | 0 |
| 1347 | chrX | 113095000 | 113096000 | 0.000 | 0.066 | 0.015 | 0.000 | HTR2C | 0.47857 | 0.47857 | 0.29694 | 0 | 0 |
| 1348 | chrX | 115676000 | 115677000 | 0.000 | 0.000 | 0.015 | 0.014 | CXorf61 | 1.00000 | 1.00000 | 0.08726 | 0 | 0 |
| 1349 | chrX | 124996000 | 124997000 | 0.000 | 0.000 | 0.029 | 0.000 | DCAF12L2 | 0.22755 | 0.54294 | 0.08726 | 0 | 0 |
| 1350 | chrX | 125708000 | 125709000 | 0.000 | 0.000 | 0.029 | 0.000 | DCAF12L1 | 0.22755 | 0.54294 | 0.29694 | 0 | 0 |
| 1351 | chrX | 128565000 | 128566000 | 0.000 | 0.000 | 0.015 | 0.014 | SMARCA1 | 1.00000 | 1.00000 | 1.00000 | 0 | 0 |
| 1352 | chrX | 129643000 | 129644000 | 0.000 | 0.000 | 0.029 | 0.027 | RBMX2 | 1.00000 | 1.00000 | 1.00000 | 0 | 0 |
| 1353 | chrX | 134903000 | 134904000 | 0.000 | 0.000 | 0.029 | 0.014 | CT45A3;CT45A4; | 0.60686 | 0.54294 | 0.08726 | 0 | 0 |
| 1354 | chrX | 140846000 | 140847000 | 0.000 | 0.000 | 0.029 | 0.000 | SPANXD;SPANXE; | 0.22755 | 0.54294 | 0.08726 | 0 | 0 |
| 1355 | chrX | 143750000 | 143751000 | 0.000 | 0.000 | 0.000 | 0.027 | SPANXN1 | 0.49735 | 1.00000 | 1.00000 | 0 | 0 |
| 1356 | chrX | 145016000 | 145017000 | 0.028 | 0.000 | 0.000 | 0.027 | TMEM257 | 0.49735 | 0.34615 | 1.00000 | 0 | 0 |

Table 2

| # | Chromosome | Region Start | Region End | ABC-subtype | GCB-subtype | ClosestGene | p_ABC_vs_GCB | PreviouslyIdentified |
|---|---|---|---|---|---|---|---|---|
| 1 | chr1 | 756000 | 757000 | 0.040 | 0.000 | AL669831.1 | 1.00000 | 0 |
| 2 | chr1 | 1963000 | 1964000 | 0.000 | 0.000 | GABRD | 1.00000 | 0 |
| 3 | chr1 | 2052000 | 2053000 | 0.000 | 0.040 | PRKCZ | 1.00000 | 0 |
| 4 | chr1 | 3789000 | 3790000 | 0.000 | 0.000 | DFFB | 1.00000 | 0 |
| 5 | chr1 | 6613000 | 6614000 | 0.000 | 0.000 | NOL9 | 1.00000 | 1 |
| 6 | chr1 | 6614000 | 6615000 | 0.120 | 0.040 | NOL9 | 0.60921 | 1 |
| 7 | chr1 | 6661000 | 6662000 | 0.000 | 0.000 | KLHL21 | 1.00000 | 0 |
| 8 | chr1 | 6662000 | 6663000 | 0.120 | 0.000 | KLHL21 | 0.23469 | 0 |
| 9 | chr1 | 9129000 | 9130000 | 0.000 | 0.080 | SLC2A5 | 0.48980 | 0 |
| 10 | chr1 | 10894000 | 10895000 | 0.040 | 0.000 | C1orf127 | 1.00000 | 0 |
| 11 | chr1 | 17019000 | 17020000 | 0.000 | 0.000 | AL137798.1 | 1.00000 | 0 |
| 12 | chr1 | 17231000 | 17232000 | 0.040 | 0.000 | CROCC | 1.00000 | 0 |
| 13 | chr1 | 19935000 | 19936000 | 0.080 | 0.000 | MINOS1-NBL1 | 0.48980 | 0 |
| 14 | chr1 | 21091000 | 21092000 | 0.040 | 0.000 | HP1BP3 | 1.00000 | 0 |
| 15 | chr1 | 23885000 | 23886000 | 0.080 | 0.040 | ID3 | 1.00000 | 1 |
| 16 | chr1 | 28408000 | 28409000 | 0.000 | 0.040 | EYA3 | 1.00000 | 0 |
| 17 | chr1 | 32373000 | 32374000 | 0.000 | 0.040 | PTP4A2 | 1.00000 | 0 |
| 18 | chr1 | 36722000 | 36723000 | 0.040 | 0.000 | THRAP3 | 1.00000 | 0 |
| 19 | chr1 | 46576000 | 46577000 | 0.040 | 0.000 | PIK3R3 | 1.00000 | 0 |
| 20 | chr1 | 51965000 | 51966000 | 0.000 | 0.040 | EPS15 | 1.00000 | 0 |
| 21 | chr1 | 51978000 | 51979000 | 0.040 | 0.080 | EPS15 | 1.00000 | 0 |
| 22 | chr1 | 51983000 | 51984000 | 0.040 | 0.000 | EPS15 | 1.00000 | 0 |
| 23 | chr1 | 72393000 | 72394000 | 0.040 | 0.000 | NEGR1 | 1.00000 | 0 |
| 24 | chr1 | 73719000 | 73720000 | 0.040 | 0.040 | LRRIQ3 | 1.00000 | 0 |
| 25 | chr1 | 77315000 | 77316000 | 0.000 | 0.040 | ST6GALNAC5 | 1.00000 | 0 |
| 26 | chr1 | 81306000 | 81307000 | 0.040 | 0.000 | LPHN2 | 1.00000 | 0 |
| 27 | chr1 | 81527000 | 81528000 | 0.000 | 0.000 | LPHN2 | 1.00000 | 0 |
| 28 | chr1 | 82009000 | 82010000 | 0.000 | 0.000 | LPHN2 | 1.00000 | 0 |
| 29 | chr1 | 84106000 | 84107000 | 0.040 | 0.000 | TTLL7 | 1.00000 | 0 |
| 30 | chr1 | 87524000 | 87525000 | 0.000 | 0.040 | HS2ST1;HS2ST1LOC339524; | 1.00000 | 0 |
| 31 | chr1 | 94551000 | 94552000 | 0.000 | 0.040 | ABCA4 | 1.00000 | 0 |
| 32 | chr1 | 94552000 | 94553000 | 0.000 | 0.040 | ABCA4 | 1.00000 | 0 |
| 33 | chr1 | 103696000 | 103697000 | 0.000 | 0.000 | COL11A1 | 1.00000 | 0 |

| 34 | chr1 | 116979000 | 116980000 | 0.000 | 0.040 | ATP1A1 | 1.00000 | 0 |
|---|---|---|---|---|---|---|---|---|
| 35 | chr1 | 149784000 | 149785000 | 0.040 | 0.040 | HIST2H3D | 1.00000 | 1 |
| 36 | chr1 | 149821000 | 149822000 | 0.040 | 0.000 | HIST2H2AA4 | 1.00000 | 1 |
| 37 | chr1 | 149857000 | 149858000 | 0.000 | 0.040 | HIST2H2BE | 1.00000 | 1 |
| 38 | chr1 | 149858000 | 149859000 | 0.080 | 0.040 | HIST2H2AC;HIST2H2BE; | 1.00000 | 0 |
| 39 | chr1 | 160616000 | 160617000 | 0.040 | 0.040 | SLAMF1 | 1.00000 | 0 |
| 40 | chr1 | 162711000 | 162712000 | 0.040 | 0.000 | DDR2 | 1.00000 | 0 |
| 41 | chr1 | 163684000 | 163685000 | 0.040 | 0.000 | NUF2 | 1.00000 | 0 |
| 42 | chr1 | 167598000 | 167599000 | 0.080 | 0.000 | RCSD1 | 0.48980 | 0 |
| 43 | chr1 | 167599000 | 167600000 | 0.040 | 0.000 | RCSD1 | 1.00000 | 0 |
| 44 | chr1 | 167600000 | 167601000 | 0.040 | 0.040 | RCSD1 | 1.00000 | 0 |
| 45 | chr1 | 174333000 | 174334000 | 0.040 | 0.000 | RABGAP1L | 1.00000 | 0 |
| 46 | chr1 | 187263000 | 187264000 | 0.000 | 0.000 | PLA2G4A | 1.00000 | 0 |
| 47 | chr1 | 187283000 | 187284000 | 0.040 | 0.000 | PLA2G4A | 1.00000 | 0 |
| 48 | chr1 | 187892000 | 187893000 | 0.040 | 0.000 | PLA2G4A | 1.00000 | 0 |
| 49 | chr1 | 195282000 | 195283000 | 0.000 | 0.040 | KCNT2 | 1.00000 | 0 |
| 50 | chr1 | 198591000 | 198592000 | 0.000 | 0.040 | PTPRC | 1.00000 | 0 |
| 51 | chr1 | 198608000 | 198609000 | 0.040 | 0.000 | PTPRC | 1.00000 | 0 |
| 52 | chr1 | 198609000 | 198610000 | 0.080 | 0.000 | PTPRC | 0.48980 | 0 |
| 53 | chr1 | 202004000 | 202005000 | 0.040 | 0.040 | ELF3 | 1.00000 | 0 |
| 54 | chr1 | 203273000 | 203274000 | 0.040 | 0.000 | BTG2 | 1.00000 | 1 |
| 55 | chr1 | 203274000 | 203275000 | 0.160 | 0.160 | BTG2 | 1.00000 | 1 |
| 56 | chr1 | 203275000 | 203276000 | 0.400 | 0.280 | BTG2 | 0.55122 | 1 |
| 57 | chr1 | 203276000 | 203277000 | 0.080 | 0.040 | BTG2 | 1.00000 | 1 |
| 58 | chr1 | 205780000 | 205781000 | 0.000 | 0.000 | SLC41A1 | 1.00000 | 0 |
| 59 | chr1 | 205781000 | 205782000 | 0.000 | 0.000 | SLC41A1 | 1.00000 | 0 |
| 60 | chr1 | 206283000 | 206284000 | 0.000 | 0.040 | CTSE | 1.00000 | 0 |
| 61 | chr1 | 206286000 | 206287000 | 0.040 | 0.000 | CTSE | 1.00000 | 0 |
| 62 | chr1 | 217044000 | 217045000 | 0.040 | 0.000 | ESRRG | 1.00000 | 0 |
| 63 | chr1 | 226924000 | 226925000 | 0.080 | 0.120 | ITPKB | 1.00000 | 1 |
| 64 | chr1 | 226925000 | 226926000 | 0.120 | 0.000 | ITPKB | 0.23469 | 1 |
| 65 | chr1 | 226926000 | 226927000 | 0.120 | 0.000 | ITPKB | 0.23469 | 1 |
| 66 | chr1 | 229974000 | 229975000 | 0.040 | 0.040 | URB2 | 1.00000 | 0 |
| 67 | chr1 | 235131000 | 235132000 | 0.000 | 0.000 | TOMM20 | 1.00000 | 0 |
| 68 | chr1 | 235141000 | 235142000 | 0.040 | 0.000 | TOMM20 | 1.00000 | 0 |

| 69 | chr1 | 238787000 | 238788000 | 0.040 | 0.000 | MTRNR2L11 | 1.00000 | 0 |
|---|---|---|---|---|---|---|---|---|
| 70 | chr1 | 248088000 | 248089000 | 0.040 | 0.000 | OR2T8 | 1.00000 | 0 |
| 71 | chr2 | 630000 | 631000 | 0.000 | 0.000 | TMEM18 | 1.00000 | 0 |
| 72 | chr2 | 1484000 | 1485000 | 0.000 | 0.000 | TPO | 1.00000 | 0 |
| 73 | chr2 | 7991000 | 7992000 | 0.000 | 0.040 | RNF144A | 1.00000 | 0 |
| 74 | chr2 | 12173000 | 12174000 | 0.000 | 0.040 | LPIN1 | 1.00000 | 0 |
| 75 | chr2 | 12175000 | 12176000 | 0.000 | 0.000 | LPIN1 | 1.00000 | 0 |
| 76 | chr2 | 12249000 | 12250000 | 0.000 | 0.040 | LPIN1 | 1.00000 | 0 |
| 77 | chr2 | 14113000 | 14114000 | 0.000 | 0.000 | FAM84A | 1.00000 | 0 |
| 78 | chr2 | 17577000 | 17578000 | 0.000 | 0.040 | RAD51AP2 | 1.00000 | 0 |
| 79 | chr2 | 19253000 | 19254000 | 0.000 | 0.000 | OSR1 | 1.00000 | 0 |
| 80 | chr2 | 24802000 | 24803000 | 0.040 | 0.000 | NCOA1 | 1.00000 | 0 |
| 81 | chr2 | 31478000 | 31479000 | 0.040 | 0.000 | EHD3 | 1.00000 | 0 |
| 82 | chr2 | 41728000 | 41729000 | 0.040 | 0.000 | C2orf91 | 1.00000 | 0 |
| 83 | chr2 | 45404000 | 45405000 | 0.000 | 0.000 | SIX2 | 1.00000 | 0 |
| 84 | chr2 | 47923000 | 47924000 | 0.000 | 0.040 | MSH6 | 1.00000 | 0 |
| 85 | chr2 | 47944000 | 47945000 | 0.000 | 0.000 | MSH6 | 1.00000 | 0 |
| 86 | chr2 | 51360000 | 51361000 | 0.040 | 0.000 | NRXN1 | 1.00000 | 0 |
| 87 | chr2 | 51655000 | 51656000 | 0.000 | 0.000 | NRXN1 | 1.00000 | 0 |
| 88 | chr2 | 56565000 | 56566000 | 0.040 | 0.000 | CCDC85A | 1.00000 | 0 |
| 89 | chr2 | 57800000 | 57801000 | 0.040 | 0.000 | VRK2 | 1.00000 | 0 |
| 90 | chr2 | 60779000 | 60780000 | 0.000 | 0.040 | BCL11A | 1.00000 | 0 |
| 91 | chr2 | 60780000 | 60781000 | 0.080 | 0.000 | BCL11A | 0.48980 | 0 |
| 92 | chr2 | 63802000 | 63803000 | 0.000 | 0.000 | WDPCP | 1.00000 | 0 |
| 93 | chr2 | 63827000 | 63828000 | 0.000 | 0.040 | MDH1 | 1.00000 | 0 |
| 94 | chr2 | 64319000 | 64320000 | 0.000 | 0.040 | PELI1 | 1.00000 | 0 |
| 95 | chr2 | 65593000 | 65594000 | 0.000 | 0.040 | SPRED2 | 1.00000 | 1 |
| 96 | chr2 | 67002000 | 67003000 | 0.040 | 0.040 | MEIS1 | 1.00000 | 0 |
| 97 | chr2 | 70315000 | 70316000 | 0.040 | 0.000 | PCBP1 | 1.00000 | 0 |
| 98 | chr2 | 79502000 | 79503000 | 0.000 | 0.000 | REG3A | 1.00000 | 0 |
| 99 | chr2 | 79644000 | 79645000 | 0.000 | 0.000 | CTNNA2 | 1.00000 | 0 |
| 100 | chr2 | 81818000 | 81819000 | 0.000 | 0.000 | CTNNA2 | 1.00000 | 0 |
| 101 | chr2 | 82310000 | 82311000 | 0.000 | 0.000 | CTNNA2 | 1.00000 | 0 |
| 102 | chr2 | 82948000 | 82949000 | 0.000 | 0.040 | SUCLG1 | 1.00000 | 0 |
| 103 | chr2 | 85335000 | 85336000 | 0.000 | 0.000 | TCF7L1 | 1.00000 | 0 |
| 104 | chr2 | 88905000 | 88906000 | 0.080 | 0.000 | EIF2AK3 | 0.48980 | 0 |

| 105 | chr2 | 88906000 | 88907000 | 0.160 | 0.040 | EIF2AK3 | 0.34868 | 0 |
|---|---|---|---|---|---|---|---|---|
| 106 | chr2 | 88907000 | 88908000 | 0.040 | 0.040 | EIF2AK3 | 1.00000 | 0 |
| 107 | chr2 | 89052000 | 89053000 | 0.000 | 0.080 | RPIA | 0.48980 | 0 |
| 108 | chr2 | 89065000 | 89066000 | 0.000 | 0.000 | RPIA | 1.00000 | 0 |
| 109 | chr2 | 89066000 | 89067000 | 0.040 | 0.000 | RPIA | 1.00000 | 0 |
| 110 | chr2 | 89095000 | 89096000 | 0.000 | 0.040 | RPIA | 1.00000 | 0 |
| 111 | chr2 | 89127000 | 89128000 | 0.120 | 0.080 | IGKC | 1.00000 | 0 |
| 112 | chr2 | 89128000 | 89129000 | 0.160 | 0.160 | IGKC | 1.00000 | 0 |
| 113 | chr2 | 89129000 | 89130000 | 0.120 | 0.000 | IGKC | 0.23469 | 0 |
| 114 | chr2 | 89130000 | 89131000 | 0.080 | 0.000 | IGKC | 0.48980 | 0 |
| 115 | chr2 | 89131000 | 89132000 | 0.040 | 0.040 | IGKC | 1.00000 | 0 |
| 116 | chr2 | 89132000 | 89133000 | 0.040 | 0.000 | IGKC | 1.00000 | 0 |
| 117 | chr2 | 89133000 | 89134000 | 0.000 | 0.040 | IGKC | 1.00000 | 0 |
| 118 | chr2 | 89137000 | 89138000 | 0.000 | 0.040 | IGKC | 1.00000 | 0 |
| 119 | chr2 | 89138000 | 89139000 | 0.040 | 0.000 | IGKC | 1.00000 | 0 |
| 120 | chr2 | 89139000 | 89140000 | 0.000 | 0.040 | IGKC | 1.00000 | 0 |
| 121 | chr2 | 89140000 | 89141000 | 0.040 | 0.120 | IGKC | 0.60921 | 0 |
| 122 | chr2 | 89141000 | 89142000 | 0.080 | 0.120 | IGKC | 1.00000 | 0 |
| 123 | chr2 | 89142000 | 89143000 | 0.040 | 0.200 | IGKC | 0.18946 | 0 |
| 124 | chr2 | 89143000 | 89144000 | 0.000 | 0.080 | IGKC | 0.48980 | 0 |
| 125 | chr2 | 89144000 | 89145000 | 0.040 | 0.040 | IGKC | 1.00000 | 0 |
| 126 | chr2 | 89145000 | 89146000 | 0.040 | 0.000 | IGKC | 1.00000 | 0 |
| 127 | chr2 | 89146000 | 89147000 | 0.000 | 0.000 | IGKC | 1.00000 | 0 |
| 128 | chr2 | 89153000 | 89154000 | 0.000 | 0.000 | IGKC | 1.00000 | 0 |
| 129 | chr2 | 89155000 | 89156000 | 0.080 | 0.080 | IGKC | 1.00000 | 0 |
| 130 | chr2 | 89156000 | 89157000 | 0.120 | 0.000 | IGKC | 0.23469 | 0 |
| 131 | chr2 | 89157000 | 89158000 | 0.240 | 0.160 | IGKC | 0.72520 | 0 |
| 132 | chr2 | 89158000 | 89159000 | 0.240 | 0.280 | IGKC | 1.00000 | 0 |
| 133 | chr2 | 89159000 | 89160000 | 0.360 | 0.640 | IGKJ5 | 0.08874 | 0 |
| 134 | chr2 | 89160000 | 89161000 | 0.320 | 0.680 | IGKJ3;IGKJ4;IGKJ5;KJ5; | 0.02271 | 0 |
| 135 | chr2 | 89161000 | 89162000 | 0.240 | 0.320 | IGKJ1;IGKJ2; | 0.75361 | 0 |
| 136 | chr2 | 89162000 | 89163000 | 0.200 | 0.200 | IGKJI | 1.00000 | 0 |
| 137 | chr2 | 89163000 | 89164000 | 0.120 | 0.240 | IGKJI | 0.46349 | 0 |
| 138 | chr2 | 89164000 | 89165000 | 0.160 | 0.280 | IGKJI | 0.49620 | 0 |
| 139 | chr2 | 89165000 | 89166000 | 0.160 | 0.360 | IGKJI | 0.19633 | 0 |

| 140 | chr2 | 89166000 | 89167000 | 0.000 | 0.040 | IGKJ1 | 1.00000 | 0 |
| 141 | chr2 | 89169000 | 89170000 | 0.000 | 0.040 | IGKJ1 | 1.00000 | 0 |
| 142 | chr2 | 89184000 | 89185000 | 0.000 | 0.000 | IGKV4-1 | 1.00000 | 0 |
| 143 | chr2 | 89185000 | 89186000 | 0.120 | 0.320 | IGKV4-1 | 0.17062 | 0 |
| 144 | chr2 | 89196000 | 89197000 | 0.000 | 0.160 | IGKV5-2 | 0.10986 | 0 |
| 145 | chr2 | 89197000 | 89198000 | 0.000 | 0.040 | IGKV5-2 | 1.00000 | 0 |
| 146 | chr2 | 89214000 | 89215000 | 0.000 | 0.040 | IGKV5-2 | 1.00000 | 0 |
| 147 | chr2 | 89246000 | 89247000 | 0.040 | 0.000 | IGKV1-5 | 1.00000 | 0 |
| 148 | chr2 | 89247000 | 89248000 | 0.160 | 0.000 | IGKV1-5 | 0.10986 | 0 |
| 149 | chr2 | 89248000 | 89249000 | 0.040 | 0.000 | IGKV1-5 | 1.00000 | 0 |
| 150 | chr2 | 89266000 | 89267000 | 0.000 | 0.040 | IGKV1-6 | 1.00000 | 0 |
| 151 | chr2 | 89291000 | 89292000 | 0.040 | 0.040 | IGKV1-8 | 1.00000 | 0 |
| 152 | chr2 | 89292000 | 89293000 | 0.000 | 0.040 | IGKV1-8 | 1.00000 | 0 |
| 153 | chr2 | 89326000 | 89327000 | 0.040 | 0.000 | IGKV3-11 | 1.00000 | 0 |
| 154 | chr2 | 89327000 | 89328000 | 0.040 | 0.000 | IGKV3-11 | 1.00000 | 0 |
| 155 | chr2 | 89442000 | 89443000 | 0.040 | 0.160 | IGKV3-20 | 0.34868 | 0 |
| 156 | chr2 | 89443000 | 89444000 | 0.000 | 0.000 | IGKV3-20 | 1.00000 | 0 |
| 157 | chr2 | 89476000 | 89477000 | 0.000 | 0.000 | IGKV2-24 | 1.00000 | 0 |
| 158 | chr2 | 89513000 | 89514000 | 0.040 | 0.000 | IGKV1-27 | 1.00000 | 0 |
| 159 | chr2 | 89521000 | 89522000 | 0.040 | 0.040 | IGKV2-28 | 1.00000 | 0 |
| 160 | chr2 | 89533000 | 89534000 | 0.080 | 0.040 | IGKV2-30 | 1.00000 | 0 |
| 161 | chr2 | 89534000 | 89535000 | 0.000 | 0.000 | IGKV2-30 | 0.48980 | 0 |
| 162 | chr2 | 89544000 | 89545000 | 0.000 | 0.080 | IGKV2-30 | 0.48980 | 0 |
| 163 | chr2 | 89545000 | 89546000 | 0.040 | 0.000 | IGKV2-30 | 1.00000 | 0 |
| 164 | chr2 | 90259000 | 90260000 | 0.080 | 0.000 | IGKV1D-8 | 1.00000 | 0 |
| 165 | chr2 | 90260000 | 90261000 | 0.120 | 0.000 | IGKV1D-8 | 0.23469 | 0 |
| 166 | chr2 | 96809000 | 96810000 | 0.040 | 0.080 | DUSP2 | 1.00000 | 1 |
| 167 | chr2 | 96810000 | 96811000 | 0.080 | 0.120 | DUSP2 | 1.00000 | 1 |
| 168 | chr2 | 96811000 | 96812000 | 0.000 | 0.080 | DUSP2 | 0.48980 | 1 |
| 169 | chr2 | 98611000 | 98612000 | 0.000 | 0.040 | TMEM131 | 1.00000 | 0 |
| 170 | chr2 | 100757000 | 100758000 | 0.080 | 0.000 | AFF3 | 0.48980 | 0 |
| 171 | chr2 | 100758000 | 100759000 | 0.120 | 0.000 | AFF3 | 0.23469 | 0 |
| 172 | chr2 | 106144000 | 106145000 | 0.000 | 0.080 | FHL2 | 0.48980 | 0 |
| 173 | chr2 | 111878000 | 111879000 | 0.000 | 0.120 | BCL2L11 | 0.23469 | 0 |
| 174 | chr2 | 111879000 | 111880000 | 0.040 | 0.120 | BCL2L11 | 0.60921 | 0 |
| 175 | chr2 | 112305000 | 112306000 | 0.000 | 0.040 | ANAPC1 | 1.00000 | 0 |

| 176 | chr2 | 116234000 | 116235000 | 0.040 | 0.000 | DPP10 | 1.00000 | 0 |
|---|---|---|---|---|---|---|---|---|
| 177 | chr2 | 116439000 | 116440000 | 0.040 | 0.000 | DPP10 | 1.00000 | 0 |
| 178 | chr2 | 124697000 | 124698000 | 0.000 | 0.040 | CNTNAP5 | 1.00000 | 0 |
| 179 | chr2 | 125235000 | 125236000 | 0.000 | 0.000 | CNTNAP5 | 1.00000 | 0 |
| 180 | chr2 | 127538000 | 127539000 | 0.000 | 0.000 | GYPC | 1.00000 | 0 |
| 181 | chr2 | 136874000 | 136875000 | 0.200 | 0.120 | CXCR4 | 0.70194 | 1 |
| 182 | chr2 | 136875000 | 136876000 | 0.240 | 0.240 | CXCR4 | 1.00000 | 1 |
| 183 | chr2 | 136996000 | 136997000 | 0.000 | 0.040 | CXCR4 | 1.00000 | 1 |
| 184 | chr2 | 137082000 | 137083000 | 0.040 | 0.000 | CXCR4 | 1.00000 | 1 |
| 185 | chr2 | 140951000 | 140952000 | 0.040 | 0.000 | LRP1B | 1.00000 | 0 |
| 186 | chr2 | 141335000 | 141336000 | 0.040 | 0.000 | LRP1B | 1.00000 | 0 |
| 187 | chr2 | 141770000 | 141771000 | 0.000 | 0.000 | LRP1B | 1.00000 | 0 |
| 188 | chr2 | 146445000 | 146446000 | 0.000 | 0.000 | ZEB2 | 1.00000 | 0 |
| 189 | chr2 | 146446000 | 146447000 | 0.000 | 0.080 | ZEB2 | 0.48980 | 0 |
| 190 | chr2 | 156443000 | 156444000 | 0.000 | 0.000 | KCNJ3 | 1.00000 | 0 |
| 191 | chr2 | 172590000 | 172591000 | 0.040 | 0.000 | DYNC1I2 | 1.00000 | 0 |
| 192 | chr2 | 176581000 | 176582000 | 0.000 | 0.000 | KIAA1715 | 1.00000 | 0 |
| 193 | chr2 | 179880000 | 179881000 | 0.000 | 0.040 | CCDC141 | 1.00000 | 0 |
| 194 | chr2 | 180358000 | 180359000 | 0.040 | 0.000 | ZNF385B | 1.00000 | 0 |
| 195 | chr2 | 189285000 | 189286000 | 0.040 | 0.000 | GULP1 | 1.00000 | 0 |
| 196 | chr2 | 189432000 | 189433000 | 0.000 | 0.040 | GULP1 | 1.00000 | 0 |
| 197 | chr2 | 194115000 | 194116000 | 0.040 | 0.000 | TMEFF2 | 1.00000 | 0 |
| 198 | chr2 | 197035000 | 197036000 | 0.040 | 0.080 | STK17B | 1.00000 | 0 |
| 199 | chr2 | 197041000 | 197042000 | 0.080 | 0.000 | STK17B | 0.48980 | 0 |
| 200 | chr2 | 215999000 | 216000000 | 0.040 | 0.000 | ABCA12 | 1.00000 | 0 |
| 201 | chr2 | 216973000 | 216974000 | 0.000 | 0.000 | XRCC5 | 1.00000 | 0 |
| 202 | chr2 | 217247000 | 217248000 | 0.000 | 0.000 | 4-Mar-19 | 1.00000 | 0 |
| 203 | chr2 | 225386000 | 225387000 | 0.040 | 0.000 | CUL3 | 1.00000 | 0 |
| 204 | chr2 | 225524000 | 225525000 | 0.000 | 0.040 | CUL3 | 1.00000 | 0 |
| 205 | chr2 | 233478000 | 233479000 | 0.040 | 0.000 | EFHD1 | 1.00000 | 0 |
| 206 | chr2 | 233980000 | 233981000 | 0.000 | 0.080 | INPP5D | 0.48980 | 0 |
| 207 | chr2 | 240641000 | 240642000 | 0.000 | 0.000 | AC093802.1 | 1.00000 | 0 |
| 208 | chr2 | 241125000 | 241126000 | 0.000 | 0.000 | OTOS | 1.00000 | 0 |
| 209 | chr3 | 8739000 | 8740000 | 0.000 | 0.000 | CAV3 | 1.00000 | 0 |
| 210 | chr3 | 16407000 | 16408000 | 0.000 | 0.000 | RFTN1 | 1.00000 | 1 |
| 211 | chr3 | 16409000 | 16410000 | 0.000 | 0.000 | RFTN1 | 1.00000 | 1 |

| 212 | chr3 | 16419000 | 16420000 | 0.040 | 0.080 | RFTN1 | 1.00000 | 1 |
|---|---|---|---|---|---|---|---|---|
| 213 | chr3 | 16472000 | 16473000 | 0.040 | 0.000 | RFTN1 | 1.00000 | 1 |
| 214 | chr3 | 16495000 | 16496000 | 0.000 | 0.080 | RFTN1 | 0.48980 | 1 |
| 215 | chr3 | 16552000 | 16553000 | 0.000 | 0.080 | RFTN1 | 0.48980 | 1 |
| 216 | chr3 | 16554000 | 16555000 | 0.120 | 0.120 | RFTN1 | 1.00000 | 1 |
| 217 | chr3 | 16555000 | 16556000 | 0.000 | 0.040 | RFTN1 | 1.00000 | 1 |
| 218 | chr3 | 21658000 | 21659000 | 0.040 | 0.000 | ZNF385D | 1.00000 | 0 |
| 219 | chr3 | 25691000 | 25692000 | 0.040 | 0.040 | TOP2B | 1.00000 | 0 |
| 220 | chr3 | 31969000 | 31970000 | 0.000 | 0.040 | OSBPL10 | 1.00000 | 1 |
| 221 | chr3 | 31993000 | 31994000 | 0.040 | 0.000 | OSBPL10 | 1.00000 | 1 |
| 222 | chr3 | 32001000 | 32002000 | 0.080 | 0.040 | OSBPL10 | 1.00000 | 1 |
| 223 | chr3 | 32022000 | 32023000 | 0.120 | 0.080 | OSBPL10 | 1.00000 | 1 |
| 224 | chr3 | 32023000 | 32024000 | 0.080 | 0.000 | OSBPL10 | 0.48980 | 1 |
| 225 | chr3 | 50128000 | 50129000 | 0.000 | 0.040 | RBM5 | 1.00000 | 0 |
| 226 | chr3 | 54913000 | 54914000 | 0.040 | 0.000 | CACNA2D3 | 1.00000 | 0 |
| 227 | chr3 | 56074000 | 56075000 | 0.040 | 0.040 | ERC2 | 1.00000 | 0 |
| 228 | chr3 | 59577000 | 59578000 | 0.000 | 0.000 | FHIT | 1.00000 | 0 |
| 229 | chr3 | 60351000 | 60352000 | 0.000 | 0.040 | FHIT | 1.00000 | 0 |
| 230 | chr3 | 60356000 | 60357000 | 0.000 | 0.000 | FHIT | 1.00000 | 0 |
| 231 | chr3 | 60357000 | 60358000 | 0.040 | 0.000 | FHIT | 1.00000 | 0 |
| 232 | chr3 | 60358000 | 60359000 | 0.040 | 0.000 | FHIT | 1.00000 | 0 |
| 233 | chr3 | 60359000 | 60360000 | 0.000 | 0.000 | FHIT | 1.00000 | 0 |
| 234 | chr3 | 60389000 | 60390000 | 0.000 | 0.040 | FHIT | 1.00000 | 0 |
| 235 | chr3 | 60392000 | 60393000 | 0.040 | 0.000 | FHIT | 1.00000 | 0 |
| 236 | chr3 | 60395000 | 60396000 | 0.000 | 0.000 | FHIT | 1.00000 | 0 |
| 237 | chr3 | 60404000 | 60405000 | 0.040 | 0.000 | FHIT | 1.00000 | 0 |
| 238 | chr3 | 60436000 | 60437000 | 0.000 | 0.000 | FHIT | 1.00000 | 0 |
| 239 | chr3 | 60437000 | 60438000 | 0.000 | 0.040 | FHIT | 1.00000 | 0 |
| 240 | chr3 | 60477000 | 60478000 | 0.040 | 0.040 | FHIT | 1.00000 | 0 |
| 241 | chr3 | 60485000 | 60486000 | 0.040 | 0.000 | FHIT | 1.00000 | 0 |
| 242 | chr3 | 60515000 | 60516000 | 0.000 | 0.040 | FHIT | 1.00000 | 0 |
| 243 | chr3 | 60535000 | 60536000 | 0.040 | 0.000 | FHIT | 1.00000 | 0 |
| 244 | chr3 | 60602000 | 60603000 | 0.000 | 0.000 | FHIT | 1.00000 | 0 |
| 245 | chr3 | 60613000 | 60614000 | 0.000 | 0.040 | FHIT | 1.00000 | 0 |
| 246 | chr3 | 60614000 | 60615000 | 0.000 | 0.040 | FHIT | 1.00000 | 0 |
| 247 | chr3 | 60632000 | 60633000 | 0.000 | 0.000 | FHIT | 1.00000 | 0 |

| 248 | chr3 | 60635000 | 60636000 | 0.000 | 0.000 | FHIT | 1.00000 | 0 |
|---|---|---|---|---|---|---|---|---|
| 249 | chr3 | 60640000 | 60641000 | 0.000 | 0.000 | FHIT | 1.00000 | 0 |
| 250 | chr3 | 60647000 | 60648000 | 0.000 | 0.040 | FHIT | 1.00000 | 0 |
| 251 | chr3 | 60648000 | 60649000 | 0.000 | 0.040 | FHIT | 1.00000 | 0 |
| 252 | chr3 | 60652000 | 60653000 | 0.000 | 0.000 | FHIT | 1.00000 | 0 |
| 253 | chr3 | 60660000 | 60661000 | 0.040 | 0.000 | FHIT | 1.00000 | 0 |
| 254 | chr3 | 60665000 | 60666000 | 0.000 | 0.040 | FHIT | 1.00000 | 0 |
| 255 | chr3 | 60666000 | 60667000 | 0.000 | 0.040 | FHIT | 1.00000 | 0 |
| 256 | chr3 | 60671000 | 60672000 | 0.000 | 0.000 | FHIT | 1.00000 | 0 |
| 257 | chr3 | 60673000 | 60674000 | 0.040 | 0.000 | FHIT | 1.00000 | 0 |
| 258 | chr3 | 60675000 | 60676000 | 0.000 | 0.040 | FHIT | 1.00000 | 0 |
| 259 | chr3 | 60678000 | 60679000 | 0.000 | 0.040 | FHIT | 1.00000 | 0 |
| 260 | chr3 | 60683000 | 60684000 | 0.000 | 0.000 | FHIT | 1.00000 | 0 |
| 261 | chr3 | 60684000 | 60685000 | 0.000 | 0.040 | FHIT | 1.00000 | 0 |
| 262 | chr3 | 60688000 | 60689000 | 0.040 | 0.000 | FHIT | 1.00000 | 0 |
| 263 | chr3 | 60717000 | 60718000 | 0.000 | 0.000 | FHIT | 1.00000 | 0 |
| 264 | chr3 | 60740000 | 60741000 | 0.040 | 0.000 | FHIT | 1.00000 | 0 |
| 265 | chr3 | 60774000 | 60775000 | 0.000 | 0.040 | FHIT | 1.00000 | 0 |
| 266 | chr3 | 60792000 | 60793000 | 0.000 | 0.000 | FHIT | 1.00000 | 0 |
| 267 | chr3 | 60806000 | 60807000 | 0.040 | 0.000 | FHIT | 1.00000 | 0 |
| 268 | chr3 | 60812000 | 60813000 | 0.000 | 0.000 | FHIT | 1.00000 | 0 |
| 269 | chr3 | 60860000 | 60861000 | 0.000 | 0.000 | FHIT | 1.00000 | 0 |
| 270 | chr3 | 71551000 | 71552000 | 0.040 | 0.000 | EIF4E3 | 1.00000 | 0 |
| 271 | chr3 | 78274000 | 78275000 | 0.000 | 0.040 | ROBO1 | 1.00000 | 0 |
| 272 | chr3 | 80273000 | 80274000 | 0.000 | 0.000 | ROBO1 | 1.00000 | 0 |
| 273 | chr3 | 83094000 | 83095000 | 0.000 | 0.000 | GBE1 | 1.00000 | 0 |
| 274 | chr3 | 83924000 | 83925000 | 0.000 | 0.000 | CADM2 | 1.00000 | 0 |
| 275 | chr3 | 84293000 | 84294000 | 0.000 | 0.040 | CADM2 | 1.00000 | 0 |
| 276 | chr3 | 85260000 | 85261000 | 0.000 | 0.040 | CADM2 | 1.00000 | 0 |
| 277 | chr3 | 85261000 | 85262000 | 0.000 | 0.000 | CADM2 | 1.00000 | 0 |
| 278 | chr3 | 85799000 | 85800000 | 0.040 | 0.000 | CADM2 | 1.00000 | 0 |
| 279 | chr3 | 86226000 | 86227000 | 0.000 | 0.000 | CADM2 | 1.00000 | 0 |
| 280 | chr3 | 88146000 | 88147000 | 0.040 | 0.000 | CGGBP1 | 1.00000 | 0 |
| 281 | chr3 | 94709000 | 94710000 | 0.000 | 0.000 | NSUN3 | 1.00000 | 0 |
| 282 | chr3 | 95460000 | 95461000 | 0.000 | 0.000 | MTRNR2L12 | 1.00000 | 0 |
| 283 | chr3 | 95724000 | 95725000 | 0.080 | 0.000 | MTRNR2L12 | 0.48980 | 0 |

| | | | | | | | | |
|---|---|---|---|---|---|---|---|---|
| 284 | chr3 | 101569000 | 101570000 | 0.000 | 0.040 | NFKBIZ | 1.00000 | 0 |
| 285 | chr3 | 111851000 | 111852000 | 0.000 | 0.000 | GCSAM | 1.00000 | 0 |
| 286 | chr3 | 111852000 | 111853000 | 0.040 | 0.040 | GCSAM | 1.00000 | 0 |
| 287 | chr3 | 122377000 | 122378000 | 0.080 | 0.040 | PARP14 | 1.00000 | 0 |
| 288 | chr3 | 150478000 | 150479000 | 0.000 | 0.000 | SIAH2 | 1.00000 | 0 |
| 289 | chr3 | 150479000 | 150480000 | 0.000 | 0.040 | SIAH2 | 1.00000 | 0 |
| 290 | chr3 | 150480000 | 150481000 | 0.000 | 0.120 | SIAH2 | 0.23469 | 0 |
| 291 | chr3 | 163237000 | 163238000 | 0.000 | 0.000 | SI | 1.00000 | 0 |
| 292 | chr3 | 163238000 | 163239000 | 0.000 | 0.000 | SI | 1.00000 | 0 |
| 293 | chr3 | 163615000 | 163616000 | 0.040 | 0.040 | SI | 1.00000 | 0 |
| 294 | chr3 | 183270000 | 183271000 | 0.000 | 0.000 | KLHL6 | 1.00000 | 0 |
| 295 | chr3 | 183271000 | 183272000 | 0.000 | 0.040 | KLHL6 | 1.00000 | 0 |
| 296 | chr3 | 183272000 | 183273000 | 0.000 | 0.120 | KLHL6 | 0.23469 | 0 |
| 297 | chr3 | 183273000 | 183274000 | 0.000 | 0.040 | KLHL6 | 1.00000 | 0 |
| 298 | chr3 | 186648000 | 186649000 | 0.000 | 0.040 | ADIPOQ | 1.00000 | 0 |
| 299 | chr3 | 186714000 | 186715000 | 0.080 | 0.160 | ST6GAL1 | 0.66710 | 1 |
| 300 | chr3 | 186715000 | 186716000 | 0.080 | 0.000 | ST6GAL1 | 0.48980 | 1 |
| 301 | chr3 | 186739000 | 186740000 | 0.120 | 0.040 | ST6GAL1 | 0.60921 | 1 |
| 302 | chr3 | 186740000 | 186741000 | 0.160 | 0.080 | ST6GAL1 | 0.66710 | 1 |
| 303 | chr3 | 186742000 | 186743000 | 0.000 | 0.000 | ST6GAL1 | 1.00000 | 1 |
| 304 | chr3 | 186783000 | 186784000 | 0.160 | 0.240 | ST6GAL1 | 0.72520 | 1 |
| 305 | chr3 | 186784000 | 186785000 | 0.040 | 0.040 | ST6GAL1 | 1.00000 | 1 |
| 306 | chr3 | 187458000 | 187459000 | 0.000 | 0.000 | BCL6 | 1.00000 | 1 |
| 307 | chr3 | 187459000 | 187460000 | 0.000 | 0.000 | BCL6 | 1.00000 | 1 |
| 308 | chr3 | 187460000 | 187461000 | 0.040 | 0.040 | BCL6 | 1.00000 | 1 |
| 309 | chr3 | 187461000 | 187462000 | 0.240 | 0.360 | BCL6 | 0.53803 | 1 |
| 310 | chr3 | 187462000 | 187463000 | 0.440 | 0.560 | BCL6 | 0.57214 | 1 |
| 311 | chr3 | 187463000 | 187464000 | 0.360 | 0.440 | BCL6 | 0.77329 | 1 |
| 312 | chr3 | 187464000 | 187465000 | 0.200 | 0.200 | BCL6 | 1.00000 | 1 |
| 313 | chr3 | 187468000 | 187469000 | 0.120 | 0.000 | BCL6 | 0.23469 | 1 |
| 314 | chr3 | 187635000 | 187636000 | 0.040 | 0.000 | BCL6 | 1.00000 | 1 |
| 315 | chr3 | 187636000 | 187637000 | 0.000 | 0.000 | BCL6 | 1.00000 | 1 |
| 316 | chr3 | 187653000 | 187654000 | 0.040 | 0.040 | BCL6 | 1.00000 | 1 |
| 317 | chr3 | 187658000 | 187659000 | 0.000 | 0.040 | BCL6 | 1.00000 | 1 |
| 318 | chr3 | 187660000 | 187661000 | 0.040 | 0.160 | BCL6 | 0.34868 | 1 |
| 319 | chr3 | 187661000 | 187662000 | 0.040 | 0.240 | BCL6 | 0.09828 | 1 |

| | | | | | | | | |
|---|---|---|---|---|---|---|---|---|
| 320 | chr3 | 187664000 | 187665000 | 0.040 | 0.080 | BCL6 | 1.00000 | 1 |
| 321 | chr3 | 187686000 | 187687000 | 0.040 | 0.000 | AC022498.1 | 1.00000 | 0 |
| 322 | chr3 | 187687000 | 187688000 | 0.000 | 0.040 | AC022498.1 | 1.00000 | 0 |
| 323 | chr3 | 187693000 | 187694000 | 0.040 | 0.040 | AC022498.1 | 1.00000 | 0 |
| 324 | chr3 | 187696000 | 187697000 | 0.040 | 0.000 | AC022498.1 | 1.00000 | 0 |
| 325 | chr3 | 187697000 | 187698000 | 0.040 | 0.000 | AC022498.1 | 1.00000 | 0 |
| 326 | chr3 | 187803000 | 187804000 | 0.000 | 0.000 | AC022498.1 | 1.00000 | 0 |
| 327 | chr3 | 187806000 | 187807000 | 0.080 | 0.080 | AC022498.1 | 1.00000 | 0 |
| 328 | chr3 | 187957000 | 187958000 | 0.120 | 0.160 | AC022498.1 | 1.00000 | 0 |
| 329 | chr3 | 187958000 | 187959000 | 0.240 | 0.280 | AC022498.1 | 1.00000 | 0 |
| 330 | chr3 | 187959000 | 187960000 | 0.120 | 0.040 | AC022498.1 | 0.60921 | 0 |
| 331 | chr3 | 187960000 | 187961000 | 0.000 | 0.040 | AC022498.1 | 1.00000 | 0 |
| 332 | chr3 | 188222000 | 188223000 | 0.000 | 0.000 | LPP | 1.00000 | 0 |
| 333 | chr3 | 188298000 | 188299000 | 0.040 | 0.000 | LPP | 1.00000 | 0 |
| 334 | chr3 | 188299000 | 188300000 | 0.080 | 0.080 | LPP | 1.00000 | 0 |
| 335 | chr3 | 188471000 | 188472000 | 0.120 | 0.240 | LPP | 0.46349 | 0 |
| 336 | chr3 | 188472000 | 188473000 | 0.000 | 0.080 | LPP | 0.48980 | 0 |
| 337 | chr4 | 50000 | 51000 | 0.080 | 0.000 | ZNF595;ZNF718; | 0.48980 | 0 |
| 338 | chr4 | 51000 | 52000 | 0.120 | 0.040 | ZNF595;ZNF718; | 0.60921 | 0 |
| 339 | chr4 | 54000 | 55000 | 0.080 | 0.000 | ZNF595;ZNF718; | 0.48980 | 0 |
| 340 | chr4 | 290000 | 291000 | 0.000 | 0.000 | ZNF732 | 1.00000 | 0 |
| 341 | chr4 | 385000 | 386000 | 0.080 | 0.000 | ZNF141 | 0.48980 | 0 |
| 342 | chr4 | 550000 | 551000 | 0.000 | 0.000 | PIGG | 1.00000 | 0 |
| 343 | chr4 | 2707000 | 2708000 | 0.000 | 0.040 | FAM193A | 1.00000 | 0 |
| 344 | chr4 | 5206000 | 5207000 | 0.080 | 0.000 | STK32B | 0.48980 | 0 |
| 345 | chr4 | 25863000 | 25864000 | 0.080 | 0.040 | SEL1L3 | 1.00000 | 0 |
| 346 | chr4 | 25864000 | 25865000 | 0.000 | 0.040 | SEL1L3 | 1.00000 | 0 |
| 347 | chr4 | 25865000 | 25866000 | 0.040 | 0.000 | SEL1L3 | 1.00000 | 0 |
| 348 | chr4 | 29657000 | 29658000 | 0.040 | 0.000 | PCDH7 | 1.00000 | 0 |
| 349 | chr4 | 30356000 | 30357000 | 0.040 | 0.000 | PCDH7 | 1.00000 | 0 |
| 350 | chr4 | 33418000 | 33419000 | 0.000 | 0.000 | PCDH7 | 1.00000 | 0 |
| 351 | chr4 | 33449000 | 33450000 | 0.000 | 0.040 | PCDH7 | 1.00000 | 0 |
| 352 | chr4 | 39348000 | 39349000 | 0.000 | 0.040 | RFC1 | 1.00000 | 0 |

| | | | | | | | | |
|---|---|---|---|---|---|---|---|---|
| 353 | chr4 | 39974000 | 39975000 | 0.000 | 0.000 | PDS5A | 1.00000 | 0 |
| 354 | chr4 | 40194000 | 40195000 | 0.000 | 0.120 | N4BP2 | 0.23469 | 0 |
| 355 | chr4 | 40195000 | 40196000 | 0.000 | 0.040 | N4BP2 | 1.00000 | 0 |
| 356 | chr4 | 40196000 | 40197000 | 0.040 | 0.000 | N4BP2 | 1.00000 | 0 |
| 357 | chr4 | 40197000 | 40198000 | 0.000 | 0.000 | N4BP2 | 1.00000 | 0 |
| 358 | chr4 | 40198000 | 40199000 | 0.120 | 0.080 | N4BP2 | 1.00000 | 0 |
| 359 | chr4 | 40199000 | 40200000 | 0.280 | 0.240 | N4BP2 | 1.00000 | 0 |
| 360 | chr4 | 40200000 | 40201000 | 0.080 | 0.080 | RHOH | 1.00000 | 1 |
| 361 | chr4 | 40201000 | 40202000 | 0.120 | 0.120 | RHOH | 1.00000 | 1 |
| 362 | chr4 | 40202000 | 40203000 | 0.080 | 0.000 | RHOH | 0.48980 | 1 |
| 363 | chr4 | 40204000 | 40205000 | 0.000 | 0.040 | RHOH | 1.00000 | 1 |
| 364 | chr4 | 45308000 | 45309000 | 0.000 | 0.000 | GNPDA2 | 1.00000 | 0 |
| 365 | chr4 | 46360000 | 46361000 | 0.000 | 0.040 | GABRA2 | 1.00000 | 0 |
| 366 | chr4 | 62375000 | 62376000 | 0.000 | 0.000 | LPHN3 | 1.00000 | 0 |
| 367 | chr4 | 62530000 | 62531000 | 0.000 | 0.000 | LPHN3 | 1.00000 | 0 |
| 368 | chr4 | 62911000 | 62912000 | 0.000 | 0.040 | LPHN3 | 1.00000 | 0 |
| 369 | chr4 | 63120000 | 63121000 | 0.040 | 0.040 | LPHN3 | 1.00000 | 0 |
| 370 | chr4 | 64015000 | 64016000 | 0.000 | 0.000 | LPHN3 | 1.00000 | 0 |
| 371 | chr4 | 65038000 | 65039000 | 0.040 | 0.000 | TECRL | 1.00000 | 0 |
| 372 | chr4 | 65165000 | 65166000 | 0.000 | 0.040 | TECRL | 1.00000 | 0 |
| 373 | chr4 | 65966000 | 65967000 | 0.000 | 0.040 | EPHA5 | 1.00000 | 0 |
| 374 | chr4 | 66827000 | 66828000 | 0.000 | 0.080 | EPHA5 | 0.48980 | 0 |
| 375 | chr4 | 71531000 | 71532000 | 0.000 | 0.040 | IGJ | 1.00000 | 0 |
| 376 | chr4 | 71532000 | 71533000 | 0.000 | 0.000 | IGJ | 1.00000 | 0 |
| 377 | chr4 | 74456000 | 74457000 | 0.040 | 0.000 | RASSF6 | 1.00000 | 0 |
| 378 | chr4 | 74483000 | 74484000 | 0.040 | 0.000 | RASSF6 | 1.00000 | 0 |
| 379 | chr4 | 74484000 | 74485000 | 0.040 | 0.000 | RASSF6 | 1.00000 | 0 |
| 380 | chr4 | 74485000 | 74486000 | 0.120 | 0.000 | RASSF6 | 0.23469 | 0 |
| 381 | chr4 | 91886000 | 91887000 | 0.040 | 0.000 | CCSER1 | 1.00000 | 0 |
| 382 | chr4 | 92787000 | 92788000 | 0.000 | 0.040 | CCSER1 | 1.00000 | 0 |
| 383 | chr4 | 113206000 | 113207000 | 0.000 | 0.000 | TIFA | 1.00000 | 0 |
| 384 | chr4 | 114466000 | 114467000 | 0.000 | 0.000 | CAMK2D | 1.00000 | 0 |
| 385 | chr4 | 114681000 | 114682000 | 0.000 | 0.080 | CAMK2D | 0.48980 | 0 |
| 386 | chr4 | 117928000 | 117929000 | 0.040 | 0.000 | TRAM1L1 | 1.00000 | 0 |
| 387 | chr4 | 123637000 | 123638000 | 0.000 | 0.000 | BBS12 | 1.00000 | 0 |
| 388 | chr4 | 125227000 | 125228000 | 0.040 | 0.000 | ANKRD50 | 1.00000 | 0 |

| | | | | | | | | |
|---|---|---|---|---|---|---|---|---|
| 389 | chr4 | 127371000 | 127372000 | 0.000 | 0.000 | FAT4 | 1.00000 | 0 |
| 390 | chr4 | 133455000 | 133456000 | 0.000 | 0.000 | PCDH10 | 1.00000 | 0 |
| 391 | chr4 | 134538000 | 134539000 | 0.000 | 0.040 | PCDH10 | 1.00000 | 0 |
| 392 | chr4 | 134743000 | 134744000 | 0.040 | 0.040 | PABPC4L | 1.00000 | 0 |
| 393 | chr4 | 134867000 | 134868000 | 0.000 | 0.000 | PABPC4L | 1.00000 | 0 |
| 394 | chr4 | 134949000 | 134950000 | 0.080 | 0.000 | PABPC4L | 0.48980 | 0 |
| 395 | chr4 | 135064000 | 135065000 | 0.040 | 0.000 | PABPC4L | 1.00000 | 0 |
| 396 | chr4 | 135077000 | 135078000 | 0.000 | 0.000 | PABPC4L | 1.00000 | 0 |
| 397 | chr4 | 136799000 | 136800000 | 0.000 | 0.000 | PCDH18 | 1.00000 | 0 |
| 398 | chr4 | 136867000 | 136868000 | 0.000 | 0.040 | PCDH18 | 1.00000 | 0 |
| 399 | chr4 | 140236000 | 140237000 | 0.040 | 0.000 | NAA15 | 1.00000 | 0 |
| 400 | chr4 | 151723000 | 151724000 | 0.000 | 0.000 | LRBA | 1.00000 | 0 |
| 401 | chr4 | 151950000 | 151951000 | 0.000 | 0.000 | LRBA | 1.00000 | 0 |
| 402 | chr4 | 152125000 | 152126000 | 0.040 | 0.040 | SH3D19 | 1.00000 | 0 |
| 403 | chr4 | 157246000 | 157247000 | 0.040 | 0.000 | CTSO | 1.00000 | 0 |
| 404 | chr4 | 164532000 | 164533000 | 0.000 | 0.000 | 1-Mar-19 | 1.00000 | 0 |
| 405 | chr4 | 178732000 | 178733000 | 0.040 | 0.040 | AGA | 1.00000 | 0 |
| 406 | chr4 | 178885000 | 178886000 | 0.040 | 0.000 | AGA | 1.00000 | 0 |
| 407 | chr4 | 179898000 | 179899000 | 0.000 | 0.040 | AGA | 1.00000 | 0 |
| 408 | chr4 | 180885000 | 180886000 | 0.040 | 0.000 | TENM3 | 1.00000 | 0 |
| 409 | chr4 | 181554000 | 181555000 | 0.040 | 0.040 | TENM3 | 1.00000 | 0 |
| 410 | chr4 | 182122000 | 182123000 | 0.000 | 0.040 | TENM3 | 1.00000 | 0 |
| 411 | chr5 | 436000 | 437000 | 0.000 | 0.000 | AHRR | 1.00000 | 0 |
| 412 | chr5 | 3982000 | 3983000 | 0.040 | 0.000 | IRX1 | 1.00000 | 0 |
| 413 | chr5 | 17218000 | 17219000 | 0.040 | 0.000 | BASP1 | 1.00000 | 0 |
| 414 | chr5 | 17219000 | 17220000 | 0.080 | 0.000 | BASP1 | 0.48980 | 0 |
| 415 | chr5 | 18514000 | 18515000 | 0.040 | 0.000 | CDH18 | 1.00000 | 0 |
| 416 | chr5 | 22356000 | 22357000 | 0.040 | 0.000 | CDH12 | 1.00000 | 0 |
| 417 | chr5 | 22517000 | 22518000 | 0.040 | 0.000 | CDH12 | 1.00000 | 0 |
| 418 | chr5 | 24632000 | 24633000 | 0.000 | 0.000 | CDH10 | 1.00000 | 0 |
| 419 | chr5 | 25275000 | 25276000 | 0.000 | 0.040 | CDH10 | 1.00000 | 0 |
| 420 | chr5 | 25541000 | 25542000 | 0.000 | 0.000 | CDH10 | 1.00000 | 0 |
| 421 | chr5 | 26119000 | 26120000 | 0.000 | 0.080 | CDH9 | 0.48980 | 0 |
| 422 | chr5 | 26450000 | 26451000 | 0.000 | 0.000 | CDH9 | 1.00000 | 0 |
| 423 | chr5 | 29224000 | 29225000 | 0.080 | 0.000 | CDH6 | 0.48980 | 0 |
| 424 | chr5 | 29492000 | 29493000 | 0.000 | 0.000 | CDH6 | 1.00000 | 0 |

| 425 | chr5 | 29648000 | 29649000 | 0.000 | 0.000 | CDH6 | 1.00000 | 0 |
|---|---|---|---|---|---|---|---|---|
| 426 | chr5 | 51521000 | 51522000 | 0.000 | 0.040 | CTD-2203A3.1 | 1.00000 | 0 |
| 427 | chr5 | 83841000 | 83842000 | 0.040 | 0.000 | EDIL3 | 1.00000 | 0 |
| 428 | chr5 | 88177000 | 88178000 | 0.040 | 0.000 | MEF2C | 1.00000 | 0 |
| 429 | chr5 | 88178000 | 88179000 | 0.040 | 0.000 | MEF2C | 1.00000 | 0 |
| 430 | chr5 | 91417000 | 91418000 | 0.000 | 0.000 | ARRDC3 | 1.00000 | 0 |
| 431 | chr5 | 103678000 | 103679000 | 0.040 | 0.000 | NUDT12 | 1.00000 | 0 |
| 432 | chr5 | 123696000 | 123697000 | 0.000 | 0.000 | ZNF608 | 1.00000 | 1 |
| 433 | chr5 | 124079000 | 124080000 | 0.000 | 0.040 | ZNF608 | 1.00000 | 1 |
| 434 | chr5 | 124080000 | 124081000 | 0.040 | 0.000 | ZNF608 | 1.00000 | 1 |
| 435 | chr5 | 127594000 | 127595000 | 0.000 | 0.040 | FBN2 | 1.00000 | 0 |
| 436 | chr5 | 127875000 | 127876000 | 0.000 | 0.000 | FBN2 | 1.00000 | 0 |
| 437 | chr5 | 131825000 | 131826000 | 0.120 | 0.040 | IRF1 | 0.60921 | 0 |
| 438 | chr5 | 131826000 | 131827000 | 0.040 | 0.040 | IRF1 | 1.00000 | 0 |
| 439 | chr5 | 149791000 | 149792000 | 0.160 | 0.240 | CD74 | 0.72520 | 1 |
| 440 | chr5 | 149792000 | 149793000 | 0.040 | 0.080 | CD74 | 1.00000 | 1 |
| 441 | chr5 | 158380000 | 158381000 | 0.000 | 0.080 | EBF1 | 0.48980 | 0 |
| 442 | chr5 | 158479000 | 158480000 | 0.000 | 0.000 | EBF1 | 1.00000 | 0 |
| 443 | chr5 | 158526000 | 158527000 | 0.040 | 0.080 | EBF1 | 1.00000 | 0 |
| 444 | chr5 | 158527000 | 158528000 | 0.040 | 0.040 | EBF1 | 1.00000 | 0 |
| 445 | chr5 | 158528000 | 158529000 | 0.040 | 0.000 | EBF1 | 1.00000 | 0 |
| 446 | chr5 | 164247000 | 164248000 | 0.040 | 0.040 | MAT2B | 1.00000 | 0 |
| 447 | chr5 | 164441000 | 164442000 | 0.000 | 0.000 | MAT2B | 1.00000 | 0 |
| 448 | chr5 | 165932000 | 165933000 | 0.000 | 0.000 | TENM2 | 1.00000 | 0 |
| 449 | chr5 | 173300000 | 173301000 | 0.000 | 0.000 | CPEB4 | 1.00000 | 0 |
| 450 | chr5 | 179166000 | 179167000 | 0.040 | 0.040 | MAML1 | 1.00000 | 0 |
| 451 | chr5 | 180102000 | 180103000 | 0.040 | 0.000 | FLT4 | 1.00000 | 0 |
| 452 | chr6 | 392000 | 393000 | 0.120 | 0.080 | IRF4 | 1.00000 | 1 |
| 453 | chr6 | 393000 | 394000 | 0.080 | 0.080 | IRF4 | 1.00000 | 1 |
| 454 | chr6 | 14118000 | 14119000 | 0.160 | 0.440 | CD83 | 0.06222 | 1 |
| 455 | chr6 | 14119000 | 14120000 | 0.000 | 0.120 | CD83 | 0.23469 | 1 |
| 456 | chr6 | 18111000 | 18112000 | 0.000 | 0.080 | NHLRC1 | 0.48980 | 0 |
| 457 | chr6 | 18387000 | 18388000 | 0.000 | 0.040 | RNF144B | 1.00000 | 1 |
| 458 | chr6 | 18388000 | 18389000 | 0.000 | 0.040 | RNF144B | 1.00000 | 1 |
| 459 | chr6 | 19573000 | 19574000 | 0.040 | 0.040 | ID4 | 1.00000 | 0 |
| 460 | chr6 | 22873000 | 22874000 | 0.040 | 0.000 | HDGFL1 | 1.00000 | 0 |

| | | | | | | | | |
|---|---|---|---|---|---|---|---|---|
| 461 | chr6 | 26031000 | 26032000 | 0.000 | 0.040 | HIST1H3B | 1.00000 | 1 |
| 462 | chr6 | 26032000 | 26033000 | 0.000 | 0.040 | HIST1H3B | 1.00000 | 1 |
| 463 | chr6 | 26056000 | 26057000 | 0.120 | 0.040 | HIST1H1C | 0.60921 | 1 |
| 464 | chr6 | 26123000 | 26124000 | 0.120 | 0.040 | HIST1H2BC | 0.60921 | 1 |
| 465 | chr6 | 26124000 | 26125000 | 0.120 | 0.080 | HIST1H2AC;HIST1H2BC; | 1.00000 | 0 |
| 466 | chr6 | 26125000 | 26126000 | 0.000 | 0.040 | HIST1H2AC | 1.00000 | 1 |
| 467 | chr6 | 26156000 | 26157000 | 0.120 | 0.080 | HIST1H1E | 1.00000 | 1 |
| 468 | chr6 | 26157000 | 26158000 | 0.080 | 0.040 | HIST1H1E | 1.00000 | 1 |
| 469 | chr6 | 26216000 | 26217000 | 0.040 | 0.040 | HIST1H2BG | 1.00000 | 1 |
| 470 | chr6 | 26234000 | 26235000 | 0.080 | 0.040 | HIST1H1D | 1.00000 | 0 |
| 471 | chr6 | 27101000 | 27102000 | 0.040 | 0.040 | HIST1H2AG | 1.00000 | 1 |
| 472 | chr6 | 27114000 | 27115000 | 0.080 | 0.040 | HIST1H2AH;HIST1H2BK; | 1.00000 | 0 |
| 473 | chr6 | 27792000 | 27793000 | 0.120 | 0.040 | HIST1H4J | 0.60921 | 0 |
| 474 | chr6 | 27833000 | 27834000 | 0.040 | 0.000 | HIST1H2AL | 1.00000 | 1 |
| 475 | chr6 | 27860000 | 27861000 | 0.000 | 0.080 | HIST1H2AM | 0.48980 | 1 |
| 476 | chr6 | 27861000 | 27862000 | 0.000 | 0.040 | HIST1H2BO | 1.00000 | 1 |
| 477 | chr6 | 29778000 | 29779000 | 0.000 | 0.040 | LOC554223 | 1.00000 | 0 |
| 478 | chr6 | 29780000 | 29781000 | 0.040 | 0.000 | HLA-G | 1.00000 | 0 |
| 479 | chr6 | 29911000 | 29912000 | 0.080 | 0.040 | HLA-A | 1.00000 | 0 |
| 480 | chr6 | 29927000 | 29928000 | 0.040 | 0.000 | HLA-A | 1.00000 | 0 |
| 481 | chr6 | 31324000 | 31325000 | 0.040 | 0.040 | HLA-B | 1.00000 | 1 |
| 482 | chr6 | 31325000 | 31326000 | 0.000 | 0.000 | HLA-B | 1.00000 | 1 |
| 483 | chr6 | 31543000 | 31544000 | 0.080 | 0.000 | TNF | 0.48980 | 1 |
| 484 | chr6 | 31549000 | 31550000 | 0.200 | 0.240 | LTB | 1.00000 | 1 |
| 485 | chr6 | 31550000 | 31551000 | 0.040 | 0.040 | LTB | 1.00000 | 1 |
| 486 | chr6 | 32440000 | 32441000 | 0.120 | 0.000 | HLA-DRA | 0.23469 | 0 |
| 487 | chr6 | 32451000 | 32452000 | 0.040 | 0.000 | HLA-DRB5 | 1.00000 | 0 |
| 488 | chr6 | 32452000 | 32453000 | 0.080 | 0.000 | HLA-DRB5 | 0.48980 | 0 |
| 489 | chr6 | 32455000 | 32456000 | 0.040 | 0.040 | HLA-DRB5 | 1.00000 | 0 |
| 490 | chr6 | 32457000 | 32458000 | 0.000 | 0.000 | HLA-DRB5 | 1.00000 | 0 |
| 491 | chr6 | 32498000 | 32499000 | 0.000 | 0.040 | HLA-DRB5 | 1.00000 | 0 |
| 492 | chr6 | 32505000 | 32506000 | 0.040 | 0.000 | HLA-DRB5 | 1.00000 | 0 |
| 493 | chr6 | 32511000 | 32512000 | 0.000 | 0.000 | HLA-DRB5 | 1.00000 | 0 |
| 494 | chr6 | 32522000 | 32523000 | 0.040 | 0.000 | HLA-DRB1 | 1.00000 | 0 |

| | | | | | | | | |
|---|---|---|---|---|---|---|---|---|
| 495 | chr6 | 32525000 | 32526000 | 0.040 | 0.000 | HLA-DRB1 | 1.00000 | 0 |
| 496 | chr6 | 32526000 | 32527000 | 0.000 | 0.000 | HLA-DRB1 | 1.00000 | 0 |
| 497 | chr6 | 32527000 | 32528000 | 0.000 | 0.000 | HLA-DRB1 | 1.00000 | 0 |
| 498 | chr6 | 32548000 | 32549000 | 0.000 | 0.000 | HLA-DRB1 | 1.00000 | 0 |
| 499 | chr6 | 32552000 | 32553000 | 0.040 | 0.000 | HLA-DRB1 | 1.00000 | 0 |
| 500 | chr6 | 32557000 | 32558000 | 0.000 | 0.080 | HLA-DRB1 | 0.48980 | 0 |
| 501 | chr6 | 32609000 | 32610000 | 0.000 | 0.040 | HLA-DQA1 | 1.00000 | 0 |
| 502 | chr6 | 32630000 | 32631000 | 0.000 | 0.040 | HLA-DQB1 | 1.00000 | 0 |
| 503 | chr6 | 32632000 | 32633000 | 0.080 | 0.040 | HLA-DQB1 | 1.00000 | 0 |
| 504 | chr6 | 32727000 | 32728000 | 0.040 | 0.040 | HLA-DQB2 | 1.00000 | 0 |
| 505 | chr6 | 32729000 | 32730000 | 0.000 | 0.040 | HLA-DQB2 | 1.00000 | 0 |
| 506 | chr6 | 33048000 | 33049000 | 0.000 | 0.040 | HLA-DPB1 | 1.00000 | 0 |
| 507 | chr6 | 34179000 | 34180000 | 0.000 | 0.040 | HMGA1 | 1.00000 | 0 |
| 508 | chr6 | 37138000 | 37139000 | 0.200 | 0.200 | PIM1 | 1.00000 | 1 |
| 509 | chr6 | 37139000 | 37140000 | 0.120 | 0.120 | PIM1 | 1.00000 | 1 |
| 510 | chr6 | 37140000 | 37141000 | 0.040 | 0.000 | PIM1 | 1.00000 | 1 |
| 511 | chr6 | 58001000 | 58002000 | 0.040 | 0.000 | PRIM2 | 1.00000 | 0 |
| 512 | chr6 | 67923000 | 67924000 | 0.040 | 0.000 | BAI3 | 1.00000 | 0 |
| 513 | chr6 | 77256000 | 77257000 | 0.040 | 0.000 | IMPG1 | 1.00000 | 0 |
| 514 | chr6 | 81437000 | 81438000 | 0.040 | 0.000 | BCKDHB | 1.00000 | 0 |
| 515 | chr6 | 88468000 | 88469000 | 0.000 | 0.040 | AKIRIN2 | 1.00000 | 0 |
| 516 | chr6 | 88630000 | 88631000 | 0.040 | 0.080 | SPACA1 | 1.00000 | 0 |
| 517 | chr6 | 88876000 | 88877000 | 0.000 | 0.000 | CNR1 | 1.00000 | 0 |
| 518 | chr6 | 89323000 | 89324000 | 0.000 | 0.000 | RNGTT | 1.00000 | 0 |
| 519 | chr6 | 89338000 | 89339000 | 0.080 | 0.000 | RNGTT | 0.48980 | 0 |
| 520 | chr6 | 89348000 | 89349000 | 0.080 | 0.000 | RNGTT | 0.48980 | 0 |
| 521 | chr6 | 89470000 | 89471000 | 0.080 | 0.000 | RNGTT | 0.48980 | 0 |
| 522 | chr6 | 89471000 | 89472000 | 0.000 | 0.000 | RNGTT | 1.00000 | 0 |
| 523 | chr6 | 90061000 | 90062000 | 0.040 | 0.040 | UBE2J1 | 1.00000 | 1 |
| 524 | chr6 | 90062000 | 90063000 | 0.040 | 0.000 | UBE2J1 | 1.00000 | 1 |
| 525 | chr6 | 90994000 | 90995000 | 0.000 | 0.080 | MAP3K7 | 0.48980 | 0 |
| 526 | chr6 | 91004000 | 91005000 | 0.040 | 0.040 | MAP3K7 | 1.00000 | 0 |
| 527 | chr6 | 91005000 | 91006000 | 0.120 | 0.280 | MAP3K7 | 0.28902 | 0 |
| 528 | chr6 | 91006000 | 91007000 | 0.040 | 0.120 | MAP3K7 | 0.60921 | 0 |
| 529 | chr6 | 91007000 | 91008000 | 0.000 | 0.040 | MAP3K7 | 1.00000 | 0 |
| 530 | chr6 | 94822000 | 94823000 | 0.000 | 0.040 | EPHA7 | 1.00000 | 0 |

143

| | | | | | | | | |
|---|---|---|---|---|---|---|---|---|
| 531 | chr6 | 107704000 | 107705000 | 0.000 | 0.000 | PDSS2 | 1.00000 | 0 |
| 532 | chr6 | 112885000 | 112886000 | 0.040 | 0.000 | RFPL4B | 1.00000 | 0 |
| 533 | chr6 | 118244000 | 118245000 | 0.040 | 0.000 | SLC35F1 | 1.00000 | 0 |
| 534 | chr6 | 121288000 | 121289000 | 0.000 | 0.000 | C6orf170 | 1.00000 | 0 |
| 535 | chr6 | 121489000 | 121490000 | 0.000 | 0.080 | C6orf170 | 0.48980 | 0 |
| 536 | chr6 | 123504000 | 123505000 | 0.040 | 0.000 | TRDN | 1.00000 | 0 |
| 537 | chr6 | 127313000 | 127314000 | 0.040 | 0.000 | RSPO3 | 1.00000 | 0 |
| 538 | chr6 | 133785000 | 133786000 | 0.080 | 0.000 | EYA4 | 0.48980 | 0 |
| 539 | chr6 | 134491000 | 134492000 | 0.000 | 0.080 | SGK1 | 0.48980 | 1 |
| 540 | chr6 | 134492000 | 134493000 | 0.080 | 0.040 | SGK1 | 1.00000 | 1 |
| 541 | chr6 | 134493000 | 134494000 | 0.040 | 0.080 | SGK1 | 1.00000 | 1 |
| 542 | chr6 | 134494000 | 134495000 | 0.040 | 0.080 | SGK1 | 1.00000 | 1 |
| 543 | chr6 | 134495000 | 134496000 | 0.160 | 0.280 | SGK1 | 0.49620 | 1 |
| 544 | chr6 | 134496000 | 134497000 | 0.000 | 0.200 | SGK1 | 0.05015 | 1 |
| 545 | chr6 | 142046000 | 142047000 | 0.000 | 0.080 | NMBR | 0.48980 | 0 |
| 546 | chr6 | 147860000 | 147861000 | 0.000 | 0.040 | SAMD5 | 1.00000 | 0 |
| 547 | chr6 | 150954000 | 150955000 | 0.040 | 0.040 | PLEKHG1 | 1.00000 | 0 |
| 548 | chr6 | 159238000 | 159239000 | 0.000 | 0.080 | EZR | 0.48980 | 0 |
| 549 | chr6 | 159239000 | 159240000 | 0.040 | 0.000 | EZR | 1.00000 | 0 |
| 550 | chr6 | 159240000 | 159241000 | 0.040 | 0.000 | EZR | 1.00000 | 0 |
| 551 | chr6 | 159464000 | 159465000 | 0.040 | 0.000 | TAGAP | 1.00000 | 0 |
| 552 | chr6 | 159465000 | 159466000 | 0.040 | 0.000 | TAGAP | 1.00000 | 0 |
| 553 | chr6 | 161265000 | 161266000 | 0.000 | 0.040 | PLG | 1.00000 | 0 |
| 554 | chr6 | 161833000 | 161834000 | 0.040 | 0.000 | PARK2 | 1.00000 | 0 |
| 555 | chr6 | 162712000 | 162713000 | 0.040 | 0.000 | PARK2 | 1.00000 | 0 |
| 556 | chr6 | 164941000 | 164942000 | 0.040 | 0.000 | C6orf118 | 1.00000 | 0 |
| 557 | chr6 | 168813000 | 168814000 | 0.040 | 0.000 | SMOC2 | 1.00000 | 0 |
| 558 | chr7 | 1898000 | 1899000 | 0.040 | 0.040 | AC110781.3 | 1.00000 | 0 |
| 559 | chr7 | 1963000 | 1964000 | 0.040 | 0.000 | MAD1L1 | 1.00000 | 0 |
| 560 | chr7 | 2080000 | 2081000 | 0.000 | 0.040 | MAD1L1 | 1.00000 | 0 |
| 561 | chr7 | 5568000 | 5569000 | 0.040 | 0.080 | ACTB | 1.00000 | 1 |
| 562 | chr7 | 5569000 | 5570000 | 0.040 | 0.120 | ACTB | 0.60921 | 1 |
| 563 | chr7 | 5570000 | 5571000 | 0.040 | 0.040 | ACTB | 1.00000 | 1 |
| 564 | chr7 | 9933000 | 9934000 | 0.040 | 0.040 | NDUFA4 | 1.00000 | 0 |
| 565 | chr7 | 13017000 | 13018000 | 0.000 | 0.040 | ARL4A | 1.00000 | 0 |
| 566 | chr7 | 13346000 | 13347000 | 0.000 | 0.000 | ETV1 | 1.00000 | 0 |

| | | | | | | | | |
|---|---|---|---|---|---|---|---|---|
| 567 | chr7 | 15459000 | 15460000 | 0.000 | 0.000 | AGMO | 1.00000 | 0 |
| 568 | chr7 | 16382000 | 16383000 | 0.040 | 0.000 | ISPD | 1.00000 | 0 |
| 569 | chr7 | 28600000 | 28601000 | 0.040 | 0.000 | CREB5 | 1.00000 | 0 |
| 570 | chr7 | 40846000 | 40847000 | 0.040 | 0.000 | C7orf10 | 1.00000 | 0 |
| 571 | chr7 | 50349000 | 50350000 | 0.040 | 0.040 | IKZF1 | 1.00000 | 0 |
| 572 | chr7 | 50350000 | 50351000 | 0.080 | 0.040 | IKZF1 | 1.00000 | 0 |
| 573 | chr7 | 53335000 | 53336000 | 0.000 | 0.000 | POM121L12 | 1.00000 | 0 |
| 574 | chr7 | 57713000 | 57714000 | 0.080 | 0.040 | ZNF716 | 1.00000 | 0 |
| 575 | chr7 | 62475000 | 62476000 | 0.040 | 0.040 | AC006455.1 | 1.00000 | 0 |
| 576 | chr7 | 70669000 | 70670000 | 0.040 | 0.000 | WBSCR17 | 1.00000 | 0 |
| 577 | chr7 | 71553000 | 71554000 | 0.000 | 0.040 | CALN1 | 1.00000 | 0 |
| 578 | chr7 | 79847000 | 79848000 | 0.040 | 0.000 | GNAI1 | 1.00000 | 0 |
| 579 | chr7 | 80694000 | 80695000 | 0.040 | 0.000 | AC005008.2 | 1.00000 | 0 |
| 580 | chr7 | 81556000 | 81557000 | 0.000 | 0.000 | CACNA2D1 | 1.00000 | 0 |
| 581 | chr7 | 84127000 | 84128000 | 0.040 | 0.040 | SEMA3A | 1.00000 | 0 |
| 582 | chr7 | 84247000 | 84248000 | 0.000 | 0.000 | SEMA3D | 1.00000 | 0 |
| 583 | chr7 | 84257000 | 84258000 | 0.000 | 0.000 | SEMA3D | 1.00000 | 0 |
| 584 | chr7 | 86914000 | 86915000 | 0.040 | 0.000 | CROT | 1.00000 | 0 |
| 585 | chr7 | 90356000 | 90357000 | 0.040 | 0.000 | CDK14 | 1.00000 | 0 |
| 586 | chr7 | 93304000 | 93305000 | 0.000 | 0.000 | CALCR | 1.00000 | 0 |
| 587 | chr7 | 93682000 | 93683000 | 0.000 | 0.040 | BET1 | 1.00000 | 0 |
| 588 | chr7 | 102644000 | 102645000 | 0.000 | 0.000 | FBXL13 | 1.00000 | 0 |
| 589 | chr7 | 105699000 | 105700000 | 0.040 | 0.000 | CDHR3 | 1.00000 | 0 |
| 590 | chr7 | 110521000 | 110522000 | 0.040 | 0.040 | IMMP2L | 1.00000 | 0 |
| 591 | chr7 | 110543000 | 110544000 | 0.000 | 0.040 | IMMP2L | 1.00000 | 0 |
| 592 | chr7 | 110545000 | 110546000 | 0.040 | 0.040 | IMMP2L | 1.00000 | 0 |
| 593 | chr7 | 110597000 | 110598000 | 0.000 | 0.000 | IMMP2L | 1.00000 | 0 |
| 594 | chr7 | 110601000 | 110602000 | 0.040 | 0.000 | IMMP2L | 1.00000 | 0 |
| 595 | chr7 | 110602000 | 110603000 | 0.000 | 0.040 | IMMP2L | 1.00000 | 0 |
| 596 | chr7 | 110609000 | 110610000 | 0.000 | 0.040 | IMMP2L | 1.00000 | 0 |
| 597 | chr7 | 110610000 | 110611000 | 0.000 | 0.040 | IMMP2L | 1.00000 | 0 |
| 598 | chr7 | 110617000 | 110618000 | 0.000 | 0.040 | IMMP2L | 1.00000 | 0 |
| 599 | chr7 | 110618000 | 110619000 | 0.000 | 0.000 | IMMP2L | 1.00000 | 0 |
| 600 | chr7 | 110619000 | 110620000 | 0.040 | 0.040 | IMMP2L | 1.00000 | 0 |
| 601 | chr7 | 110621000 | 110622000 | 0.000 | 0.000 | IMMP2L | 1.00000 | 0 |
| 602 | chr7 | 110628000 | 110629000 | 0.040 | 0.040 | IMMP2L | 1.00000 | 0 |

| 603 | chr7 | 110629000 | 110630000 | 0.000 | 0.000 | IMMP2L | 1.00000 | 0 |
|---|---|---|---|---|---|---|---|---|
| 604 | chr7 | 110631000 | 110632000 | 0.000 | 0.040 | IMMP2L | 1.00000 | 0 |
| 605 | chr7 | 110632000 | 110633000 | 0.040 | 0.000 | IMMP2L | 1.00000 | 0 |
| 606 | chr7 | 110636000 | 110637000 | 0.040 | 0.000 | IMMP2L | 1.00000 | 0 |
| 607 | chr7 | 110637000 | 110638000 | 0.000 | 0.000 | IMMP2L | 1.00000 | 0 |
| 608 | chr7 | 110638000 | 110639000 | 0.000 | 0.040 | IMMP2L | 1.00000 | 0 |
| 609 | chr7 | 110639000 | 110640000 | 0.000 | 0.040 | IMMP2L | 1.00000 | 0 |
| 610 | chr7 | 110641000 | 110642000 | 0.000 | 0.000 | IMMP2L | 1.00000 | 0 |
| 611 | chr7 | 110650000 | 110651000 | 0.000 | 0.000 | IMMP2L | 1.00000 | 0 |
| 612 | chr7 | 110651000 | 110652000 | 0.000 | 0.040 | IMMP2L | 1.00000 | 0 |
| 613 | chr7 | 110666000 | 110667000 | 0.000 | 0.000 | IMMP2L | 1.00000 | 0 |
| 614 | chr7 | 110671000 | 110672000 | 0.000 | 0.080 | IMMP2L | 0.48980 | 0 |
| 615 | chr7 | 110677000 | 110678000 | 0.000 | 0.000 | IMMP2L | 1.00000 | 0 |
| 616 | chr7 | 110679000 | 110680000 | 0.000 | 0.000 | IMMP2L | 1.00000 | 0 |
| 617 | chr7 | 110680000 | 110681000 | 0.000 | 0.000 | IMMP2L | 1.00000 | 0 |
| 618 | chr7 | 110685000 | 110686000 | 0.000 | 0.000 | LRRN3 | 1.00000 | 0 |
| 619 | chr7 | 110686000 | 110687000 | 0.000 | 0.040 | LRRN3 | 1.00000 | 0 |
| 620 | chr7 | 110688000 | 110689000 | 0.000 | 0.000 | LRRN3 | 1.00000 | 0 |
| 621 | chr7 | 110699000 | 110700000 | 0.080 | 0.080 | LRRN3 | 0.48980 | 0 |
| 622 | chr7 | 110700000 | 110701000 | 0.040 | 0.040 | LRRN3 | 1.00000 | 0 |
| 623 | chr7 | 110709000 | 110710000 | 0.000 | 0.040 | LRRN3 | 1.00000 | 0 |
| 624 | chr7 | 110711000 | 110712000 | 0.000 | 0.040 | LRRN3 | 1.00000 | 0 |
| 625 | chr7 | 110714000 | 110715000 | 0.000 | 0.040 | LRRN3 | 1.00000 | 0 |
| 626 | chr7 | 110727000 | 110728000 | 0.000 | 0.040 | LRRN3 | 1.00000 | 0 |
| 627 | chr7 | 110728000 | 110729000 | 0.040 | 0.000 | LRRN3 | 1.00000 | 0 |
| 628 | chr7 | 110729000 | 110730000 | 0.000 | 0.040 | LRRN3 | 1.00000 | 0 |
| 629 | chr7 | 110734000 | 110735000 | 0.000 | 0.040 | LRRN3 | 1.00000 | 0 |
| 630 | chr7 | 110737000 | 110738000 | 0.000 | 0.000 | LRRN3 | 1.00000 | 0 |
| 631 | chr7 | 110740000 | 110741000 | 0.040 | 0.080 | LRRN3 | 1.00000 | 0 |
| 632 | chr7 | 110744000 | 110745000 | 0.000 | 0.000 | LRRN3 | 1.00000 | 0 |
| 633 | chr7 | 110746000 | 110747000 | 0.000 | 0.040 | LRRN3 | 1.00000 | 0 |
| 634 | chr7 | 110747000 | 110748000 | 0.000 | 0.000 | LRRN3 | 1.00000 | 0 |
| 635 | chr7 | 110748000 | 110749000 | 0.000 | 0.000 | LRRN3 | 1.00000 | 0 |
| 636 | chr7 | 110755000 | 110756000 | 0.000 | 0.000 | LRRN3 | 1.00000 | 0 |
| 637 | chr7 | 110764000 | 110765000 | 0.000 | 0.000 | LRRN3 | 1.00000 | 0 |
| 638 | chr7 | 110767000 | 110768000 | 0.040 | 0.000 | LRRN3 | 1.00000 | 0 |

| | | | | | | | | |
|---|---|---|---|---|---|---|---|---|
| 639 | chr7 | 110769000 | 110770000 | 0.000 | 0.040 | LRRN3 | 1.00000 | 0 |
| 640 | chr7 | 110771000 | 110772000 | 0.040 | 0.040 | LRRN3 | 1.00000 | 0 |
| 641 | chr7 | 110779000 | 110780000 | 0.000 | 0.000 | LRRN3 | 1.00000 | 0 |
| 642 | chr7 | 110780000 | 110781000 | 0.000 | 0.040 | LRRN3 | 1.00000 | 0 |
| 643 | chr7 | 110783000 | 110784000 | 0.000 | 0.040 | LRRN3 | 1.00000 | 0 |
| 644 | chr7 | 110785000 | 110786000 | 0.000 | 0.080 | LRRN3 | 0.48980 | 0 |
| 645 | chr7 | 110801000 | 110802000 | 0.000 | 0.040 | LRRN3 | 1.00000 | 0 |
| 646 | chr7 | 110802000 | 110803000 | 0.000 | 0.040 | LRRN3 | 1.00000 | 0 |
| 647 | chr7 | 110810000 | 110811000 | 0.000 | 0.000 | LRRN3 | 1.00000 | 0 |
| 648 | chr7 | 110816000 | 110817000 | 0.000 | 0.000 | LRRN3 | 1.00000 | 0 |
| 649 | chr7 | 110821000 | 110822000 | 0.000 | 0.040 | LRRN3 | 1.00000 | 0 |
| 650 | chr7 | 110824000 | 110825000 | 0.000 | 0.000 | LRRN3 | 1.00000 | 0 |
| 651 | chr7 | 110827000 | 110828000 | 0.040 | 0.000 | LRRN3 | 1.00000 | 0 |
| 652 | chr7 | 110836000 | 110837000 | 0.040 | 0.040 | LRRN3 | 1.00000 | 0 |
| 653 | chr7 | 110847000 | 110848000 | 0.000 | 0.040 | LRRN3 | 1.00000 | 0 |
| 654 | chr7 | 111567000 | 111568000 | 0.000 | 0.000 | DOCK4 | 1.00000 | 0 |
| 655 | chr7 | 119056000 | 119057000 | 0.040 | 0.000 | KCND2 | 1.00000 | 0 |
| 656 | chr7 | 121380000 | 121381000 | 0.040 | 0.000 | PTPRZ1 | 1.00000 | 0 |
| 657 | chr7 | 123887000 | 123888000 | 0.000 | 0.000 | TMEM229A | 1.00000 | 0 |
| 658 | chr7 | 125262000 | 125263000 | 0.000 | 0.040 | POT1 | 1.00000 | 0 |
| 659 | chr7 | 145723000 | 145724000 | 0.000 | 0.000 | CNTNAP2 | 1.00000 | 0 |
| 660 | chr7 | 148508000 | 148509000 | 0.000 | 0.000 | EZH2 | 1.00000 | 0 |
| 661 | chr7 | 155127000 | 155128000 | 0.000 | 0.000 | BLACE | 1.00000 | 0 |
| 662 | chr7 | 157162000 | 157163000 | 0.040 | 0.000 | DNAJB6 | 1.00000 | 0 |
| 663 | chr7 | 158684000 | 158685000 | 0.000 | 0.040 | WDR60 | 1.00000 | 0 |
| 664 | chr8 | 1646000 | 1647000 | 0.000 | 0.040 | DLGAP2 | 1.00000 | 0 |
| 665 | chr8 | 5558000 | 5559000 | 0.000 | 0.040 | MCPH1 | 1.00000 | 0 |
| 666 | chr8 | 5612000 | 5613000 | 0.000 | 0.000 | MCPH1 | 1.00000 | 0 |
| 667 | chr8 | 8602000 | 8603000 | 0.000 | 0.120 | MFHAS1 | 0.23469 | 0 |
| 668 | chr8 | 8706000 | 8707000 | 0.000 | 0.000 | MFHAS1 | 1.00000 | 0 |
| 669 | chr8 | 8717000 | 8718000 | 0.000 | 0.040 | MFHAS1 | 1.00000 | 0 |
| 670 | chr8 | 11352000 | 11353000 | 0.040 | 0.040 | BLK | 1.00000 | 0 |
| 671 | chr8 | 14080000 | 14081000 | 0.000 | 0.040 | SGCZ | 1.00000 | 0 |
| 672 | chr8 | 14796000 | 14797000 | 0.040 | 0.000 | SGCZ | 1.00000 | 0 |
| 673 | chr8 | 16090000 | 16091000 | 0.000 | 0.040 | MSR1 | 1.00000 | 0 |
| 674 | chr8 | 16187000 | 16188000 | 0.000 | 0.080 | MSR1 | 0.48980 | 0 |

| | | | | | | | | |
|---|---|---|---|---|---|---|---|---|
| 675 | chr8 | 23101000 | 23102000 | 0.000 | 0.040 | CHMP7 | 1.00000 | 0 |
| 676 | chr8 | 24207000 | 24208000 | 0.000 | 0.000 | ADAM28 | 1.00000 | 0 |
| 677 | chr8 | 29155000 | 29156000 | 0.000 | 0.040 | KIF13B | 1.00000 | 0 |
| 678 | chr8 | 35657000 | 35658000 | 0.000 | 0.000 | AC012215.1 | 1.00000 | 0 |
| 679 | chr8 | 38759000 | 38760000 | 0.040 | 0.000 | PLEKHA2 | 1.00000 | 0 |
| 680 | chr8 | 54986000 | 54987000 | 0.040 | 0.000 | LYPLA1 | 1.00000 | 0 |
| 681 | chr8 | 60031000 | 60032000 | 0.040 | 0.000 | TOX | 1.00000 | 0 |
| 682 | chr8 | 67525000 | 67526000 | 0.040 | 0.000 | MYBL1 | 1.00000 | 0 |
| 683 | chr8 | 77105000 | 77106000 | 0.000 | 0.000 | ZFHX4 | 1.00000 | 0 |
| 684 | chr8 | 78400000 | 78401000 | 0.000 | 0.040 | PEX2 | 1.00000 | 0 |
| 685 | chr8 | 90322000 | 90323000 | 0.040 | 0.000 | RIPK2 | 1.00000 | 0 |
| 686 | chr8 | 93199000 | 93200000 | 0.000 | 0.040 | RUNX1T1 | 1.00000 | 0 |
| 687 | chr8 | 94618000 | 94619000 | 0.000 | 0.040 | FAM92A1 | 1.00000 | 0 |
| 688 | chr8 | 110586000 | 110587000 | 0.000 | 0.040 | SYBU | 1.00000 | 0 |
| 689 | chr8 | 126687000 | 126688000 | 0.000 | 0.000 | TRIB1 | 1.00000 | 0 |
| 690 | chr8 | 128748000 | 128749000 | 0.080 | 0.280 | MYC | 0.13833 | 1 |
| 691 | chr8 | 128749000 | 128750000 | 0.080 | 0.320 | MYC | 0.07375 | 1 |
| 692 | chr8 | 128750000 | 128751000 | 0.080 | 0.120 | MYC | 1.00000 | 1 |
| 693 | chr8 | 128751000 | 128752000 | 0.040 | 0.080 | MYC | 1.00000 | 1 |
| 694 | chr8 | 128752000 | 128753000 | 0.000 | 0.000 | MYC | 1.00000 | 1 |
| 695 | chr8 | 137918000 | 137919000 | 0.000 | 0.040 | FAM135B | 1.00000 | 0 |
| 696 | chr8 | 138274000 | 138275000 | 0.000 | 0.000 | FAM135B | 1.00000 | 0 |
| 697 | chr8 | 143183000 | 143184000 | 0.000 | 0.040 | TSNARE1 | 1.00000 | 0 |
| 698 | chr9 | 144123000 | 144124000 | 0.000 | 0.040 | C8orf31 | 1.00000 | 0 |
| 699 | chr9 | 6411000 | 6412000 | 0.040 | 0.040 | UHRF2 | 1.00000 | 0 |
| 700 | chr9 | 6413000 | 6414000 | 0.040 | 0.040 | UHRF2 | 1.00000 | 0 |
| 701 | chr9 | 6414000 | 6415000 | 0.000 | 0.000 | UHRF2 | 1.00000 | 0 |
| 702 | chr9 | 9928000 | 9929000 | 0.000 | 0.000 | PTPRD | 1.00000 | 0 |
| 703 | chr9 | 13965000 | 13966000 | 0.040 | 0.000 | NFIB | 1.00000 | 0 |
| 704 | chr9 | 22824000 | 22825000 | 0.040 | 0.000 | DMRTA1 | 1.00000 | 0 |
| 705 | chr9 | 25260000 | 25261000 | 0.040 | 0.000 | TUSC1 | 1.00000 | 0 |
| 706 | chr9 | 29890000 | 29891000 | 0.040 | 0.000 | LINGO2 | 1.00000 | 0 |
| 707 | chr9 | 30656000 | 30657000 | 0.000 | 0.040 | ACO1 | 1.00000 | 0 |
| 708 | chr9 | 37003000 | 37004000 | 0.040 | 0.000 | PAX5 | 1.00000 | 1 |
| 709 | chr9 | 37005000 | 37006000 | 0.040 | 0.000 | PAX5 | 1.00000 | 1 |
| 710 | chr9 | 37024000 | 37025000 | 0.040 | 0.040 | PAX5 | 1.00000 | 1 |

| ID | chr | start | end | gene | val1 | val2 | val3 | flag |
|---|---|---|---|---|---|---|---|---|
| 711 | chr9 | 37025000 | 37026000 | PAX5 | 0.160 | 0.120 | 1.00000 | 1 |
| 712 | chr9 | 37026000 | 37027000 | PAX5 | 0.240 | 0.120 | 0.46349 | 1 |
| 713 | chr9 | 37027000 | 37028000 | PAX5 | 0.080 | 0.040 | 1.00000 | 1 |
| 714 | chr9 | 37033000 | 37034000 | PAX5 | 0.120 | 0.040 | 0.60921 | 1 |
| 715 | chr9 | 37034000 | 37035000 | PAX5 | 0.120 | 0.040 | 0.60921 | 1 |
| 716 | chr9 | 37035000 | 37036000 | PAX5 | 0.000 | 0.040 | 1.00000 | 1 |
| 717 | chr9 | 37196000 | 37197000 | ZCCHC7 | 0.040 | 0.000 | 1.00000 | 0 |
| 718 | chr9 | 37197000 | 37198000 | ZCCHC7 | 0.040 | 0.000 | 1.00000 | 0 |
| 719 | chr9 | 37293000 | 37294000 | ZCCHC7 | 0.000 | 0.000 | 1.00000 | 0 |
| 720 | chr9 | 37294000 | 37295000 | ZCCHC7 | 0.080 | 0.000 | 0.48980 | 0 |
| 721 | chr9 | 37327000 | 37328000 | ZCCHC7 | 0.040 | 0.000 | 1.00000 | 0 |
| 722 | chr9 | 37336000 | 37337000 | ZCCHC7 | 0.080 | 0.000 | 0.48980 | 0 |
| 723 | chr9 | 37337000 | 37338000 | ZCCHC7 | 0.000 | 0.000 | 1.00000 | 0 |
| 724 | chr9 | 37338000 | 37339000 | ZCCHC7 | 0.000 | 0.040 | 1.00000 | 0 |
| 725 | chr9 | 37369000 | 37370000 | ZCCHC7 | 0.040 | 0.000 | 1.00000 | 0 |
| 726 | chr9 | 37371000 | 37372000 | ZCCHC7 | 0.080 | 0.080 | 1.00000 | 0 |
| 727 | chr9 | 37372000 | 37373000 | ZCCHC7 | 0.000 | 0.000 | 1.00000 | 0 |
| 728 | chr9 | 37383000 | 37384000 | ZCCHC7 | 0.080 | 0.080 | 1.00000 | 0 |
| 729 | chr9 | 37384000 | 37385000 | ZCCHC7 | 0.120 | 0.040 | 0.60921 | 0 |
| 730 | chr9 | 37385000 | 37386000 | ZCCHC7 | 0.040 | 0.000 | 1.00000 | 0 |
| 731 | chr9 | 37387000 | 37388000 | ZCCHC7 | 0.080 | 0.040 | 1.00000 | 0 |
| 732 | chr9 | 37397000 | 37398000 | GRHPR | 0.040 | 0.120 | 0.60921 | 0 |
| 733 | chr9 | 37398000 | 37399000 | GRHPR | 0.040 | 0.000 | 1.00000 | 0 |
| 734 | chr9 | 37399000 | 37400000 | GRHPR | 0.080 | 0.000 | 0.48980 | 0 |
| 735 | chr9 | 37402000 | 37403000 | GRHPR | 0.000 | 0.040 | 1.00000 | 0 |
| 736 | chr9 | 37406000 | 37407000 | GRHPR | 0.000 | 0.040 | 1.00000 | 0 |
| 737 | chr9 | 37407000 | 37408000 | GRHPR | 0.200 | 0.080 | 0.41743 | 0 |
| 738 | chr9 | 37408000 | 37409000 | GRHPR | 0.080 | 0.000 | 0.48980 | 0 |
| 739 | chr9 | 37410000 | 37411000 | GRHPR | 0.000 | 0.000 | 1.00000 | 0 |
| 740 | chr9 | 37424000 | 37425000 | GRHPR | 0.040 | 0.040 | 1.00000 | 0 |
| 741 | chr9 | 37425000 | 37426000 | GRHPR | 0.000 | 0.040 | 1.00000 | 0 |
| 742 | chr9 | 112811000 | 112812000 | AKAP2 | 0.080 | 0.080 | 1.00000 | 0 |
| 743 | chr9 | 117037000 | 117038000 | COL27A1 | 0.000 | 0.040 | 1.00000 | 0 |
| 744 | chr9 | 119779000 | 119780000 | ASTN2 | 0.040 | 0.000 | 1.00000 | 0 |
| 745 | chr9 | 126232000 | 126233000 | DENND1A | 0.040 | 0.000 | 1.00000 | 0 |
| 746 | chr9 | 130741000 | 130742000 | FAM102A | 0.040 | 0.000 | 1.00000 | 1 |

| | | | | | | | | |
|---|---|---|---|---|---|---|---|---|
| 747 | chr9 | 130742000 | 130743000 | 0.040 | 0.080 | FAM102A | 1.00000 | 1 |
| 748 | chr9 | 132767000 | 132768000 | 0.000 | 0.040 | FNBP1 | 1.00000 | 0 |
| 749 | chr9 | 132785000 | 132786000 | 0.040 | 0.000 | FNBP1 | 1.00000 | 0 |
| 750 | chr9 | 132803000 | 132804000 | 0.000 | 0.040 | FNBP1 | 1.00000 | 0 |
| 751 | chr9 | 132804000 | 132805000 | 0.040 | 0.120 | FNBP1 | 0.60921 | 0 |
| 752 | chr9 | 134551000 | 134552000 | 0.040 | 0.000 | RAPGEF1 | 1.00000 | 0 |
| 753 | chr9 | 138874000 | 138875000 | 0.000 | 0.040 | UBAC1 | 1.00000 | 0 |
| 754 | chr10 | 3333000 | 3334000 | 0.000 | 0.000 | PITRM1 | 1.00000 | 0 |
| 755 | chr10 | 5707000 | 5708000 | 0.040 | 0.040 | ASB13 | 1.00000 | 0 |
| 756 | chr10 | 5728000 | 5729000 | 0.000 | 0.040 | ASB13 | 1.00000 | 0 |
| 757 | chr10 | 15393000 | 15394000 | 0.000 | 0.000 | FAM171A1 | 1.00000 | 1 |
| 758 | chr10 | 20796000 | 20797000 | 0.040 | 0.000 | PLXDC2 | 1.00000 | 1 |
| 759 | chr10 | 35424000 | 35425000 | 0.000 | 0.000 | CREM | 1.00000 | 1 |
| 760 | chr10 | 56678000 | 56679000 | 0.000 | 0.000 | PCDH15 | 1.00000 | 1 |
| 761 | chr10 | 63440000 | 63441000 | 0.000 | 0.040 | C10orf107 | 1.00000 | 1 |
| 762 | chr10 | 63659000 | 63660000 | 0.040 | 0.000 | ARID5B | 1.00000 | 1 |
| 763 | chr10 | 63660000 | 63661000 | 0.040 | 0.080 | ARID5B | 1.00000 | 0 |
| 764 | chr10 | 63662000 | 63663000 | 0.000 | 0.000 | ARID5B | 1.00000 | 0 |
| 765 | chr10 | 63720000 | 63721000 | 0.000 | 0.000 | ARID5B | 1.00000 | 0 |
| 766 | chr10 | 63803000 | 63804000 | 0.000 | 0.000 | ARID5B | 1.00000 | 0 |
| 767 | chr10 | 63809000 | 63810000 | 0.000 | 0.080 | ARID5B | 0.48980 | 0 |
| 768 | chr10 | 63810000 | 63811000 | 0.040 | 0.040 | ARID5B | 1.00000 | 0 |
| 769 | chr10 | 67907000 | 67908000 | 0.000 | 0.040 | CTNNA3 | 1.00000 | 0 |
| 770 | chr10 | 68474000 | 68475000 | 0.000 | 0.000 | CTNNA3 | 1.00000 | 0 |
| 771 | chr10 | 98510000 | 98511000 | 0.080 | 0.000 | PIK3AP1 | 0.48980 | 0 |
| 772 | chr10 | 101384000 | 101385000 | 0.000 | 0.000 | SLC25A28 | 1.00000 | 0 |
| 773 | chr10 | 108276000 | 108277000 | 0.040 | 0.040 | SORCS1 | 1.00000 | 0 |
| 774 | chr10 | 113473000 | 113474000 | 0.040 | 0.040 | GPAM | 1.00000 | 0 |
| 775 | chr10 | 113636000 | 113637000 | 0.040 | 0.000 | GPAM | 1.00000 | 0 |
| 776 | chr10 | 116458000 | 116459000 | 0.000 | 0.040 | ABLIM1 | 1.00000 | 0 |
| 777 | chr10 | 121623000 | 121624000 | 0.040 | 0.000 | MCMBP | 1.00000 | 0 |
| 778 | chr10 | 132973000 | 132974000 | 0.040 | 0.000 | TCERG1L | 1.00000 | 0 |
| 779 | chr10 | 134326000 | 134327000 | 0.000 | 0.040 | INPP5A | 1.00000 | 0 |
| 780 | chr11 | 871000 | 872000 | 0.040 | 0.000 | CHID1 | 1.00000 | 0 |
| 781 | chr11 | 1149000 | 1150000 | 0.000 | 0.000 | MUC5AC | 1.00000 | 0 |
| 782 | chr11 | 25065000 | 25066000 | 0.040 | 0.000 | LUZP2 | 1.00000 | 0 |

| | | | | | | | | |
|---|---|---|---|---|---|---|---|---|
| 783 | chr11 | 25289000 | 25290000 | LUZP2 | 0.040 | 0.040 | 1.00000 | 0 |
| 784 | chr11 | 27216000 | 27217000 | BBOX1 | 0.040 | 0.000 | 1.00000 | 0 |
| 785 | chr11 | 28849000 | 28850000 | METTL15 | 0.000 | 0.000 | 1.00000 | 0 |
| 786 | chr11 | 29253000 | 29254000 | KCNA4 | 0.000 | 0.040 | 1.00000 | 0 |
| 787 | chr11 | 29900000 | 29901000 | KCNA4 | 0.000 | 0.000 | 1.00000 | 0 |
| 788 | chr11 | 40626000 | 40627000 | LRRC4C | 0.000 | 0.000 | 1.00000 | 0 |
| 789 | chr11 | 40845000 | 40846000 | LRRC4C | 0.000 | 0.000 | 1.00000 | 0 |
| 790 | chr11 | 40868000 | 40869000 | LRRC4C | 0.000 | 0.000 | 1.00000 | 0 |
| 791 | chr11 | 41066000 | 41067000 | LRRC4C | 0.000 | 0.000 | 1.00000 | 0 |
| 792 | chr11 | 41844000 | 41845000 | AP15 | 0.000 | 0.000 | 1.00000 | 0 |
| 793 | chr11 | 57171000 | 57172000 | SLC43A3 | 0.000 | 0.040 | 1.00000 | 0 |
| 794 | chr11 | 60224000 | 60225000 | MS4A1 | 0.080 | 0.040 | 1.00000 | 1 |
| 795 | chr11 | 65190000 | 65191000 | FRMD8 | 0.120 | 0.080 | 1.00000 | 0 |
| 796 | chr11 | 65191000 | 65192000 | FRMD8 | 0.120 | 0.080 | 1.00000 | 0 |
| 797 | chr11 | 65266000 | 65267000 | SCYL1 | 0.040 | 0.000 | 1.00000 | 0 |
| 798 | chr11 | 65267000 | 65268000 | SCYL1 | 0.040 | 0.120 | 0.60921 | 0 |
| 799 | chr11 | 85963000 | 85964000 | EED | 0.000 | 0.000 | 1.00000 | 0 |
| 800 | chr11 | 92261000 | 92262000 | FAT3 | 0.040 | 0.000 | 1.00000 | 0 |
| 801 | chr11 | 102117000 | 102118000 | YAP1 | 0.000 | 0.000 | 1.00000 | 0 |
| 802 | chr11 | 102188000 | 102189000 | BIRC3 | 0.280 | 0.200 | 0.74164 | 1 |
| 803 | chr11 | 102189000 | 102190000 | BIRC3 | 0.080 | 0.040 | 1.00000 | 1 |
| 804 | chr11 | 107497000 | 107498000 | ELMOD1 | 0.000 | 0.000 | 1.00000 | 0 |
| 805 | chr11 | 108781000 | 108782000 | DDX10 | 0.040 | 0.000 | 1.00000 | 0 |
| 806 | chr11 | 108975000 | 108976000 | DDX10 | 0.000 | 0.040 | 1.00000 | 0 |
| 807 | chr11 | 109066000 | 109067000 | C11orf87 | 0.000 | 0.000 | 1.00000 | 0 |
| 808 | chr11 | 111248000 | 111249000 | POU2AF1 | 0.040 | 0.000 | 1.00000 | 1 |
| 809 | chr11 | 111249000 | 111250000 | POU2AF1 | 0.160 | 0.120 | 1.00000 | 1 |
| 810 | chr11 | 115761000 | 115762000 | CADM1 | 0.040 | 0.000 | 1.00000 | 0 |
| 811 | chr11 | 118723000 | 118724000 | CXCR5 | 0.000 | 0.040 | 1.00000 | 0 |
| 812 | chr11 | 126496000 | 126497000 | KIRREL3 | 0.000 | 0.040 | 1.00000 | 0 |
| 813 | chr11 | 128390000 | 128391000 | ETS1 | 0.040 | 0.040 | 1.00000 | 1 |
| 814 | chr11 | 128391000 | 128392000 | ETS1 | 0.040 | 0.160 | 0.34863 | 1 |
| 815 | chr12 | 6554000 | 6555000 | CD27 | 0.040 | 0.000 | 1.00000 | 0 |
| 816 | chr12 | 8762000 | 8763000 | AICDA | 0.000 | 0.040 | 1.00000 | 0 |
| 817 | chr12 | 8763000 | 8764000 | AICDA | 0.040 | 0.080 | 1.00000 | 0 |
| 818 | chr12 | 8764000 | 8765000 | AICDA | 0.000 | 0.080 | 0.48980 | 0 |

| 819 | chr12 | 8765000 | 8766000 | 0.040 | 0.000 | AICDA | 1.00000 | 0 |
|---|---|---|---|---|---|---|---|---|
| 820 | chr12 | 9823000 | 9824000 | 0.040 | 0.000 | CLEC2D | 1.00000 | 0 |
| 821 | chr12 | 11710000 | 11711000 | 0.000 | 0.040 | ETV6 | 1.00000 | 1 |
| 822 | chr12 | 11803000 | 11804000 | 0.040 | 0.000 | ETV6 | 1.00000 | 1 |
| 823 | chr12 | 14923000 | 14924000 | 0.040 | 0.040 | HIST4H4 | 1.00000 | 1 |
| 824 | chr12 | 16717000 | 16718000 | 0.000 | 0.000 | LMO3 | 1.00000 | 0 |
| 825 | chr12 | 23805000 | 23806000 | 0.000 | 0.040 | SOX5 | 1.00000 | 0 |
| 826 | chr12 | 25149000 | 25150000 | 0.000 | 0.040 | C12orf77 | 1.00000 | 0 |
| 827 | chr12 | 25151000 | 25152000 | 0.000 | 0.040 | C12orf77 | 1.00000 | 0 |
| 828 | chr12 | 25174000 | 25175000 | 0.040 | 0.040 | C12orf77 | 1.00000 | 0 |
| 829 | chr12 | 25205000 | 25206000 | 0.040 | 0.040 | LRMP | 1.00000 | 1 |
| 830 | chr12 | 25206000 | 25207000 | 0.080 | 0.120 | LRMP | 1.00000 | 1 |
| 831 | chr12 | 25207000 | 25208000 | 0.080 | 0.120 | LRMP | 1.00000 | 1 |
| 832 | chr12 | 25208000 | 25209000 | 0.000 | 0.040 | LRMP | 1.00000 | 1 |
| 833 | chr12 | 25665000 | 25666000 | 0.000 | 0.000 | IFLTD1 | 1.00000 | 0 |
| 834 | chr12 | 38920000 | 38921000 | 0.000 | 0.000 | CPNE8 | 1.00000 | 0 |
| 835 | chr12 | 48027000 | 48028000 | 0.080 | 0.080 | RPAP3 | 1.00000 | 0 |
| 836 | chr12 | 57496000 | 57497000 | 0.040 | 0.000 | STAT6 | 1.00000 | 0 |
| 837 | chr12 | 69203000 | 69204000 | 0.000 | 0.040 | MDM2 | 1.00000 | 0 |
| 838 | chr12 | 76202000 | 76203000 | 0.000 | 0.000 | PHLDA1 | 1.00000 | 0 |
| 839 | chr12 | 79270000 | 79271000 | 0.000 | 0.000 | SYT1 | 1.00000 | 0 |
| 840 | chr12 | 82572000 | 82573000 | 0.000 | 0.040 | CCDC59 | 1.00000 | 0 |
| 841 | chr12 | 84837000 | 84838000 | 0.000 | 0.000 | SLC6A15 | 1.00000 | 0 |
| 842 | chr12 | 86114000 | 86115000 | 0.040 | 0.000 | RASSF9 | 1.00000 | 0 |
| 843 | chr12 | 86115000 | 86116000 | 0.040 | 0.000 | RASSF9 | 1.00000 | 0 |
| 844 | chr12 | 92538000 | 92539000 | 0.080 | 0.080 | BTG1 | 1.00000 | 1 |
| 845 | chr12 | 92539000 | 92540000 | 0.080 | 0.040 | BTG1 | 1.00000 | 1 |
| 846 | chr12 | 96030000 | 96031000 | 0.000 | 0.040 | NTN4 | 1.00000 | 0 |
| 847 | chr12 | 110171000 | 110172000 | 0.000 | 0.040 | FAM222A | 1.00000 | 0 |
| 848 | chr12 | 110980000 | 110981000 | 0.000 | 0.040 | PPTC7 | 1.00000 | 0 |
| 849 | chr12 | 113493000 | 113494000 | 0.080 | 0.000 | DTX1 | 0.48980 | 1 |
| 850 | chr12 | 113494000 | 113495000 | 0.240 | 0.040 | DTX1 | 0.09828 | 1 |
| 851 | chr12 | 113495000 | 113496000 | 0.160 | 0.080 | DTX1 | 0.66710 | 1 |
| 852 | chr12 | 113496000 | 113497000 | 0.160 | 0.040 | DTX1 | 0.34868 | 1 |
| 853 | chr12 | 113497000 | 113498000 | 0.080 | 0.040 | DTX1 | 1.00000 | 1 |
| 854 | chr12 | 113499000 | 113500000 | 0.000 | 0.000 | DTX1 | 1.00000 | 1 |

| | | | | | | | | |
|---|---|---|---|---|---|---|---|---|
| 855 | chr12 | 113512000 | 113513000 | 0.000 | 0.000 | DTX1 | 1.00000 | 1 |
| 856 | chr12 | 115966000 | 115967000 | 0.000 | 0.000 | MED13L | 1.00000 | 0 |
| 857 | chr12 | 122432000 | 122433000 | 0.040 | 0.000 | WDR66 | 1.00000 | 0 |
| 858 | chr12 | 122433000 | 122434000 | 0.080 | 0.000 | WDR66 | 0.48980 | 0 |
| 859 | chr12 | 122447000 | 122448000 | 0.000 | 0.040 | WDR66 | 1.00000 | 0 |
| 860 | chr12 | 122458000 | 122459000 | 0.080 | 0.120 | BCL7A | 1.00000 | 1 |
| 861 | chr12 | 122459000 | 122460000 | 0.240 | 0.320 | BCL7A | 0.75361 | 1 |
| 862 | chr12 | 122460000 | 122461000 | 0.120 | 0.280 | BCL7A | 0.28902 | 1 |
| 863 | chr12 | 122461000 | 122462000 | 0.240 | 0.240 | BCL7A | 1.00000 | 1 |
| 864 | chr12 | 122462000 | 122463000 | 0.160 | 0.200 | BCL7A | 1.00000 | 1 |
| 865 | chr12 | 122463000 | 122464000 | 0.120 | 0.200 | BCL7A | 0.70194 | 1 |
| 866 | chr12 | 124054000 | 124055000 | 0.000 | 0.080 | TMED2 | 0.48980 | 0 |
| 867 | chr12 | 127965000 | 127966000 | 0.000 | 0.000 | TMEM132C | 1.00000 | 0 |
| 868 | chr12 | 131303000 | 131304000 | 0.000 | 0.120 | STX2 | 0.23469 | 0 |
| 869 | chr12 | 131649000 | 131650000 | 0.000 | 0.000 | GPR133 | 1.00000 | 0 |
| 870 | chr12 | 133306000 | 133307000 | 0.000 | 0.000 | ANKLE2 | 1.00000 | 0 |
| 871 | chr13 | 21913000 | 21914000 | 0.040 | 0.040 | ZDHHC20 | 1.00000 | 0 |
| 872 | chr13 | 32116000 | 32117000 | 0.040 | 0.040 | RXFP2 | 1.00000 | 0 |
| 873 | chr13 | 35498000 | 35499000 | 0.000 | 0.000 | NBEA | 1.00000 | 0 |
| 874 | chr13 | 38371000 | 38372000 | 0.040 | 0.000 | TRPC4 | 1.00000 | 0 |
| 875 | chr13 | 38630000 | 38631000 | 0.040 | 0.000 | TRPC4 | 1.00000 | 0 |
| 876 | chr13 | 41156000 | 41157000 | 0.000 | 0.040 | FOXO1 | 1.00000 | 1 |
| 877 | chr13 | 41240000 | 41241000 | 0.000 | 0.040 | FOXO1 | 1.00000 | 1 |
| 878 | chr13 | 46958000 | 46959000 | 0.000 | 0.000 | KIAA0226L | 1.00000 | 0 |
| 879 | chr13 | 46959000 | 46960000 | 0.040 | 0.000 | KIAA0226L | 1.00000 | 0 |
| 880 | chr13 | 46960000 | 46961000 | 0.160 | 0.040 | KIAA0226L | 0.34868 | 0 |
| 881 | chr13 | 46961000 | 46962000 | 0.000 | 0.040 | KIAA0226L | 1.00000 | 0 |
| 882 | chr13 | 46962000 | 46963000 | 0.000 | 0.040 | KIAA0226L | 1.00000 | 0 |
| 883 | chr13 | 55239000 | 55240000 | 0.040 | 0.000 | OLFM4 | 1.00000 | 0 |
| 884 | chr13 | 55386000 | 55387000 | 0.040 | 0.000 | OLFM4 | 1.00000 | 0 |
| 885 | chr13 | 55598000 | 55599000 | 0.000 | 0.000 | OLFM4 | 1.00000 | 0 |
| 886 | chr13 | 57222000 | 57223000 | 0.000 | 0.040 | PRR20A;PRR20DPRR20BPRR20E; | 1.00000 | 0 |
| 887 | chr13 | 61343000 | 61344000 | 0.000 | 0.000 | TDRD3 | 1.00000 | 0 |
| 888 | chr13 | 62830000 | 62831000 | 0.000 | 0.000 | PCDH20 | 1.00000 | 0 |

| | | | | | | | | |
|---|---|---|---|---|---|---|---|---|
| 889 | chr13 | 63049000 | 63050000 | 0.080 | 0.000 | PCDH20 | 0.48980 | 0 |
| 890 | chr13 | 63157000 | 63158000 | 0.000 | 0.000 | AL45989.1 | 1.00000 | 0 |
| 891 | chr13 | 63214000 | 63215000 | 0.040 | 0.000 | AL45989.1 | 1.00000 | 0 |
| 892 | chr13 | 64802000 | 64803000 | 0.000 | 0.040 | AL45989.1 | 1.00000 | 0 |
| 893 | chr13 | 65637000 | 65638000 | 0.000 | 0.040 | PCDH9 | 1.00000 | 0 |
| 894 | chr13 | 68656000 | 68657000 | 0.000 | 0.000 | PCDH9 | 1.00000 | 0 |
| 895 | chr13 | 69418000 | 69419000 | 0.000 | 0.000 | KLHL1 | 1.00000 | 0 |
| 896 | chr13 | 70956000 | 70957000 | 0.040 | 0.000 | KLHL1 | 1.00000 | 0 |
| 897 | chr13 | 74542000 | 74543000 | 0.000 | 0.040 | KLF12 | 1.00000 | 0 |
| 898 | chr13 | 75983000 | 75984000 | 0.000 | 0.040 | TBC1D4 | 1.00000 | 0 |
| 899 | chr13 | 75984000 | 75985000 | 0.000 | 0.160 | TBC1D4 | 0.10986 | 0 |
| 900 | chr13 | 83450000 | 83451000 | 0.000 | 0.000 | SLITRK1 | 1.00000 | 0 |
| 901 | chr13 | 84641000 | 84642000 | 0.040 | 0.000 | SLITRK1 | 1.00000 | 0 |
| 902 | chr13 | 87793000 | 87794000 | 0.040 | 0.000 | SLITRK5 | 1.00000 | 0 |
| 903 | chr13 | 91480000 | 91481000 | 0.000 | 0.000 | GPC5 | 1.00000 | 0 |
| 904 | chr13 | 106081000 | 106082000 | 0.040 | 0.000 | DAOA | 1.00000 | 0 |
| 905 | chr13 | 114786000 | 114787000 | 0.040 | 0.000 | RASA3 | 1.00000 | 0 |
| 906 | chr13 | 114916000 | 114917000 | 0.000 | 0.000 | RASA3 | 1.00000 | 0 |
| 907 | chr14 | 22948000 | 22949000 | 0.040 | 0.000 | TRAJ56 | 1.00000 | 0 |
| 908 | chr14 | 22949000 | 22950000 | 0.040 | 0.000 | TRAJ56 | 1.00000 | 0 |
| 909 | chr14 | 22950000 | 22951000 | 0.040 | 0.000 | TRAJ54 | 1.00000 | 0 |
| 910 | chr14 | 22977000 | 22978000 | 0.000 | 0.040 | TRAJ33 | 1.00000 | 0 |
| 911 | chr14 | 27286000 | 27287000 | 0.000 | 0.000 | NOVA1 | 1.00000 | 0 |
| 912 | chr14 | 28645000 | 28646000 | 0.000 | 0.000 | FOXG1 | 1.00000 | 0 |
| 913 | chr14 | 49407000 | 49408000 | 0.000 | 0.000 | RPS29 | 1.00000 | 0 |
| 914 | chr14 | 50864000 | 50865000 | 0.000 | 0.000 | CDKL1 | 1.00000 | 0 |
| 915 | chr14 | 54812000 | 54813000 | 0.000 | 0.000 | CDKN3 | 1.00000 | 0 |
| 916 | chr14 | 55348000 | 55349000 | 0.040 | 0.000 | GCH1 | 1.00000 | 0 |
| 917 | chr14 | 59827000 | 59828000 | 0.000 | 0.040 | DAAM1 | 1.00000 | 0 |
| 918 | chr14 | 63143000 | 63144000 | 0.000 | 0.040 | KCNH5 | 1.00000 | 0 |
| 919 | chr14 | 64194000 | 64195000 | 0.000 | 0.040 | SGPP1 | 1.00000 | 0 |
| 920 | chr14 | 69258000 | 69259000 | 0.240 | 0.200 | ZFP36L1 | 1.00000 | 1 |
| 921 | chr14 | 69259000 | 69260000 | 0.360 | 0.240 | ZFP36L1 | 0.53803 | 1 |
| 922 | chr14 | 78418000 | 78419000 | 0.000 | 0.040 | ADCK1 | 1.00000 | 0 |
| 923 | chr14 | 81685000 | 81686000 | 0.000 | 0.040 | GTF2A1 | 1.00000 | 0 |
| 924 | chr14 | 84420000 | 84421000 | 0.040 | 0.000 | FLRT2 | 1.00000 | 0 |

| 925 | chr14 | 91883000 | 91884000 | 0.040 | 0.000 | CCDC88C | 1.00000 | 0 |
|---|---|---|---|---|---|---|---|---|
| 926 | chr14 | 94941000 | 94942000 | 0.000 | 0.120 | SERPINA9 | 0.23469 | 1 |
| 927 | chr14 | 94942000 | 94943000 | 0.040 | 0.200 | SERPINA9 | 0.18946 | 1 |
| 928 | chr14 | 96179000 | 96180000 | 0.160 | 0.120 | TCL1A | 1.00000 | 1 |
| 929 | chr14 | 96180000 | 96181000 | 0.080 | 0.160 | TCL1A | 0.66710 | 1 |
| 930 | chr14 | 101597000 | 101598000 | 0.000 | 0.000 | AL117190.3 | 1.00000 | 0 |
| 931 | chr14 | 102285000 | 102286000 | 0.040 | 0.000 | PPP2R5C | 1.00000 | 0 |
| 932 | chr14 | 105954000 | 105955000 | 0.040 | 0.040 | CRIP1 | 1.00000 | 0 |
| 933 | chr14 | 106031000 | 106032000 | 0.040 | 0.000 | IGHA2 | 1.00000 | 0 |
| 934 | chr14 | 106042000 | 106043000 | 0.080 | 0.200 | IGHA2 | 0.41743 | 0 |
| 935 | chr14 | 106048000 | 106049000 | 0.040 | 0.040 | IGHA2 | 1.00000 | 0 |
| 936 | chr14 | 106054000 | 106055000 | 0.040 | 0.040 | IGHA2 | 1.00000 | 0 |
| 937 | chr14 | 106055000 | 106056000 | 0.080 | 0.240 | IGHA2 | 0.24672 | 0 |
| 938 | chr14 | 106056000 | 106057000 | 0.040 | 0.200 | IGHA2 | 0.18946 | 0 |
| 939 | chr14 | 106057000 | 106058000 | 0.000 | 0.080 | IGHA2 | 0.48980 | 0 |
| 940 | chr14 | 106058000 | 106059000 | 0.000 | 0.080 | IGHA2 | 0.48980 | 0 |
| 941 | chr14 | 106066000 | 106067000 | 0.000 | 0.120 | IGHE | 0.23469 | 0 |
| 942 | chr14 | 106067000 | 106068000 | 0.000 | 0.120 | IGHE | 0.23469 | 0 |
| 943 | chr14 | 106068000 | 106069000 | 0.040 | 0.120 | IGHE | 0.60921 | 0 |
| 944 | chr14 | 106069000 | 106070000 | 0.040 | 0.200 | IGHE | 0.18946 | 0 |
| 945 | chr14 | 106070000 | 106071000 | 0.000 | 0.160 | IGHE | 0.10986 | 0 |
| 946 | chr14 | 106071000 | 106072000 | 0.000 | 0.160 | IGHE | 0.10986 | 0 |
| 947 | chr14 | 106072000 | 106073000 | 0.000 | 0.120 | IGHE | 0.23469 | 0 |
| 948 | chr14 | 106082000 | 106083000 | 0.000 | 0.000 | IGHG4 | 1.00000 | 0 |
| 949 | chr14 | 106092000 | 106093000 | 0.040 | 0.000 | IGHG4 | 1.00000 | 0 |
| 950 | chr14 | 106094000 | 106095000 | 0.160 | 0.200 | IGHG4 | 1.00000 | 0 |
| 951 | chr14 | 106095000 | 106096000 | 0.080 | 0.160 | IGHG4 | 0.66710 | 0 |
| 952 | chr14 | 106110000 | 106111000 | 0.080 | 0.040 | IGHG2 | 1.00000 | 0 |
| 953 | chr14 | 106111000 | 106112000 | 0.000 | 0.040 | IGHG2 | 1.00000 | 0 |
| 954 | chr14 | 106112000 | 106113000 | 0.280 | 0.200 | IGHG2 | 0.74164 | 0 |
| 955 | chr14 | 106113000 | 106114000 | 0.240 | 0.320 | IGHG2 | 0.75361 | 0 |
| 956 | chr14 | 106114000 | 106115000 | 0.320 | 0.200 | IGHG2 | 0.52019 | 0 |
| 957 | chr14 | 106146000 | 106147000 | 0.000 | 0.000 | IGHA1 | 1.00000 | 0 |
| 958 | chr14 | 106151000 | 106152000 | 0.040 | 0.000 | IGHA1 | 1.00000 | 0 |
| 959 | chr14 | 106152000 | 106153000 | 0.040 | 0.000 | IGHA1 | 1.00000 | 0 |
| 960 | chr14 | 106161000 | 106162000 | 0.000 | 0.040 | IGHA1 | 1.00000 | 0 |

| 961 | chr14 | 106173000 | 106174000 | 0.040 | 0.040 | IGHA1 | 1.00000 | 0 |
|---|---|---|---|---|---|---|---|---|
| 962 | chr14 | 106174000 | 106175000 | 0.040 | 0.000 | IGHA1 | 1.00000 | 0 |
| 963 | chr14 | 106175000 | 106176000 | 0.040 | 0.000 | IGHA1 | 1.00000 | 0 |
| 964 | chr14 | 106176000 | 106177000 | 0.080 | 0.040 | IGHA1 | 1.00000 | 0 |
| 965 | chr14 | 106177000 | 106178000 | 0.000 | 0.000 | IGHA1 | 1.00000 | 0 |
| 966 | chr14 | 106178000 | 106179000 | 0.120 | 0.000 | IGHA1 | 0.23469 | 0 |
| 967 | chr14 | 106208000 | 106209000 | 0.040 | 0.040 | IGHG1 | 1.00000 | 0 |
| 968 | chr14 | 106209000 | 106210000 | 0.160 | 0.080 | IGHG1 | 0.66710 | 0 |
| 969 | chr14 | 106210000 | 106211000 | 0.160 | 0.120 | IGHG1 | 1.00000 | 0 |
| 970 | chr14 | 106211000 | 106212000 | 0.440 | 0.120 | IGHG1 | 0.02548 | 0 |
| 971 | chr14 | 106212000 | 106213000 | 0.520 | 0.120 | IGHG1 | 0.00544 | 0 |
| 972 | chr14 | 106213000 | 106214000 | 0.520 | 0.120 | IGHG1 | 0.00544 | 0 |
| 973 | chr14 | 106214000 | 106215000 | 0.240 | 0.000 | IGHG1 | 0.02229 | 0 |
| 974 | chr14 | 106237000 | 106238000 | 0.080 | 0.040 | IGHG3 | 1.00000 | 0 |
| 975 | chr14 | 106238000 | 106239000 | 0.320 | 0.120 | IGHG3 | 0.17062 | 0 |
| 976 | chr14 | 106239000 | 106240000 | 0.440 | 0.040 | IGHG3 | 0.00192 | 0 |
| 977 | chr14 | 106240000 | 106241000 | 0.480 | 0.080 | IGHG3 | 0.00361 | 0 |
| 978 | chr14 | 106241000 | 106242000 | 0.320 | 0.040 | IGHG3 | 0.02322 | 0 |
| 979 | chr14 | 106242000 | 106243000 | 0.040 | 0.000 | IGHG3 | 1.00000 | 0 |
| 980 | chr14 | 106321000 | 106322000 | 0.040 | 0.000 | IGHM | 1.00000 | 0 |
| 981 | chr14 | 106322000 | 106323000 | 0.240 | 0.040 | IGHM | 0.09828 | 0 |
| 982 | chr14 | 106323000 | 106324000 | 0.400 | 0.160 | IGHM | 0.11366 | 0 |
| 983 | chr14 | 106324000 | 106325000 | 0.320 | 0.120 | IGHM | 0.17062 | 0 |
| 984 | chr14 | 106325000 | 106326000 | 0.160 | 0.320 | IGHM | 0.32089 | 0 |
| 985 | chr14 | 106326000 | 106327000 | 0.920 | 0.920 | IGHJ6 | 1.00000 | 0 |
| 986 | chr14 | 106327000 | 106328000 | 0.800 | 0.760 | IGHJ6 | 1.00000 | 0 |
| 987 | chr14 | 106328000 | 106329000 | 0.680 | 0.800 | IGHJ6 | 0.52019 | 0 |
| 988 | chr14 | 106329000 | 106330000 | 0.880 | 0.920 | IGHJ6 | 1.00000 | 0 |
| 989 | chr14 | 106330000 | 106331000 | 0.720 | 0.520 | IGHJ3;IGHJ4;IGHJ5; | 0.24363 | 0 |
| 990 | chr14 | 106331000 | 106332000 | 0.120 | 0.080 | IGHD7-27;IGHJ1;IGHJ2; | 1.00000 | 0 |
| 991 | chr14 | 106338000 | 106339000 | 0.040 | 0.000 | IGHD7-27 | 1.00000 | 0 |
| 992 | chr14 | 106350000 | 106351000 | 0.040 | 0.000 | IGHD4-23 | 1.00000 | 0 |
| 993 | chr14 | 106352000 | 106353000 | 0.000 | 0.040 | IGHD3-22 | 1.00000 | 0 |

| 994 | chr14 | 106353000 | 106354000 | 0.000 | 0.000 | IGHD2-21 | 1.00000 | 0 |
|---|---|---|---|---|---|---|---|---|
| 995 | chr14 | 106354000 | 106355000 | 0.000 | 0.040 | IGHD2-21 | 1.00000 | 0 |
| 996 | chr14 | 106355000 | 106356000 | 0.000 | 0.040 | IGHD2-21 | 1.00000 | 0 |
| 997 | chr14 | 106357000 | 106358000 | 0.040 | 0.080 | IGHD1-20;IGHD6-19; | 1.00000 | 0 |
| 998 | chr14 | 106358000 | 106359000 | 0.000 | 0.040 | IGHD5-18 | 1.00000 | 0 |
| 999 | chr14 | 106362000 | 106363000 | 0.000 | 0.000 | IGHD3-16 | 1.00000 | 0 |
| 1000 | chr14 | 106364000 | 106365000 | 0.040 | 0.000 | IGHD2-15 | 1.00000 | 0 |
| 1001 | chr14 | 106367000 | 106368000 | 0.040 | 0.000 | IGHD6-13 | 1.00000 | 0 |
| 1002 | chr14 | 106370000 | 106371000 | 0.080 | 0.000 | IGHD3-10;IGHD3-9; | 0.48980 | 0 |
| 1003 | chr14 | 106371000 | 106372000 | 0.040 | 0.000 | IGHD3-9 | 1.00000 | 0 |
| 1004 | chr14 | 106372000 | 106373000 | 0.040 | 0.000 | IGHD2-8 | 1.00000 | 0 |
| 1005 | chr14 | 106375000 | 106376000 | 0.000 | 0.000 | IGHD1-7 | 1.00000 | 0 |
| 1006 | chr14 | 106376000 | 106377000 | 0.000 | 0.040 | IGHD6-6 | 1.00000 | 0 |
| 1007 | chr14 | 106380000 | 106381000 | 0.000 | 0.040 | IGHD3-3 | 1.00000 | 0 |
| 1008 | chr14 | 106381000 | 106382000 | 0.000 | 0.040 | IGHD2-2 | 1.00000 | 0 |
| 1009 | chr14 | 106382000 | 106383000 | 0.040 | 0.120 | IGHD2-2 | 0.60921 | 0 |
| 1010 | chr14 | 106383000 | 106384000 | 0.080 | 0.040 | IGHD2-2 | 1.00000 | 0 |
| 1011 | chr14 | 106384000 | 106385000 | 0.040 | 0.040 | IGHD1-1 | 1.00000 | 0 |
| 1012 | chr14 | 106385000 | 106386000 | 0.080 | 0.040 | IGHD1-1 | 1.00000 | 0 |
| 1013 | chr14 | 106387000 | 106388000 | 0.040 | 0.080 | KIAA0125 | 1.00000 | 0 |
| 1014 | chr14 | 106405000 | 106406000 | 0.000 | 0.040 | IGHV6-1 | 1.00000 | 0 |
| 1015 | chr14 | 106406000 | 106407000 | 0.000 | 0.040 | IGHV6-1 | 1.00000 | 0 |
| 1016 | chr14 | 106419000 | 106420000 | 0.000 | 0.080 | IGHV6-1 | 0.48980 | 0 |
| 1017 | chr14 | 106452000 | 106453000 | 0.040 | 0.000 | IGHV1-2 | 1.00000 | 0 |
| 1018 | chr14 | 106453000 | 106454000 | 0.080 | 0.000 | IGHV1-2 | 0.48980 | 0 |
| 1019 | chr14 | 106454000 | 106455000 | 0.040 | 0.000 | IGHV1-2 | 1.00000 | 0 |
| 1020 | chr14 | 106494000 | 106495000 | 0.000 | 0.040 | IGHV2-5 | 1.00000 | 0 |
| 1021 | chr14 | 106518000 | 106519000 | 0.000 | 0.080 | IGHV3-7 | 0.48980 | 0 |
| 1022 | chr14 | 106519000 | 106520000 | 0.000 | 0.080 | IGHV3-7 | 0.48980 | 0 |
| 1023 | chr14 | 106539000 | 106540000 | 0.000 | 0.040 | IGHV1-8 | 1.00000 | 0 |
| 1024 | chr14 | 106552000 | 106553000 | 0.000 | 0.000 | IGHV3-9 | 1.00000 | 0 |
| 1025 | chr14 | 106573000 | 106574000 | 0.040 | 0.000 | IGHV3-11 | 1.00000 | 0 |
| 1026 | chr14 | 106574000 | 106575000 | 0.040 | 0.000 | IGHV3-11 | 1.00000 | 0 |
| 1027 | chr14 | 106578000 | 106579000 | 0.040 | 0.000 | IGHV3-11 | 1.00000 | 0 |

| 1028 | chr14 | 106579000 | 106580000 | 0.040 | 0.000 | IGHV3-11 | 1.00000 | 0 |
|------|-------|-----------|-----------|-------|-------|----------|---------|---|
| 1029 | chr14 | 106610000 | 106611000 | 0.000 | 0.000 | IGHV3-15 | 1.00000 | 0 |
| 1030 | chr14 | 106641000 | 106642000 | 0.040 | 0.040 | IGHV1-18 | 1.00000 | 0 |
| 1031 | chr14 | 106642000 | 106643000 | 0.040 | 0.000 | IGHV1-18 | 1.00000 | 0 |
| 1032 | chr14 | 106691000 | 106692000 | 0.000 | 0.000 | IGHV3-21 | 1.00000 | 0 |
| 1033 | chr14 | 106692000 | 106693000 | 0.000 | 0.040 | IGHV3-21 | 1.00000 | 0 |
| 1034 | chr14 | 106725000 | 106726000 | 0.120 | 0.160 | IGHV3-23 | 1.00000 | 0 |
| 1035 | chr14 | 106726000 | 106727000 | 0.040 | 0.080 | IGHV3-23 | 1.00000 | 0 |
| 1036 | chr14 | 106733000 | 106734000 | 0.000 | 0.080 | IGHV1-24 | 0.48980 | 0 |
| 1037 | chr14 | 106757000 | 106758000 | 0.000 | 0.040 | IGHV2-26 | 1.00000 | 0 |
| 1038 | chr14 | 106758000 | 106759000 | 0.000 | 0.040 | IGHV2-26 | 1.00000 | 0 |
| 1039 | chr14 | 106791000 | 106792000 | 0.040 | 0.040 | IGHV3-30 | 1.00000 | 0 |
| 1040 | chr14 | 106804000 | 106805000 | 0.040 | 0.040 | IGHV4-31 | 1.00000 | 0 |
| 1041 | chr14 | 106805000 | 106806000 | 0.040 | 0.040 | IGHV4-31 | 1.00000 | 0 |
| 1042 | chr14 | 106806000 | 106807000 | 0.000 | 0.000 | IGHV4-31 | 1.00000 | 0 |
| 1043 | chr14 | 106815000 | 106816000 | 0.000 | 0.040 | IGHV3-33 | 1.00000 | 0 |
| 1044 | chr14 | 106816000 | 106817000 | 0.000 | 0.160 | IGHV3-33 | 0.10986 | 0 |
| 1045 | chr14 | 106817000 | 106818000 | 0.000 | 0.080 | IGHV3-33 | 0.48980 | 0 |
| 1046 | chr14 | 106829000 | 106830000 | 0.160 | 0.080 | IGHV4-34 | 0.66710 | 0 |
| 1047 | chr14 | 106830000 | 106831000 | 0.160 | 0.000 | IGHV4-34 | 0.10986 | 0 |
| 1048 | chr14 | 106877000 | 106878000 | 0.040 | 0.080 | IGHV4-39 | 1.00000 | 0 |
| 1049 | chr14 | 106878000 | 106879000 | 0.000 | 0.080 | IGHV4-39 | 0.48980 | 0 |
| 1050 | chr14 | 106967000 | 106968000 | 0.040 | 0.040 | IGHV1-46 | 1.00000 | 0 |
| 1051 | chr14 | 106994000 | 106995000 | 0.000 | 0.120 | IGHV3-48 | 0.23469 | 0 |
| 1052 | chr14 | 106995000 | 106996000 | 0.000 | 0.000 | IGHV3-48 | 1.00000 | 0 |
| 1053 | chr14 | 107034000 | 107035000 | 0.040 | 0.000 | IGHV5-51 | 1.00000 | 0 |
| 1054 | chr14 | 107035000 | 107036000 | 0.080 | 0.000 | IGHV5-51 | 0.48980 | 0 |
| 1055 | chr14 | 107048000 | 107049000 | 0.000 | 0.000 | IGHV3-53 | 1.00000 | 0 |
| 1056 | chr14 | 107049000 | 107050000 | 0.000 | 0.000 | IGHV3-53 | 1.00000 | 0 |
| 1057 | chr14 | 107083000 | 107084000 | 0.040 | 0.040 | IGHV4-59 | 1.00000 | 0 |
| 1058 | chr14 | 107084000 | 107085000 | 0.000 | 0.040 | IGHV4-59 | 1.00000 | 0 |
| 1059 | chr14 | 107095000 | 107096000 | 0.040 | 0.000 | IGHV4-61 | 1.00000 | 0 |
| 1060 | chr14 | 107113000 | 107114000 | 0.080 | 0.000 | IGHV3-64 | 0.48980 | 0 |
| 1061 | chr14 | 107114000 | 107115000 | 0.080 | 0.000 | IGHV3-64 | 0.48980 | 0 |
| 1062 | chr14 | 107169000 | 107170000 | 0.200 | 0.240 | IGHV1-69 | 1.00000 | 0 |
| 1063 | chr14 | 107170000 | 107171000 | 0.360 | 0.280 | IGHV1-69 | 0.76241 | 0 |

| 1064 | chr14 | 107176000 | 107177000 | 0.200 | 0.200 | IGHV2-70 | 1.00000 | 0 |
|---|---|---|---|---|---|---|---|---|
| 1065 | chr14 | 107177000 | 107178000 | 0.080 | 0.040 | IGHV2-70 | 1.00000 | 0 |
| 1066 | chr14 | 107178000 | 107179000 | 0.200 | 0.520 | IGHV2-70 | 0.03776 | 0 |
| 1067 | chr14 | 107179000 | 107180000 | 0.240 | 0.360 | IGHV2-70 | 0.53803 | 0 |
| 1068 | chr14 | 107183000 | 107184000 | 0.000 | 0.000 | IGHV2-70 | 1.00000 | 0 |
| 1069 | chr14 | 107199000 | 107200000 | 0.000 | 0.080 | IGHV3-72 | 0.48980 | 0 |
| 1070 | chr14 | 107218000 | 107219000 | 0.000 | 0.080 | IGHV3-74 | 0.48980 | 0 |
| 1071 | chr14 | 107219000 | 107220000 | 0.000 | 0.160 | IGHV3-74 | 0.10986 | 0 |
| 1072 | chr14 | 107221000 | 107222000 | 0.000 | 0.080 | IGHV3-74 | 0.48980 | 0 |
| 1073 | chr14 | 107232000 | 107233000 | 0.000 | 0.000 | IGHV3-74 | 1.00000 | 0 |
| 1074 | chr14 | 107253000 | 107254000 | 0.000 | 0.000 | IGHV7-81 | 1.00000 | 0 |
| 1075 | chr14 | 107258000 | 107259000 | 0.000 | 0.040 | IGHV7-81 | 1.00000 | 0 |
| 1076 | chr14 | 107259000 | 107260000 | 0.160 | 0.200 | IGHV7-81 | 1.00000 | 0 |
| 1077 | chr15 | 45003000 | 45004000 | 0.040 | 0.040 | B2M | 1.00000 | 0 |
| 1078 | chr15 | 45007000 | 45008000 | 0.000 | 0.000 | B2M | 1.00000 | 0 |
| 1079 | chr15 | 45814000 | 45815000 | 0.000 | 0.040 | SLC30A4 | 1.00000 | 0 |
| 1080 | chr15 | 59664000 | 59665000 | 0.000 | 0.080 | MYO1E | 0.48980 | 0 |
| 1081 | chr15 | 65588000 | 65589000 | 0.040 | 0.000 | PARP16 | 1.00000 | 0 |
| 1082 | chr15 | 78332000 | 78333000 | 0.000 | 0.000 | TBC1D2B | 1.00000 | 0 |
| 1083 | chr15 | 83227000 | 83228000 | 0.000 | 0.040 | CPEB1 | 1.00000 | 0 |
| 1084 | chr15 | 86226000 | 86227000 | 0.040 | 0.040 | AKAP13 | 1.00000 | 0 |
| 1085 | chr15 | 86233000 | 86234000 | 0.040 | 0.000 | AKAP13 | 1.00000 | 0 |
| 1086 | chr15 | 86245000 | 86246000 | 0.080 | 0.120 | AKAP13 | 1.00000 | 0 |
| 1087 | chr16 | 368000 | 369000 | 0.000 | 0.040 | AXIN1 | 1.00000 | 0 |
| 1088 | chr16 | 3788000 | 3789000 | 0.040 | 0.000 | CREBBP | 1.00000 | 0 |
| 1089 | chr16 | 10971000 | 10972000 | 0.080 | 0.120 | CIITA | 1.00000 | 1 |
| 1090 | chr16 | 10972000 | 10973000 | 0.120 | 0.320 | CIITA | 0.17062 | 1 |
| 1091 | chr16 | 10973000 | 10974000 | 0.120 | 0.240 | CIITA | 0.46349 | 1 |
| 1092 | chr16 | 10974000 | 10975000 | 0.080 | 0.120 | CIITA | 1.00000 | 1 |
| 1093 | chr16 | 11348000 | 11349000 | 0.080 | 0.200 | SOCS1 | 0.41743 | 1 |
| 1094 | chr16 | 11349000 | 11350000 | 0.120 | 0.240 | SOCS1 | 0.46349 | 1 |
| 1095 | chr16 | 21167000 | 21168000 | 0.040 | 0.000 | DNAH3 | 1.00000 | 0 |
| 1096 | chr16 | 27325000 | 27326000 | 0.000 | 0.040 | CTD-3203P2.2 | 1.00000 | 0 |
| 1097 | chr16 | 27326000 | 27327000 | 0.080 | 0.080 | CTD-3203P2.2 | 1.00000 | 0 |
| 1098 | chr16 | 27327000 | 27328000 | 0.000 | 0.000 | IL4R | 1.00000 | 0 |
| 1099 | chr16 | 27414000 | 27415000 | 0.040 | 0.000 | IL21R | 1.00000 | 0 |

| ID | chr | start | end | val1 | val2 | gene | score | flag |
|---|---|---|---|---|---|---|---|---|
| 1100 | chr16 | 29248000 | 29249000 | 0.000 | 0.000 | 61E3.4 | 1.00000 | 0 |
| 1101 | chr16 | 31910000 | 31911000 | 0.040 | 0.000 | ZNF267 | 1.00000 | 0 |
| 1102 | chr16 | 46821000 | 46822000 | 0.000 | 0.040 | C16orf87 | 1.00000 | 0 |
| 1103 | chr16 | 50985000 | 50986000 | 0.040 | 0.000 | CYLD | 1.0000 | 0 |
| 1104 | chr16 | 64351000 | 64352000 | 0.000 | 0.040 | CDH11 | 1.00000 | 0 |
| 1105 | chr16 | 78398000 | 78399000 | 0.000 | 0.000 | WWOX | 1.00000 | 0 |
| 1106 | chr16 | 78615000 | 78616000 | 0.040 | 0.040 | WWOX | 1.00000 | 0 |
| 1107 | chr16 | 78753000 | 78754000 | 0.000 | 0.000 | WWOX | 1.00000 | 0 |
| 1108 | chr16 | 78811000 | 78812000 | 0.000 | 0.040 | WWOX | 1.00000 | 0 |
| 1109 | chr16 | 79988000 | 79989000 | 0.000 | 0.040 | MAF | 1.00000 | 0 |
| 1110 | chr16 | 81836000 | 81837000 | 0.000 | 0.000 | PLCG2 | 1.00000 | 1 |
| 1111 | chr16 | 85932000 | 85933000 | 0.040 | 0.040 | IRF8 | 1.0000 | 1 |
| 1112 | chr16 | 85933000 | 85934000 | 0.080 | 0.240 | IRF8 | 0.24672 | 1 |
| 1113 | chr16 | 85934000 | 85935000 | 0.040 | 0.000 | IRF8 | 1.00000 | 1 |
| 1114 | chr16 | 85936000 | 85937000 | 0.000 | 0.000 | IRF8 | 1.00000 | 1 |
| 1115 | chr16 | 88441000 | 88442000 | 0.040 | 0.000 | ZNF469 | 1.00000 | 0 |
| 1116 | chr17 | 3598000 | 3599000 | 0.040 | 0.040 | P2RX5;P2RX5-TAX1BP3P2RX5; | 1.00000 | 0 |
| 1117 | chr17 | 17286000 | 17287000 | 0.080 | 0.000 | SMCR9 | 0.48980 | 0 |
| 1118 | chr17 | 21194000 | 21195000 | 0.000 | 0.040 | MAP2K3 | 1.00000 | 0 |
| 1119 | chr17 | 29646000 | 29647000 | 0.000 | 0.000 | EVI2A | 1.00000 | 0 |
| 1120 | chr17 | 38020000 | 38021000 | 0.000 | 0.040 | IKZF3 | 1.00000 | 0 |
| 1121 | chr17 | 43662000 | 43663000 | 0.040 | 0.000 | PLEKHM1 | 1.00000 | 0 |
| 1122 | chr17 | 56408000 | 56409000 | 0.120 | 0.040 | BZRAP1 | 0.60921 | 0 |
| 1123 | chr17 | 56409000 | 56410000 | 0.360 | 0.200 | BZRAP1 | 0.34513 | 0 |
| 1124 | chr17 | 57916000 | 57917000 | 0.040 | 0.080 | VMP1 | 1.00000 | 1 |
| 1125 | chr17 | 57917000 | 57918000 | 0.040 | 0.080 | VMP1 | 1.00000 | 1 |
| 1126 | chr17 | 62007000 | 62008000 | 0.040 | 0.000 | CD79B | 1.00000 | 0 |
| 1127 | chr17 | 62008000 | 62009000 | 0.040 | 0.000 | CD79B | 1.00000 | 0 |
| 1128 | chr17 | 63067000 | 63068000 | 0.040 | 0.000 | GNA13 | 1.00000 | 0 |
| 1129 | chr17 | 65676000 | 65677000 | 0.040 | 0.000 | PITPNC1 | 1.00000 | 0 |
| 1130 | chr17 | 69365000 | 69366000 | 0.000 | 0.040 | AC007461.1 | 1.00000 | 0 |
| 1131 | chr17 | 70083000 | 70084000 | 0.000 | 0.000 | SOX9 | 1.00000 | 0 |
| 1132 | chr17 | 74733000 | 74734000 | 0.000 | 0.000 | SRSF2 | 1.00000 | 0 |
| 1133 | chr17 | 75447000 | 75448000 | 0.080 | 0.000 | 9-Sep-19 | 0.48980 | 0 |

| | | | | | | | | |
|---|---|---|---|---|---|---|---|---|
| 1134 | chr17 | 75448000 | 75449000 | 0.040 | 0.000 | 9-Sep-19 | 1.00000 | 0 |
| 1135 | chr17 | 76775000 | 76776000 | 0.000 | 0.000 | CYTH1 | 1.00000 | 0 |
| 1136 | chr17 | 80928000 | 80929000 | 0.000 | 0.000 | B3GNTL1 | 1.00000 | 0 |
| 1137 | chr17 | 80976000 | 80977000 | 0.000 | 0.040 | B3GNTL1 | 1.00000 | 0 |
| 1138 | chr18 | 2709000 | 2710000 | 0.000 | 0.000 | SMCHD1 | 1.00000 | 0 |
| 1139 | chr18 | 3600000 | 3601000 | 0.040 | 0.000 | DLGAP1 | 1.00000 | 0 |
| 1140 | chr18 | 12062000 | 12063000 | 0.000 | 0.000 | ANKRD62 | 1.00000 | 0 |
| 1141 | chr18 | 27771000 | 27772000 | 0.040 | 0.000 | DSC3 | 1.00000 | 0 |
| 1142 | chr18 | 28066000 | 28067000 | 0.000 | 0.040 | DSC3 | 1.00000 | 0 |
| 1143 | chr18 | 30349000 | 30350000 | 0.000 | 0.000 | AC012123.1;KLHL14; | 1.00000 | 0 |
| 1144 | chr18 | 36806000 | 36807000 | 0.040 | 0.000 | CELF4 | 1.00000 | 0 |
| 1145 | chr18 | 37751000 | 37752000 | 0.000 | 0.040 | PIK3C3 | 1.00000 | 0 |
| 1146 | chr18 | 38672000 | 38673000 | 0.000 | 0.040 | PIK3C3 | 1.00000 | 0 |
| 1147 | chr18 | 42168000 | 42169000 | 0.000 | 0.000 | SETBP1 | 1.00000 | 0 |
| 1148 | chr18 | 51952000 | 51953000 | 0.040 | 0.000 | C18orf54 | 1.00000 | 0 |
| 1149 | chr18 | 52447000 | 52448000 | 0.000 | 0.080 | RAB27B | 0.48980 | 0 |
| 1150 | chr18 | 52988000 | 52989000 | 0.040 | 0.000 | TCF4 | 1.00000 | 0 |
| 1151 | chr18 | 54653000 | 54654000 | 0.000 | 0.000 | WDR7 | 1.00000 | 0 |
| 1152 | chr18 | 60794000 | 60795000 | 0.000 | 0.080 | BCL2 | 0.48980 | 1 |
| 1153 | chr18 | 60805000 | 60806000 | 0.000 | 0.000 | BCL2 | 1.00000 | 1 |
| 1154 | chr18 | 60806000 | 60807000 | 0.000 | 0.120 | BCL2 | 0.23469 | 1 |
| 1155 | chr18 | 60809000 | 60810000 | 0.000 | 0.080 | BCL2 | 0.48980 | 1 |
| 1156 | chr18 | 60821000 | 60822000 | 0.000 | 0.040 | BCL2 | 1.00000 | 1 |
| 1157 | chr18 | 60825000 | 60826000 | 0.000 | 0.080 | BCL2 | 0.48980 | 1 |
| 1158 | chr18 | 60826000 | 60827000 | 0.000 | 0.040 | BCL2 | 1.00000 | 1 |
| 1159 | chr18 | 60828000 | 60829000 | 0.000 | 0.000 | BCL2 | 1.00000 | 1 |
| 1160 | chr18 | 60873000 | 60874000 | 0.000 | 0.040 | BCL2 | 1.00000 | 1 |
| 1161 | chr18 | 60875000 | 60876000 | 0.000 | 0.000 | BCL2 | 1.00000 | 1 |
| 1162 | chr18 | 60876000 | 60877000 | 0.000 | 0.040 | BCL2 | 1.00000 | 1 |
| 1163 | chr18 | 60983000 | 60984000 | 0.000 | 0.040 | BCL2 | 1.00000 | 1 |
| 1164 | chr18 | 60984000 | 60985000 | 0.040 | 0.240 | BCL2 | 0.02229 | 1 |
| 1165 | chr18 | 60985000 | 60986000 | 0.080 | 0.320 | BCL2 | 0.02322 | 1 |
| 1166 | chr18 | 60986000 | 60987000 | 0.080 | 0.320 | BCL2 | 0.07375 | 1 |
| 1167 | chr18 | 60987000 | 60988000 | 0.080 | 0.320 | BCL2 | 0.07375 | 1 |
| 1168 | chr18 | 60988000 | 60989000 | 0.080 | 0.280 | BCL2 | 0.13833 | 1 |

| | | | | | | | | |
|---|---|---|---|---|---|---|---|---|
| 1169 | chr18 | 61810000 | 61811000 | 0.040 | 0.000 | SERPINB8 | 1.00000 | 0 |
| 1170 | chr18 | 63080000 | 63081000 | 0.000 | 0.000 | CDH7 | 1.00000 | 0 |
| 1171 | chr18 | 63791000 | 63792000 | 0.000 | 0.000 | CDH7 | 1.00000 | 0 |
| 1172 | chr18 | 63875000 | 63876000 | 0.040 | 0.000 | CDH19 | 1.00000 | 0 |
| 1173 | chr18 | 64643000 | 64644000 | 0.000 | 0.000 | CDH19 | 1.00000 | 0 |
| 1174 | chr18 | 65863000 | 65864000 | 0.000 | 0.000 | TMX3 | 1.00000 | 0 |
| 1175 | chr18 | 66328000 | 66329000 | 0.040 | 0.040 | TMX3 | 1.00000 | 0 |
| 1176 | chr18 | 70462000 | 70463000 | 0.000 | 0.000 | NETO1 | 1.00000 | 0 |
| 1177 | chr18 | 73767000 | 73768000 | 0.040 | 0.000 | ZNF516 | 1.00000 | 1 |
| 1178 | chr18 | 76515000 | 76516000 | 0.040 | 0.000 | SALL3 | 1.00000 | 0 |
| 1179 | chr18 | 76724000 | 76725000 | 0.040 | 0.000 | SALL3 | 1.00000 | 0 |
| 1180 | chr18 | 76725000 | 76726000 | 0.040 | 0.000 | SALL3 | 1.00000 | 1 |
| 1181 | chr19 | 1612000 | 1613000 | 0.000 | 0.040 | TCF3 | 1.00000 | 1 |
| 1182 | chr19 | 2476000 | 2477000 | 0.040 | 0.040 | GADD45B | 1.00000 | 1 |
| 1183 | chr19 | 10304000 | 10305000 | 0.040 | 0.080 | DNMT1 | 1.00000 | 0 |
| 1184 | chr19 | 10305000 | 10306000 | 0.000 | 0.080 | DNMT1 | 0.48980 | 1 |
| 1185 | chr19 | 10335000 | 10336000 | 0.000 | 0.040 | S1PR2 | 1.00000 | 0 |
| 1186 | chr19 | 10340000 | 10341000 | 0.080 | 0.160 | S1PR2 | 0.66710 | 0 |
| 1187 | chr19 | 10341000 | 10342000 | 0.120 | 0.280 | S1PR2 | 0.28902 | 1 |
| 1188 | chr19 | 16030000 | 16031000 | 0.000 | 0.000 | CYP4F11 | 1.00000 | 0 |
| 1189 | chr19 | 16436000 | 16437000 | 0.040 | 0.000 | KLF2 | 1.00000 | 1 |
| 1190 | chr19 | 20889000 | 20890000 | 0.000 | 0.040 | ZNF626 | 1.00000 | 0 |
| 1191 | chr19 | 21073000 | 21074000 | 0.040 | 0.000 | ZNF85 | 1.00000 | 0 |
| 1192 | chr19 | 21092000 | 21093000 | 0.000 | 0.040 | ZNF85 | 1.00000 | 0 |
| 1193 | chr19 | 23841000 | 23842000 | 0.040 | 0.000 | ZNF675 | 1.00000 | 0 |
| 1194 | chr19 | 29256000 | 29257000 | 0.040 | 0.000 | UQCRFS1 | 1.00000 | 0 |
| 1195 | chr19 | 44183000 | 44184000 | 0.040 | 0.000 | PLAUR | 1.00000 | 0 |
| 1196 | chr19 | 50399000 | 50400000 | 0.040 | 0.040 | IL4I1 | 1.00000 | 0 |
| 1197 | chr19 | 53419000 | 53420000 | 0.000 | 0.000 | ZNF321P;ZNF816-ZNF321PZNF816-ZNF321PZNF321PZNF321PZNF816-ZNF321P; | 1.00000 | 0 |
| 1198 | chr20 | 15470000 | 15471000 | 0.000 | 0.040 | MACROD2 | 1.00000 | 0 |
| 1199 | chr20 | 23359000 | 23360000 | 0.000 | 0.000 | NAPB | 1.00000 | 0 |
| 1200 | chr20 | 23912000 | 23913000 | 0.000 | 0.000 | CST5 | 1.00000 | 0 |

| # | chr | | | | | gene | | |
|---|---|---|---|---|---|---|---|---|
| 1201 | chr20 | 46131000 | 46132000 | 0.040 | 0.120 | NCOA3 | 0.60921 | 1 |
| 1202 | chr20 | 49127000 | 49128000 | 0.000 | 0.000 | PTPN1 | 1.00000 | 0 |
| 1203 | chr20 | 49648000 | 49649000 | 0.040 | 0.000 | KCNG1 | 1.00000 | 0 |
| 1204 | chr20 | 61607000 | 61608000 | 0.000 | 0.000 | SLC17A9 | 1.00000 | 0 |
| 1205 | chr21 | 21597000 | 21598000 | 0.000 | 0.000 | NCAM2 | 1.00000 | 0 |
| 1206 | chr21 | 23458000 | 23459000 | 0.000 | 0.040 | NCAM2 | 1.00000 | 0 |
| 1207 | chr21 | 24998000 | 24999000 | 0.000 | 0.040 | MRPL39 | 1.00000 | 0 |
| 1208 | chr21 | 26935000 | 26936000 | 0.000 | 0.080 | MRPL39 | 0.48980 | 0 |
| 1209 | chr21 | 35779000 | 35780000 | 0.000 | 0.000 | SMIM11 | 1.00000 | 0 |
| 1210 | chr21 | 38779000 | 38780000 | 0.000 | 0.000 | DYRK1A | 1.00000 | 0 |
| 1211 | chr21 | 43254000 | 43255000 | 0.000 | 0.040 | PRDM15 | 1.00000 | 0 |
| 1212 | chr21 | 44612000 | 44613000 | 0.040 | 0.000 | CRYAA | 1.00000 | 0 |
| 1213 | chr21 | 45381000 | 45382000 | 0.000 | 0.000 | AGPAT3 | 1.00000 | 0 |
| 1214 | chr21 | 46058000 | 46059000 | 0.000 | 0.000 | KRTAP10-10 | 1.00000 | 0 |
| 1215 | chr22 | 19050000 | 19051000 | 0.000 | 0.000 | DGCR2 | 1.00000 | 0 |
| 1216 | chr22 | 20212000 | 20213000 | 0.040 | 0.000 | RTN4R | 1.00000 | 0 |
| 1217 | chr22 | 20708000 | 20709000 | 0.040 | 0.040 | FAM230A | 1.00000 | 0 |
| 1218 | chr22 | 21994000 | 21995000 | 0.000 | 0.000 | SDF2L1 | 1.00000 | 0 |
| 1219 | chr22 | 22379000 | 22380000 | 0.040 | 0.040 | IGLV4-69 | 1.00000 | 0 |
| 1220 | chr22 | 22380000 | 22381000 | 0.040 | 0.080 | IGLV4-69 | 1.00000 | 0 |
| 1221 | chr22 | 22381000 | 22382000 | 0.040 | 0.040 | IGLV4-69 | 1.00000 | 0 |
| 1222 | chr22 | 22385000 | 22386000 | 0.040 | 0.080 | IGLV8-61 | 1.00000 | 0 |
| 1223 | chr22 | 22452000 | 22453000 | 0.000 | 0.040 | IGLV8-61 | 1.00000 | 0 |
| 1224 | chr22 | 22453000 | 22454000 | 0.000 | 0.040 | IGLV4-60 | 1.00000 | 0 |
| 1225 | chr22 | 22516000 | 22517000 | 0.000 | 0.160 | IGLV4-60 | 0.10986 | 0 |
| 1226 | chr22 | 22517000 | 22518000 | 0.000 | 0.080 | IGLV6-57 | 0.48980 | 0 |
| 1227 | chr22 | 22550000 | 22551000 | 0.160 | 0.000 | IGLV10-54 | 0.10986 | 0 |
| 1228 | chr22 | 22569000 | 22570000 | 0.040 | 0.000 | IGLV1-51 | 1.00000 | 0 |
| 1229 | chr22 | 22676000 | 22677000 | 0.040 | 0.000 | IGLV1-51 | 1.00000 | 0 |
| 1230 | chr22 | 22677000 | 22678000 | 0.040 | 0.080 | IGLV5-48 | 1.00000 | 0 |
| 1231 | chr22 | 22707000 | 22708000 | 0.040 | 0.040 | IGLV1-47 | 1.00000 | 0 |
| 1232 | chr22 | 22712000 | 22713000 | 0.160 | 0.000 | IGLV7-46 | 0.34868 | 0 |
| 1233 | chr22 | 22723000 | 22724000 | 0.000 | 0.080 | IGLV7-46 | 1.00000 | 0 |
| 1234 | chr22 | 22724000 | 22725000 | 0.080 | 0.040 | IGLV5-45 | 1.00000 | 0 |
| 1235 | chr22 | 22730000 | 22731000 | 0.040 | 0.040 | IGLV5-45 | 1.00000 | 0 |
| 1236 | chr22 | 22731000 | 22732000 | 0.000 | 0.000 | | 1.00000 | 0 |

| | | | | | | | | |
|---|---|---|---|---|---|---|---|---|
| 1237 | chr22 | 22735000 | 22736000 | 0.080 | 0.120 | IGLV1-44 | 1.00000 | 0 |
| 1238 | chr22 | 22749000 | 22750000 | 0.120 | 0.040 | IGLV7-43 | 0.60921 | 0 |
| 1239 | chr22 | 22758000 | 22759000 | 0.080 | 0.040 | IGLV1-40 | 1.00000 | 0 |
| 1240 | chr22 | 22759000 | 22760000 | 0.080 | 0.080 | IGLV1-40 | 1.00000 | 0 |
| 1241 | chr22 | 22764000 | 22765000 | 0.120 | 0.080 | IGLV1-40 | 1.00000 | 0 |
| 1242 | chr22 | 23028000 | 23029000 | 0.000 | 0.040 | IGLV3-25 | 1.00000 | 0 |
| 1243 | chr22 | 23029000 | 23030000 | 0.040 | 0.120 | IGLV3-25 | 0.60921 | 0 |
| 1244 | chr22 | 23035000 | 23036000 | 0.000 | 0.040 | IGLV2-23 | 1.00000 | 0 |
| 1245 | chr22 | 23039000 | 23040000 | 0.000 | 0.000 | IGLV2-23 | 1.00000 | 0 |
| 1246 | chr22 | 23040000 | 23041000 | 0.120 | 0.040 | IGLV2-23 | 0.60921 | 0 |
| 1247 | chr22 | 23041000 | 23042000 | 0.040 | 0.000 | IGLV2-23 | 1.00000 | 0 |
| 1248 | chr22 | 23055000 | 23056000 | 0.040 | 0.000 | IGLV3-21 | 1.00000 | 0 |
| 1249 | chr22 | 23063000 | 23064000 | 0.040 | 0.000 | IGLV3-19 | 1.00000 | 0 |
| 1250 | chr22 | 23090000 | 23091000 | 0.120 | 0.000 | IGLV3-16 | 0.23469 | 0 |
| 1251 | chr22 | 23100000 | 23101000 | 0.040 | 0.000 | IGLV2-14 | 1.00000 | 0 |
| 1252 | chr22 | 23101000 | 23102000 | 0.120 | 0.040 | IGLV2-14 | 0.60921 | 0 |
| 1253 | chr22 | 23114000 | 23115000 | 0.000 | 0.000 | IGLV3-12 | 1.00000 | 0 |
| 1254 | chr22 | 23134000 | 23135000 | 0.000 | 0.000 | IGLV2-11 | 1.00000 | 0 |
| 1255 | chr22 | 23154000 | 23155000 | 0.120 | 0.000 | IGLV3-10 | 0.23469 | 0 |
| 1256 | chr22 | 23161000 | 23162000 | 0.000 | 0.000 | IGLV3-9 | 1.00000 | 0 |
| 1257 | chr22 | 23162000 | 23163000 | 0.000 | 0.000 | IGLV3-9 | 1.00000 | 0 |
| 1258 | chr22 | 23165000 | 23166000 | 0.000 | 0.000 | IGLV2-8 | 1.00000 | 0 |
| 1259 | chr22 | 23192000 | 23193000 | 0.080 | 0.080 | IGLV4-3 | 1.00000 | 0 |
| 1260 | chr22 | 23197000 | 23198000 | 0.040 | 0.000 | IGLV4-3 | 1.00000 | 0 |
| 1261 | chr22 | 23198000 | 23199000 | 0.160 | 0.040 | IGLV4-3 | 0.34868 | 0 |
| 1262 | chr22 | 23199000 | 23200000 | 0.200 | 0.200 | IGLV4-3 | 1.00000 | 0 |
| 1263 | chr22 | 23203000 | 23204000 | 0.000 | 0.000 | IGLV4-3 | 1.00000 | 0 |
| 1264 | chr22 | 23204000 | 23205000 | 0.080 | 0.000 | IGLV4-3 | 0.48980 | 0 |
| 1265 | chr22 | 23205000 | 23206000 | 0.000 | 0.000 | IGLV4-3 | 1.00000 | 0 |
| 1266 | chr22 | 23207000 | 23208000 | 0.000 | 0.040 | IGLV4-3 | 1.00000 | 0 |
| 1267 | chr22 | 23209000 | 23210000 | 0.000 | 0.040 | IGLV4-3 | 1.00000 | 0 |
| 1268 | chr22 | 23213000 | 23214000 | 0.120 | 0.040 | IGLV4-3 | 0.60921 | 0 |
| 1269 | chr22 | 23214000 | 23215000 | 0.040 | 0.040 | IGLV4-3 | 1.00000 | 0 |
| 1270 | chr22 | 23219000 | 23220000 | 0.080 | 0.000 | IGLV3-1 | 0.48980 | 0 |
| 1271 | chr22 | 23220000 | 23221000 | 0.080 | 0.000 | IGLV3-1 | 0.48980 | 0 |
| 1272 | chr22 | 23222000 | 23223000 | 0.040 | 0.120 | IGLV3-1 | 0.60921 | 0 |

| | | | | | | | | |
|---|---|---|---|---|---|---|---|---|
| 1273 | chr22 | 23223000 | 23224000 | 0.320 | 0.520 | IGLV3-1 | 0.25159 | 0 |
| 1274 | chr22 | 23224000 | 23225000 | 0.080 | 0.080 | IGLV3-1 | 1.00000 | 0 |
| 1275 | chr22 | 23226000 | 23227000 | 0.120 | 0.000 | IGLV3-1 | 0.23469 | 0 |
| 1276 | chr22 | 23227000 | 23228000 | 0.200 | 0.360 | IGLL5 | 0.34513 | 0 |
| 1277 | chr22 | 23228000 | 23229000 | 0.240 | 0.200 | IGLL5 | 1.00000 | 0 |
| 1278 | chr22 | 23229000 | 23230000 | 0.040 | 0.160 | IGLL5 | 0.34868 | 0 |
| 1279 | chr22 | 23230000 | 23231000 | 0.440 | 0.600 | IGLL5 | 0.39610 | 0 |
| 1280 | chr22 | 23231000 | 23232000 | 0.480 | 0.440 | IGLL5 | 1.00000 | 0 |
| 1281 | chr22 | 23232000 | 23233000 | 0.320 | 0.240 | IGLL5 | 0.75361 | 0 |
| 1282 | chr22 | 23233000 | 23234000 | 0.200 | 0.040 | IGLJ1 | 0.18946 | 0 |
| 1283 | chr22 | 23234000 | 23235000 | 0.200 | 0.080 | IGLJ1 | 0.41743 | 0 |
| 1284 | chr22 | 23235000 | 23236000 | 0.320 | 0.080 | IGLJ1;IGLL5; | 0.07375 | 0 |
| 1285 | chr22 | 23236000 | 23237000 | 0.240 | 0.200 | IGLJ1;IGLL5; | 1.00000 | 0 |
| 1286 | chr22 | 23237000 | 23238000 | 0.040 | 0.160 | IGLC1;IGLL5; | 0.34868 | 0 |
| 1287 | chr22 | 23241000 | 23242000 | 0.040 | 0.040 | IGLJ2 | 1.00000 | 0 |
| 1288 | chr22 | 23242000 | 23243000 | 0.120 | 0.040 | IGLC2 | 0.60921 | 0 |
| 1289 | chr22 | 23243000 | 23244000 | 0.080 | 0.040 | IGLC2 | 1.00000 | 0 |
| 1290 | chr22 | 23244000 | 23245000 | 0.000 | 0.040 | IGLC2 | 1.00000 | 0 |
| 1291 | chr22 | 23247000 | 23248000 | 0.280 | 0.160 | IGLJ3 | 0.49620 | 0 |
| 1292 | chr22 | 23248000 | 23249000 | 0.040 | 0.040 | IGLC3 | 1.00000 | 0 |
| 1293 | chr22 | 23249000 | 23250000 | 0.040 | 0.000 | IGLC3 | 1.00000 | 0 |
| 1294 | chr22 | 23260000 | 23261000 | 0.000 | 0.000 | IGLJ6 | 1.00000 | 0 |
| 1295 | chr22 | 23261000 | 23262000 | 0.000 | 0.000 | IGLJ6 | 1.00000 | 0 |
| 1296 | chr22 | 23263000 | 23264000 | 0.000 | 0.040 | IGLJ7 | 1.00000 | 0 |
| 1297 | chr22 | 23264000 | 23265000 | 0.040 | 0.040 | IGLC7 | 1.00000 | 0 |
| 1298 | chr22 | 23273000 | 23274000 | 0.080 | 0.040 | IGLC7 | 1.00000 | 0 |
| 1299 | chr22 | 23277000 | 23278000 | 0.040 | 0.040 | IGLC7 | 1.00000 | 0 |
| 1300 | chr22 | 23278000 | 23279000 | 0.000 | 0.120 | IGLC7 | 0.23469 | 0 |
| 1301 | chr22 | 23281000 | 23282000 | 0.040 | 0.000 | IGLC7 | 1.00000 | 0 |
| 1302 | chr22 | 23282000 | 23283000 | 0.080 | 0.160 | IGLC7 | 0.66710 | 0 |
| 1303 | chr22 | 23284000 | 23285000 | 0.000 | 0.000 | IGLC7 | 1.00000 | 0 |
| 1304 | chr22 | 23523000 | 23524000 | 0.000 | 0.080 | BCR | 0.48980 | 0 |
| 1305 | chr22 | 23524000 | 23525000 | 0.000 | 0.000 | BCR | 1.00000 | 0 |
| 1306 | chr22 | 27236000 | 27237000 | 0.000 | 0.000 | CRYBA4 | 1.00000 | 0 |
| 1307 | chr22 | 29195000 | 29196000 | 0.040 | 0.040 | XBP1 | 1.00000 | 0 |
| 1308 | chr22 | 29196000 | 29197000 | 0.040 | 0.040 | XBP1 | 1.00000 | 0 |

| | | | | | | | | |
|---|---|---|---|---|---|---|---|---|
| 1309 | chr22 | 31826000 | 31827000 | 0.040 | 0.000 | DRG1 | 1.00000 | 0 |
| 1310 | chr22 | 32982000 | 32983000 | 0.000 | 0.040 | SYN3 | 1.00000 | 0 |
| 1311 | chr22 | 39852000 | 39853000 | 0.040 | 0.000 | TAB1 | 1.00000 | 0 |
| 1312 | chr22 | 39854000 | 39855000 | 0.000 | 0.000 | TAB1 | 1.00000 | 0 |
| 1313 | chr22 | 43360000 | 43361000 | 0.000 | 0.000 | PACSIN2 | 1.00000 | 0 |
| 1314 | chr22 | 47186000 | 47187000 | 0.000 | 0.000 | TBC1D22A | 1.00000 | 0 |
| 1315 | chr22 | 47738000 | 47739000 | 0.000 | 0.000 | LL22NC03-75H12.2 | 1.00000 | 0 |
| 1316 | chr22 | 50336000 | 50337000 | 0.000 | 0.000 | CRELD2 | 1.00000 | 0 |
| 1317 | chrX | 228000 | 229000 | 0.000 | 0.000 | GTPBP6 | 1.00000 | 0 |
| 1318 | chrX | 1514000 | 1515000 | 0.000 | 0.040 | SLC25A6 | 1.00000 | 0 |
| 1319 | chrX | 1611000 | 1612000 | 0.040 | 0.040 | P2RY8 | 1.00000 | 1 |
| 1320 | chrX | 12993000 | 12994000 | 0.320 | 0.280 | TMSB4X | 1.00000 | 1 |
| 1321 | chrX | 12994000 | 12995000 | 0.200 | 0.160 | TMSB4X | 1.00000 | 1 |
| 1322 | chrX | 13419000 | 13420000 | 0.000 | 0.040 | ATXN3L | 1.00000 | 0 |
| 1323 | chrX | 27031000 | 27032000 | 0.080 | 0.040 | DCAF8L2 | 1.00000 | 0 |
| 1324 | chrX | 32315000 | 32316000 | 0.000 | 0.000 | DMD | 1.00000 | 1 |
| 1325 | chrX | 32317000 | 32318000 | 0.000 | 0.000 | DMD | 1.00000 | 1 |
| 1326 | chrX | 33144000 | 33145000 | 0.000 | 0.000 | DMD | 1.00000 | 1 |
| 1327 | chrX | 33145000 | 33146000 | 0.000 | 0.040 | DMD | 1.00000 | 1 |
| 1328 | chrX | 33146000 | 33147000 | 0.080 | 0.120 | DMD | 1.00000 | 1 |
| 1329 | chrX | 41366000 | 41367000 | 0.040 | 0.000 | CASK | 1.00000 | 0 |
| 1330 | chrX | 42802000 | 42803000 | 0.080 | 0.120 | MAOA | 1.00000 | 0 |
| 1331 | chrX | 48775000 | 48776000 | 0.120 | 0.040 | PIM2 | 0.60921 | 1 |
| 1332 | chrX | 48776000 | 48777000 | 0.080 | 0.000 | PIM2 | 0.48980 | 1 |
| 1333 | chrX | 64071000 | 64072000 | 0.120 | 0.080 | ZC4H2 | 1.00000 | 0 |
| 1334 | chrX | 67030000 | 67031000 | 0.000 | 0.000 | AR | 1.00000 | 0 |
| 1335 | chrX | 80258000 | 80259000 | 0.000 | 0.000 | HMGN5 | 1.00000 | 0 |
| 1336 | chrX | 81172000 | 81173000 | 0.040 | 0.000 | SH3BGRL | 1.00000 | 0 |
| 1337 | chrX | 87742000 | 87743000 | 0.040 | 0.000 | CPXCR1 | 1.00000 | 0 |
| 1338 | chrX | 87831000 | 87832000 | 0.000 | 0.000 | CPXCR1 | 1.00000 | 0 |
| 1339 | chrX | 88263000 | 88264000 | 0.000 | 0.000 | CPXCR1 | 1.00000 | 0 |
| 1340 | chrX | 88458000 | 88459000 | 0.040 | 0.000 | CPXCR1 | 1.00000 | 0 |
| 1341 | chrX | 92647000 | 92648000 | 0.000 | 0.000 | NAP1L3 | 1.00000 | 0 |
| 1342 | chrX | 93279000 | 93280000 | 0.040 | 0.000 | FAM133A | 1.00000 | 0 |
| 1343 | chrX | 94079000 | 94080000 | 0.040 | 0.000 | FAM133A | 1.00000 | 0 |

| 1344 | chrX | 104006000 | 104007000 | 0.040 | 0.000 | IL1RAPL2 | 1.00000 | 0 |
|------|------|-----------|-----------|-------|-------|----------|---------|---|
| 1345 | chrX | 104269000 | 104270000 | 0.040 | 0.000 | IL1RAPL2 | 1.00000 | 0 |
| 1346 | chrX | 106132000 | 106133000 | 0.000 | 0.000 | RIPPLY1 | 1.00000 | 0 |
| 1347 | chrX | 113095000 | 113096000 | 0.000 | 0.040 | HTR2C | 1.00000 | 0 |
| 1348 | chrX | 115676000 | 115677000 | 0.040 | 0.000 | CXorf61 | 1.00000 | 0 |
| 1349 | chrX | 124996000 | 124997000 | 0.000 | 0.000 | DCAF12L2 | 1.00000 | 0 |
| 1350 | chrX | 125708000 | 125709000 | 0.000 | 0.000 | DCAF12L1 | 1.00000 | 0 |
| 1351 | chrX | 128565000 | 128566000 | 0.040 | 0.040 | SMARCA1 | 1.00000 | 0 |
| 1352 | chrX | 129643000 | 129644000 | 0.000 | 0.040 | RBMX2 | 1.00000 | 0 |
| 1353 | chrX | 134903000 | 134904000 | 0.000 | 0.000 | CT45A3;CT45A4; | 1.00000 | 0 |
| 1354 | chrX | 140846000 | 140847000 | 0.040 | 0.000 | SPANXD;SPANXE; | 1.00000 | 0 |
| 1355 | chrX | 143750000 | 143751000 | 0.000 | 0.000 | SPANXN1 | 1.00000 | 0 |
| 1356 | chrX | 145016000 | 145017000 | 0.040 | 0.000 | TMEM257 | 1.00000 | 0 |

Table 3

| # | Chromosome | Region Start | Region End | Closest Gene | Reason for Inclusion |
|---|---|---|---|---|---|
| 1 | chr1 | 2306311 | 2306832 | MORN1 | Genotyping |
| 2 | chr1 | 2334441 | 2334664 | RER1 | Genotyping |
| 3 | chr1 | 2334671 | 2335161 | RER1 | Genotyping |
| 4 | chr1 | 2488006 | 2488247 | TNFRSF14 | Phased Variants |
| 5 | chr1 | 2489111 | 2489330 | TNFRSF14 | Genotyping |
| 6 | chr1 | 2489726 | 2489973 | TNFRSF14 | Genotyping |
| 7 | chr1 | 2491206 | 2491455 | TNFRSF14 | Genotyping |
| 8 | chr1 | 2492036 | 2492175 | TNFRSF14 | Genotyping |
| 9 | chr1 | 2493051 | 2493333 | TNFRSF14 | Genotyping |
| 10 | chr1 | 2494241 | 2494376 | TNFRSF14 | Genotyping |
| 11 | chr1 | 2494556 | 2494745 | TNFRSF14 | Genotyping |
| 12 | chr1 | 3547350 | 3547715 | WRAP73 | Genotyping |
| 13 | chr1 | 3747620 | 3747798 | CEP104 | Genotyping |
| 14 | chr1 | 3800045 | 3800148 | DFFB | Genotyping |
| 15 | chr1 | 3800155 | 3800363 | DFFB | Genotyping |
| 16 | chr1 | 4472438 | 4472621 | AJAP1 | Genotyping |
| 17 | chr1 | 4476348 | 4476627 | AJAP1 | Genotyping |
| 18 | chr1 | 9784432 | 9784540 | PIK3CD | Genotyping |
| 19 | chr1 | 23885407 | 23885541 | ID3 | Genotyping |
| 20 | chr1 | 23885582 | 23885938 | ID3 | Genotyping |
| 21 | chr1 | 27059146 | 27059321 | ARID1A | Genotyping |
| 22 | chr1 | 27101071 | 27101294 | ARID1A | Genotyping |
| 23 | chr1 | 27101401 | 27101613 | ARID1A | Genotyping |
| 24 | chr1 | 27105466 | 27105671 | ARID1A | Genotyping |
| 25 | chr1 | 27106311 | 27106523 | ARID1A | Genotyping |
| 26 | chr1 | 27106711 | 27106920 | ARID1A | Genotyping |
| 27 | chr1 | 29069531 | 29070185 | YTHDF2 | Genotyping |
| 28 | chr1 | 34404022 | 34404171 | CSMD2 | Phased Variants |
| 29 | chr1 | 35472492 | 35472739 | ZMYM6 | Genotyping |
| 30 | chr1 | 61553802 | 61554330 | NFIA | Genotyping |
| 31 | chr1 | 72334891 | 72335045 | NEGR1 | Phased Variants |
| 32 | chr1 | 72335051 | 72335120 | NEGR1 | Phased Variants |
| 33 | chr1 | 85733207 | 85733640 | BCL10 | Phased Variants |
| 34 | chr1 | 85736272 | 85736619 | BCL10 | Genotyping |
| 35 | chr1 | 85741932 | 85742068 | BCL10 | Genotyping |
| 36 | chr1 | 86591437 | 86591909 | COL24A1 | Genotyping |
| 37 | chr1 | 107866871 | 107867579 | NTNG1 | Genotyping |
| 38 | chr1 | 109649126 | 109649304 | C1orf194 | Genotyping |
| 39 | chr1 | 109822181 | 109822805 | PSRC1 | Genotyping |
| 40 | chr1 | 110561141 | 110561757 | AHCYL1 | Genotyping |
| 41 | chr1 | 111441722 | 111442219 | CD53 | Genotyping |
| 42 | chr1 | 111715727 | 111715908 | CEPT1 | Genotyping |

| 43 | chr1 | 117078642 | 117078856 | CD58 | Genotyping |
|----|------|-----------|-----------|------|------------|
| 44 | chr1 | 117086927 | 117087172 | CD58 | Genotyping |
| 45 | chr1 | 120457960 | 120459297 | NOTCH2 | Genotyping |
| 46 | chr1 | 160319283 | 160319532 | NCSTN | Genotyping |
| 47 | chr1 | 181452914 | 181453131 | CACNA1E | Genotyping |
| 48 | chr1 | 185833555 | 185833832 | HMCN1 | Genotyping |
| 49 | chr1 | 185972790 | 185973006 | HMCN1 | Genotyping |
| 50 | chr1 | 186062580 | 186062797 | HMCN1 | Genotyping |
| 51 | chr1 | 186083050 | 186083301 | HMCN1 | Genotyping |
| 52 | chr1 | 186143590 | 186143828 | HMCN1 | Genotyping |
| 53 | chr1 | 186158895 | 186159102 | HMCN1 | Genotyping |
| 54 | chr1 | 190067139 | 190068194 | FAM5C | Genotyping |
| 55 | chr1 | 201038552 | 201038756 | CACNA1S | Genotyping |
| 56 | chr1 | 203274697 | 203275926 | BTG2 | Phased Variants |
| 57 | chr1 | 203276207 | 203276586 | BTG2 | Genotyping |
| 58 | chr1 | 226923691 | 226925200 | ITPKB | Phased Variants |
| 59 | chr1 | 227842646 | 227842718 | ZNF678 | Genotyping |
| 60 | chr2 | 1652010 | 1652858 | PXDN | Genotyping |
| 61 | chr2 | 48027958 | 48028159 | MSH6 | Genotyping |
| 62 | chr2 | 48059883 | 48060051 | FBXO11 | Genotyping |
| 63 | chr2 | 48065973 | 48066184 | FBXO11 | Genotyping |
| 64 | chr2 | 55237198 | 55237610 | RTN4 | Genotyping |
| 65 | chr2 | 56149510 | 56150116 | EFEMP1 | Genotyping |
| 66 | chr2 | 58520800 | 58521222 | FANCL | Genotyping |
| 67 | chr2 | 59821914 | 59822083 | BCL11A | Genotyping |
| 68 | chr2 | 60773084 | 60773479 | BCL11A | Genotyping |
| 69 | chr2 | 61118794 | 61118998 | REL | Genotyping |
| 70 | chr2 | 61145504 | 61145785 | REL | Genotyping |
| 71 | chr2 | 61148869 | 61149644 | REL | Genotyping |
| 72 | chr2 | 61441169 | 61441870 | USP34 | Genotyping |
| 73 | chr2 | 61719434 | 61719642 | XPO1 | Genotyping |
| 74 | chr2 | 62934009 | 62934460 | EHBP1 | Genotyping |
| 75 | chr2 | 63217829 | 63218002 | EHBP1 | Genotyping |
| 76 | chr2 | 63335242 | 63335600 | WDPCP | Genotyping |
| 77 | chr2 | 63631157 | 63631817 | WDPCP | Genotyping |
| 78 | chr2 | 63826277 | 63826429 | MDH1 | Genotyping |
| 79 | chr2 | 65258145 | 65258367 | SLC1A4 | Phased Variants |
| 80 | chr2 | 65593035 | 65593153 | SPRED2 | Phased Variants |
| 81 | chr2 | 65593180 | 65593250 | SPRED2 | Phased Variants |
| 82 | chr2 | 77746602 | 77746988 | LRRTM4 | Genotyping |
| 83 | chr2 | 80801235 | 80801513 | CTNNA2 | Genotyping |
| 84 | chr2 | 88906681 | 88906861 | EIF2AK3 | Phased Variants |
| 85 | chr2 | 89127261 | 89127335 | IGKC | Phased Variants |

| 86 | chr2 | 89127461 | 89127946 | IGKC | Phased Variants |
|---|---|---|---|---|---|
| 87 | chr2 | 89128431 | 89128574 | IGKC | Phased Variants |
| 88 | chr2 | 89131726 | 89132295 | IGKC | Phased Variants |
| 89 | chr2 | 89140556 | 89140755 | IGKC | Phased Variants |
| 90 | chr2 | 89140886 | 89141350 | IGKC | Phased Variants |
| 91 | chr2 | 89157326 | 89157609 | IGKC | Phased Variants |
| 92 | chr2 | 89157626 | 89158011 | IGKC | Phased Variants |
| 93 | chr2 | 89158036 | 89158938 | IGKC | Phased Variants |
| 94 | chr2 | 89158941 | 89159493 | IGKJ5 | Phased Variants |
| 95 | chr2 | 89159511 | 89161445 | IGKJ1 | Phased Variants |
| 96 | chr2 | 89161926 | 89162149 | IGKJ1 | Phased Variants |
| 97 | chr2 | 89162776 | 89163285 | IGKJ1 | Phased Variants |
| 98 | chr2 | 89163306 | 89163837 | IGKJ1 | Phased Variants |
| 99 | chr2 | 89163861 | 89164838 | IGKJ1 | Phased Variants |
| 100 | chr2 | 89164866 | 89165181 | IGKJ1 | Phased Variants |
| 101 | chr2 | 89165191 | 89165644 | IGKJ1 | Phased Variants |
| 102 | chr2 | 89184966 | 89185186 | IGKV4-1 | Phased Variants |
| 103 | chr2 | 89185196 | 89185704 | IGKV4-1 | Phased Variants |
| 104 | chr2 | 89196226 | 89196411 | IGKV5-2 | Phased Variants |
| 105 | chr2 | 89196851 | 89197324 | IGKV5-2 | Phased Variants |
| 106 | chr2 | 89214836 | 89215040 | IGKV5-2 | Phased Variants |
| 107 | chr2 | 89246681 | 89246772 | IGKV1-5 | Phased Variants |
| 108 | chr2 | 89246786 | 89246857 | IGKV1-5 | Phased Variants |
| 109 | chr2 | 89246911 | 89247053 | IGKV1-5 | Phased Variants |
| 110 | chr2 | 89247096 | 89247215 | IGKV1-5 | Phased Variants |
| 111 | chr2 | 89247526 | 89247628 | IGKV1-5 | Phased Variants |
| 112 | chr2 | 89247641 | 89247735 | IGKV1-5 | Phased Variants |
| 113 | chr2 | 89247831 | 89248010 | IGKV1-5 | Phased Variants |
| 114 | chr2 | 89265756 | 89265829 | IGKV1-6 | Genotyping |
| 115 | chr2 | 89265936 | 89266013 | IGKV1-6 | Genotyping |
| 116 | chr2 | 89291906 | 89291981 | IGKV1-8 | Phased Variants |
| 117 | chr2 | 89292131 | 89292217 | IGKV1-8 | Phased Variants |
| 118 | chr2 | 89442291 | 89442561 | IGKV3-20 | Phased Variants |
| 119 | chr2 | 89442616 | 89443259 | IGKV3-20 | Phased Variants |
| 120 | chr2 | 89475781 | 89476009 | IGKV2-24 | Genotyping |
| 121 | chr2 | 89476041 | 89476122 | IGKV2-24 | Genotyping |
| 122 | chr2 | 89544331 | 89544608 | IGKV2-30 | Genotyping |
| 123 | chr2 | 89544656 | 89544899 | IGKV2-30 | Phased Variants |
| 124 | chr2 | 89976276 | 89976426 | IGKV2D-30 | Genotyping |
| 125 | chr2 | 89986776 | 89987023 | IGKV2D-29 | Genotyping |
| 126 | chr2 | 89987031 | 89987108 | IGKV2D-29 | Genotyping |
| 127 | chr2 | 90025206 | 90025289 | IGKV2D-26 | Genotyping |
| 128 | chr2 | 90025296 | 90025378 | IGKV2D-26 | Genotyping |

| 129 | chr2 | 90025471 | 90025554 | IGKV2D-26 | Genotyping |
|-----|------|----------|----------|-----------|------------|
| 130 | chr2 | 90077981 | 90078054 | IGKV3D-20 | Genotyping |
| 131 | chr2 | 90078136 | 90078222 | IGKV3D-20 | Genotyping |
| 132 | chr2 | 90078251 | 90078335 | IGKV3D-20 | Genotyping |
| 133 | chr2 | 90121891 | 90122008 | IGKV1D-17 | Genotyping |
| 134 | chr2 | 90122021 | 90122157 | IGKV1D-17 | Genotyping |
| 135 | chr2 | 90212016 | 90212093 | IGKV3D-11 | Genotyping |
| 136 | chr2 | 90212196 | 90212278 | IGKV3D-11 | Genotyping |
| 137 | chr2 | 90249151 | 90249275 | IGKV1D-43 | Genotyping |
| 138 | chr2 | 90249346 | 90249419 | IGKV1D-43 | Genotyping |
| 139 | chr2 | 90259931 | 90260059 | IGKV1D-8 | Genotyping |
| 140 | chr2 | 90260181 | 90260258 | IGKV1D-8 | Genotyping |
| 141 | chr2 | 96809889 | 96810144 | DUSP2 | Genotyping |
| 142 | chr2 | 96810164 | 96810374 | DUSP2 | Phased Variants |
| 143 | chr2 | 100758483 | 100758660 | AFF3 | Phased Variants |
| 144 | chr2 | 103148733 | 103148948 | SLC9A4 | Genotyping |
| 145 | chr2 | 117951919 | 117952057 | DDX18 | Phased Variants |
| 146 | chr2 | 136872525 | 136872740 | CXCR4 | Genotyping |
| 147 | chr2 | 136874415 | 136874797 | CXCR4 | Phased Variants |
| 148 | chr2 | 136874920 | 136875662 | CXCR4 | Phased Variants |
| 149 | chr2 | 141245127 | 141245373 | LRP1B | Genotyping |
| 150 | chr2 | 145162401 | 145162624 | ZEB2 | Genotyping |
| 151 | chr2 | 145187091 | 145187638 | ZEB2 | Genotyping |
| 152 | chr2 | 145270956 | 145271394 | ZEB2 | Genotyping |
| 153 | chr2 | 145275631 | 145275744 | ZEB2 | Genotyping |
| 154 | chr2 | 145275756 | 145276174 | ZEB2 | Genotyping |
| 155 | chr2 | 145278026 | 145278305 | ZEB2 | Genotyping |
| 156 | chr2 | 145278311 | 145278659 | ZEB2 | Genotyping |
| 157 | chr2 | 145692901 | 145693081 | ZEB2 | Genotyping |
| 158 | chr2 | 148680516 | 148680692 | ACVR2A | Genotyping |
| 159 | chr2 | 169781120 | 169781352 | ABCB11 | Genotyping |
| 160 | chr2 | 170101185 | 170101401 | LRP2 | Genotyping |
| 161 | chr2 | 198950434 | 198951003 | PLCL1 | Genotyping |
| 162 | chr2 | 242793232 | 242793447 | PDCD1 | Genotyping |
| 163 | chr2 | 242794037 | 242794192 | PDCD1 | Genotyping |
| 164 | chr2 | 242794317 | 242794537 | PDCD1 | Genotyping |
| 165 | chr2 | 242794822 | 242795040 | PDCD1 | Genotyping |
| 166 | chr2 | 242800887 | 242801093 | PDCD1 | Genotyping |
| 167 | chr3 | 7620223 | 7620990 | GRM7 | Genotyping |
| 168 | chr3 | 16419204 | 16419479 | RFTN1 | Phased Variants |
| 169 | chr3 | 38180129 | 38180549 | MYD88 | Genotyping |
| 170 | chr3 | 38181334 | 38181509 | MYD88 | Genotyping |
| 171 | chr3 | 38181854 | 38182099 | MYD88 | Genotyping |

| 172 | chr3 | 38182194 | 38182407 | MYD88 | Genotyping |
|---|---|---|---|---|---|
| 173 | chr3 | 38182554 | 38182844 | MYD88 | Genotyping |
| 174 | chr3 | 49397608 | 49397717 | RHOA | Genotyping |
| 175 | chr3 | 49397718 | 49397827 | RHOA | Genotyping |
| 176 | chr3 | 49399903 | 49400084 | RHOA | Genotyping |
| 177 | chr3 | 49405833 | 49406013 | RHOA | Genotyping |
| 178 | chr3 | 49412838 | 49413046 | RHOA | Genotyping |
| 179 | chr3 | 64547204 | 64547477 | ADAMTS9 | Genotyping |
| 180 | chr3 | 64579889 | 64580094 | ADAMTS9 | Genotyping |
| 181 | chr3 | 71551101 | 71551497 | EIF4E3 | Phased Variants |
| 182 | chr3 | 140281598 | 140281875 | CLSTN2 | Genotyping |
| 183 | chr3 | 164730700 | 164730888 | SI | Genotyping |
| 184 | chr3 | 165548198 | 165548680 | BCHE | Genotyping |
| 185 | chr3 | 176750699 | 176750928 | TBL1XR1 | Genotyping |
| 186 | chr3 | 176767759 | 176767977 | TBL1XR1 | Genotyping |
| 187 | chr3 | 176769304 | 176769543 | TBL1XR1 | Genotyping |
| 188 | chr3 | 176771659 | 176771732 | TBL1XR1 | Genotyping |
| 189 | chr3 | 183209758 | 183209937 | KLHL6 | Genotyping |
| 190 | chr3 | 183210258 | 183210544 | KLHL6 | Genotyping |
| 191 | chr3 | 183272308 | 183272521 | KLHL6 | Phased Variants |
| 192 | chr3 | 183273063 | 183273456 | KLHL6 | Phased Variants |
| 193 | chr3 | 184580663 | 184580872 | VPS8 | Genotyping |
| 194 | chr3 | 185146278 | 185146873 | MAP3K13 | Genotyping |
| 195 | chr3 | 185197923 | 185198317 | MAP3K13 | Genotyping |
| 196 | chr3 | 185236908 | 185237109 | LIPH | Genotyping |
| 197 | chr3 | 185446223 | 185446389 | C3orf65 | Genotyping |
| 198 | chr3 | 185538773 | 185538951 | IGF2BP2 | Genotyping |
| 199 | chr3 | 185697423 | 185697669 | TRA2B | Genotyping |
| 200 | chr3 | 186714604 | 186715001 | ST6GAL1 | Phased Variants |
| 201 | chr3 | 186782529 | 186782790 | ST6GAL1 | Phased Variants |
| 202 | chr3 | 186783389 | 186784291 | ST6GAL1 | Phased Variants |
| 203 | chr3 | 187440189 | 187440445 | BCL6 | Genotyping |
| 204 | chr3 | 187442669 | 187442920 | BCL6 | Genotyping |
| 205 | chr3 | 187443239 | 187443438 | BCL6 | Genotyping |
| 206 | chr3 | 187446814 | 187447831 | BCL6 | Genotyping |
| 207 | chr3 | 187449434 | 187449655 | BCL6 | Genotyping |
| 208 | chr3 | 187451284 | 187451667 | BCL6 | Genotyping |
| 209 | chr3 | 187460134 | 187460530 | BCL6 | Phased Variants |
| 210 | chr3 | 187460824 | 187461302 | BCL6 | Phased Variants |
| 211 | chr3 | 187461319 | 187461381 | BCL6 | Phased Variants |
| 212 | chr3 | 187461454 | 187461918 | BCL6 | Phased Variants |
| 213 | chr3 | 187461924 | 187462343 | BCL6 | Phased Variants |
| 214 | chr3 | 187462374 | 187462887 | BCL6 | Phased Variants |

| 215 | chr3 | 187462924 | 187462999 | BCL6 | Phased Variants |
|-----|------|-----------|-----------|------|-----------------|
| 216 | chr3 | 187463004 | 187463525 | BCL6 | Phased Variants |
| 217 | chr3 | 187463709 | 187463781 | BCL6 | Phased Variants |
| 218 | chr3 | 187463794 | 187464109 | BCL6 | Phased Variants |
| 219 | chr3 | 187619334 | 187619708 | BCL6 | Phased Variants |
| 220 | chr3 | 187660817 | 187661390 | BCL6 | Phased Variants |
| 221 | chr3 | 187957432 | 187957507 | AC022498.1 | Phased Variants |
| 222 | chr3 | 187957512 | 187957754 | AC022498.1 | Phased Variants |
| 223 | chr3 | 187957767 | 187958110 | AC022498.1 | Phased Variants |
| 224 | chr3 | 187958282 | 187958675 | AC022498.1 | Phased Variants |
| 225 | chr3 | 187958787 | 187959184 | AC022498.1 | Phased Variants |
| 226 | chr3 | 187959462 | 187959686 | AC022498.1 | Phased Variants |
| 227 | chr3 | 188299217 | 188299605 | LPP | Phased Variants |
| 228 | chr3 | 188471412 | 188471549 | LPP | Phased Variants |
| 229 | chr3 | 188471567 | 188471937 | LPP | Phased Variants |
| 230 | chr4 | 7728456 | 7728661 | SORCS2 | Genotyping |
| 231 | chr4 | 40198810 | 40199653 | N4BP2 | Phased Variants |
| 232 | chr4 | 40199660 | 40199873 | N4BP2 | Phased Variants |
| 233 | chr4 | 40199990 | 40200211 | N4BP2 | Phased Variants |
| 234 | chr4 | 40200505 | 40200727 | RHOH | Phased Variants |
| 235 | chr4 | 40200730 | 40201571 | RHOH | Phased Variants |
| 236 | chr4 | 80327792 | 80328151 | GK2 | Genotyping |
| 237 | chr4 | 88011077 | 88011285 | AFF1 | Genotyping |
| 238 | chr4 | 106157604 | 106157813 | TET2 | Genotyping |
| 239 | chr4 | 134727698 | 134727916 | PABPC4L | Phased Variants |
| 240 | chr4 | 153249285 | 153249507 | FBXW7 | Genotyping |
| 241 | chr4 | 154624670 | 154625050 | TLR2 | Genotyping |
| 242 | chr4 | 187509884 | 187510410 | FAT1 | Genotyping |
| 243 | chr4 | 187557779 | 187557985 | FAT1 | Genotyping |
| 244 | chr4 | 188924114 | 188924897 | ZFP42 | Genotyping |
| 245 | chr5 | 5182145 | 5182494 | ADAMTS16 | Genotyping |
| 246 | chr5 | 11110990 | 11111137 | CTNND2 | Genotyping |
| 247 | chr5 | 11236740 | 11236956 | CTNND2 | Genotyping |
| 248 | chr5 | 11364700 | 11364923 | CTNND2 | Genotyping |
| 249 | chr5 | 11397080 | 11397377 | CTNND2 | Genotyping |
| 250 | chr5 | 11411600 | 11411807 | CTNND2 | Genotyping |
| 251 | chr5 | 13864465 | 13864696 | DNAH5 | Genotyping |
| 252 | chr5 | 21783415 | 21783668 | CDH12 | Genotyping |
| 253 | chr5 | 54964698 | 54964921 | SLC38A9 | Phased Variants |
| 254 | chr5 | 67590966 | 67591183 | PIK3R1 | Genotyping |
| 255 | chr5 | 75913716 | 75914448 | F2RL2 | Genotyping |
| 256 | chr5 | 83258967 | 83259183 | EDIL3 | Genotyping |
| 257 | chr5 | 112176756 | 112176958 | APC | Genotyping |

| 258 | chr5 | 124079827 | 124080721 | ZNF608 | Phased Variants |
|-----|------|-----------|-----------|--------|-----------------|
| 259 | chr5 | 131825017 | 131825239 | IRF1 | Genotyping |
| 260 | chr5 | 135381969 | 135382218 | TGFBI | Genotyping |
| 261 | chr5 | 137801487 | 137801637 | EGR1 | Genotyping |
| 262 | chr5 | 137801697 | 137801804 | EGR1 | Genotyping |
| 263 | chr5 | 140208033 | 140208874 | PCDHA6 | Genotyping |
| 264 | chr5 | 158527642 | 158528019 | EBF1 | Phased Variants |
| 265 | chr5 | 176522449 | 176522613 | FGFR4 | Genotyping |
| 266 | chr6 | 392760 | 392967 | IRF4 | Phased Variants |
| 267 | chr6 | 393090 | 393309 | IRF4 | Phased Variants |
| 268 | chr6 | 394815 | 395025 | IRF4 | Genotyping |
| 269 | chr6 | 14117992 | 14118654 | CD83 | Phased Variants |
| 270 | chr6 | 14131732 | 14132021 | CD83 | Genotyping |
| 271 | chr6 | 14133857 | 14133996 | CD83 | Genotyping |
| 272 | chr6 | 14135317 | 14135496 | CD83 | Genotyping |
| 273 | chr6 | 26020709 | 26020958 | HIST1H3A | Genotyping |
| 274 | chr6 | 26032014 | 26032217 | HIST1H3B | Genotyping |
| 275 | chr6 | 26045744 | 26046077 | HIST1H3C | Genotyping |
| 276 | chr6 | 26056034 | 26056315 | HIST1H1C | Genotyping |
| 277 | chr6 | 26056319 | 26056558 | HIST1H1C | Genotyping |
| 278 | chr6 | 26123614 | 26123778 | HIST1H2BC | Phased Variants |
| 279 | chr6 | 26123879 | 26124098 | HIST1H2BC | Genotyping |
| 280 | chr6 | 26124544 | 26124640 | HIST1H2AC | Genotyping |
| 281 | chr6 | 26124714 | 26124889 | HIST1H2AC | Genotyping |
| 282 | chr6 | 26156649 | 26157377 | HIST1H1E | Phased Variants |
| 283 | chr6 | 26158529 | 26158608 | HIST1H2BD | Genotyping |
| 284 | chr6 | 26158739 | 26158835 | HIST1H2BD | Genotyping |
| 285 | chr6 | 26197104 | 26197182 | HIST1H3D | Genotyping |
| 286 | chr6 | 26197189 | 26197465 | HIST1H3D | Genotyping |
| 287 | chr6 | 26216779 | 26216920 | HIST1H2BG | Genotyping |
| 288 | chr6 | 26217214 | 26217431 | HIST1H2AE | Genotyping |
| 289 | chr6 | 26234654 | 26234976 | HIST1H1D | Genotyping |
| 290 | chr6 | 26250459 | 26250537 | HIST1H3F | Genotyping |
| 291 | chr6 | 26250594 | 26250703 | HIST1H3F | Genotyping |
| 292 | chr6 | 26252154 | 26252232 | HIST1H2BH | Genotyping |
| 293 | chr6 | 27100079 | 27100185 | HIST1H2BJ | Genotyping |
| 294 | chr6 | 27100939 | 27101039 | HIST1H2AG | Genotyping |
| 295 | chr6 | 27101159 | 27101300 | HIST1H2AG | Genotyping |
| 296 | chr6 | 27114004 | 27114216 | HIST1H2BK | Phased Variants |
| 297 | chr6 | 27114319 | 27114396 | HIST1H2BK | Genotyping |
| 298 | chr6 | 27114494 | 27114592 | HIST1H2BK | Genotyping |
| 299 | chr6 | 27277284 | 27277495 | POM121L2 | Genotyping |
| 300 | chr6 | 27777783 | 27777900 | HIST1H3H | Genotyping |

| 301 | chr6 | 27777928 | 27778106 | HIST1H3H | Genotyping |
| 302 | chr6 | 27782718 | 27782926 | HIST1H2BM | Genotyping |
| 303 | chr6 | 27799168 | 27799381 | HIST1H4K | Genotyping |
| 304 | chr6 | 27833408 | 27833516 | HIST1H2AL | Genotyping |
| 305 | chr6 | 27834968 | 27835075 | HIST1H1B | Genotyping |
| 306 | chr6 | 27839658 | 27839805 | HIST1H3I | Genotyping |
| 307 | chr6 | 27860479 | 27860659 | HIST1H2AM | Genotyping |
| 308 | chr6 | 27860794 | 27860938 | HIST1H2AM | Genotyping |
| 309 | chr6 | 27861244 | 27861344 | HIST1H2BO | Genotyping |
| 310 | chr6 | 27861399 | 27861485 | HIST1H2BO | Genotyping |
| 311 | chr6 | 37138284 | 37139559 | PIM1 | Phased Variants |
| 312 | chr6 | 37140749 | 37140956 | PIM1 | Genotyping |
| 313 | chr6 | 37141679 | 37141903 | PIM1 | Genotyping |
| 314 | chr6 | 41903611 | 41903834 | CCND3 | Genotyping |
| 315 | chr6 | 41904271 | 41904477 | CCND3 | Genotyping |
| 316 | chr6 | 41904941 | 41905155 | CCND3 | Genotyping |
| 317 | chr6 | 41908071 | 41908365 | CCND3 | Genotyping |
| 318 | chr6 | 41909196 | 41909441 | CCND3 | Genotyping |
| 319 | chr6 | 75965846 | 75966046 | TMEM30A | Genotyping |
| 320 | chr6 | 75969006 | 75969288 | TMEM30A | Genotyping |
| 321 | chr6 | 91004618 | 91004828 | MAP3K7 | Phased Variants |
| 322 | chr6 | 91005793 | 91005998 | MAP3K7 | Phased Variants |
| 323 | chr6 | 94120219 | 94120743 | EPHA7 | Genotyping |
| 324 | chr6 | 106534266 | 106534477 | PRDM1 | Genotyping |
| 325 | chr6 | 106536046 | 106536340 | PRDM1 | Genotyping |
| 326 | chr6 | 106543466 | 106543637 | PRDM1 | Genotyping |
| 327 | chr6 | 106547146 | 106547437 | PRDM1 | Genotyping |
| 328 | chr6 | 106552761 | 106552932 | PRDM1 | Genotyping |
| 329 | chr6 | 106552961 | 106553841 | PRDM1 | Genotyping |
| 330 | chr6 | 106554221 | 106554400 | PRDM1 | Genotyping |
| 331 | chr6 | 106554766 | 106555383 | PRDM1 | Genotyping |
| 332 | chr6 | 108040228 | 108040856 | SCML4 | Genotyping |
| 333 | chr6 | 108041553 | 108042219 | SCML4 | Genotyping |
| 334 | chr6 | 110777718 | 110778244 | SLC22A16 | Genotyping |
| 335 | chr6 | 134491382 | 134491589 | SGK1 | Genotyping |
| 336 | chr6 | 134491892 | 134492111 | SGK1 | Genotyping |
| 337 | chr6 | 134492132 | 134492333 | SGK1 | Genotyping |
| 338 | chr6 | 134492717 | 134492923 | SGK1 | Genotyping |
| 339 | chr6 | 134493307 | 134493474 | SGK1 | Genotyping |
| 340 | chr6 | 134493732 | 134494308 | SGK1 | Phased Variants |
| 341 | chr6 | 134494342 | 134494514 | SGK1 | Genotyping |
| 342 | chr6 | 134494552 | 134494718 | SGK1 | Phased Variants |
| 343 | chr6 | 134494722 | 134494795 | SGK1 | Phased Variants |

| 344 | chr6 | 134494967 | 134495974 | SGK1 | Phased Variants |
|---|---|---|---|---|---|
| 345 | chr6 | 138188483 | 138188650 | TNFAIP3 | Genotyping |
| 346 | chr6 | 138192338 | 138192683 | TNFAIP3 | Genotyping |
| 347 | chr6 | 138195963 | 138196172 | TNFAIP3 | Genotyping |
| 348 | chr6 | 138196803 | 138197021 | TNFAIP3 | Genotyping |
| 349 | chr6 | 138197108 | 138197313 | TNFAIP3 | Genotyping |
| 350 | chr6 | 138198193 | 138198407 | TNFAIP3 | Genotyping |
| 351 | chr6 | 138199548 | 138200525 | TNFAIP3 | Genotyping |
| 352 | chr6 | 138201178 | 138201404 | TNFAIP3 | Genotyping |
| 353 | chr6 | 138202138 | 138202494 | TNFAIP3 | Genotyping |
| 354 | chr6 | 150954420 | 150954823 | PLEKHG1 | Phased Variants |
| 355 | chr6 | 159238415 | 159238794 | EZR | Phased Variants |
| 356 | chr7 | 2963818 | 2963952 | CARD11 | Genotyping |
| 357 | chr7 | 2963953 | 2964056 | CARD11 | Genotyping |
| 358 | chr7 | 2969593 | 2969738 | CARD11 | Genotyping |
| 359 | chr7 | 2976668 | 2976876 | CARD11 | Genotyping |
| 360 | chr7 | 2977493 | 2977712 | CARD11 | Genotyping |
| 361 | chr7 | 2978258 | 2978502 | CARD11 | Genotyping |
| 362 | chr7 | 2979398 | 2979601 | CARD11 | Genotyping |
| 363 | chr7 | 2983918 | 2984199 | CARD11 | Genotyping |
| 364 | chr7 | 2985403 | 2985610 | CARD11 | Genotyping |
| 365 | chr7 | 2987163 | 2987382 | CARD11 | Genotyping |
| 366 | chr7 | 5569095 | 5569200 | ACTB | Genotyping |
| 367 | chr7 | 5569210 | 5569359 | ACTB | Genotyping |
| 368 | chr7 | 80285799 | 80286074 | CD36 | Genotyping |
| 369 | chr7 | 82387830 | 82388061 | PCLO | Genotyping |
| 370 | chr7 | 82453520 | 82453733 | PCLO | Genotyping |
| 371 | chr7 | 82763800 | 82764050 | PCLO | Genotyping |
| 372 | chr7 | 82784490 | 82784643 | PCLO | Genotyping |
| 373 | chr7 | 106508490 | 106509161 | PIK3CG | Genotyping |
| 374 | chr7 | 110545276 | 110545445 | IMMP2L | Phased Variants |
| 375 | chr7 | 110697971 | 110698144 | LRRN3 | Phased Variants |
| 376 | chr7 | 110737411 | 110737634 | LRRN3 | Phased Variants |
| 377 | chr7 | 110746681 | 110746893 | LRRN3 | Phased Variants |
| 378 | chr7 | 110762936 | 110764629 | LRRN3 | Genotyping |
| 379 | chr7 | 110764636 | 110764981 | LRRN3 | Genotyping |
| 380 | chr7 | 119915406 | 119915800 | KCND2 | Genotyping |
| 381 | chr7 | 122634905 | 122635140 | TAS2R16 | Genotyping |
| 382 | chr7 | 140453012 | 140453121 | BRAF | Genotyping |
| 383 | chr7 | 140453162 | 140453268 | BRAF | Genotyping |
| 384 | chr7 | 146997183 | 146997422 | CNTNAP2 | Genotyping |
| 385 | chr7 | 148506318 | 148506416 | EZH2 | Genotyping |
| 386 | chr7 | 148506448 | 148506551 | EZH2 | Genotyping |

| 387 | chr7 | 148508658 | 148508867 | EZH2 | Genotyping |
|---|---|---|---|---|---|
| 388 | chr7 | 148513738 | 148513900 | EZH2 | Genotyping |
| 389 | chr7 | 148523533 | 148523743 | EZH2 | Genotyping |
| 390 | chr7 | 151943421 | 151943500 | KMT2C | Phased Variants |
| 391 | chr8 | 623880 | 624090 | ERICH1 | Genotyping |
| 392 | chr8 | 3141724 | 3141942 | CSMD1 | Genotyping |
| 393 | chr8 | 4494931 | 4495105 | CSMD1 | Genotyping |
| 394 | chr8 | 8748687 | 8749284 | MFHAS1 | Genotyping |
| 395 | chr8 | 8750067 | 8750281 | MFHAS1 | Genotyping |
| 396 | chr8 | 18729445 | 18729937 | PSD3 | Genotyping |
| 397 | chr8 | 75898190 | 75898400 | CRISPLD1 | Genotyping |
| 398 | chr8 | 101730376 | 101730457 | PABPC1 | Genotyping |
| 399 | chr8 | 103663491 | 103664160 | KLF10 | Genotyping |
| 400 | chr8 | 104897561 | 104898479 | RIMS2 | Genotyping |
| 401 | chr8 | 113308014 | 113308283 | CSMD3 | Genotyping |
| 402 | chr8 | 113364624 | 113364791 | CSMD3 | Genotyping |
| 403 | chr8 | 113568994 | 113569205 | CSMD3 | Genotyping |
| 404 | chr8 | 116616145 | 116616886 | TRPS1 | Genotyping |
| 405 | chr8 | 122626847 | 122627163 | HAS2 | Genotyping |
| 406 | chr8 | 128492947 | 128493338 | POU5F1B | Genotyping |
| 407 | chr8 | 128746807 | 128748893 | MYC | Genotyping |
| 408 | chr8 | 128748902 | 128749969 | MYC | Genotyping |
| 409 | chr8 | 128750367 | 128751183 | MYC | Phased Variants |
| 410 | chr8 | 128752612 | 128753235 | MYC | Genotyping |
| 411 | chr8 | 128754007 | 128754731 | MYC | Genotyping |
| 412 | chr8 | 128754752 | 128756424 | MYC | Genotyping |
| 413 | chr8 | 128756707 | 128756931 | MYC | Genotyping |
| 414 | chr8 | 128756947 | 128757361 | MYC | Genotyping |
| 415 | chr8 | 128757737 | 128757921 | MYC | Genotyping |
| 416 | chr8 | 128764072 | 128764292 | MYC | Genotyping |
| 417 | chr8 | 128951724 | 128951896 | TMEM75 | Genotyping |
| 418 | chr8 | 130692149 | 130692503 | GSDMC | Genotyping |
| 419 | chr8 | 130760594 | 130761023 | GSDMC | Genotyping |
| 420 | chr8 | 131373024 | 131373443 | ASAP1 | Genotyping |
| 421 | chr8 | 136569669 | 136569842 | KHDRBS3 | Genotyping |
| 422 | chr8 | 136659204 | 136659414 | KHDRBS3 | Genotyping |
| 423 | chr8 | 137101252 | 137101464 | KHDRBS3 | Genotyping |
| 424 | chr8 | 137528187 | 137528570 | KHDRBS3 | Genotyping |
| 425 | chr8 | 138849937 | 138850149 | FAM135B | Genotyping |
| 426 | chr8 | 139600457 | 139601255 | COL22A1 | Genotyping |
| 427 | chr8 | 139601392 | 139601569 | COL22A1 | Genotyping |
| 428 | chr9 | 5450474 | 5450616 | CD274 | Genotyping |
| 429 | chr9 | 5456059 | 5456200 | CD274 | Genotyping |

| 430 | chr9 | 5457054 | 5457446 | CD274 | Genotyping |
|---|---|---|---|---|---|
| 431 | chr9 | 5462809 | 5463160 | CD274 | Genotyping |
| 432 | chr9 | 5465489 | 5465622 | CD274 | Genotyping |
| 433 | chr9 | 5466724 | 5466867 | CD274 | Genotyping |
| 434 | chr9 | 5467814 | 5468022 | CD274 | Genotyping |
| 435 | chr9 | 5510589 | 5510804 | PDCD1LG2 | Genotyping |
| 436 | chr9 | 5522484 | 5522636 | PDCD1LG2 | Genotyping |
| 437 | chr9 | 5534764 | 5535047 | PDCD1LG2 | Genotyping |
| 438 | chr9 | 5549309 | 5549627 | PDCD1LG2 | Genotyping |
| 439 | chr9 | 5557589 | 5557762 | PDCD1LG2 | Genotyping |
| 440 | chr9 | 5563119 | 5563251 | PDCD1LG2 | Genotyping |
| 441 | chr9 | 5569929 | 5570140 | PDCD1LG2 | Genotyping |
| 442 | chr9 | 13222185 | 13222409 | MPDZ | Genotyping |
| 443 | chr9 | 16435498 | 16436307 | BNC2 | Genotyping |
| 444 | chr9 | 19957356 | 19958178 | SLC24A2 | Genotyping |
| 445 | chr9 | 20820916 | 20821095 | FOCAD | Genotyping |
| 446 | chr9 | 20946676 | 20946849 | FOCAD | Genotyping |
| 447 | chr9 | 21808814 | 21808891 | MTAP | Genotyping |
| 448 | chr9 | 21808894 | 21808973 | MTAP | Genotyping |
| 449 | chr9 | 21859249 | 21859469 | MTAP | Genotyping |
| 450 | chr9 | 21970834 | 21971023 | CDKN2A | Genotyping |
| 451 | chr9 | 21971069 | 21971170 | CDKN2A | Genotyping |
| 452 | chr9 | 21974409 | 21974881 | CDKN2A | Genotyping |
| 453 | chr9 | 21989304 | 21989976 | CDKN2A | Genotyping |
| 454 | chr9 | 21994084 | 21994405 | CDKN2A | Genotyping |
| 455 | chr9 | 22005929 | 22006067 | CDKN2B | Genotyping |
| 456 | chr9 | 22006109 | 22006187 | CDKN2B | Genotyping |
| 457 | chr9 | 22008649 | 22009012 | CDKN2B | Genotyping |
| 458 | chr9 | 24545399 | 24545922 | IZUMO3 | Genotyping |
| 459 | chr9 | 24905444 | 24905729 | IZUMO3 | Genotyping |
| 460 | chr9 | 27950144 | 27950532 | LINGO2 | Genotyping |
| 461 | chr9 | 37024919 | 37025642 | PAX5 | Phased Variants |
| 462 | chr9 | 37025829 | 37025996 | PAX5 | Phased Variants |
| 463 | chr9 | 37026269 | 37027015 | PAX5 | Phased Variants |
| 464 | chr9 | 37033619 | 37033797 | PAX5 | Phased Variants |
| 465 | chr9 | 37293169 | 37293378 | ZCCHC7 | Phased Variants |
| 466 | chr9 | 37371494 | 37371879 | ZCCHC7 | Phased Variants |
| 467 | chr9 | 37384684 | 37384911 | ZCCHC7 | Phased Variants |
| 468 | chr9 | 37407369 | 37407588 | GRHPR | Phased Variants |
| 469 | chr9 | 78686579 | 78686854 | PCSK5 | Genotyping |
| 470 | chr9 | 139390582 | 139390950 | NOTCH1 | Genotyping |
| 471 | chr9 | 139390952 | 139391172 | NOTCH1 | Genotyping |
| 472 | chr9 | 139402662 | 139402868 | NOTCH1 | Genotyping |

| 473 | chr10 | 5755066 | 5755273 | FAM208B | Phased Variants |
|---|---|---|---|---|---|
| 474 | chr10 | 89500957 | 89501139 | PAPSS2 | Genotyping |
| 475 | chr10 | 89603602 | 89604077 | KLLN | Genotyping |
| 476 | chr10 | 89624272 | 89624350 | PTEN | Genotyping |
| 477 | chr10 | 89653752 | 89653825 | PTEN | Genotyping |
| 478 | chr10 | 89653832 | 89653909 | PTEN | Genotyping |
| 479 | chr10 | 89685272 | 89685379 | PTEN | Genotyping |
| 480 | chr10 | 89690752 | 89690894 | PTEN | Genotyping |
| 481 | chr10 | 89692737 | 89692810 | PTEN | Genotyping |
| 482 | chr10 | 89692877 | 89692951 | PTEN | Genotyping |
| 483 | chr10 | 89692972 | 89693037 | PTEN | Genotyping |
| 484 | chr10 | 89711837 | 89711966 | PTEN | Genotyping |
| 485 | chr10 | 89711982 | 89712058 | PTEN | Genotyping |
| 486 | chr10 | 89717577 | 89717714 | PTEN | Genotyping |
| 487 | chr10 | 89717742 | 89717811 | PTEN | Genotyping |
| 488 | chr10 | 89720637 | 89720904 | PTEN | Genotyping |
| 489 | chr10 | 90074239 | 90074419 | RNLS | Genotyping |
| 490 | chr10 | 90537736 | 90538027 | LIPN | Genotyping |
| 491 | chr10 | 90579966 | 90580319 | LIPM | Genotyping |
| 492 | chr10 | 90699126 | 90699647 | ACTA2 | Genotyping |
| 493 | chr10 | 90773866 | 90774076 | FAS | Genotyping |
| 494 | chr10 | 91092211 | 91092423 | IFIT3 | Genotyping |
| 495 | chr10 | 91358986 | 91359298 | PANK1 | Genotyping |
| 496 | chr10 | 131640289 | 131640505 | EBF3 | Genotyping |
| 497 | chr11 | 58978692 | 58978791 | MPEG1 | Genotyping |
| 498 | chr11 | 58978927 | 58979095 | MPEG1 | Genotyping |
| 499 | chr11 | 58979112 | 58979365 | MPEG1 | Genotyping |
| 500 | chr11 | 65190342 | 65190557 | FRMD8 | Phased Variants |
| 501 | chr11 | 65266552 | 65266924 | SCYL1 | Phased Variants |
| 502 | chr11 | 65267397 | 65267603 | SCYL1 | Phased Variants |
| 503 | chr11 | 65623422 | 65623506 | CFL1 | Genotyping |
| 504 | chr11 | 69346691 | 69346940 | CCND1 | Genotyping |
| 505 | chr11 | 102188381 | 102188945 | BIRC3 | Phased Variants |
| 506 | chr11 | 111234536 | 111235068 | POU2AF1 | Genotyping |
| 507 | chr11 | 111249311 | 111249530 | POU2AF1 | Phased Variants |
| 508 | chr11 | 111613196 | 111613432 | PPP2R1B | Genotyping |
| 509 | chr11 | 111781036 | 111781641 | CRYAB | Genotyping |
| 510 | chr11 | 111904096 | 111904291 | DLAT | Genotyping |
| 511 | chr11 | 112405016 | 112405330 | AP002884.2 | Genotyping |
| 512 | chr11 | 112405341 | 112405621 | AP002884.2 | Genotyping |
| 513 | chr11 | 117101043 | 117101217 | PCSK7 | Genotyping |
| 514 | chr11 | 117712683 | 117712997 | FXYD6 | Genotyping |
| 515 | chr11 | 118754793 | 118755011 | CXCR5 | Phased Variants |

| 516 | chr11 | 118764838 | 118765408 | CXCR5 | Genotyping |
|---|---|---|---|---|---|
| 517 | chr11 | 118967323 | 118968029 | DPAGT1 | Genotyping |
| 518 | chr11 | 120127163 | 120127588 | POU2F3 | Genotyping |
| 519 | chr11 | 120189028 | 120189629 | POU2F3 | Genotyping |
| 520 | chr11 | 125472640 | 125472915 | STT3A | Genotyping |
| 521 | chr11 | 128391383 | 128391629 | ETS1 | Phased Variants |
| 522 | chr11 | 128391648 | 128392132 | ETS1 | Phased Variants |
| 523 | chr11 | 129739778 | 129740102 | NFRKB | Genotyping |
| 524 | chr11 | 131747549 | 131748030 | NTM | Genotyping |
| 525 | chr11 | 134027789 | 134027980 | NCAPD3 | Genotyping |
| 526 | chr11 | 134118684 | 134118873 | THYN1 | Genotyping |
| 527 | chr11 | 134129469 | 134130211 | ACAD8 | Genotyping |
| 528 | chr11 | 134130464 | 134131097 | ACAD8 | Genotyping |
| 529 | chr11 | 134133389 | 134133972 | ACAD8 | Genotyping |
| 530 | chr12 | 6439713 | 6439920 | TNFRSF1A | Genotyping |
| 531 | chr12 | 15813487 | 15813687 | EPS8 | Genotyping |
| 532 | chr12 | 18534682 | 18534856 | PIK3C2G | Genotyping |
| 533 | chr12 | 18544037 | 18544241 | PIK3C2G | Genotyping |
| 534 | chr12 | 18573807 | 18574017 | PIK3C2G | Genotyping |
| 535 | chr12 | 18699197 | 18699459 | PIK3C2G | Genotyping |
| 536 | chr12 | 18747397 | 18747562 | PIK3C2G | Genotyping |
| 537 | chr12 | 18800762 | 18801046 | PIK3C2G | Genotyping |
| 538 | chr12 | 18891267 | 18891560 | CAPZA3 | Genotyping |
| 539 | chr12 | 25205888 | 25206105 | LRMP | Phased Variants |
| 540 | chr12 | 25206398 | 25206616 | LRMP | Phased Variants |
| 541 | chr12 | 25206748 | 25206877 | LRMP | Phased Variants |
| 542 | chr12 | 25207088 | 25207474 | LRMP | Phased Variants |
| 543 | chr12 | 25398218 | 25398299 | KRAS | Genotyping |
| 544 | chr12 | 48190731 | 48190983 | HDAC7 | Genotyping |
| 545 | chr12 | 49415991 | 49416144 | KMT2D | Genotyping |
| 546 | chr12 | 49418306 | 49418550 | KMT2D | Genotyping |
| 547 | chr12 | 49420531 | 49420750 | KMT2D | Genotyping |
| 548 | chr12 | 49426451 | 49426592 | KMT2D | Genotyping |
| 549 | chr12 | 49427886 | 49428116 | KMT2D | Genotyping |
| 550 | chr12 | 49433331 | 49433507 | KMT2D | Genotyping |
| 551 | chr12 | 49437926 | 49438391 | KMT2D | Genotyping |
| 552 | chr12 | 49444391 | 49444595 | KMT2D | Genotyping |
| 553 | chr12 | 49447196 | 49447491 | KMT2D | Genotyping |
| 554 | chr12 | 57496552 | 57496735 | STAT6 | Genotyping |
| 555 | chr12 | 57498222 | 57498396 | STAT6 | Genotyping |
| 556 | chr12 | 57498912 | 57499150 | STAT6 | Genotyping |
| 557 | chr12 | 86198698 | 86199622 | RASSF9 | Genotyping |
| 558 | chr12 | 92537875 | 92538647 | BTG1 | Phased Variants |

| 559 | chr12 | 92538790 | 92539374 | BTG1 | Phased Variants |
|---|---|---|---|---|---|
| 560 | chr12 | 113495364 | 113496458 | DTX1 | Phased Variants |
| 561 | chr12 | 113496509 | 113496679 | DTX1 | Phased Variants |
| 562 | chr12 | 113496694 | 113496945 | DTX1 | Phased Variants |
| 563 | chr12 | 113497059 | 113497278 | DTX1 | Phased Variants |
| 564 | chr12 | 113515199 | 113515658 | DTX1 | Genotyping |
| 565 | chr12 | 113515664 | 113515934 | DTX1 | Genotyping |
| 566 | chr12 | 113530924 | 113531055 | DTX1 | Genotyping |
| 567 | chr12 | 113531319 | 113531531 | DTX1 | Genotyping |
| 568 | chr12 | 113531799 | 113531930 | DTX1 | Genotyping |
| 569 | chr12 | 113532569 | 113532781 | DTX1 | Genotyping |
| 570 | chr12 | 113532809 | 113533032 | DTX1 | Genotyping |
| 571 | chr12 | 113533099 | 113533237 | DTX1 | Genotyping |
| 572 | chr12 | 113534494 | 113534778 | DTX1 | Genotyping |
| 573 | chr12 | 122458781 | 122459524 | BCL7A | Phased Variants |
| 574 | chr12 | 122460811 | 122461193 | BCL7A | Phased Variants |
| 575 | chr12 | 122461316 | 122461882 | BCL7A | Phased Variants |
| 576 | chr12 | 122462001 | 122462210 | BCL7A | Phased Variants |
| 577 | chr12 | 122462716 | 122462935 | BCL7A | Phased Variants |
| 578 | chr12 | 122463031 | 122463137 | BCL7A | Phased Variants |
| 579 | chr13 | 32907206 | 32907376 | BRCA2 | Genotyping |
| 580 | chr13 | 32912226 | 32912828 | BRCA2 | Genotyping |
| 581 | chr13 | 41133662 | 41133842 | FOXO1 | Genotyping |
| 582 | chr13 | 41133922 | 41135026 | FOXO1 | Genotyping |
| 583 | chr13 | 41239682 | 41239755 | FOXO1 | Genotyping |
| 584 | chr13 | 41239827 | 41240356 | FOXO1 | Genotyping |
| 585 | chr13 | 41240362 | 41240788 | FOXO1 | Genotyping |
| 586 | chr13 | 46959165 | 46959379 | KIAA0226L | Phased Variants |
| 587 | chr13 | 46961680 | 46962067 | KIAA0226L | Phased Variants |
| 588 | chr13 | 51915233 | 51915552 | SERPINE3 | Genotyping |
| 589 | chr13 | 58207131 | 58209129 | PCDH17 | Genotyping |
| 590 | chr13 | 84453542 | 84455255 | SLITRK1 | Genotyping |
| 591 | chr13 | 113516229 | 113516436 | ATP11A | Phased Variants |
| 592 | chr14 | 23344697 | 23345206 | LRP10 | Genotyping |
| 593 | chr14 | 32615405 | 32615617 | ARHGAP5 | Genotyping |
| 594 | chr14 | 35873671 | 35873838 | NFKBIA | Genotyping |
| 595 | chr14 | 64330252 | 64330462 | SYNE2 | Phased Variants |
| 596 | chr14 | 69258238 | 69259642 | ZFP36L1 | Phased Variants |
| 597 | chr14 | 84420586 | 84420796 | FLRT2 | Phased Variants |
| 598 | chr14 | 96179592 | 96180295 | TCL1A | Phased Variants |
| 599 | chr14 | 106048955 | 106049032 | IGHA2 | Phased Variants |
| 600 | chr14 | 106054695 | 106055541 | IGHA2 | Genotyping |
| 601 | chr14 | 106055740 | 106055827 | IGHA2 | Genotyping |

| 602 | chr14 | 106055910 | 106055995 | IGHA2 | Genotyping |
|---|---|---|---|---|---|
| 603 | chr14 | 106056035 | 106056121 | IGHA2 | Genotyping |
| 604 | chr14 | 106068705 | 106068911 | IGHE | Phased Variants |
| 605 | chr14 | 106069045 | 106069384 | IGHE | Phased Variants |
| 606 | chr14 | 106071060 | 106071135 | IGHE | Phased Variants |
| 607 | chr14 | 106071190 | 106071271 | IGHE | Phased Variants |
| 608 | chr14 | 106092380 | 106092608 | IGHG4 | Genotyping |
| 609 | chr14 | 106092670 | 106093406 | IGHG4 | Genotyping |
| 610 | chr14 | 106093435 | 106093575 | IGHG4 | Genotyping |
| 611 | chr14 | 106093610 | 106094215 | IGHG4 | Genotyping |
| 612 | chr14 | 106094235 | 106094479 | IGHG4 | Genotyping |
| 613 | chr14 | 106094580 | 106094654 | IGHG4 | Genotyping |
| 614 | chr14 | 106094675 | 106094915 | IGHG4 | Genotyping |
| 615 | chr14 | 106095335 | 106095417 | IGHG4 | Phased Variants |
| 616 | chr14 | 106095480 | 106095560 | IGHG4 | Phased Variants |
| 617 | chr14 | 106110675 | 106110814 | IGHG2 | Phased Variants |
| 618 | chr14 | 106110830 | 106110904 | IGHG2 | Phased Variants |
| 619 | chr14 | 106110950 | 106111025 | IGHG2 | Phased Variants |
| 620 | chr14 | 106111100 | 106111311 | IGHG2 | Genotyping |
| 621 | chr14 | 106111390 | 106112121 | IGHG2 | Genotyping |
| 622 | chr14 | 106112160 | 106112302 | IGHG2 | Genotyping |
| 623 | chr14 | 106112335 | 106113010 | IGHG2 | Phased Variants |
| 624 | chr14 | 106113020 | 106113438 | IGHG2 | Phased Variants |
| 625 | chr14 | 106113450 | 106113625 | IGHG2 | Phased Variants |
| 626 | chr14 | 106113695 | 106113901 | IGHG2 | Phased Variants |
| 627 | chr14 | 106113905 | 106113984 | IGHG2 | Phased Variants |
| 628 | chr14 | 106114175 | 106114414 | IGHG2 | Phased Variants |
| 629 | chr14 | 106174970 | 106175819 | IGHA1 | Genotyping |
| 630 | chr14 | 106175820 | 106176042 | IGHA1 | Genotyping |
| 631 | chr14 | 106176070 | 106176217 | IGHA1 | Genotyping |
| 632 | chr14 | 106176235 | 106176320 | IGHA1 | Genotyping |
| 633 | chr14 | 106176375 | 106176932 | IGHA1 | Phased Variants |
| 634 | chr14 | 106176985 | 106177069 | IGHA1 | Phased Variants |
| 635 | chr14 | 106177425 | 106177536 | IGHA1 | Genotyping |
| 636 | chr14 | 106211960 | 106212864 | IGHG1 | Phased Variants |
| 637 | chr14 | 106212870 | 106212948 | IGHG1 | Phased Variants |
| 638 | chr14 | 106212980 | 106213124 | IGHG1 | Phased Variants |
| 639 | chr14 | 106213125 | 106213200 | IGHG1 | Phased Variants |
| 640 | chr14 | 106213210 | 106213525 | IGHG1 | Phased Variants |
| 641 | chr14 | 106213660 | 106214042 | IGHG1 | Phased Variants |
| 642 | chr14 | 106239250 | 106239357 | IGHG3 | Phased Variants |
| 643 | chr14 | 106239455 | 106239900 | IGHG3 | Phased Variants |
| 644 | chr14 | 106239990 | 106240155 | IGHG3 | Phased Variants |

| 645 | chr14 | 106240170 | 106240815 | IGHG3 | Phased Variants |
|-----|-------|-----------|-----------|-------|-----------------|
| 646 | chr14 | 106240820 | 106240892 | IGHG3 | Phased Variants |
| 647 | chr14 | 106240915 | 106241118 | IGHG3 | Phased Variants |
| 648 | chr14 | 106241200 | 106241278 | IGHG3 | Phased Variants |
| 649 | chr14 | 106241345 | 106241627 | IGHG3 | Phased Variants |
| 650 | chr14 | 106241630 | 106241705 | IGHG3 | Genotyping |
| 651 | chr14 | 106241710 | 106241975 | IGHG3 | Genotyping |
| 652 | chr14 | 106318100 | 106318327 | IGHM | Phased Variants |
| 653 | chr14 | 106322055 | 106322271 | IGHM | Phased Variants |
| 654 | chr14 | 106322905 | 106323129 | IGHM | Phased Variants |
| 655 | chr14 | 106323470 | 106323656 | IGHM | Phased Variants |
| 656 | chr14 | 106323805 | 106323896 | IGHM | Phased Variants |
| 657 | chr14 | 106324010 | 106324087 | IGHM | Phased Variants |
| 658 | chr14 | 106324155 | 106324245 | IGHM | Phased Variants |
| 659 | chr14 | 106324290 | 106324369 | IGHM | Phased Variants |
| 660 | chr14 | 106324490 | 106324577 | IGHM | Phased Variants |
| 661 | chr14 | 106324750 | 106325340 | IGHM | Phased Variants |
| 662 | chr14 | 106325360 | 106325513 | IGHM | Phased Variants |
| 663 | chr14 | 106325515 | 106325791 | IGHM | Phased Variants |
| 664 | chr14 | 106325820 | 106326095 | IGHJ6 | Phased Variants |
| 665 | chr14 | 106326245 | 106326338 | IGHJ6 | Phased Variants |
| 666 | chr14 | 106326450 | 106331808 | IGHD7-27 | Phased Variants |
| 667 | chr14 | 106357890 | 106357967 | IGHD6-19 | Phased Variants |
| 668 | chr14 | 106380360 | 106380541 | IGHD3-3 | Phased Variants |
| 669 | chr14 | 106380550 | 106380901 | IGHD3-3 | Phased Variants |
| 670 | chr14 | 106380910 | 106381109 | IGHD3-3 | Phased Variants |
| 671 | chr14 | 106381275 | 106381351 | IGHD3-3 | Phased Variants |
| 672 | chr14 | 106381485 | 106381633 | IGHD2-2 | Phased Variants |
| 673 | chr14 | 106381655 | 106381724 | IGHD2-2 | Phased Variants |
| 674 | chr14 | 106381890 | 106381968 | IGHD2-2 | Phased Variants |
| 675 | chr14 | 106381990 | 106382161 | IGHD2-2 | Phased Variants |
| 676 | chr14 | 106382325 | 106382403 | IGHD2-2 | Phased Variants |
| 677 | chr14 | 106382905 | 106383014 | IGHD2-2 | Phased Variants |
| 678 | chr14 | 106383030 | 106383140 | IGHD2-2 | Phased Variants |
| 679 | chr14 | 106383980 | 106384142 | IGHD1-1 | Phased Variants |
| 680 | chr14 | 106384630 | 106384702 | IGHD1-1 | Phased Variants |
| 681 | chr14 | 106384720 | 106384798 | IGHD1-1 | Phased Variants |
| 682 | chr14 | 106384825 | 106384957 | IGHD1-1 | Phased Variants |
| 683 | chr14 | 106405615 | 106405963 | IGHV6-1 | Genotyping |
| 684 | chr14 | 106452660 | 106452748 | IGHV1-2 | Genotyping |
| 685 | chr14 | 106452755 | 106452907 | IGHV1-2 | Genotyping |
| 686 | chr14 | 106452940 | 106453023 | IGHV1-2 | Genotyping |
| 687 | chr14 | 106471395 | 106471476 | IGHV1-3 | Genotyping |

| 688 | chr14 | 106471510 | 106471609 | IGHV1-3 | Genotyping |
|-----|-------|-----------|-----------|---------|------------|
| 689 | chr14 | 106494090 | 106494168 | IGHV2-5 | Phased Variants |
| 690 | chr14 | 106494210 | 106494365 | IGHV2-5 | Phased Variants |
| 691 | chr14 | 106494445 | 106494553 | IGHV2-5 | Phased Variants |
| 692 | chr14 | 106494565 | 106494640 | IGHV2-5 | Phased Variants |
| 693 | chr14 | 106494650 | 106494806 | IGHV2-5 | Phased Variants |
| 694 | chr14 | 106518495 | 106518570 | IGHV3-7 | Phased Variants |
| 695 | chr14 | 106518855 | 106518962 | IGHV3-7 | Phased Variants |
| 696 | chr14 | 106518970 | 106519111 | IGHV3-7 | Phased Variants |
| 697 | chr14 | 106539175 | 106539315 | IGHV1-8 | Genotyping |
| 698 | chr14 | 106552365 | 106552502 | IGHV3-9 | Genotyping |
| 699 | chr14 | 106573315 | 106573414 | IGHV3-11 | Genotyping |
| 700 | chr14 | 106573445 | 106573524 | IGHV3-11 | Genotyping |
| 701 | chr14 | 106573540 | 106573645 | IGHV3-11 | Phased Variants |
| 702 | chr14 | 106573685 | 106574021 | IGHV3-11 | Phased Variants |
| 703 | chr14 | 106586200 | 106586343 | IGHV3-13 | Genotyping |
| 704 | chr14 | 106610380 | 106610479 | IGHV3-15 | Genotyping |
| 705 | chr14 | 106610480 | 106610557 | IGHV3-15 | Genotyping |
| 706 | chr14 | 106610690 | 106610765 | IGHV3-15 | Phased Variants |
| 707 | chr14 | 106621885 | 106622026 | IGHV3-16 | Genotyping |
| 708 | chr14 | 106622035 | 106622108 | IGHV3-16 | Genotyping |
| 709 | chr14 | 106641655 | 106641789 | IGHV1-18 | Genotyping |
| 710 | chr14 | 106642110 | 106642265 | IGHV1-18 | Phased Variants |
| 711 | chr14 | 106667545 | 106667628 | IGHV3-20 | Genotyping |
| 712 | chr14 | 106667675 | 106667750 | IGHV3-20 | Genotyping |
| 713 | chr14 | 106667805 | 106667882 | IGHV3-20 | Genotyping |
| 714 | chr14 | 106691755 | 106691904 | IGHV3-21 | Genotyping |
| 715 | chr14 | 106725295 | 106725442 | IGHV3-23 | Phased Variants |
| 716 | chr14 | 106725550 | 106725663 | IGHV3-23 | Phased Variants |
| 717 | chr14 | 106725780 | 106725952 | IGHV3-23 | Phased Variants |
| 718 | chr14 | 106725995 | 106726188 | IGHV3-23 | Phased Variants |
| 719 | chr14 | 106732970 | 106733077 | IGHV1-24 | Phased Variants |
| 720 | chr14 | 106733185 | 106733270 | IGHV1-24 | Phased Variants |
| 721 | chr14 | 106733275 | 106733487 | IGHV1-24 | Phased Variants |
| 722 | chr14 | 106757725 | 106757888 | IGHV2-26 | Genotyping |
| 723 | chr14 | 106758470 | 106758653 | IGHV2-26 | Phased Variants |
| 724 | chr14 | 106780610 | 106780752 | IGHV4-28 | Genotyping |
| 725 | chr14 | 106791090 | 106791169 | IGHV3-30 | Phased Variants |
| 726 | chr14 | 106805290 | 106805428 | IGHV4-31 | Genotyping |
| 727 | chr14 | 106805945 | 106806076 | IGHV4-31 | Phased Variants |
| 728 | chr14 | 106806120 | 106806219 | IGHV4-31 | Phased Variants |
| 729 | chr14 | 106815805 | 106815910 | IGHV3-33 | Phased Variants |
| 730 | chr14 | 106829685 | 106829757 | IGHV4-34 | Phased Variants |

| 731 | chr14 | 106829765 | 106829986 | IGHV4-34 | Phased Variants |
|---|---|---|---|---|---|
| 732 | chr14 | 106830125 | 106830196 | IGHV4-34 | Phased Variants |
| 733 | chr14 | 106830240 | 106830312 | IGHV4-34 | Phased Variants |
| 734 | chr14 | 106830315 | 106830884 | IGHV4-34 | Phased Variants |
| 735 | chr14 | 106831185 | 106831594 | IGHV4-34 | Phased Variants |
| 736 | chr14 | 106845300 | 106845540 | IGHV3-35 | Genotyping |
| 737 | chr14 | 106846385 | 106846557 | IGHV3-35 | Phased Variants |
| 738 | chr14 | 106866380 | 106866461 | IGHV3-38 | Genotyping |
| 739 | chr14 | 106866475 | 106866638 | IGHV3-38 | Genotyping |
| 740 | chr14 | 106877715 | 106877858 | IGHV4-39 | Phased Variants |
| 741 | chr14 | 106877930 | 106878498 | IGHV4-39 | Phased Variants |
| 742 | chr14 | 106878540 | 106878612 | IGHV4-39 | Phased Variants |
| 743 | chr14 | 106878680 | 106878759 | IGHV4-39 | Phased Variants |
| 744 | chr14 | 106926180 | 106926405 | IGHV3-43 | Genotyping |
| 745 | chr14 | 106962965 | 106963167 | IGHV1-45 | Genotyping |
| 746 | chr14 | 106963170 | 106963280 | IGHV1-45 | Genotyping |
| 747 | chr14 | 106967130 | 106967209 | IGHV1-46 | Genotyping |
| 748 | chr14 | 106967315 | 106967397 | IGHV1-46 | Genotyping |
| 749 | chr14 | 106994300 | 106994376 | IGHV3-48 | Phased Variants |
| 750 | chr14 | 106994430 | 106994534 | IGHV3-48 | Phased Variants |
| 751 | chr14 | 106994545 | 106994618 | IGHV3-48 | Phased Variants |
| 752 | chr14 | 106994660 | 106994745 | IGHV3-48 | Phased Variants |
| 753 | chr14 | 106994760 | 106994904 | IGHV3-48 | Phased Variants |
| 754 | chr14 | 107013035 | 107013204 | IGHV3-49 | Genotyping |
| 755 | chr14 | 107034665 | 107034845 | IGHV5-51 | Genotyping |
| 756 | chr14 | 107034955 | 107035097 | IGHV5-51 | Genotyping |
| 757 | chr14 | 107078455 | 107078631 | IGHV1-58 | Genotyping |
| 758 | chr14 | 107083565 | 107083726 | IGHV4-59 | Phased Variants |
| 759 | chr14 | 107083790 | 107083923 | IGHV4-59 | Phased Variants |
| 760 | chr14 | 107113405 | 107113560 | IGHV3-64 | Phased Variants |
| 761 | chr14 | 107113820 | 107113922 | IGHV3-64 | Phased Variants |
| 762 | chr14 | 107114095 | 107114238 | IGHV3-64 | Phased Variants |
| 763 | chr14 | 107136755 | 107136899 | IGHV3-66 | Phased Variants |
| 764 | chr14 | 107169645 | 107169841 | IGHV1-69 | Phased Variants |
| 765 | chr14 | 107169970 | 107170195 | IGHV1-69 | Phased Variants |
| 766 | chr14 | 107170220 | 107170472 | IGHV1-69 | Phased Variants |
| 767 | chr14 | 107170475 | 107170563 | IGHV1-69 | Phased Variants |
| 768 | chr14 | 107170660 | 107170871 | IGHV1-69 | Phased Variants |
| 769 | chr14 | 107178305 | 107178377 | IGHV2-70 | Phased Variants |
| 770 | chr14 | 107178415 | 107178869 | IGHV2-70 | Phased Variants |
| 771 | chr14 | 107178880 | 107179116 | IGHV2-70 | Phased Variants |
| 772 | chr14 | 107179130 | 107179339 | IGHV2-70 | Phased Variants |
| 773 | chr14 | 107179360 | 107180001 | IGHV2-70 | Phased Variants |

| 774 | chr14 | 107199020 | 107199094 | IGHV3-72 | Genotyping |
|---|---|---|---|---|---|
| 775 | chr14 | 107199095 | 107199173 | IGHV3-72 | Genotyping |
| 776 | chr14 | 107210955 | 107211159 | IGHV3-73 | Genotyping |
| 777 | chr14 | 107218755 | 107218891 | IGHV3-74 | Genotyping |
| 778 | chr14 | 107258910 | 107259078 | IGHV7-81 | Phased Variants |
| 779 | chr14 | 107259100 | 107259206 | IGHV7-81 | Phased Variants |
| 780 | chr14 | 107259235 | 107259444 | IGHV7-81 | Phased Variants |
| 781 | chr14 | 107259555 | 107259635 | IGHV7-81 | Phased Variants |
| 782 | chr14 | 107282770 | 107282884 | IGHV7-81 | Genotyping |
| 783 | chr14 | 107282945 | 107283018 | IGHV7-81 | Genotyping |
| 784 | chr15 | 45003678 | 45003861 | B2M | Genotyping |
| 785 | chr15 | 45007718 | 45007927 | B2M | Genotyping |
| 786 | chr15 | 45008463 | 45008603 | B2M | Genotyping |
| 787 | chr15 | 66727354 | 66727536 | MAP2K1 | Genotyping |
| 788 | chr15 | 66729014 | 66729123 | MAP2K1 | Genotyping |
| 789 | chr15 | 66729139 | 66729292 | MAP2K1 | Genotyping |
| 790 | chr15 | 86312062 | 86312565 | KLHL25 | Genotyping |
| 791 | chr16 | 2812096 | 2812786 | SRRM2 | Genotyping |
| 792 | chr16 | 3779106 | 3779320 | CREBBP | Genotyping |
| 793 | chr16 | 3781171 | 3781464 | CREBBP | Genotyping |
| 794 | chr16 | 3781756 | 3781972 | CREBBP | Genotyping |
| 795 | chr16 | 3786011 | 3786223 | CREBBP | Genotyping |
| 796 | chr16 | 3786591 | 3786885 | CREBBP | Genotyping |
| 797 | chr16 | 3788511 | 3788716 | CREBBP | Genotyping |
| 798 | chr16 | 3789521 | 3789770 | CREBBP | Genotyping |
| 799 | chr16 | 3790376 | 3790580 | CREBBP | Genotyping |
| 800 | chr16 | 3794846 | 3794994 | CREBBP | Genotyping |
| 801 | chr16 | 3808801 | 3809009 | CREBBP | Genotyping |
| 802 | chr16 | 3817706 | 3817915 | CREBBP | Genotyping |
| 803 | chr16 | 3823711 | 3823942 | CREBBP | Genotyping |
| 804 | chr16 | 3824536 | 3824719 | CREBBP | Genotyping |
| 805 | chr16 | 3832716 | 3832942 | CREBBP | Genotyping |
| 806 | chr16 | 3900236 | 3900462 | CREBBP | Genotyping |
| 807 | chr16 | 3900561 | 3900914 | CREBBP | Genotyping |
| 808 | chr16 | 10971440 | 10973882 | CIITA | Phased Variants |
| 809 | chr16 | 10973885 | 10974203 | CIITA | Phased Variants |
| 810 | chr16 | 11348520 | 11349249 | SOCS1 | Phased Variants |
| 811 | chr16 | 30093722 | 30093935 | PPP4C | Genotyping |
| 812 | chr16 | 33523607 | 33523675 | IGHV3OR16-12 | Phased Variants |
| 813 | chr16 | 81946175 | 81946356 | PLCG2 | Genotyping |
| 814 | chr16 | 81953055 | 81953307 | PLCG2 | Genotyping |
| 815 | chr16 | 81962120 | 81962263 | PLCG2 | Genotyping |
| 816 | chr16 | 85933003 | 85933569 | IRF8 | Phased Variants |

| 817 | chr16 | 85936563 | 85936836 | IRF8 | Genotyping |
|---|---|---|---|---|---|
| 818 | chr16 | 85942563 | 85942821 | IRF8 | Genotyping |
| 819 | chr16 | 85945108 | 85945330 | IRF8 | Genotyping |
| 820 | chr16 | 85946708 | 85946887 | IRF8 | Genotyping |
| 821 | chr16 | 85948018 | 85948170 | IRF8 | Genotyping |
| 822 | chr16 | 85951993 | 85952448 | IRF8 | Genotyping |
| 823 | chr16 | 85953683 | 85953837 | IRF8 | Genotyping |
| 824 | chr16 | 85954723 | 85954937 | IRF8 | Genotyping |
| 825 | chr17 | 5366796 | 5367031 | DHX33 | Genotyping |
| 826 | chr17 | 7576949 | 7577197 | TP53 | Genotyping |
| 827 | chr17 | 7577444 | 7577683 | TP53 | Genotyping |
| 828 | chr17 | 7578129 | 7578336 | TP53 | Genotyping |
| 829 | chr17 | 7578344 | 7578591 | TP53 | Genotyping |
| 830 | chr17 | 7579259 | 7579428 | TP53 | Genotyping |
| 831 | chr17 | 18001529 | 18001704 | DRG2 | Genotyping |
| 832 | chr17 | 18022119 | 18022791 | MYO15A | Genotyping |
| 833 | chr17 | 40467709 | 40467857 | STAT3 | Genotyping |
| 834 | chr17 | 40469104 | 40469321 | STAT3 | Genotyping |
| 835 | chr17 | 40474309 | 40474530 | STAT3 | Genotyping |
| 836 | chr17 | 40474974 | 40475190 | STAT3 | Genotyping |
| 837 | chr17 | 40475254 | 40475394 | STAT3 | Genotyping |
| 838 | chr17 | 40478074 | 40478252 | STAT3 | Genotyping |
| 839 | chr17 | 40485844 | 40486132 | STAT3 | Genotyping |
| 840 | chr17 | 40489754 | 40489903 | STAT3 | Genotyping |
| 841 | chr17 | 40491284 | 40491489 | STAT3 | Genotyping |
| 842 | chr17 | 41847058 | 41847241 | DUSP3 | Genotyping |
| 843 | chr17 | 51900441 | 51900897 | KIF2B | Genotyping |
| 844 | chr17 | 56408574 | 56408755 | BZRAP1 | Phased Variants |
| 845 | chr17 | 56408884 | 56409615 | BZRAP1 | Phased Variants |
| 846 | chr17 | 62006520 | 62006919 | CD79B | Genotyping |
| 847 | chr17 | 62007105 | 62007279 | CD79B | Genotyping |
| 848 | chr17 | 62007410 | 62007761 | CD79B | Genotyping |
| 849 | chr17 | 62008645 | 62008786 | CD79B | Genotyping |
| 850 | chr17 | 62009505 | 62009659 | CD79B | Genotyping |
| 851 | chr17 | 63010240 | 63010308 | GNA13 | Phased Variants |
| 852 | chr17 | 63010315 | 63010973 | GNA13 | Phased Variants |
| 853 | chr17 | 63014313 | 63014461 | GNA13 | Genotyping |
| 854 | chr17 | 63049573 | 63049774 | GNA13 | Genotyping |
| 855 | chr17 | 63052443 | 63052678 | GNA13 | Genotyping |
| 856 | chr17 | 75447868 | 75448421 | 9-Sep | Phased Variants |
| 857 | chr17 | 78343503 | 78343715 | RNF213 | Genotyping |
| 858 | chr17 | 79478953 | 79479026 | ACTG1 | Genotyping |
| 859 | chr18 | 1477565 | 1477666 | ADCYAP1 | Phased Variants |

| 860 | chr18 | 6947104 | 6947347 | LAMA1 | Genotyping |
|-----|-------|---------|---------|-------|------------|
| 861 | chr18 | 6980464 | 6980680 | LAMA1 | Genotyping |
| 862 | chr18 | 13825915 | 13826461 | MC5R | Genotyping |
| 863 | chr18 | 30349775 | 30350300 | AC012123.1 | Phased Variants |
| 864 | chr18 | 48231684 | 48232112 | MAPK4 | Genotyping |
| 865 | chr18 | 48327694 | 48327901 | MRO | Genotyping |
| 866 | chr18 | 48512954 | 48513347 | ELAC1 | Genotyping |
| 867 | chr18 | 48591759 | 48592011 | SMAD4 | Genotyping |
| 868 | chr18 | 48593364 | 48593571 | SMAD4 | Genotyping |
| 869 | chr18 | 48604604 | 48604852 | SMAD4 | Genotyping |
| 870 | chr18 | 48703169 | 48703965 | MEX3C | Genotyping |
| 871 | chr18 | 53804515 | 53804796 | TXNL1 | Genotyping |
| 872 | chr18 | 55274405 | 55274580 | NARS | Genotyping |
| 873 | chr18 | 55319680 | 55319999 | ATP8B1 | Genotyping |
| 874 | chr18 | 55329690 | 55329857 | ATP8B1 | Genotyping |
| 875 | chr18 | 55359005 | 55359259 | ATP8B1 | Genotyping |
| 876 | chr18 | 56054915 | 56055594 | NEDD4L | Genotyping |
| 877 | chr18 | 56063365 | 56063826 | NEDD4L | Genotyping |
| 878 | chr18 | 60763829 | 60764032 | BCL2 | Genotyping |
| 879 | chr18 | 60764299 | 60764540 | BCL2 | Genotyping |
| 880 | chr18 | 60774414 | 60774660 | BCL2 | Genotyping |
| 881 | chr18 | 60793369 | 60793654 | BCL2 | Genotyping |
| 882 | chr18 | 60795829 | 60796006 | BCL2 | Genotyping |
| 883 | chr18 | 60806264 | 60806836 | BCL2 | Phased Variants |
| 884 | chr18 | 60983784 | 60983991 | BCL2 | Phased Variants |
| 885 | chr18 | 60984454 | 60986731 | BCL2 | Phased Variants |
| 886 | chr18 | 60986844 | 60987047 | BCL2 | Phased Variants |
| 887 | chr18 | 60987964 | 60988511 | BCL2 | Phased Variants |
| 888 | chr18 | 64172116 | 64172531 | CDH19 | Genotyping |
| 889 | chr18 | 64176241 | 64176518 | CDH19 | Genotyping |
| 890 | chr18 | 64239166 | 64239357 | CDH19 | Genotyping |
| 891 | chr18 | 65179856 | 65181824 | DSEL | Genotyping |
| 892 | chr18 | 73944893 | 73945380 | ZNF516 | Genotyping |
| 893 | chr18 | 75683734 | 75684502 | GALR1 | Genotyping |
| 894 | chr18 | 77092820 | 77093034 | ATP9B | Genotyping |
| 895 | chr18 | 77170715 | 77171032 | NFATC1 | Genotyping |
| 896 | chr18 | 77208755 | 77208996 | NFATC1 | Genotyping |
| 897 | chr18 | 77227415 | 77227661 | NFATC1 | Genotyping |
| 898 | chr18 | 77288040 | 77288611 | NFATC1 | Genotyping |
| 899 | chr18 | 77794425 | 77795130 | RBFA | Genotyping |
| 900 | chr19 | 1376440 | 1376662 | MUM1 | Genotyping |
| 901 | chr19 | 6586161 | 6586445 | CD70 | Genotyping |
| 902 | chr19 | 6590026 | 6590238 | CD70 | Genotyping |

| 903 | chr19 | 6590786 | 6591079 | CD70 | Genotyping |
|-----|-------|---------|---------|------|------------|
| 904 | chr19 | 8028408 | 8028583 | ELAVL1 | Genotyping |
| 905 | chr19 | 10334563 | 10335187 | S1PR2 | Genotyping |
| 906 | chr19 | 10335308 | 10335585 | S1PR2 | Genotyping |
| 907 | chr19 | 10340823 | 10341376 | S1PR2 | Phased Variants |
| 908 | chr19 | 10341833 | 10341984 | S1PR2 | Genotyping |
| 909 | chr19 | 12902574 | 12902861 | JUNB | Genotyping |
| 910 | chr19 | 19256469 | 19256851 | MEF2B | Genotyping |
| 911 | chr19 | 19257044 | 19257222 | MEF2B | Genotyping |
| 912 | chr19 | 19257339 | 19257480 | MEF2B | Genotyping |
| 913 | chr19 | 19257489 | 19257741 | MEF2B | Genotyping |
| 914 | chr19 | 19257824 | 19258036 | MEF2B | Genotyping |
| 915 | chr19 | 19258484 | 19258662 | MEF2B | Genotyping |
| 916 | chr19 | 19259984 | 19260176 | MEF2B | Genotyping |
| 917 | chr19 | 19261414 | 19261588 | MEF2B | Genotyping |
| 918 | chr19 | 19293309 | 19293478 | MEF2BNB | Genotyping |
| 919 | chr19 | 42599890 | 42600121 | POU2F2 | Genotyping |
| 920 | chr19 | 51525626 | 51525937 | KLK11 | Genotyping |
| 921 | chr19 | 51559441 | 51560040 | KLK13 | Genotyping |
| 922 | chr19 | 51561771 | 51561943 | KLK13 | Genotyping |
| 923 | chr19 | 52381611 | 52381786 | ZNF577 | Genotyping |
| 924 | chr19 | 52403336 | 52403586 | ZNF649 | Genotyping |
| 925 | chr19 | 52961146 | 52961224 | ZNF534 | Genotyping |
| 926 | chr19 | 52961226 | 52961578 | ZNF534 | Genotyping |
| 927 | chr19 | 53598586 | 53599055 | ZNF160 | Genotyping |
| 928 | chr20 | 23028372 | 23028858 | THBD | Genotyping |
| 929 | chr20 | 25003526 | 25003774 | ACSS1 | Genotyping |
| 930 | chr20 | 46131072 | 46131213 | NCOA3 | Phased Variants |
| 931 | chr20 | 46131217 | 46131287 | NCOA3 | Phased Variants |
| 932 | chr21 | 18981233 | 18981504 | BTG3 | Genotyping |
| 933 | chr21 | 28213258 | 28213536 | ADAMTS1 | Genotyping |
| 934 | chr21 | 28216763 | 28217005 | ADAMTS1 | Genotyping |
| 935 | chr22 | 22380472 | 22381038 | IGLV4-69 | Phased Variants |
| 936 | chr22 | 22385622 | 22385767 | IGLV4-69 | Genotyping |
| 937 | chr22 | 22385777 | 22385898 | IGLV4-69 | Genotyping |
| 938 | chr22 | 22453287 | 22453502 | IGLV8-61 | Genotyping |
| 939 | chr22 | 22453527 | 22453608 | IGLV8-61 | Genotyping |
| 940 | chr22 | 22516707 | 22516785 | IGLV4-60 | Phased Variants |
| 941 | chr22 | 22516827 | 22517113 | IGLV4-60 | Phased Variants |
| 942 | chr22 | 22550337 | 22550812 | IGLV6-57 | Genotyping |
| 943 | chr22 | 22556227 | 22556630 | IGLV11-55 | Genotyping |
| 944 | chr22 | 22569332 | 22569655 | IGLV10-54 | Genotyping |
| 945 | chr22 | 22673242 | 22673607 | IGLV5-52 | Genotyping |

| 946 | chr22 | 22677077 | 22677216 | IGLV1-51 | Phased Variants |
| 947 | chr22 | 22677227 | 22677337 | IGLV1-51 | Genotyping |
| 948 | chr22 | 22681927 | 22682007 | IGLV1-50 | Genotyping |
| 949 | chr22 | 22682097 | 22682213 | IGLV1-50 | Genotyping |
| 950 | chr22 | 22697727 | 22698123 | IGLV9-49 | Genotyping |
| 951 | chr22 | 22707427 | 22707509 | IGLV5-48 | Genotyping |
| 952 | chr22 | 22707517 | 22707658 | IGLV5-48 | Phased Variants |
| 953 | chr22 | 22707742 | 22707823 | IGLV5-48 | Genotyping |
| 954 | chr22 | 22712077 | 22712496 | IGLV1-47 | Phased Variants |
| 955 | chr22 | 22712512 | 22712625 | IGLV1-47 | Genotyping |
| 956 | chr22 | 22723897 | 22724189 | IGLV7-46 | Phased Variants |
| 957 | chr22 | 22724207 | 22724494 | IGLV7-46 | Phased Variants |
| 958 | chr22 | 22730452 | 22730552 | IGLV5-45 | Phased Variants |
| 959 | chr22 | 22730607 | 22730756 | IGLV5-45 | Phased Variants |
| 960 | chr22 | 22730887 | 22730955 | IGLV5-45 | Phased Variants |
| 961 | chr22 | 22735417 | 22735604 | IGLV1-44 | Phased Variants |
| 962 | chr22 | 22735792 | 22735878 | IGLV1-44 | Phased Variants |
| 963 | chr22 | 22749602 | 22749701 | IGLV7-43 | Phased Variants |
| 964 | chr22 | 22749732 | 22749853 | IGLV7-43 | Phased Variants |
| 965 | chr22 | 22749857 | 22749939 | IGLV7-43 | Phased Variants |
| 966 | chr22 | 22749942 | 22750074 | IGLV7-43 | Phased Variants |
| 967 | chr22 | 22750092 | 22750342 | IGLV7-43 | Phased Variants |
| 968 | chr22 | 22758647 | 22759294 | IGLV1-40 | Phased Variants |
| 969 | chr22 | 22759297 | 22759377 | IGLV1-40 | Phased Variants |
| 970 | chr22 | 22764167 | 22764309 | IGLV1-40 | Phased Variants |
| 971 | chr22 | 22764367 | 22764450 | IGLV1-40 | Phased Variants |
| 972 | chr22 | 22764552 | 22764634 | IGLV1-40 | Phased Variants |
| 973 | chr22 | 22782037 | 22782325 | IGLV5-37 | Genotyping |
| 974 | chr22 | 22786477 | 22786702 | IGLV1-36 | Genotyping |
| 975 | chr22 | 22786727 | 22786842 | IGLV1-36 | Genotyping |
| 976 | chr22 | 22930852 | 22931173 | IGLV2-33 | Genotyping |
| 977 | chr22 | 22937192 | 22937341 | IGLV3-32 | Genotyping |
| 978 | chr22 | 22937347 | 22937548 | IGLV3-32 | Genotyping |
| 979 | chr22 | 23010977 | 23011143 | IGLV3-27 | Genotyping |
| 980 | chr22 | 23011172 | 23011316 | IGLV3-27 | Genotyping |
| 981 | chr22 | 23029497 | 23029581 | IGLV3-25 | Genotyping |
| 982 | chr22 | 23029622 | 23029778 | IGLV3-25 | Genotyping |
| 983 | chr22 | 23040452 | 23040527 | IGLV2-23 | Phased Variants |
| 984 | chr22 | 23040592 | 23040811 | IGLV2-23 | Phased Variants |
| 985 | chr22 | 23040852 | 23041365 | IGLV2-23 | Phased Variants |
| 986 | chr22 | 23047067 | 23047329 | IGLV3-22 | Genotyping |
| 987 | chr22 | 23055367 | 23055445 | IGLV3-21 | Genotyping |
| 988 | chr22 | 23055497 | 23055577 | IGLV3-21 | Phased Variants |

| 989 | chr22 | 23055727 | 23055857 | IGLV3-21 | Phased Variants |
|---|---|---|---|---|---|
| 990 | chr22 | 23063307 | 23063661 | IGLV3-19 | Genotyping |
| 991 | chr22 | 23077337 | 23077435 | IGLV2-18 | Genotyping |
| 992 | chr22 | 23077537 | 23077615 | IGLV2-18 | Genotyping |
| 993 | chr22 | 23090122 | 23090205 | IGLV3-16 | Genotyping |
| 994 | chr22 | 23090287 | 23090372 | IGLV3-16 | Genotyping |
| 995 | chr22 | 23101392 | 23101473 | IGLV2-14 | Phased Variants |
| 996 | chr22 | 23101532 | 23101605 | IGLV2-14 | Phased Variants |
| 997 | chr22 | 23101652 | 23101735 | IGLV2-14 | Genotyping |
| 998 | chr22 | 23114792 | 23114874 | IGLV3-12 | Genotyping |
| 999 | chr22 | 23114947 | 23115052 | IGLV3-12 | Genotyping |
| 1000 | chr22 | 23135152 | 23135230 | IGLV2-11 | Genotyping |
| 1001 | chr22 | 23135247 | 23135399 | IGLV2-11 | Genotyping |
| 1002 | chr22 | 23135437 | 23135521 | IGLV2-11 | Genotyping |
| 1003 | chr22 | 23154347 | 23154477 | IGLV3-10 | Phased Variants |
| 1004 | chr22 | 23154597 | 23154815 | IGLV3-10 | Phased Variants |
| 1005 | chr22 | 23161917 | 23162052 | IGLV3-9 | Genotyping |
| 1006 | chr22 | 23162072 | 23162290 | IGLV3-9 | Genotyping |
| 1007 | chr22 | 23165422 | 23165496 | IGLV2-8 | Phased Variants |
| 1008 | chr22 | 23165542 | 23165680 | IGLV2-8 | Phased Variants |
| 1009 | chr22 | 23165727 | 23165811 | IGLV2-8 | Phased Variants |
| 1010 | chr22 | 23192412 | 23192818 | IGLV4-3 | Phased Variants |
| 1011 | chr22 | 23197917 | 23198053 | IGLV4-3 | Phased Variants |
| 1012 | chr22 | 23198067 | 23198475 | IGLV4-3 | Phased Variants |
| 1013 | chr22 | 23198587 | 23198732 | IGLV4-3 | Phased Variants |
| 1014 | chr22 | 23198797 | 23198869 | IGLV4-3 | Phased Variants |
| 1015 | chr22 | 23199022 | 23199127 | IGLV4-3 | Phased Variants |
| 1016 | chr22 | 23199182 | 23199261 | IGLV4-3 | Phased Variants |
| 1017 | chr22 | 23199277 | 23199671 | IGLV4-3 | Phased Variants |
| 1018 | chr22 | 23213857 | 23214141 | IGLV4-3 | Genotyping |
| 1019 | chr22 | 23214167 | 23214249 | IGLV4-3 | Genotyping |
| 1020 | chr22 | 23222927 | 23223065 | IGLV3-1 | Phased Variants |
| 1021 | chr22 | 23223077 | 23223319 | IGLV3-1 | Phased Variants |
| 1022 | chr22 | 23223327 | 23224010 | IGLV3-1 | Phased Variants |
| 1023 | chr22 | 23227062 | 23227279 | IGLL5 | Phased Variants |
| 1024 | chr22 | 23227567 | 23227896 | IGLL5 | Phased Variants |
| 1025 | chr22 | 23227897 | 23228624 | IGLL5 | Phased Variants |
| 1026 | chr22 | 23229332 | 23229550 | IGLL5 | Phased Variants |
| 1027 | chr22 | 23229562 | 23229739 | IGLL5 | Phased Variants |
| 1028 | chr22 | 23230012 | 23231063 | IGLL5 | Phased Variants |
| 1029 | chr22 | 23231072 | 23231764 | IGLL5 | Phased Variants |
| 1030 | chr22 | 23231927 | 23232005 | IGLL5 | Phased Variants |
| 1031 | chr22 | 23232062 | 23232346 | IGLL5 | Phased Variants |

| 1032 | chr22 | 23232362 | 23232465 | IGLL5 | Phased Variants |
|---|---|---|---|---|---|
| 1033 | chr22 | 23232517 | 23232737 | IGLL5 | Phased Variants |
| 1034 | chr22 | 23234612 | 23235837 | IGLJ1 | Phased Variants |
| 1035 | chr22 | 23235847 | 23236276 | IGLJ1 | Phased Variants |
| 1036 | chr22 | 23236277 | 23236378 | IGLJ1 | Phased Variants |
| 1037 | chr22 | 23236387 | 23236526 | IGLJ1 | Phased Variants |
| 1038 | chr22 | 23236557 | 23236851 | IGLJ1 | Phased Variants |
| 1039 | chr22 | 23236877 | 23237366 | IGLC1 | Phased Variants |
| 1040 | chr22 | 23241762 | 23241835 | IGLJ2 | Genotyping |
| 1041 | chr22 | 23242602 | 23242981 | IGLC2 | Phased Variants |
| 1042 | chr22 | 23244157 | 23244373 | IGLC2 | Phased Variants |
| 1043 | chr22 | 23247137 | 23247209 | IGLJ3 | Genotyping |
| 1044 | chr22 | 23247257 | 23247444 | IGLJ3 | Phased Variants |
| 1045 | chr22 | 23247467 | 23247630 | IGLJ3 | Phased Variants |
| 1046 | chr22 | 23248182 | 23248404 | IGLC3 | Phased Variants |
| 1047 | chr22 | 23252687 | 23252824 | IGLJ4 | Genotyping |
| 1048 | chr22 | 23256362 | 23256504 | IGLJ5 | Genotyping |
| 1049 | chr22 | 23260267 | 23260399 | IGLJ6 | Genotyping |
| 1050 | chr22 | 23263507 | 23263653 | IGLJ7 | Genotyping |
| 1051 | chr22 | 23263872 | 23264263 | IGLJ7 | Phased Variants |
| 1052 | chr22 | 23278157 | 23278381 | IGLC7 | Phased Variants |
| 1053 | chr22 | 23282767 | 23282839 | IGLC7 | Phased Variants |
| 1054 | chr22 | 23282842 | 23282956 | IGLC7 | Phased Variants |
| 1055 | chr22 | 23523567 | 23524204 | BCR | Genotyping |
| 1056 | chr22 | 23524212 | 23524419 | BCR | Genotyping |
| 1057 | chr22 | 23610547 | 23610791 | BCR | Genotyping |
| 1058 | chr22 | 29191136 | 29191455 | XBP1 | Genotyping |
| 1059 | chr22 | 29191461 | 29191746 | XBP1 | Genotyping |
| 1060 | chr22 | 29192006 | 29192215 | XBP1 | Genotyping |
| 1061 | chr22 | 29193041 | 29193205 | XBP1 | Genotyping |
| 1062 | chr22 | 29196261 | 29196547 | XBP1 | Genotyping |
| 1063 | chr22 | 41513340 | 41513562 | EP300 | Genotyping |
| 1064 | chr22 | 41525845 | 41526047 | EP300 | Genotyping |
| 1065 | chr22 | 41527440 | 41527664 | EP300 | Genotyping |
| 1066 | chr22 | 41536110 | 41536291 | EP300 | Genotyping |
| 1067 | chr22 | 41545740 | 41545940 | EP300 | Genotyping |
| 1068 | chr22 | 41545995 | 41546223 | EP300 | Genotyping |
| 1069 | chr22 | 41565485 | 41565650 | EP300 | Genotyping |
| 1070 | chr22 | 41566385 | 41566592 | EP300 | Genotyping |
| 1071 | chr22 | 41568480 | 41568693 | EP300 | Genotyping |
| 1072 | chr22 | 41569600 | 41569814 | EP300 | Genotyping |
| 1073 | chr22 | 41572225 | 41572436 | EP300 | Genotyping |
| 1074 | chr22 | 41572800 | 41573022 | EP300 | Genotyping |

| 1075 | chr22 | 41573300 | 41573515 | EP300 | Genotyping |
|---|---|---|---|---|---|
| 1076 | chr22 | 41574255 | 41574486 | EP300 | Genotyping |
| 1077 | chr22 | 41574685 | 41574904 | EP300 | Genotyping |
| 1078 | chr22 | 47570209 | 47570414 | TBC1D22A | Phased Variants |
| 1079 | chrX | 1584324 | 1585521 | P2RY8 | Genotyping |
| 1080 | chrX | 1655789 | 1656029 | AKAP17A | Genotyping |
| 1081 | chrX | 12993264 | 12993539 | TMSB4X | Phased Variants |
| 1082 | chrX | 12993544 | 12994173 | TMSB4X | Phased Variants |
| 1083 | chrX | 12994289 | 12994397 | TMSB4X | Phased Variants |
| 1084 | chrX | 12994444 | 12994514 | TMSB4X | Phased Variants |
| 1085 | chrX | 33146106 | 33146490 | DMD | Phased Variants |
| 1086 | chrX | 35820576 | 35821268 | MAGEB16 | Genotyping |
| 1087 | chrX | 70347816 | 70348034 | MED12 | Genotyping |
| 1088 | chrX | 70612661 | 70612778 | TAF1 | Genotyping |
| 1089 | chrX | 73962123 | 73963110 | KIAA2022 | Genotyping |
| 1090 | chrX | 86772953 | 86773345 | KLHL4 | Genotyping |
| 1091 | chrX | 90026453 | 90026652 | PABPC5 | Phased Variants |
| 1092 | chrX | 100610984 | 100611308 | BTK | Genotyping |
| 1093 | chrX | 119509280 | 119509492 | ATP1B4 | Genotyping |
| 1094 | chrX | 141291052 | 141291326 | MAGEC2 | Genotyping |
| 1095 | chrX | 141291357 | 141291566 | MAGEC2 | Genotyping |
| 1096 | chrX | 153997383 | 153997622 | DKC1 | Genotyping |

Table 4

| # | Chromosome | Region Start | Region End | Number of 50bp bins | Gene | Mean frac DLBCL with PV | Mean frac GCB with PV | Mean frac ABC with PV | Mean frac PMBCL with PV | Mean frac cHL with PV | ranksumP ABCvsGCB | ranksumP PMBCLvsDLBCL | ranksumP cHLvsDLBCL |
|---|---|---|---|---|---|---|---|---|---|---|---|---|---|
| 1 | chr22 | 23227063 | 23237340 | 135 | IGLL5 | 0.184 | 0.158 | 0.224 | 0.242 | 0.088 | 0.00000 | 0.00003 | 0.00000 |
| 2 | chr18 | 60763830 | 60988465 | 104 | BCL2 | 0.111 | 0.165 | 0.029 | 0.056 | 0.004 | 0.00000 | 0.00000 | 0.00000 |
| 3 | chr14 | 106239251 | 106241954 | 49 | IGHG3 | 0.193 | 0.155 | 0.251 | 0.105 | 0.032 | 0.00000 | 0.00000 | 0.00000 |
| 4 | chr14 | 106092381 | 106095531 | 51 | IGHG4 | 0.179 | 0.155 | 0.217 | 0.136 | 0.056 | 0.00000 | 0.00000 | 0.00000 |
| 5 | chr6 | 37138285 | 37141880 | 36 | PIM1 | 0.073 | 0.039 | 0.124 | 0.068 | 0.000 | 0.00000 | 0.00251 | 0.00000 |
| 6 | chr22 | 22758648 | 22764603 | 22 | IGLV1-40 | 0.064 | 0.098 | 0.013 | 0.102 | 0.000 | 0.00000 | 0.46986 | 0.00001 |
| 7 | chr2 | 89161240 | 89165610 | 66 | IGKJ1 | 0.144 | 0.134 | 0.160 | 0.140 | 0.109 | 0.00000 | 0.00006 | 0.36296 |
| 8 | chr14 | 106829686 | 106831586 | 30 | IGHV4-34 | 0.077 | 0.049 | 0.121 | 0.100 | 0.012 | 0.00000 | 0.10144 | 0.01432 |
| 9 | chr2 | 89158619 | 89160190 | 32 | IGKJ5 | 0.307 | 0.286 | 0.339 | 0.350 | 0.219 | 0.00000 | 0.28398 | 0.00000 |
| 10 | chr22 | 23222928 | 23223998 | 22 | IGLV3-1 | 0.266 | 0.300 | 0.215 | 0.429 | 0.208 | 0.00000 | 0.00000 | 0.22589 |
| 11 | chr14 | 106211961 | 106214011 | 39 | IGHG1 | 0.229 | 0.197 | 0.277 | 0.131 | 0.035 | 0.00000 | 0.00000 | 0.00000 |
| 12 | chr14 | 106329751 | 106330201 | 10 | IGHJ5 | 0.320 | 0.261 | 0.410 | 0.375 | 0.148 | 0.00000 | 0.24822 | 0.00000 |
| 13 | chr3 | 187957433 | 188471931 | 54 | LPP | 0.080 | 0.102 | 0.046 | 0.168 | 0.062 | 0.00001 | 0.00027 | 0.00345 |
| 14 | chr2 | 89160890 | 89161190 | 7 | IGKJ2 | 0.151 | 0.096 | 0.236 | 0.116 | 0.062 | 0.00001 | 0.02569 | 0.00086 |
| 15 | chr6 | 134491383 | 134495968 | 64 | SGK1 | 0.039 | 0.053 | 0.018 | 0.075 | 0.001 | 0.00002 | 0.58192 | 0.99403 |
| 16 | chr6 | 150954421 | 150954821 | 9 | PLEKHG1 | 0.067 | 0.049 | 0.094 | 0.063 | 0.000 | 0.00002 | 0.11666 | 0.00114 |
| 17 | chr2 | 89246682 | 89247982 | 18 | IGKV1-5 | 0.031 | 0.023 | 0.043 | 0.097 | 0.024 | 0.00003 | 0.01798 | 0.00005 |
| 18 | chr8 | 128746808 | 128764273 | 164 | MYC | 0.037 | 0.047 | 0.021 | 0.039 | 0.001 | 0.00003 | 0.00000 | 0.86966 |
| 19 | chr22 | 23040453 | 23041334 | 17 | IGLV2-23 | 0.051 | 0.073 | 0.018 | 0.088 | 0.005 | 0.00003 | 0.77724 | 0.04594 |
| 20 | chr2 | 89160240 | 89160540 | 7 | IGKJ4 | 0.259 | 0.225 | 0.311 | 0.241 | 0.130 | 0.00003 | 0.04157 | 0.00006 |
| 21 | chr22 | 22516708 | 22517100 | 8 | IGLV4-60 | 0.084 | 0.117 | 0.034 | 0.078 | 0.022 | 0.00003 | 0.17854 | 0.01628 |
| 22 | chr12 | 122458782 | 122463132 | 48 | BCL7A | 0.091 | 0.106 | 0.068 | 0.173 | 0.041 | 0.00005 | 0.00033 | 0.01552 |
| 23 | chr14 | 107178306 | 107179990 | 33 | IGHV2-70 | 0.224 | 0.242 | 0.195 | 0.182 | 0.115 | 0.00006 | 0.00002 | 0.00004 |
| 24 | chr2 | 89160590 | 89160840 | 6 | IGKJ3 | 0.185 | 0.137 | 0.258 | 0.135 | 0.109 | 0.00006 | 0.00291 | 0.00284 |
| 25 | chr22 | 22730453 | 22730938 | 7 | IGLV5-45 | 0.069 | 0.108 | 0.011 | 0.107 | 0.019 | 0.00010 | 0.70241 | 0.37522 |
| 26 | chr22 | 23248183 | 23248383 | 5 | IGLC3 | 0.164 | 0.236 | 0.055 | 0.113 | 0.035 | 0.00014 | 0.00837 | 0.00072 |
| 27 | chr2 | 89127262 | 89158569 | 66 | IGKC | 0.089 | 0.077 | 0.107 | 0.164 | 0.041 | 0.00022 | 0.00008 | 0.04625 |
| 28 | chr9 | 37293170 | 37384885 | 18 | ZCCHC7 | 0.055 | 0.075 | 0.025 | 0.069 | 0.002 | 0.00023 | 0.36871 | 0.42872 |
| 29 | chr14 | 106732971 | 106733441 | 9 | IGHV1-24 | 0.036 | 0.060 | 0.000 | 0.090 | 0.000 | 0.00026 | 0.33149 | 0.77291 |
| 30 | chr2 | 89184967 | 89185677 | 15 | IGKV4-1 | 0.103 | 0.133 | 0.057 | 0.133 | 0.078 | 0.00035 | 0.83189 | 0.36813 |
| 31 | chr2 | 59821915 | 60773435 | 12 | BCL11A | 0.035 | 0.053 | 0.008 | 0.089 | 0.000 | 0.00075 | 0.19138 | 0.80319 |
| 32 | chr20 | 46131073 | 46131277 | 5 | NCOA3 | 0.071 | 0.102 | 0.025 | 0.025 | 0.009 | 0.00085 | 0.00670 | 0.02848 |
| 33 | chr22 | 23165423 | 23165766 | 6 | IGLV2-8 | 0.045 | 0.022 | 0.079 | 0.083 | 0.043 | 0.00090 | 0.90873 | 0.01148 |
| 34 | chr8 | 8748688 | 8750268 | 17 | MFHAS1 | 0.033 | 0.051 | 0.004 | 0.055 | 0.000 | 0.00099 | 0.48925 | 0.69644 |
| 35 | chr19 | 52961147 | 52961549 | 9 | ZNF534 | 0.029 | 0.018 | 0.044 | 0.063 | 0.000 | 0.00113 | 0.75367 | 0.44231 |
| 36 | chr9 | 16435499 | 16436299 | 17 | BNC2 | 0.034 | 0.049 | 0.012 | 0.077 | 0.000 | 0.00119 | 0.51920 | 0.84956 |
| 37 | chr22 | 23264173 | 23282921 | 11 | IGLC7 | 0.041 | 0.061 | 0.011 | 0.131 | 0.000 | 0.00129 | 0.00884 | 0.29860 |
| 38 | chr14 | 106318101 | 106325773 | 50 | IGHM | 0.181 | 0.175 | 0.190 | 0.139 | 0.024 | 0.00192 | 0.00000 | 0.00000 |
| 39 | chr22 | 23235813 | 23235973 | 4 | IGLJ1 | 0.059 | 0.033 | 0.100 | 0.266 | 0.000 | 0.00225 | 0.00168 | 0.05724 |
| 40 | chr16 | 11348521 | 11349221 | 15 | SOCS1 | 0.108 | 0.126 | 0.080 | 0.292 | 0.046 | 0.00303 | 0.00000 | 0.07342 |
| 41 | chr16 | 10971441 | 10974194 | 56 | CIITA | 0.072 | 0.084 | 0.054 | 0.289 | 0.082 | 0.00307 | 0.00000 | 0.00000 |
| 42 | chr5 | 13864466 | 13864666 | 5 | DNAH5 | 0.034 | 0.056 | 0.000 | 0.088 | 0.000 | 0.00408 | 0.40676 | 0.90937 |

| | | | | | | | | | | | | | |
|---|---|---|---|---|---|---|---|---|---|---|---|---|---|
| 43 | chr6 | 27777784 | 27778062 | 6 | HIST1H3H | 0.041 | 0.025 | 0.067 | 0.042 | 0.000 | 0.00488 | 0.21081 | 0.62256 |
| 44 | chr22 | 23192413 | 23214234 | 46 | IGLV4-3 | 0.061 | 0.074 | 0.042 | 0.162 | 0.025 | 0.00501 | 0.00000 | 0.65960 |
| 45 | chr14 | 106330251 | 106330601 | 8 | IGHJ4 | 0.166 | 0.143 | 0.200 | 0.180 | 0.943 | 0.00606 | 0.43909 | 0.00002 |
| 46 | chr14 | 106877716 | 106877731 | 18 | IGHV4-39 | 0.050 | 0.064 | 0.028 | 0.059 | 0.053 | 0.00685 | 0.08333 | 0.00000 |
| 47 | chr10 | 90773867 | 90774067 | 5 | FAS | 0.042 | 0.066 | 0.005 | 0.038 | 0.000 | 0.00715 | 0.19681 | 0.45229 |
| 48 | chr22 | 22723898 | 22724466 | 12 | IGLV7-46 | 0.057 | 0.081 | 0.021 | 0.094 | 0.000 | 0.00728 | 0.81618 | 0.00596 |
| 49 | chr5 | 137801488 | 137801798 | 6 | EGR1 | 0.031 | 0.052 | 0.000 | 0.167 | 0.000 | 0.00799 | 0.01126 | 0.75859 |
| 50 | chr22 | 23242603 | 23244358 | 13 | IGLC2 | 0.139 | 0.164 | 0.160 | 0.163 | 0.094 | 0.00835 | 0.72971 | 0.51511 |
| 51 | chr22 | 22930853 | 22931153 | 7 | IGLV2-33 | 0.030 | 0.021 | 0.043 | 0.045 | 0.000 | 0.00870 | 0.55261 | 0.56841 |
| 52 | chr14 | 106325852 | 106329701 | 73 | IGHJ6 | 0.474 | 0.471 | 0.478 | 0.470 | 0.362 | 0.00948 | 0.02862 | 0.00000 |
| 53 | chr3 | 185697424 | 185697624 | 5 | TRA2B | 0.040 | 0.059 | 0.010 | 0.075 | 0.000 | 0.00954 | 0.90180 | 0.48859 |
| 54 | chr6 | 26056035 | 26056539 | 11 | HIST1H1C | 0.059 | 0.079 | 0.027 | 0.017 | 0.000 | 0.00967 | 0.00022 | 0.00680 |
| 55 | chr3 | 71551102 | 71551452 | 8 | FOXP1 | 0.015 | 0.006 | 0.028 | 0.031 | 0.011 | 0.00999 | 0.57172 | 0.00116 |
| 56 | chr3 | 187448190 | 187661368 | 137 | BCL6 | 0.106 | 0.116 | 0.089 | 0.126 | 0.044 | 0.01002 | 0.04210 | 0.00007 |
| 57 | chr11 | 128391384 | 128392103 | 15 | ETS1 | 0.061 | 0.059 | 0.065 | 0.021 | 0.000 | 0.01042 | 0.00001 | 0.00039 |
| 58 | chr13 | 46959166 | 46962031 | 13 | KIAA0226L | 0.034 | 0.029 | 0.042 | 0.067 | 0.000 | 0.01112 | 0.97915 | 0.84801 |
| 59 | chr11 | 118754794 | 118765389 | 17 | CXCR5 | 0.035 | 0.029 | 0.044 | 0.077 | 0.000 | 0.01378 | 0.40303 | 0.93788 |
| 60 | chr17 | 62006521 | 62009656 | 27 | CD79B | 0.041 | 0.039 | 0.044 | 0.083 | 0.002 | 0.01401 | 0.66941 | 0.59741 |
| 61 | chr1 | 2334442 | 2335149 | 15 | RER1 | 0.019 | 0.016 | 0.023 | 0.088 | 0.000 | 0.01514 | 0.02024 | 0.00677 |
| 62 | chr8 | 139600458 | 139601543 | 20 | COL22A1 | 0.031 | 0.043 | 0.011 | 0.078 | 0.000 | 0.01532 | 0.28495 | 0.48626 |
| 63 | chr1 | 34404023 | 34404123 | 3 | CSMD2 | 0.073 | 0.104 | 0.025 | 0.042 | 0.000 | 0.01556 | 0.06834 | 0.05288 |
| 64 | chr6 | 26216780 | 26216880 | 3 | HIST1H2BG | 0.040 | 0.066 | 0.000 | 0.063 | 0.000 | 0.01575 | 0.79954 | 0.58401 |
| 65 | chr19 | 52381612 | 52381762 | 4 | ZNF577 | 0.032 | 0.053 | 0.000 | 0.063 | 0.000 | 0.01627 | 0.93639 | 0.94029 |
| 66 | chr11 | 65266553 | 65267598 | 13 | SCYL1 | 0.030 | 0.045 | 0.008 | 0.048 | 0.003 | 0.01646 | 0.43210 | 0.34042 |
| 67 | chr22 | 23029498 | 23029739 | 5 | IGLV3-25 | 0.085 | 0.108 | 0.050 | 0.113 | 0.043 | 0.01712 | 0.97583 | 0.80122 |
| 68 | chr9 | 78686580 | 78686830 | 6 | PC5K5 | 0.035 | 0.052 | 0.008 | 0.073 | 0.000 | 0.01813 | 0.77106 | 0.87235 |
| 69 | chr14 | 106048956 | 106056101 | 25 | IGHA2 | 0.071 | 0.071 | 0.072 | 0.180 | 0.007 | 0.01828 | 0.00255 | 0.02269 |
| 70 | chr14 | 69258239 | 69259639 | 29 | ZFP36L1 | 0.088 | 0.103 | 0.065 | 0.159 | 0.013 | 0.01945 | 0.03212 | 0.00000 |
| 71 | chr5 | 75913717 | 75914417 | 15 | F2RL2 | 0.030 | 0.044 | 0.010 | 0.108 | 0.000 | 0.01980 | 0.01754 | 0.55332 |
| 72 | chr14 | 106926181 | 106926381 | 5 | IGHV3-43 | 0.038 | 0.056 | 0.010 | 0.038 | 0.000 | 0.01981 | 0.22178 | 0.96725 |
| 73 | chr6 | 27782719 | 27782919 | 5 | HIST1H2BM | 0.032 | 0.052 | 0.000 | 0.000 | 0.000 | 0.02014 | 0.01525 | 0.81176 |
| 74 | chr2 | 100758484 | 100758634 | 4 | AFF3 | 0.037 | 0.025 | 0.056 | 0.078 | 0.033 | 0.02064 | 0.69126 | 0.04169 |
| 75 | chr8 | 136569670 | 137528538 | 22 | KHDRBS3 | 0.029 | 0.041 | 0.011 | 0.065 | 0.000 | 0.02090 | 0.60391 | 0.32890 |
| 76 | chr6 | 392761 | 395016 | 15 | IRF4 | 0.035 | 0.031 | 0.042 | 0.021 | 0.000 | 0.02146 | 0.00420 | 0.95404 |
| 77 | chr8 | 3141725 | 4495082 | 9 | CSMD1 | 0.034 | 0.051 | 0.008 | 0.076 | 0.000 | 0.02188 | 0.57834 | 0.96296 |
| 78 | chr14 | 106330651 | 106331101 | 10 | IGHJ3 | 0.057 | 0.075 | 0.030 | 0.150 | 0.009 | 0.02210 | 0.00851 | 0.25752 |
| 79 | chr16 | 300093723 | 300093923 | 5 | PPP4C | 0.034 | 0.023 | 0.050 | 0.050 | 0.000 | 0.02254 | 0.59983 | 0.95843 |
| 80 | chr12 | 92537876 | 92539341 | 28 | BTG1 | 0.058 | 0.057 | 0.059 | 0.074 | 0.012 | 0.02452 | 0.27041 | 0.12731 |
| 81 | chr17 | 53366797 | 53366997 | 5 | DHX33 | 0.022 | 0.010 | 0.040 | 0.025 | 0.000 | 0.02494 | 0.30467 | 0.19851 |
| 82 | chr22 | 22697728 | 22698078 | 8 | IGLV9-49 | 0.041 | 0.035 | 0.050 | 0.047 | 0.000 | 0.02532 | 0.32106 | 0.47874 |
| 83 | chr22 | 232256363 | 23256463 | 3 | IGLJ5 | 0.059 | 0.082 | 0.025 | 0.042 | 0.000 | 0.02682 | 0.15950 | 0.08878 |
| 84 | chr5 | 176522450 | 176522600 | 4 | FGFR4 | 0.037 | 0.025 | 0.056 | 0.063 | 0.000 | 0.02722 | 0.79786 | 0.74613 |
| 85 | chr13 | 113516230 | 113516430 | 5 | ATP11A | 0.050 | 0.069 | 0.020 | 0.113 | 0.000 | 0.02729 | 0.27017 | 0.10654 |
| 86 | chr14 | 106331551 | 106331651 | 3 | IGHJ1 | 0.046 | 0.033 | 0.067 | 0.104 | 0.029 | 0.02734 | 0.59010 | 0.16336 |

| No. | Chr | Start | End | Count | Gene | | | | | | | | |
|---|---|---|---|---|---|---|---|---|---|---|---|---|---|
| 87 | chr2 | 117951920 | 117952020 | 3 | DDX18 | 0.033 | 0.055 | 0.000 | 0.063 | 0.000 | 0.02815 | 0.98381 | 0.97542 |
| 88 | chr14 | 107210956 | 107211156 | 5 | IGHV3-73 | 0.046 | 0.033 | 0.065 | 0.113 | 0.000 | 0.02872 | 0.30080 | 0.42892 |
| 89 | chr12 | 6439714 | 6439914 | 5 | TNFRSF1A | 0.038 | 0.056 | 0.010 | 0.050 | 0.000 | 0.02933 | 0.46779 | 0.82988 |
| 90 | chr2 | 136872526 | 136875621 | 28 | CXCR4 | 0.105 | 0.101 | 0.113 | 0.100 | 0.025 | 0.03071 | 0.00337 | 0.00000 |
| 91 | chr5 | 165548199 | 165548649 | 10 | BCHE | 0.012 | 0.008 | 0.018 | 0.081 | 0.000 | 0.03118 | 0.04749 | 0.00098 |
| 92 | chr4 | 188924115 | 188924865 | 16 | ZFP42 | 0.033 | 0.046 | 0.014 | 0.066 | 0.000 | 0.03190 | 0.74698 | 0.62135 |
| 93 | chr20 | 25003527 | 25003727 | 5 | ACSS1 | 0.032 | 0.049 | 0.005 | 0.138 | 0.000 | 0.03215 | 0.03660 | 0.87436 |
| 94 | chr14 | 106994301 | 106994899 | 11 | IGHV3-48 | 0.041 | 0.036 | 0.048 | 0.125 | 0.043 | 0.03245 | 0.00471 | 0.00001 |
| 95 | chr16 | 3779107 | 3900912 | 82 | CREBBP | 0.035 | 0.043 | 0.022 | 0.070 | 0.001 | 0.03490 | 0.47515 | 0.61294 |
| 96 | chr2 | 89544332 | 89544880 | 11 | IGKV2-30 | 0.029 | 0.042 | 0.009 | 0.091 | 0.000 | 0.03816 | 0.14785 | 0.41409 |
| 97 | chr5 | 112176757 | 112176957 | 5 | APC | 0.028 | 0.046 | 0.000 | 0.088 | 0.000 | 0.03821 | 0.23210 | 0.50694 |
| 98 | chr3 | 185146279 | 185198274 | 20 | MAP3K13 | 0.022 | 0.033 | 0.006 | 0.103 | 0.000 | 0.03855 | 0.00439 | 0.01617 |
| 99 | chr11 | 129739779 | 129740879 | 7 | NFRKB | 0.037 | 0.030 | 0.046 | 0.054 | 0.000 | 0.03877 | 0.49619 | 0.72943 |
| 100 | chr12 | 86198699 | 86199599 | 19 | RASSF9 | 0.035 | 0.047 | 0.017 | 0.066 | 0.000 | 0.04167 | 0.79797 | 0.81991 |
| 101 | chr12 | 15813488 | 15813638 | 4 | EPS8 | 0.035 | 0.025 | 0.050 | 0.031 | 0.000 | 0.04189 | 0.24118 | 0.93977 |
| 102 | chr2 | 63826278 | 63826428 | 4 | MDH1 | 0.017 | 0.008 | 0.031 | 0.203 | 0.000 | 0.04203 | 0.00443 | 0.12932 |
| 103 | chr14 | 107083566 | 107083891 | 7 | IGHV4-59 | 0.040 | 0.054 | 0.018 | 0.179 | 0.043 | 0.04296 | 0.00035 | 0.00040 |
| 104 | chr22 | 22735418 | 22735843 | 6 | IGLV1-44 | 0.059 | 0.079 | 0.029 | 0.073 | 0.000 | 0.04311 | 0.62445 | 0.18113 |
| 105 | chr12 | 18891268 | 18891518 | 6 | CAPZA3 | 0.012 | 0.005 | 0.021 | 0.125 | 0.000 | 0.04368 | 0.00589 | 0.00868 |
| 106 | chr14 | 106174971 | 106177526 | 44 | IGHA1 | 0.117 | 0.117 | 0.116 | 0.125 | 0.027 | 0.04581 | 0.05495 | 0.00009 |
| 107 | chr13 | 58207132 | 58209682 | 40 | PCDH7 | 0.038 | 0.047 | 0.024 | 0.092 | 0.000 | 0.04705 | 0.03043 | 0.23893 |
| 108 | chr6 | 26156650 | 26157350 | 15 | HIST1H1E | 0.064 | 0.077 | 0.045 | 0.008 | 0.000 | 0.04776 | 0.00000 | 0.00658 |
| 109 | chr8 | 75898191 | 75898391 | 5 | CRISPLD1 | 0.012 | 0.007 | 0.020 | 0.050 | 0.000 | 0.04779 | 0.61717 | 0.01894 |
| 110 | chr9 | 37024920 | 37033770 | 38 | PAX5 | 0.059 | 0.060 | 0.059 | 0.107 | 0.015 | 0.04840 | 0.84733 | 0.06185 |
| 111 | chr17 | 18001530 | 18001680 | 4 | DRG2 | 0.015 | 0.008 | 0.025 | 0.031 | 0.000 | 0.04924 | 0.70570 | 0.06008 |
| 112 | chr10 | 91092212 | 91092412 | 5 | IFIT3 | 0.026 | 0.016 | 0.040 | 0.050 | 0.000 | 0.05027 | 0.89626 | 0.41400 |
| 113 | chr2 | 56149511 | 56150111 | 13 | EFEMP1 | 0.030 | 0.029 | 0.031 | 0.115 | 0.000 | 0.05115 | 0.00217 | 0.49133 |
| 114 | chr6 | 26032015 | 26032215 | 5 | HIST1H3B | 0.030 | 0.046 | 0.005 | 0.013 | 0.000 | 0.05360 | 0.05680 | 0.72269 |
| 115 | chrX | 1584325 | 1655990 | 29 | P2RY8 | 0.031 | 0.041 | 0.016 | 0.093 | 0.001 | 0.05546 | 0.01173 | 0.29622 |
| 116 | chr4 | 187509885 | 187557980 | 16 | FAT1 | 0.028 | 0.039 | 0.013 | 0.094 | 0.000 | 0.05661 | 0.05492 | 0.36536 |
| 117 | chr5 | 11110991 | 11411801 | 24 | CTNND2 | 0.031 | 0.040 | 0.016 | 0.060 | 0.000 | 0.05690 | 0.95068 | 0.19315 |
| 118 | chr14 | 106118676 | 106114376 | 65 | IGHG2 | 0.213 | 0.210 | 0.217 | 0.147 | 0.049 | 0.05698 | 0.00000 | 0.00000 |
| 119 | chr1 | 4472439 | 4476599 | 10 | AJAP1 | 0.039 | 0.026 | 0.035 | 0.031 | 0.000 | 0.05889 | 0.10905 | 0.59078 |
| 120 | chr1 | 110561142 | 110561742 | 13 | AHCYL1 | 0.019 | 0.018 | 0.021 | 0.058 | 0.000 | 0.05908 | 0.58438 | 0.01312 |
| 121 | chr14 | 106725296 | 106726174 | 14 | IGHV3-23 | 0.099 | 0.111 | 0.080 | 0.027 | 0.000 | 0.05952 | 0.00000 | 0.00001 |
| 122 | chr1 | 111715728 | 111715878 | 4 | CEPT1 | 0.022 | 0.016 | 0.031 | 0.047 | 0.000 | 0.06085 | 0.91905 | 0.26127 |
| 123 | chr11 | 118967324 | 118968024 | 15 | DPAGT1 | 0.032 | 0.044 | 0.028 | 0.046 | 0.000 | 0.06151 | 0.19789 | 0.69126 |
| 124 | chr2 | 55237199 | 55237599 | 9 | RTN4 | 0.047 | 0.060 | 0.008 | 0.063 | 0.000 | 0.06231 | 0.41805 | 0.17702 |
| 125 | chr11 | 111781037 | 111781637 | 13 | CRYAB | 0.025 | 0.037 | 0.021 | 0.082 | 0.000 | 0.06377 | 0.11838 | 0.14383 |
| 126 | chr14 | 106573316 | 106574003 | 13 | IGHV3-11 | 0.041 | 0.054 | 0.025 | 0.082 | 0.007 | 0.06792 | 0.84332 | 0.93964 |
| 127 | chr18 | 48231685 | 48232085 | 9 | MAPK4 | 0.022 | 0.020 | 0.013 | 0.021 | 0.000 | 0.07104 | 0.07945 | 0.10112 |
| 128 | chr2 | 62934010 | 63217980 | 14 | EHBP1 | 0.030 | 0.042 | 0.015 | 0.080 | 0.000 | 0.07190 | 0.51773 | 0.62080 |
| 129 | chr2 | 226677078 | 226677289 | 5 | IGLV1-51 | 0.046 | 0.066 | 0.016 | 0.113 | 0.000 | 0.07234 | 0.37625 | 0.20872 |
| 130 | chr7 | 119915407 | 119915757 | 8 | KCND2 | 0.038 | 0.053 | 0.016 | 0.039 | 0.000 | 0.07723 | 0.12619 | 0.48614 |
| 131 | chr22 | 23154348 | 23154798 | 8 | IGLV3-10 | 0.024 | 0.020 | 0.028 | 0.102 | 0.000 | 0.07866 | 0.03037 | 0.15798 |

| 132 | chr6 | 26045745 | 26046045 | 7 | HIST1H3C | 0.030 | 0.026 | 0.036 | 0.045 | 0.019 | 0.08101 | 0.47189 | 0.03046 |
|---|---|---|---|---|---|---|---|---|---|---|---|---|---|
| 133 | chr10 | 131640290 | 131640490 | 5 | EBF3 | 0.040 | 0.036 | 0.045 | 0.100 | 0.000 | 0.08357 | 0.26942 | 0.76490 |
| 134 | chr1 | 109822182 | 109822782 | 13 | PSRC1 | 0.027 | 0.038 | 0.012 | 0.072 | 0.000 | 0.08367 | 0.51165 | 0.24502 |
| 135 | chr17 | 18022120 | 18022770 | 14 | MYO15A | 0.039 | 0.036 | 0.043 | 0.085 | 0.000 | 0.08686 | 0.51095 | 0.37846 |
| 136 | chr16 | 85933004 | 85954924 | 56 | IRF8 | 0.037 | 0.047 | 0.024 | 0.065 | 0.012 | 0.08712 | 0.41154 | 0.04982 |
| 137 | chr2 | 89986777 | 89987085 | 7 | IGKV2D-29 | 0.024 | 0.021 | 0.029 | 0.045 | 0.000 | 0.09053 | 0.66530 | 0.22260 |
| 138 | chr2 | 90249152 | 90249397 | 5 | IGK1D-43 | 0.040 | 0.033 | 0.050 | 0.063 | 0.009 | 0.09076 | 0.87053 | 0.96927 |
| 139 | chr2 | 242793233 | 242801088 | 24 | PDCD1 | 0.047 | 0.048 | 0.046 | 0.083 | 0.000 | 0.09248 | 0.64737 | 0.01000 |
| 140 | chr6 | 27100080 | 27100180 | 3 | HIST1H2BJ | 0.033 | 0.027 | 0.042 | 0.000 | 0.029 | 0.09735 | 0.05014 | 0.09524 |
| 141 | chr7 | 110545277 | 110698122 | 8 | IMMP2L | 0.004 | 0.002 | 0.006 | 0.063 | 0.000 | 0.10148 | 0.15804 | 0.00010 |
| 142 | chr1 | 111441723 | 111442173 | 10 | CD53 | 0.027 | 0.038 | 0.010 | 0.100 | 0.000 | 0.10715 | 0.04221 | 0.30553 |
| 143 | chrX | 70612662 | 70612762 | 3 | TAF1 | 0.007 | 0.000 | 0.017 | 0.063 | 0.000 | 0.10731 | 0.45417 | 0.02634 |
| 144 | chr21 | 18981234 | 18981484 | 6 | BTG3 | 0.020 | 0.033 | 0.000 | 0.073 | 0.000 | 0.10744 | 0.29340 | 0.11987 |
| 145 | chr14 | 107113406 | 107114196 | 10 | IGHV3-64 | 0.015 | 0.013 | 0.018 | 0.050 | 0.000 | 0.10843 | 0.80649 | 0.00490 |
| 146 | chr22 | 22380473 | 22385883 | 18 | IGLV4-69 | 0.044 | 0.054 | 0.029 | 0.073 | 0.000 | 0.10860 | 0.97247 | 0.18279 |
| 147 | chr9 | 5510590 | 5570130 | 34 | PDCD1LG2 | 0.026 | 0.028 | 0.024 | 0.057 | 0.000 | 0.11075 | 0.98596 | 0.05983 |
| 148 | chr1 | 27059147 | 27106912 | 29 | ARID1A | 0.035 | 0.043 | 0.023 | 0.073 | 0.006 | 0.11182 | 0.58280 | 0.43378 |
| 149 | chr13 | 32907207 | 32912827 | 17 | BRCA2 | 0.013 | 0.013 | 0.013 | 0.088 | 0.000 | 0.11539 | 0.00502 | 0.00005 |
| 150 | chr18 | 48703170 | 48703920 | 16 | MEX3C | 0.022 | 0.023 | 0.022 | 0.059 | 0.000 | 0.11749 | 0.74407 | 0.02655 |
| 151 | chr1 | 203274698 | 203276558 | 33 | BTG2 | 0.131 | 0.129 | 0.133 | 0.133 | 0.012 | 0.11791 | 0.01136 | 0.00000 |
| 152 | chr8 | 128492948 | 128493298 | 8 | POU5F1B | 0.022 | 0.035 | 0.003 | 0.047 | 0.000 | 0.11971 | 0.87638 | 0.11243 |
| 153 | chr6 | 27834969 | 27835069 | 3 | HIST1H1B | 0.043 | 0.038 | 0.050 | 0.042 | 0.000 | 0.12081 | 0.31080 | 0.40430 |
| 154 | chr22 | 23010978 | 23011307 | 7 | IGLV3-27 | 0.045 | 0.059 | 0.025 | 0.045 | 0.000 | 0.12123 | 0.15843 | 0.35845 |
| 155 | chr1 | 117078643 | 117087128 | 10 | CD58 | 0.022 | 0.021 | 0.023 | 0.025 | 0.000 | 0.12266 | 0.14627 | 0.06157 |
| 156 | chr14 | 106380361 | 106381326 | 17 | IGHD3-3 | 0.040 | 0.040 | 0.040 | 0.022 | 0.010 | 0.12443 | 0.00226 | 0.54240 |
| 157 | chr12 | 49415992 | 49447447 | 47 | KMT2D | 0.029 | 0.031 | 0.026 | 0.097 | 0.000 | 0.12454 | 0.00102 | 0.09879 |
| 158 | chr22 | 22782038 | 22782288 | 6 | IGLV5-37 | 0.051 | 0.066 | 0.029 | 0.052 | 0.000 | 0.12900 | 0.22779 | 0.08945 |
| 159 | chr8 | 18729446 | 18729896 | 10 | PSD3 | 0.036 | 0.048 | 0.018 | 0.100 | 0.000 | 0.12911 | 0.49227 | 0.67922 |
| 160 | chr14 | 106552366 | 106552466 | 3 | IGHV3-9 | 0.020 | 0.011 | 0.033 | 0.063 | 0.000 | 0.12919 | 0.69275 | 0.24178 |
| 161 | chrX | 35820577 | 35821227 | 14 | MAGEB16 | 0.021 | 0.032 | 0.005 | 0.080 | 0.000 | 0.13076 | 0.08392 | 0.03514 |
| 162 | chr16 | 81946176 | 81962221 | 13 | PLCG2 | 0.027 | 0.028 | 0.027 | 0.058 | 0.000 | 0.13686 | 0.98920 | 0.29436 |
| 163 | chr22 | 22712078 | 22712594 | 11 | IGLV1-47 | 0.050 | 0.063 | 0.032 | 0.108 | 0.000 | 0.13854 | 0.36497 | 0.04398 |
| 164 | chr3 | 16419205 | 16419455 | 6 | RFTN1 | 0.050 | 0.046 | 0.054 | 0.063 | 0.000 | 0.14045 | 0.43890 | 0.10024 |
| 165 | chr11 | 111613197 | 111613397 | 5 | PPP2R1B | 0.026 | 0.039 | 0.005 | 0.000 | 0.000 | 0.14058 | 0.02490 | 0.46424 |
| 166 | chr14 | 106331151 | 106331501 | 8 | IGHJ2 | 0.048 | 0.047 | 0.050 | 0.102 | 0.027 | 0.14335 | 0.33135 | 0.15651 |
| 167 | chr1 | 226923692 | 226925192 | 31 | ITPKB | 0.044 | 0.053 | 0.031 | 0.139 | 0.000 | 0.14412 | 0.00007 | 0.03739 |
| 168 | chr6 | 27100940 | 27101260 | 5 | HIST1H2AG | 0.024 | 0.020 | 0.030 | 0.038 | 0.000 | 0.14525 | 0.54138 | 0.28737 |
| 169 | chr10 | 91358987 | 91359287 | 7 | PANK1 | 0.021 | 0.019 | 0.025 | 0.107 | 0.000 | 0.15224 | 0.01412 | 0.10864 |
| 170 | chr14 | 32615406 | 32615606 | 5 | ARHGAP5 | 0.020 | 0.033 | 0.000 | 0.100 | 0.000 | 0.15384 | 0.16273 | 0.16433 |
| 171 | chrX | 119509281 | 119509481 | 5 | ATP1B4 | 0.016 | 0.013 | 0.020 | 0.088 | 0.000 | 0.15508 | 0.23890 | 0.07712 |
| 172 | chr18 | 77794426 | 77795126 | 15 | RBFA | 0.014 | 0.014 | 0.013 | 0.075 | 0.000 | 0.15602 | 0.08296 | 0.00029 |
| 173 | chr10 | 89624273 | 89720888 | 32 | PTEN | 0.015 | 0.016 | 0.013 | 0.023 | 0.000 | 0.15663 | 0.04633 | 0.00000 |
| 174 | chr14 | 64330253 | 64330453 | 5 | SYNE2 | 0.006 | 0.003 | 0.010 | 0.025 | 0.000 | 0.15837 | 0.74245 | 0.00357 |
| 175 | chr9 | 24545400 | 24905695 | 17 | IZUMO3 | 0.030 | 0.039 | 0.016 | 0.037 | 0.000 | 0.15955 | 0.10765 | 0.43759 |

| | | | | | | | | | | | | |
|---|---|---|---|---|---|---|---|---|---|---|---|---|
| 176 | chr5 | 54964699 | 54964899 | 5 | SLC38A9 | 0.002 | 0.000 | 0.005 | 0.013 | 0.000 | 0.16320 | 0.46997 | 0.00144 |
| 177 | chr8 | 101730377 | 101730427 | 2 | PABPC1 | 0.015 | 0.008 | 0.025 | 0.000 | 0.000 | 0.16445 | 0.26379 | 0.18377 |
| 178 | chr8 | 131373025 | 131373425 | 9 | ASAP1 | 0.030 | 0.040 | 0.014 | 0.028 | 0.000 | 0.16655 | 0.08650 | 0.59884 |
| 179 | chr22 | 23101393 | 23101730 | 6 | IGLV2-14 | 0.048 | 0.044 | 0.054 | 0.073 | 0.022 | 0.16893 | 0.83695 | 0.56495 |
| 180 | chr1 | 109649127 | 109649277 | 4 | C1orf194 | 0.047 | 0.045 | 0.050 | 0.078 | 0.022 | 0.17014 | 0.88867 | 0.40591 |
| 181 | chr11 | 65623423 | 65623473 | 2 | CFL1 | 0.025 | 0.041 | 0.000 | 0.031 | 0.000 | 0.17060 | 0.58174 | 0.54924 |
| 182 | chr22 | 22707428 | 22707793 | 7 | IGLV5-48 | 0.035 | 0.047 | 0.018 | 0.071 | 0.000 | 0.17227 | 0.95304 | 0.82874 |
| 183 | chr14 | 106331701 | 106331801 | 3 | IGHD7-27 | 0.026 | 0.022 | 0.033 | 0.125 | 0.000 | 0.17412 | 0.05590 | 0.56584 |
| 184 | chr14 | 96179593 | 96180293 | 15 | TCL1A | 0.050 | 0.050 | 0.050 | 0.071 | 0.000 | 0.17445 | 0.59106 | 0.01278 |
| 185 | chr22 | 23063308 | 23063658 | 8 | IGLV3-19 | 0.031 | 0.029 | 0.034 | 0.039 | 0.000 | 0.17496 | 0.31060 | 0.64225 |
| 186 | chr17 | 7576950 | 7579410 | 24 | TP53 | 0.040 | 0.051 | 0.023 | 0.107 | 0.000 | 0.17822 | 0.03641 | 0.51953 |
| 187 | chr2 | 148680517 | 148680667 | 4 | ACVR2A | 0.025 | 0.037 | 0.006 | 0.031 | 0.000 | 0.18073 | 0.41320 | 0.38140 |
| 188 | chr19 | 10334564 | 10341984 | 35 | S1PR2 | 0.064 | 0.077 | 0.044 | 0.104 | 0.002 | 0.18105 | 0.40386 | 0.00014 |
| 189 | chr6 | 108040229 | 108042204 | 27 | SCML4 | 0.025 | 0.026 | 0.023 | 0.060 | 0.005 | 0.18315 | 0.54097 | 0.01195 |
| 190 | chr6 | 27277285 | 27277485 | 5 | POM121L2 | 0.042 | 0.039 | 0.045 | 0.050 | 0.000 | 0.18414 | 0.38135 | 0.41604 |
| 191 | chr3 | 186714605 | 186784290 | 33 | ST6GAL1 | 0.084 | 0.091 | 0.072 | 0.087 | 0.018 | 0.18556 | 0.01425 | 0.00007 |
| 192 | chr19 | 12902575 | 12902825 | 6 | JUNB | 0.053 | 0.052 | 0.054 | 0.010 | 0.000 | 0.18604 | 0.00259 | 0.04452 |
| 193 | chr14 | 107199021 | 107199172 | 4 | IGHV3-72 | 0.045 | 0.041 | 0.050 | 0.000 | 0.000 | 0.18636 | 0.00860 | 0.27305 |
| 194 | chr11 | 102188382 | 102188932 | 12 | BIRC3 | 0.104 | 0.123 | 0.075 | 0.104 | 0.043 | 0.18760 | 0.23061 | 0.02703 |
| 195 | chr1 | 185833556 | 186159096 | 32 | HMCN1 | 0.021 | 0.023 | 0.018 | 0.074 | 0.000 | 0.18799 | 0.04332 | 0.00092 |
| 196 | chr12 | 18534683 | 18801013 | 30 | PIK3C2G | 0.017 | 0.020 | 0.013 | 0.054 | 0.000 | 0.18947 | 0.52931 | 0.00001 |
| 197 | chrX | 100610985 | 100611285 | 7 | BTK | 0.021 | 0.021 | 0.021 | 0.116 | 0.000 | 0.18957 | 0.01363 | 0.10957 |
| 198 | chr18 | 64172117 | 64239317 | 19 | CDH19 | 0.023 | 0.032 | 0.009 | 0.072 | 0.002 | 0.19120 | 0.37384 | 0.02195 |
| 199 | chr2 | 1652011 | 1652811 | 17 | PXDN | 0.045 | 0.054 | 0.031 | 0.092 | 0.000 | 0.19342 | 0.57240 | 0.03398 |
| 200 | chr11 | 111904097 | 111904247 | 4 | DLAT | 0.037 | 0.049 | 0.019 | 0.016 | 0.000 | 0.19688 | 0.06546 | 0.70963 |
| 201 | chr22 | 22556228 | 22556628 | 9 | IGLV11-55 | 0.039 | 0.038 | 0.039 | 0.111 | 0.000 | 0.19910 | 0.04960 | 0.53925 |
| 202 | chr2 | 103148734 | 103148934 | 5 | SLC9A4 | 0.024 | 0.036 | 0.005 | 0.063 | 0.000 | 0.20039 | 0.78808 | 0.29891 |
| 203 | chr2 | 48027959 | 48028159 | 5 | MSH6 | 0.012 | 0.010 | 0.015 | 0.000 | 0.000 | 0.20189 | 0.09865 | 0.01894 |
| 204 | chr4 | 134727699 | 134727899 | 5 | PABPC4L | 0.012 | 0.010 | 0.015 | 0.150 | 0.000 | 0.20189 | 0.02007 | 0.01894 |
| 205 | chr11 | 134027790 | 134027940 | 4 | NCAPD3 | 0.047 | 0.061 | 0.025 | 0.078 | 0.000 | 0.20429 | 0.99130 | 0.21830 |
| 206 | chr2 | 77746603 | 77746953 | 8 | LRRTM4 | 0.026 | 0.037 | 0.009 | 0.047 | 0.000 | 0.20711 | 0.60835 | 0.35208 |
| 207 | chr1 | 160319284 | 160319484 | 5 | NCSTN | 0.044 | 0.039 | 0.050 | 0.025 | 0.000 | 0.21582 | 0.05416 | 0.28073 |
| 208 | chr18 | 65179857 | 65181807 | 40 | DSEL | 0.021 | 0.029 | 0.009 | 0.073 | 0.000 | 0.21609 | 0.19591 | 0.00018 |
| 209 | chr15 | 45003679 | 45008564 | 12 | B2M | 0.035 | 0.046 | 0.017 | 0.031 | 0.007 | 0.21616 | 0.04427 | 0.31773 |
| 210 | chr1 | 29069532 | 29070182 | 14 | YTHDF2 | 0.043 | 0.052 | 0.030 | 0.040 | 0.006 | 0.21620 | 0.03795 | 0.84925 |
| 211 | chr4 | 80327793 | 80328143 | 8 | GK2 | 0.030 | 0.041 | 0.013 | 0.117 | 0.000 | 0.21872 | 0.01766 | 0.70075 |
| 212 | chr5 | 158527643 | 158527993 | 8 | EBF1 | 0.052 | 0.064 | 0.034 | 0.055 | 0.000 | 0.22009 | 0.11870 | 0.13982 |
| 213 | chr1 | 3747621 | 3747771 | 4 | CEP104 | 0.025 | 0.037 | 0.006 | 0.109 | 0.000 | 0.22034 | 0.26105 | 0.39687 |
| 214 | chr2 | 48059884 | 48066174 | 9 | FBXO11 | 0.014 | 0.015 | 0.014 | 0.063 | 0.000 | 0.22199 | 0.44292 | 0.00401 |
| 215 | chrX | 33146107 | 33146457 | 8 | DMD | 0.059 | 0.059 | 0.059 | 0.359 | 0.082 | 0.22404 | 0.00000 | 0.00004 |
| 216 | chr6 | 26124545 | 26124865 | 6 | HIST1H2AC | 0.051 | 0.063 | 0.033 | 0.010 | 0.000 | 0.22855 | 0.00394 | 0.11588 |
| 217 | chr14 | 106791091 | 106791141 | 2 | IGHV3-30 | 0.045 | 0.041 | 0.050 | 0.063 | 0.000 | 0.24046 | 0.72117 | 0.43844 |
| 218 | chr3 | 183209759 | 183273414 | 23 | KLHL6 | 0.036 | 0.036 | 0.036 | 0.052 | 0.006 | 0.24437 | 0.12177 | 0.41139 |
| 219 | chr17 | 79478954 | 79479004 | 2 | ACTG1 | 0.005 | 0.000 | 0.013 | 0.125 | 0.043 | 0.24604 | 0.05674 | 0.01689 |

| 220 | chr22 | 47570210 | 47570410 | 5 | TBC1D22A | 0.030 | 0.043 | 0.010 | 0.175 | 0.000 | 0.24818 | 0.00334 | 0.70762 |
|-----|-------|----------|----------|----|----------|-------|-------|-------|-------|-------|---------|---------|---------|
| 221 | chr6 | 27799169 | 27799369 | 5 | HIST1H4K | 0.022 | 0.033 | 0.005 | 0.038 | 0.000 | 0.24870 | 0.54640 | 0.19851 |
| 222 | chr2 | 65258146 | 65258346 | 5 | SLC1A4 | 0.018 | 0.030 | 0.000 | 0.050 | 0.000 | 0.25016 | 0.78384 | 0.08170 |
| 223 | chr14 | 106586201 | 106586301 | 3 | IGHV3-13 | 0.033 | 0.027 | 0.042 | 0.021 | 0.000 | 0.25073 | 0.17545 | 0.97542 |
| 224 | chr6 | 26158530 | 26158790 | 4 | HIST1H2BD | 0.030 | 0.041 | 0.013 | 0.016 | 0.000 | 0.25147 | 0.13295 | 0.69509 |
| 225 | chr14 | 106691756 | 106691856 | 3 | IGHV3-21 | 0.053 | 0.066 | 0.033 | 0.042 | 0.000 | 0.25208 | 0.23957 | 0.18828 |
| 226 | chr10 | 90579967 | 90580317 | 8 | LIPM | 0.035 | 0.035 | 0.034 | 0.047 | 0.000 | 0.25854 | 0.32941 | 0.85606 |
| 227 | chr7 | 82387831 | 82784641 | 19 | PCLO | 0.035 | 0.044 | 0.022 | 0.049 | 0.000 | 0.25896 | 0.17138 | 0.85294 |
| 228 | chr22 | 23090123 | 23090338 | 4 | IGLV3-16 | 0.030 | 0.041 | 0.013 | 0.063 | 0.065 | 0.26082 | 0.88005 | 0.00186 |
| 229 | chr2 | 89475782 | 89476114 | 7 | IGKV2-24 | 0.044 | 0.042 | 0.046 | 0.125 | 0.000 | 0.26354 | 0.03650 | 0.25182 |
| 230 | chr2 | 90121892 | 90122155 | 6 | IGKV1D-17 | 0.030 | 0.041 | 0.013 | 0.083 | 0.000 | 0.26708 | 0.50393 | 0.47148 |
| 231 | chr14 | 107034666 | 107035056 | 7 | IGHV5-51 | 0.038 | 0.049 | 0.021 | 0.071 | 0.000 | 0.26981 | 0.83901 | 0.54622 |
| 232 | chr6 | 26217215 | 26217415 | 5 | HIST1H2AE | 0.024 | 0.023 | 0.025 | 0.038 | 0.000 | 0.26983 | 0.53539 | 0.29891 |
| 233 | chr14 | 84420587 | 84420787 | 5 | FLRT2 | 0.000 | 0.000 | 0.000 | 0.025 | 0.000 | 0.27098 | 0.90753 | 0.00089 |
| 234 | chr4 | 40198811 | 40201559 | 49 | RHOH | 0.062 | 0.068 | 0.053 | 0.028 | 0.015 | 0.27123 | 0.00000 | 0.12156 |
| 235 | chr14 | 106539176 | 106539276 | 3 | IGHV1-8 | 0.040 | 0.038 | 0.042 | 0.063 | 0.000 | 0.27246 | 0.79783 | 0.70059 |
| 236 | chr5 | 83258968 | 83259168 | 5 | EDIL3 | 0.022 | 0.033 | 0.005 | 0.063 | 0.000 | 0.27662 | 0.67082 | 0.19851 |
| 237 | chrX | 70347817 | 70348017 | 5 | MED12 | 0.022 | 0.033 | 0.005 | 0.075 | 0.000 | 0.27662 | 0.38460 | 0.19851 |
| 238 | chr18 | 48512955 | 48513305 | 8 | ELAC1 | 0.026 | 0.027 | 0.025 | 0.102 | 0.000 | 0.27685 | 0.05340 | 0.35208 |
| 239 | chrX | 12993265 | 12994487 | 23 | TMSB4X | 0.098 | 0.108 | 0.083 | 0.177 | 0.057 | 0.27705 | 0.03023 | 0.53439 |
| 240 | chr19 | 6586162 | 6591037 | 17 | CD70 | 0.052 | 0.064 | 0.035 | 0.121 | 0.000 | 0.27742 | 0.02768 | 0.05558 |
| 241 | chr9 | 13222186 | 13222386 | 5 | MPDZ | 0.018 | 0.016 | 0.020 | 0.050 | 0.000 | 0.27845 | 0.92556 | 0.10149 |
| 242 | chr19 | 8028409 | 8028559 | 4 | ELAVL1 | 0.037 | 0.049 | 0.019 | 0.094 | 0.000 | 0.28231 | 0.39328 | 0.68881 |
| 243 | chr17 | 63010241 | 63052644 | 28 | GNA13 | 0.033 | 0.035 | 0.029 | 0.051 | 0.005 | 0.29192 | 0.20921 | 0.55174 |
| 244 | chr6 | 75965847 | 75969257 | 10 | TMEM30A | 0.017 | 0.018 | 0.015 | 0.063 | 0.000 | 0.29877 | 0.61973 | 0.01289 |
| 245 | chr2 | 61118795 | 61149620 | 27 | REL | 0.024 | 0.030 | 0.014 | 0.053 | 0.006 | 0.29909 | 0.79282 | 0.00093 |
| 246 | chr8 | 103663492 | 103664142 | 14 | KLF10 | 0.032 | 0.034 | 0.029 | 0.103 | 0.000 | 0.29943 | 0.04753 | 0.77217 |
| 247 | chr7 | 122634906 | 122635106 | 5 | TAS2R16 | 0.040 | 0.036 | 0.045 | 0.050 | 0.000 | 0.30121 | 0.42497 | 0.50451 |
| 248 | chr7 | 106508491 | 106509141 | 14 | PIK3CG | 0.043 | 0.044 | 0.041 | 0.058 | 0.000 | 0.30584 | 0.28865 | 0.12742 |
| 249 | chr19 | 1376441 | 1376641 | 5 | MUM1 | 0.053 | 0.066 | 0.035 | 0.063 | 0.000 | 0.30591 | 0.40617 | 0.10207 |
| 250 | chr10 | 90074240 | 90074390 | 4 | RNLS | 0.012 | 0.012 | 0.013 | 0.141 | 0.000 | 0.30697 | 0.04146 | 0.05611 |
| 251 | chr17 | 56408575 | 56409585 | 19 | BZRAP1 | 0.107 | 0.116 | 0.095 | 0.122 | 0.050 | 0.31066 | 0.24386 | 0.00835 |
| 252 | chr18 | 48327695 | 48327895 | 5 | MRO | 0.034 | 0.033 | 0.035 | 0.088 | 0.000 | 0.32051 | 0.36874 | 0.94107 |
| 253 | chr2 | 90212017 | 90212247 | 4 | IGKV3D-11 | 0.000 | 0.000 | 0.000 | 0.063 | 0.000 | 0.32488 | 0.18259 | 0.00295 |
| 254 | chr3 | 164730701 | 164730851 | 4 | SI | 0.000 | 0.000 | 0.000 | 0.031 | 0.000 | 0.32488 | 0.89232 | 0.00295 |
| 255 | chr18 | 75683735 | 75684485 | 16 | GALR1 | 0.025 | 0.026 | 0.023 | 0.055 | 0.000 | 0.32688 | 0.88862 | 0.08570 |
| 256 | chr10 | 90699127 | 90699627 | 11 | ACTA2 | 0.022 | 0.030 | 0.009 | 0.074 | 0.000 | 0.32826 | 0.22549 | 0.05225 |
| 257 | chr7 | 146997184 | 146997384 | 5 | CNTNAP2 | 0.020 | 0.030 | 0.005 | 0.063 | 0.000 | 0.33654 | 0.72508 | 0.12531 |
| 258 | chr10 | 90537737 | 90537987 | 6 | LIPN | 0.021 | 0.022 | 0.021 | 0.063 | 0.000 | 0.33950 | 0.63054 | 0.15262 |
| 259 | chr8 | 116616146 | 116616846 | 15 | TRPS1 | 0.033 | 0.042 | 0.020 | 0.088 | 0.000 | 0.34027 | 0.10857 | 0.96046 |
| 260 | chr6 | 14117993 | 14135468 | 27 | CD83 | 0.061 | 0.069 | 0.049 | 0.146 | 0.006 | 0.34145 | 0.00006 | 0.25221 |
| 261 | chr14 | 106610381 | 106610741 | 6 | IGHV3-15 | 0.036 | 0.046 | 0.021 | 0.042 | 0.000 | 0.34253 | 0.25513 | 0.68243 |
| 262 | chr14 | 106962966 | 106963269 | 7 | IGHV1-45 | 0.023 | 0.023 | 0.021 | 0.036 | 0.000 | 0.34439 | 0.45188 | 0.16111 |
| 263 | chr6 | 27833409 | 27833509 | 3 | HIST1H2AL | 0.017 | 0.027 | 0.000 | 0.042 | 0.000 | 0.34503 | 0.82367 | 0.13637 |
| 264 | chr7 | 2963819 | 2987364 | 44 | CARD11 | 0.047 | 0.055 | 0.035 | 0.075 | 0.000 | 0.34677 | 0.68708 | 0.00272 |

EP 4 334 476 B1

| 265 | chr11 | 134118685 | 134118835 | 4 | THYN1 | 0.017 | 0.016 | 0.019 | 0.094 | 0.000 | 0.35301 | 0.26225 | 0.10870 |
|-----|-------|-----------|-----------|-----|----------|-------|-------|-------|-------|-------|---------|---------|---------|
| 266 | chr14 | 107258911 | 107282996 | 17 | IGHV7-81 | 0.031 | 0.040 | 0.019 | 0.088 | 0.026 | 0.35469 | 0.15903 | 0.00002 |
| 267 | chrX | 73962124 | 73963074 | 20 | KIAA2022 | 0.020 | 0.028 | 0.009 | 0.103 | 0.000 | 0.35514 | 0.00284 | 0.00632 |
| 268 | chr3 | 185236909 | 185237109 | 5 | LIPH | 0.022 | 0.033 | 0.005 | 0.038 | 0.000 | 0.35786 | 0.57454 | 0.20093 |
| 269 | chr3 | 64547205 | 64580090 | 11 | ADAMTS9 | 0.028 | 0.030 | 0.025 | 0.091 | 0.000 | 0.35888 | 0.08153 | 0.38328 |
| 270 | chr14 | 106405616 | 106405916 | 7 | IGHV6-1 | 0.028 | 0.037 | 0.014 | 0.098 | 0.000 | 0.36129 | 0.28061 | 0.53891 |
| 271 | chr11 | 117712684 | 117712984 | 7 | FXYD6 | 0.035 | 0.035 | 0.036 | 0.045 | 0.000 | 0.36200 | 0.39501 | 0.93264 |
| 272 | chr8 | 130692150 | 130760995 | 17 | GSDMC | 0.029 | 0.037 | 0.018 | 0.051 | 0.000 | 0.36490 | 0.59248 | 0.38946 |
| 273 | chr22 | 22749603 | 22750309 | 14 | IGLV7-43 | 0.021 | 0.022 | 0.018 | 0.067 | 0.000 | 0.36721 | 0.26604 | 0.01881 |
| 274 | chr22 | 23135153 | 23135508 | 7 | IGLV2-11 | 0.020 | 0.021 | 0.018 | 0.098 | 0.000 | 0.36740 | 0.03964 | 0.07222 |
| 275 | chr6 | 26234655 | 26234955 | 7 | HIST1H1D | 0.042 | 0.044 | 0.039 | 0.018 | 0.000 | 0.36781 | 0.01092 | 0.23508 |
| 276 | chr11 | 112405017 | 112405578 | 12 | C11orf34 | 0.029 | 0.037 | 0.017 | 0.099 | 0.000 | 0.36795 | 0.03866 | 0.51208 |
| 277 | chr1 | 2488007 | 2494707 | 36 | TNFRSF14 | 0.035 | 0.042 | 0.024 | 0.082 | 0.000 | 0.37037 | 0.15033 | 0.73903 |
| 278 | chr18 | 48591760 | 48604805 | 16 | SMAD4 | 0.019 | 0.020 | 0.016 | 0.035 | 0.000 | 0.37088 | 0.36837 | 0.00422 |
| 279 | chr18 | 55274406 | 55274556 | 4 | NARS | 0.015 | 0.025 | 0.000 | 0.047 | 0.000 | 0.37631 | 0.84014 | 0.07298 |
| 280 | chrX | 90026454 | 90026604 | 4 | PABPC5 | 0.015 | 0.025 | 0.000 | 0.031 | 0.000 | 0.37790 | 0.70713 | 0.06008 |
| 281 | chr8 | 623881 | 624081 | 5 | ERICH1 | 0.020 | 0.020 | 0.020 | 0.025 | 0.000 | 0.38591 | 0.34374 | 0.13521 |
| 282 | chr18 | 1477566 | 1477666 | 3 | ADCYAP1 | 0.043 | 0.055 | 0.025 | 0.000 | 0.000 | 0.38723 | 0.02764 | 0.48180 |
| 283 | chr12 | 48190732 | 48190982 | 6 | HDAC7 | 0.043 | 0.041 | 0.046 | 0.021 | 0.000 | 0.38786 | 0.03107 | 0.34087 |
| 284 | chr14 | 106381486 | 106383981 | 18 | IGHD2-2 | 0.029 | 0.032 | 0.025 | 0.059 | 0.024 | 0.39142 | 0.82914 | 0.00001 |
| 285 | chr5 | 135381970 | 135382170 | 5 | TGFBI | 0.034 | 0.030 | 0.040 | 0.038 | 0.000 | 0.39274 | 0.28309 | 0.98151 |
| 286 | chr3 | 184580664 | 184580864 | 5 | VPS8 | 0.006 | 0.007 | 0.005 | 0.075 | 0.000 | 0.40112 | 0.15248 | 0.00357 |
| 287 | chr14 | 106805291 | 106806190 | 8 | IGHV4-31 | 0.038 | 0.041 | 0.034 | 0.117 | 0.000 | 0.40201 | 0.02655 | 0.49158 |
| 288 | chr22 | 23077338 | 23077588 | 4 | IGLV2-18 | 0.025 | 0.025 | 0.025 | 0.063 | 0.000 | 0.40450 | 0.82223 | 0.42774 |
| 289 | chr11 | 134129470 | 134133940 | 40 | ACAD8 | 0.027 | 0.034 | 0.016 | 0.063 | 0.000 | 0.40456 | 0.61602 | 0.02024 |
| 290 | chr1 | 190067140 | 190068190 | 22 | FAM5C | 0.028 | 0.035 | 0.017 | 0.077 | 0.000 | 0.40678 | 0.18209 | 0.12955 |
| 291 | chr19 | 52403337 | 52403537 | 5 | ZNF649 | 0.026 | 0.026 | 0.025 | 0.075 | 0.000 | 0.41027 | 0.52307 | 0.41005 |
| 292 | chr15 | 66727355 | 66729281 | 10 | MAP2K1 | 0.035 | 0.044 | 0.020 | 0.069 | 0.000 | 0.41169 | 0.93852 | 0.81159 |
| 293 | chr6 | 94120220 | 94120720 | 11 | EPHA7 | 0.024 | 0.027 | 0.020 | 0.119 | 0.000 | 0.41348 | 0.00251 | 0.10186 |
| 294 | chr20 | 23028373 | 23028823 | 10 | THBD | 0.044 | 0.052 | 0.030 | 0.075 | 0.009 | 0.41401 | 0.97196 | 0.91852 |
| 295 | chr19 | 42599891 | 42600091 | 5 | POU2F2 | 0.038 | 0.049 | 0.020 | 0.125 | 0.000 | 0.41703 | 0.03149 | 0.68257 |
| 296 | chrX | 86772954 | 86773304 | 8 | KLHL4 | 0.026 | 0.035 | 0.013 | 0.086 | 0.000 | 0.41822 | 0.64743 | 0.29530 |
| 297 | chr9 | 37407370 | 37407570 | 5 | GRHPR | 0.046 | 0.056 | 0.030 | 0.113 | 0.000 | 0.42725 | 0.84925 | 0.34749 |
| 298 | chr9 | 20820917 | 20946827 | 8 | FOCAD | 0.015 | 0.016 | 0.013 | 0.078 | 0.000 | 0.43273 | 0.41122 | 0.00842 |
| 299 | chr6 | 91004619 | 91005994 | 10 | BACH2 | 0.051 | 0.061 | 0.038 | 0.100 | 0.017 | 0.43292 | 0.62927 | 0.61655 |
| 300 | chr9 | 139390583 | 139402863 | 17 | NOTCH1 | 0.038 | 0.045 | 0.028 | 0.140 | 0.000 | 0.44217 | 0.00038 | 0.66264 |
| 301 | chr14 | 106452661 | 106453001 | 7 | IGHV1-2 | 0.020 | 0.021 | 0.018 | 0.080 | 0.000 | 0.44604 | 0.33603 | 0.09047 |
| 302 | chr6 | 26020710 | 26020910 | 5 | HIST1H3A | 0.036 | 0.036 | 0.035 | 0.000 | 0.000 | 0.44876 | 0.01256 | 0.96541 |
| 303 | chr9 | 27950145 | 27950495 | 8 | LINGO2 | 0.022 | 0.031 | 0.009 | 0.117 | 0.000 | 0.45177 | 0.00957 | 0.11783 |
| 304 | chr7 | 80285800 | 80286050 | 6 | CD36 | 0.013 | 0.022 | 0.000 | 0.135 | 0.000 | 0.45506 | 0.00452 | 0.01644 |
| 305 | chr18 | 13825916 | 13826416 | 11 | MC5R | 0.035 | 0.043 | 0.023 | 0.085 | 0.000 | 0.45807 | 0.35320 | 0.85391 |
| 306 | chr9 | 5450475 | 5468015 | 33 | CD274 | 0.026 | 0.029 | 0.020 | 0.049 | 0.000 | 0.46045 | 0.38390 | 0.02293 |
| 307 | chr3 | 185446224 | 185538924 | 8 | IGF2BP2 | 0.019 | 0.027 | 0.006 | 0.102 | 0.000 | 0.47564 | 0.05373 | 0.03579 |
| 308 | chr1 | 3800046 | 3800353 | 7 | DFFB | 0.042 | 0.044 | 0.039 | 0.107 | 0.000 | 0.47590 | 0.23069 | 0.43666 |
| 309 | chr22 | 23055368 | 23055828 | 7 | IGLV3-21 | 0.034 | 0.035 | 0.032 | 0.107 | 0.000 | 0.47614 | 0.19555 | 0.90440 |

| | | | | | | | | | | | | |
|---|---|---|---|---|---|---|---|---|---|---|---|---|
| 310 | chr6 | 27114005 | 27114545 | 9 | HIST1H2BK | 0.023 | 0.031 | 0.011 | 0.021 | 0.000 | 0.48388 | 0.09402 | 0.14560 |
| 311 | chr14 | 107013036 | 107013186 | 4 | IGHV3-49 | 0.020 | 0.029 | 0.006 | 0.109 | 0.000 | 0.48557 | 0.05983 | 0.17265 |
| 312 | chr22 | 22453288 | 22453563 | 6 | IGLV8-61 | 0.053 | 0.055 | 0.050 | 0.083 | 0.000 | 0.48567 | 0.96231 | 0.04559 |
| 313 | chr14 | 106357891 | 106357941 | 2 | IGHD6-19 | 0.000 | 0.000 | 0.000 | 0.000 | 0.000 | 0.48646 | 0.45288 | 0.03556 |
| 314 | chr16 | 33523608 | 33523658 | 2 | IGHV3OR16-12 | 0.000 | 0.000 | 0.000 | 0.125 | 0.022 | 0.48646 | 0.05020 | 0.02436 |
| 315 | chr7 | 151943422 | 151943472 | 2 | KMT2C | 0.000 | 0.000 | 0.000 | 0.125 | 0.000 | 0.48646 | 0.10655 | 0.03556 |
| 316 | chr22 | 23114793 | 23115048 | 5 | IGLV3-12 | 0.018 | 0.026 | 0.005 | 0.000 | 0.000 | 0.49420 | 0.05467 | 0.09497 |
| 317 | chr2 | 80801236 | 80801486 | 6 | CTNNA2 | 0.017 | 0.025 | 0.004 | 0.146 | 0.000 | 0.50036 | 0.00472 | 0.03774 |
| 318 | chr22 | 23161918 | 23162288 | 8 | IGLV3-9 | 0.036 | 0.039 | 0.031 | 0.063 | 0.000 | 0.50251 | 0.65174 | 0.76665 |
| 319 | chr12 | 113495365 | 113534745 | 80 | DTX1 | 0.058 | 0.065 | 0.047 | 0.075 | 0.000 | 0.50409 | 0.06246 | 0.00000 |
| 320 | chr11 | 65190343 | 65190543 | 5 | FRMD8 | 0.050 | 0.049 | 0.050 | 0.038 | 0.009 | 0.51163 | 0.10472 | 0.60740 |
| 321 | chr14 | 106967131 | 106967366 | 4 | IGHV1-46 | 0.022 | 0.033 | 0.006 | 0.063 | 0.000 | 0.51321 | 0.66087 | 0.32094 |
| 322 | chr12 | 25205889 | 25207439 | 21 | LRMP | 0.038 | 0.041 | 0.033 | 0.080 | 0.027 | 0.51555 | 0.36573 | 0.00948 |
| 323 | chr14 | 106780611 | 106780711 | 3 | IGHV4-28 | 0.036 | 0.038 | 0.033 | 0.125 | 0.000 | 0.51984 | 0.19368 | 0.92185 |
| 324 | chr11 | 125472641 | 125472891 | 6 | STT3A | 0.046 | 0.055 | 0.033 | 0.052 | 0.000 | 0.52125 | 0.24640 | 0.20117 |
| 325 | chr11 | 69346692 | 69346892 | 5 | CCND1 | 0.024 | 0.026 | 0.020 | 0.113 | 0.000 | 0.52233 | 0.04449 | 0.30659 |
| 326 | chr13 | 51915234 | 51915534 | 7 | SERPINE3 | 0.035 | 0.044 | 0.021 | 0.152 | 0.000 | 0.53028 | 0.03239 | 0.74664 |
| 327 | chr5 | 21783416 | 21783666 | 6 | CDH12 | 0.020 | 0.022 | 0.017 | 0.083 | 0.000 | 0.53207 | 0.16100 | 0.13344 |
| 328 | chr12 | 25398219 | 25398269 | 2 | KRAS | 0.015 | 0.025 | 0.000 | 0.000 | 0.000 | 0.53308 | 0.26379 | 0.18377 |
| 329 | chr1 | 85733208 | 85742033 | 19 | BCL10 | 0.021 | 0.025 | 0.016 | 0.056 | 0.000 | 0.53493 | 0.60987 | 0.00831 |
| 330 | chr1 | 107866872 | 107867572 | 15 | NTNG1 | 0.013 | 0.015 | 0.010 | 0.063 | 0.000 | 0.53686 | 0.17297 | 0.00018 |
| 331 | chr1 | 86591438 | 86591888 | 10 | COL24A1 | 0.029 | 0.036 | 0.018 | 0.075 | 0.000 | 0.53874 | 0.54478 | 0.46033 |
| 332 | chr18 | 30349776 | 30350276 | 11 | KLHL14 | 0.033 | 0.036 | 0.030 | 0.091 | 0.000 | 0.53960 | 0.49213 | 0.94697 |
| 333 | chr14 | 106641656 | 106642261 | 7 | IGHV1-18 | 0.023 | 0.026 | 0.018 | 0.063 | 0.019 | 0.54851 | 0.55397 | 0.01550 |
| 334 | chr17 | 78343504 | 78343704 | 5 | RNF213 | 0.014 | 0.016 | 0.010 | 0.038 | 0.000 | 0.54949 | 0.86764 | 0.04664 |
| 335 | chr1 | 120457961 | 120459261 | 27 | NOTCH2 | 0.036 | 0.039 | 0.031 | 0.053 | 0.000 | 0.55999 | 0.22789 | 0.63380 |
| 336 | chr17 | 40467710 | 40491485 | 39 | STAT3 | 0.034 | 0.040 | 0.023 | 0.059 | 0.000 | 0.56418 | 0.51376 | 0.71754 |
| 337 | chr9 | 19957357 | 19958157 | 17 | SLC24A2 | 0.027 | 0.031 | 0.022 | 0.063 | 0.000 | 0.56498 | 0.75617 | 0.22788 |
| 338 | chr3 | 38180130 | 38182805 | 29 | MYD88 | 0.045 | 0.053 | 0.033 | 0.073 | 0.000 | 0.56578 | 0.70668 | 0.03867 |
| 339 | chr18 | 73944894 | 73945344 | 10 | ZNF516 | 0.018 | 0.025 | 0.008 | 0.056 | 0.000 | 0.56926 | 0.67544 | 0.01359 |
| 340 | chr7 | 140453013 | 140453254 | 5 | BRAF | 0.012 | 0.020 | 0.000 | 0.075 | 0.000 | 0.56966 | 0.30182 | 0.01894 |
| 341 | chr6 | 159238416 | 159238766 | 8 | EZR | 0.050 | 0.057 | 0.038 | 0.016 | 0.000 | 0.57311 | 0.00246 | 0.08463 |
| 342 | chr18 | 77092821 | 77093021 | 5 | ATP9B | 0.008 | 0.010 | 0.005 | 0.075 | 0.000 | 0.57396 | 0.16232 | 0.00549 |
| 343 | chr22 | 23523568 | 23610748 | 22 | BCR | 0.038 | 0.045 | 0.028 | 0.097 | 0.000 | 0.57399 | 0.04814 | 0.27043 |
| 344 | chr22 | 22673243 | 22673593 | 8 | IGLV5-52 | 0.027 | 0.035 | 0.016 | 0.117 | 0.000 | 0.57479 | 0.00701 | 0.30927 |
| 345 | chr4 | 88011078 | 88011278 | 5 | AFF1 | 0.014 | 0.016 | 0.010 | 0.038 | 0.000 | 0.57733 | 0.89980 | 0.03303 |
| 346 | chr11 | 131747550 | 131748000 | 10 | NTM | 0.029 | 0.036 | 0.018 | 0.119 | 0.000 | 0.57801 | 0.02773 | 0.42832 |
| 347 | chr2 | 90077982 | 90078316 | 6 | IGKV3D-20 | 0.025 | 0.033 | 0.013 | 0.031 | 0.000 | 0.57996 | 0.26904 | 0.32350 |
| 348 | chr2 | 96809890 | 96810360 | 10 | DUSP2 | 0.063 | 0.066 | 0.060 | 0.006 | 0.000 | 0.58190 | 0.00002 | 0.00216 |
| 349 | chr2 | 89265757 | 89265987 | 4 | IGKV1-6 | 0.010 | 0.012 | 0.006 | 0.047 | 0.000 | 0.59812 | 0.84325 | 0.02299 |
| 350 | chr19 | 53598587 | 53599037 | 10 | ZNF160 | 0.024 | 0.031 | 0.013 | 0.063 | 0.000 | 0.60291 | 0.98122 | 0.12855 |
| 351 | chr2 | 63335243 | 63631808 | 22 | WDPCP | 0.026 | 0.033 | 0.016 | 0.091 | 0.000 | 0.60661 | 0.01199 | 0.09457 |
| 352 | chr9 | 21808815 | 21859450 | 9 | MTAP | 0.019 | 0.026 | 0.008 | 0.042 | 0.000 | 0.61688 | 0.80480 | 0.03120 |

| 353 | chr6 | 27860480 | 27860895 | 7 | HIST1H2AM | 0.030 | 0.033 | 0.025 | 0.045 | 0.000 | 0.61920 | 0.45404 | 0.60865 |
|---|---|---|---|---|---|---|---|---|---|---|---|---|---|
| 354 | chr6 | 27839659 | 27839759 | 3 | HIST1H3I | 0.036 | 0.038 | 0.033 | 0.021 | 0.000 | 0.62267 | 0.15955 | 0.75106 |
| 355 | chr6 | 26252155 | 26252205 | 2 | HIST1H2BH | 0.015 | 0.016 | 0.013 | 0.063 | 0.000 | 0.62577 | 0.55784 | 0.18377 |
| 356 | chr19 | 19256470 | 19293460 | 41 | MEF2B | 0.040 | 0.045 | 0.032 | 0.091 | 0.000 | 0.62683 | 0.04274 | 0.29098 |
| 357 | chr14 | 107169646 | 107170861 | 21 | IGHV1-69 | 0.091 | 0.098 | 0.082 | 0.107 | 0.029 | 0.63032 | 0.38178 | 0.00266 |
| 358 | chr8 | 113308015 | 113569195 | 15 | CSMD3 | 0.013 | 0.020 | 0.003 | 0.046 | 0.000 | 0.63047 | 0.85436 | 0.00010 |
| 359 | chr22 | 22550338 | 22550788 | 10 | IGLV6-57 | 0.042 | 0.049 | 0.030 | 0.131 | 0.017 | 0.64049 | 0.04005 | 0.29687 |
| 360 | chr4 | 153249286 | 153249486 | 5 | FBXW7 | 0.026 | 0.026 | 0.025 | 0.038 | 0.000 | 0.64551 | 0.50853 | 0.39977 |
| 361 | chr11 | 120127164 | 120189629 | 22 | POU2F3 | 0.027 | 0.033 | 0.018 | 0.091 | 0.000 | 0.64824 | 0.02013 | 0.09628 |
| 362 | chr12 | 57496553 | 57499113 | 13 | STAT6 | 0.046 | 0.054 | 0.035 | 0.072 | 0.013 | 0.65115 | 0.71967 | 0.94722 |
| 363 | chr22 | 22937193 | 22937499 | 7 | IGLV3-32 | 0.018 | 0.026 | 0.007 | 0.063 | 0.000 | 0.65348 | 0.49810 | 0.05644 |
| 364 | chr6 | 138188484 | 138202489 | 64 | TNFAIP3 | 0.024 | 0.028 | 0.018 | 0.035 | 0.004 | 0.65552 | 0.00591 | 0.00002 |
| 365 | chr8 | 138849938 | 138850138 | 5 | FAM135B | 0.020 | 0.023 | 0.015 | 0.038 | 0.000 | 0.65643 | 0.70665 | 0.12531 |
| 366 | chr14 | 107218756 | 107218856 | 3 | IGHV3-74 | 0.073 | 0.082 | 0.058 | 0.104 | 0.058 | 0.66142 | 0.98960 | 0.26299 |
| 367 | chr14 | 23344698 | 23345198 | 11 | LRP10 | 0.059 | 0.063 | 0.052 | 0.034 | 0.000 | 0.66215 | 0.00576 | 0.01137 |
| 368 | chr14 | 106866381 | 106866595 | 5 | IGHV3-38 | 0.032 | 0.033 | 0.030 | 0.163 | 0.000 | 0.66584 | 0.01626 | 0.86538 |
| 369 | chr1 | 3547351 | 3547701 | 8 | WRAP73 | 0.024 | 0.027 | 0.019 | 0.063 | 0.000 | 0.66789 | 0.68610 | 0.19690 |
| 370 | chr21 | 28213259 | 28216964 | 11 | ADAMTS1 | 0.028 | 0.036 | 0.016 | 0.108 | 0.012 | 0.67094 | 0.03930 | 0.06299 |
| 371 | chr2 | 169781121 | 169781321 | 5 | ABCB11 | 0.016 | 0.023 | 0.005 | 0.125 | 0.000 | 0.67664 | 0.00990 | 0.06041 |
| 372 | chr22 | 41513341 | 41574886 | 72 | EP300 | 0.031 | 0.037 | 0.022 | 0.067 | 0.000 | 0.67996 | 0.51033 | 0.09373 |
| 373 | chr18 | 56054916 | 56063816 | 24 | NEDD4L | 0.016 | 0.020 | 0.009 | 0.031 | 0.000 | 0.68133 | 0.24138 | 0.00003 |
| 374 | chr14 | 106845301 | 106846536 | 9 | IGHV3-35 | 0.055 | 0.064 | 0.042 | 0.097 | 0.000 | 0.68499 | 0.76566 | 0.05591 |
| 375 | chr14 | 107136756 | 107136856 | 3 | IGHV3-66 | 0.030 | 0.038 | 0.017 | 0.021 | 0.000 | 0.68512 | 0.22171 | 0.79848 |
| 376 | chr22 | 23047068 | 23047318 | 6 | IGLV3-22 | 0.043 | 0.049 | 0.033 | 0.042 | 0.014 | 0.68905 | 0.16524 | 0.80319 |
| 377 | chr22 | 22786478 | 22786803 | 7 | IGLV1-36 | 0.040 | 0.047 | 0.029 | 0.080 | 0.000 | 0.69080 | 0.82010 | 0.41665 |
| 378 | chr8 | 122626848 | 122627148 | 7 | HAS2 | 0.030 | 0.033 | 0.025 | 0.063 | 0.000 | 0.70243 | 0.90117 | 0.66520 |
| 379 | chr5 | 131825018 | 131825218 | 5 | IRF1 | 0.026 | 0.030 | 0.020 | 0.138 | 0.000 | 0.70868 | 0.00725 | 0.42851 |
| 380 | chr22 | 23252688 | 23252788 | 3 | IGLJ4 | 0.020 | 0.022 | 0.017 | 0.021 | 0.000 | 0.71377 | 0.39782 | 0.24178 |
| 381 | chr14 | 107078456 | 107078606 | 4 | IGHV1-58 | 0.050 | 0.053 | 0.044 | 0.063 | 0.000 | 0.71737 | 0.53128 | 0.17192 |
| 382 | chr4 | 154624671 | 154625021 | 8 | TLR2 | 0.017 | 0.020 | 0.013 | 0.125 | 0.000 | 0.72168 | 0.00257 | 0.03397 |
| 383 | chr2 | 89196227 | 89215037 | 19 | IGKV5-2 | 0.024 | 0.028 | 0.017 | 0.036 | 0.007 | 0.73228 | 0.12196 | 0.02080 |
| 384 | chr18 | 55319681 | 55359256 | 17 | ATP8B1 | 0.028 | 0.031 | 0.024 | 0.044 | 0.000 | 0.73256 | 0.29761 | 0.29755 |
| 385 | chr1 | 61553803 | 61554303 | 11 | NFIA | 0.030 | 0.033 | 0.025 | 0.097 | 0.000 | 0.73331 | 0.11994 | 0.58902 |
| 386 | chr10 | 89603603 | 89604053 | 10 | KLLN | 0.024 | 0.028 | 0.018 | 0.044 | 0.000 | 0.73666 | 0.57207 | 0.12653 |
| 387 | chr22 | 23247138 | 23247609 | 9 | IGLJ3 | 0.165 | 0.169 | 0.158 | 0.153 | 0.048 | 0.73794 | 0.02871 | 0.00093 |
| 388 | chr11 | 117101044 | 117101194 | 4 | PCSK7 | 0.042 | 0.049 | 0.031 | 0.016 | 0.000 | 0.73868 | 0.05815 | 0.47968 |
| 389 | chr6 | 27861245 | 27861450 | 4 | HIST1H2BO | 0.037 | 0.045 | 0.025 | 0.031 | 0.000 | 0.74033 | 0.21815 | 0.85767 |
| 390 | chr2 | 61441170 | 61441870 | 15 | USP34 | 0.025 | 0.028 | 0.020 | 0.042 | 0.000 | 0.74279 | 0.23146 | 0.11749 |
| 391 | chr11 | 111234537 | 111249512 | 16 | POU2AF1 | 0.030 | 0.034 | 0.023 | 0.105 | 0.008 | 0.74326 | 0.02352 | 0.08875 |
| 392 | chr5 | 5182146 | 5182446 | 7 | ADAMTS16 | 0.038 | 0.044 | 0.029 | 0.107 | 0.000 | 0.75162 | 0.19189 | 0.54007 |
| 393 | chr14 | 106667546 | 106667856 | 6 | IGHV3-20 | 0.021 | 0.025 | 0.017 | 0.063 | 0.000 | 0.75404 | 0.64784 | 0.15262 |
| 394 | chr2 | 145162402 | 145693052 | 53 | ZEB2 | 0.041 | 0.046 | 0.032 | 0.048 | 0.008 | 0.76200 | 0.00643 | 0.47223 |
| 395 | chr14 | 106494091 | 106494768 | 12 | IGHV2-5 | 0.027 | 0.034 | 0.017 | 0.063 | 0.014 | 0.76623 | 0.78849 | 0.01259 |

| | | | | | | | | | | | | | |
|---|---|---|---|---|---|---|---|---|---|---|---|---|---|
| 396 | chr2 | 65593036 | 65593213 | 4 | SPRED2 | 0.057 | 0.061 | 0.050 | 0.250 | 0.033 | 0.77068 | 0.00195 | 0.40243 |
| 397 | chr2 | 141245128 | 141245328 | 5 | LRP1B | 0.010 | 0.016 | 0.000 | 0.088 | 0.000 | 0.77497 | 0.10161 | 0.00830 |
| 398 | chr22 | 23241763 | 23241813 | 2 | IGLJ2 | 0.030 | 0.033 | 0.025 | 0.094 | 0.000 | 0.77602 | 0.38252 | 0.80404 |
| 399 | chrX | 153997384 | 153997584 | 5 | DKC1 | 0.042 | 0.046 | 0.035 | 0.075 | 0.000 | 0.77946 | 0.93861 | 0.49207 |
| 400 | chr10 | 5755067 | 5755267 | 5 | FAM208B | 0.016 | 0.020 | 0.010 | 0.000 | 0.000 | 0.77955 | 0.06988 | 0.04606 |
| 401 | chr1 | 35472493 | 35472693 | 5 | ZMYM6 | 0.016 | 0.020 | 0.010 | 0.025 | 0.000 | 0.77955 | 0.46246 | 0.04606 |
| 402 | chr6 | 26250460 | 26250695 | 5 | HIST1H3F | 0.028 | 0.033 | 0.020 | 0.013 | 0.000 | 0.78052 | 0.07461 | 0.50252 |
| 403 | chr3 | 176750700 | 176771710 | 17 | TBL1XR1 | 0.020 | 0.024 | 0.013 | 0.051 | 0.003 | 0.78556 | 0.88935 | 0.00559 |
| 404 | chr18 | 77170716 | 77288591 | 29 | NFATC1 | 0.038 | 0.043 | 0.031 | 0.082 | 0.000 | 0.78831 | 0.61891 | 0.47180 |
| 405 | chr13 | 41133663 | 41240784 | 49 | FOXO1 | 0.025 | 0.031 | 0.016 | 0.042 | 0.000 | 0.78900 | 0.09626 | 0.00465 |
| 406 | chr8 | 128951725 | 128951875 | 4 | TMEM75 | 0.042 | 0.049 | 0.031 | 0.016 | 0.000 | 0.78980 | 0.05059 | 0.43332 |
| 407 | chr22 | 22681928 | 22682198 | 5 | IGLV1-50 | 0.020 | 0.026 | 0.010 | 0.088 | 0.000 | 0.79643 | 0.39142 | 0.12531 |
| 408 | chr2 | 89976277 | 89976377 | 3 | IGKV2D-30 | 0.066 | 0.071 | 0.058 | 0.125 | 0.000 | 0.79654 | 0.28677 | 0.06295 |
| 409 | chr14 | 106757726 | 106758621 | 8 | IGHV2-26 | 0.026 | 0.033 | 0.016 | 0.039 | 0.000 | 0.80101 | 0.48691 | 0.27328 |
| 410 | chr1 | 2306312 | 2306812 | 11 | MORN1 | 0.028 | 0.034 | 0.018 | 0.102 | 0.000 | 0.80151 | 0.03618 | 0.25568 |
| 411 | chr14 | 106384031 | 106384926 | 9 | IGHD1-1 | 0.039 | 0.046 | 0.028 | 0.132 | 0.024 | 0.81269 | 0.00673 | 0.00968 |
| 412 | chr8 | 104897562 | 104898462 | 19 | RIMS2 | 0.030 | 0.036 | 0.021 | 0.099 | 0.000 | 0.81294 | 0.04875 | 0.36772 |
| 413 | chr10 | 89500958 | 89501108 | 4 | PAPSS2 | 0.025 | 0.029 | 0.019 | 0.047 | 0.000 | 0.81562 | 0.75051 | 0.38140 |
| 414 | chr1 | 201038553 | 201038753 | 5 | CACNA1S | 0.034 | 0.033 | 0.035 | 0.113 | 0.000 | 0.82537 | 0.08167 | 0.99310 |
| 415 | chr13 | 84453543 | 84455243 | 35 | SLITRK1 | 0.034 | 0.039 | 0.026 | 0.073 | 0.000 | 0.82863 | 0.60871 | 0.95353 |
| 416 | chr22 | 23263508 | 23264123 | 9 | IGLJ7 | 0.062 | 0.069 | 0.050 | 0.042 | 0.000 | 0.84212 | 0.02446 | 0.00290 |
| 417 | chr5 | 140208034 | 140208834 | 17 | PCDHA6 | 0.026 | 0.031 | 0.019 | 0.051 | 0.000 | 0.84499 | 0.73711 | 0.13168 |
| 418 | chr1 | 23885408 | 23885899 | 10 | ID3 | 0.015 | 0.020 | 0.008 | 0.081 | 0.000 | 0.84648 | 0.06666 | 0.00452 |
| 419 | chr14 | 106518496 | 106519064 | 7 | IGHV3-7 | 0.035 | 0.040 | 0.029 | 0.054 | 0.000 | 0.84779 | 0.54879 | 0.79096 |
| 420 | chr9 | 22005930 | 22009000 | 13 | CDKN2B | 0.031 | 0.035 | 0.025 | 0.038 | 0.000 | 0.85460 | 0.20627 | 0.52500 |
| 421 | chr11 | 58978693 | 58979345 | 11 | MPEG1 | 0.032 | 0.036 | 0.025 | 0.080 | 0.000 | 0.85627 | 0.50475 | 0.70735 |
| 422 | chr1 | 227842647 | 227842697 | 2 | ZNF678 | 0.010 | 0.016 | 0.000 | 0.156 | 0.000 | 0.85664 | 0.04034 | 0.09510 |
| 423 | chr6 | 106534267 | 106555367 | 60 | PRDM1 | 0.031 | 0.036 | 0.023 | 0.065 | 0.000 | 0.86083 | 0.99103 | 0.15072 |
| 424 | chr2 | 198950435 | 198950985 | 12 | PLCL1 | 0.021 | 0.027 | 0.013 | 0.094 | 0.000 | 0.86126 | 0.14473 | 0.05072 |
| 425 | chr18 | 6947105 | 6980665 | 10 | LAMA1 | 0.027 | 0.033 | 0.018 | 0.094 | 0.000 | 0.86312 | 0.22629 | 0.28027 |
| 426 | chr6 | 26197105 | 26197462 | 8 | HIST1H3D | 0.021 | 0.027 | 0.013 | 0.000 | 0.000 | 0.86864 | 0.00995 | 0.09168 |
| 427 | chr19 | 51525627 | 51525927 | 7 | KLK11 | 0.028 | 0.033 | 0.021 | 0.089 | 0.000 | 0.87219 | 0.14799 | 0.45199 |
| 428 | chr2 | 61719435 | 61719635 | 5 | XPO1 | 0.012 | 0.016 | 0.005 | 0.000 | 0.000 | 0.87795 | 0.09496 | 0.02531 |
| 429 | chrX | 141291053 | 141291534 | 10 | MAGEC2 | 0.019 | 0.023 | 0.013 | 0.081 | 0.000 | 0.88059 | 0.07959 | 0.02755 |
| 430 | chr14 | 35873672 | 35873822 | 4 | NFKBIA | 0.035 | 0.041 | 0.025 | 0.000 | 0.000 | 0.88119 | 0.02331 | 0.96205 |
| 431 | chr2 | 89442292 | 89443217 | 19 | IGKV3-20 | 0.042 | 0.047 | 0.036 | 0.148 | 0.050 | 0.88608 | 0.00002 | 0.00006 |
| 432 | chr1 | 72334892 | 72335098 | 5 | NEGR1 | 0.014 | 0.020 | 0.005 | 0.025 | 0.000 | 0.88638 | 0.51822 | 0.02712 |
| 433 | chr1 | 9784433 | 9784533 | 3 | PIK3CD | 0.007 | 0.011 | 0.000 | 0.083 | 0.000 | 0.89151 | 0.14993 | 0.02634 |
| 434 | chr2 | 170101186 | 170101386 | 5 | LRP2 | 0.032 | 0.036 | 0.025 | 0.100 | 0.000 | 0.89564 | 0.18901 | 0.76737 |
| 435 | chr7 | 110737412 | 110764944 | 51 | LRRN3 | 0.019 | 0.024 | 0.011 | 0.086 | 0.002 | 0.90183 | 0.00080 | 0.00000 |
| 436 | chr3 | 7620224 | 7620974 | 16 | GRM7 | 0.032 | 0.038 | 0.023 | 0.078 | 0.000 | 0.90333 | 0.28646 | 0.77891 |
| 437 | chr22 | 22569333 | 22569633 | 7 | IGLV10-54 | 0.031 | 0.037 | 0.021 | 0.063 | 0.000 | 0.90702 | 0.86839 | 0.77523 |
| 438 | chr17 | 75447869 | 75448419 | 12 | 9-Sep | 0.031 | 0.037 | 0.021 | 0.036 | 0.000 | 0.90976 | 0.14194 | 0.64487 |
| 439 | chr7 | 148506319 | 148523734 | 19 | EZH2 | 0.019 | 0.025 | 0.011 | 0.082 | 0.000 | 0.91143 | 0.05741 | 0.00268 |
| 440 | chr14 | 106621886 | 106622095 | 5 | IGHV3-16 | 0.024 | 0.030 | 0.015 | 0.063 | 0.000 | 0.91521 | 0.67996 | 0.28737 |

EP 4 334 476 B1

| 441 | chr1 | 181452915 | 181453115 | 5 | CACNA1E | 0.032 | 0.036 | 0.025 | 0.025 | 0.000 | 0.91767 | 0.14135 | 0.76209 |
| 442 | chr2 | 58520801 | 58521201 | 9 | FANCL | 0.029 | 0.035 | 0.019 | 0.069 | 0.000 | 0.92005 | 0.73186 | 0.57669 |
| 443 | chr19 | 51559442 | 51561922 | 16 | KLK13 | 0.032 | 0.038 | 0.023 | 0.113 | 0.000 | 0.92076 | 0.04033 | 0.89701 |
| 444 | chr16 | 2812097 | 2812747 | 14 | SRRM2 | 0.056 | 0.062 | 0.046 | 0.045 | 0.000 | 0.92192 | 0.02154 | 0.01164 |
| 445 | chr6 | 41903612 | 41909397 | 26 | CCND3 | 0.041 | 0.047 | 0.033 | 0.058 | 0.000 | 0.92504 | 0.14949 | 0.21095 |
| 446 | chr14 | 106068706 | 106071241 | 16 | IGHE | 0.118 | 0.124 | 0.108 | 0.215 | 0.158 | 0.92648 | 0.00059 | 0.00000 |
| 447 | chr6 | 110777719 | 110778219 | 11 | SLC22A16 | 0.027 | 0.033 | 0.018 | 0.034 | 0.000 | 0.92796 | 0.19315 | 0.23193 |
| 448 | chr9 | 21970835 | 21994385 | 37 | CDKN2A | 0.027 | 0.031 | 0.020 | 0.039 | 0.000 | 0.92888 | 0.04082 | 0.03393 |
| 449 | chr2 | 90025207 | 90025522 | 6 | IGKV2D-26 | 0.012 | 0.016 | 0.004 | 0.031 | 0.000 | 0.92990 | 0.73921 | 0.01161 |
| 450 | chr4 | 7728457 | 7728657 | 5 | SORCS2 | 0.034 | 0.039 | 0.025 | 0.038 | 0.000 | 0.93035 | 0.30875 | 0.99310 |
| 451 | chr7 | 5569096 | 5569356 | 6 | ACTB | 0.048 | 0.055 | 0.038 | 0.208 | 0.007 | 0.93481 | 0.00069 | 0.95055 |
| 452 | chr3 | 140281599 | 140281849 | 6 | CLSTN2 | 0.036 | 0.038 | 0.033 | 0.031 | 0.000 | 0.94099 | 0.11813 | 0.72422 |
| 453 | chr2 | 89291907 | 89292182 | 4 | IGKV1-8 | 0.020 | 0.025 | 0.013 | 0.047 | 0.022 | 0.94155 | 0.86146 | 0.00511 |
| 454 | chr22 | 23260268 | 23260368 | 3 | IGLJ6 | 0.043 | 0.049 | 0.033 | 0.063 | 0.000 | 0.94574 | 0.74604 | 0.48180 |
| 455 | chr14 | 106815806 | 106815906 | 3 | IGHV3-33 | 0.059 | 0.066 | 0.050 | 0.063 | 0.043 | 0.94598 | 0.41907 | 0.10857 |
| 456 | chr6 | 26123615 | 26124080 | 9 | HIST1H2BC | 0.031 | 0.036 | 0.022 | 0.028 | 0.000 | 0.95616 | 0.07091 | 0.75304 |
| 457 | chr3 | 49397609 | 49413039 | 18 | RHOA | 0.030 | 0.035 | 0.022 | 0.045 | 0.000 | 0.95622 | 0.26281 | 0.40030 |
| 458 | chr22 | 29191137 | 29196512 | 28 | XBP1 | 0.032 | 0.039 | 0.022 | 0.085 | 0.003 | 0.95630 | 0.05799 | 0.16891 |
| 459 | chr14 | 106471396 | 106471580 | 4 | IGHV1-3 | 0.007 | 0.012 | 0.000 | 0.141 | 0.000 | 0.95914 | 0.00935 | 0.01524 |
| 460 | chr17 | 41847059 | 41847209 | 4 | DUSP3 | 0.032 | 0.037 | 0.025 | 0.094 | 0.000 | 0.96078 | 0.74050 | 0.94029 |
| 461 | chr17 | 51900442 | 51900892 | 10 | KIF2B | 0.035 | 0.039 | 0.028 | 0.088 | 0.000 | 0.96080 | 0.24029 | 0.71768 |
| 462 | chr15 | 86312063 | 86312563 | 11 | KLHL25 | 0.032 | 0.037 | 0.025 | 0.074 | 0.000 | 0.96521 | 0.83987 | 0.74482 |
| 463 | chr18 | 53804516 | 53804766 | 6 | TXNL1 | 0.036 | 0.041 | 0.029 | 0.115 | 0.000 | 0.96529 | 0.05667 | 0.84317 |
| 464 | chr5 | 67590967 | 67591167 | 5 | PIK3R1 | 0.018 | 0.023 | 0.010 | 0.075 | 0.009 | 0.97792 | 0.39415 | 0.02207 |
| 465 | chr5 | 124079828 | 124080678 | 18 | ZNF608 | 0.026 | 0.031 | 0.019 | 0.063 | 0.000 | 0.98245 | 0.74836 | 0.14794 |
| 466 | chr2 | 90259932 | 90260232 | 5 | IGKV1D-8 | 0.034 | 0.039 | 0.025 | 0.163 | 0.000 | 0.98690 | 0.17514 | 0.96394 |
| 467 | chr2 | 88906682 | 88906832 | 4 | EIF2AK3 | 0.059 | 0.066 | 0.050 | 0.063 | 0.000 | 0.98750 | 0.34568 | 0.07429 |
| 468 | chr4 | 106157605 | 106157805 | 5 | TET2 | 0.018 | 0.023 | 0.010 | 0.075 | 0.000 | 0.99542 | 0.34309 | 0.09635 |

204

Table 5

| Reference Coordinates | Nearest Gene | Percent Non-Reference | Total Non-Reference Bases | Plus Strand Oligonuclotide | SEQ ID NOS: |
|---|---|---|---|---|---|
| chr8:128,750550-128,750.699 | *MYC* | 0 | 0 | CGACTACGACTCGGTGCAGCCGTATTTCTACTGCGACGAGGAGGAGAACTT CTACCAGCAGCAGCAGCAGAGCGAGCTGCAGCCCCGGCGCCCAGCGAGG ATATCTGGAAGAAATTCGAGCTGCTGCCCACCCCGCCCCTGTCCCCTAG | 1331 |
| chr8:128.750,550-128.750,699 | *MYC* | 2.5 | 4 | CGACTACGACTCGGTGCAGCCGTAGTTCTACTGCGACGAGGAGGAAAACT TCTACCAGCAGCAGCAGCAGAGCGAGCTGCAGCCCCTGGCGCCCAGCGAG GATATCTGGAAGAACTTCGAGCTGCTGCCCACCCCGCCCCTGTCCCCTAG | 1332 |
| chr8:128,750550-128,750.699 | *MYC* | 5 | 8 | CGACTACGACTCGGTGCAGCCGTAGTTCTACTGCGACGAGGAGGAATACTT CTACCAGCAGCAGCCGCAGAGCGAGCTGCAGCCCCTGGCGCCCAGCGAGG GTATCTGGAAGAACTTCGAGCTACTGCCCACCCCGCCCCTGTCCCCTAG | 1333 |
| chr8:128.750,550-128.750,699 | *MYC* | 7.5 | 11 | CGACTACGACTCGTTGCAGCCGTAGTTCTACTGCGACGAGGAGGAATACTT CTACCAGCAGCAGCCGCAGAGCGAGCTGCAGCGCCTGGCGCCCAGCGAGG GTATCTGGAAGAACTTCGAGCTACAGCCCACCCCGCCCCTGTCCCCTAG | 1334 |
| chr8:128,750,550-128,750,699 | *MYC* | 10 | 15 | CGACTACGACTCGTTGCAGCCGTAGATCTACTGCGACGAGGAGGAATACTT CTACCTGCAGCAGCCGCAGAGCGAGCTGCAGCGCCTGGCGCCCAGCGAGC GTATCTGGAAGAACTTCGAGCTACAGCCCACCCCGCCCTTGTCCCCTAG | 1335 |
| chr8:128.750,550-128.750,699 | *MYC* | 12.5 | 19 | CGACAACGACTCGTTGCACCCGTAGATCTACTGCGACGAGGAGGAATACTT CTACCTGCAGCAGCCGCAGAGCGAGCTGCAGCGCCTGGCGCCCAGCGAGC GTATCTGAAAGAACTTCGAGCTACAGCCCACGCCGCCCTTGTCCCCTAG | 1336 |

205

| Reference Coordinates | Nearest Gene | Percent Non-Reference | Total Non-Reference Bases | Plus Strand Oligonuclotide | SEQ ID NOS: |
|---|---|---|---|---|---|
| chr8:128,750,550-128,750,699 | *MYC* | 15 | 23 | CGACAACGACTCGTTGCACCCGTAGATCTACTGCGACGAGGAGGAATACTT CTACCTGCAGCAGCCGCAGAGCGAGCTGCAGCGCCTGGCGCCCAGCGAGC GTATCTGAAAGAACTTCGAGCTACAGCCCACGCCGCCCTTGTCCCCTAG | 1337 |
| chr3:187,443.281-187,443.430 | *BCL6* | 0 | 0 | GCTCACCTGTACAAATCTGGCTCCGCAGGTTTCGCATTTGTAGGGCTTCTCT CCAGAGTGAATTCGAGTGTGGGTTTTCAGGTTGGCTGGCCGGTTGAACTGG GCCCCACAGATGTTGCAACGATAGGGTTTCTCACCTATTACCAAGAA | 1338 |
| chr3:187,443,281-187,443,430 | *BCL6* | 2.5 | 4 | GCTCACCTGTACAAATCTGCCTCCGCAGGTTTCGCATTTGTAGGGCTCCTCT CCAGAGTGAATTCGAGTGTGGGTTTTCAGGTTGGCTGGGCGGTTGAACTGG GCCCCACAGATGTTGCAACGCTAGGGTTTCTCACCTATTACCAAGAA | 1339 |
| chr3: 187,443,281-187,443,430 | *BCL6* | 5 | 8 | GCTCACCTGTACAAATCTGCCTCCGCAGGTTTCGCCTTTGTAGGGCTCCTCT CCAGAGTGAATTCGAGTGTAGGTTTTCAAGTTGGCTGGGCGGTTGAACTGG GCCCCACGGATGTTGCAACGCTAGGGTTTCTCACCTATTACCAAGAA | 1340 |
| chr3:187,443,281-187,443,430 | *BCL6* | 7.5 | 11 | GCTCACCTGTACAAATCTGCCTCCGCCGGTTTCGCCTTTTTAGGGCTCCTCT CCAGAGTGAATTCGAGTGTAGGTTTTCAAGTTGGCTGGGCGGTTGAACTGG GCCCCACGGATGTTGCAACGCTAGGGTTTCTCACCTATTTCCAAGAA | 1341 |
| chr3:187,443.281-187,443.430 | *BCL6* | 10 | 15 | GCTCACCTGTACAAGTCTGCCTCCGCCGGTTACGCCTTTTTAGGGCTCCTCT CCAGAGTGAATTCGAGTGTAGGTTTTCAAGTTGGCTGGGCGGTTGAACTGG GCTCCACGGATGTTGCAACGCTAGGGATTCTCACCTATTTCCAAGAA | 1342 |
| chr3:187,443,281-187,443,430 | *BCL6* | 12.5 | 19 | GCTCACCTGGACAAGTCTGCCTCCGCCGGTTACGACTTTTTAGGGCTCCTCT CCAGAGTGAATTCGAGTGTAGGCTTTCAAGTTGGCTGGGCGGTTGAACTGG GCTCCACGGCTGTTGCAACGCTAGGGATTCTCACCTATTTCCAAGAA | 1343 |
| ehr3: 187.443,281-187.443,430 | *BCL6* | 15 | 23 | GCTCACCTGGACAAGTCTGCCTCCGCCGGTTACGACTTTTTAGGGCACCTCT CCAGAGTGAATTCGAGTGTAGGCTTTCAAGTTGGCTGGGAGCTTGAACTGG GCTGCACGGCTGTTGCAACGCTAGGGATTCTCACCTATTTCCAAGAA | 1344 |

206

EP 4 334 476 B1

| - | - | - | - | Minus Strand Oligonucleotide | - |
|---|---|---|---|---|---|
| chr8:128,750,550-128,750,699 | MYC | 0 | 0 | CTAGGGGACAGGGGCGGGGTGGGCAGCAGCTCGAATTTCTTCCAGATATC CTCGCTGGGCGCCGGGGGCTGCAGCTCGCTCTGCTGCTGCTGCTGGTAGAA GTTCTCCTCCTCGTCGCAGTAGAAATACGGCTGCACCGAGTCGTAGTCG | 1345 |
| chr8:128.750,550-128.750,699 | MYC | 2.5 | 4 | CTAGGGGACAGGGGCGGGGTGGGCAGCAGCTCGAAGTTCTTCCAGATATC CTCGCTGGGCGCCAGGGGCTGCAGCTCGCTCTGCTGCTGCTGCTGGTAGAA GTTTTCCTCCTCGTCGCAGTAGAACTACGGCTGCACCGAGTCGTAGTCG | 1346 |
| chr8:128,750.550-128,750.699 | MYC | 5 | 8 | CTAGGGGACAGGGGCGGGGTGGGCAGTAGCTCGAAGTTCTTCCAGATACC CTCGCTGGGCGCCAGGGGCTGCAGCTCGCTCTGCGGCTGCTGCTGGTAGAA GTATTCCTCCTCGTCGCAGTAGAACTACGGCTGCACCGAGTCGTAGTCG | 1347 |
| chr8:128,750,550-128,750,699 | MYC | 7.5 | 11 | CTAGGGGACAGGGGCGGGGTGGGCTGTAGCTCGAAGTTCTTCCAGATACC CTCGCTGGGCGCCAGGCGCTGCAGCTCGCTCTGCGGCTGCTGCTGGTAGAA GTATTCCTCCTCGTCGCAGTAGAACTACGGCTGCAACGAGTCGTAGTCG | 1348 |
| chr8:128.750,550-128.750.699 | MYC | 10 | 15 | CTAGGGGACAAGGGCGGGGTGGGCTGTAGCTCGAAGTTCTTCCAGATACG CTCGCTGGGCGCCAGGCGCTGCAGCTCGCTCTGCGGCTGCTGCAGGTAGAA GTATTCCTCCTCGTCGCAGTAGATCTACGGCTGCAACGAGTCGTAGTCG | 1349 |
| chr8:128,750.550-128,750,699 | MYC | 12.5 | 19 | CTAGGGGACAAGGGCGGCGTGGGCTGTAGCTCGAAGTTCTTTCAGATACG CTCGCTGGGCGCCAGGCGCTGCAGCTCGCTCTGCGGCTGCTGCAGGTAGAA GTATTCCTCCTCGTCGCAGTAGATCTACGGGTGCAACGAGTCGTTGTCG | 1350 |
| chr8:128,750.550-128,750.699 | MYC | 15 | 23 | CTAGGCGACAAGGGCGGCGTGGGCTGTAGCTCGAAGTTCTTTCAGATACGC TCGGTGGGCGCCAGGCGCTGCAGCACGCTCTGCGGCTGCTGCAGGTAGAA GTATTCCTCCTCGTCGCAGTAGATCTACGGGTGCAACGAGTCGCTGTCG | 1351 |
| chr3:187,443,281-187.443,430 | BCL6 | 0 | 0 | TTCTTGGTAATAGGTGAGAAACCCTATCGTTGCAACATCTGTGGGGCCCAG TTCAACCGGCCAGCCAACCTGAAAACCCACACTCGAATTCACTCTGGAGAG AAGCCCTACAAATGCGAAACCTGCGGAGCCAGATTTGTACAGGTGAGC | 1352 |

207

EP 4 334 476 B1

| - | - | - | - | Minus Strand Oligonucleotide | - |
|---|---|---|---|---|---|
| chr3:187,443,281-187,443,430 | BCL6 | 2.5 | 4 | TTCTTGGTAATAGGTGAGAAACCCTAGCGTTGCAACATCTGTGGGGCCCAG TTCAACCGCCCAGCCAACCTGAAAACCCACACTCGAATTCACTCTGGAGAG GAGCCCTACAAATGCGAAACCTGCGGAGGCAGATTTGTACAGGTGAGC | 1353 |
| chr3:187,443,281-187443.430 | BCL6 | 5 | 8 | TTCTTGGTAATAGGTGAGAAACCCTAGCGTTGCAACATCCGTGGGGCCCAG TTCAACCGCCCAGCCAACTTGAAAACCTACACTCGAATTCACTCTGGAGAG GAGCCCTACAAAGGCGAAACCTGCGGAGGCAGATTTGTACAGGTGAGC | 1354 |
| chr3:187,443,281-187,443,430 | BCL6 | 7.5 | 11 | TTCTTGGAAATAGGTGAGAAACCCTAGCGTTGCAACATCCGTGGGGCCCAG TTCAACCGCCCAGCCAACTTGAAAACCTACACTCGAATTCACTCTGGAGAG GAGCCCTAAAAAGGCGAAACCGGCGGAGGCAGATTTGTACAGGTGAGC | 1355 |
| chr3:187,443,281-187443.430 | BCL6 | 10 | 15 | TTCTTGGAAATAGGTGAGAATCCCTAGCGTTGCAACATCCGTGGAGCCCAG TTCAACCGCCCAGCCAACTTGAAAACCTACACTCGAATTCACTCTGGAGAG GAGCCCTAAAAAGGCGTAACCGGCGGAGGCAGACTTGTACAGGTGAGC | 1356 |
| chr3:187,443,281-187,443,430 | BCL6 | 12.5 | 19 | TTCTTGGAAATAGGTGAGAATCCCTAGCGTTGCAACAGCCGTGGAGCCCAG TTCAACCGCCCAGCCAACTTGAAAGCCTACACTCGAATTCACTCTGGAGAG GAGCCCTAAAAAGTCGTAACCGGCGGAGGCAGACTTGTCCAGGTGAGC | 1357 |
| chr3:187,443,281-187443.430 | BCL6 | 15 | 23 | TTCTTGGAAATAGGTGAGAATCCCTAGCGTTGCAACAGCCGTGCAGCCCAG TTCAAGCTCCCAGCCAACTTGAAAGCCTACACTCGAATTCACTCTGGAGAG GTGCCCTAAAAAGTCGTAACCGGCGGAGGCAGACTTGTCCAGGTGAGC | 1358 |

208

EP 4 334 476 B1

Table 6

| Name | Sequence | SEQ ID NOs. |
|------|----------|-------------|
| TNFRSF14_chr1:2488006-2488106 | TCTCTTCTGGCCCACAGCCGCAGCAATGGCGCTGAGTTCCTCTGCTGGAGTTCATCCTGCTAGCTGGGTTCCCGAGCTGCCGGTCTGAGCCTGAGGCATG | 1 |
| TNFRSF14_chr1:2488106-2488206 | GAGCCTCCTGGAGACTGGGGGCCTCCTCCCTGGAGATCCACCCCCAAAACCGACGTCTTGAGGCTGGTGAGCCCCCGAGCCTCCTCTCCGTCTGCTCGCA | 2 |
| TNFRSF14 chr1:2488206-2488306 | GATCCCAGTTCTGACCCCAGGGCCTCCCACAGATCTCTTCCCCATGCCCCTGTCCTGGCCGTTGCTGGCTCCGGCGTCCAGCCCGTCCCCTGCTGCCTGG | 3 |
| CSMD2_chrl:34404022-34404122 | CCATGTTGCTGGCTTACTTGGCATTTCCCATGATCTCACACTGCTGGCTTATTTGGCATTTCCCATGATCCCCTGCTGCTGGTTTACTTGGCATTCCCTA | 4 |
| CSMD2_chr1:34404122-34404222 | TGATCCCATGTTGCTGGTTTACTTAGCATTTCCCATGATCCCATGTTGCTGGCTTACTTGGCATTTCCCATGATACCATGTTGCTGGCTTACTTGGCATT | 5 |
| NEGR1_chr1:72334891-72334991 | ATAGATTAGAGGAAGGAATTCTAGATGAAATTAAGTAAATGAGTTATTTAAGTCAACTAATACAAGTCCTCAAAACTTTGATTATATAGAGAGCTAAACT | 6 |
| NEGR1_chr1:72334991-72335091 | GATAAATATAGACAAATATAGTGAGCCTATAAATTAAAGCTATACTATGATGAAAAAATAAATGAATAATTGTGAAATAGCCAAAAATACTAAAATACAG | |
| NEGR1_chr1:72335051-72335151 | AATGAATAATTGTGAAATAGCCAAAAATACTAAAATACAGCTATAAGGTTAAAAATAAATCTGAATAAAAAATGTAGGAGGGAAAAGTGATTACCTTACC | 8 |
| BCL10_chr1:85733207-85733307 | GACATGCATCAAATGTAAACAAATGATTACAGCCATTTTATAAAAAGTCATATTCTTTAAAACATTTTTTGTCATCATTAAAAATTAAAAGGCAATAAAG | 9 |
| BCL10_chr1:85733307-85733407 | TGTCATTGTCGTGAAACAGTACGTGATCTTAAGGGAAGAAACATCTCACTAGAGTTTGCACAAGTTCCTTCTTCTTCTAACTGTAGATCTGGTGGCAAAG | 10 |
| BCL10_chr1:85733407-85733507 | GAGGAGCCCCTGGGTCCCCAGGTCTGGGAAGTGTAGTTGAAGAGAAGATGGTATTTTCAGTTCTGCCTACTTCTAGAACAGGCAAATTCAGAGAAGAATT | 11 |
| BCL10_chr1:85733507-85733607 | AGTAGAAAAAAAGGGCGTCGTGCTGGATTCTCCTTCTGGATGGTACATGACAGTGGATGCCCTCAGTTTTTCAGAGAAATTACTCTCATCTGAATTTGAT | 12 |

EP 4 334 476 B1

209

| Name | Sequence | SEQ ID NOs. |
|------|----------|-------------|
| BCL10_chr1:85733607-85733707 | CTGGAGAGGTTGTTCGTGGCTCCATCTGGAAAAGGTTCACAACTGCTACATTTTAGTCCTACAATAAAATTATTCAGATGTAAATGAAAAAGTAACTAAA | 13 |
| BTG2_chr1:203274697-203274797 | ACCCGAGACCTCTCACTGAGCCCGAGCCGCGCGCGACATGAGCCACGGGAAGGGAACCGACATGCTCCCGGAGATCGCCGCCGCCGTGGGCTTCCTCTCC | 14 |
| BTG2_chr1:203274797-203274897 | AGCCTCCTGAGGACCCGGGGCTGCGTGAGCGAGCAGAGGCTTAAGGTCTTCAGCGGGGCGCTCCAGGAGGCACTCACAGGTGAGCGCATGCCGAGGGGCC | 15 |
| BTG2_chr1:203274897-203274997 | TGGCGCCACCGGGGGTCGGCCCCATCCCTGCCAGGGCCGTCTTTCTTCTACTCCTGCGGCAGGGTGACCCACGGGAGCAGCTTTGGGACTCGGTGGCCCT | 16 |
| BTG2_chr1:203274997-203275097 | CCTCCGACCCCCGGGGCGGCCCGCAGTCCCCAGTTTCCTGGGTCCTCCTCCCCAGCCCTGTGCTCGGGTCTCGGCCGTGGCGGTTCTGATGGGGCGCGCC | 17 |
| BTG2_chr1:203275097-203275197 | CCTCTACGCTCTCGGAGGCGCAGACCCTGGTCCTGGAGTGCCAGCCCGAGTCCCCAGCTTATGCCCCTGTCTCATTACGGGCTCGTCTCCCTCGCTGGAC | 18 |
| BTG2 chr1:203275197-203275297 | CCTCGAGATCTTAAGACCCTCGATGGATGTTGTTGCGGGCCGCCCGGTCGGCCGAGGGGTCCCGATGAGGGAAGAAGGTGCAGTCGAGCCTTTTCAACAA | 19 |
| BTG2_chr1:203275297-203275397 | TTTGGAGTCCCAGTGCGGTTCTTCCTGCCGGTCGGGGTGCGCTGTGCCTGGGGTAGTCCACTGGTTGCTGACTGGCTTCAAGTTGGAATTTGGGCCCCCT | 20 |
| BTG2_chr1:203275397-203275497 | TTGTGTTATCTTTGGTTCCCCTTAGCCATCTGCCACCTATTGTGGTAGGGAGGAGAGCCTCGTAGCTCGTGACCCTGCCGTGCGGGCCTTCAAGTTGGGA | 21 |
| BTG2_chr1:203275497-203275597 | GGTGAAGAGATAAGCAGCCCGCTCGCTGGCTGGGGAGAGACCTCTCTCCCAGCTGTTTCTAGCTGGTTACTGTCAGTTTTGGGAAGCGATAGCCATCTCG | 22 |
| BTG2_chr1:203275597-203275697 | GAACGCACCCACACAGACCCTGCCTTCTGAGGAAAACAGATGTTTCATCAAAACAACCCAGTTTTCACTCCCTTAGGCACTGCTAAGGAAGGTTCTCTGA | 23 |
| BTG2_chr1:203275697-203275797 | CTCTTCTGAAGGAAGCAGAGGGAACACAGGGTGGGAGGTCCAGTGACTTGCTGTGGACCCAACAATGTTGGCAGCCTTCCTGGCCCTGAAACTTCAGCTC | 24 |

| Name | Sequence | SEQ ID NOs. |
|---|---|---|
| BTG2_chr1:203275797-203275897 | ACAGGTCTCCAGAGGCCCTGCCTGGACATGCCAGTCCCAGTCACACCCTTCCCTTGCTTTGGGGGTGTGCC AAAAGCAATACACTGGCCACTAGAGAGTA | 25 |
| BTG2_chr1:203275897-203275997 | CCCTAGAGCTCTAGAATCCCTCCCAACACGCACACACACACACACACACTCTCTCTCACACACACA CACTCAGTCACACACACACACACACAC | 26 |
| ITPKB_chr1:226923691-226923791 | CTTTCAGATCTTTCGCAGCGTCCCAACAGGGCAAAGGCTCCAGCATTCTGCCAGAAGGAATTCCCGCCTCC ACATTCCCGGTCCCCGGCTGTGCTGAGGG | 27 |
| ITPKB_chr1:226923791-226923891 | GCTGCCCCCAAGCAAGCCCAGCGTTGGGGACCCTCCCTCCACTCTGTCGGAGAGCTGCCAACGCCCCCCG CCCACGGGGGCCCCACTTCGGGCCTCCTCA | 28 |
| ITPKB_chr1:226923891-226923991 | GGGCCTACGGAGGCCAGGGCCCTGGGCAGCCTGGACCAGCTCAGGGAATCAGAGGACTCTGCGCTTTGC ACGCTCACAGTCGTCTCCTCTGGCCTTTTGC | 29 |
| ITPKB_chr1:226923991-226924091 | CCACTTCAGGCTCCCCAGAGCCCGGCATGCCACAGGGCAGATATCCTTTCCCCATCTTCCCAGGGGGTTCT CCATCGCGGGGCCCGCCCCTTTCTGGGGC | 30 |
| ITPKB_chr1:226924091-226924191 | TGGGCTTGTCTCACTGCCCAGAAACTGCCCCTGCCTCTCCACCAGGGCCTCTGGGGGCTGCAGGTCCTCAA GCTCACGGGCTCTCCCAGACGGCTCAGTG | 31 |
| ITPKB_chr1:226924191-226924291 | AGGGCAAGATCCTGTGGACGGTGTGGCCCAGTGGATGTAACTCTCGCTGCCACTTCCGTGGCCATCGTTA AGCTAGCTCCGAACAGCCCCAATGAGGGAG | 32 |
| ITPKB_chr1:226924291-226924391 | CTAGGCAGCTCCGAGTTCCCGGGGTAGGAGAGCCCCTTTTGTCAATTTCCATAGCTGTGGGTGAGCCACA GCGGGGACTGGCAGGGATACCCTTCTCCAT | 33 |
| ITPKB_chr1:226924391-226924491 | CCTTACAAAAGCGGATGGACCCTGAGCCTCTGATCCTGTAGGGGCAGCCCGGCCGGGAAGAGGTGGCATT CCTTTCTTCACCTGCGAGGAGCATAGGCTG | 34 |
| ITPKBchr1:226924491-226924591 | GGCCCTCCTTTCCTCCCGGAGTCGGTTCCTGAAGTCTCTGGACATTGCTCCCCCCAGGACTTTGTCCTCCG TTCCTCGCTCCGGGCGCCCTGAACCAGGA | 35 |
| ITPKB_chr1:226924591-226924691 | CCCTTCCAGGGGGGCTGACTGCTGCTGCGGAAGGGGCACGGGGAGGGCGAGCGAGCCCTGCCCAAACGCG GGCTGCGGGGCGCTTGAATGGCGGAGCTCTG | 36 |

EP 4 334 476 B1

211

| Name | Sequence | SEQ ID NOs. |
|---|---|---|
| ITPKB_chr1:226924691-226924791 | TGCCTGGATGTGCGCCTCAAACATGCCCACTTTCTGGTTCACCTGCACGTTCTGCAACTCGCGCTGCAAGATCCGCAGCTTCCTCTTGGCCTCCTCCGGC | 37 |
| ITPKB_chr1:226924791-226924891 | CCTGGCGGGGAGAGGGTACCGGCTGCCACCACCTGCTGCCGGTCCCCTCGCAGGCGACCAGCCCAACTTGGGCTGCTCACGCTACTGCCGCTGCTGCCGC | 38 |
| ITPKB_chr1:226924891-226924991 | TGCCACTGCCGCTGCTACTATTCAGCCTGCGCCGGCCGCTCCGCCAGCCCCCGGGGCTCCGGGGCTCCTCGGGGGACAGCGACTCGGCTGGGGGGAAGAG | 39 |
| ITPKB_chr1:226924991-226925091 | GAAAGAGGCGCCTCTCCCGGGGCTGAAAACGCTGCCGGGGCTCAGCACTGCCCTCCTCGGGGGCGGGGGCGTCTCGCTGCCACTGGGCCCCGGGCCGCCG | 40 |
| ITPKB_chr1:226925091-226925191 | CCGCTCTTCATCTCGTTGGCGCTATTCATGATCACCAGGCTATTGAGCGCATAGCAGTACACAGCCATAGTACTGGGTCCCGCGCTGCCCGCCGCCGCGG | 41 |
| ITPKB_chr1:226925191-226925291 | CTCCCGCTCCTGCTCCGCCGCCGGCGCCTCCTCCTCCCGGCGCTCCCGGCTCAGCCCCGGAGGCCCGGCAGCCGCGGCTCCGCGCGCAGATGGGGCGGCA | 42 |
| SLC1A4_chr2:65258145-65258245 | AAGTGCGAAGGAAGTGTCAGGCTGGATGTCAAAATGAACACCTTGGAGAACTGGATGATGGAACAGACGGTAAAAATCAGCTAAACATCAGAGAAAATGG | 43 |
| SLC1A4_chr2:65258245-65258345 | AGGAAGAGGTCAAAACTGTGAACAGGAACTAGAAGAAAGTGTAGCAGAAAAAGACTTGTCACAAACTTCGAGAGATTTGGAGAAAATGATGTCAAAACAC | 44 |
| SLC1A4_chr2:65258345-65258445 | ATCTTCCTCAAGCCCATGCTGAGTATCTCTGATTTGGTTAATTTCTTGGTAAGTGTTCCAAGTACAGACAACAAAGCAGAAAAGCACTGATTACAGGGAA | 45 |
| SPRED2_chr2:65593035-65593135 | TATGCAGAATGATCCTTCAGATCATGTGAACGCTATAATTAAATGTTGCTACCAAATCCCCACTACCCTTTCTCCCACCTAGAAAAAGTTAATGCATGAA | 46 |
| SPRED2_chr2:65593135-65593235 | TTCAGTATGAGCAAATTGTGATTTATAAAAACAAACAAACAAACAAACAAAACCCACCCTATTCACTCCGTAGGGGAATAAAGCTTTCTTGCATTA | 47 |
| SPRED2_chr2:65593 180-65593280 | AACAAACAAAACCCACCCTATTCACTCCGTAGGGGAATAAAGCTTTCTTGCATTAAGTCACGCATCATGGGGGTAGGAAAAAAGCACAGTACTGAAAGAA | 48 |

212

EP 4 334 476 B1

| Name | Sequence | SEQ ID NOs. |
|---|---|---|
| EIF2AK3_chr2:38906681-88906781 | GTGAAGTGATCCAAATGTAGCCCAGAGATCCTAAAGAAAAAACGATGCTCATGTGTTACAAAACAAAATTTTAAGGCAATCAGTGAGGAATCACAGACAA | 49 |
| EIF2AK3_chr2:88906781-88906881 | ATTTCCTTAGTGCTTTTATCAAGGTTGAATCTGAATATAAATTACTAGAGGAAAGCAAATCAGATTTCACATCTGAAAATTAAAAACAAAATTCTTAGCT | 50 |
| IGKC_chr2:89127261-89127361 | AGGCAACAAAATGAGATCCTGTCCCTAGAAAACATTTCAAAAAATTAACAGCATGGTGACGCACACTTGTAGCCCTAGCTACTTGGGAGGCTGAGTGGGA | 51 |
| IGKC_chr2:89127461-89127561 | AAGAACTTAAGCAGACTAGGATATAAAGTATAGGAGCGTATTGTGTACAGGAACGGGAAATACTGTTTCCTGGATCTTTTGTTTCACTTACGCACACACC | 52 |
| IGKC_chr2:89127561-89127661 | CACACCCGCCAGTAGTGTACCAGGTTGCGATGGAAATCTCTCTCTTTCTGTGGATGAGTTTGTGGAAGCCCTTGCTCCAGCATGCCCTCCTTCCTGCCCA | 53 |
| IGKC_chr2:89127001-89127761 | CCCCTGGACCATTCCTTCCCTTCACAGCACTGTCCCATGGGTAGGCCACAGCCCAGCACAGGCCCCAGCCTGGCGGCTGCAGCAGGAGCCCCATCCCAGG | 54 |
| IGKC_chr2:89127761-89127861 | GCCTGAGGGGCCATGCGGGGGTCTGGGTGGGAGTGGGAACCGCTGAGGAAGGTGAAGGGAAATATGGTGAGATGACAGGCCCGCTGTCAGGGAGAGTGGG | 55 |
| IGKC_chr2:89127861-89127961 | AGGAGCCCTGGAGTGCCCTACCTCTGTGGGGCTGGAACTCCCTGTATCCGAGCTAGGGTCTTCCACACGCATGCTACTACCCCAAGTGCCACAGCTGGAG | 56 |
| IGKC chr2:89128431-89128531 | TCATCTCCCACTGGATAACAGTGTTGTCGGGAACTTCCATCCAGCACTGGCGGACACTCCCGTCGCAGCTGCTCCTGACTGAGCAAGTCATTTAAGGGGG | 57 |
| IGKC_chr2:89128531-89128631 | TCCTTGGCACTCATAAGCACTCACAGAATGGGGCTGGCAGTGCGCCCGGCCTCCCTGGGATGGGTCCAGAATGGTAGGAAGCGCAGTCCGGGAGGGACCC | 58 |
| IGKC_chr2:89131726-89131826 | ACTGCTTAGAGCTCTCAGCCCTAGATGGCGTATCACAGTTAATGCTCTATAAAACCCATCATGGCTTTTCCCTAGTAAGCCTCAAATCGCTGCAAGCAAG | 59 |
| IGKC_chr2:89131826-89131926 | GCTTCATATATGAGAGTTTCTGCTGTCTCCTGGAGCCATCTCACCCAAAGCCACTGACTCTGGGAGACCAGCCCAGGCCACAAACCAGCAAAGCACCAGT | 60 |
| IGKC_chr2:89131926-89132026 | TATAGTTAGAGCTGCATTATAAAGTGGCCAGAGGACATTTCTTTGCAGTGAGATGTGTATCGTGAACGTTTGGGGCCTGTGCTCGCCTAGTCCTCATCTT | 61 |

213

EP 4 334 476 B1

| Name | Sequence | SEQ ID NOs. |
|---|---|---|
| IGKC_chr2:89132026-89132126 | TGCTTTTCTAGGTACACAAAGCCATCCCATGGCTGCAAATGTTAGCTGGGCTGGGCTCCCTACTTGCCTCA AGCCCCTTCATAGACCCTTCAGGCACATG | 62 |
| IGKC_chr2:89132126-89132226 | CTTTTCTCTGGACGTTTACAGACAGGTCCTCAGAGGTCAGAGCAGGTTGTCCTAGGGAGCAGGGAGGCTT CCTAGGGAGGTCAGACTCCAAATAGTGGAT | 63 |
| IGKC_chr2:89132226-89132326 | ATGGCAAAAATGCAGCTGCAGACTCATGAGGAGTCGCCCTGGGCTGCCACTAGGGCTCCCACAGTGTGCG CTGCCAACCTGCTGCCCGTGCAGAAACTCT | 64 |
| IGKC_chr2:89140556-89140656 | CAACTGTGCCCTGCACTGTTAGGGCCCTTGTCAAAACAACACATTTCTCAGTGATTCTGAGACTCTTTCTC TTATCTATAGAAGTCATAACTCAAGAGTA | 65 |
| IGKC_chr2:89140656-89140756 | AAATCATACCAATATTTTACATAAACCCTAGAATTTTTATAGATCTATTATTTCTTTTTAGAGTACATATTG GAAGTAACTTCACAAGGAACATTTTCTT | 66 |
| IGKC_chr2:89140886-89140986 | TCTGGTCAAACCACTCCACAAATAAAGTGGACTGATCCTCTTGACTCTATGTGTAAGTGCCCATTGTGTGT GCACAGAGCTGGTGAGAACGGCCATGGTG | 67 |
| IGKC_chr2:89140986-89141086 | CTAGGTGGGGGTGGTGTTGGTGGAGTTGGACTAGATTATCTGGGATCATGCGAAATGGAAATTCATTTCT AGCTGGCTGGCTTCAGAAGGTGCCATCTCC | 68 |
| IGKC_chr2:89141086-89141186 | TATTTTTATATGAAGCGTGCTTTGGAACTCAGGGCAACGAAGGGTGGGTGTGCTGCACAAGGACAGCAGA AGAGTGAGCTGACTGGTCCCTGAAATCGCA | 69 |
| IGKC_chr2:89141186-89141286 | GTTGGAAAGTGGATTACCAGTGCAGTAGAACTCTTCACGGAGGCCTGGACCATCAGGTCTAATGGTGTTG TTCCAGGTGGGTGGTCATGTGGAGCAAAAA | 70 |
| IGKC_chr2:89141286-89141386 | TATTTGAAATCAGCGAGCACGTACCTGAGAGATGACTTTTCCACTTGGGCTAGTCTCTTGATATTTCTGGT CCTGTTTCTTCATCTGTAAACTGGGTTAG | 71 |
| IGKC_chr2:89157326-89157426 | AAGGAGACCAAGAAGCGTATTTAAAATCTTGATGTTTTGAGTTTCTTCCTAGCTTCCCCCTATTCCTTAAT AAAGTTCTAAATTGTTTTGTTGGAGCTCT | 72 |
| IGKCchr2:89157426-89157526 | TTGCAGCCATTCTGAGGGCTTTGCATGCTTTTCTGACCTTGCAGTAAACTCAATGCTTTAGGCAAAGAATG GCCACGTCATCCGACCCCCTCAGAGTTTA | 73 |
| IGKC_chr2:89157526-89157626 | GAATTCAGAACAGGTCTGAAGAAGACCAGGCAGCGGCTGAGTCAAGGAAAGCCTCCGTCCGCTTTTATTT CCCCTGTGCCTCTTCCAGGACTGTGCTGGG | 74 |

| Name | Sequence | SEQ ID NOs. |
|---|---|---|
| IGKC_chr2:89157626-89157726 | ATAACAGGCTCCCGGGGGGTTACTTTGGCTGGGCTGGGCTAAAACCTCCCTGCAGAGCAGGCCCTGAGCCCTGCCTCTGCGCCTGGGTGGTGTCAGCCCCT | 75 |
| IGKC chr2:89157726-89157826 | CCACCTTCTGACTGTTCCAGCAACTCTCTAAGCCCTCCCAAAGGCCTCAAGGCCTGTAACCATATGCAGCAATTTTCAGCCATACCAGGAGAGGTCAACT | 76 |
| IGKC_chr2:89157826-89157926 | GTAATCTTGGCCACCTGCCTAAGAGGAAGTGGCTAGCTTCACTTCTGACCCTCAGCAACTGCCAGGTGGCCTCTTGGAAATCCCCCTCTGGGGGATTCCA | 77 |
| IGKC_chr2:89157926-89158026 | CCCGTTGGGTGGGAGAGCAGTAGTTAAAATGTAAAATAAGAATCTTTTGCTGGGAGAAGTCAACAGATAGGGAGAAGTCAGCTGATAACAGAAATAGTTT | 78 |
| IGKC_chr2:89158036-89158136 | TAAAACTAACTTCACTGTTAACCAAGCAGTTCAACATGAAAGACTGAATCTCTTATGTTTAATATTTTCTTCTCTTTTAATCTTCATAACTAATTTTTTT | 79 |
| IGKCchr2:89158136-89158236 | CAGATAATTGTATAAAATAACCATGGTAGCAAAATAATGTGATCACTGGAAAATAAGCAGGGAAAAACATGCTATGAAGATACTCCTATCTGGGTGAATT | 80 |
| IGKC_chr2:89158236-89158336 | CTTGATAGCTTTACATTTTTCATCTGGCATTTAAACATTAAACAGTTAATGTATTTGACATGAAAATTATTTCAAGTTATCTTATTAGTTTTAATAGAGT | 81 |
| IGKC_chr2:89158336-89158436 | TTAAAAAGTGTTTAAAAGAGTTTTCAAAAGGCTCTAAAATCATTTTGAAATAGTTTAAAACAGTTTTGAATCGTTGTAAGTTAGTTTTAATAGAGCTTTA | 82 |
| IGKC_chr2:89158436-89158536 | AAAAGGCCCTAAAATAGTCCTATCAAGTTGTTGCAGACCAAAATAATCTCCTTAAATATCACTTTTGAGATCAGCTGGGGTAAACGACAGCAACACAATG | 83 |
| IGKC chr2:89158536-89158636 | ACAAATCATTAAACTATTTTAGAGATTATGAAATTAAAATACTCAGATTAAAATTTTCCTATCACAGAATTAAGGTACTGGAAAATATGTTTAAGTTTTT | 84 |
| IGKJ5_chr:89158636-89158736 | ATTAATCACATTGCTATAGGTTTAGATATTTTGTACAACTGAAATAAAATCACACACTGGCAGCTACATTTTTGAAAGTTAAAAACATGGTCACGAATAT | 85 |
| IGKJ5_chr2:89158736-89158836 | ATCTTATTTTAAAATCAGTTAATATACCTTAATGGTATTTAATGCCAAATTCAAAGTGAATTGATCAAGCCCTCAGTGGCCAGGTCATGGGTGTGATTTT | 86 |
| IGKJ5_chr2:89158836-89158936 | TACTCTGAAAGAATTACATATTTCTTTCTTTTTGGTTGAGCTTTTGTTATTTAAATACATTTGATGAGAGGATATTGAAATAATTAAATAGCACTGAAAA | 87 |

EP 4 334 476 B1

215

| Name | Sequence | SEQ ID NOs. |
|---|---|---|
| IGKJ5_chr2:89158936-89159036 | AAAAAAAGCTTTAAATTATTTACAATCCCCTAATGGAAATTTTCACTAATGAGATATCATAATGAATGTGA ATTTTATTTCTGAAATCTCTAATAAATCA | 88 |
| IGKJ5_chr2:89158941-89159041 | AAGCTTTAAATTATTTACAATCCCCTAATGGAAATTTTCACTAATGAGATATCATAATGAATGTGAATTTT ATTTCTGAAATCTCTAATAAATCAGTCTT | 89 |
| IGKJ5_chr2:89159041-89159141 | CTCCCTGGTTTTCCCAGCTCAGCGCCCATTACGTTTCTGTTCTCTTTCCCTTAGTGGCATTATTTGTATCAC TGTGCATCAGGAAAGCTGGCTACGGCAG | 90 |
| IGKJ5_chr2:89159141-89159241 | CATCAATCGGGCAGACACAGGGTGGCCACGGCCACTAGCGGCAAGGCGGCTGCCCCAAGAGCGCGGTGG CATGGCCACCAAAGCCACTCAATCGAGAAAG | 91 |
| IGKJ5_chr2:89159241-89159341 | ACCGCGGCTCTGTCTACAGCTCGCGGTGCCACGGCCTTCTTGGCAGAATAAAAATGTAGACAAGTAATAA CAGAGGATAATGAAAGAACATACTCTTTAA | 92 |
| IGKJ5_chr2:89159341-89159441 | AATATTTCCTATTTTTTTCACAGACCCACGGTCATTAAAAAATGCAATTATTTACTTTTTTTCATTTAAACA CATTTCTTTGAGATTGAGCTTTGGGAA | 93 |
| IGKJ5_chr2:89159441-89159541 | TAACCACCTTTCCACCATTACAATAAGAGATAATTTCACGTTTAGTCTAATGTACAAATTGGATTTTTAAA AAATGAGCTCTATCTGTGAAGCCCTTATT | 94 |
| IGKJ5 chr2:89159511-89159611 | AAAATGAGCTCTATCTGTGAAGCCCTTATTCCTATAGAATGTGTCTTTTTGAGTTTATTACTTATTACAGA CTCTAAAAACAACATTGCTGCTGATTTTC | 95 |
| IGKJ5_chr2:89159611-89159711 | AAGTAAGCTGCCTCTTCTACATAGCAAATAGGTACACTTCACTTTTCCCTGATTTTTCTTAGGGCGTGCTA TTGATTTTTATTGTTGTCTGACAAAATAA | 96 |
| IGKJ5_chr2:8959711-89159811 | TTTATCAAACAAAAGGGAGAAAGACTAAAAAATGTATTTTTCCACTTTTCTGTATCATGCATAATCAGCAA CAACCAATACAATATTTGGCAAGAGTGAA | 97 |
| IGKJ5_chr2:89159811-89159911 | CAAAAATAAATTTACTTTTGCTCCTTAGAAATACAAGGGTTCCTTTTTAGTTACACTTTTTTTTTTTACTTT GTGTCATTCAGTTTAGAGCAATTTAATC | 98 |
| IGKJ5_chr2:89159911-89160011 | TTTTTTTCTCCAAATCCATTTTTGAAGCTGAGTTTAACTTTTGCAACCCATGGCAAATCTTAAATGCCCTCA TTTACCAATCTTTACCAAACTCCTATTT | 99 |
| IGKJ5_chr2:89160011-89160111 | AAGCCTCTAAAAGTCAATACTGGCCATCAGACCCAAATTTCAGAAGACAATAGTGAAAAATTACTTACGT TTAATCTCCAGTCGTGTCCCTTGGCCGAAG | 100 |

EP 4 334 476 B1

| Name | Sequence | SEQ ID NOs. |
|---|---|---|
| IGKJ5_chr2:89160111-89160211 | GTGATCCACAGTGTTAACTTAATTACTTTCCCCTTAACAAAAATCTCTTTTCGCTGTTAATATCACTAACCTGACCGATGCAGAGAAAATCTTGCAATTG | 101 |
| IGKJ4_chr2:89160211-89160311 | AGATGCCTCACTTAACTGGCTAGCGCTTGGCTGTTCCTTAAGATGAACTAATTTTCTATCCCTTACTCATCTGACTTTTTGAAAGAATCTGGTACTCTTT | 102 |
| IGK14_chr2:89160311-89160411 | GGAATTGACCTGAGCTAATATCTCAAACACAAAAACGCTCCAAATTTAAAACCTTATAAGAAAAAGCATTAGGAAAGTGCACTTACGTTTGATCTCCACC | 103 |
| IGKJ4_chr:89160411-89160511 | TTGGTCCCTCCGCCGAAAGTGAGCCACAGTGAGGGATCTCACCCTTTCCCCTCAACAAAAACCTCTCTTGAAGCCAATCATATGAGATAGGCTGCTTGTT | 104 |
| IGKJ4_chr2:89160511-89160611 | CAGAGAAAAATCTAGCTATTTCTTCCCCATTTCCCCCATGAATCCTATTCTCCTCTCAAACCCAATGATTCGTCTATTTGCTCAGCTTTTTAAGTTCATT | 105 |
| IGKJ3_chr2:89160611-89160711 | TTCTGGTGTCCTGCTATTTACTTCTGGGTCACCAGGTTTATTCAACCAAAATATCACAAAACTTGCACAAATGATACAATGGCACTAAAATCTCACGAAT | 106 |
| IGKJ3_chr2:89160711-89160811 | AATTGAGACAGATGTACTTACGTTTGATATCCACTTTGGTCCCAGGGCCGAAAGTGAATCACAGTGATTCGTCTTAACTTTTCCCTTTACAAAAACCTCC | 107 |
| IGKJ3_chr2:89160811-89160911 | CTGAAAGCTCAGCAAGCCTCTTTCCCCCAATGAAGTTATTTTGATTTAGAAATCTTAAAAATTAGCCACAAGCTAGCGTCCTGTGGAACAATTTCCCCTC | 108 |
| IGKJ2_chf2:89160911-89161011 | CTCTGTACCTAACCTGGGAATGAAGTTTGTTAGATCCCTGGCATCCGACTAATGAAAATCCACACAAAGGAACACAAAGTAAACTAATTAGCAACAGTGA | 109 |
| IGKJ2_chr2:89161011-89161111 | AGAATCAGTGGAAAAAGTACTTACGTTTGATCTCCAGCTTGGTCCCCTGGCCAAAAGTGTACACACAATGGTTCCTCTTAACTTCCCTCCTATACAAA | 110 |
| IGKJ2_chr2:89161111-89161211 | ACTCCCTTTCTGACAATTGACCAAGGCTCTGTCCAGAACATGTTATGTTCCCCAGGACATTTCTGAAGCTATTACTTAGACAAGTTATTCTCACCCAATG | 111 |
| IGKJ1_chr2:89161211-89161311 | ACTGAATCTTGCTTGCTCTTCAAAGAAAATGTGCAATCAATTCTCGAGTTTGACTACAGACTTATCTTTATCTTTTCCCTGAAGGATATCAGAGGCTGAT | 112 |
| IGKJ1_chr2:89161311-89161411 | TGCAGAGTCACCTTATAGATCACTTCATAGACACAGGGAACAGAAGACACAGACAACTGAGGAAGCAAAGTTTAAATTCTACTCACGTTTGATTTCCACC | 113 |

217

| Name | Sequence | SEQ ID NOs. |
|---|---|---|
| IGKJ1_chr2:89161411-89161511 | TTGGTCCCTTGGCCGAACGTCCACCACAGTGAGAGCTCTCCATTGTCTTGCTGAACAAAAACCCTTCTCACCAAAGGGGAACAGAGTCCTGGGTCAGCTG | 114 |
| IGKJ1_chr2:89161926-89162026 | ATCAACTTAAGGCTCATAACTTTGAAATGCATTTTGAAATGTAGCTCCAGATGGTATACGAAACCAAAGTGAAGACTAATAGAGTAGAAAAGTAGACTTT | 115 |
| IGKJ1_chr2:89162026-89162126 | ACTTGGTTGGTTTGTCTGTTTTCACAGCACAGGAAGAGCTCAGCTCTTACTGAGCTGGACCAGGCGCATGCCATCTTTGGAGCTGCCATGGAGTCCCAGT | 116 |
| IGKJ1_chr2:89162126-89162226 | GTTCCATAGTGTTTCCATAGTAATCTCATCAACAACACTGAAGACCTTTTCAGTATTTTCTTTTGAGTCCAGCTCCATTTTTGCAGCCTTGTATCTCTCT | 117 |
| IGKJ1_chr2:89162776-89162876 | CCGCGCCCAGCCGAGTGCCTGTTTATTTTTACCTGCTTTCAGATTCTCTTCTACCCTTCTAAATTATAAGCTGTTTGATGTTTATTTGCCCTGTATTTG | 118 |
| IGKJ1_chr2:89162876-89162976 | GGAGGCTCCGTCCAGTATCTTTACTTAGCAAATGCTTAACAAACATTTTCAGAATAAATAAAAAAAAATACCTAATTGAAAGTCAATAATAGATCAGAGA | 119 |
| IGKJ1_chr2:89162976-89163076 | TGCTATCATAGACCAAAGACTAATACTGACTGCCACAACAGTAACTTTTACAACAGAAATCATAACTACAATTCTAAAGATTAGGGGTAGGTTTATTTGA | 120 |
| IGKJ1_chr2:89163076-89163176 | TTCTGTCACTGGCAGCTTTGCTAGTTGCCTTGAATAGCAGAATTAGCATTTGGTCTCACCAGAAGATGAGGAAGGAGAGGGATCAAGTTAGAGGTGGAGA | 121 |
| IGKJ1chr2:89163176-89163276 | GTTAACATTGGCAAGTGAAATTTAATGTGCAAAATAGCTGACCAAGGGCATAGTCCTTTTTTAAAGGGGACACAAAGTGATTTTCTCTGCAGACATACAC | 122 |
| - IGKJ1_chr2:89163276-89163376 | GCAATACCAATCATAAAGGGTGACATTTATTGAGCACTTACTAAGTGCCAGACATTGTACATGGATCATCACATTTAATTATTCCCAAGACTCTATGAAC | 123 |
| IGKJ1_chr2:89163306-89163406 | TGAGCACTTACTAAGTGCCAGACATTGTACATGGATCATCACATTTAATTATTCCCAAGACTCTATGAACTAGGAACTAATATTATCCCCTACTTTGTAG | 124 |
| IGKJ1_chr2:89163406-89163506 | GTGCAAAAACTTGAGGGCAGAGAGGTCAAGGAACTGGCTTATGGCAGTAAGTGGCAGAGCTGTGACCTAAACTCAGATCCCATGTTTTTAACTGAACTAT | 125 |
| IGK J 1 chr2:89163506-89163606 | ATGCAGATTATACTCCAGGAGTAAAGTCACTCAACGGAAGCAACAAGCGTGACAGGGAATGCTGGGATGGGGGAAGGTAAAAGGAACTCCTTAGACTGGG | 126 |

EP 4 334 476 B1

| Name | Sequence | SEQ ID NOs. |
|---|---|---|
| IGKJ1_chr2:89163606-89163706 | ATAAGTGTGTACAGACGTATGTATAAGACTACACATGGAAATATTGTTTAAAGAGTGAAAAATAACTAAA ATCCTCATTAATAGGAGTTTGGTTAAACTG | 127 |
| IGKJ1_chr2:89163706-89163806 | TGCTAGAGCTTTACAATGTAGCACAAAGCAGACATTAAGGGGAAGACGTAGACTTCTATATAGTTACGTG GAAGGTGTTTGTGAAAATGCAGGTCACTGA | 128 |
| IGKJ1_chr2:89163806-89163906 | AGAGTATGTGTGGTGAGATATCATGATCCCATCTACATTGAATATATATGTATATAAATACGGGCTGAAT TTTAAAGACATAAATTGTGCTTGGTAGTT | 129 |
| IGKJ1_chr2:89163861-89163961 | AAATACGGGCTGAATTTTAAAAGACATAAATTGTGCTTGGTAGTTATCTCCTGGGATTGCAGAGGAGGAA CAATGACACTTTATGCCATCTCCTCCTACT | 130 |
| IGKJ1_chf1:89163961-89164061 | CTTCTGTATGGTGATGTGAATATATTCATTTTATAGTTTTTAGAAATAATAAAACTGTACTAATTTTGAAA AACAGTAAACTCTGACATTGCCTATTAGC | 131 |
| IGKJ1_chr2:89164061-89164161 | ATTCTCGATATTCCTGTGCAATGCATAAACATAACTTTTTAAAAGATATGTACACACATGTGTGAGTTTTC TTTGTCAAATACTTTTCTATAATCTTTAA | 132 |
| IGKJI chr2:89164161-89164261 | ATCAAGCATGCCAAAAAGGTAAAAGCTTTCCTGTTTCAGTGTAGGAGATAGTCGTCTGCAAAGGAAAGAG ATGTAGGGGATAGAAACAGGAATGAAAAAG | 133 |
| IGKJ1_chr2:89164261-89164361 | ATGACTGAGCTGTTCGAGGGACTTATGTTCCTAAGTGAGCTAATTGGAAATCTAATATGAACAGTGCAAC CGAATAACTATTGTAAAGCAGTATTTGTAA | 134 |
| IGKJ1_chr2:89164361-89164461 | ACAATAAAGATGATTATCATAAGTACCATTGTTGCAAAAACTATTTTATTGATCACATGCAGTGGTGATC TGTAGGAATGATTGTTGTGATGTTTGCTG | 135 |
| IGKJ1_chr2:89164461-89164561 | TAACATAAAATGAAACATGGGAAGTGGCTGAGATCTTTAGGATGTGTGTGGTTCATTTTTTGAAAGCAAA TGTTGTCTCAGAAGCATCTGTGAGACTCTG | 136 |
| IGKJ1-chr2:89164561-89164661 | CCAGGATCCACCGTTCTACAAAATATCTGTGATGGACATTGATAAGATTGATCTGTTGAGGAAAGGCAAG GTGTCAGTAAGATAGTCTGAGAGCTTCTTG | 137 |
| IGKJ1_chr2:89164661-89164761 | GATTTCATGTAAAAGAGTGCTGGAAATAGAATTTCTTGGGGAACATTCCAACTAACTCATCACTGAAGGT GCTTTACATTGAACCCTCAGCAAAGTTAGA | 138 |
| IGKJ1_chr2:89164761-89164861 | TTATCAGAAAAAAAATATAAACTGCTGTGGAGGGGACAGGAAGGAAAGTCAGGGAGGGAGGGGGGCAA GGAGAGAAAGAGCGAGAGAGAGGAGAGAAAGA | 139 |

(continued)

| Name | Sequence | SEQ ID NOs. |
|------|----------|-------------|
| IGKJ1_chr2:89164866-89164966 | AGAGAGGAGAGAGAGAGCACAAGTACACACTTCAATGCACATCTATAAATCATCCTGAAAACTACTGATA AATTATTTTAGCAATGTTCCTCAGATGTAA | 140 |
| IGKJ1chr2:89164966-89165066 | CATTTCAAGAAATATCATTTTTGCTTTTTATTTGGCATAATTTACTAGCCAATTTAGGAAGTTCCCCTCACA TCAGTAACATACAGTACATCACCCAGTA | 141 |
| IGKJ1_chr2:89165066-89165166 | TGTCAGAGGACACAATGGCATAAGTTTGCCTTTTGCAAGGTTTGAGGGATGGCCATTTCCCTACCTGACTC AGGAAAGTCTGTAGCTGATATCCATCTTC | 142 |
| IGKJ1-chr2:89165166-89165266 | AAGTTTGTGGTTCTTTCTCTCTATATATATATTTGAGCTCAGCAGTCATGCTGGAGTCCAGAGTAGGTGAT TCTTTCTGCTTTAGCTTGACTCCTCCTTA | 143 |
| 1GKJ1_chr2:89165191-89165291 | TATATATTTGAGCTCAGCAGTCATGCTGGAGTCCAGAGTAGGTGATTCTTTCTGCTTTAGCTTGACTCCTC CTTAAGATTGTAACTCTCTCAGTTTTACA | 144 |
| IGKJ1_chr2:89165291-89165391 | TTTTTTGTCAGACGTAAGCTGACATTCCACAAGGAGAGGAGGAAATTCTGTGGTTCACATCCAGTGGTGC TTGGAACCTGATTGGTTGTCATTCTTCCAG | 145 |
| IGKJ1_chr2:89165391-89165491 | CTAGTTTGTCACGAGTGGATATCTGTCCTGGATTCCCAAGGATCAAGGCTGCCCCATTAGCCAGGAAGTA GGGAGATAGAGGAGGTCACTTGAGAAAGAG | 146 |
| IGKJ1_chr2:89165491-89165591 | CTGCTTCTTTGCCGCCTCCAGGTTGTGTCTGTTTCCTCTCATATCTGAAGACAGATGTGCTGGCAGAAGCA AAGTCCTTTGTCCGGCCACGTGCAAATGC | 147 |
| IGKJ1_chr2:89165591-89165691 | ATGGGACATAAATATGAACAGAGATTCTTGTCCCACTCTAGAAAATGTAGATGTTCATCTTGTTTCCAAG GGGACAGTAAGGCTGCAGGTGTTTTTTGAC | 148 |
| IGKV4-1_chr2:89184966-89185006 | CTTTTGTACTCACTGGTTGTTTTTGCATAGGCCCCTCCAGGCCACGACCAGCTGTTTGGATTTTATAAACG GGCCGTTTGCATTGTGAACTGAGCTACAA | 149 |
| IGKV4-1_chr2:89185066-89185166 | CAGGCAGGCAGGGGCAGCAAGATGGTGTTGCAGACCCAGGTCTTCATTTCTCTGTTGCTCTGGATCTCTG GTGAGGAATTAAAAAGTGCCACAGTCTTTT | 150 |
| IGKV4-1_chr2:89185166-89185266 | CAGAGTAATATCTGTGTAGAAATAAAAAAAAATTAAGATATAGTTGGAAATAATGACTATTTCCAATATGG ATCCAATTATCTGCTGACTTATAATACTAC | 151 |
| IGKV4-1_chr2:89185196-89185296 | ATTAAGATATAGTTGGAAATAATGACTATTTCCAATATGGATCCAATTATCTGCTGACTTATAATACTACT AGAAAGCAAATTTAAATGACATATTTCAA | 152 |

EP 4 334 476 B1

| Name | Sequence | SEQ ID NOs. |
|---|---|---|
| IGKV4-1_chr2:89185296-89185396 | TTATATCTGAGACAGCGTGTATAAGTTTATGTATAATCATTGTCCATTACTGACTACAGGTGCCTACGGGGACATCGTGATGACCCAGTCTCCAGACTCC | 153 |
| IGKV4-1_chr2:89185396-89185496 | CTGGCTGTGTCTCTGGGCGAGAGGGCCACCATCAACTGCAAGTCCAGCCAGAGTGTTTTATACAGCTCCAACAATAAGAACTACTTAGCTTGGTACCAGC | 154 |
| IGKV4-1_chr2:89185496-89185596 | AGAAACCAGGACAGCCTCCTAAGCTGCTCATTTACTGGGCATCTACCCGGGAATCCGGGGTCCCTGACCGATTCAGTGGCAGCGGGTCTGGGACAGATTT | 155 |
| IGKV4-1_chr2:89185596-89185696 | CACTCTCACCATCAGCAGCCTGCAGGCTGAAGATGTGGCAGTTTATTACTGTCAGCAATATTATAGTACTCCTCCCACAGTGCTTCAGCCTCGAACACAA | 156 |
| IGKV4-1_chr2:89185696-89185796 | ACCTCCTCCCCATACGCTGGGCCAGTAGGTCTTTGCTGCAGCAGCTGCTTCCTCTGCACACAGCCCCCAACATGCATGCTTCCTCTGTGTGTTGGGGAGG | 157 |
| IGKV5-2_chr2:89196226-89196326 | AATACATGAAAACAACTACCGAAATGTTATGAAATTATAGTTTAGTAGAACTAACAAGTGCATTAATGCAAAAGAAAAGTAGGGCTCAGTAATCAGGGAA | 158 |
| IGKV5-2_chr2:89196326-89196426 | CCAAGTGTGCATTGTAAAAGTGCAGCCTCTCTAACACTGGGTTTCATCACAAGTAACAGAACAGGATGCCTGATGCAGGGAAAAAGAAAGGCAATTGTT | 159 |
| IGKV5-2 chr2:89196851-89196951 | GATCTCTGGTAAGAGAAACACTTCCTCTCCTCTGTGCCACCAAGTCCCCTGCATATCCACAAAAATAATATATTTTCATAAGGAATTGATTTTCCTCATT | 160 |
| IGKV5-2_chr2:89196951-89197051 | CTCTGCAAATATGATGCATTTGATTTATGTTTTTTACTTTGCTCCATAATCAGATACCAGGGCAGAAACGACACTCACGCAGTCTCCAGCATTCATGTCA | 161 |
| IGKV5-2_ch2:89197051-89197151 | GCGACTCCAGGAGACAAAGTCAACATCTCCTGCAAAGCCAGCCAAGACATTGATGATGATATGAACTGGTACCAACAGAAACCAGGAGAAGCTGCTATTT | 162 |
| 1GKV5-2_chr2:89197151-89197251 | TCATTATTCAAGAAGCTACTACTCTCGTTCCTGGAATCCCACCTCGATTCAGTGGCAGCGGGTATGGAACAGATTTTACCCTCACAATTAATAACATAGA | 163 |
| IGKV5-2_chr2:89197251-89197351 | ATCTGAGGATGCTGCATATTACTTCTGTCTACAACATGATAATTTCCCTCTCACAGTGATACACCCTGTTACAAAAACCTCCAAGTTCTCTCAGTGGGAT | 164 |
| IGKV5-2_chr2:89214836-89214936 | GCCCTCTGTCCTGGAGACACGGCCAAGGAGGCTGGAGACTGGGTCAGCACAATGTCCCCATTGCAGCCTGAAATGATAAAGACAGATAAATTATATCAGA | 165 |

| Name | Sequence | SEQ ID NOs. |
|---|---|---|
| IGKV5-2_chr2:89214936-89215036 | TATACTGAGACTGTCCCCATGTAGGCCATGCATTGGTGACACTTGTAACCACAGTCATATGCAACATCTTG AGTAACCAGAAAACAAAAGATAACTGGGG | 166 |
| IGKV5-2_chr2:89215036-89215136 | AACTTACAACCTACAATGAGTGCCCTAAATCCAACAACCAAGAATCCAGAGACACAAAAAACAATGATGC CCACATGAGTTTGCCCGATGTTTCCCTATA | 167 |
| IGKV1-5_chr2:89246681-89246781 | TACCAACACCATCAGAGTGTGGCTGCATCTGAGGACCACTCTCAGCTGATAGAGGCATCAGGAGGAGCAG CTGGGGCAGCCCTGCCTCACACATCTGCTT | 168 |
| IGKV1-5_chr2:89246786-89246886 | GGGGTTTATGTTCGGGTGTGTAACACTGTGGGAGAATAACTATTATACTGTTGGCAGTAATAAGTTGCAA AATCATCAGGCTGCAGGCTGCTGATGGTGA | 169 |
| IGKV1-5_chr2:89246911-8924011 | GCCGCTGAACCTTGATGGGACCCCACTTTCTAAACTAGACGCCTTATAGATCAGGAGCTTAGGGGCTTTCC CTGGTTTCTGCTGATACCAGGCCAACCAG | 170 |
| 1GKV1-5chr2:89247011-89247111 | CTACTAATACTCTGACTGGCCCGGCAAGTGATGGTGACTCTGTCTCCTACAGATGCAGACAGGGTGGAAG GAGACTGGGTCATCTGGATGTCACATTTGG | 171 |
| IGKV1-5_chr2:89247096-89247196 | GGATGTCACATTTGGCACCTGAGATTGGAAATAGAAACACAAATATTCATACTATTGATCATATTATAGG AAGACTTCCCTGAATAACCAGGCAGTACTG | 172 |
| IGKV1-5_chr2:89247196-89247296 | AGCACACTGGGCTGAGTAAATTCCTAGTGTTCTCCTTCCTTACCTGGGAGCCAGAGCAGCAGGAGCCCCA GGAGCTGAGCGGGGACCCTCATGTCCATGC | 173 |
| IGKV1-5_chr2:89247526-89247626 | GGGACTATTTTATTATGAGAAACAATTTTTAGGTATTTTTTTGAGAATTTTAAATATTCCTCAGGAGCCGA TAGAGTAATGTATTTCATTGGTGTATCAG | 174 |
| IGKV1-5_chr2:89247626-89247726 | GATTATTTAGGAGAATATTCTTGTTTGTAGGAAACACATAGTAAAATGTTAGATGGTAGGATTCTCAAGT CTTCAAAAGACTCTCATAAGATTCCGGGTA | 175 |
| IGKV1-5_chr2:89247641-89247741 | TATTCTTGTTTGTAGGAAACACATAGTAAAATGTTAGATGGTAGGATTCTCAAGTCTTCAAAAGACTCTCA TAAGATTCCGGGTAGGGAAGGGGGTAATT | 176 |
| IGKV1-5_chr2:89247831-89247931 | TGTAAGTATTAGGTAATGGTGTTATGCCTTTGTTCTTACTAGTATTAGATCAAGCAATTTATTACAGATAT ACAAAGATGATACCGTGTTGTCTCCATGC | 177 |
| IGKV1-5_chr2:8924793 1-89248031 | ATGCAGCACTCACAGATCCACCACTATCAAGAACTGCAGGTCTCTTTAATACCCAGAGACTAAATGAGGT GCACCTTATTCTTGTTTTGGGTACCTTCAT | 178 |

| Name | Sequence | SEQ ID NOs. |
|------|----------|-------------|
| JGKV1-8_chr2:89291906-89292006 | TTGGGTGTGTAACACTGTGGGAGGGTAACTATAATACTGTTGACAGTAATAAGTTGCAAAATCTTCAGAC TGCAGGCAGCTGATGGTGAGAGTGAAATCT | 179 |
| IGKV1-8chr2:89292131-89292231 | CTGACTCGCCCGACAAGTGATGGTGACTCTGTCTCCTGTAGATGCAGAGAATGAGGATGGAGACTGGGTC ATCCGGATGGCACATCTGGCACCTGAGATT | 180 |
| IGKV3-20_chr2:89442291-89442391 | CTTTCCCCTGGAGACAAAGACAGGGTGCCTGGAGACTGCGTCAACACAATTTCTCCGGTGGTATCTGAGA TTGGAAATAAAACAGAAAAGTCACCCATGT | 181 |
| IGKV3-20_chr2:89442391-89442491 | AATCTAAATCAAACCCATTGTCTTCCCAGAAGAGCCAGAATTATTGCTTTATATTGAGCTTTAATTATTGT ATTGACTGAGCAGAGTTGCCAGGTAACAG | 182 |
| IGKV3-20 chr2:89442491-89442591 | GACTTGAGAGGGTTTTCACTGACATGCAAAACCATCCCATGTTCCCCTCACCTGGGAGCCAGAGTAGCAG GAGGAAGAGAAGCTGCGCTGGGGTTTCCAT | 183 |
| IGKV3-20_chr2:89442616-89442716 | AGCTCTTCTCCAGAGCTCTGACCCAGGCATTGATATGGGCTCTGGACTGCAGGGCGGCTGGGAGGGACAT GCAAAGCAGCTGGGGCGGGTGCTGGGCTTG | 184 |
| IGKV3-20_chr2:89442716-89442816 | CAGCTGCAGAGACAATCTGCCTCCCCTTTCTGCTCTCAGCAGCCCATGCCCAGGTGATCAGGCCAGAAAA GGCCGTTGGCTCAGTCTGAGGGTAGAACTT | 185 |
| IGKV3-20_chr2:89442816-89442916 | CTCCCCTGCGGCCACAGAATTTAACCCCTGTGTCCTCTTGTCTCACCATCACCTAGATTGAGCCACAGAAT GTTTGGTACAAGTCTGTTAGAAACAAAAT | 186 |
| IGKV3-20 chr2:89442916-89443016 | AGAAGGCTGTGGTTTCATTTTTCTCTTTCTGCTCCAACTTGTGCCCAGTCAGCTCCCTAAATGCATGATGG ATCAGGTTGAAAGGAAGAGTCTATTACAA | 187 |
| IGKV3-20_chr2:89443016-89443116 | CTTTATCTTCCGGATATACTTGTATTTACTTGTTAGTGATCTTTCCTGAGGGTCCAGAAGCTGTCTCATTCT TTGCAGAAATTAAAAGAGTAACATTCAA | 188 |
| IGKV3-20_chr2:89443116-89443216 | TTAACCTCAGCACTGTGGGTGTGAGGACTTTCACAACTGCACAGATAAGTGAGACCTGGGCTCCAAATCC TCAGGGTAGTGATACCATTTCCCTAAAGAC | 189 |
| IGKV3-20_chr2:8944.3216-89443316 | AGAAGATGGTTTTGTCCATGCAGGCAAAGAACTATTTCTTGGGTGATCCTCTAAACTATCCAGTCTTTTTA TTCTGTATAGCTGGTATAGTTTACCCTTA | 190 |
| IGKV2-30_chr2:89544656-89544756 | GGCTATATATGTATTTGTTCATATTTCAAAAATACACAGTTTCAAAATGGAACTCAAGGGATCCAAGGCTC AAAGGGGTCTCCAGAAGACCCCACACCAT | 191 |

| Name | Sequence | SEQ ID NOs. |
|---|---|---|
| IGKV2-30_chr2:89544756-89544856 | CCCCTTTCTGTGTCAGTCTTCCCCAGAGCACAGATCCTTGTTTCTGCTTGAATCTTCCTCACTCTCACAGAT CTGATCATCACATGCCCCACTCTGGAGG | 192 |
| IGKV2-30_chr2:89544856-89544956 | ACAACATGTGCATGTCCAATACAGGAAAGGAACACACATAGGAGTGTAGTGAGACCCCCAGAGATCACT GTTGTTAGAGGCAGTGGGGCCCCAGAACTCA | 193 |
| DUSP2_chr2:96810164-96810264 | GGAGCAGCAGCGGGTGGAGACCCCATGGGCTGGCCGAGACAAGAGGACTCCTCAGCCAGTCCTCCTGAC CTGAGACAGGTCTCAGGAATGTGCGGAGGAC | 194 |
| DUSP2_chr2:96810264-96810364 | ACACCGGGACATACATTTCCCTTCATGCTCCCAACATACACATGCAAACATACACAGACCCATACAGGCA CGCGCGAGCAGCCATGCCCCACCCCCTCCC | 195 |
| DIISP2_chr2:96810364-96810464 | CCAACACACACGTATAAAAGTGTGTGTATATGGGCAAACTGCTCGCATCCCCAAATGGCAGGCTCTTT CCCTAGAGGCGCCCAGTCCGCGGCGGGGAG | 196 |
| AFF3_chr2:100758483-100758583 | AAGCTCACTCACTGGGGCCATTGACTGGGATCCAGTCTGTGGCCATGTCATGGTTTCTATTTTTGAGGTTA TAGCTAATGAGCAACATGAGGTTAAGACA | 197 |
| AFF3_chr2:100758583-100758683 | CACTTTTCATAAGGCCCCAGCCAGCATCATAAATATGTGTGTGAGCATGTTCACACTCAGGTTATGTCTTC TTTATGTGCACCCTCTACCACACACAC | 198 |
| DDX18_chr2:117951919-1 17952019 | GCCAAGAACCACGACTCTCTAATTTTACTTCCCAGCAGGTATTCAGTGCATAATAGTTCCTACTTAGAAGT ATCATATTTGCCCAAACACAAGGTGATAC | 199 |
| DDX18_chr2:117952019-117952119 | CCAAAATGAGGTAAGTTTCCTGTTTTCTCAGTGAGATCTTTTGTTGTTGTTGTTGTTGTTGTTTTGTTG TCGATGTTGTTGTTTTTGGTTTTGGTCT | 200 |
| CXCR4_chr2:136874415-136874515 | CCGGGTCGTCCAGCCCCGGGCCGCCGCGGCTGCCCACTACACCCACGCCAACCGCCCGCAAGCAGCGCTG CAGGGGCTCCGCTGGGCGACACGCCAGGCT | 201 |
| CXCR4_chr2:136874515-136874615 | CTGTCCCACAGGGTGCTGGGGAGCGACTGGGCGGCTCCGCCGCGAGCGTCTTTGAATTGCGCGCCGCTGC AGGAAACCAAAAACTCCCTAGCAAGAGGGT | 202 |
| CXCR4_chr2:136874615-136874715 | TTCAAAAGGTTTCTGGAAACCACCGACGGTTAAACATCACAACTGGACTCGGAGAGAGCCAAACGGTTTC CCCACTTGCACCTGCCAGTCTTCGCGGCGG | 203 |
| CXCR4_chr:136874715-136874815 | CGACCTGGCAGCCCAGGTGCGGTCTTAACCGCCCCCGCCCCTCACCCCGTACCCGCTCCTATCCCCGGAGC GCAAATCTCAGGGCTGGCAGCTGCGCGGT | 204 |

EP 4 334 476 B1

| Name | Sequence | SEQ ID NOs. |
|---|---|---|
| CXCR4_chr2:136874920-136875020 | GGAAGGTTTTCCCCCTCAAACCCAAAGCGCGCGGGCGGATCAACTCCTAGCTGCTGCCACCACTCGATCC CCTCAGAGGATCGGCGCGGTGGGTCCACCC | 205 |
| CXCR4_chr2:136875020-136875120 | GCCTCTCCCGCCCTCTGCCTACTGTGCTGGGAGACTGGCACAGCTCCGTCGGCCGCACAGAGTTTAACAA ACACGCACCCAGTGTCAAGAACAGTCACCA | 206 |
| CXCR4_chr2:136875120-136875220 | GGCGCTTAACCCCGAAGTTAAAGCGGGCGCAATCTCCTCCTGGGAACTCAGCCCAGGCACGCCGCCCTCC GCCTCTAAATTCAGACAATGTAACTCGCTC | 207 |
| CXCR4_chr2:136875220-136875320 | CAAGACATCCCCGCTTCCCCAAGGAAGAGACCGGTGGTCTGAGTCCCGAGGCAGCGCGCACGCCTTCTCT GCACTTGTGCACAGAATGTTCTTACGTTTG | 208 |
| CXCR4_chr2:136875320-136875420 | CAAACAGCGTGCAAGCCGCCGCGCGCGGCGGGACTCAAGGGGGAGACACATGCAGCCACTGGAACGCTC TTTCCAGTCGTTTCTCCTCGACTCACAGAGA | 209 |
| CXCR4_chr2:136875420-136875520 | AAAAGATTCCAATCCTGCTCCCCCCCCACCCACCCGCACTATATAGGCATGGTCAAGAAAACTCCTTTCGG TGACCCTTTTTTGGAGTACGGGTACCTCC | 210 |
| CXCR4_chr2:136875520-136875620 | AATGTCCTGGCCGCTTCTGCCCGCTCGGAGAGGGGCTGCGCTCTAAGTTCAAACGTTTGTACATTTATGAC AAAGCAGGTTGAAACTGGACTTACACTGA | 211 |
| CXCR4_chr2:136875620-136875720 | TCCCCTCCATGGTAACCGCTGGTTCTCCAGATGCGGTGGCTACTGGAGCACTCAGGCCCTCGGCGTCACTT TGCTACCTGCTGCCGCAGCCAACAAACTG | 212 |
| RFTN1_ chr3:16419204-16419304 | CCCATTGCTGACATACTTACTCCCTGAGAGTGGCTCTTCATGCACCTCCAAGGGGTTGCTCTCCGGTCCAT CCAGTGTCTTGCTCACCCCCTGTGGTGAA | 213 |
| RFTNI1_chr3:16419304-16419404 | AGTTCTCCACCATCTCCCTCTCCGGAGGGTGAGCTGGGCTGCTTGGCGAGGGGCACCTCCCCTCTGGGGC CTGAGCTGGGCTCTGGGCTTTGGTTTCTCC | 214 |
| RFTN1_chr3:16419404-16419504 | CAGCCGGAGCACTGCACACATCCCCAGTCCCCGGTTTCTCATTCTCCAGTGACGCGTGATCCCCACGTGCG TTTTTTGCATCTCTGGCATCCTCGGTGCT | 215 |
| EIF4E3_chr3:71551101-71551201 | ATTTGCAGGTTATATCCTGGATGGTGGCACGACAGCGCCTGGAACACAGAAGGTTGGGAGGCGTGACGCT CATCAGGAAGGCTCTTTTGGGGAGCCAGGA | 216 |
| EIF4E3 chr3:71551201-71551301 | AGAGTCCCCCAGAAGCCCACTTGGCACCCTATCTATAACAAGTTGCTCTTTAAGAATCATGGGAACTCCA GAATCATTTTCACAAATACCTTCCACTCAT | 217 |

(continued)

| Name | Sequence | SEQID NOs. |
|---|---|---|
| EIF4E3_chr3:71551301-71351401 | GAATTCAATTAAAATGGCAGAAAACACAAACCTTCCGTTCCCCACTGGCAAACTGGGTCTAGCTAACTGAGCACAGCTAGCACAAGGCACAAGGCAGGCCCCTGCTAGC | 218 |
| EIF4E3_chr3:71551401-71551501 | AGGGCAAGTGGCGGCCGGTCCCAGGCCCAGGGGAGCCCTCTGCAGCTCCCTGGAAGGACGGTCAAGTGAACAGAGAGCTGGCTGCCATCTGGGTTCTT | 219 |
| KLHL6_chr3:183272308-183272408 | ATGAGATCACCAGTTTATCGTAACTAGAGGCCTCTCCCATCTAAAGCATCTTTGTAACTGCTTTCCCTTTCCCCACACTGCCTACACATAAAGGAAGAGCCCC | 220 |
| KLHL6_chr3:183272408-183272508 | TAATTTGTAACAAGTCATTTGACAACTCCAGAGAGAGGGGCCACATCCTTTTCTCTATGTCTGTTGATTAACAAAGACACAACATTAGTTTCCAACACCAG | 221 |
| KLHL6_chr3:183272508-183272608 | TCAGACCAAGGGGGAAAAAAAGTCCCATGACTTCAGTAATTTCCATCCTTTGGAACAAGGAAATATACACAAAAGGTTTACTATAGAAATGTAAGCATTG | 222 |
| KLHL6_chr3:183273063-183273163 | AACTGTTCAAGATTGGGCTCTCACACTAACACACCTCTTCCTTGCAACTTGCACCCAATTTGACTCTGGTCCTAGGCATGCTGACCTGAAAATAGTTGCTG | 223 |
| KLHL6_chr3:183273163-183273263 | GCTGCCGGCAAGCACCACCGCGGTGGCAGGAGAATTCCTGAATGTCCACACACAAGATGACATCTGTCAGACCGTTTTCCATTCGCAGGGTTTCCAGGCCAT | 224 |
| KLHL6_chr3:183273263-183273363 | TCTGAAGAATTAAGGAGGAGTCCCGCGGTCGTCAAATTTGACCTTTCCCCATTTAAGATCTCGACCAAGTCTCCTGTTTTCTGGGAGGGGCTCATCTGTAGA | 225 |
| KLHL6_chr3:183273363-183273463 | AGGTGCCAGGGCGGCCCTTCCAAACCTCTTCTCGACCACCATCACCCATGGTCCAGGCGCGCCCCTTTGTCCTGCCATCAACATCGAGAGACTGAAGGAGCGGCCCAAG | 226 |
| ST6GAL1_chr3:186714604-186714704 | CCTTCCTGTGTTGGCCACTACCATACGTGTCCCCGCGGCGTTCTTGCCCCCTCTCTGCTTGGGGTCCCTGCTACACTGGTATCCTGCCACTTTCCCACCTTGTATTGCCA | 227 |
| ST6GAL1_chr3:186714704-186714804 | GTTTGTTTCCCAAGGCCAATCTCCACTTTGAGCTTGTTCATGACCACCTCACACAGCACACTTGGTCTGTGTGGTGGTTTGAGGGGTTTCTGTCTGTACACTG | 228 |
| ST6GAL1-chr3:186714804-186714904 | TGCTTTGGCTGTGTTGGAGGCGGCGGCAGGTGGGCAGGTGGGAAGGAAGAGAAATGTATTCTTGGGGAGATTTGTTTTTAGAGACATGAGACATGGAAAATAGTTAAGTAAT | 229 |
| ST6GAL1-chr3:186714904-186715004 | AATATAAATATGGGAGGAGGCATGGACTATCAGAGGAGGCAGGCAGGACTGCCCAACCTCCTCACTGGGCACGTTACGCTACTTCCTCCTGACCTCTATAGTCC | 230 |

| Name | Sequence | SEQ ID NOs. |
|---|---|---|
| ST6GAL1_chr3:186782529-186782629 | CTATCATTGCCCTTTCTTACCTTGATATCCTAAAAAGCTGGTGGTCTGTCTTCTCTATCTTTTGTCCTGGTCAGTTATCCTAACTATTTTGTGTCTGTTT | 231 |
| ST6GAL1_chr3:186782629-186782729 | CTGTGGATTAGTAAACGGGGTCCCCACCCCCACTCCACAAGGAGAACATCTGGCACCCAGAAGTCACTGAGAGAATAGCTGTTGCTTTGGTAGAATTCTG | 232 |
| ST6GAL1_chr3:186782729-186782829 | CCTCTGAGTGGCTTGTTCTTTTCCCAGACGGAGAGGTCTCCTGACAGCAGCTCTCTTCTTTTTCTTTTTTTTTTTTTTGAGACAGAGTTTTGCTCTTGC | 233 |
| ST6GAL1_chr3:186783389-186783489 | CTCCTGTACCCTGTGGGCCTGAGAGAGGAGACAATGGGACAAGAAGACCCAGTGGCTTCCTTGGAAGCTTTTGTGCTAGCTGGAGAGAGAAGACCTACTT | 234 |
| ST6GAL1_chr3:186783489-186783589 | CCTATATGCCTAGCAACAGTCCACACTGACTGGACTGCAACCAGGACATTTCCAGATTACTCAGTGGGGCTTATCTTGAAATAATAGTTGATGCCATTTG | 235 |
| ST6GAL1_chr3:186783589-186783689 | TTAAATATATTATATATACCATCTAAGGGTCTTACATGCCTTCTCTCATTTGATCTTCATGGCAAACCCTGTGAGGTATGACCACCAACCACCATTTTAC | 236 |
| ST6GAL1_chr3:186783689-186783789 | CTCAGAACTCAGGCTCCCAGAGTTTAAGTTGCTCACAGGAGCCCAGAAAGTAAGCGACAGAGGTGGGATTTGGTTCTAGGTGTTTGCCACCAGCACTTTA | 237 |
| ST6GAL1_chr3:186783789-186783889 | AATCACCAAAGCTTTCTGGAAGCTCCAACTTTTCTTCTCAAGATACTGAAAGACAGGTATCTGGATGGGTTGGCAGGGCGGGTGGGAGGTGGGCGAGATT | 238 |
| ST6GAL1_chr3:186783889-186783989 | TCCATCAACAACGGGTCTAAAACCAGCGATGGTGAGCTGGGTGATTTTGATGGAACCCCTGCCATACAGTCTATTAATATCATAATTGGAGCTAAAATTT | 239 |
| ST6GAL1_chr3:186783989-186784089 | AATCATGATGGCAATCATGAGTTCTGGGGCTTCTTGATTTGGGCCAGCAGACACAGTCTCAGTCACTAGTTCTCCGAATCAGAGAAAGGATGCCTTCAGG | 240 |
| ST6GAL1-chr3:186784089-186784189 | CTGTGTCTTCACATGGCTTTTCCTCTGTGCGTGGTGGAAAGAGAGAGCTCTGCGGGTCTCTTCTTGTTGTAAGGACACTGGCCCCATTGGATTAGGGCCC | 241 |
| ST6GAL1_chr3:186784189-186784289 | CACCACATGACACATTTAATCCTAATTACCTCCCTCACAGCCCTATTTCCAAACAGGGTATTAGTCACATTAGGGATTAGGGCTTCAACATAGGAATTCT | 242 |
| ST6GA1_chr3:186784289-186784389 | GGGGGCACACAATTCAGTCTATAACAGAGGGAAAACAGATTTGAGAAGAAAAAAGTCCAAAATATGCACAGTGGTAATATCTGAAGATGTGCGTGCGTGC | 243 |

| Name | Sequence | SEQID NOs. |
|---|---|---|
| BCL6_chr3:187460134-187460234 | TCAAGGGCTCAGCAAACGACAACTTAAGCATTTAGAGTCCCATCCCTATCCACCAAACCCAGAATAAGTT AGTCTTTTCAAGAAAGCATTGGTATAAAAC | 244 |
| BCL6_chr3:187460234-187460334 | CCTTCAAAACTGAAAAGAAGAAAGGGGCAATTGGAGAATTCCCACTTTTTCTGGCTGTCTCCTTCAAGTC GCCCAGTTTTTATGAACAGCATCTAGCCTT | 245 |
| BCL6_chr3:187460334-187460434 | ACTGTCACTATCAACAACCCTTAAAACTAGCCAATGCTTCGGCCTCTAGTATTGGAAAGTCTTCCAAATAG GATACTGGAAACTTCTATTTATAAGCTTG | 246 |
| BCL6_chr3:187460434-187460534 | GGGTGGCGGGCGGGGCGGGGAGGTGGAGAGAGAGTTGCCATCTACAGGTTTCTATTTTGGCCTGAAGAC TCAACTGCAGTCATTAGAGTAAGGGAATGCC | 247 |
| BCL6_chr3:187460824-187460924 | TTATTTATTAAAACCACACACACCTTGCAAAGAAAAAGGGAAACTGGCAGTCTCTGTAGAGGAAGCCGGT GGCATCGCTCAGAGCCACAAACTGTATTTC | 248 |
| BCL6_chr3:187460924-187461024 | TAAACAGCCCTTTCCCTGGTTCCCTCTCTCCTGCCCCACTTTTTTTAAAATCCAGACTGTAAAAAACACATC TACTGACACTCACTTTACTTTAAAAAAA | 249 |
| BCL6_chr3:187461024-187461124 | GAAGAGAAAAAGTAAAGCGTTACAAGACTTTCCTCCTGGAAACTATAAACTGAAAAAAAAAATCCATAAA AGATTAAATCCTGGCGGGTTGTGGGGTGGCG | 250 |
| BCL6..chr3:187461124-187461224 | GGGGCCGGCGGGGAGGGGGCGCGGAGTGGAGATTGGCTCTCTGAGGTGGTCAGGGGCCCTGTGACAGCT TGGGACTTTCAGCACCTGGTTTGGGGTCATT | 251 |
| BCL6_chr3:187461224-187461324 | TATCTGCTCAACTGTCAGGACCCCCCACCCCCAAACCCCAGCCACCAACACAACCATCGTAGAAGGGAAC ACAACACAGAGGGTCTTTTTTCATTTTTTT | 252 |
| BCL6_chr3:187461319-187461419 | TTTTTAAAAAATCGGTTTGGTTGTGTTTTTGTTTTCCATGGGGGAGCTTTAAAACTCATTATTGCAACACT AGTTCCATTTTTCGCCAGGGTTCCAATAA | 253 |
| BCL6_ch3:187461454-187461554 | CAAGACATTTACCACGGTCACTACATCCGGCAGCGGGGTGGCCCCTAGCTCCTGCTGCCCCCCCGCCCTTT CTCCCCGCCCGCCCCCGGAGCTCAGCCGA | 254 |
| BCL6 _chr3:187461554-187461654 | TTTCTGAGGCTCCAACTCTACCCACTCCCTCCCCGGGCCGCCGCCGCCGCGCCTTCCCCCATTCTTACTCCC TCGAGGAGAGCCACAGGTTGCAAATCCA | 255 |
| BCL6_chr3:187461654-187461754 | ACCAACCTCGCAATCTATTTTTGCAAAATCACTCACAAAGATCTCCCTTTCGCGCCCGCGCCCGCTCCTCC CGCGCCGGGTCCCCTCAGCCACGGCCACA | 256 |

228

EP 4 334 476 B1

| Name | Sequence | SEQ ID NOs. |
|---|---|---|
| BCL6_chr3:187461754-187461854 | AAGTGCCCTTCTCTCCTCCTGAGTCTTGCACATAAGGAACGCGGGCTGGGGCTCTGTTCGTCTTTCTCCTC GCCCAAGGTAAGGACCTCGGGAATCTGAA | 257 |
| BCL6_chr3:187461854-187461954 | GCCTGGCGTCCACTACGCTCAGGCCCGCAGTTCCCTTTTTACAGAGCTTGCACCATGGGAAAAAATAAAA TAAAATTTAGGAAAGGGAGGCAACAGCCAT | 258 |
| BCL6 chr3:187461924-187462024 | TAAAATTTAGGAAAGGGAGGCAACAGCCATTGGGAGCCAACACAGAGTCACGCAGCGCCCAAAATACAA ACACCGCAGCGGCCAGAAATCCCGCCACCTT | 259 |
| BCL6_chr3:187462024-187462124 | TCTCGTTCTCCCAGGCTGTCCTGTCGAGGTTCCCTGAGTCCCCCCGCACACTGAAAGGCATCGCAGGTGCA GTGCGCACCCCTTTCCCACCCACCCCAAG | 260 |
| BCL6_ehr3:187462124-187462224 | AAGCCCTGTCCCGCCATCAGTCTCTCTCCTCGGGATGAGCAGGGAGAGCGCGCGGAGGTTCCCGACTCCC TCGACTACAACCAAGAAAGAATAATTTTCA | 261 |
| BCL6 _chr3:187462224-187462324 | AAGTGTTCAACATCCCCGCCCCCAAGCTCCCCAAAACACAGGGGCAGGGAACACCAAAACACTCGGCTCT CATTAGGAAGATCACGGCTCTGAAAGGAAA | 262 |
| BCL6_chr3:187462324-187462424 | TAGTAGACACGATACTTCATCTCATCTGGATTTATGACCAAAAAAACAAAAACAAAAACCCAAAGAGTTC GCTTGCATTTTTTCCTTCCAAATCTCGGTT | 263 |
| BCL6_chr3:187462374-187462474 | AACAAAAACCCAAAGAGTTCGCTTGCATTTTTTCCTTCCAAATCTCGGTTCGGCTCGAAGGCAGGGAATCT AAAAGACCGAGGCCGATGGAAGAGAGCCA | 264 |
| BCL6_chr3:187462474-187462574 | GCGGGGCGAGCGAGCGGGCAGCCTCCCTTTTTGCCTCCCGGAGTTACCCAGAAGGACAGGGGAAGGGAA GGAAGAAGAGGCGAGGAAAAAGAGGAGGGAG | 265 |
| BCL6chr3:187462574-187462674 | GGAAGCGGAGGCCAGGAGCGACGGAGCAAGGAAAGCAGTTTGCAAGCGAGAAAAGAGGGAAAAAACAC AGCCGCACGAATCCAGAGAGATCACAAGCCGT | 266 |
| BCL6_chr3:187462674-187462774 | ACGCAAGCAGCAGCAGAAAGAGCGAGAGCGCGAGCGCGCGTCCTCTCCGCGGTCTGGGGCCAGACAGCC CCCAGACTAGCCCGAATCACCCCCCAAGCAC | 267 |
| BCL6_chr3:187462774-187462874 | TGTCTCGTCCTCTCTGCTCCGGCCGCCCCCTAATTCCCCTCCTTCCTCTCCTCCACCTCCTTTCCAAAAACC AAAACAACACAAGGGAGGGTGGCAAAAG | 268 |
| BCL6_chr3:187462874-187462974 | CCTCCCCAAAACCGGCCGATTCACTCAAAGACAACAATAATAATAATAAATACATAACAATCTATATCCTA TGGTGGGAGAGACGTGGGACTAATCTTCGG | 269 |

| Name | Sequence | SEQ ID NOs. |
|---|---|---|
| BCL6 chr3:187462924-187463024 | ACATAACAATCTATATCCTATGGTGGGAGAGACGTGGGACTAATCTTCGGCATTTATTTTAACACCTGACAGCTAGAATAAATAAATATATACATTTATA | 270 |
| BCL6_chr3:187463004-187463104 | AATAAATATATACATTTATATCAATAGATACACATAGAAAACTTGGAGCCAAAGCATTTGGCAAGAGCGGAAAAAAAAAGAATTAAAAGGTAAAATAATG | 271 |
| BCL6_chr3:187463104-187463204 | ATCATGAGCAGCGGCGGCGGCAGCGGCACCAGCGGCAACAGCGGCGGCGGCGGCAGTAGCAGCAGCAGCGGCGGCAGCAACAGCAATAATCACCTGGTGT | 272 |
| BCL6_chr3:187463204-187463304 | CCGGCCTTTCCTAGAAACTTCTTGCATCACCACTTCTAAGAACCCCAGTTCTAAGAATCAACAGAGCTCAATTCTCGGAATTTGAGCTTCGGACTTTACC | 273 |
| BCL6_chr3:187463304-187463404 | ACTGCTACGTGGCAGGGGAGGACTTGGTGTCAGCTCTCCGAGATTTTTACTGCCCCTGGCCAACCAAAAGCCCTCAAAGCCACAAGATTTTTTCACTGGC | 274 |
| BCL6_chr3:187463404-187463504 | CGGCATATTTCGAGGTCCTCATAAGCAGAGCGTCTCGGATTTGGAGGTTCCGGTTCGAGGCTCGAGGGGCCTGAAGGTGGCTCTCCCTCCCCGGGCCCAA | 275 |
| BCL6_chr3:187463504-187463604 | GACGATGGTATGGCCTGCTCCGCCACCATCACGTGGGCTCCTCCTCTGTGACGTCGGCGCCTTCGCTGTAGCAAAGCTCGGCCTCTGGAATTCTGAGAAC | 276 |
| BCL6_chr3:187463709-187463809 | GCACAAAAGGGAGCGAGAGGTTTGAACCACTGGGAAAAGTATGTTATATATAGTAGGGTTAGAGAGGCGAGTAAGAGAAAAATAAAATAAAATAAACA | 277 |
| BCL6_chr3:187463794-187463894 | AAAATAAAATAAACATCACAGCTCTTTCCAACTAGAATATTAGGCACCACGAGAAAAATATTTGCCAAGCAGTTTTCGGTGGGTTCATTTGCTTTATTTT | 278 |
| BCL6_chr3:187463894-187463994 | TATTTAGGACAGGGGTTTTTGCTGTTGTTCTGGGTTTTTTTCTTTCTGGTGTGGTGGCTTGGGATTTTTGGTTTCTGTATTTTGATGGTTTATGGATTTT | 279 |
| BCL6_ehr3:187463994-187464094 | TGCTTCTGATTTTTTGCCTTTTGCAAGTTTGTGGTGTTACGTAAATCACAGGATCGGCATCGGTTGGATTTTTTTGTACGTGCCTTTTCTTTCCCTATCT | 280 |
| BCL6_ch3:187464094-187464194 | AATCCCTCAAGCGTTTTAAAGATGTATTATTTCAATACTAATACTATTGAAAGAAGCTTAAATTTTTGGCCATATGTAACAATCCCAGCCCCCACTTTTT | 281 |

| Name | Sequence | SEQ ID NOs. |
|------|----------|-------------|
| BCL6 chr3:187619334-187619434 | ATTATCATCATCACCACCAACATCCTCTGCCCTGGAGACCAAGAGAATTCAAACAGGTCAGCACCTCTAATTGCTGTATAGAACATTGACCCTACTGTCT | 282 |
| BCL6_chr3:187619434-187619534 | CCCAGTTCCTGAGGATGGTGTGATAATAATACATCTCAGAGTTCTGTAGTTTCTTCACCACTGTGCAGGTGTGGTTGGTGGGAGCAATGCCCTGGATGGA | 283 |
| BCL6_chr3:187619534-187619634 | TAAGCCAAGCTCTTGTGTCCTGGCAGATAAACAAGGTGAACCCTCAATCCGTGTAGCAGGAGTTTCCAGACAAACTCACTTTGCATGGAAGGACACTAAC | 284 |
| BCL6chr3:187619634-187619734 | CCTTCCAGGTGCATGGAAATATTTTGTAGTTTTTACTGTCTCCCCCTTCCTCCACTGCCTCATCTTTTTTGTTTTTTCCCCTGTGAGACTATTTGCTCTG | 285 |
| BCL6_chr3:187660817-187660917 | CCTTTCCAACACTGGCCTGCCTTAGGGACTCACCGTCTGCACTCCGCCTGCACAGGTGGAACTGAGTTCAGATGAGGGAGAATTGCTTTCCATTGTTCAG | 286 |
| BCL6_chr3:187660917-187661017 | TAGGCTTTTTGTAATTTCTAGTTTTGCTTACCTTTCCTACTCACCACACACACAAAACAGTGTGAGCTTTCTCATTCTAGTGCATAAACACAGGTCGGTC | 287 |
| BCL6_chr3:187661017-187661117 | AATACCCACAAGTGTTCCAAAAGGTGAGCTGGCATTGCTGCCCAACTGGGCATTATAGTCCCTTCTGTCCCTGCCCATCAGGCTTGCCTTCCTCGGCAAC | 288 |
| BCL6_chr3:187661117-187661217 | CTTTCTAGCTTGAATTGTACTGTGACTCCTTCTCACGGACCACTCCCGGAGACTGGTGAAAGTTGGGCCCATTCTTGAAGCCTCTGCTTCTAAATCATGT | 289 |
| BCL6_chr3:187661217-187661317 | TTTCCATAAAGTCTCCCTCATCGTGCTTGCTTCCACCTTCTCCTATTTGGAATTACTGGTGGGCTCTTCCACTGTCCCATAGCAAGTGTTCTATACATTC | 290 |
| BCL6_chr3:187661317-187661417 | TGAAGGCACATTTGAATATATACTTTGTCATGGTTGCTTGGAACCATGTCGTCTTTTCCAAGTAGGCTGTGAACATTCAGTGGCATGGATCATACCGTGC | 291 |
| AC022498.1_chr3:187957432-187957532 | CCCATTGTTCAAAGAAAGGCATTATGGAGTCTCCAAAAGCCATTGGCAGGTGGTGTCTGTGACTTCCTTAGCCTGGAAATAAACAAATAAACAAGCACAA | 292 |
| AC022498.1_chr3:187957512-187957612 | AAACAAATAAACAAGCACAAATTAGAAGTCTTTGCCCTATTACTGCACTATTAGTATTGATTGCGCAACATCATGCAAAAGTCACTTTAATTTATCTGG | 293 |
| AC022498.1_chr3:187957612-187957712 | CAGGTCCTATGTAAACACCAATACAGTCAAGAGGGCTTGGATGGGTATTTGCTTTCATTTCTAATGAAATTTCAGGCCTCTAGGGTAGGATATCAAAATT | 294 |

| Name | Sequence | SEQ ID NOs. |
|---|---|---|
| AC022498.1_chr3:187957712-187957812 | GGTAGATCATTTGCAATTTATTTTATCCCAAACACCTCACTTTACAGTCAGAGAAACTGAGGCCCAGAGAAGTAAAATGAGTTGCTCAAGGTCTCAGAGA | 295 |
| AC022498.1chr3:187957767-187957867 | ACTGAGGCCCAGAGAAGTAAAATGAGTTGCTCAAGGTCTCAGAGAGCAAGAAATAGAGATGGGACTTGAGCACCTAGATCTCTGGTATTGCTGTCCTGTA | 296 |
| AC022498.1__chr3:187957867-187957967 | GTTCATGGAGCTGGCAGATGGATACATCTGTGACCTGGGATGATGGAGAGACTGCTGGACCCTTCAGAGGATCTCATCTCAAGGTGGGGTTTATGTGTAA | 297 |
| AC022498.1_chr3:187957967-187958067 | ATGATATCTGTGTGTTTCATTTTCCTTTCATAAACTAATTTAAAAATCCTTTTGGTATCAAATTTTAAGCCAAAAAGTAGTGAGGGGGAACATGGGTAGG | 298 |
| AC022498.1_chr3:187958067-187958167 | AATAGCTTACAGCTTGCCTAACAAGGTTGTTGACTGCATAAGAGTCAGGAGTTTTGGGTAAGAGTGTGTGTGTGTGTGTGTGTGTGTGTGTGTGTGAG | 299 |
| AC0224981_chr3:187958282-187958382 | CGTACTGAATTTGACTGCTTTATTTTGTAGGGAAGGAAACTGATGTGCCTAGAGTAGTTGAGAGCTTTATTCAAACTCATTCCACTGTTATTGAGTAGTT | 300 |
| AC022498.1_chr3:187958382-187958482 | AGGATATTAGACCAGCAACATATTTGGGTAGAAACTTTCATATAAAAAAGCGTAATCATAACTATCCAATCATGTCAACTAGTAAGGCTGCTCAGGTGGG | 301 |
| AC022498.1_chr3:187958482-187958582 | ATAACACATCAACCTTCTTTGGGATTCTTCCCTCAGACATGGTTTTGGTGGGAGGAGCATGGCAAGGGAGGGGCGAGCTCCAAATGCAGGGCTGCTCTGT | 302 |
| AC022498.1_chr3:187958582-187958682 | CCTCGGCGACCTGAGCAGACACACGAGCAGAGATCAGAGACACTCTTAGTGAATGAACCTCCCTATTGGCTATATTAAAGTAATGCTCTGAAAAAGTTCC | 303 |
| AC022498.1_chr3:187958787-187958887 | TATGTATGCATAGTCTAAAGTGATGATTTTAGAGGTAGCAAGACAGTGAGAATGTCCCTACATGTGAAATGGGCACAGTTTTATCAGGGAAGTGTCAATA | 304 |
| AC022498.1_chr3:187958887-187958987 | GAGGGTTAATGTTCCACGTAGTGGCTGCAAGAATGATAAGTGGTCATGGGGATAGCCTGACACTCTAGGAGCAGAAGGTGGTGGGTATGGATAGAACTAC | 305 |
| AC022498.1_chr3:187958987-187959087 | TGATATAGCATGAATCCAACCTGCTGTTATCTGCGCAGGCCTCTCTGCAGCTGTTTGCCCTGAAGTACATGCTGTACGTTTCTCCAGCTGATCCTGCATG | 306 |
| AC022498.1_chr3:187959087-187959187 | ACTGGGTATAAACGCCTGTCCGCTGTGTGCTGGACAGCCCCAGACACCCTCGGCAGCCTGCTGTGTTTGTGTGAGACATGCTGTGTTAGGGATTTAAGCA | 307 |

EP 4 334 476 B1

232

| Name | Sequence | SEQ ID NOs. |
|---|---|---|
| AC022498.1_chr3:187959402-187959562 | ACAGCTTTCTCATCTACATGGACAACCTATTTTTAAAGAATCTTCAGAGAGTCGTTGACTTTGTTATAACTACTACTATATACGTAATTTCAGATGATAG | 308 |
| AC022498.1_chr3:187959562-187959662 | AATTGAAAATTTAACTTGTTTTTCTAGAAAGAGTTTATTTTCCCTATAACTTCAAAGAGTAATGGTGGGGAGTAGGACATTCTGAAAATAAGAAGAAACA | 309 |
| AC022498.1_chr3:187959662-187959762 | TGTCAAATGAATTTCTGACTTCCAGCTAGGCATATGGAATAAAGGTCTTTATTCCAGTGACCTCTGCTCATTGGAAAACTTTGGGCTGGTAGATTTCATG | 310 |
| LPP_chr3:188299217-188299317 | TCTCTTGCATTCTTAACTTGCAATTTAGTACTGTTTATATTCTGCTTGAAGGTTAGAGACATTCGACTAAATGGTCTTTTCTCCACATTGCTGTCATTCA | 311 |
| LPP_chr3:188299317-188299417 | TTAATGTCCTGGTCCTGGACTTTACTCATTGACCACAGGACAAGTGGCTCAACTCTCCTGCCACTACCCAGGCTGTTAGTCCTGTTGGGAGGCTCAGG | 312 |
| LPP_chr3:188299417-188299517 | GCCCAACTCACTCATCTGTAACTCTCATCTCCATTCAGCTGCAGCCTCTACAGCCCCTGGTTATACCCTGGATCTTATCATTGCTTCGCTCTATTTTACC | 313 |
| LPP_chr3:188299517-188299617 | TCCTAAATCGTAAAAATTAAAACCAGCCTCGGAACACAACCCCTCATTCTTCCAGCACTCTCTCTCATTCAGGTAACTCCTATTCTACTTTTCTTCAGCA | 314 |
| LPP_chr3:188471412-188471512 | TTGTTTTTTTTTACTTTACCTTAATTTCTCTTTTTGGACTAAGATGTTAAAATGTTTCTTAATGTGACTGTCTCCGAAACTGTTTTGTGTCTACCACTCA | 315 |
| LPP_chr3:188471512-188471612 | TCCTAGTGGCAGTCATTGATCCTTTTCTTGTTGCGAGTGTTTGAGTGTGGGTGTGTGTGAGTGTGTATATGTATTTGTAGAGGGAAAAACAAGAGAGAGG | 316 |
| LPP_chr3:188471567-18847166 | TGTGAGTGTGTATATGTATTTGTAGAGGGAAAAACAAGAGAGAGGGAAACAGACATTGGAGCCACCTTTCCCCCACTAGCCACGTACCTGTTGAACCTTC | 317 |
| LPP_chr3:188471667-188471767 | AAGCCTCTCTATAGAATCAGATATACACAAGCACAGTGACAGAACTACATGTGTCCTACAGTCCAGCTTTTAAGATATGATAAAAACTCTTGTATTCACA | 318 |
| LPP_chr3:188471767-188471867 | GAGCTAAATGGCAATAACCATAGGAGATTGCATATTGCTACATTATGTAAAGACAGAGTCCCAAGAAAATAGTGAGAACTCAGTTTGATGTATGATGTGA | 319 |
| LPP_chr3:188471867-188471967 | TATGTGATATCTTACTTTACATGGCTAACAGTTGACATTCTTTGTGGATTCTATATTGTCTAAGGCTACAGAAGAGCCATATGATAAATTCATCGGCAAC | 320 |

| Name | Sequence | SEQ ID NOs. |
|------|----------|-------------|
| N4BP2_chr4:40198810-40198910 | CAGTGAAAAGGCTTGGGCCGCTTTTGTTTTCACCTGCTTTTGTTGAACAAATTTGATTTCCGGAGTCAGTC ATTTTACTGTCAAGACATTTCTTCGGCAT | 321 |
| N4BP2_chr4:40198910-40199010 | TCTGCAACAGGTAAGGATTTTGCTTCCTTAAAAGTATTTCTTTGGTGTCAAAAGAAATTTTTCTAATTTTA TTTAGCTTTTACTCTAGGCCAAACATCGT | 322 |
| N4BP2 chr4:40 199010-401991 10 | AATGACTCTGAGCTACCTGCTGTAAGGTGTAGAATCAATTTACAGGGGGACGGGGGTCGGGGGGGTGAG TGTTGCTTTGATATTCACTGCCCCTCACCAC | 323 |
| N4BP2_chr4:40199110-40199210 | AGTCCTAACAAGATTTTTGAAACATGAAAAGTTACAATAGTTGGCTTTTTGGTTTTCCAGATATTCTAGAG AATGCATATGCTTGTGACTGTGGCTGAGC | 324 |
| N4BP2_chr4:40199210-40199310 | TCAACTGTATGGGTAGTTTAAATACTACCCAAGGTTTGATGAAGTAAATCTAAAGATGCTCTAAGTTGTG CAAATATGAATTTTAAAGTTGTCTAGTTCA | 325 |
| N4BP2_chr4:40199310-40199410 | GAAAAGAAACAGAACCGAAGTCTAAATGATGTAGATTTCAATCTGGAATTTCTAGCTTGTGTTTTTCACCT ATTGCCAATGTTAATGACCATTTCCCAAA | 326 |
| N4BP2_ chr4:40199410-40199510 | AGTGCTCTATGATGTATAACATGTATTTTTTAATTAAATTTAATCTTTCTTCTGAGGTGGTTTGATTTGGAG ATATGCTACGAGGTACCAGTCAGTAGCC | 327 |
| N4BP2_chr4:40199510-40199610 | TGAGTTGTAACTAAACAAAGTTTGGGAAATCACCGGTTTTAGGTGCTTTACTAAATGAAAGTTGCCATTG ACGTATTCAAGCAGGCAACAAGTAGTTGGT | 328 |
| N4BP2_chr4:40199610-40199710 | GTCCCCTTATTGGTTCTAAGCTGGTGCCGTGGAGGATATAAGAGAAATATTTTAAAAATCTCTACTTTGAA GGACCCTATAATCTGGTAGTTGTGATAAG | 329 |
| N4BP2_chr4:40199660-40199760 | TTTAAAAATCTCTACTTTGAAGGACCCTATAATCTGGTAGTTGTGATAAGAAGTAAAATTTAGGAAGCAA TGCAAGATGAGAATTCAGTGATGAGTGGGG | 330 |
| N4BP2_chr4:40199760-40199860 | CAGCACAGGCTTGAAGAGTTCTGTGAATTCCATGGAGGGGGCCTGGGGGCAAACTGGAGTTGTCAGGAA GATCTGGGCTTTGGAAGAATGCGAAGTGTCG | 331 |
| N4BP2_chr4:40199860-40199960 | GTAGAAGGAGAAGGGGCAGGTGATTTCAGACTGGGAGGACCTTGTGGGCAAAGGCACAAAGGCGAGACT GACCTGGAGATGATAAGGCCAGTTGAAGAGA | 332 |
| N4BP2_chr4:40199990-40200090 | ACATTGCAGGAAATCAGATTAGACAGTTAGGGTGTGGACACAAAAGCGAGGACCTTGCAGGCACTGGGG AGAAGTGACCCCATTCAATAGTCCTTGGTCT | 333 |

| Name | Sequence | SEQ ID NOs. |
|---|---|---|
| N4BP2_chr4:4020009040200190 | CCTTCTGCCCTGCGGCTGCGCTTCCTCGGCTCTCACGGCACCAGCAGAATTCCATGTGAGAGGGAGCTTGT CGAGCGTGGCCTCTTCCCACTTGGGGCTG | 334 |
| N4BP2_chr4:40200190-40200290 | CTTTCTGCATCCCTGTGCCTGGCTGTGGGCCTCCATTTGCCCTCTACTGTCTTCCCTTAGGACATCATTTAT GCAGAGAAAGGTTCGTGTGGCTCGGGGT | 335 |
| RHOH_chr4:40200505-40200605 | GGACGTTGTTTAGAGAGTCAGTAGATCATAATAATTCAGACACTTTTTTTCTGGACCATAAAATATCTGAA CCCATATAATAACAAACATACAGCACGGT | 336 |
| RHOH_chr4:40200605-40200705 | GAATAAGAACCCAACTTTTGAGCCAGATCACTTTGCATGGAATCCCCATTCTATCATTCTATCATTTCTGG GCTGTGGGAACCTCAGACAAGTTACTTAA | 337 |
| RHOH_chr4:40200705-40200805 | CTTCTTCAATGCTCAGATTAAAAAAAAAATTCACAAATATCTCTAATAACAGTAATAATAACTGAAAAT ACCTACCTCAGAGGGTTGTCGTAGAGATCA | 338 |
| RHOH_chr4:40200730-40200830 | AAAATTCACAAATATCTCTAATAACAGTAATAATAACTGAAAATACCTACCTCAGAGGGTTGTCGTAGA GATCAAATGAGATAAAAATATGTAAAGCAT | 339 |
| RHOH_chr4:40200830-40200930 | GTAGCCTAGTGCCTGACTGAAAAAAAAAATCTCTCAATAGATGCAACTCTTATGATTCTTATTAAGGACTTG GCTATTGCCACAAATGAAGGTGTTATGAG | 340 |
| RHOH_chr4:40200930-40201030 | CCCTGGCTTAAGAGCAAGAAGCCTGCAAAGCTAACTCTCCTAATCCCAACATTCCTTTCCAGGGAAAGTA GGGTGACAGGTGGAGGCTGGGAATTAACGT | 341 |
| RHOH_chr4:40201030-40201130 | TTTTTGAGCACCAAATATGGACAAGGCACAGGGGTTGGGTGTTTTTCTAGTGAGAATACATATGAAAGAA GGAAAACAAACTTGGAAACCGCTATTTTAA | 342 |
| RHOH_chr4:40201130-40201230 | GCCATTTGGTAACAGTTTCTCTAGCTTATGAGATGAGAGAGGTCCTCTCAGTATCCGCTGCATTACTTGTG GGCCTCCTTGGTTGACGTCGCTCTCTGAA | 343 |
| RHOH_chr4:40201230-40201330 | CGCTTGGGGTGGAATTCTAGAGGTGCTTTTCATTAGAGGCAGAGAGCATGACCTTTCTTCCTTGCCCAGTT TAAATTAAATTATTTTATCTTACAATGTG | 344 |
| RHOH_chr4:40201330-40201430 | TTAATTTTAGTGCTAGCAAGGCACAGCTAAAATTCCATTTCTACTTAGGAGTGGGGATCATTGTGGCAGT GAGTGCTTATTTGGGTTTGGGATGCTTGGA | 345 |
| RHOH_ chr4:40201430-40201530 | TCTGGGTGAAAGCCAGGATTAAAAAGCATCCTCCTTCCCCATTCCACTCTCTAGGTTATAAATATTTTTTT GGATTAAAAGCCTCCTTTAAAAAAATGCA | 346 |

| Name | Sequence | SEQ ID NOs. |
|---|---|---|
| RHOH_chr4:40201530-40201630 | AATCCACCTGGCATGTTAATTGTGCAGGGGATTCCTAATTATGTGTGCAGATGACGTGAGTCACACGGTGATAGTGTTCCTTCTAGAGTCCCACTGGTGT | 347 |
| PABPC4L_chr4:134727698-134727798 | ACTAGGCGTTCATCCTGTGTAATTTGAAAATATGTCACACGTGGTGATGAGAATCTATTTGAGGAACATGGGCAGTTTGAAATAATATATGCAATGTATG | 348 |
| PABPC4L_chr4:134727798-134727898 | ACTAGTTTATATAATGAAAGGAAGTATTTAAAAAGATAGAATGACATAGACTAATCTAATTGAGAAATATGAAAGTCTAACAGAAATGATTGCTTGTGAA | 349 |
| PABPC4L_chr4:134727898-134727998 | ATTTTATGAAGAAATCCACAGATAAATTCTCCACCTTGATCTATGTAATCCGAAATTTAGATGTTAAAAATATGTTGATTCTGAAAATTTATATTTATTC | 350 |
| SLC38A9_chr5:54964698-54964798 | TTTGGTATGAATAGGTCAAAACAAGTCACCATTAACTGACAGGAAGCACAGAATTCTCAATTTAGTTTTGGCAAAGACATTATTTTATAAATATGAGTTT | 351 |
| SLC38A9_chr5:54964798-54964898 | TTAAATGATTCTTATGAAGAAACTAGCACCAAAGTGAATGCACTCTGCAAATAACTCCCAGCTTCTCTGAATTTCAAAAGCAGCCACTAAATATTATTAG | 352 |
| SLC38A9_chr5:54964898-54964998 | CAAATCAATTTAGCTGAAAGCGATGAATTACAGAAGTAAATCTTTAGGTACAAAGTAGACAGCTGACACACATGTAGCATATACACTAGTGATCTGCC | 353 |
| ZNF608_chr5:124079827-124079927 | TTCCTTCTTTACCAACATAGAGTTTCCCATGAGCCCTGAATCCGGGGCACTTTTGCTAACTTCCCCTGCAGCGGCGACGCTGCCACTCCCAGTGCCCCCG | 354 |
| ZNF608_chr5:124079927-124080027 | CAGTGGAAGGGGCTCGCGCCACCTCCATTGCTCTTGGCCCCAAAGCCATAGAGGTGCCCCCCGGAAGGGGCCTGGCTGCCACTGCCATTCTGGTGGCCCT | 355 |
| ZNF608_chr5:124080027-124080127 | GAAGCAGGTCGTGCTTGTCCTTCCTGGATTTCCCCGCATCCTTATCCCGCTTGGCGCCTCGGCTGCTCTGGCTTTTACCTGGCTTCTCCTCTTTGCTTTT | 356 |
| ZNF608_chr5: 124080127-124080227 | CCCACAGGAGCCTGCCCCCGCGGTGGCGGCAGAGGTGCTGGTGCTGGTACTATTGCTGTTTGGGTTGCCGCTGCCGCCGCTGCTCACACTTTGACCCAGC | 357 |
| ZNF608chr5:124080227-124080327 | GCTGAATTCATGCCAGTTGCCTCTCCAGGGCGCCCTTGGACTTCCTGCCTCTTGCCAGTGCTGCTGATCTCGGGAATCCCATACAAGGCAGCAGAAGGCA | 358 |
| ZNF608_chr5:124080327-124080427 | GAGATTTATTAGCATCCTTAGAAGTTTTACTCCTTTTCACTTTTGATTTGCTGGTCTCTTTGTGTGAATTCCCCTGGGGAGCAGAGGCCTGAACAGAAGC | 359 |

236

EP 4 334 476 B1

| Name | Sequence | SEQ ID NOs. |
|---|---|---|
| ZNF608_chr5:124080427-124080527 | AAATTTTAGGCCATCAGCTAAGGCTGCGGTAGCACCAGCCCCACTGGAGGCCGGACCTCCACAATCCTTGGAGTTGCTGCTACTAGTGGTGGTGGTGGAA | 360 |
| ZNF608_chr5:124080527-124080627 | TTATTCATCTCAAATTTCTGTCTGTCCTTCTCCAAATCAGCGTCCAAATCAATTATTAAATTTCCAACCCCGATTTCCCAATCATCGCCACTGTCATAAG | 361 |
| ZNF608_chr5:124080627-124080727 | TATCAACTGTATTTGGATCCACACCTTTTCCTGCAGTAGAAATGTTCACTGACATCCTGAAGATGAGCTCTCTAGAATAAAAATCCGATGAACTTTTCTT | 362 |
| EBF1_chr5:158527642-158527742 | TTCCTCAGGAATTTGAGCTGGGGATCTGCATCCTGGCCATTGCAGTCCTTTAGCATCCTCGCCGCGCCCTGAGCGCGCTGGAGGCTCGCAGGCTGCGCCC | 363 |
| EBF1_chr5:158527742-158527842 | TCCCAGGGCTGATGCCGCGTCCTGCTCCGCCGTTCTGGGACGTCGGGGACAAAAGTGGAGGAGACGGGAGAGCCCGGGCAGAAAAAGCAGGACGCGCGTC | 364 |
| EBF1 chr5:158527842-158527942 | CCAGGTGCCCACCTCTTCGCTTTGAGGCGGGGGCGGTGGGATGGAATATGGGTGCGCGAGGTCGGGGCTGGTAACTCTCGGAGGGGCACGGCCTCCACGC | 365 |
| EBF1-chr5:158527942-158528042 | TGGGAGGGATGAATGGACGCTGGGCCCCGGCAAATGAGGCGCTGTGGGTCCCCAGGAAGTGGGGTACCAGGCTCTACTCCCACCCCGGCCTCTGAAACGC | 366 |
| IRF4_chr6:392760-392860 | GGCCAGGAGGGGTGGCGGCTGGGTGGGGAGAGAGGGTGCAAGACGAGCGGCGCGTGTCGGGAGCCTTTGGGCTGCGGGTGCGTTACAGGAGAGCAGGCGG | 367 |
| IRF4_chr6:392860-392960 | GTAGGAGCCTTCGCGGGGGCCGAGCTCGGAAGGCGGACGGCTGTGCCCGCCCAGGGGATGCGCCCGGGCCGGCCGCGAAGGTGCCTTCTTCCGGGGGCCC | 368 |
| IRF4_chr6:392960-393060 | GGACGACCCTGACACGGCACGCGCGCGCTTCGCAGCCTCAAAGACTCCGGGGCCTCGTGGTCACTGGCGCAGGGGATCGGGGCGGGGTGCCCGGAGTGCG | 369 |
| IRF4_chr6:393090-393190 | CCCGCAGTGCAGAGCAGAGCGGGCGGAGGACCCCGGGCGCGGGCGCGGACGGCACGCGGGGCATGAACCTGGAGGGCGGCGGCCGAGGCGGAGAGTTCGG | 370 |
| IRF4_chr6:393190-393290 | CATGAGCGCGGTGAGCTGCGGCAACGGGAAGCTCCGCCAGTGGCTGATCGACCAGATCGACAGCGGCAAGTACCCCGGGCTGGTGTGGGAGAACGAGGAG | 371 |
| IRF4_chr6:393290-393390 | AAGAGCATCTTCCGCATCCCCTGGAAGCACGCGGGCAAGCAGGACTACAACCGCGAGGAGGACGCCGCGCTCTTCAAGGTCTCCGGCCTCGGGAGCCGGC | 372 |

237

EP 4 334 476 B1

(continued)

| Name | Sequence | SEQID NOs. |
|---|---|---|
| CD83_chr6:14117992-14118092 | CCCGGCGGCGGCCACAGCTCTGCAGCTCGTGCTGGCAGCGGCGGCAGCGGCTCCAGCCATGTCGCGGCGGCCTCCAGCTTCTGCTCCTGAGCTGCGGTAGGGCTCGCGA | 373 |
| CD83_chr6:14118092-14118192 | GCGGCCTGTCTGCGCTGCGCCCCGCGCCCCTCCAGGACACCCCTCCCGTCGGTGCTTGCTCACGACGCGCTCTCTCTTTCTTGTGTAGCCTACAGCCTGG | 374 |
| CD83_chr6:14118192-14118292 | CTCCCGCGACGCGCGGAGGTGAAGGTGGCTTGCTCCGAAGATGTGGACTTGCCTGCACCGCCCCCTGGGATCCGCAGGTTCCCTACACGGTCTCCTGGGT | 375 |
| CD83_chr6:14118292-14118392 | CAAGGTAGGTGCTGCCGATACCCACCGGGCTGGGGTTTGGTGGGCTCATTTGAAGACAGCAGGAACCATCTCCCCTAGGCTGGCGACCCTCTGTGGCTGCCA | 376 |
| CD83chr6:14118392-14118492 | GGTGGGGGCGGAGGGGCGGTCTCCCGCAGCTGAACCTGGAGTACCAGCCTCCCGTGCCGGCCTCCCCCACCCCATCCGCATCCAGGTACAGGGCCGAATTAG | 377 |
| CD83_chr6:14118492-14118592 | GTTTTTGCTCTCCGCAGACCTCAATCCCCTTCCTGTCACTGAAGGTGGCCTGAGATGAATGATCCACTTAAGATGTTTTTGGAAGGGCAGAGACTCTCATTT | 378 |
| CD83_chr6:14118592-14118692 | GGGATTAATTCTGGAGGCCACCTGTTGTTGGGCCAGCAGGTCAGGAAGAAAGCAACAGGGACCTAGATTTGGGCATTGGACAGGGGGAATGTCTCCAGA | 379 |
| HIST1H2BC_chr6:26123614-26123714 | CTCTCCAGTTCCTATATTCTAATACCCTCCGCGCCGCCAAATAAAATTTGGCGTCTGGCCACAGCTCTTTTAGTGGGTATCTGGGTGGCTCTTAAAAGAGC | 380 |
| HIST1H2BC_chr6:26123714-26123814 | CTTTGGGGTTAGGTGTTAAGAGCGCTTACTTGGAATGTTTACTTGGAGCTGGTGTTACTTGGTGTACGGCCTTGGTGCCCTCCGACACGGCGTGCTTGGCCAG | 381 |
| HIST1H1E_chr6:26156649-26156749 | CTCCGGCCCCCTGCCGAGAAGAGACTCCCGTGAAGAAGAGAGGCCCCGCAAGTCTGCAGGTGCGGGCCAAGGCGCAAAGCGGTCTGGGCCCCGGTCCGAGCTCAT | 382 |
| HIST1H1E_chr6:26156749-26156849 | TACTAAAGCTGTTGCCGCCTCCAAGGAGGCGAGCGGCGTATCTTTGGCCGCTCTCAAGAAAGCGCTGGCAGCGGCTGGCTATGACGTGGAGAAGAACAAC | 383 |
| HIST1H1E_chr6:26156849-26156949 | AGCCGCATCAAGCTGGGTCTCAAGAGCCTGGTGAGCAAGGGCACCCTGGTGCAGACCAAGGGCACCGGCGCGGTCGGGGTTCCTTCAAACTCAACAGGAAGAGG | 384 |
| HIST1H1E_chr6:26156949-26157049 | CGGCCTCTGGGGAAGCCAAGGCCAAGAAGGCCAGCAGGAGCGGCGAAGAAGCCCAAGAAGGGCGCGGCCAAGGCCAAGAAGGCCAGCAGGAGCGGCGAAGAAGCCCAAGAAGGGCGACGGGGGC | 385 |

(continued)

| Name | Sequence | SEQID NOs. |
|---|---|---|
| HIST1H1E_chr:26157049-26157149 | GGCCACCCCCAAGAAGAAGCGGCCAAGAAGAGACCCAAAGAAGAAGGCGGAAGAAGCCGGCCTGCAGCTGCTGGAGCCAAAAAAGCGAAAAGCCGAAAAAGGCGAAA | 386 |
| HIST1H1E_chr6:26157149-26157249 | GCAGCCAAGCCAAAAAAAGGCGGCCAAGAGACCCAGAGAGGCCAAAGCCCAGCGAAGGCCAAAGCCAGTTAAACCAAGGCGGCTAAACCAAAGACCGGCCCAAGCCCAAGGCAGCCAAGC | 387 |
| HIST1H1E_chr6:26157249-26157349 | CAAAGAAGAGGCGGCAGCCAAGAAGAAAAGTTAGAAAAGTTCCTTGGCCAACTGCTTAGAAGCCCAACACAACCCAAAGGCTCTTTTCAGAGCCACCCACCGCTC | 388 |
| HIST1H1E_chr6:26157349-26157449 | TCAGTAAAAGAGAGCTGTTGCACTATTAGGGGGCGTGGCTCGGGAAAAACGCTGCTAAGCAGGGGGCGGGTCTCCCGGGAACAAAGTCGGGGAGAGGAGTGGGA | 389 |
| HIST1H2BK_chr6:27114004-27114104 | CTCCTTAGCCAGACTCGATTACAAGCACTGCATGCATTACTCAGTGTGATAAGATCATGATAATCCCTTTAAAAAGATCGCCCGAATTTAAGCCTGGATT | 390 |
| HISTIH2BK_chr6:27114104-27114204 | AGGAACACGTGTTACAGCTCTAATATCGATAATTTAAGTGGCTCTTAAAAGAGCCTTTGGGGTTGGGCTTTAAGACGCTTACTTGGCAAGTTTACTTAG | 391 |
| HIST1H2BK_chr6:27114204-27114304 | CGCTGGTGTACTTGGTGACGGCCTTGGTGCCCTCGGACACGGCGTGCTTGGCCAACTCCCCGGGCAGCAGCAGGCGCACGCGCCGTCTGATCTCCCTGA | 392 |
| PIM1_chr6:37138284-37138384 | CCCGGCTCCGGCGTCCTGCGGCAGCTCCTCTGGGCACCGTCCCTGCGCCGACATCCTGGAGGTTGGGATGCTCTTGTCCAAAATCAACTCGCTTGCCCAC | 393 |
| PIM1_chr6:37138384-37138484 | CTGCGGCGCCGCGCCCTGCAACGACCTGCACCGCACCCACCAAGCTGGCGCCCGGTGAGAGCACCCCCCGCCTCCGGCCCGGCGGGGATGCGGGGCGGCGGCGGGGATC | 394 |
| PIM1_chr6:37138484-37138584 | TCCTGGGTGGGGGAGTCGGCGGCTCGCGGGGGCCGGCACTGAGTCCCCGTGCTTCCCCCTTTCCTAGGCAAGGAGAAGGAGCCCCTGGAGTCGCAGTACCAGG | 395 |
| PIM1_chr6:37138584-37138684 | TGGGGCCCGCTACTGGGCAGCGGCGGCTTGGCTCGGTCTACTCAGGCATCCGGTCCGGTCTCCGACAACTTGCCGGTGAGTGGGGCCCCGGCCCGGTGGGGAGGGC | 396 |
| PIM1_chr6:37138684-37138784 | GCGCCGGCGGCGGGGGCGGCGCACGGCGTGCTTTAGCCCGGACGAGGGAACCTGACGGAGACCCTGGGCTTCCAGGTGGCCATCAAACACGTGGAGAAGGACC | 397 |
| PIM1_chr6:37138784-37138884 | GGATTTCCGACTGGGGGAGAGCTGGTGAGTGCCCTGCAGGAGGCGACCCCAGGATGAGTGGGTGGGGTGAGGGGCGCCCCGACTCCCGCCCTAACGCGGC | 398 |

239

| Name | Sequence | SEQ ID NOs. |
|---|---|---|
| PIM1_chr6:37138884-37138984 | CCCCTCGCCCCTGCAGCCTAATGGCACTCGAGTGCCCATGGAAGTGGTCCTGCTGAAGAAGGTGAGCTCG GGTTTCTCCGGCGTCATTAGGCTCCTGGAC | 399 |
| PIM1_chr6:37138984-37139084 | TGGTTCGAGAGGCCCGACAGTTTCGTCCTGATCCTGGAGAGGCCCGAGCCGGTGCAAGATCTCTTCGACT TCATCACGGAAAGGGGAGCCCTGCAAGAGG | 400 |
| PIM1_chr6:37139084-37139184 | AGCTGGCCCGCAGCTTCTTCTGGCAGGTGCTGGAGGCCGTGCGGCACTGCCACAACTGCGGGGTGCTCCA CCGCGACATCAAGGACGAAAACATCCTTAT | 401 |
| PIM1_chr6:37139184-37139284 | CGACCTCAATCGCGGCGAGCTCAAGCTCATCGACTTCGGGTCGGGGGGCGCTGCTCAAGGACACCGTCTAC ACGGACTTCGATGGTGAGCCAGGCCCGGGA | 402 |
| PIM1_chr6:37139284-37139384 | GGGAGCTGCCCAGGTGACTCGGCCCGGCCCGGCCCAGTCCGGAGGCCTCGGCCAGTCTCCCGCGCCAGCC TTTTGTAAAGGTCATTGGGCCGCCTGGCTC | 403 |
| PIM1_chr6:37139384-37139484 | GATGCTAGCCGGGGTGGGACGCAGGAGAGCCTCCCAGCGTAGTAAAGCCGGGGATTTTCAGCCAGCTGA ACCTGTAATGTTTCTGGCATGATTTTATTCT | 404 |
| PIM1_chr6:37139484-37139584 | TCAAGTGGAATTCAGTTAGTTCCAGGCTTTCCCGATGAATAAGAGGTTGTGGGCAACCGGCGGTAGCCCA GATTTTTCTAAAGTCTGACCCAGTTTCCCC | 405 |
| MAP3K7_chr6:91004618-91004718 | CTCTAAACAGACAAAAGCAAAATATCTCATTAGGCATCATCTCCGCCAAGGTTCCCACTAGGCAGGAAAG GATTTTTATCTAAAGTAATTACCCTTTTTA | 406 |
| MAP3K7_chr6:21004718-91004818 | GTTAAATACACTCAACAGATGAAATTTACAGAGAGTGAGAGACTGCAGCACTAGACAGCGAAGGTGAAA ACCAGGAACGCCGCGTCTCGCCGCCCGCGGG | 407 |
| MAP3K7_chr6:91004818-91004918 | CCCGCCGGGAGACTGCGGGTCCGTCTCGCGGGTGGGGCGCCCCGGTCCCTCTCGTTTCCTGGAGGCCACA GGTCACGGCGACGGCGGTGACCGGGAGAGC | 408 |
| MAP3K7_chr6:91005793-91005893 | CGGGTCTGACAGCTGCTGCGGCTCGCGCGGACGCGCGCCTCCTGCAGCCCGCCCTCCCCATGCCTGACTT ATTACTCTCTGCTCCTCCTCCCTCTGCTGT | 409 |
| MAP3K7_chr6:91005893-91005993 | TCCAAAACACCCTTCGACGCCAGCAAAATACAATGCGCCTCGGCCGCCGTAAACAGCCGGGAGGGAGAG CACACATTCGGCGCGGCGCGGCCGCCGGCTC | 410 |
| MAP3K7 chr6:91005993-91006093 | GGCTCCCACCCCCTTCCCGTTCCTAGAAAATGCCATAAAAGCGGGCAGGGCGCGGGGAGGGCGGCTGCGC GCCCGGCGGCCGGGGCTCCCTTCCCGCGCC | 411 |

EP 4 334 476 B1

| Name | Sequence | SEQ ID NOs. |
|---|---|---|
| SGK1_chr6:134493732-134493832 | TATGAAACAGCCAGTGCTACGTCTCCTTTATACCAAAACTGGTAGCCTGAAGAGCTCTCAGGCTTACCTAT AAACGATGTTCAGTGAATGCAGGTAGCCC | 412 |
| SGK1_clr6:134493832-134493932 | AAGGCACTGGCTATTTCAGCAGCATAGAAACGAGCCCGTGGTTCCAGGAAGCAGCGTTCCCTCTGGAGAT GGTAGAACAACTGCAGGAGACAGAACAAAG | 413 |
| SGK1_chr6:134493932-134494032 | TCATTCTGGGTTGCAAATGAATTTAATTAGTTTTGACATACACAGCAAAAGAACAACTGCAGGAAGTGGC CCCAAGTAATCTATTAACTATAAACCTGAC | 414 |
| SGK1_chr6:134494032-134494132 | AGGTTGAAGGAAATGCTAATTCTGGTAACATTCTCCCCACCAAAAATCTTTGAAAACTTTTTTCTCAAACT AAAACAAAGCAGGCTGTGCAGAGACACTA | 415 |
| SGKI_clr6:134494132-134494232 | AGAGTTGACTTCTATCCCCCCTGCTCACCTCTCCACCATTAATGTAGTCTAGGACAAAGTACAATTTGTCA GCAGTCTGGAAAGAGAAGTGAAGGCCCAC | 416 |
| SGK1_chr6:134494232-134494332 | CAGGAAAGGGTGCTTCACATTCTTCAACAGAACATTCCGCTCCGACATAATATGCTTCTCCTAGGAAAAT GACGATTCAGATTTAGTGGCATGTTTCAAC | 417 |
| SGK1_chr6:134494552-134494652 | GAGGACATGAAGGAAGTGTACCAAAAGATCTTCAGATTTGAAATTACCTTTCCAAAACTGCCCTTTCCGA TCACTTTCAAGAAGTGAAAGTCAGATGGTT | 418 |
| SGK1_chr6:134494652-134494752 | TAGCATGAGGATTGGACGACGGGCCAAGGTTGATTTGCTGAGAAGGACTTGGCTAGAAAAAAAAAAAA GAATTTCTTTTAATACCATTGCTTCAAAGGA | 419 |
| SGK1_chr6:134494722-134494822 | AATTTCTTTTAATACCATTGCTTCAAAGGAAGACATCTATAACATAAACGATGTAGAAAATGTTACATCTA CAAATGACTGATGCAAATGACCATACATC | 420 |
| SGK1_chr6:134494967-134495067 | AATAAAATAATACTCTGACTCAATACTTAAATATTTATATCACTTGTTATGCCATAATGAAGCATTCCTGC CTTGATACTAATTTCTAGAAATGCTATTT | 421 |
| SGK1_chr6:134495067-134495167 | TAATCCATTAATGTAGGAATACTAACTGACTCCCTTACAGTTCTCCACAGATGCACGGCACATACAAAAA CTTACTGGAGGAGAAGGGTTGGCATTCATA | 422 |
| SGK1_chr6:134495167-134495267 | AGCTCAGGCTCCTGAGGTTGGGAGATCTTCAAGATGGACTGAACTTCAGGGCTGCAGGGAATAAAGGGC ACGATTTAGAATCCAGCTCGCCACTAGGGGG | 423 |
| SGK1_chr6:134495267-134495367 | CACACCAACATCAAAAGTGAGTTTCTGGCTCTACCGACTTCTACCCGGATAATTCACTGTTTAAACTGAAA ATACCCCAATACATTAGTCAGTTAAAGAA | 424 |

| Name | Sequence | SEQ ID NOs. |
|------|----------|-------------|
| SGK1_chr6:134495367-134495467 | AATAATAAACCCCATTAAATACAGAAATAAGGATTGTTGCTCATGGAGAAAGGCCGTGAATTCGGCCAAC ACGAACCATTTATCTTACATCTCCAGTTCA | 425 |
| SGK1_chr6:134495467-134495567 | AGCCAAATCAGCAAATTAACTTTAATGTTTAAAATGTGTCAAATATATTAGAATTTAAGGAGAAATGAGA TCCCCACCCCAGAAGAAGTCTTCGCCTTCC | 426 |
| SGK1_chr6:134495567-134495667 | CGATAAACGCCGTGATGAGAATGTTTACCGCTGGCAAATTCAAACTATACTAGTTATTTCCTCAAATCCGG TCAAACTTACTGTTTGCATGCATAGGAGT | 427 |
| SGK1_chr6:134495667-134495767 | TATTGGCAATCTTCTGAATAAAGTCGTTCAGACCCATCCTCCTCTGCTTCATGAAAGCTGTGGATGAAGGA GGAGAAATAAAGAAACGTTTAGACGGCTT | 428 |
| SGK1_chr6:134495767-134495867 | CATAACGTCCGGCGCCACACACTAATCTGATCCGGGACTTTCAAAAAATTTCCACTTTGCGTCTCCTGG AGCAGAAGTCCCGCAAGATTCCTGCACTC | 429 |
| SGK1_chr6:134495867-134495967 | ACCGATGAGAATTGCCACCATGCCCCTCATCCTGGAGTAAGTGAGGGTGCCCTTAGCAGCCTCAGTTTTC ACCGTCATCACCACCGCGGGGAGACAGAAA | 430 |
| SGK1_chr6:134495967-134496067 | GACGTTAGCGCTCAAAGACCGGCTCGGCGTATGCTGCGCCAGGCCGCGCGCTCGGCCTTATAAAAAAGGC ACCGCCGCGGGGGCGGGGCCTGCGCGACAG | 431 |
| PLEKHG1_chr6:150954420-150954520 | AGGGTGAGAGGAGTCACCAGGTAAAGATGGGTTGGAAGGACCTGGCAGGCAGAGCAGGGAGCAGGACC CCAGTCCAGGGCAGCAGGGAAGCGGGAGTCTG | 432 |
| PLEKHG1_chro:150954520-150954620 | GGCAGAGCTGATTCCAGGCAGCTCAGTATTGCTGGCCTGTGCATCCTGAGACTTATCCGAGTCGCAGGTG AAGCTGGTGGGAATCAGGCAGAGTGCAGAG | 433 |
| PLEKHG1_chr6:150954620-150954720 | CTTTAGCTGGGGCAGGGTTAGCCAAGAGCCTGTCATGGAGCTGCTCTCTGGGCACTGGGAAACATAAGTC TGGAGGCTTTGGCTGCAGCTGCAGATAAAG | 434 |
| PLEKHG1_chr6:150954720-150954820 | ATGCAGGGGCCTCTGACGATGGGGGCCTTAGTCATCTCAGAGGTGGTGCAGAGGGTAGAAGCCTGACTG GGGTCAGAGATGAGGAAGGAGAGGGTCAGAA | 435 |
| PLEKHG1_chr6:150954820-150954920 | ACAGTGATTCTAAACCAATTTGGTTGAGGCAGAAGATACTAATGGCCGAGGGGAGGAGAGAGGGAGCGT AGGCTCTAAAGGGGAAGCTTGTTAGGAATGA | 436 |
| EZR_chr6:159238415-159238515 | AGACAGAGGCGCAGGCACAGCCCTTTCATCAGCTGACCAGGAGTGCTCGGCCCGGCCTGCCAGGAACCTC TTATCAAACTCCACCGGCTGCCTGCATCTA | 437 |

| Name | Sequence | SEQ ID NOs. |
|------|----------|-------------|
| EZR_chr6:159238515-159238615 | CAATTCAAGTCCATGGCTAACCTTCTGTTAGAGACAGAAATTCTGCTGCAGCCAGCAAGTTTGCTGGTGTACAGGGCACCGCTTCATGGGCCTAGTAGGA | 438 |
| EZR_chr6:159238615-159238715 | AGCGAAGCTGAAAGGCAACTTCCGAAAGCCAGTCTCCTCTCCCAAACGCCCTTTAATATCTCCCCAGTTGGATCTGGGGCGCCTGTGGTTTCGGACCCTT | 439 |
| EZR_chr6: 159238715-159238815 | AGGAGCTCTGAGAACTGGTGTGTGTGGTCGGAAGCCATCTGAGTCTCCCTGTGATTTGGACTTTTTAAGAAACTTCTAAGTTGTATTACTATACCCTTTA | 440 |
| IMMP2L _chr7:110545276-110545376 | TTCCCTTGTCATATGACTTCCATCCTCAGCACTACAATATTATCATTAATGTTTAAATCATTGTCAAGTCTGTGATTGCCTTAGAGATTTATTAAGAATA | 441 |
| IMMP2L_chr7:110545376-110545476 | ACATGCTAGGATTAGGAAAGTTTAACTTTTTACCATCCTTAAAATTAGATTTTTGAAAACTGTCTTATCCCCATTAAAGAAAAAAATAAAAAGGATGAAT | 442 |
| LRRN3_chr7:110697971-110698071 | TATACATACCTGCACATATATACAGCATATGTATATGTGTCTGTATTATATGTATTAAATGAAAGATTATCCACATTTTGTTCTTAGGATCTTCAGCAG | 443 |
| LRRN3_chr7:110698071-110698171 | CTCTCTTCCCATCACAATAGAAAGGCCTGAGCTAACATTTCCATTTCTGCAAAAGGCAGATTTTGTTCAATTAAAAATTATAATGCCTTAAATTTCCACA | 444 |
| LRRN3_ chr7:110737411-110737511 | GACATTTAAGAGACTTCGTTTTCACTGTGATAAACAGGTTTGATTTGGACTTATAACTTTTTTCTAAAATTATCAAATTAATAACGACTATAATGAAATA | 445 |
| LRRN3_chr7:110737511-110737611 | GAGGCAAATATTTTAGAGGATTCATTCCTTGGGGTAACATTTGTTCTATAATTTATAGTCTCATAATGTTGAGAGATTAAAGCATTTAAATAACATTGTC | 446 |
| LRRN3_chr7:110737611-110737711 | AACTAACTTTCAGCTTACCTTTCTTAAGGAAAAAAAACAAAAAAATGTTAAAAATAGACATGTATTTTTCAAACATACAATTCATGTTTTTATGTCATTA | 447 |
| LRRN3_chr7:110746681-110746781 | AAGAGATGTGAGGGACTTATAAATAATATTAAGATAACAGGAATTAAAGTCTCGGTGTGTGAAAATACTGTATATCTAGGATGCACATAAAAACTGCCCT | 448 |
| LRRN3_chr7:110746781-110746881 | TACAGATCTTGCAGGGAAAAGTACCTGACTATACTGTATAAGACTTCTGCTGTACCATTTAATCATACCAAAAAAAATGGAATCAACACACAAATAGATT | 449 |
| LRRN3_chr7:110746881-110746981 | TCTTTTCCACTGTTCTCAATTTAAAAATAATTGGAGAAATGTGTGCTTTGTTTAGAAGAGTAAAGGAAAACATTCATTCAATAGTACCATGCAGAATGAT | 450 |

EP 4 334 476 B1

(continued)

| Name | Sequence | SEQID NOs. |
|------|----------|------------|
| KMT2C_chr7:151943421-151943521 | CAGAAAAATAGAAAGATTATCATGGATTTGGGAATCAAAGACAGCTCAGCAAAATACTAGGACATGGCTCATATAAGATGGAATAAGCCTGGAAATACA | 451 |
| MYC_chr8:128750367-128750467 | CTTTAGGGGATAGCTCTGCAAGGGGAGAGGTTCGGGACTGTGGCGGCCACTGCGCGCTGCGCCAGGTTTCCGCACCAAGACCCCTTTAACTCAAGACTGC | 452 |
| MYC_chr8:128750467-128750567 | CTCCCGCTTTGTGTGCCCCGCTCCAGCAGCCTCCCCGCGACGATGCCCCTCAACGTTAGCTTCACCAACAGGAACTATGACCTCGACTACGACTCGGTGCA | 453 |
| MYC_chr8:128750567-128750667 | GCCGTATTTCTACTGCGACGAGGAGGAGAGAACTTCTACCAGCAGCAGCAGAGCCGAGCTGCAGCCCCCGGCGCCCAGCGAGGATATCTGGAAGAAATTC | 454 |
| MYC_chr8:128750667-128750767 | GAGCTGCTGCCCACCCGCCCCGTCCCCTAGCGCGCCGGCTCTGCTGCCCTCCGGGGCTCTGCTCCGCCTCCTACGTTGCGGTCACACCCTTCTCCCTTCGGGGAGACAACG | 455 |
| MYC_chr8:128750767-128750867 | ACGGGCGGTGGGGGAGCTTCTCCACGGCCGACCAGCTGGAGATGGTGACCGAGCTGCTGGGAGGAGACATGGTGAACCAGAGTTCATCTGCGACCCGGA | 456 |
| MYC_chr8:128750867-128750967 | CGACGAGACCTTCATCAAAAACATCATCATCCAGGACTGTATGTGGAGCGGCTTCTCGGCCGCCGCCAAGCTCGTCTCAGAGAAGCTGGCCTCCTACCAG | 457 |
| MYC_chr8:128750967-128751067 | GCTGCGCGCAAAGACAGCGGCAGCCCGGCAGCCCGAACCCGGCGCCCACAGCGTCTCGCTCCCACCTCCAGCTTGTACCTGCAGGATCTGAGCGCCGCGCCGCCTCAG | 458 |
| MYC_chr8:128751067-128751167 | AGTGCATCGACCCCTCGGTGGTCTTCCCCTACCCTCTCAACGACAGCAGCTCGCCCAAGTCCTCGCCCTCGCAAGACTCCAGCGGCCTTCTCTCCGTCCTC | 459 |
| MYC_chr8:128751167-128751267 | GGATTCTCTGCTCTCCTGCGACGGAGTCCTCCCCGCAGGGCAGCCCCGAGCCCCTGGTGCTCCATGAGGAGACACCGCCCACCACCAGCAGCGACTCTGT | 460 |
| PAX5_chr9:37024919-37025019 | GCTCCCCATCTGTCCCCACAGTTGCTCCTTGGCTGAGCCAGGTCCGAGGCAGGCCAAGGGCTTGCTCACCTCTCAGAGCAATTGCCCTAACTGGTTTGTTTTGGGCTTACATTGCAA | 461 |
| PAX5_chr9:37025019-37025119 | GATCAGGTCCTCCCCAGAGCCAGGCTGGAGTCCGAGGCAGGCTGGAGTCCGAGGCACTGGGGTCACCACAGACTGGAAACCGGTTGGGCGGCCAGGCCCC | 462 |
| PAX5_chr9:37025119-37025219 | AAACCTTGAGGAATCGTTTGGGCTGGGACCAGAACAGGGGGCTCCTCTGCACAGAGCTCCCCACCGCTTTGGTGGATTACTTCAGAGACTCAGAAAATTGAC | 463 |

| Name | Sequence | SEQ ID NOs. |
|---|---|---|
| PAX5 chr9:37025219-37025319 | ACAAAGAGAAACTGACCTGCCCGCAGCCAGCCCTGGCTGCCTACACAAGCTTTCCCCTGCTTGCCAGGCC ACTCAGCACTGCGTGGCAGACACGGACATG | 464 |
| PAX5_chr9:37025319-37025419 | CTCGCCCCGGGAAGCTCACCTTCACTCCAGCCGGGTCTCTGCTGCCTTTGTTAAATAGGGGACCTGCGGCT AGGAAAGCTGGATCCCAGGCTGTTGGGAT | 465 |
| PAX5_chr9:37025419-37025519 | GGGGGGGAGCGGGGTGGGAGGACCAGGCATGGGGACGGCTCCTAGCCCGGGAGCAACTCCCTGACCTGA AGCCCGCAGAGACCCCGAGCGGCACCCGAGC | 466 |
| PAX5_chr9:37025519-37025619 | CGAGGCTGCCGAAGCCTGTCACCTTCCTCCAGCCTGGCTCTGCAGCAAACAGAAAGGAAACGCGATTCGT TCCACTTGGAATTTCCTTGAAATCTCCGAA | 467 |
| PAX5 chr9:37025619-37025719 | TCTAATCCGGCGTTAACTCACCGTGAGAGGAGCGCTCATCTCACAGGAGGCTGTGGTAATGGGTGAATTG GCAGGATCCCTGCGGGCCAGGCAGCCAGGC | 468 |
| PAX5_chr9:37025829-37025929 | TTTTCGTTTCTTATCCTCTTTTTTTTAAAGGGGAGAAGCCATGAGAAAAGGCGTCCTGCAGAGAAGGACCC AATGGGGTCTTTAAGGGTCTCTGTATGAAC | 469 |
| PAX5_chr9:37025929-37026029 | TGGCCGGCTCCTAAGCAGAAGCTGAACTCAGAAACCGCTACTTCCTTGATTTTTCAAAGCCCCCTCCTCAA CTCCAGGACGCCTTTGGAGCCCTAGCCCC | 470 |
| PAX5_chr9:37026269-37026369 | TGTCGCCGCCGGAGCCTTGAAAGGCTGCAGCTCGCTGCCCAAGCTACGCGTTGCCGGAGGCGGGATTCCC AGGTGCCTCAGCCCGGGCGGCCAAGTGCGT | 471 |
| PAX5_chr9:37026369-37026469 | TGTTTCAGGTCCCCTGCCTGGGATCCCTGCACTTTGCAAAGTTAGCTGCGCGGCTGCAGAGGTCCGAGATC CTTCCGGCCTTAGTACCTGACCCACGGTC | 472 |
| PAX5_chr9:37026469-37026569 | CGGCACCCCCAACCCGGTCCCGGCGGGAGAGTGAGAGAAGCGAGCTCGCCGCCTACTTACTATGCATGGA TGCAAACGGGTCGTGCTTACAGTGTATTTC | 473 |
| PAX5_chr9:37026569-37026669 | CATCGGGGCGCTCCAGACTGCAGGCCGGCCCACGCCGCCGCCTCCCGGCGCCAAGGGGCTGCCCAGGGCG GATAGGGAGCCTCGCCACCAGGCCAGGCAC | 474 |
| PAX5_chr9:37026669-37026769 | TGTGCGAGCTGGGCTCAGAAAACACTGCTGGAGCTTCGGGGTCTCTCTCAGAGCCTCCCTGCTGGAGACC GCCCGGAGCTGCGCGGAGAGGCGGGAAATG | 475 |
| PAX5_chr9:37026769-37026869 | GTGCTAGCGCACCCGGGCTAGGAGCGGGTGCCCAACTCCGGCTGGCTTCCCTCCCTGGCTGGCTCAAGCA GCAGCTCCGGGCCCAGCCCGGGGTAGCTGC | 476 |

| Name | Sequence | SEQ ID NOs. |
|---|---|---|
| PAX5_chr9:37026869-37026969 | GGCCAAGGCGCCCGCGGCTTCGGGGGGCATAGCGTAGGGGCCCGCCTCCGGGACAGCCAGCAGCCCCCGG CCCCAGGAAGGAGCAGCTTTGAGGAGGCCGC | 477 |
| PAX5_chr9:37026969-37027069 | CGGAACAATCGGCCCTTGACTTCACTCAGGGGGCGGAGAGACCCGGGGGCTGCCAGGCTGGTTCCGCGGC CTCGATGCTTCTGAGGTCCCTCCTCGACCC | 478 |
| PAX5 chr9:37033619-37033719 | CACACAGGCAAACAACTTTTGGACACAAACTCATATATTTTTACATCTTTTAAAAATACATATACTGTAAT GAACACACTGAGTCCCTTATATAAACACA | 479 |
| PAX5_chr9:37033719-37033819 | CAGGCCCTAACTTGCAGACCCCCGGAAGGACGCCAGCGTGAACATTCAGAAACAGAGAAAAACACAGAC AAACTCACAGATATTTGGACTGATGCAGAAG | 480 |
| ZCCHC7_chr9:37293169-37293269 | ACAGTTTGAAGTGTGAGCCTGAACATGTTTGATCTAAGGTCTGGAGGAAGATGTGAAGCAAATCTGACCT AAAAAAAATTATAGGAAAAAAGCAAATTGT | 481 |
| ZCCHC7_chr9:37293269-37293369 | TCTGGATTTGTTTCACCAAGGAACAAGTAAGCAGAGAACCAGACACTGGAGAAAAAAAGGAGTCAGGAA GTAGACAAGGAAATGTTAAAAGAAATAATAG | 482 |
| ZCCHC7 chr9:37293369-37293469 | GATAACTGAAAGAATGTAGCTTCCAGATTGCTAGCTATCAGCAGATAGATAGAAACTTTTATACAGCCTT TAAATCTTCCCTAGAAACCTTTTTAAAAGT | 483 |
| ZCCHC7_chr9:37371494-37371594 | CAAGGGCCTGCCAGGATGAGAACGGGCAAACCTGGCCAAGGTGACCCCATTAGGGACTACCCTCCTAGG GACAGCACTCAGGGCCGTTCCCAATCACCCC | 484 |
| ZCCHC7_chr9:37371594-37371694 | GGATTTCCTGTCCTGCTCGTCTCCTGCCACACCTCCTTTTGATCTACCCCCAAGACACCCCTACCTTTTTAT TCTGTGAAAATTTACTCATGCTGTGGGC | 485 |
| ZCCHC7_ch:9:37371694-37371794 | CCTGCTGGAAATGCCCTCCTACTGTTTCCCCAAACCCCGTCAGAAATTCCACGGGGAAACTCCCTTCCCTT CTGCTGCAGGCACCGTCACTGTGTCTCTC | 486 |
| ZCCHC7_chr9:37371794-37371894 | AGCTCTGCCCCCCAGCCTCTGAGTACCACCTTATCCTAGCCCTTAGCTACTGGCTTGTCATTGTCTCTTTAC GTTCTCAGCCTCCCACAGAAGCCTGGGA | 487 |
| ZCCHC7_chr9:37384684-37384784 | AGGCACACTCGCCCCTGGTCTCCAAGGCTCTGGGTCCTCAGACTGGCTGAGTACTGGGGACCAAGGTCAC CCAAGAAGCCCTGAGTGGCCCTCTTGAGGG | 488 |
| ZCCHC7_chr9:37384784-37384884 | TTAGCAGAGCTTCTCTCTGTCCAAGACAGGTCAGGCTCTCTCCCCTGGCCCCAGCTCCACCGTCACTCAGA GGAGTGGCCTAAACAAACGCTGCAGGTGA | 489 |

| Name | Sequence | SEQ ID NOs. |
|---|---|---|
| ZCCHC7_chr9:37384884-37384984 | GGCTCCCGAGCCCCTGACATGGATGTTTATGGAAGAGGACTCTTGGCATCAGCACCTGGGCAAGGTGGGTAGAGGCAGGAGTGGGCAAATGGGAAAGTCT | 490 |
| GRHPR_chr9:37407369-37407469 | GGAGAGCCGTTTGAGATTCACCAGGTGAATGAACCCCGGTTTTTTTCTGGGTAACAGGTCGAATGTGAATTACTTATTTTCACAAGCTCTTGACATGTTC | 491 |
| GRHPR_chr9:37407469-37407569 | CGTCAAATTGCTGTTCCCCAAAGAGTGGACTCTGGTGACATATAAGTGTGTGGGACCATTGCATCTTACCCCAGAGATCCACTCCTGATCTGGCATTATT | 492 |
| GRHPR_chr9:37407569-37407669 | CAAAATCTGCTGAATTCAAAACGATCCTGTACTTCCTGCTCACCAGGTCTGAAAAGAAAAAGAAAAAAGAAGAAGGAAAGACTACACCTGACAAAGAC | 493 |
| FAM208B_chr10:5755066-5755166 | TTCACGGTTTCTCTTTAGTTTTATCTGAAATACATTTGTAAGCTTAGGGTGCAATTTGGATTAAAACAGTTTTCTTTAGTGTCAATAATGGCCTTTACTA | 494 |
| FAM208B_chr10:5755166-5755266 | GAGTGAATGGATATTTTTCCATTCTGGATTATCGTTTAATCGAAACTTTGTTTCCTGTGGAAATTTTTCTGGTTTAAGTTATTTGATTTGGGAGATAAAT | 495 |
| FAM208B_chr10:5755266-5755366 | CATGTAACTTAATAAACTTTGGCATCCTGGTTAACTGAAATTGCTTCATTCAATATTTGAAGACTGAAATCTGTATTGTTGCCTGTACCTAAATTATGGG | 496 |
| FRMD8_chr11:65190342-65190442 | GGACAGACAGGGAGAGATGACTGAGTTAGATGAGACGAGGGGGCGGGCTGGGGGTGCGAGAAGGAAGCTTGGCAAGGAGACTAGGTCTAGGGGGACCACA | 497 |
| FRMD8_chr11:65190442-65190542 | GTGGGGCAGGCTGCATGGAAAATATCCGCAGGGTCCCCCAGGCAGAACAGCCACGCTCCAGGCCAGGCTGTCCCTACTGCCTGGTGGAGGGGGAACTTGA | 498 |
| FRMD8_chr11:65190542-65190642 | CCTCTGGGAGGGCGCCGCTCTTGCATAGCTGAGCGAGCCCGGGTGCGCTGGTCTGTGTGGAAGGAGGAAGGCAGGGAGAGGTAGAAGGGGTGGAGGAGTC | 499 |
| SCYL1_chr11:65266552-65266652 | GGGGCAGGCGGAGCTTGAGGAAACCGCAGATAAGTTTTTTTCTCTTTGAAAGATAGAGATTAATACAACTACTTAAAAAATATAGTCAATAGGTTACTAA | 500 |
| SCYL1_chr11:65266652-65266752 | GATATTGCTTAGCGTTAAGTTTTTAACGTAATTTTAATAGCTTAAGATTTTAAGAGAAAATATGAAGACTTAGAAGAGTAGCATGAGGAAGGAAAAGATA | 501 |

EP 4 334 476 B1

| Name | Sequence | SEQ ID NOs. |
|------|----------|-------------|
| SCYL1_chr11:65266752-65266852 | AAAGGTTTCTAAAACATGACGGAGGTTGAGATGAAGCTTCTTCATGGAGTAAAAAATGTATTTAAAAGAA AATTGAGAGAAAGGACTACAGAGCCCCGAA | 502 |
| SCYL1_chr11:65266852-65266952 | TTAATACCAATAGAAGGGCAATGCTTTTAGATTAAAATGAAGGTGACTTAAACAGCTTAAAGTTTAGTTT AAAAGTTGTAGGTGATTAAAATAATTTGAA | 503 |
| SCYL1_chr11:65267397-65267497 | TTGGAGAAGTATAGAAGATAGAAAAATATAAAGCCAAAAATTGGATAAAATAGCACTGAAAAAATGAGG AAATTATTGGTAACCAATTTATTTTAAAAGC | 504 |
| SCYL1_chr11:65267497-65267597 | CCATCAATTTAATTTCTGGTGGTGCAGAAGTTAGAAGGTAAAGCTTGAGAAGATGAGGGTGTTTACGTAG ACCAGAACCAATTTAGAAGAATACTTGAAG | 505 |
| SCYL1_chr11:65267597-65267697 | CTAGAAGGGGAAGTTGGTTAAAAATCACATCAAAAAGCTACTAAAAGGACTGGTGTAATTTAAAAAAAA CTAAGGCAGAAGGCTTTTGGAAGAGTTAGAA | 506 |
| BIRC3_chr11:102188381-102188481 | TGGTGTAAGAGATGTGCCAGCGGCTGGCCGAGGGGCGCTTAGGGCTAGAGCCCGGGGCGCTGCAGAGGT TGAGAGTCAGTGGGTGGGGCGCAGTTATCAA | 507 |
| BIRC3_chr11:102188481-102188581 | ACACCAGGGCCCAAAAGCAGGCTCTAGATAGGTTCCAGGTGCTCAATTTCTATTTCACGTTTGGAGTGAG CCAGTGGAATTGTGAAGTTGTGGCATTTTG | 508 |
| BIRC3_chr11:102188581-102188681 | ATTCGGTTGCCAAGAGTTATCACTGGGCCTTTGCAGGTGCCAAATAAATTTCAGGACAGAGCCTAAGGCA GAGCTCTGGCACAGGAAGGAAGTAAAACGT | 509 |
| BIRC3_chr11:102188681-102188781 | TTAATGAGCAAATGGACGCATGTTTCCAAGCGGTGGTAGGAAGACAGCAGTTTTTGGTTGTCTTCCTGGT GATCAGCATGGAAACCTAGTAGTGCTCTTA | 510 |
| BIRC3_chr11:102188781-102188881 | CTCTGATCAATACATTGTCGAAGGCATGTACCTGATGCTAACGTAACAATAATATTAAATATTGACTTTAT TTGCTATTATTTATTGCTAACATTAAGTA | 511 |
| BIRC3_chr11:102188881-102188981 | CTGCTACCTGCTATGTGCTAGGTTTGTCTCTGAAGACTTTACATGTATTTTTCACGTTTAATTATCATAATC TTAAGAAGCAGGTACCATAATTATCTCC | 512 |
| POU2AF1_chr11:111249311-111249411 | GGGAAAAAGAATGACGAAAGGCAAGACAGTGGAGCAAGTGAGGACACGCTTCACCGAGCCAGATCTCCA CTCCTCCCAGGGTATCCACAGGGACAAGTCA | 513 |
| POU2AF1_chr11:111249411-111249511 | CACCTGGCAGAAAGCTAAGTCACTCAGCTAGAAACAGGCCCAGGGAATTCAACAGAAGGCTGAAGAGCC ACTGCTTATGGAAATAAAGCCCCTCCTGTAA | 514 |

248

(continued)

| Name | Sequence | SEQID NOs. |
|---|---|---|
| POU2AF1_chr11:111249511-111249611 | AGAACTGCATGGCTTTCCCTCCCAACCCCAAACCCATCCCACATCTCGGCTTTCTTGTGTGAATCATAAACTGCCCTTTCTTCACCACAGTGATTCATG | 515 |
| CXCR5_chr11:118754793-118754893 | AATCCTCTCCCACTGTGGATCTGTAAAATCTAGACACAGGTCAGTCAGCTCCGCGCCCTTTAAGAGTTTATTTTCCATTCTGTGGAAGAAGCAGATAAGGAGA | 516 |
| CXCR5_chr11:118754893-118754993 | GCTGCTGTCCTTAGGAGAGACATCCTTAGAGGAAGCTGGAAGAGACACGGGTTCAGGCCCTGCATCCTCCTCTGAGTTGCTATGTGACTGGGAACAGGATACT | 517 |
| CXCR5_chr11:118754993-118755093 | TCACCCTCCATTCTTCTTCTCCTTTCTCTTAGGGTCGGAATATGGAACTAGACACAGGAAAGTACTTTGGAGGTTTTCTTACCGTAAGGAGGCTGGCATT | 518 |
| ETS1_chr11:128391383-128391483 | GGGCCCTCCACCCAGCCTCAGTTCTATGGGGGACGTGGAGTCAGGCGATGATGTCCTCTGAGGCAGCGTCCATCTCCCCTTAACATTAAGGAATAAGGCC | 519 |
| ETS1_chr11:128391483-128391583 | AGAGGGGTTCTCGCTCATTTGGGAAAATAAAAAAAGCAGGAATGGGGCGCTGGAAATTCTATAAGCTTTTCCCCACCACTCACAAAAACACAGCTGTGAAA | 520 |
| ETS1_chr11:128391583-128391683 | ATAAATACCACCCCCCAAACCAAGGGTCTAGGGCCACCAACAGTCCTCCTCCTCCTCCTCCTCCTTCTCCTCCTCGTCCTCCAGATCCAGCTGCCAA | 521 |
| ETS1_chr11:128391648-128391748 | CCTTCTCCTCCTCCTCGTCCTCCAGATCCAGCTGCCAACAGCATCCCCGCGTCCTGAAGAAATGCACCGCCCAGAAGGGAACGGCGAAAGGGGAAGAAGTCC | 522 |
| ETS1_chr11:128391748-128391848 | AGGGGGACCCCGGCCTCTGGCCGAGAGCTTGGGTGGGGGGCCTCGCCGCGTCGCCACTCACCCGGGGGAGGGGAAAAGCTCCAGATCGGACTTTTTCCGTCTTG | 523 |
| ETS1_chr11:128391848-128391948 | ATGATGGTGAGAGTCGGCTGGAGATCGACGGCGCCGCCCTTCATGGTGCCAGGAGTGGGGGACGTACGGGATGGTAGCAAGTTGCAGTTACTGTGTTTTTC | 524 |
| ETS1_chr11:128391948-128392048 | TTTTTAATGAGGATTAGTAACACAGGGGAAGGGGACGGGGGAAATCCGACTTTCTTCCCAAAAATCTCAAATTCCCGCTGCCTTTCTTTCCCCGCGCCCG | 525 |
| ETS1_chr11:128392048-128392148 | GACGGTGCGCGCCCGGCACTCCAGGGGAAGTTGGCACTTTGCGGCGGAAGTGAGCGGCGCTCGGGTCCCAGCCTCGCCCCGGCGCGCCCCGGCGCCGCTCCTCCTGCC | 526 |

249

| Name | Sequence | SEQ ID NOs. |
|---|---|---|
| LRMP_chr12:25205888-25205988 | GAGTGAGTAGCAAATATTCATTTATGACCCAGTTTTTGTCCACCCTCAGGCGGGGCATAGGACTACAGAC ATTTTTCTAGATTACAGCTAGGATATTATT | 527 |
| LRMP_chr12:25205988-25206088 | CCTGAGTTTATGACAATGAAATGGTTTGAGAAGGCAATATTGTGGGGCTTTCAGAGAGGTTTGCTGAGTG GCTAGGTGCATGCATGGGTTTAACCATTAA | 528 |
| LRMP_chr12:25206088-25206188 | CTTCCCTTTTTGCCTTTTTATTATAAGCTGGTTTTGTCTGTGGCTGTTTTTTTCTTTTAAAATTAATTAAAAC TTCTCAAAATTTCTAAAGTAAACAAG | 529 |
| LRMP_chr12:25206398-25206498 | GCATTCTCTACATACATCTACATACATATTTTGCATTTTAAAAATTGGAATATTTGTCATTTTTCTGTATTA CCCAAAAGTATATAAACAGTTACCAGAG | 530 |
| LRMP_chr12:25206498-25206598 | ATTTATGTGAGAAGACAGTTGTCACATTACAGATGTCAGATTAGCTATAAAATTGTTTCATTCTAGAAACC TAATATGGTAAAAATAAACCTTACTTATT | 531 |
| LRMP_chr12:25206598-25206698 | TAGCCATTTATCAGACAATTGCTTTTGTTCAGCCAGTTTCTTGTTCTAGCAGTATAAATATTCTTTTTATAG AAAGTTACTTGGTTTGAGAAATAAACAT | 532 |
| LRMP_chr12:25206748-25206848 | ATAAGCTTAAGGTAGGCTAGAGATGAAAAATTTCAGACTTGTGTTTGTTTTGGATTTATTGTACCCTTTCT ACTATTATCTGAGAAAGCTATTTAGGAGT | 533 |
| LRMP_chr12:25206848-25206948 | TTAAGAAATAGTCTAGTTTTAAAATAGCAATGGTTTGCCGGACACAGTGGCTCACCCCTGTAATCCCAGC ATTTTGGGAGGCCGAGGTGGGCAGATTGCT | 534 |
| LRMP_chr12:25207088-25207188 | GAATTTGCCAGTTTTCAATATTCTGATTCACTCTGTTAAGCTAGTAAGGCAGTCTTTAAATTACACAGTCT GTGTGTTATTTACTACTGCTCAGAGGGC | 535 |
| LRMP chr12:25207188-25207288 | ATTGGAGAAGGTTCCCTTGTGATTAGAACTGTTCATGTTGAGACATGAATCATAAGGCATTCCAAAGTTG GTTTAAGGTGTGTCTGCTTTAGACACTGTG | 536 |
| LRMP_chr12:25207288-25207388 | CCCAGGACTATTCTTTTGCTCCAGTTTTGCCTTTTGATTAAATCAATATTATACCTGAGTTTTATAAACTAC TAAGAATTTGTTCCCCTTCCTCACTGTG | 537 |
| LRMP_chr12:25207388-25207488 | ATTTTCTTGCAGTATTTTCTTAGAAGAGTCAACTTTAATAACTTACCCCAAAGTGCACGTTCTTGATATTA TGAACTTGCTATTGTTGTCTTCCCAGTTT | 538 |
| BTG1_chr12:92537875-92537975 | TATTGTAGTTTTTGGAAGGGCTCGTTCTGCCCAAGAGAAGTTCCTCCTTACAGCTGATTCGGCTGTCTACC ATTTGCACGTTGGTGCTGTTTTGAGTGCT | 539 |

| Name | Sequence | SEQ ID NOs. |
|---|---|---|
| BTG1_chr12:92537975-92538075 | ACCTCCTGCTGGTGAGGCTTCATACAGCACACAGATGGAGCCATCCTCTCCAATTCTGTAGGACACTTCAT AGGGGTCAACCCAGAGTGTGAGTTCACTT | 540 |
| BTG1_chr12:92538075-92538175 | GGGAGAAGCCTGAACAGCTCCTGACTGCTCAGTCCAATCCGCTGTGCTGCCTGTCCAATCAGAGGATCCA TTTTATGGTTGATGCGAATACAACGGTAAC | 541 |
| BTG1_chr12:92538175-92538275 | CCGATCCCTTGCATGGCTTTTCTGGGAACCAGTGATGTTTATAATGTTCTATAGAAGAAAAGAAGAACAG AGAAACAACGCTTAGGATCGTTAGCTCCCA | 542 |
| BTG1_chr12:92538275-92538375 | CTGCGGATTCCTCCTACCCCAGGCTCCTTTGAGGAGCGAAAATGAAAACTATCAACTTTTTAAAATGTCCA GGATTGCATCCGTTGTTGTGCATGTGCGG | 543 |
| BTG1_chr12:92538375-92538475 | GGATGGAAAAGCGGGCAGGGTTTTAGAAATAACACAGTAGTACCGGACAAAACAATCTCCAGGAACCA ACCGGTTGAGCCGCCAAAACAGGAATCAGGC | 544 |
| BTG1_chr12:92538475-92538575 | GCGCAGCCTCGGCCAGTCGGGAAGCCACTGGCACCTATGGCCAGGCGAGAAACTGTTTACTTTCTCCACC CCACCCCAGATGCACACAATGGAGTTGATG | 545 |
| BTG1_chr12:92538575-92538675 | GCTTTGGAGATGAGAAGCGCCACCGGACTGTTAACCCCGAAGGGAAGAAAAACAAGCAACCCTAAACCA CGCTCTGGGCAGGGCTGTTAATTGTGCCGGT | 546 |
| BTG1_chr12:92538790-92538890 | ACGCAACGGTTGGAGGGGGCTGAGGAAAGGGGACGTCGAACCCACCCCAGCCCCACGGCTCCTTTGTCCC CAAATCCGCCGACGGTCCTCGGACCGCAGC | 547 |
| BTG1_chr12:92538890-92538990 | TCCCGCCTCGGTGGGCTTAAGTTTCTTTGTTGTGCGTGTTGTCTTCTCCTCTCCGTTTTGCCAGCTGGGGGG AAGGGGGCGCCCTCCGTCCAGCCCCTAA | 548 |
| BTG1_chr12:92538990-92539090 | AGCCTCGCGGGGAACCGCTGTTAGCGGCCACCCAGCGCAACCACACCGGTCCCGCGGCGGGGCCCAAGC GCGACCGGCCCCGGGGCGCTGCCGAGGTTCC | 549 |
| BTG1_chr12:92539090-92539190 | CGCAGCCCCGACGGCCGGACTCTGACCCAGGGATGTGGGGCCCGCGTCCCTCCGACGCCCTCGCCCTGCT CACCTGCCAGCAGCTCCTGCAGGCTCTGGC | 550 |
| BTG1_chr12:92539190-92539290 | TGAAGGTCTGCAGCTGTCGCTCGCTCGTGAGCCCCTTGGTGCGGAGAAACTTGGAGATGAAGGACACGGC GGCGGCGATCTCGCCTATCATGGTGGCGGC | 551 |
| BTG1_chr12:92539290-92539390 | CCGGGTGTAGAAGGGATGCATGGGGGCGGCGTGCGGGGGCGGCCCGGGGCGGCTGGGGCTCGGCGGCGC GGCCCCGACGGCGGAGCAGCCACCCCGGGCT | 552 |

251

EP 4 334 476 B1

| Name | Sequence | SEQ ID NOs. |
|---|---|---|
| DTX1_chr12:113495364-1 13495464 | ACGCCGCACCCCTCCCCCGTGCGTTCTGCGGCCACCCAGGCCTTCCAGGACACCGTGGAGAGGGAACAAG GGGGCAGGGACGCCCCCTTCGGCAGGAGCC | 553 |
| DTX1_chr12:113495464-113495564 | GTCGGAGAAGGGGGCCCAGACCGGAGGGAGGCGAGAAGCCCCACTGAAGCCGGGCGCAGGGTCTGGGA CGCAGTTGGGAGTGCAAAGGGCTGGCTGAGAG | 554 |
| DTX1_chr12:113495564-113495664 | CCGCAGGAGCAGCAGGCTGTGGCCCAGGCCTCCTGGGTGACAGGCCCTGTCTGGCGGGGAAGAGGGACC AAGAGACAACACGGAAGAGGCTGGACCTCGA | 555 |
| DTX1_chr12:113495664-113495764 | ACAGGGGCGGCTGCCTCACTCCCTACCTGAGCCAGCCGAGGGGGCCAAGGACTTTAGAGCTGTTTCCTCC GGCATAAGAGAGACACTTGCTTTCCAGGGC | 556 |
| DTX1_chr12:113495764-113495864 | AGCACCCTTTATCGGAGAAGGCTCTACAGGGAAGGGGTCTTTGCAGCCTGGATGGCCATCCCACATTCCT TTAACGGAGGTCTCTAGGCCTCAGAGAGAA | 557 |
| DTX1_chr12:113495864-1 13495964 | CCCAGAGTTAGAAAGGAGGCCAGACGGTCCTTGCTGTCCCCCTGGGGAGAGAGGAAGTTGCCGCCTGCTG CCAGGCCCAGGAGGAGCTGGGCCTGCAATA | 558 |
| DTX1_chr12:113495964-113496064 | GTGGGGGACCTGGCCCCTGAGGCAGTGGCGGCCATGTCACGGCCAGGCCACGGTGGGCTGATGCCTGTG AATGGTCTGGGCTTCCCACCGCAGAACGTGG | 559 |
| DTX1_chr12:113496064-113496164 | CCCGGGTGGTGGTGTGGGAGTGGCTGAATGAGCACAGCCGCTGGCGGCCCTACACGGCCACCGTGTGCCA CCACATTGAGAACGTGCTGAAGGAGGACGC | 560 |
| DTX1_chr12:113496164-1 13496264 | TCGCGGTTCCGTGGTCCTGGGGCAGGTGGACGCCCAGCTTGTGCCCTACATCATCGACCTGCAGTCCATG CACCAGTTTCGCCAGGACACAGGTGAGCAG | 561 |
| DTX1_chr12:113496264-113496364 | ACACCCACCCCATGCCACCCGCCCCGCCGAGCCATCACTACCTTGCAGCGTAGGATGCTGAAAATCCCAG TAAATCTGCTGATGCCAAATCCCTTCCCCA | 562 |
| DTX1_chr12:113496364-113496464 | TCTCCCTGCCTCACCTCCAGAAAAACAGGGCAGTCTAACCTTGTCCAGTTTAAGACTTGGATTCCAATGCA GCCTCTGAGCAAGCTGTAGGGCCTTGAGC | 563 |
| DTX1_chr12:113496509-113496609 | GGGTAGATCAATATCTCTCACAGCTGAGTGAGGATTAAATAAAATTGTGCTCACTGAGCACAGAACCTAG AACAGCAGTAGCATGGGATTGTAGAATAAG | 564 |
| DTX1_chr12:113496609-113496709 | GGCTTTACATGCACTTCCTCATTTGATTTTTCCCAAGAATCACAGGCAGTAAGTCTGTGTATTGTTGTATT ATTATGAGTCCCATTTTATAGATGAAGAA | 565 |

| Name | Sequence | SEQ ID NOs. |
|---|---|---|
| DTX1_chr12:113496694-113496794 | TTTATAGATGAAGAAACCGAGTCTCCCAGAAGCTGAGTGATTTAAACTCAGAGCTGGGATTTAAACCCAGGCGGTTGAGTTCCAGAACCAAAGTTCTTAA | 566 |
| DTX1_chr12:113496794-113496894 | CTGGTATCCTATACTGGCTCCAAGTGTTGGTTTGTGGGGTGGAGTCGTGCTGGTGGTAATTAATTGGGGATGGGGGGCGTTGGTGGTGTTGATGGTGGGG | 567 |
| DTX1_chr12:113496894-113496994 | TGAGGTGGCAATGATGGAGGAGACAGTGTTAGCGGTTGTGTTGGTGGTGACTCAGTGATAGTATTGATGGTGGTGGGGTCTTGGTGACAATGGAGGGATG | 568 |
| DTX1_chr12:113497059-113497159 | TGTTGGTGACATTGATAGTTGTGTTGGTGGTGGTGCTGGAAGTGGTGTGATGGGGTGGTGATGATGGAGAAAATGAGAGAATGATGTTGGTGGCAGTCTT | 569 |
| DTX1_chr12:113497159-113497259 | CGTGGCCATGTGGTGTGGCTGGTAGCCCTGTGTGTGGCTGTTACTTAGTGGTATTGGTGATCCTGTTGTGGTTGTAATGATGGTGATGTTGATGGTTGCG | 570 |
| DTX1_chr12:113497259-113497359 | TTGGTGGTAATGTGATGGCTGATGATGGAGATAAAATCGATGAGGTCCCACTCTCAGGCCTACTCTCTTTTGTTCTGGAGATTTGTCATCGTTGGGGAGA | 571 |
| BCL7A_chr12:122458781-122458881 | TGAAATGGCTGCTGTCGGGCTGTCATCTCCAGGCCCGGGGCGCTGACATTTGGGCCACTCTCGGTCTCCCTCTTCATTCTGGGCGCGCATTAGCTCTGGT | 572 |
| BCL7A_chr12:122458881-122458981 | CCGGCCGGTTCCGCTGCAGCTGAACAGCAAGATGCGGCACCCAGGTTACCCTGATCATCGCAGATTTCTCCCCGGGGCTCTGTTCTGAGGCCTCAAAAGT | 573 |
| BCL7A_chr12:122458981-122459081 | GCTCCTTGTAGATGGGACCAGGGGTCATTTGGGCAGTAGCAGCGCCTGGTCTCAGTCTGGTACTGAAGTCAGGAATGGCTTAAGGTGAAATCGTGGTCCT | 574 |
| BCL7A_chr12:122459081-122459181 | CTGGTGAAGCTCAGCGAAGACCCCCTCGCCTTGTTTATGACAAGAGAACTTCTGGGGGCGGGAGGAAGAGTCCCTGTTACGATGCTGATCATCATTGAGC | 575 |
| BCL7A_chr12:122459181-122459281 | TTTTGCTGAGCAGAAAACTCTTTAGTACTCAAGGTCGAGAGTCTCTGGTGGTCTGCCTGGCACCAGGCACCTTCCTACAACCCTAGTTTTCCAAAAGGAC | 576 |
| BCL7A_chr12:122459281-122459381 | AAAGCCTGGGGCAGGCGACGTCCTAGCTCGCATTTGAACAGGGCCGCGGGCCAGCAGAGATGCGCGATGCCCAACTCTTTCCAAGAGCACCTCGCGTCCC | 577 |
| BCL7A_chr12:122459381-122459481 | GAACCGGTGCCTTCAACTCGGAGAAGTCAAGAGACCCGCAAGAAACTTGCACGACTGCACCCGCCGCCGCGCTCTGGGGGCTGGGCAGGGGCAGCTGGGC | 578 |

EP 4 334 476 B1

| Name | Sequence | SEQ ID NOs. |
|---|---|---|
| BCL7A_chr12:122459481-122459581 | TGGCTCCCGGGGAACGCGACCCCCCCGCGCCCCGCAGACCGGCTGTCTCCCATGGACCCCTCGGCACCTG CAGCCTCCGAGGAAGGGTCAGCGCGCGTGT | 579 |
| BCL7A_chr12:122460811-122460911 | GGGGGGCTCGGGCCAGCCGATGTTTTTGGCCAGAAGCCGTTCGTCCTGGGCCGCGGCTGCCTCTCCACAC CGGGAGCTCGTGTTTGTTTTGCGGAGGGAG | 580 |
| BCL7A_chr12:122460911-122461011 | CTGTTGTTTTTGTTCTCTGCACCGGGGAGAGGGGGACTTGGTGGCGGCCGCGCGTGGTTTTCGGGATCAC ATTAGCGTCCGCCCGGCGTGGCCCGGTCGA | 581 |
| BCL7A_chr12:122461011-122461111 | CATTAAGGGGATCGAACCTTTCCGCGGCCTCGTCGGGGTCTGCTCGGAATCGGCCCCTGGGCCAGGCCCG AGGCGCAAGCAGATCGCCAGGTTGGGTCAG | 582 |
| BCL7A_chr12:122461111-122461211 | AGTTGTTGAAAACTCCCCGCTGCCTGATTTCAACTTTATTATTTTTTTCCCACGCCTTCACTGGGGTCCCGG AGGGAGAGGAGCCGCCGCAACGCTGGCT | 583 |
| BCL7A_chr12:122461316-122461416 | AGTAGCGCCTCGGTCTCTAAAAGCCACTGGGGGCGAGCCTCCGGTGTGGCGGTGTCACAAGTTAGCTGTC CTTTCTGAGTCAAACCCAACAAAAAAGGCA | 584 |
| BCL7A_chr12:122461416-122461516 | AGAGGAAAATCAATAAAGTCCACGTGCTCCCCGGCCTCCTATGGAAAGGGCTGGCTGCGATGGCCGGATG CCCGGCCGTGGGCTGGGTTTGGCTCCAGTG | 585 |
| BCL7A_chr12:122461516-122461616 | GGACAAAGAATTTTCAGAACCGTGAGAAGGGGAGGCTTTCCAAAGTTGAGATCCAAGTCGTCGGTGTCTC GGGAGCTCCCCTGGTACACAGGGTGCCCGG | 586 |
| BCL7A_chr12:122461616-122461716 | TGCCCGACTGGAGCCATTTAAAAATGGCAGAAACAGCTGCAGGCCAACACACACGCTGGAAAACAAC CCGCAGCCCCCTCTACTGTGGGATTCCCCGC | 587 |
| BCL7A_chr12:122461716-122461816 | GGGAAGCCCGGAGTTGCTCCCCTCCTTGCCTCAGCCCCTGTGCAAAGAAAGAACTGGTGTCTGTGCCTGG GTCCCTTCTGTCGCCGGCCTGGAGGTTGGG | 588 |
| BCL7A_chr12:122461816-122461916 | AAACAGCCGGCAAGCCGCCTTTCTCTGCTCGAGGAGGCGTGGTGGGGCCTCCTACTCCAGGTTCCCGGCT GGACAGAGGCTCCTGCACCCTGACAGCTGC | 589 |
| BCL7A_chr12:122462001-122462101 | GGAGGCCTTCCAGCCCGCTGACCCCGCGGGGACCAGGCCTGTAGTTGGAGCTTGAGGGGCTGTACCTCTG CGCCTCCCTGGGTTTGGGGAAACAACACAT | 590 |
| BCL7A_chr12:122462101-122462201 | CGTGTCCTCTGAAGACCTCAGGCTTTGGGATCTCATGGTCCAGCTTCCAGTTCACTTCGTTGCCGCGACCT TGGGCATATCATTGTCACTTCTCTAACCA | 591 |

| Name | Sequence | SEQ ID NOs. |
|---|---|---|
| BCL7A_chr12:122462201-122462301 | TGGTGACCCGGGGTTTTGTGCTTGGCTTCCAGGTCCCCTCGGGTTATTGAGGACGATTGAGGTCATGCCTC CGAGAGCACCGCGCCCTGGGCGCAGGAGG | 592 |
| BCL7A_chr12:122462716-122462816 | AATGCAAATTTAACAGGGCACCCTGTATTTTACCCAGAGGGAAGCCGAAGTGTTTGGCAGATCATTTGGC CCCATGAGCCTTGGGTGGGTTTCTCCTCAG | 593 |
| BCL7A_chr12:122462816-122462916 | CCCTAGTGACCCCTAAAATTACCCCCCCGACCCACCCACTGTCCCCTGATGCTTCCCCCACCCCCGGAAAA AGCTGTGGCCTCCCTCTCATTTGGGGCAG | 594 |
| BCL7A_chr12:122462916-122463016 | GCTGCCTCCTGTTCTCTTTTTCTGGTGTTTCAGCAAGGCAGGCCAGTGGAGGTGAGGTGACCAGAAGATG GCTAAAGGGAAAACAAAATGGTGGGCCTCT | 595 |
| BCL7A_chr12:122463031-122463131 | CCAGGGTTTGGGGGCCCTGTGCTGGTGGAGGAGAGAAGACCCCAGGGCGATGGTAGGAGACGAAAGCTT GGGCTGCAGCGTAAGCTTGGAGGCCCGCTGC | 596 |
| BCL7A chr12:122463131-122463231 | GGTGGCTCACGCCTGTAATCCCAGAGCTTTGGGAGGCTGAGACAGGAGGATTGCTTGAGCCCAGGAGTTT GAGACCAGCCTGGGTCTCAAACCAAAAAAA | 597 |
| KIAA0226L_chr13:46959165-46959265 | TAAATATAATTTTAACGCCAATCTGAGAAAAATGACTTATTAGCTGTGTGATTTTGAGCAATGCTCTTAAC CTCCCCCATGAAGGATGGTGTGAGAACGA | 598 |
| KIAA0226L_chr13:46959265-46959365 | ACAGAATTGTAGCACGTGTATCAGTCTGGTACACAATGTCCTATGAAGGTTAGCTTTATTATCACCATCAT TATTATTGCAGAAAGACTTTCAGTTCAGA | 599 |
| KIAA0226L_chr13:46959365-46959465 | ATAAGACAGCACAGTTACAGAGACCTGGTTTTATTTTCCAGCTTCTTAACTGAGTCATCTTTCAGCTCCTT TTAATTAAAAAGAAAAAACAATCAGAGAT | 600 |
| KIAA0226L_chr13:46961680-46961780 | TCAAAGACCTGGCAGAAATGACTTCCCAACCCCAGATGCCCCCAGCAGCAGTATTTAGCAGTCATACAAT TGCCTGAAATGAAGAATGAGTAATCTGGAT | 601 |
| KIAA0226L_chr13:46961780-46961880 | GAGTCGGCCCTGAAATCGACCTGCAACTTACCCGGAACGTGAGCTGTCTCTCTCTGACCTCTGCTGGCTGC TTCACCTGGAGTCTGAGTCCGACTCATGT | 602 |
| KIAA0226L_chr13:46961880-46961980 | AGCACTTCACTGTCCGCGTTAGTTTAGCCTTCACTGTCAGCAACTCGTCACCTTGTCCTCTTGCAGCGAAG GTTTGGAATCCCATCACGGGTGTGCAGTG | 603 |
| KIAA0226L_chr13:46961980-46902080 | GTTAGTCCTGAGATCATGGTGGTGCTAGGAGAACCTGCCAACCAATACAGAAAGTTGTCACGAATAGAAA CCTAAGCTCTGGCCGGGTGCGGTGGTTCAA | 604 |

| Name | Sequence | SEQ ID NOs. |
|---|---|---|
| ATP11A_chr13:113516229-113516329 | AGATATACTGTTCTAGACATGTGTCTGAAAGGAATCCTGCAAATTCTGTCTTATTGAACAGGCATAAGGT GTCACGTCAGGCGTAAGGTGTCACAGCAGG | 605 |
| ATP11A_chr13:113516329-113516429 | CGTAAGGCGTCACGTCAGGCGTAAGGTGTCACAGCAGGCGTAAGGCATCACGTCAGGCGTAAGGCGTCA CGTCAGGCGTAAGGTGTCACAAGCTCGGTGA | 606 |
| ATP11A_chr13:113516429-113516529 | ACGTCAGGGGTGTGCCTTGTGTTCTCTGTTCGTTGCTTTCAGAAGCAGCAGCATGTGGCAGCATCTCTGTG CCTATGACGATATTGCAGTGAATATGAGA | 607 |
| SYNE2_chr14:64330252-64330352 | AATTGTACATTTCAACAACATAAATAAGCTGTTCAAGACTGTCTCCCATGCCTCCAAAACAAATAAAAAC CCCCCACAACTCAAATGCATATAAGCTGTT | 608 |
| SYNE2_chr14:64330352-64330452 | ACTATAGTATAATGGTGAGTTATAGCCAGTGTATGATGGGATTGTTGATAGAATAATGCATATTAGAGCT TTTAGTTCAAAAATTTGAGATAGTGATTCA | 609 |
| SYNE2_chr14:64330452-64330552 | GAAAGAAAAAAAGGAATGATTATCATGAATTCTGTTTATTAGAATTCTGTTTATTAAAGAGTTAAAGATA TGTTTTATTTTTTTATCTTTATTATCATTA | 610 |
| ZFP36L1_chr14:69258238-69258338 | AATTCTAATGTTGGTCCCTTAGGATCAGCAGGGGGGGGACCGGGAATCTGTAACTGCAACCACCCCACCGA GAGGATTACAGGAACCCAGTCGAGAGCTGG | 611 |
| ZFP36L1_chr14:69258338-69258438 | TTCCCAACAATGAGGTTCATTTAAAAAGTCGTGAGGGGGGGAGGGGGGCCAAAGAAAGAAATAGATCAAA GAGCGGGAGAGTCGAGAAAAGAAGGAAGAAA | 612 |
| ZFP36L1_chr14:69258438-69258538 | TGTTGGGGAGCGCTGGCAGCCGGGCTGGCAAGTGGAGTTTGGGAATGTGCAGGGAGGGAAGGAAGCTGA AAAATTCAAACTTTTTAAATGCTACTCTTCA | 613 |
| ZFP36L1_chr14:69258538-69258638 | GCTCCTCGGCGTCCCTGCACCCCAACCCTGCAGCCCTGGGGCGTTGGCAGCTGCACCAACAGGAGCAGCA AGCTGGGAAAACAGAGCAACATGACCCGAC | 614 |
| ZFP36L1_chr14:69258638-69258738 | GTGTTAAGAGAAGGCAAAACACTTCAGCAATTAAAAAGTAGCCCAGCAGCTTCACCCTTTCAAATTGGGA GGGGGAGGTTGGAAAGAAATTTAACAACAT | 615 |
| ZFP36L1_chr14:69258738-69258838 | CCATAGACTTTTGCTATGTACATTTAAACCGCAGTCCTGGAACATTCCGAGTTTAAAACTTGCTTTTTCAA CACTGGCTGACAAGCAACATGTTTTAAGG | 616 |
| ZFP36L1_chr14:69258838-69258938 | AGCCCCCCATTAAATCCTTACTCGCGGGACTCTCGAGTTCAAGCCAGCATTTTGTCGCCACCTCCCCCCCCC AACCCCGCCCGCAATCGATGAGCCGCAAT | 617 |

EP 4 334 476 B1

| Name | Sequence | SEQ ID NOs. |
|------|----------|-------------|
| ZFP36L1_chr14:69258938-69259038 | GCCTCGGCAACACAGGTAAGCGGGTCAACCTGAATGCCTCTTTCACCCCAAAGTTTGCTGCACGATCGGCTATCGCGGGAAGAAGCCCAACGGAGCTAGG | 618 |
| ZFP36L1_chr14:69259038-69259138 | GCGGACTCAAGCCCCACTGCAAACTTGTTCTGCAACATCTTTTTGAATCACAACTTGGCCTTTCTTCCTCGCATATCCCCAGCTCCCCCCAAAGAGTGGA | 619 |
| ZFP36L1_chr14:69259138-69259238 | GGAAAACATTGTCCCGAGACTCACTTCCCCGAGGGACCTCCCACTCCCAACCCCACGGGTGGGTAATGCCGCTGGACAGACCTAGGGCGCAGACTGGGAA | 620 |
| ZFP36L1_chr14:69259238-69259338 | CCCGATCAGACCAGCAAACCTGGGATCCAGCAGCACGTTACGTAAAACAGGATCGCCCAAAACTTGTCCCAATCCCAGCCCTCCCCCCGAAGCCCCCGGG | 621 |
| ZFP36L1_chr14:69259338-69259438 | CTGCCCTGCCAGGCAAACTTCGCCCCTCAAAACCCTGGCCTCCAGATTCACATGTAATCCCCGCCAGCAACTGTTGAAACTCAAAGGGTGGGAAGGACGG | 622 |
| ZFP36L1_chr14:69259438-69259538 | GGCCAAATTCCTTCAAACTTGGGAGAAATGCCGGAGGAGAAAAGAATCATCTCGCTGCACCACTTTCCCCATTGCCTTCCAAGACCCAAACTTTTGGGGG | 623 |
| ZFP36L1_chr14:69259538-69259638 | TTCTTTCTTAAGGCAAAAGAAAAAGACTTTTTGAAAAGCAAATGCTCCGCCCCCCTTTACCTTGCATAAAACTTCGCTCAAGTCGAAGATGGTGGCAGAC | 624 |
| ZFP36L1_chr14:69259638-69259738 | ACGAGGGTGGTGGTCATCCTGTGCGTTCGCGCGAGCCAGGGGCGAGGATCTGGTGTGTCGCGAAGGTCCCGGTGCGGGGAAGGCGCAGCCTCTCCTGTCT | 625 |
| FLRT2_chr14:84420586-84420686 | TTATTTTTTATATTAAGATTTATTCTAAATTTTGATTCTTCTAAATATAGTATATATTTAGTATATATATAATGCACCTCTCTTACCTAATGATCATTT | 626 |
| FLRT2_chr14:84420686-84420786 | CTAAATAATCATAACAACATCGAGTAAAACTATGTAATAACACATATTATTATTAAGATAAGTATAAGAAATATAATAATAAATTGTCCCTGTTCTAAAA | 627 |
| FLRT2_chr14:84420786-84420886 | GGTAATTATATAATGCTGAATGTGTCAGAGGCATTCGAACCAGAGTGACTCCATTTGAGTGAGGGCTAGGAAAATGAGGCTGAGACTTGCTGGGATGCA | 628 |
| TCL1A_chr14:96179592-96179692 | TTTAATTTTTATGCTTTCTTCAGTGTATGTTTGGAGAGAGTTTGAACATTTTTTGACTCTTTTTCATTGAGTAAATCCAAATACTTGTAAAAGACTTATC | 629 |
| TCL1A_chr14:96179692-96179792 | TATTTCTTTAACAAAAACTTAACATGGATTAAGGACCCATCTTAAGGCATCACACATTAAAAAAGTCAATATTGATTCAATACCGGCGCTTATACTACGA | 630 |

| Name | Sequence | SEQ ID NOs. |
|---|---|---|
| TCL 1A chr14:96179792-96179892 | CATCACTTGTTAAATTTGTTTTCTAAATAAAGCCCAGAGGTAGTGGAAAATACTTCACACTCTAGGCCAGT GTTTGCTATGCCTGGTTGACCCTAAACTG | 631 |
| TCL1A_chr14:96179892-96179992 | TTGAGGGTTCTTTTTAAAAATACAGATTTCTGGGACCCACCTGAGATGATTCCGATAATCGGCCATATGGA TGAGTCACTTAGAGATACCCATTTTTAAG | 632 |
| TCL1A_chr14:96179992-96180092 | GATTAGGACCCCGAAGCCCAGAAAATGCCTGCTGTAGTCAACATTATAGTCACACTCCACAGGCACTGGG TCCACCCCTTTGACCGACATTCCTTTGCGG | 633 |
| TCL1A_chr14:96180092-96180192 | TTTTCCCACCCTTCTTCCCTGCCTGGAGAACTCCTATTCATCCTCCAGAGCCCGGCTCAAAGTGGCTTCATC TGTGGGGATCCTCCCTGCCCCATAGTGA | 634 |
| TCL1A_chr14:96180192-96180292 | GTGCTCCTTGAGTCCTCGCCCTTCCTAGGGCATCCCAAGCTCCCAGGGGCTGCCCCTGCTGCCTCGCCATC CGCTCCAAAGCTGGCTGTACCTCGATGGT | 635 |
| TCL1A_chr14:96180292-96180392 | TAAGGGCAGCCAGGCGTGCTGCTTCTCGTCCAAATACACGAACTTCTCCCAGGCCCACAGGCGGTCCGGG TGGTCGGTGACTGCCTCCCCGAGTGTCGGG | 636 |
| IGHA2_chr14:106048955-106049055 | AGGAATCAGATTTCAAAATGAATATGTATAAGAAAAGAACCGGGGATCAGTGATCAGGAACAGGGATCC ATGATCTGGTCCAGGGCTCAGCGGTCAGGAA | 637 |
| IGHE_chr14:100068705-100068805 | CCCTGGCCTGGAGTCCCAAGTCCCCAGCCCATCCTGCCCCTGGAGCCCAGTTTAGCTTGGTCTTGAAGTCT GCTCTAGGTACCCCCAAAATCACAGTATC | 638 |
| IGHE_chr14:106068805-106068905 | CAGCCCCGCTCTGCCCACCGGGACAGCCAAGTTCAGCTGAGACTGGCCTACCGGGGGAGTCGCCCTCTGA AGTTCACTCTAAGCCAGCCTGGTTCAGCCT | 639 |
| IGHE_chr14:106068905-106069005 | GGCCCAGGTCAGCCCAGGACCTCCCCTTGCAGGCAGCAAACTCTTATTTCAGTCCAGCCAGCTCAACCAG CTTGCTTCTGACTCAGCTCCTCTTAGCCAG | 640 |
| IGHE_chr14:106069045-106069145 | TTAGCTCAGCAAAGCTGGACCTAAAGTAGCCACCTCACCCCAGCTTCATCCAGATGAATACAGTCCAGAT CAGCTTAGTCAGTTAAGCCTAGCCTAGCTA | 641 |
| IGHE_chr14:106069145-106069245 | GTTAAATCCAGTTACGACCAGCTCAACTAATCCTGCTCAGGCCTGCTCAGCCCAGCCCAGCTGAACCCAGT TTAGCCGAGGCCAGGCCAGCCCAGCTGAA | 642 |
| IGHE_chr14:100069245-100069345 | TACAGTTGCCCAGTCTAGCTCAGCCCAGTCCAGCACTGCCCAGTTTAGCTGAGCTCAGCCTGGCCCAGCCC AGCTCATATCAGCCCATCTCAGCTGAACC | 643 |

258

EP 4 334 476 B1

| Name | Sequence | SEQ ID NOs. |
|---|---|---|
| IGHE_chr14:106069345-106069445 | AGTTTGACCCAGTCTAACCCAACCCCGCTCAGCTGAACCCAGCCCAGCCCAGCCCAGCCCAGCCAAACCC AGTTTAGCCTAGCTCAGCTCAGCCCATTTC | 644 |
| IGHE_chr14:106071060-106071 160 | CCTGTCCTAGGGGTGGCAGGCAGTCTGCACCAGCCTAGCCCTGCCCAGCGTGGGGTCTCTGACCTTCTTG GTCTTGGGCCCAGCCAAGATTCCCAGCCC | 645 |
| IGHE_chr14:106071190-106071290 | TTCTAGCTTTCCTGTGTCCCCATGCAGGGAAGGGATGCCTAGAGTCCACGCAGTGACCAAGAAGCTTGGT TGATGCTGTGAGGGTGGCCCAGGAGTCCCC | 646 |
| IGHG4_chr14:106095335-106095435 | CACCTGCTGTCCTTGGTCCTGGCTGAGAGGAGGGCCCTACGGCCAGCTCTGCTGACCCTGCCCTGGGCTCT GGTGATGCTGCCGGCCTGGACAAGCCCCT | 647 |
| IGHG4_chr14:106095480-106095580 | GAGCTCAGGTCGGTCGTGCCCATCCTGGCATCACCCCACAGCCGGTTCTGCCGCATCCCGTCATGTTCCTC GTGCTCCCAGCCCGGTCGTCCTGGAGGCC | 648 |
| IGHG2_chr14:106110675-106110775 | TGAGCATGAGTGGGGCGGGCAGAGGCCTCCGGGTGAGGAGACAGATGGGGCCTGCCTTGCTGCCCTGGG CTGGGGCTGCACAGCCGGGGTGCGTCCAGGC | 649 |
| IGHG2_chr14:106110775-1061 10875 | AGGAGGGCTGAGCCTGGCTTCCAGCAGACACCCTCCCTCCCTGAGCTGGCCTCTCACCAACTGTCTTGTCC ACCTTGGTGTTGCTGGGCTTGTGATCTAC | 650 |
| IGHG2_chr14:106110830-106110930 | ACCAACTGTCTTGTCCACCTTGGTGTTGCTGGGCTTGTGATCTACGTTGCAGGTGTAGGTCTGGGTGCCGA AGTTGCTGGAGGGCACGGTCACCACGCTG | 651 |
| IGHG2_chr14:106110950-106111050 | GGACTGTAGGACAGCCGGGAAGGTGTGCACGCCGCTGGTCAGAGCGCCTGAGTTCCACGACACCGTCACC GGTTCGGGGAAGTAGTCCTTGACCAGGCAG | 652 |
| IGHG2_chr14:106112335-106112435 | TGCTACACTGCCCTGCACCACCTCCACTCAGCTTCATTGTGCTGGTGGCCCTGGCTCCTGGCAGCCCATCT TGCTCCTTCTGGGGCGCCAGCCTCAGAGG | 653 |
| IGHG2_chr14:106112435-106112535 | CCTTCCTGCCTAGGGTCCGCTGGGGCCAGCCCTGGGACCCTCCTGGTCTCAAGCACACATTCCCCCTGCAG CCACACCTGCCCCTGCCTGAGAGCTCAGC | 654 |
| IGHG2_chr14:106112535-106112635 | CCCGAGCCCTGGAATGCCTTCCCTTCTCCATCCCAGCTCACCCTTGCCAACTGCTCAGTGGGATGGGCTCA CACTCCCTTCCTGGCACCAGGAGGCTGCA | 655 |
| IGHG2_chr14:106112635-106112735 | CTGCACTTTCACCAGCCCTCAGCTGTCTGCTGCCAGCAACTACCCAGCTCCTGCCAAAATCTAGGAGCTGA GTGATGCCTCCCACCGGCCCTGCTCACCT | 656 |

| Name | Sequence | SEQ ID NOs. |
|---|---|---|
| IGHG2_chr14:106112735-106112835 | GTGGTTGCCTTGCCCTGAGCTCTAGTGCCTGTCCCCTGCTCGTCCTGCCTCCCACCGGCCCTGCTCACCTG TGGCTGCTCTGCTCTGATTCCCTGAGGCT | 657 |
| IGHG2_chr14:106112835-1001 12935 | AAGCCTCAGTCCTGCTCACCTTCTGATGCTCTCCTCTGTCCCCTGAGCTCCAGGGGCTGTCCCCTGCTCGT CCTGCCTCCTACCTGCCCCTGCTTACCTG | 658 |
| IGHG2_chr14:106112935-106113035 | AGGGTGCTCTGCCCTGGTGCTCTGAGCTCCAGGGGCTGTCCCCTGCTCCTCCTGCTTCCTACCAGCCCCTG CTCACCTGTGGCTGCTCTGCCCTGGTCCC | 659 |
| IGHG2_chr14:106113020-106113120 | CTCTGCCCTGGTCCCCTGAGCTCCAGGGGCTTCCCCCTGCTCTTCCTGCCCCCACCAGCCCCTGTTCACCTT CAGATGCCCTCCCCTGGTCCCCTGAAGT | 660 |
| IGHG2_chr14:106113120-106113220 | CCCAGAGCTGCCCCCTGTTCCTCCTGCCTCCCACCAGCCCGTGCTCACCTGCCGCTGCTCTGCCCTGGTCC CGAGTTCCAGGGGCTGCACCCTGTTCGCC | 661 |
| IGHG2_chr14:106113220-106113320 | CACCTCCCACTAGCCATGCTCAGCTCTTGATGCTCTGTCCTGGTCCCCTGAGCTCCAGGAGCTGTCCCCTA CTCGTCCTGCCACCCACCAGCCCCTGCTC | 662 |
| IGHG2_chr14:106113320-106113420 | ACCTGAGGCACCTGAGGCTGCTCTGCCCTGGTCCCCTGAGCTCCAGGGTCTTCCCCCTGCTCATCCTGCCT CCCACCTGCCCTTGTTCACCTTCAGTTGC | 663 |
| IGHG2_chr14:106113420-106113520 | TCTGCCCTGGTCTGCTGAGCTCCAGGAGGTGCCCCCTGCTCCTTCTGCCCCCACCTGCCCTGCTCACCTGT GGCTGCTCGGTCCTGGTACCCTGAACTCC | 664 |
| IGHG2_chr14:106113450-106113550 | GCCCCCTGCTCCTTCTGCCCCCACCTGCCCTGCTCACCTGTGGCTGCTCGGTCCTGGTACCCTGAACTCCA ATGCCTGCCCCCTGCTCACTCTGCCCTCC | 665 |
| IGHG2_chr14:106113550-106113650 | CTCAACCCGGGCAGCAATGTCACTCAGGTCACTGTTGCCCCCCTGCCTGTCCTGGCACCCTCTGTCCAGGT TTGGGCTGTTTTTCTGGCCTCATTTTTGT | 666 |
| IGHG2_chr14:106113695-106113795 | TGTCCAGTCAGGTCTCCCCAACAGAGCCCCTTGCCCTTGCCCATGTGCCCCTCCTGGGTGAGCTCCCAGAT CCTCCCGTCCCTGCACTGCTCCTGCTCTG | 667 |
| IGHG2_chr14:106113795-106113895 | GAAGCCTCTCCAGAACCTCAGCTCCTCAGTGGCCTCTGCTCTGCTGGGTCAGCTCCCTGAACGCACGGAG CCTCACCCCTCCCCTCGCCCCAGGCCTGCT | 668 |
| IGHG2_chr14:106113895-106113995 | GCACTCTGGGCCTTTCTGGGCCTCCCTGGACTCTTCCCTCCTCCCATCTGTGCACTCAGCACAGCTCTCCCC TCCACTCCGCTGCTGACCACAGCCCTGC | 669 |

EP 4 334 476 B1

260

| Name | Sequence | SEQ ID NOs. |
|---|---|---|
| IGHG2_chr14:106113905-106114005 | CCTTTCTGGGCCTCCCTGGACTCTTCCCTCCTCCCATCTGTGCACTCAGCACAGCTCTCCCCTCCACTCCGCTGCTGACCACAGCCCTGCTCCCCGCCAG | 670 |
| IGHG2_chr14:106114175-106114275 | CCCACGGCCAGCACTGCTGACCCTGCCCTGGGCTCCAGTGATGCTGCTGGCCTGGACAAGCCCCTCCGTTCACCTGGGGCCTCTCCTCCTCCCTCGTTCT | 671 |
| IGHG2_chr14:106114275-106114375 | ACTGCCTCCTCAGCTCAGGTGGGTCCTGCCCATGCTGGCATCACCCCACGGCCGGCTCTGCCGCATCCCGTCAGGTTCCTCGTGCTCCCAGCCTGGTCGT | 672 |
| IGHG2_chr14:106114375-106114475 | CATGGAGGCCTCAGTCAGCCTCTGGTGTGTCCTGCCCTGTTGGCTTGGAAGCCCCTGCCCACGGTCCCCGTCATCTTGCACTGGGTGGGCGTTGGTGCCT | 673 |
| IGHA1_chr14:106176375-106176475 | AGCTCAGCCCAGCCTAGTCCAGCCCAGCCCAGCACAGGTCAGCCCAGCTTAGCTTAGCCCAGGTCAGTCCAGCTCAGCTCAGTCCACTTAAGCTCACCCA | 674 |
| IGHA1_chr14:106176475-106176575 | GGTCAGCTCCGTCCAGCTCAGCCCAGCCTAGCCCAGCTTAGCCCAGCCCAGCCCAACACAGGTCAGCCCAGCTCAGCCTAGCCCAGCCCAGCTCAGCACA | 675 |
| IGHA1_chr14:106176575-106176675 | GGTCAGACCAGCTCAGTACAGCTCAGGTCAGCCCAGACCAGTCCAACCCAGCCCAGCGCAGTCCAACCCAGCCCAGCTCAGCTCATCCAAGCCTAGCTCA | 676 |
| IGHA1_chr14:106176675-106176775 | GCTCAGCCCAGCCCAGGTCAGCCTAGCCCAGCCGAACCCAGCTCAGCCCAGGTCAACCCAATTCAGCTCAGCTCAGCCCAGGTCAACCCAACCAAGCTCA | 677 |
| IGHA1_chr14:106176775-106176875 | GCTCAGCCTAGCCCAGTCCAGCTCAGCCCAGCTCAGCTCAGCCCAGTCCAGCTCAATCCACCTAAGCTCACCCAGCTCAGCCCAGTCTGGCTCAGCTTAG | 678 |
| IGHA1_chr14:106176875-106176975 | GTCAGCCCAGCCCAGCCTAGCCCAGATCAGTCCAGCTTAGCCCAGCCCAGGTCAGCCCAGCCCAGGTCAGCCCAGCTCAGCTCAGCCCAGCCCAGCTCAG | 679 |
| IGHA1_chr14:106176985-106177085 | CCCAGCCCAGCTCAGCGCAGCCCAGCCTAGCTCACCCCAGCCAGGTCCAGCTTAGCCCAGCTCAGCCCAGCCCAACTCAGCTCAGCCCAGCTCAGCCCAA | 680 |
| IGHG1_chr14:106211960-106212060 | TCTGAGCTCCAGGGGCTGCCCACCTGCTCCTCCTGCTTCCCACCGGCCCTGCTCACCTGCAGCTGCTCTGCCCTGGCTCCCTGAGGCTGAGCCTCAGTCC | 681 |
| IGHG1_chr14:106212060-106212160 | TGCTCACCTTCTGATGCTCTCCCCTTGTCCCCTGAGCTCCAGGGGCTGACCCCTGATCTTTCTGCTTCCTACCTGCCCCTGCTCACCTGTGGCTGCTCTG | 682 |

(continued)

| Name | Sequence | SEQ ID NOs. |
|---|---|---|
| IGHG1_chr14:106212160-106212260 | CCCTGATCCCCTGAGCTCCCAGGAGCTGCCTCCTGCTCCTCCTGCCTCCCACCTGCCCCTGCTCACCTGCAGAATCTGCCCTGGCTCTCTGAGGTCCCAGGGGG | 683 |
| IGHG1_chr14:106212260-106212360 | CTGCCCCTGCTGCTGCGCCACCTCCACCAGCCATGCTGAGCGTTGTGATGCTCTGCCCTGGTCTCCTGAGGTCCAGGGGCTGTCCCCTGCTTATTCTGCCTC | 684 |
| IGHG1_chr14:1062 12360-1062 12460 | CCACCTGCCCCTTCTCACCTGAGGCTCTTCTGCCCTGGTGCTCTGAGCTCCAAAAGCTGCCCACTTGCTCCTCCTGCTTCCTACCAGCCCCTGCTCTCCT | 685 |
| IGHG1_chr14:106212460-106212560 | GTGGATGAATCTGCCCTGGCCTCTCTGAGCTCCAGGGGCTGCCCACCTGCTCCCCACCTGCCCCTGCTGACCTGCGGCTGCTCTGCCTTGGCT | 686 |
| IGHG1_chr14:106212560-106212660 | CCCTGAGCTCCAGGAGCTTCCCCTGCTCATCCTGCCCCCACTGCCCCTGTTCACCTTCAGATGCCCTCCCTGGTCCCCTGCTGCCCACCTCCCACT | 687 |
| IGHG1_chr14:106212000-100212760 | CCCTGTTCCTCCCGCCTCCCACCAGCCCGTGCTCACCTGCGGCTCTGCCCTGAGTTCCAGGGGCTGCCCCTGCTCGCCCACTCCCACT | 688 |
| IGHG1_chr14:106212760-106212860 | AGCCATGCTCACCTGCCTGATGCTCTGTCCTGGTCCCTGAGCTCCAGGGGCTCCCCTGCTTGCCCATCTCCCACTAGCCATGCTCACCTTCTGATGCT | 689 |
| IGHG1_chr14:106212860-106212960 | CTGCCCTGGTCCCCTGAGCTCCAGGGGTCTTCCCCCTGCTCATCCTGCGCGCCCAGCCCCTGCTCACCTGAGGCTGCTCTGCCCTGGTCCCCTGAGCTC | 690 |
| IGHG1_chr14:106212870-106212970 | CCCCTGAGCTCCAGGGTCTCTTCCCCTCCTCCATCCTGCCGCGCCACCAGCCCCTGCTCACCTGAGGCTGCTCTGCCCTGGTCCCCTGAGCTCCAGGAGGTGC | 691 |
| IGHG1_chr14:106212980-106213080 | TTCTGCCCCCACCTGCCCCTGCTCACCTGTGGCGGCTGCTTGGGTCCCTGAGCTCCAATGCCTGCTCCCCTGCTCACTCTGCCCCTCCCTCAACCGGGCA | 692 |
| IGHG1_chr14:106213080-106213180 | GCAAATGCACTCAGGTCACTGTTGCCCCCCCTGCTGTCCTGGCACCCTGTCCAGGTTTGGGCTGTTTTTCTGCCCTCATTTTTGATTTTGCAGCACTT | 693 |
| IGHG1_chr14:106213125-106213225 | CCCTCTGTCCAGGTTTGGGCTGTTTTCTGCCCTCATTTTTGCAGCACTTGGGCGTGTTCCCTATGCTGTGGAGCAGCCCAGTGTCCAGTCAGGT | 694 |
| IGHG1_chr14:106213210-106213310 | AGTGTCCAGTCAGGTCTCCCCAACAGAGCCCCTTGCCCATGTGCCCCTCCTGAATGAGCTCCCGGATCCTCCTGTCCCTGCACTGCTCCTGCTC | 695 |

| Name | Sequence | SEQ ID NOs. |
|---|---|---|
| IGHG1_chr14:106213310-106213410 | TGGAAGCCTCTCTGGAACCTCAGCTCCTCAGTGGCCTCTGCTCTGCTGGGTCAGTTCCCTGAACGCACGGAGCCTCAGCCCTTCCCCTCGCCCCAGGCCT | 696 |
| IGHGI1_chr14:106213410-106213510 | GCTGCACTCTGGGCCTTTCTGGGCCTCCCTGGACTCTTCCCTTCTCCCGCCCGTGCACTCAGCACAGCTCTCCCCTCCTCTCCACTGCTGACCACAGCCC | 697 |
| IGHG1_chr14:106213510-106213610 | TGCTCCCCGCCAGCAGGTGCCCCAACCCCATCAGCTGGCTCTGAGCCCAGCCCCTGTGCCTCCCCTGTCCCTGCCTCTGCCTCTGGGCTCCTTGGCTTCC | 698 |
| IGHG1_chr14:106213660-106213760 | ACCTGCTGTCCTTGGTCCTGGCTGAGAGGAGGGCCCCACGGCCAGCACTGCTGACCCTGCCCTGGGCTCCGGTGATGCTGCCGGCCTGGACAAGCCCCTC | 699 |
| IGHG1_chr14:106213760-106213860 | CGTTCACCTGGGGCCTCTCCTCCTCCCTCGCTCTGCTGCCTCCTGAGCTCAGGTCGGTCGTGCCCATCCTGGCATCACCCCACGGCCGGCTCTGCCGCAT | 700 |
| IGHG I_chr14:1062 13860-1062 13960 | CCAGTCATGTTCCTCGTGCTCCCAGCCCGGTCGTCCTGGAGGCCTCAGTCAGCCTCTGGTGTGTCCTGCCCTGTTGGCTTGGAAGCCCCTGCCCACGGTC | 701 |
| IGHG1_chr14:106213960-106214060 | CCCGTCGTCTCGCACTGGGTGGGCATCGGTGCCTGAAGGCTGCCCACCTCCCCCGTGCTGGCTCCGCTTGGGCCTCCATGTGGGGCCGGCCTCGACCCCA | 702 |
| IGHG3_chr14:1062.39250-1062393.50 | CACTGCACTTTCACCAGCCCTCAGCTGTCTGCTGCCGGCAACTACCCAGCTCCTGCCAAAGTCTAGGAGCTGCGTGCTGCCTCCCACCGTCCCTGCTCAC | 703 |
| IGHG3_chr14:106239350-106239450 | CTGTGGCTGCTCTGCCCTGGTGCTCTGAGCTCCAGGAGATGCCCCCTGCTCCTCCTGCCCCCCACCTGCCCCTGCTCACCTGCAGCGGCTCTGCCCTGGT | 704 |
| IGHG3_chr14:106239455-106239555 | GAGCTCCAAGAGCTGCCCCCTGCTCCTCCTGTCCCCTGACCCTGCTCCTGTTTGCCTATGGCTGCTCTGCCCTTGTCCCCTGAGCTCCAGGAGCTGCCCC | 705 |
| IGHG3_chr14:106239555-106239655 | TGCTCATTCTGCCGCCCACCTGCCCCTGTTCACCTGTGGCTGCTCTTCCCTGGTCCTCTGAGCTCCATGAGCTGCCCCTTGCTCCTCCTGCTTTCCACCA | 706 |
| IGHG3_chr14:106239655-106239755 | GCCCCTGCTCACCTACCGATGATCTTCCCCGGCTCTCTGAGCTCCAGGGGCTGCCCACCTGCTACCCCTGCTTCCCACCAGCCCTGCTTACCTGCAGCTG | 707 |

263

EP 4 334 476 B1

| Name | Sequence | SEQ ID NOs. |
|---|---|---|
| IGHG3_chr14:106239755-106239855 | CTCTGCCCTGGCTGGCAGAGCTGCAGAAGCTGCCCCCTGCTCTGCAACCTCCCACCGGCCCTTCTCATCTTCTGATGTTCTCCCTGTTCCCTGAGCTCC | 708 |
| IGHG3_chr14:106239855-106239955 | AGGAGCTGCCCCCTACTCGTTCTACCTCCCACCAACCCGTGCTCACCTGCGACTGCTCTGCCCTGGTCCCCTGAGCTCCAGGGGCTGCCCCCTGCTCGCC | 709 |
| IGHG3_chr14:106239990-106240090 | TGCCCTGATCCCCTGAGCTCCAGGACTGCCCCCTGCTCGTCCTGCCCCTCACCTGCCCCTGCTCACCTGAGGCTGCTCTGCCCTGGTCCCCTGAGCTAAA | 710 |
| IGHG3_chr14:106240090-106240190 | GGGGCTGCCCCTTACTCATCCTGCCTCCCACCAGCCCCTGCTCACCTTCTGATGCCCTCCCCTGGTCCCCTGAGCTCCAGGGGCTGCCCCCTGCTCGTCC | 711 |
| IGHG3_chr14:106240170-106240270 | GGGCTGCCCCCTGCTCGTCCTGCCTCCCACCAGCCCCTGCTCACCTGCAGCTACACTGCCCTGGTTCCCTGAGCTCCAGGAGCTGCCACCTGCTTGTCCT | 712 |
| IGHG3_chr14:106240270-106240370 | GCCTTCCACCAGCCCCTGCTCACCTGCAGCTACACTGCCCTGGTTCCCTGAGCTCCGGGAGCTGCCGCCTGCTTGTCCTGCCTCCCACCAGCCCCTGCTC | 713 |
| IGHG3_chr14:106240370-106240470 | ACCTGTGGCTACACTGCCCTGGTGCCCTGAGCTCCAGGAGCTGCCCCCTGCTTGCCCATCTTCCACTGAGCCCTGCTCACCTGCAACTGCTCTGCCCTGG | 714 |
| IGHG3_chr14:106240470-106240570 | CTCTATGAGCTCCAGGGGCTGCCCCCTGCTGGTCCTGCCTCCCACCTGCCCTGCGCACCTGTGGCTGCCTCCTCACCTGTGGCTGCTCTGCCCTGGTCCC | 715 |
| IGHG3_chr14:106240570-106240670 | CTGAGCTCCAGGGTCTTCCTCCTGCTCATCCTGCCCCTCCACCGGCTCCTGTTCACCTTCAGATGCTCTCCCGTGGTCCCCTGAGCTCCAGGAGCTGCCC | 716 |
| IGHG3_chr14:106240670-106240770 | CCTGTTCTTCCTGCCTCCCACCTGCCCTGTGCACCTGTGGCTGCTTGGTCCTGGTCCCCTGAACTCCAATGCCTGCCCCCTGCTCACTCTGCCCTCCCTC | 717 |
| IGHG3_chr14:106240770-106240870 | AACCTGGGGCAGCAACGTCACTCGGTCCACTGTTGCCCCCCTGCCTGTCCTGGCACCCTCTGTCCAGGTTTAGGCTGTTTTTCTTGCCTCATTTTTGTTT | 718 |
| IGHG3_chr14:106240820-106240920 | TGGCACCCTCTGTCCAGGTTTAGGCTGTTTTTCTTGCCTCATTTTTGTTTTTGCAGCACTTGGCGTGTTCCCTATGCTGTGGAGCAGCCCCAGTGTCCAG | 719 |

| Name | Sequence | SEQ ID NOs. |
|---|---|---|
| IGHG3_chr14:106240915-106241015 | TCCAGTCAGGTCTCCCCAACAGAGCCCCTTGCCCTTGCCCATGTGCCCCTCCTGGATGAGCTCCCGGATCCTCCCGTCCCTGCACTGCTCCTGCTCTGGA | 720 |
| IGHG3_chr14:106241015-106241115 | AGCCTCTCCAGAACCTCAGCTCCTCAGTGGCCTCTGCTCTGCTGGGTCAGTTCCCTGAACGCACGGAGCCTCAGCCCCTCCCCTCGCCCCAGGCCTGCTG | 721 |
| IGHG3_chr14:1062d1115-106241215 | CACTCTGGGCCTTTCTGGGCCTCCCTGGACTCTTCCCTCCTCCCGCCCGTGCACTCAGCACAGCTCTCCCCTCCTCTCCGCTGCTGACCACAGCCCTGCT | 722 |
| IGHG3_chr14:106241200-106241300 | GACCACAGCCCTGCTCCCGGCCAGCAGGTGCCCCAACCCCATCAGCTGGCTCTGAGCCCAGCCCCTGTGCCTCCCCTGTCCCTGCCTCTGCCTCTGGGCT | 723 |
| IGHG3_chr14:106241345-106241445 | GCTCTGCTCCCAGCTCACCTGCTGTCCTTGGTCCTGGCTGAGAGGAGGGCCCTACGGCCAGCTCTGCTGACCCTGCCCTGGGCTCCGGTGATGCTGCCGG | 724 |
| IGHG3_chr14:106241445-106241545 | CCTGGACAAGCCCCTCGGTTCACCTGGGGCCTCTCCTCCTCCCTCTCTCTGCTGCCTCCTGAGCTCAGGTCGGTCATGCCCATCCTGGCATCACCCCATG | 725 |
| IGHG3_chr14:106241545-106241645 | GCTGGCTCTGCCCCATCCCGTCATGTTCCTCACACTCCCAGCCCGGTCGTCCTGGAGGCCTCAGTCAGCCTCTGGTGTGTCCTGCCCTGTTGGCTTGGAA | 726 |
| IGHM_chr14:106318100-106318200 | GGGTAGAGCCCACCTCGTGGCCTGCAAGCCAGCCAGCCCCTGCCGGTCGAGAAGGAAGCCTGTGTGAGAGCACACAACTGGAGGCCGGGCGGGGAAGAGA | 727 |
| IGHM_chr14:106318200-106318300 | AACACGTGCCAACAGGCCACGCAGGCCAGGACCCCAGACCCGGAGGCAGCGCCCCTTTGAGTTCCTCTCTCTGGTCTCCGATGTTCTTCTGTTGGGATCA | 728 |
| IGHM_chr14:106318300-106318400 | TTTCACCTACAGGCAACAGAGACAGTGTGAAATGCTTTCCCTGTGGTCGGGAAGGGAGCCGGGGCAGAGATGACCCAGTGGGGTGGTGTGGGGGCCTCCG | 729 |
| IGHM_chr14:106322055-106322155 | CTTTGCACACCACGTGTTCGTCTGTGCCCTGCATGACGTCCTTGGAAGGCAGCAGCACCTGTGAGGTGGCTGCGTACTTGCCCCCTCTCAGGACTGATGG | 730 |
| IGHM_chr14:106322155-106322255 | GAAGCCCCGGGTGCTGCTGATGTCAGAGTTGTTCTTGTATTTCCAGGAGAAAGTGATGGAGTCGGGAAGGAAGTCCTGTGCGAGGCAGCCAACGGCCACG | 731 |

| Name | Sequence | SEQ ID NOs. |
|---|---|---|
| IGHM_chr14:106322255-106322355 | CTGCTCGTATCCGACGGGGAATTCTCACAGGAGACGAGGGGGAAAAGGGGTTGGGGCGGATGCACTCCCTGAGGACCCGCAGGACAAAAGAGAAAGGGAGG | 732 |
| IGHM_chr14:106322905-106323005 | ACTCCAGCTACCCTGAAGTCTCCCCAGGCAGACAACCCAGGCCTGGGAGTGAGTATAGGGAGGGTGGGTGTGATGGGGAACGCAGTGTAGACTCAGCTGA | 733 |
| IGHM_chr14:106323005-106323105 | GGCTATCCATCTATGTCCAACAAGATCATGAAGATTGGCCCAGTGCCATGTCCTCCAGTTCATCCCAGCCCAGGCCAGCTCAATCCAGTTCATCCCAGCC | 734 |
| IGHM_chr14:106323105-106323205 | CAGGCCAGCTCAATCCAGCCCAGCCCACCCCACCCCAGCTCAGCAAAGCCAAGCTCAGCTCAGCCCAACTCAGATGAGCTCAGACCAGCTCAGCCCAGCC | 735 |
| IGHM_chr14:106323470-106323570 | CAGCTCAGCTCAGCCCAACCCAGCCCAGCTCGCTCAACCTTGCTCGGCTCAGCTTAGCCCAGCCCAGCCCAGCTCAATCCAGCCTGGCTCAGCCCAGCCC | 736 |
| IGHM_chr14:106323570-106323670 | AGCCCAGTTTGGCTCAACCCAGCTTGGCTCAGCCCAGGTCAGCCTGGCTCAACTCAGCCCAGCCCAGCCCAGCTCTGCTCAACCCAGCTCTGCTCAACTC | 737 |
| IGHMchr14:106323805-106323905 | AGCCCAGCTCATCCCAGCTCAGCCCAGCCCAGCCTAGCTTAGCTCAACCCAGCTCAGCTCAGTTCAGCTCAGCCCTGCTCAGCACAGCACAGCAGAGCCC | 738 |
| - IGHM_chr14:106324010-106324110 | AGCCCGGATCGGCTCAACCCAGCTTAGCTCAGCCCAGGTCAGCCCAGCTTAACTCAGCCCAGGTCAGCCCAGCTTAACTCAGCCCAGCCCAGCCCAGCTC | 739 |
| IGHM_chr14:106324155-106324255 | TCAGCCCAGTTCAGCCCAGCTCAGCCCAGCCCAGCCTAGCTTGGCTCAACACAGCTCAGCTCAGCCAGCCCAGACCAGCTCAGCTCAGCCCAGTCCAGCT | 740 |
| IGHM_chr14:106324290-106324390 | CAACCCAGCCCAGCCCAACCCAGCTCGGCTTAACCCAGCTCGGCTCAGCCCAGATCAGCCTGGCTCAACTCAGCCCAGCCCAGCTCAACCCAGCCCAGTT | 741 |
| IGHM_chr14:106324490-106324590 | CAGCTCAGCTGAGCCCAGCCCAGCCCAGTCCGGCTCAGCTCAGCCCCGCCCCACTCAGCCCAGCTCAGCTCAGCCCAGCTCAGCCCAGCTCAGCTTAGCC | 742 |
| IGHM_chr14:106324750-106324850 | CAGCCCAGATCATCCCAGCTCAGCTCAGCTCAGCTCGGCTTAGCCCAGCTCAACCTGGCCCAGCCTGGTCCAGGTCAGCCCAGCCTGGACCACCCAGCCC | 743 |
| IGHM_chr14:106324850-106324950 | AGCTCAGCTCAGCCCAGCTCATCCTGGTTCAGCTCAGCTCAACCCGGCTCAGCCCAGGTCTGCTCAACCCAGCCCAAATCAGCTCAGCCCAGCCCAGGTC | 744 |

| Name | Sequence | SEQ ID NOs. |
|---|---|---|
| IGHM_chr14:106324950-106325050 | ATCCCAGCTCAGCCCAGCACAGCCTACTTCAGCTCAGCTCAGCTCAGCCTAGGTCAGCTCAGTTGAGGTCAGCTCAACTCAGCCCAATCCAGCCTGGCTC | 745 |
| IGHM_chr14:106325050-106325150 | AGCCCAGCTCACCCTAGCTCAGCTTAGCTCAGCCCAACTCAACCCAGCCCAGCCTTGCCCAACCCAGCTCAGCTCAGCCCAGCCCAGGTTAGCCCAGCCC | 746 |
| IGHM_chr14:106325150-106325250 | AGCCTCGGCTTAGCTCTGCTCAGCTCGGCCCTGCTCGCCTCAGCCCGTTCAGCCCAGTTCAGCTCAGCTCAGCTCAGCCCAGCTCAGCCCAGCCCTGGTT | 747 |
| IGHM_chr14:106325250-106325350 | AGCTCAGCCCAGCTAAGCTCAGCTCGGCTTGGCTCTGCTGAGCTTGGCCCAGCTTGGCTTAGCCTGATACAACCTGCTCAGCCCAGTTCAGCTCGGCTCA | 748 |
| IGHMchr14:106325360-106325460 | GCCCAGCGTAGCTCAGCTCAGCTGAGCCCAGCCCAGGTTAGCTCAGCCCCAGTCCAGGTCAGCTCAACTCAGCCCAAACCAGCCTGGCTCGGCCCAGCTC | 749 |
| - IGHM_chr14:106325460-106325560 | ACCCTAGTTCAGCTTAGCTCAGCCCAGCCCAGCCCTGCCCAACCCAGCTCAGCTCAGCCCAGCCCAGGTTAGCCCAGCCCAGCCTCGGCTTAGCTCTGCT | 750 |
| IGHM_chr14:106325515-106325615 | AGCCCAGCCCAGGTTAGCCCAGCCCAGCCTCGGCTTAGCTCTGCTCAGCTCGGCCCAGCCCAGGTTAGCCCAGCCCAGCCTCGGCTTAGCTCTGCTCAGC | 751 |
| IGHM_chr14:106325615-106325715 | TCGGCCCTGCTCGCCTCAGCCCGTTCAGCCCAGTTCAGCTCAGCTCAGCTCAGCCCAGCTCAGCCCAGCCCTGGTTAGCTCAGCCCAGCTAAGCTCAGCT | 752 |
| IGHM_chr14:106325715-100325815 | CGGCTCAGCTCTGCTGAGCTCGGCCCAGCTTGGCTCAGCCCGACACAGCCTGCTCAGCCCAGTTCAGCTCGGCTCAGCCCAGCCCAGCCCAGCGTAGCTC | 753 |
| IGHJ6_chr14:106325820-106325920 | AGCTGAGCCCAGCCCAGGTTAGCTCAGCCCCAGCCCAGGTTAGCTCAGCCCAGCTCAGCTCTGCCCAGGTTAGCTCAGCCCCAGTCCAGGTTAGCTCAGC | 754 |
| IGHJ6_chr14:106325920-106326020 | CCAGCTCAGCTCTGCCCAGGTTAGCTCAGCCCCAGTCCAGGTTAGCTCAGCCCAGCTCAGCCTTGCCCAGGTTAGCTCAGCCCAGCTAAGCTCAACTTGG | 755 |
| IGHJ6_chr14:106326020-106326120 | CTCAGCTCAGCCTAGCTTGGCTCAGCCCAGCACAGCACGCTCAACCCGGTTCAGCTTGGCTCAGCCCAGCCCAGCCCAGCCTAGCTCAGCTCAGCCCCGC | 756 |
| IGHJ6chr14:106326245-106326345 | CCAGCTCAGCGCAGCCCAGCTCAGCTCAGCTCAGCCTAGCCTTGCTCGGCCCAGCTCAGCTCAGCCCAGCTCAGCCTAGCCTTGCTCAGCCCAGCTCAGC | 757 |

EP 4 334 476 B1

| Name | Sequence | SEQ ID NOs. |
|---|---|---|
| - IGHJ6_chr14:106326450-106326550 | TCAGCCCAGCCCTGCCCAGCTCAGCCCAGCTTAGTGCAGCCAAGCCCAGCTCAGCTCAGCTCACCTGGTG CAACTTAGCCCAGCTCAGCTCAGCTCAGCT | 758 |
| IGHJ6_chr14:106326550-106326650 | CAACCCAGTTCAACTCAGCCCAGTTCAGCTCAGCTCAGCCCAGTTCAGCCTTGTTTAGTCTAGGTCAGCTT AGGTCAGTTTTGCCCATCTGAGTCCATTT | 759 |
| IGHJ6_chr14:106326650-106326750 | CTGAAAGCTGGATGGAGTTGTCATGGCCAGAAATGGTCAGCCCACCAGACCTGCTTGTCTCAGCTAAAGC CATCTCATTGCCAGGTTCCTGCACAGCCAG | 760 |
| IGHJ6_chr14:106326750-106326850 | GCTGGCTTCCATCTTTTGTCTCCCTCTACTTGATACCCCAGTTCCCTGCAGTCCTGCCCCAGCGCCACCTGG GTTTTGGTTCCAAAGCATTACCAATCAT | 761 |
| IGHJ6_chr14:106326850-106326950 | TACCACCCTCCACTACCTGGGTGGAATATTTCTTTGCTGCTTTAAAGTCATTAAAACATCTTGAGAATGAG ACCAAGAATTTAGGAGCCTGTGCTGTGAT | 762 |
| IGHJ6_chr14:106326950-106327050 | AAAAATGAGCAGGTCCCCTTGCTCTAGAAGTGGCAGCATATCTTCTGCACCAAGAGGAGGGTATTGAGAT GCTCAGAGCCTCCACCTTCCCGGAGCATCC | 763 |
| IGHJ6_chr14:106327050-100327150 | CCTCCCTTCTGAGTCTGCAGTAAACCCCTGCCTTTAAATTCCCTCTAGATAACAGTCATCATTGGAAACAA CCAAGAAATGCATTTTATCTGAATTTGCC | 764 |
| IGHJ6_chr14:106327150-106327250 | ACTTAAAATTCTGCCATTTACCATAAATCGCTTTGGAAGGCATGGGCTACTTTCAAGGGTGCGATGATGA CCTACAGTCAATGACTTAGACAAGGGCGAT | 765 |
| IGHJ6_chr14:106327250-106327350 | GCCAGTGGGGCTTGGTATGTTCTCAAGCATCATTACCCATGCCATCCCCATTCAGAGGTTGTGGAGCAGCT CGTGCGACCTCTCCTTCAAATGGGCTTTA | 766 |
| IGHJ6_chr14:106327350-106327450 | GGGAAAGTTAAATGGGAGTGACCCAGACAATGGTCACTCAAAAGACTCACATAAATGAGTCTCCTGCTCT TCATCAAGCAATTAAGACCAGTTCCCCTTC | 767 |
| IGHJ6chr14:106327450-106327550 | TAGTGGAAATAAGACGTCAAATACAAAGTTTTAAGAGAAGCAAATGCAGCAGCGGCGGCTGCCTGTCTCT TACCATGTCGGGCGCCTGGTCACTGCGAGC | 768 |
| - IGHJ6_chr14:106327550-106327650 | CTTGCAAAGCTTTGGCATGGAATCATTCCTCCAAGTCCATTAACAAGGGCTGGGGCCTGAGCAGCCAGTC GGCCCGGCAGCAGAAGCCACGCATCCCAGC | 769 |
| IGHJ6_chr14:106327650-106327750 | TCTGGGTAGTCCGGGGAGACCCAAAGCCCAGGCCGGGCCTGGCAGCCACCCTCCCAGAGCCTCCGCTAGG CCAGTCCTGCTGACGCCGCATCGGTGATTC | 770 |

268

EP 4 334 476 B1

| Name | Sequence | SEQ ID NOs. |
|---|---|---|
| IGHJ6_chr14:106327750-106327850 | GGAACAGAATCTGTCCTTCTAAGGTGTCTCCACAGTCCTGTCTTCAGCACTATCTGATTGAGTTTTCTCTT ATGCCACCAACTAACATGCTTAACTGAAA | 771 |
| IGHJ6_chr14:106327850-100327950 | TAATTCAGGATAATGATGCACATTTTACCTAAAACTTATCCTAAAGTGAGTAGTTGAAAAGTGGTCTTGA AAAATACTAAAATGAAGGCCACTCTATCAG | 772 |
| IGHJ6_chr14:106327950-106328050 | AATATCAAAGTGTTTCTCCTTAATCACAAAGAGAAAACGAGTTAACCTAAAAAGATTGTGAACACAGTCA TTATGAAAATAATGCTCTGAGGTATCGAAA | 773 |
| IGHJ6_chr14:106328050-106328150 | AAGTATTTGAGATTAGTTATCACATGAAGGGATAACAAGCTAATTTAAAAAACTTTTTGAATACAGTCAT AAACTCTCCCTAAGACTGTTTAATTTCTTA | 774 |
| IGHJ6_chr14:106328150-106328250 | AACATCTTACTTTAAAAATGAATGCAGTTTAGAAGTTGATATGCTGTTTGCACAACTAGCAGTTGATAA GCTAAGATTGGAAATGAAATTCAGATAGTT | 775 |
| IGHJ6_chr14:106328250-106328350 | AAAAAAAGCCTTTTCAGTTTCGGTCAGCCTCGCCTTATTTTAGAAACGCAAATTGTCCAGGTGTTGTTTTG CTCAGTAGAGCACTTTCAGATCTGGGCCT | 776 |
| - IGHJ6_chr14:106328350-106328450 | GGGCAAAACCACCTCTTCACAACCAGAAGTGATAAATTTACCAATTGTGTTTTTTTGCTTCCTAAAATAGA CTCTCGCGGTGACCTGCTTCCTGCCACCT | 777 |
| IGHJ6_chr14:106328450-106328550 | GCTGTGGGTGCCGGAGACCCCCATGCAGCCATCTTGACTCTAATTCATCATCTGCTTCCAGCTTCGCTCAA TTAATTAAAAAAAATAAACTTGATTTATGA | 778 |
| IGHJ6_chr14:106328550-106328650 | TGGTCAAAACGCAGTCCCGCATCGGGGCCGACAGCACTGTGCTAGTATTTCTTAGCTGAGCTTGCTTTGGC CTCAATTCCAGACACATATCACTCATGGG | 779 |
| IGHJ6_chr14:106328650-106328750 | TGTTAATCAAATGATAAGAATTTCAAATACTTGGACAGTTAAAAAAATTAATATACTTGAAAATCTCTCAC ATTTTTAAGTCATAATTTTCTTAACCATT | 780 |
| IGHJ6_chr14:106328750-106328850 | TTTCTCAGAAGCCACTTCAAACATATCCTGTCTTTTAACAGTAAGCATGCCTCCTAAGATAAACAATCCTT TTCTCTTGGAAACCAGCTTCAAGGCACTG | 781 |
| IGHJ6_chr14:106328850-106328950 | AGGTCCTGGAGCCTCCCTAAGCCCTGTCAGGACGGCAGCCACCGTTTCTGGGCTACCCCTGCCCCCAACC CTGCTCTCATCAAGACCGGGGCTACGCGT | 782 |
| IGHJ6_chr14:106328950-100329050 | CCCTCCTGGCTGGATTCACCCACTCCGACAGTTCTCTTTCCAGCCAATAAAGAATTTAAGATGCAGGTTGA CACACAGCGCACCTCATAATTCTAAAGAA | 783 |

EP 4 334 476 B1

| Name | Sequence | SEQ ID NOs. |
|---|---|---|
| IGHJ6_chr14:106329050-106329150 | AATATTTCACGATTCGCTGCTGTGCAGCGATCTTGCAGTCCTACAGACACCGCTCCTGAGACACATTCCTCAGCCATCACTAAGACCCCTGGTTTGTTCA | 784 |
| IGHJ6_chr14:106329150-106329250 | GGCATCTCGTCCAAATGTGGCTCCCCAAGCCCCCAGGCTCAGTTACTCCATCAGACGCACCCAACCTGAGTCCCATTTTCCAAAGGCATCGGAAAATCCA | 785 |
| IGHJ6_chr14:106329250-106329350 | CAGAGGCTCCCAGATCCTCAAGGCACCCCAGTGCCCGTCCCCTCCTGGCCAGTCCGCCCAGGTCCCCTCGGAACATGCCCCGAGGACCAACCTGCAATGC | 786 |
| IGHJ6_chr14:100329350-100329450 | TCAGGAAACCCCACAGGCAGTAGCAGAAAACAAAGGCCCTAGAGTGGCCATTCTTACCTGAGGAGACGGTGACCGTGGTCCCTTTGCCCCAGACGTCCAT | 787 |
| IGHJ6_chr14:106329450-106329550 | GTAGTAGTAGTAGTAGTAATCACAATGGCAGAATGTCCATCCTCACCCCACAAAAACCCAGCCACCCAGAGACCTTCTGTCTCCGGGCGTCACATGGAAG | 788 |
| IGHJ6_chr14:106329550-106329650 | CTGACTGTCCGTGGCCCTGTCCTGCCCTTCTCATGGAACCCTCTGCTGGCCTCCCACGTACCCCACATTCTGGCCTGACCCCTCAGAAGCCAGACCACTG | 789 |
| IGHJ6_chr14:106329650-106329750 | TCGGCCTGGGAAGTCCAACTGCAAGCAGACGGCTGCTAAGTCACCCCCAGGAGTCCAAAAACCCCGGGGGGCACCCGTCCCAGAGAGCGGGTGCCTTGGA | 790 |
| IGHJ5_chr14:106329750-100329850 | GCGGGACAGAGTCCCACCACGCAATCATCACGACAGCCCCTGAGAATGCTCCAGGTGAAGCGGAGAGAGGTCACCCCAGACCAGCCGAAGGAGCCCCCCA | 791 |
| IGHJ5_chr14:106329850-106329950 | GCTGCCGACATCTGTGGCCGGACTTGGGGAGGACAGGCTGGGTTCCCATTCGAAGGGTCCCTCTCCCCGGCTTTCTTTCCTGACCTCCAAAATGCCTCCA | 792 |
| IGHJ5_chr14:106329950-106330050 | AGACTCTGACCCTGAGACCCTGGCAAGCTGAGTCTCCCTAAGTGGACTCAGAGAGGGGGTGGTGAGGACTCACCTGAGGAGACGGTGACCAGGGTTCCCT | 793 |
| IGHJ5_chr14:106330050-100330150 | GGCCCCAGGGGTCGAACCAGTTGTCACATTGTGACAACAATGCCAGGACCCCAGGCAAGAACTGGCGCCCCGCTACGTCCCTGGGACCCTCTCAGACTGA | 794 |
| IGHJ5chr14:106330150-106330250 | GCCCGGGGAGGGCCCGGGGGTTGTTGGGCATTGGACCCCAGAGGCCTAGGGTGGCCCTGGCCACAGAGAGACCCGTGCTGCTGGGCTCAGGAGGAAGGAG | 795 |
| IGHJ4_chr14:106330250-106330350 | CATCTGGAGCCCTTGCCCCTCGTCTGTGTGGCCGCTGTTGCCTCAGGGCATCCTCCTGAGCCCCCCAGGATGCTCCGGGGCTCTCTTGGCAGGAGACCCA | 796 |

| Name | Sequence | SEQ ID NOs. |
|---|---|---|
| IGHJ4_chr14:106330350-106330450 | GCACCCTTATTTCCCCCCAGAAATGCAGCAAAACCCTTCAGAGTTAAAGCAGGAGAGAGGTTGTGAGGAC TCACCTGAGGAGACGGTGACCAGGGTTCCC | 797 |
| IGHJ4_chr14:106330450-106330550 | TGGCCCCAGTAGTCAAAGTAGTCACATTGTGGGAGGCCCCATTAAGGGGTGCACAAAAACCTGACTCTCC GACTGTCCCGGGCCGGCCGTGGCAGCCAGC | 798 |
| IGHJ4_chr14:106330550-106330650 | CCCGTGTCCCAAGGTCATTTTGTCCCCAGCACAAGCATGACTCTGCCCACCCTTTGCCCCAGCAGCAGAGT CCCAGTTCCCAAAGAAAGGCCTTCTGCTG | 799 |
| IGHJ3 _chr14:106330650-106330750 | AACGTGGTCCCAAACAGCCGGAGAAGGAGCCCCGGAGGGCCCCACATGGCCCAGCGCAGACCAAGGAGC CCCCGGACATTATCTCCCAGCTCCAGGACAG | 800 |
| IGHJ3_chr14:106330750-106330850 | AGGACGCTGGGCCCAGAGAAAGGAGGCAGAAGGAAAGCCATCTTACCTGAAGAGACGGTGACCATTGTC CCTTGGCCCCAGATATCAAAAGCATCACACA | 801 |
| IGHJ3_chr14:106330850-106330950 | GGGACACAGTCCCTGTTCCTGCCCAGACACAAACCTGTGCCCGTGCAGGACACTCGAATGGGTCACATGG CCCAAGCACAGAGCAGAGGCAGCCGGCGTC | 802 |
| IGHJ3_chr14:106330950-106331050 | CCTGTCCCCAGCCACACAGACCCCCGGGCTGAGACCCAGGCAGGGAGGGGTGACGTTCCCAGGGAGACG GTGGCCGGGCTGCCCTGGCCCCAGTGCTCCA | 803 |
| IGHJ3_chr14:106331050-106331150 | AGCACTTGTAGCCACACTAAAGCGCAGGCCTGGTCCCCGGCACATGAACAGCCAGCGCCCAGCCCCAGCC CAGGCTCTGCCCACAACTTCTCCTTCCCGT | 804 |
| IGHJ2_chr14:106331150-106331250 | CCCTGCCCTCGGCCTGCTTGCTACCTGTGGAGGGTCCCTGACGGGGCTGAAGCCCAGCGGGGTCCCTGCC TGTCCTTGGGGGCTCCAGCTGGCCCCAGGG | 805 |
| IGHJ2_chr14:106331250-106331350 | CTAAGTGACAGCAGGGCTCTGGCATGCAGCCCATGGCGGAGACCCCAGGGATGGCAGCTGGTGTGGCCTC AGGCCAGACCCAGGCCGGCTGCAGACCCCA | 806 |
| IGHJ2_chr14:106331350-106331450 | GATACCTGGCCTGGTGCCTGGACAGAGAAGACTGGGAGGGGGGCTGCAGTGGGACTCACCTGAGGAGACA GTGACCAGGGTGCCACGGCCCCAGAGATCGA | 807 |
| IGHJ2_chr14:106331450-106331550 | AGTACCAGTAGCACAGCCTCTGCCCTCCTGCTTCTCCCATACAAAAACACACCCTCCGCCCTCCTGCCGAC CTCCTTTGCTGAGCACCTGTCCCCAAGTC | 808 |

EP 4 334 476 B1

| Name | Sequence | SEQ ID NOs. |
|------|----------|-------------|
| IGHJ1_chr14:106331550-106331650 | TGAAGCCAAAGCCCTTGCCTGGCCCAGTACACCTGGCTCCCCGCTATCCCCAGACAGCAGACTCACCTGAGGAGACGGTGACCAGGGTGCCCTGGCCCCA | 809 |
| IGHJ1_chr14:106331650-100331750 | GTGCTGGAAGTATTCAGCCACGGTGAGTCAGCCCTGAGCCAGGGGCTACAGAAACCCACAGCCCGGGGTCCCGGGGGAGCATGGTTTTTGTAGAGCTGCC | 810 |
| IGHD7-27_chr14:106331750-106331850 | AATCACTGTGTCCCCAGTTAGCACAGTGGTTCTCAGCTCAGCCAAAACCCTGCGGCTGGTAGGGGGCCTGTGGGGCTGGGGGCTGATGTGGCTGCGGTCT | 811 |
| IGHD6-19_chr14:106357890-106357990 | TGCTGGGTCTGTCCTCTGTGGGAGGGGCTGCTACCCAGGCCCAGGACTGCAGTGGAGGGCTCACTGAGGGGCTTTTGGGTCTGGCCTGAGCCGCTGTGGG | 812 |
| IGHD3-3_chr14:106380360-106380460 | GCTCTCAGGTCTACTGCGGGGACACTCGGGTCTGCCCCTGGCTTAGGTGGACAGTGTCCGTGCCCACCTGTGCCCTGAGGCTCCATTTCAGGCTGATATC | 813 |
| IGHD3-3_chr14:106380460-106380560 | TGTCTGTATTGTCCCTACCCGCTGCATGGCCATGTCCTTTTGGGTTTATAAATTGCCCCCAAATCACGCAGGCATCATTCAGGCTTTTTATATTCCCTGG | 814 |
| IGHD3-3_chr14:106380550-106380650 | TATTCCCTGGGCCACCAGGTGCCTCCACCCAGAAAGCTGAGATGTGGGAGGTTCTAGAGTCATTCTGCAACCCTGGATGAGCCCCTGCAGCCTCAGTGCT | 815 |
| IGHD3-3_chr14:106380650-106380750 | ACTGAGGTTCCAGCAAGACCTGGAGCAGGTGCAGATGAGGCCTGAGGCCAGGTGAAGCCCAGGCCAGGTGAGGTCCAGGCCAGTGAGGCCCAGGTCAGAT | 816 |
| IGHD3-3_chr14:106380750-106380850 | GAGGCCCAGGTCAGGTGAAGCCCAGGTCAGGTGAAACCCAGGTCAGGTGAGGCCCAGATCATGTGAGCTCAGGACAGGCAAGGTCCAAGTCAGGTGAGGC | 817 |
| IGHD3-3_chr14:106380850-106380950 | CGAGCTCAGGTGAAGCCCAGAGGTGAGGTCTAGGCCAGGTGAGGTCCAGGCCAGGTGAGGTCCAGGTCAGGTGAGGCCCAGGTCAGGCAAGGCTGAGGTA | 818 |
| 1GHD3-3_chr14:106380910-106381010 | TCCAGGTCAGGTGAGGCCCAGGTCAGGCAAGGCTGAGGTAGATGTATGAGACTTCTGTAATTTTCAGTTGGTGCCAACCCTGCCTGGTGTCCCTGCCCCT | 819 |
| IGHD3-3_chr14:106381010-106381110 | CCTCCCAGCCCATGCTCTGTGCCTGCCAGATGGCGGCCCCTGCACAGGTGCTGCTGGCTGTGGAGGAGCTGGGCTCTGCCTCCCTGTGCATGGGCGTCCC | 820 |

272

(continued)

| Name | Sequence | SEQ ID NOs. |
|---|---|---|
| IGHD3-3_chr14:106381275-106381375 | GCCTGCAGCCTGTCCGGGGATGCCCAGGGAGGTGAGTGCCACCACATATCAGGCCTTTTCTCTTTAAAGTCATTTCTTTGGGGATACATCATCAATGTCT | 821 |
| IGHD2-2_chr14:106381485-106381585 | TCTAAACACAGCTGTGTGCATTTTCCTCTTCTTGCAATTTAGAATTTTAACTGCTGTTTTCAAGGTACTGTAATGTATTTGTTCTCTTCTTGTTAGGAGA | 822 |
| IGHD2-2_chr14:106381585-106381685 | CTTGCCAACCCTGTGTGTCTCAGTTCATACCCTCTTCCTTCCCCAGTAGAAGTAACGACCACTGTGTTTATGTGATCATCCTTTTCTTGATTTTCCTTAT | 823 |
| IGHD2-2_chr14:106381655-106381755 | TGTGATCATCCTTTTCTTGATTTTCCTTATAGTTTTCCTAGTGGAAAGTTTATCCCTTAAGAAGATAGTTCATTTTGCCGGCTGTAAATTTTATTTAGAA | 824 |
| IGHD2-2_chr14:106381890-106381990 | CTGCCATCGTTTATTTGCCTGTTTTCCTTCAGATGGCTGTTTGCTTCATTCTCAGTTTGGGGCTATGACAAACATATGTTCTGCACATCTTTGCCCATGA | 825 |
| IGHD2-2_chr14:106381990-106382090 | GGCTCTCAGGGAGGGCTCTGGAGCTGGCATTGCCTGCAGGGCTCTGCTTTGTTGCAGGGAGTTCCTGCCAAGGCTTTTCAGAGTGTCTGTGCCCAGCCTG | 826 |
| IGHD2-2_chr14:106382090-106382190 | AAGGTACACACTGTACTTTGCCCTTGCATCAGGCACTTTCCTTGTGCTTGCTTCTGTGTGGCTCCACATTCTGGAGAATTTATTCAGATCTGTGCTGCAA | 827 |
| IGHD2-2_chr14:106382.325-106382425 | CTTCCCACACTGTCCTCCTGGGCTCACTCCCAGCCATCGATCTTGAACACCAGTTTATGGAACTATCTGCACAGGAAAGCAGAAACAGCAAAAGGCCCTG | 828 |
| IGHD2-2_chr14:106382905-106383005 | TTGCGTGGACCCTGTTTTTGGTCAAGGGAAGTACTTGCTGGTGAAGGAGACCTCCCCTCCTTTCTTTCTCAGGAGCCCCCTCTGATGCCGTTGCCTGGTG | 829 |
| IGHD2-2_chr14:106383005-106383105 | TTTCTCAGGGCTGGTGCTGGGGGCTCAGCAGTGTCTGCCCTGTTCCAGGTGGGAATGTGGGTCTGTTCTGTTTCCACGCGGTGTTCTGGGGCCGCCAGTG | 830 |
| IGHD2-2_chr14:106383030-106383130 | CAGCAGTGTCTGCCCTGTTCCAGGTGGGAATGTGGGTCTGTTCTGTTTCCACGCGGTGTTCTGGGGCCGCCAGTGAGGGGCTCGGGATGTCAGCGGCTGG | 831 |
| IGHD2-2_chr14:106383130-106383230 | TCTCTGTCCCTATGGTCTGGGCTCCGGTTCACTGCTCCCCTGCCCTCCAGGTCGGTCACTGACTCAGTTACTATCCAGCGGGCTCCGTGGCTGTTCAGTG | 832 |
| IGHD2-2_chr14:106383980-106384080 | GGGAGCAAATGGAGAGGGAAGTGGCAGCGGCCCGAGTGCCAGGCGGTCCCGGTTTGGGGGTTGATCTTTGTGGAACAGCTCCCTGGCCCGTGTGTAAGTGG | 833 |

273

EP 4 334 476 B1

(continued)

| Name | Sequence | SEQ ID NOs. |
|---|---|---|
| IGHD1-1_chr14:106384080-106384180 | TCGGGGGGAGGCACGGAGGTCTGGAGCTACAAGCGGTGGCAGGAAGGCAGGTCCCAGTCTTGGGGGTCTGGAGCTTATCTTCTTCCTGTGAACTGAGTGTG | 834 |
| IGHD1-1_chr14:106384630-106384730 | ATGGAGGACCTGCCTCGGATGACACCCCTATCTTAAGAAGGTCATGGTGGGTTCCAGCTGGGAGGAAGGGAAGTGGGCCACCTCCTGGGGGTCTTCCACC | 835 |
| IGHD1-1_chr14:106384720-106384820 | GTCTTCCACCCCCACCACCTCAGCCTGGGGCCTCTGTGATTCCTCTCTGCACAGACCCCAAAGTCTGTGCTGCCGCAGGGCAGGAAGGAAGGGCCTGTGG | 836 |
| IGHD1-1_chr14:106384825-106384925 | TCGAGGTTGGGGCCACAGTGGTGTTCCCTAAGCCCGAGTCTGGTCTCATGGCCCGCCCCGCAGCAGGTCCTGAGTGAGGGACAGAGACCGGGGCGGGGTC | 837 |
| IGHD1-1_chr14:106384925-106385025 | TTTGGTCCTGGTGGACTCTGGGGTGGATTCCAGTGGGGAGTCATCAGGGTCGGTGTCCCCCAGGGTACTGGGGTGTCTGCTCCTGGAGTCGGCTCTGG | 838 |
| IGHV2-5_chr14:106494090-106494190 | CCTGGGTTTTTGTACAGGAGGTGCCCTGGGCTGTGTCTTTGTGGTCTGTGTGCACAGTAATATGTGGCTGTGTCCACAGGGTCCATGTTGGTCATTGTAA | 839 |
| IGHV2-5_chr14:106494210-106494310 | GTGTCCTTGGTGATGGTGAGCCTGCTCTTCAGAGATGGGCTGTAGCGCTTATCATCATTCCAATAAATGAGTGCAAGCCACTCCAGGGCCTTTCCTGGGG | 840 |
| IGHV2-5_chr14:106494310-106494410 | GCTGACGGATCCAGCCCACACCCACTCCACTAGTGCTGAGTGAGAACCCAGAGAAGGTGCAGGTCAGCGTGAGGGTCTGTGTGGGTTTCACCAGCGTAGG | 841 |
| IGHV2-5_chr14:106494445-106494545 | CTGTGGAGAAAGCATAAGAAGATGAAGCCCACAAACAAGAAAACTGATGTTTCACCCGTGAAGGAGTCCCTGACCACAGCACTCACATGAAGGGATGGTC | 842 |
| IGHV2-5_chr14:106494545-106494645 | AGCAGCAGGAGCGTGGAGCAAAGTGTGTCCATGGTGGGGCACAGGAGTCACTGAGCTGGGACCTGTGCTCGGCTTTTTCAACCCAGAGGAGGGTGGAGCT | 843 |
| IGHV2-5_chr14:106494565-106494665 | AAGTGTGTCCATGGTGGGGCACAGGAGTCACTGAGCTGGGACCTGTGCTCGGCTTTTTCAACCCAGAGGAGGGTGGAGCTGGTGGAGATTTGCATTCCCC | 844 |
| IGHV2-5_chr14:106494650-106494750 | AGATTTGCATTCCCCTCATCTGTGCCCTACTCTATGGGATGGAGTCAGGTTTCAGGACTCAGGAGGGTGTTGCATCTGTGGTGAGGACCAGTGATAGTAA | 845 |
| IGHV2-5_chr14:106494750-106494850 | CATGATCAGTGTAATTCAGATGGCATTAATCTAAGGCTGGGCAAGTAGATTCTGAGTAGAAGTCTTTGCAGAAGTCATGATTATGAGGTCATGTTGGTCT | 846 |

EP 4 334 476 B1

274

| Name | Sequence | SEQ ID NOs. |
|---|---|---|
| IGHV3-7_chr14:106518495-106518595 | GCCCTTCACAGAGTCCACATAGTATTTCTCACTTCCATCTTGCTTTATGTTGGCCACCCACTCCAGCCCCTT CCCTGGAGCCTGGCGGACCCAGCTCATC | 847 |
| IGHV3-7_chr14:106518855-106518955 | TGAGTCCTCTGTGCTCAGTGCTGATCACCAAGTGGAAAGGCCTTGGAGTCCAGGGCTAAGGCTCCTCTCT GAGACCTGCAGGGTCAGGGTTGGGTTGGTT | 848 |
| IGHV3-7_chr14:106518955-106519055 | TTCATCAGTAGAGGGAGGGCCCTATTTGCATGTCTCCTACTATATAAGAAGCTCTAGTGGGATGCTGGAG GAATAGGCTGTACCCATATAAGAAGACGGT | 849 |
| IGHV3-7_chr14:106518970-106519070 | AGGGCCCTATTTGCATGTCTCCTACTATATAAGAAGCTCTAGTGGGATGCTGGAGGAATAGGCTGTACCC ATATAAGAAGACGGTGCTCTGCAGAAGTTT | 850 |
| IGHV3-7_chr14:106519070-106519170 | GCTGACAATGATGGTATTTGGAAAATATGCTGTCTTATGAAATTGTGCTGTGATAAACACTTTGCCCTGAT CACCCTATTACATTTTTTAAAAAATGTGT | 851 |
| IGHV3-11_chr14:106573540-106573640 | CAAACACAGAGACAACCTAGTCAGAAACTGCCACATATATTCACTGCTTATCTCACTCACGTCCACTCAAT GTCTCTAGTTCTCCATAAATCACCTTTTA | 852 |
| IGHV3-11_chr14:106573640-106573740 | TAATAGCAACAAGGAAAACCCAGCTCAGCCCAAACTCCATGGTGAGTCCTCTGTGTTCAGTGCTGATCAC CGAATGGAAACTCCTGGGAATTCTGGGGCT | 853 |
| IGHV3-11_chr14:106573685-106573785 | GTCCTCTGTGTTCAGTGCTGATCACCGAATGGAAACTCCTGGGAATTCTGGGGCTGGGGCTCTTCTCCCAG AGCTGCAGGGTCTGGGCTCGGCTGGTTTT | 854 |
| IGHV3-11_chr14:10657.3785-106573885 | TATCAGCAGAGGGAGGGCCCTATTTGCATGTCTCCTACTATATAGCAAGCTCTAGTGGGACGCTGGAGGA GAGGGCAGTGCCCAGAGCAGATGAGAGGGT | 855 |
| IGHV3-11_chr14:106573885-106573985 | CCCGGAAAACACTGGAGGTAATCCTATCTCTCAGGAAAATATAACTTCAGATTATGTGATTGTGACTTGA TGATCAATTAGCAGTCATCATCTTATTTAA | 856 |
| IGHV3-11_chr14:106573985-106574085 | TGTTTACATATTTGCAGAATATATTCAGTGCAAGTGTCAATGTTACATTTTTAGAGAAGATGAATTACATA CATAACAGAGCAGTTGTGCAATGTGTCCA | 857 |
| IGHV3-15_chr14:106610690-106610790 | ACTCACACTTAATCTCTCTAGTTCTCCATAAATCACCTTTTAAAATAGCAGCAAGGAAAATCCAGCTCAGC CCAAACTCCATGGTGAGTCCTCTGTGTTC | 858 |
| IGHV1-18_chr14:106642110-106642210 | GATGCTATTTAATAGCCCAATTCCTGACCCAGGATGAGAAAGAGCAAATACATGACACATGGACGACACA ATTGTAGAAGCTGAGGGTTCAAGCCGTAAT | 859 |

| Name | Sequence | SEQ ID NOs. |
|------|----------|-------------|
| IGHV1-18_chr14:106642210-106642310 | CCTGTTAGAGGCCACGCATCCCCTACCCATCCCTGAACTCTGTGTTGACAGAGCTTCCCCCACTGGAGAAC AAGCTCCCCCAGGACACGCACCTCACTTA | 860 |
| IGHV3-23_chr14:106725295-106725395 | GGCCCTTCACGGAGTCTGCGTAGTATGTGCTACCACCACTACCACTAATAGCTGAGACCCACTCCAGCCCC TTCCCTGGAGCCTGGCGGACCCAGCTCAT | 861 |
| IGHV3-23_chr14:106725395-106725495 | GGCATAGCTGCTAAAGGTGAATCCAGAGGCTGCACAGGAGAGTCTCAGGGACCCCCCAGGCTGTACCAA GCCTCCCCCAGACTCCAACAGCTGCACCTCA | 862 |
| IGHV3-23_chr14:106725550-106725650 | ACTGTTTCTCTCACTCTTATCCATTCACACTCAATTTTTCTATTTCTCCATGAATTACCTTTTAAAATAGCC ACAAGAAAAAGCCAGCTCAGCCCAAACT | 863 |
| IGHV3-23_chr14:106725650-106725750 | CCATGGTGAGTTCTCTCTGTTCAGTCCTGATCACCAAATGAAAACACCTGAAAATCCCAGGGCTGGGCTC CTCTCTCAGAGCTGCAGGGTCAGGGCTGGG | 864 |
| IGHV3-23_chr14:106725780-106725880 | TTTGCATATCTCCTACTATATAGTAAGCTCTGGGGTGAGAGGCCTTTGGAGATAGTGGGGCTCAGAGCAT GTCAGAATGTCCTCGGGGAGATCTGTGATA | 865 |
| IGHV3-23_chr14:106725880-106725980 | TTGAAAGCATTGGGAAATTGTGCTTTCCTATTGTCAGTTTGTTTTGTGATAAACTTAAACCTTAAAACCTA AAAATCTTATAATTTTGTAATTTTTATTT | 866 |
| IGHV3-23_chr14:106725995-106726095 | GAGGTACCATAGATCTACATAAACTGCATATTTTTAAAGTTAGCACCAATCATCTTTTATTTTTACATACG CAGAGAAACCATGGTATATAGTATCAATA | 867 |
| IGHV3-23_chr14:106726095-106726195 | TTATTTCCATGTTAAAGATGAAAAATTATCAGCAAAAGCACAGGTGGGTTTTACAATGTCCCCAGTGCTC ACTTTTGGTCAGAGTGAGCCTGGGCATCTG | 868 |
| IGHV1-24_chr14:106732970-106733070 | TCCTACATAATGACAGTGTACACATCTTTCCATTGCTGTTTTACTCAATTACTCAACCCATTTTCTAAACAG ATTTAAACTTCATAAATCCTGTCATCTC | 869 |
| IGHV1-24_chr14:106733070-106733170 | CTCAGCCTCAGCACAGCTGCCTCATTCCTCAGGGTTTCTGACGCTCTCAGGATGTGGGTTTTCACACTGTG TCTGTTGCACAGTAATACACGGCCGTGTC | 870 |
| IGHV1-24_chr14:106733185-100733285 | GCTCAGCTCCATGTAGGCTGTGTCTGTAGATGTGTCCTCGGTCATGGTGACTCTGCCCTGGAACTTCTGTG CGTAGATTGTTTCACCATCTTCAGGATCA | 871 |
| IGHV1-24_chr14:106733275-106733375 | TTCAGGATCAAAACCTCCCATCCACTCAAGCCCTTTTCCAGGAGCCTGTCGCACCCAGTGCATGGATAATT CAGTGAGGGTGTATCCGGAAACCTTGCAG | 872 |

| Name | Sequence | SEQ ID NOs. |
|------|----------|-------------|
| IGHV1-24_chr14:106733375-106733475 | GAGACCTTCACTGAGGCCCCAGGCTTCTTCACCTCAGCCCCAGACTGTACCAGCTGGACCTGGGCGTGGG TGCCTGTGGAGAGGACAGAGGAGTGGATGA | 873 |
| IGHV1-24_chr14:106733475-106733575 | GACACCACTTAACTGGACCCAGTCCCCTCATCAGCCCTGGAACTCAGGATTCTCTTGCCTGTAGCTGCTGC CACCAAGAAGAGGATCCTCCAGGTGCAGT | 874 |
| IGHV2-26_chr14:106758470-106758570 | GAGGGTGGGAATCTGGGAGAGCAAGGGGCTTCCCATAAGTGTTCTGATAAAAATCCTCTTTGTTTAGGGG GAAAGTGATGATTTTTTTGAATGATAGAGA | 875 |
| IGHV2-26_chr14:106758570-106758670 | ATACATCACCCAAACATTTAAAAATGTATTGTGTAAAGAAGTGTAAATGGCATCTCAGCCATTTACACAC TGCAAGACACACAGCTTATTAGTGTGCCTG | 876 |
| IGHV3-30_chr14:106791090-106791190 | TGGTGAATCGGCCCTTCACGGAGTCTGCATAGTATTTATTACTTCCATCATATGATATAACTGCCACCCAC TCCAGCCCCTTGCCTGGAGCCTGGCGGAC | 877 |
| IGHV4-31_chr14:106805945-106806045 | ACAATCACTTGAGTTCAGACACACCAGGATTCACTTAATGTTATTTTTAGTTCAGAACCTCTATCAGGTTT AGAGGGAATCGCTCTGTCCCAGGGAGTGG | 878 |
| IGHV4-31_chr14:106806045-106806145 | ATCTTACAATAGCAAAACGGTCTTAGAAAACCCAACATAATCTACAGCGAGACCTCAGCATGGCAAGCAA GGAATCACTAAAGCCACCAGGGAGATCCGG | 879 |
| IGHV4-31_chr14:106806120-106806220 | CACTAAAGCCACCAGGGAGATCCGGATGCACTGATACGATCCAGAAACATAGCGAGTCCGGGAACTGAT GCGGACTTTGAGGCAGCCTCTTTTTTTTTTT | 880 |
| IGHV3-33_chr14:106815805-106815905 | GATGGTGAATCGGCCCTTCACGGAGTCTGCATAGTATTTATTACTTCCATCATACCATATAACTGCCACCC ACTCCAGCCCCTTGCCTGGAGCCTGGCGG | 881 |
| IGHV3-33_chr14:106815905-106816005 | ACCCAGTGCATGCCATAGCTACTGAAGGTGAATCCAGACGCTGCACAGGAGAGTCTCAGGGACCTCCCAG GCTGGACCACGCCTCCCCCAGACTCCACCA | 882 |
| IGHV4-34_chr14:106829685-106829785 | CTCGACTCTTGAGGGACGGGTTGTAGTTGGTGCTTCCACTATGATTGATTTCCCCAATCCACTCCAGCCCC TTCCCTGGGGGCTGGCGGATCCAGCTCCA | 883 |
| IGHV4-34_chr14:106829765-106829865 | GGCTGGCGGATCCAGCTCCAGTAGTAACCACTGAAGGACCCACCATAGACAGCGCAGGTGAGGGACAGG GTCTCCGAAGGCTTCAACAGTCCTGCGCCCC | 884 |
| IGHV4-34_chr14:106829865-106829965 | ACTGCTGTAGCTGCACCTGGGACAGGACCCCTGTGAACAGAGAAACCCACAGTGAGCCCTGGGATCAGA GGCAGCATCTCATATCTTCATATCCGCATTC | 885 |

| Name | Sequence | SEQ ID NOs. |
|------|----------|-------------|
| IGHV4-34_chr14:10682965-106830065 | CTGAGACACTCACATCTGGGAGCTGCCACCAGGAGGAGGAAGAACCACAGGTGTTTCATGTTCTTGTGCAGGAGGTCCATGACTCTCAGAAAGCACTTCC | 886 |
| IGHV4-34_chr14:106830125-106830225 | GAGGATTTGCATGTGGGTGGTGCCTTTGTATGGATAGGTAAAAAGGGATGAGGGAGGCCCCAGTCTTTTGGGCTCACCCTGGGAGGTGTATGCTGGCTGT | 887 |
| IGHV4-34_chr14:106830240-106830340 | AGTTCTCTTCCTGTGGCCTCCCCTCACCAAACCCAGAGTCCTCTTCTTCCAGGTAGGAAATGTGCTGAAGGAGCTGGTCTGGGAGACAAGTGTGATCATG | 888 |
| IGHV4-34_chr14:106830315-106830415 | GGTCTGGGAGACAAGTGTGATCATGGATCAAAGACAGATTTTGGAATACAGTTAATACTGTTCTACATTTAAAGATTCATATAACACCAACCATACACCC | 889 |
| IGHV4-34_chr14:106830415-106830515 | AGGTCACCTAAATTGTCATTTACCCCTTCAGACATATTGAAACAGCTGCTGAGTGTAATAATCACAGTGAATTGAGACAAACCTGGATCCATGCAATGTG | 890 |
| IGHV4-34_chr14:106830515-106830615 | TACTGTAGTTCAGAACATCCATCATGGTTAGAAGGATGCTACCTGTCCCAGGAAGTGGGTTATTTTTAAATAGTACCTGAGAGCTGCCCTTCTGAGACCT | 891 |
| IGHV4-34_chr14:106830615-106830715 | TTTGAAATTTGAGATTGTGTGTGAGATCTCAGGAGAAGGTAGTAGAATATATCTCCATCCTTCTCAATGTGTAACCCTGAGAATATGGCCTGACCTCTAA | 892 |
| IGHV4-34_chr14:106830715-106830815 | ACATTTCTGTGTGAAAAGATGTACATTGGGGATAGCAGTGACAGCTTCAGATGAAAACTCTATAGTACATCAGCACTGGAGGATAGTCTCATCACCAAGA | 893 |
| IGHV4-34_chr14:106830815-106830915 | TTAGTGAAATTACCTTTCCTGGGAACCAGAGAGGACCTCTGTGAGCTCTACCCTCTGAGAGAACAAGGAACTCTGGTTCTTCCCTGACAGGTCACACCTG | 894 |
| IGHV4-34_chr14:106831185-106831285 | AACAAGTGGGCTGGCCTTCTATGAGACGACAGAGGGAAAGAGACAGACTCAATATCCAGAGCGAGGTGAGCTCCTTACCTACCTACCAGGTGGTCTCTGG | 895 |
| IGHV4-34_chr14:106831285-106831385 | GCCATTTGTTTGAGCAGACCCAGAAGTACCTTCCTCACCCTCAGGAGAATTATGAACATTGAGAGAAACTGAGATACTTTTTTTATTTACAGGGAATATT | 896 |
| IGHV4-34_chr14:106831385-106831485 | TCATCGGCGTGTTTACATCTACCTGGGTGTGTACAGGGATGCTAGGATGTGCTCATACACAGAAGAGCAAGAATTATATTTCGTGGAAAGAAAACCAAAG | 897 |
| IGHV4-34_chr14:106831485-106831585 | AGCTTCTGAATTTGTAGGTATTGTTTGCTGCAAATGTGTCAGGTCACTAGATCATGTTATGCTGCTAGAAGAAAAACTTCCCAACATTGTCATGGAGACA | 898 |

| Name | Sequence | SEQ ID NOs. |
|---|---|---|
| IGHV4-34_chr14:106831585-106831685 | AAATGCAAAACAGTAAAGATTCAACTGAGATTCCCTTGAAAATCACCAGTAATGAACAGGCCAAAAGAAATCAACCATTGTGGAAAGAGTGGTCATTAAG | 899 |
| IGHV3-35_chr14:106846385-106846485 | CCCAGTGTCACCTTACACATCCTGCAGGTCACCTCACACATCCACCAGGTCACCGCACATATACCCCACATCACCTCAGACACACCCTGGTCACCTCATA | 900 |
| IGHV3-35_chr14:106846485-100846585 | CATACGTCAGGTCACCTCACGCTCACCCAAGGTCACCTCACACATCCCGCAGGTCACCTCGTAAATCCCCCAGGTCACCACATACATGCACCAGTTCACC | 901 |
| IGHV4-39_chr14:106877715-106877815 | CTCTTGAGGGACGGGTTGTAGTAGGTGCTCCCACTATAATAGATACTCCCAATCCACTCCAGCCCCTTCCCTGGGGGCTGGCGGATCCAGCCCCAGTAGT | 902 |
| IGHV4-39_chr14:106877815-106877915 | AACTACTACTGCTGATGGAGCCACCAGAGACAGTGCAGGTGAGGGACAGGGTCTCCGAAGGCTTCACCAGTCCTGGGCCCGACTCCTGCAGCTGCAGCTG | 903 |
| IGHV4-39_chr14:106877930-106878030 | GAACAGAAAAACCCACAGTGAGCCCTGGGATCAGAGGCAGCCTCCCATATCTCCATGTCTGCATCCTAGAAACACTCACATCTGGGAGCCGCCACCAGCA | 904 |
| IGHV4-39_chr14:106878030-106878130 | GGAGGAAGAACCACAGGTGCTTCATTTTCTTGCACATGAGATCCATGACTCTCAGAAAGCATTTCCCTTATGAGTTGGACCTGAATTTAAGGAAATGTGT | 905 |
| IGHV4-39_chr14:106878130-106878230 | GGTGGCTTCCTGTGGGCGCCTAAGTGAGGATTTGCATGGGGGTGGTGCGTTTGTACGGAGCAGTGAAAAGGGATGAGAGAGGCGCCAGTCTTTTGAGCTC | 906 |
| IGHV4-39_chr14:106878230-106878330 | ACCCTGGGAGGAGAATGCTGGCTGTGCCCTTTGAGAACTCAGTTCTCTTCTTGGGCCTCCCCTCTCCAAGCCCAGAGTCCTCTTCTTCCAGGTAAAGAGA | 907 |
| IGHV4-39_chr14:106878330-106878430 | TGTGCTGAAGGAGCTGGTCTGAGAGATGAGTGTGATCCTGGATCAAGGACAGATTTTGGAATAGGGTCAGTACTGTTCAACCCTTAAAGATTCATATAAA | 908 |
| IGHV4-39_chr1d:106878430-100878530 | ACCCACCACACACCCAGGCCATCTAAATAGTCATTTACCCTTTCAGACACATTGAAACAACAGCTGAATGTAATAATGACAGTGACTTCAAACAATACTG | 909 |
| IGHV4-39_chr14:106878540-106878640 | ATGTTTATTGTAGTTCAGAACATCCACCATGGTTACAGGGAAGCTCACTGTCCCTGGAAGTGGGTCATTTTTTAAAAGCACCTGAGAGCTGTCCTTCTGT | 910 |
| IGHV4-39_chr14:106878680-106878780 | AAGGTAGTGGGACATATCTCCATACTTCTCAATGTGTGACCTTGAAGATGTGTCCTGCCCTCTAAACACTTCTGATTGAAAATATGTAGATTGGGGATTA | 911 |

| Name | Sequence | SEQ ID NOs. |
|---|---|---|
| IGHV3-48_chr14:106994300-106994400 | GTGGAAATGCCTTGGAATCCAGGGCTAAGGCACCTCTCTGAGAGCTGCAGGGTCAGGGTTGGGTTGGTTTTCATCAGTAGAGGGAGGGCCCTATTTGCAT | 912 |
| IGHV3-48_chr14:106994430-106994530 | GGACCCTTGAGGAGTAGGCTGTACCCAGATAAGACGACGGTGCCCTGTAGAAGTTTGCTGGCAATGATTGCATTTGGAAAATATGCTGTCTTATTATGAA | 913 |
| IGHV3-48_chr14:106994530-106994630 | ATTGTGCTGTGATAAACACTTTGCACTAATCACCCTATTTCATTTTAAATATTCATGTAAACTATGTTCTGTAGGAGACAATATTTTCTCCATTTACAGA | 914 |
| IGHV3-48_chr14:106994545-106994645 | ACACTTTGCACTAATCACCCTATTTCATTTTAAATATTCATGTAAACTATGTTCTGTAGGAGACAATATTTTCTCCATTTACAGAAGTGGAAGTAAACCC | 915 |
| IGHV3-48_chr14:106994660-106994760 | CTGTATGCATCTAGGAGCTCATGTCTGGGATGAGTGAACCCCGGTATCTGGCCCTGTGCTCTTCATCACTGTCTCTGACATCCCCCTAAACCAACTCCAG | 916 |
| IGHV3-48_chr14:106994760-106994860 | GACAAAGCTGGATGTGTCTAGTGTTTTTATCAGAACCCACTTTCCGTAATAAGAGCATGTGTGGTTTTGCTGCCCTCCAGCACTCTTCTGAAAATATGGA | 917 |
| IGHV3-48_chr14:106994860-106994960 | GAGAACTAGGATCCAGGCACATTAATTTTCAGGTACTTCTGACATTGAACTTATTTTTTCTATCTTTCTATTACTCTTTCCTTGTCTAAGTTTCCATTTG | 918 |
| IGHV4-59_chr14:107083565-107083665 | AGAGAGACCCACAGTGAGCCCTGGGATCAGAGGCACCTCCCATATCCCCATGTCTGGATCCCTGAGATACTCACATCTGGGAGCTGCCACCAGGAGAAGG | 919 |
| IGHV4-59_chr14:107083665-107083765 | AAGAACCACAGATGTTTCATGTTCTTGCACAGGAGGTCCAGGACTCTCAGAAAGTATTTCCCATGTGAGCTGGAACCTGAATTTAAGGAAATGTGTGGTG | 920 |
| IGHV4-59_chr14:107083790-107083890 | ATTTGCATGTGGGTGGTGCCTTTGTATGGAGAGGTGAAAAAGGAGGAGGGAGGCCCCAGTCTTTTGGGCTCGCCCTGGGAGTAGGATGCTGGCTGTGCCC | 921 |
| IGHV4-59_chr14:107083890-107083990 | TTTGAGAACTCAGTTGTCTTCTTGGGGTCTCCCCTCTCCAAGCCCAGAGTCCTCTTCTTTCAGGTAAAGAGACGTGCTGAAGGACCTGGTCTGGGAGATG | 922 |
| IGHV3-64_chr14:107113405-107113505 | CTGACAGTGGTGACCATGGTTGAGAACTTTTCATCTCCTCTGTGAGGATCAATCTGCATTTTCTGCATAGGAGAATAGGTTTTCATATTAAAACAATCAT | 923 |
| IGHV3-64_chr14:107113505-107113605 | TTTAAAAATATGTAGAAATGACCCTAGTAATCACAGAATTCCGAACTTAGGTTCAGTAGAGAAACTTTAAGAAGATGAAGTCCCACATCGTGACAGGAAA | 924 |

280

EP 4 334 476 B1

| Name | Sequence | SEQ ID NOs. |
|---|---|---|
| IGHV3-64_chr14:107113820-107113920 | TGGAGATGGTGAATCTGCCCTTCACAGAGTCTGCATAATATGTGCTACCCCCATTACTACTAATAGCTGAAACATATTCCAGTCCCTTCCCTGGAGCCTG | 925 |
| IGHV3-64_chr14:107113920-107114020 | GCGGACCCAGTGCATAGCATAGCTACTGAAGGTGAATCCAGAGGCTGCACAGGAGAGTCTCAGGGACCCCCCAGGCTGGACCAAGCCTTCCCCAGACTCC | 926 |
| IGHV3-64_chr14:107114095-107114195 | TTCTCTCACTCATGTCCACTCACACTCAATATCTCTATTTCCTCATGAATCACCTTTAAAAATAGCAACAAGGAAAACCCAGCTCAGCCCAAACTCCATC | 927 |
| IGHV3-64_chr14:107114195-107114295 | ATGACTCTTCTGTGTTCAGTGCTGATCACCAAATGAAAACACCTGGGAATCCCAGGGCGGGGGCTCCTCTCCCAGAGCTGCGGAGTCAGGGCTGGGCTGG | 928 |
| IGHV3-66_chr14:107136755-107136855 | TAGGGCACATCCTTCCCATCCACTCAAGCCCTTGTGCATGGGCCTGGCGCACCTAGTGCATAGAGTAACTGGTGAAGGTAGGTGTATCCACAAGTCTTGC | 929 |
| IGHV3-66_chr14:107136855-107136955 | AGGAGACTTTCACTGATGCCCCAGCCTTCTTCATCTCATCCCCAGACTGCACCAGCTGCACCTGGGACTGGGCACCTGTGGAGAGGACACGGGAGTGGAT | 930 |
| IGHV1-69_chr14:107169645-107169745 | GAAAACTTGTTCACAGTAGCACCTTCATGGAATGTTTGTATCAACGTTATAGAGTGTGGCCTTTTCCACTCTGTGAATTTGGCTTATATTACGACTCTTG | 931 |
| IGHV1-69_chr14:107169745-107169845 | AATGGAATATTTATCTTAAAATTAGAGTATGTACTTGTTTCTACTGTTCTTTTTTTCTCAAATATATAACCCATTTTGTAAACAGCCTTAAACCTAATAA | 932 |
| IGHV1-69_chr14:107169970-107170070 | CTGCTCAGCTCCATGTAGGCTGTGCTCGTGGATTTGTCCGCGGTAATCGTGACTCTGCCCTGGAACTTCTGTGCGTAGTTTGCTGTACCAAAGATAGGGA | 933 |
| IGHV1-69_chr14:107170070-107170170 | TGATCCCTCCCATCCACTCAAGCCCTTGTCCAGGGGCCTGTCGCACCCAGCTGATAGCATAGCTGCTGAAGGTGCCTCCAGAAGCCTTGCAGGAGACCTT | 934 |
| IGHV1-69_chr14:107170170-107170270 | CACCGAGGACCCAGGCTTCTTCACCTCAGCCCCAGACTGCACCAGCTGCACCTGGGACTGGACACCTGTGGAGAGGACACAGGGGTGAATAAAATCCTCT | 935 |
| IGHV1-69_chr14:107170220-107170320 | CCTGGGACTGGACACCTGTGGAGAGGACACAGGGGTGAATAAAATCCTCTTTAACTAAACCAGGATCCCTTCCTCAGCCTTAGGACTAGGAAGCCCCTTA | 936 |
| IGHV1-69_chr14:107170320-107170420 | CCTGTAGCTGCTGCCACCACAAAGAGGAACCTCCAGGTCCAGTCCATGGTGATGAGCTGTGCTCCCAGGGGCTTCTTCAGAGGAGGAATGTGGTTGTTAT | 937 |

| Name | Sequence | SEQ ID NOs. |
|---|---|---|
| IGHV1-69_chr14:107170420-107170520 | GTGATGCTCTCAGGGCACCAATATATCTATATTTATCTCAGAAGACCTCAGGTTATTTGCATATGCATGAGGCAGGGTATTTCACAGCTCAAAGCCTGAT | 938 |
| IGHVI-69_chr14:107170475-107170575 | TTTGCATATGCATGAGGCAGGGTATTTCACAGCTCAAAGCCTGATCTAGGATGAGAAAGAAAACACAGATGCCACATCAGCTGTACAAGTGTGGGATGCT | 939 |
| IGHV1-69_chr14:107170660-107170760 | CAGAACAAACCCCAACCCCAGGATGCACTCCTCACTGTGAACCCACATTTTATTGGCCTAAAGATTACCTGGGTTTTTTGTGGGACCATTGCTGTCTCTG | 940 |
| IGHV1-69_chr14:107170760-107170860 | ACATTGAGCAGGCACCTAGACCCATCCTGGTCCCATTAGGAACACTCAGAGCTCACTGGTAACACTGAAAAGGTGGCCACTCGTTACCCTACATGAGTGT | 941 |
| IGHV1-69_chr14:107170860-107170960 | CCAGCAGGACCCATGGAGAGTTCTGAGATCTGCTGGGCACTCCCAAGACAGGGTCCCCAGCACTTTCCTGAGGGTCCTGACCTCCCAGGTCCTTCAGTGG | 942 |
| IGHV2-70_chr14:107178305-107178405 | TTATCCATTTCTATGTGTTCTTTTGAAAATGTCTACTCATGTCCTTTGCTCATTTTAACGGAGTTATTTGGTTCTTGTTGCTGTTGTTGTTGTAGAGTTG | 943 |
| IGHV2-70_chr14:107178415-107178515 | TTGCAAATTCTTCATATTAGTTCCCTGTCACAGGCAAAGTGTGCAAAAGTTTTCTGTCATTCTGTAAATTGCGTATTCACTCTGTTGTTGTGAAAAAAAT | 944 |
| IGHV2-70_ch14:107178515-107178615 | TATTTAGGTTAATTAAATCTCATCTGTCTATTTTTTTTTTAGGTAGCAGGACCTTTCATGCTGAATCTTTGTCAAACAGGATACAGCTTCTGCTTGCATGA | 945 |
| IGHV2-70_chr14:107178615-107178715 | ACCACTAACAGGGGACATGCCATTTATTAGTAAAGAAAAAGGAGGAAAACAAGGCTCTGAGTCAGATGGGGATGGGAAACGCACGCCCTGGGCAGGAAAT | 946 |
| IGHV2-70_chr14:107178715-107178815 | GGCATCTCAGCCACACTATCCTGTTCTGCAGAAGTGGGGAGGGAGCACCACTGAAAAACACCTGGGTTCTTGTACAGGAAGCGCCCTGGGCTGTGTCTCT | 947 |
| IGHV2-70 _chr14:107178815-107178915 | GTGGTATCCGTGCACAATAATACGTGGCTGTGTCCACAGGGTCCATGTTGGTCATTGTAAGGACCACCTGGTTTTTGGAGGTGTCCTTGGAGATGGTGAG | 948 |
| IGHV2-70_chr14:107178880-107178980 | ACCTGGTTTTTGGAGGTGTCCTTGGAGATGGTGAGCCTGGTCTTCAGAGATGTGCTGTAGTATTTATCATCATCCCAATCAATGAGTGCAAGCCACTCCA | 949 |
| IGHV2-70_chr14:107178980-107179080 | GGGCCTTCCCTGGGGGGCTGACGGATCCAGCTCACACACATTCCACTAGTGCTGAGTGAGAACCCAGAGAAGGTGCAGGTCAGTGTGAGGGTCTGTGTGGG | 950 |

282

EP 4 334 476 B1

| Name | Sequence | SEQ ID NOs. |
|---|---|---|
| IGHV2-70_chr14:107179080-107179180 | TTTCACCAGCGCAGGACCAGACTCCCTCAAGGTGACCTGGGATAAGACCCCTGTGGAGAAGACATAAGAAGATGAAGCCCACAAAGGAGAGAATAGATTT | 951 |
| IGHV2-70_chr14:107179130-107179230 | CTGTGGAGAAGACATAAGAAGATGAAGCCCACAAAGGAGAGAATAGATTTTTTGCTTCTGAAGTACTACCTGACCACAGCACTCACAGGACGGGACAGTC | 952 |
| IGHV2-70_chr14:107179230-107179330 | AGTAGCAGGAGCGTGGAACAAAGTATGTCCATGGTGGAGAGCAGGATTCACTGAGCGAGGCCCTGTCCTCGTCTTTTGAACCCAGGGGAGGGTGGAGCTG | 953 |
| IGHV2-70_chr14:107179330-107179430 | GTGGAGATTTGCATCCCCTCATCTGAGCCCTACTCTATGGGGTGCACTCAGGTCTCAGGACTCAGTAGGGGAGTGCATCTGTGGTGAGGAGCAGTGAGCC | 954 |
| IGHV2-70_chr14:107179360-107179460 | TACTCTATGGGGTGCACTCAGGTCTCAGGACTCAGTAGGGGAGTGCATCTGTGGTGAGGAGCAGTGAGCCCTCAGGTGTGGGGGTCCACGTGTGCTCTCC | 955 |
| IGHV2-70_chr14:107179460-107179560 | ATCAGGGAATCTATCTCATTTCAGCACCATGGCTCTCAGTCAAGTCTTGACGCTCCTGCTTCTACAGACAGGATCTTCTTCGATGCTCCCGCACCGGACA | 956 |
| IGHV2-70_chr14:107179560-107179660 | TGCAACCTTCTGGTTTTAGTCCTAGAGGATTAGAGTAGAAATCAAGAGAGCTGCCGTTCCTCCTCCCTTCAAGAATAATGATGGTGGGCATCTGGGGGGC | 957 |
| IGHV2-70_chr14:107179660-107179760 | AAGGGGCTCCCCACAAGCATTCTGATCAAAATCCTCTTTGATTATGGGGAAAAGTGATGAATTTGTGTAAAAAAATTGGAGAGAATAAATAAGAAAATAC | 958 |
| IGHV2-70_chr14:107179760-107179860 | AGTTACAAGTAATTATGTAAAGAAGTGTGTGCTTAGCAGTGTGTGTGCACACAGCTGCATTCCTAGAGGCATGTTCCATGAAAAATCGATGTTGTCCTTG | 959 |
| IGHV2-70_chr14:107179860-107179960 | TGCCCCGTCAGTTCTGTGGAGAGAGTAGACTGCATGAATGACTTCCCTTTTCTCAGCCCATGAATGAGCGGATGCTTTGGACAAGGGAATTGGAAGACTC | 960 |
| IGHV2-70_chr14:107179960-107180060 | CTGAGGGAGCAGCAGGCTGACTGTTGCAGCCTTGCTCTGCACCTGCACTGGATGTGGTCTCTGTGCTCATAAGGCCGTGGAAACTCATCAATCCAGGTTC | 961 |
| IGHV7-81_chr14:107258910-107259010 | CAAAAAGGGGTTAAATGATTTTGGAAAAGTAAGTAGAAAATAAAAGAAGGAGGGAGTAAGAGCGGACAGAAGGGAGGAAGGCAAGCAAGCAATGATGAAC | 962 |
| IGHV7-81_chr14:107259010-107259110 | TGTGTAAAATTTTCACTAATTAAAAGACTATTATATTGAAGAGGTGCCTATTAGGCAGCCTTTTGATGTTAACCATGTAATATACACCATGAACAACCTT | 963 |

283

EP 4 334 476 B1

| Name | Sequence | SEQ ID NOs. |
|---|---|---|
| IGHV7-81_chr14:107259100-107259200 | GAACAACCTTGTAGAACACACAAGAGCCCCCTCAGAGAACTGGATGGGTCAGGTCTCCCATCCAGTTGCC TTAGGGGTTAGGAACGCTCCCATGTTGTTC | 964 |
| IGHV7-81_chr14:107259200-107259300 | TCTGGTTTTTGCTCCTGAGGACACAAACAGCCAGTGTTTCCTCCCCGGATGAATAGAGAGGCCCCTGGGG AGGGTGTGTCTGGCAGCTCACTCTGCACCT | 965 |
| IGHV7-81_chr14:107259235-107259335 | GTTTCCTCCCCGGATGAATAGAGAGGCCCCTGGGGAGGGTGTGTCTGGCAGCTCACTCTGCACCTGCACC GCGGAAGGTTTTAGATGGTCCCTCTCACAC | 966 |
| IGHV7-81_chr14:107259335-107259435 | AATAATACATGGCGGCGTCCGAGGCCTTCAGGCTGCTCCACTGCAGGTAGGCGGTGCTGCTGGAGCTGTC GGCTGAGATGGTGACGTGGCCTTGGAAGGA | 967 |
| IGHV7-81_chr14:107259435-107259535 | TGGGCTGTATCTGGTATCAGAGTTCCCAGGATAGATGCTCCCCATCCACTCCAGTTCTTTCCCGGGCATCT GGCGCACCCAGTGGATCCAGTAGCTGGTA | 968 |
| IGHV7-81_chr14:107259555-107259655 | ACAGGAGATCCTCAGAGACTCCCCGGGTCTTTTCACCTCTGCTGCAGACTGCAACAGCTGCACCTCGGCA AAGACACCTGTGTGGGAGACACAAAATTTG | 969 |
| CIITA_chr16:10971440-10971540 | GTGTCTGGAGTATGAACCATGTATCAGCACCGAAAGGTTCTAGAAGTCAGACTTTCGGGCAGTGTGTCAC TAACTCTCAGCATGCTGGCCTGGCTCGGCC | 970 |
| CIITA_chr16:10971540-10971640 | CACAGCAAGGTCTTCTCGCCTCCCTTTGGGTAAATACTGAGGGGTGCCTCTGCAGGACGGGACCTCTGCC AGACTCCACTCCATACCCAGAGAAGCAGGG | 971 |
| CIITA_chr16:10971640-10971740 | AAACCAAAATTGGAGTCAGCCTTGAGGTGTAGCTGTTGAGCCCTCAGCAGCTGGGGAGAGCTGGCGGAT GCTGCCCTCCCCCCAGTTTCCTAATGGTGTT | 972 |
| CIITA_chr16:10971740-10971840 | GTTTAAAAAGGGTCAGGGGACGGGGGAACAGATGGTGGGAAGAGCACAGTGCAGACACCTGGCACCGGC TCTGAAGGCAGCATGGCAGCTACACCGTTGG | 973 |
| CIITA_chr16:10971840-10971940 | CTGGGAAGGGTGTGCCCCTGAAGAAGTCGTTTACATTCTCGAGTCAATTTTCCTGGAGTGTACAATGGAC CTGTGGGAAAGCCTGTATGAAAGGGTAATG | 974 |
| CIITA_chr16:10971940-10972040 | ATGAGGGACCTAGCACAGTGTCCAATATTTTATAGGAACTGGAATTGAGCTCATAGGAGCTCAATTTTAT TGGCATTGCTGTTGTTGGATGGTTAAAGGG | 975 |
| CIITA_chr16:10972040-10972140 | GTGGTATCCCTTTTCTCAGACTCCCCTGAAATGTATGGTTTGCTTTGAACCCAGAGACTGATGACAGGTCT GCCGGTGTGGTTGGGTGCAGCCTTAAGTT | 976 |

(continued)

| Name | Sequence | SEQID NOs. |
|---|---|---|
| CIITA_chr16:10972140-10972240 | GCTACGGGAAAGTGTTGGAGGGGGAGAAGTCAGAGGTAACCTTGCCCCTCCCTCAATTCCAGATGAGGAAATTCAGGCCTGAAAAGGGAAAGTGACCAC | 977 |
| CIITA_chr16:10972240-10972340 | CTCAAAGTCTCATGCCTTGGAGGACCCAGCAGGAATCCAAGACCTGAAAAGGACCGGCAGGGCTCTTGCCACGGCTGGGGGTGTGGTCATGGTAACAC | 978 |
| CIITA_chr16:10972340-10972440 | AGGTTTTCCATCCATGGAAGGTACCTGAGGGGGATTTTCTCTTCCTCCCTCCCTAGGGCCAGCATCAGAGGAGTGAATAGCTCAGTTAGCTCATCTCAGGGGCCAT | 979 |
| CIITA_chr16:10972440-10972540 | GTGCCCTCCGGAGGTGGTTTGCCACTTTCACGGTTGGACTGAGTTGGAGAGAAACAGAGACCCACCCAGGGGTGGGGACAAGCTCCTGCAACTCAGGACT | 980 |
| CIITA_chr16:10972540-10972640 | TGCAGATCACTTGCCCAAGTGGCTCCCTAGCTCCTGCCTCCTGGCCCGGGCCTGGGACTCTCCCGAAGTGGGGCTGGCCACTGTGAGGAACCGACTGG | 981 |
| CIITA_chr16:10972640-10972740 | AGGCAGGGACCTCTTGGATGCCCCAGGCAGTTGGGATGCCACTTCTGATAAAGCACGTGGTGGCCACAGTAGGTGCTTGGTTGCTCCACAGCCTGGCCCG | 982 |
| CIITA_chr16:10972740-10972840 | AGCTCAGCGCTGCAGAAAGAAGAAAGTGAAAAGGGAAAAAAGAACTGCGGGGAGGCGGGGAGGTAGGATGACCAGCGGACGAGCTGCCACAGACTTGCCGCGGGCC | 983 |
| CIITA_chr16:10972840-10972940 | CCAGAGCTGGCGGGAGGGAGGAGAGGCCACCAGCAGCGCGGGAGCCGGGGAACAGCGGTAGGTGACCAAAGTCTCCTCTGTTAACCCCTAAGGTCGGGC | 984 |
| CIITA_chr16:10972940-10973040 | TGAGAATCGAGGCTCCGAGACTGTCAGCTACTTGCTCAAGGTCACACAGCAAGTCTGGGAGGATGGGGGGATGGAATATGCAAAATGTAGGGCCGGGAAA | 985 |
| CIITA_chr16:10973040-10973140 | CACCTCGTTTCCAGCATCCCCGCAACGACTCTGCGCGGGAACCAGGAGCCGGGACCCGGAGCTTGGCTTGCTGTGCCCAGAGCTCCGGGGCCGTGGGCG | 986 |
| CIITA_chr16:10973140-10973240 | GGTGGCAGGAAAGCCTGGCGGCAGCTGGCGGCTCTGCAGAGAAGCCGGAGCGCGGAGACTGGCGAGCGCGGGAGCAGACACACTCCCGGCCACCCTTGGCCGACTCCG | 987 |
| CIITA_chr16:10973240-10973340 | CGCGCCCCGGGATCCTGCGAGAGGTGCGCGCCCCTTCTTGTACGCCAGACTTTGGACCCAGGGGCCGCCGTTCCCTGAGCTTCACTTTCCCTGTTGGGTCATATT | 988 |
| CIITA_chr16:10973340-10973440 | CCATCTCTAACTCTGGAATCTTGGGTATTGGGCTCTCCAGGCGGGGGGGCCCTGCTCAGGGGAGGCAGTAGGGAGCCAAAACCTTTAACCAGAGGATGGGATA | 989 |

(continued)

| Name | Sequence | SEQID NOs. |
|---|---|---|
| CIITA_chr16:10973440-10973540 | AGTCCTCAACTCTCGTTGAACATCTTGGCGAAGGTGTGTTGTTGGGAGGGGTGGGGGAGGGATCCCCCCGGACTGAAACCGATCTCTTGATCTCTCACT | 990 |
| CIITA_chr16:10973540-10973640 | TCTCTACCTGCTTTGGGCGCCCTGAGTCACACCCTCTAAGGAGAGAGGCTAAAGCGCCCGGAAAGCCAGCGTGCGAATGCCGGGGGGTGGGGAGTGGGGAGAT | 991 |
| CIITA_chr16:10973640-10973740 | TGGATCTCCCTGGGGTCCAGGAAAGCCGGAATCGGAGCCACCATGCTTAGCTTAGTCTGGAACTCTTAAAAGCCGGCGGGTCCTGAGTCCCACAGCCCC | 992 |
| CIITA_chr16:10973740-10973840 | TCTCCACCCTAGGTGGCACAGGAGAGGTGGCAAAGCCTAGAAGTTCAAGGCATGGCTCCCTCCCCAGCCGCAGGCCTGGAGTGTCTAACTTTGGCAGGAA | 993 |
| CIITA_chr16:10973840-10973940 | GTCTTCCGTTTCTGCTCCCCACTCCAGAGAAAAAATAAATAAATACTTCTCCGGAGTGAGATTAAGGAAACAGGTACTTCTTCCTCTTGGAGAAAGAGGA | 994 |
| CIITA_chr16:10973885-10973985 | CTTCTCCGGAGTGAGATTAAGGAAACAGGTACTTCTTCCTCTTGGAGAAAGAGGAGCCAAAGGAACTTGACTCCAACAAATGATCACCTTGCAAACCCCC | 995 |
| CIITAchr16:10973985-10974085 | GGCTCCCTTAGGGGATGACCTGGTCTCCAACAATCTCAGAGGCGTTTGGAGGCAGGGTCTTTGGAGATGACTGAGTGGGGAATCCCAGGCTCCCCACACAT | 996 |
| - CIITA_chr16:10974085-10974185 | GAACATCACCTGGGATGATCAACCTGTTCAGGATGTAGGTTCCGGGCTCACCCCCAGGCCCGGTTGGCTAGGCCTGGGGTGAGGCTGAGATCCTGCAGG | 997 |
| CIITA_chr16:10974185-10974285 | TTAAAACCATCTATCCCAGGTGACTCCAATGTTCGTTTGTGTGGGCAAAAGTCCCTCAAGTCAGAGACACTGGGAGGCGCTGATGTGTGGTCTCATCTCTTTAC | 998 |
| SOCS1_chr16:11348520-11348620 | CAAGAGGTGAGAAGGGGGTCTGCGGCCTCGTCTCCAGCCGGAGGGGCGGGAGGCGCCTCGCCCCTACACCCATCCGCTCCCTCCAACCCAGGCCGGGGAGGGT | 999 |
| SOCS1_chr16:11348620-11348720 | ACCCACATGGTTCCAGGCAAGTAATAACAAAATAAACACGGCATCCCAGTTAATGCTGCGTGCACGGCGGGGCGCTGCCGGTCAAATCTGGAAGGGGAAGGA | 1000 |
| SOCS1_chr16:11348720-11348820 | GCTCAGGTAGTCGCGGAGGACGGGGTTGAGGGGGATGCGAGCCAGGTTCTCGCGGCCCCACGGTGCCACGATGCGCTGGCGGCCACAGCTCCTGCAGCGGC | 1001 |
| SOCS1_chr16:11348820-11348920 | CGCACGCGCGGCGCTGGCGCAGCGGGGCCCCAGCATGCGGCGGCCGGCGCCCACGTAGTGCTCCAGCAGCTCGAAGAGGCAGTGAAGCTCTCGCGGCTGC | 1002 |

| Name | Sequence | SEQ ID NOs. |
|---|---|---|
| SOCS1_chr16:11348920-1 1349020 | CATCCAGGTGAAAGCGGCCGGCCTGAAAGTGCACGCGGATGCTCGTGGGTCCCGAGGCCATCTTCACGCT AAGGGCGAAAAAGCAGTTCCGCTGGCGGCT | 1003 |
| SOCS1_chr16:11349020-11349120 | GTCGCGCACCAGGAAGGTGCCCACGGGCTCGGCGCGCAGCCGCTCGTGCGCCCCGTGCACGCTCAGGGGC CCCCAGTAGAATCCGCAGGCGTCCAGGAGC | 1004 |
| SOCS1_chr16:11349120-11349220 | GCGCTGGCGCGCGTGATGCGCCGGTAATCGGCGTGCGAACGGAATGTGCGGAAGTGCGTGTCGCCGGGG GCCGGGGCCGGGACCGCGGGGCACGGCCGCG | 1005 |
| SOCS1_chr16:11349220-11349320 | GGCGCGCGGGGGCCGCGGGCGAGGAGGAGGAAGAGGAGGAAGGTTCTGGCCGCCGTCGGGGCTCTGCTG CTGTGGAGACTGCATTGTCGGCTGCCACCTG | 1006 |
| IGHV3OR16-12_chr16:33523607-33523707 | TTTAAAATCACCCAAATCAAAATAATTTTATCTTCATTAATAAATAATCATCAGAAGTTTAACTAATTTTT ACTTTATAATACTAGGTTTAAAAATTCTT | 1007 |
| IRF8_chr16:85933003-85933103 | AATCTGAATGCCCAAGTCGTTGATTGTCGTTTGCCTGTTTCCAAAGATTGGTAGATAGATGCCTTTTTAAA AATCTCATTTTTCTTTAAATCTGGTTTAC | 1008 |
| IRF8_chr16:85933103-835933203 | ATGGAAAACGTTAGGAGAGCTCATATAATGAACGGCAATAGCAACCCCCTATCTTGAAACGCGCTCTATC ATCCCACTGAAATTCTACCACGTGGAATAA | 1009 |
| IRF8_chr16:85933203-85933303 | TGCTTGGAGGGTCAGAGTTGTGGAACTGCCCAATAACCAGTCGTTACTGAGGGTTAGTTTGTGAAGGAGG GGACAGACTGCTTCTAAAATTCTGTTTAAT | 1010 |
| IRF8_chr16:85933303-85933403 | GACAGTCAATTAAGATTTCTGAGTCTGGCTTGAGGGCCTTTGCTTCCATCACAGCCCAGTCGTCCTTGGCA AGAGAGTCTGTATATGGGCCACAGCTCAC | 1011 |
| IRF8_chr16:85933403-85933503 | AAAAGCATTGTTTGAAAAAATTTATTGAAAGAACATTGTTTGTAAAATGAGTCCCAATACATAGGACAGA CTTTCCTAAGGTGAGATGTGTTACTTACCC | 1012 |
| IRF8_chr16:85933503-85933603 | AGAGCTGTGAAAGGCTTTACGGATGGAAACTAGAGACTGAATTTTCCAGAATTTTAAGAAGTCTCCCCAA CCAATGGCCCCCCACTTTCTTTTTTTAAAC | 1013 |
| BZRAP1_chr17:56408574-56408674 | GGCGTGATCTCCGAAGCCCACAGTACACTCATCCATAAAGTAGGAAACACTACACCCTCCAGTGCTGTTA GTAGTGCTTTCTACTTTATGGGTGACTGCA | 1014 |

EP 4 334 476 B1

| Name | Sequence | SEQ ID NOs. |
|---|---|---|
| BZRAP1_chr17:56408674-56408774 | CTGTCTGTCTGTCCGTCGGCGTGTACTCTTCAGGCTGCCCAGGCCTCCTGACTCCTGCTCCAAGAGCCCCCCAGCCCTCCTTGTGGCTTCCTAAGATCCC | 1015 |
| BZRAP1_chr17:56408884-56408984 | CCCTCTTCCCTTCCCCCTAAAGGCTCCACCCCATCCCCCCAGTTTCAGAGACACTCAGGTAGAGACTAGGGCCTCTGGAGGCCTCACCTTCAGTTCTGTG | 1016 |
| BZRAP1_chr17:56408984-56409084 | AACCCCTGGCTGGCCGCTTCCAGCCACGCTAGCCACCCTCCAGCGTCCAAATGAGGCAGCCACAGCTCCCCTGCCAAGGTCTTGGTCTCCAGTCCACCCC | 1017 |
| BZRAP1_chr17:56409084-56409184 | AACCGTGAGGTCCTGACTGCCCAGAGCCTCAGTCCCCACCCTTCAGCCTCCCCACCAGCCCAAGATCCTGACCCCCCAGGGCCTAAGTCCCCAGCCTCCC | 1018 |
| BZRAP1_chr17:56409184-56409284 | CAACAGCCCAGGGTCCTGACCCCCCAGGGCCTCAGGCCCTGGCCTCCCCACCAGCCCAAGGTCTTGAACACACCAGGGCCTCAATTCCCAGCCTCCCCAC | 1019 |
| BZRAP1_chr17:56409284-56409384 | CAGCTCAAGGTCCTGACTCCCCAGAGCCTCAGTCCCAGCCTCCATAGCAGCCCAAGGTCCTGACCCCCCAGGGCCTCAGTCCCCAGCCACTCCACCAGC | 1020 |
| BZRAP1_chr17:56409384-56409484 | CCCAAAGTCCTGACTCCCCAGAGCCTTGATTCTCGGCCTCCCCACCAGCCCAAAGTCCTGACTCCCTCACTGCCCTGCTGTTCCCCTGGCAGGAGCCCAA | 1021 |
| BZRAP1_chr17:56409484-56409584 | GGCTATCCCAACAAAAATGGTGGCCATGTTGGGCGGAGGAAGAGGCTGGCGCCCCTTGAGACACTGGTCCCACTTCTCAGCCTCTGCGTACCCTCTGCCA | 1022 |
| BZRAP1_chr17:56409584-56409684 | TCCCCGCCTTACTCTCCAGCCCTCCTCCTTGGACACCTCTTTCCCCGCCTGGGGTCCCGGAGCCATTTTACCTTCCTTCACTAGAGAGGGTTTCAAGGCG | 1023 |
| GNA13_chr17:63010240-63010340 | CTAAGATTTTCAAGAAGTTAAACGTAGAATTAAGATTGTTCTAATTCTGGTTGTAAACTGCTATTTAAAAAACAAAACAAACAGAAAACATCAAAAACA | 1024 |
| GNA13_chr17:63010315-63010415 | AAACAAACAGAAAACATCAAAAACACAAAAAGATATTAAAACAGCAAGTCTTTTGTACATCACTGTAGCATAAGCTGCTTGAGGTTGTCATGCAGAATAG | 1025 |
| GNA13_chr17:63010415-63010515 | TATCCTTCACGTCACGGAAAACAAGGCGGATGTTCTCCGTGTTGATAGCAGTGGTGAAGTGGTGGTATAAGGGCTTCTGTTGCTGGTCCCGGCGTTTGTT | 1026 |

| Name | Sequence | SEQ ID NOs. |
|---|---|---|
| GNA13_chr17:63010515-63010615 | CCGGAAACATTCCACCAGGAATTTTTGGACGTCTCTTAAGCAGTGGGGATCCCCTTCAAATTCTAGGAAATAGTCTTTGATGCTCACAATTTGCACCTTC | 1027 |
| GNA13_chr17:63010615-63010715 | TCCTCAAGCAAGTCTGTCTTGTTTAAGAACAGAATTATGGAGACATTGCTGAAAACCCGGTTATTGACGATTGTTTCAAAAATGTTCAGAGACTCTGTAA | 1028 |
| GNA13_chr17:63010715-63010815 | GGCGATTGGTCAGTCGATCTTCCATAAGCACCTGGTCAAATTCACTTGAGGAAACAAGGAAAAGTATTGATGTCACACTGTCGAAACATTCAAACCAACG | 1029 |
| GNA13_chr17:63010815-63010915 | TTTCCTTTCTGATCTCTGACCACCTACATCAACCATTTTGAAAGGAACATTTTTTATTTCAAAGTCGTATTCATGGATGCCTTTGGTGGGTCTTCTGGCA | 1030 |
| GNA13_chr17:63010915-63011015 | AGCAGAATATCTTGTTGTGATGGAATATAATCCTGGAAAAGAAAAAACTTGTTTTATACCTATTAATCCCGAAGTAATGCGAATTTTTAATGGACTACTA | 1031 |
| 43717_chr17:75447868-75447968 | TGTAAATATTTGGCCAACTAAGCTGAGTGGCTAAGTTCTCCTGCTGCCCGGAGCTTCTTGGAACATGTTTCCTTTTCGCAAGGGGTTTCCCTGGCTTCCA | 1032 |
| 43717_chr17:75447968-75448068 | GGAGGGCCAGGAAGAAATTCGAATTGGCCACCGCTTTCTCTAAAATCACTCCGCTCAAGTTATCACCCCTCTGGGCTCCCGAAGACCGGCTGGCTGGAGG | 1033 |
| 43717_chr17:75448068-75448168 | CTGGAGATAGTCTCAATGCTCGAAATGCCGTAACCGAAGCTCCCCGCGGCGCCGGCACTGGGATCCAGGGAGCTGCTGCTACAGCGCAGCTCTGGATTCC | 1034 |
| 43717_chr17:75448168-75448268 | TGGATGTGTTGGATATGTGCAGGGCGTTCCTGGGAGGAGCGGGGAGGGAGGGTGCTGCTGGCGGGGCTGGTCTGCGTGTGCTTTGCTTCTCTACAATGGC | 1035 |
| 43717_chr17:75448268-75448368 | ATGCTGCGTGTCGGCCATGCAGAGGCATGTCAGTGAGCAGGGGCTGAGGGATCTCCCTAACGGACCTGCTTTCAGAGGGTCTTTTCATGCTGGGAGAACC | 1036 |
| 43717_chr17:75448368-75448468 | CCAGAGACTAAATCATGCAGCCAACGGGGTGGTCCCCGGCCTCAAAGCAGGGAGGGGCGAGGAGCTTTGTAGGCAATGCCATCTGCTCCTGAAACGCCGT | 1037 |
| ADCYAP1_chr18:1477565-1477665 | CAGCCTCCTTAGTAGCTACCGCCTTAGTAAGTACCACTTAGTAAGTACCGCCTTAGTAAGTACCACTTAGTAGCTACCTCCTTAGTAAGTACCACTTAGT | 1038 |
| ADCYAP1_chr18:1477665-1477765 | AAGTACCTCCTTAGTAAGTACCACTTAGTACTACCACCACGCCTGGCTAATTTCGTATTTTTTTTTTTTTAGTAGAGACGGGGTTTCTCCATATTGGTCA | 1039 |

| Name | Sequence | SEQ ID NOs. |
|---|---|---|
| AC012123.1_chr18:30349775-30349875 | AGGTCAGGCGCATACTGCATGCGGGTCTCGCGGTCGTGCTCCAGCCACAGCACGGACATCTGGAAGAGCG CCAGCTCCGACTCCACGGGGGGGCGGCAGCG | 1040 |
| AC012123.1_chr18:30349875-30349975 | AGTCCAGCAGGGCGCGCATCTCCTCGAAGTTGAGCAGCAGCACATCCTCCACCAGGTACTTGTTGGCCAG CTTCTTGGTCTCCTCCAGGCCGTGCAGCGC | 1041 |
| KLHL14_chr18:30349975-30350075 | GGCGATCTTGCACACCTGCTTGTAGTTCTGCACCGAGATCTGGTCGTTGAGGAACTGCACGCAGAGCTTG GTGACCTGGGGGATGTGCAGGATCTTGCTG | 1042 |
| KLHL14_chr18:30350075-30350175 | ACCGACAGCACCTCCTCCACCGTGTCCAGGGACAGGGTCACGTTGGCCGTGTAGAGGTACTCGAGCACCA GGCGCAGCCCGATGGACGAGCAGCCCTGCA | 1043 |
| KLHL14_chr18:30350175-30350275 | GCACCAGGTTGTTGATGGCCCGGGGGCTGGTCAGCAGCTTGTCGTCGGGGGAGGAAGAAGGAGTCCCGG GCTCCTCCTGCGGCGGCGGCTGCTGCTGCTG | 1044 |
| KLHL14_chr18:30350275-30350375 | TGACGGCTGCTGCTGCGGCGGCTGCTGCTGGTCCTTGGGGGCCCCCAGGCCGTCCTGGCCGCCGACCCCT CCCCCGAGAGGGGGGTGGCTGGAGAAGAGC | 1045 |
| BCL2_chr18:60806264-60806364 | GAGACTTCAGCCGGAGCTGGCTATTCCAGAGATGGACCTCAGAGGATTCCTTAGTCTAATTACCTTCTGG GCTGGGGTAGAAGATGGTGTCTGGAGGGAA | 1046 |
| BCL2_chr18:60806364-60806464 | GCACAGAACCAAGTTCCCTACTGCCGCACTAGCTATGCAAATACTGCAGGGCACCTGTGGGCTCATGTCC CTCCTGCAAGAAGGTGTGGTCAGTCCAGTA | 1047 |
| BCL2_chr18:60806464-60806564 | ATTCAAAAGACGTACTTCTGAAATAGGTGGAGAAATGCATTTATAGCAAAAAGTGCTAAAAATATGTTAA TAGTTATGCTATTTGGTTCACCAGGTTAGT | 1048 |
| BCL2_chr18:60806564-60806664 | GTAATAAACCATAACAAGAGAGACTAAAGGCCGTATCTATATGACCTTGAAATCTCATCTTCAGCGGGCT TATTCATTCAGTAACCAAACTATTTTTGTA | 1049 |
| BCL2_chr18:60806664-60806764 | AGGTGCTGAGTATTTAGCTTAAAGCTAAATAAGACACATGCCCTGCCCTATAGTAACTGCTTGGTAATATT CCCAGTGGCTTCCATGGGCCTGATAATTT | 1050 |
| BCL2_chr18:60806764-60806864 | TCTTAGTACTGAATTCAAAGCACTTTGTGTCTTGTCTGCAGGCCCATTTGCCCAGCAGTGGCCTTGCCAGG AGAGAACAGGCCCATGCTCCTGTCCTCAT | 1051 |
| BCL2_chr18:60983784-60983884 | CAAACAAACAATTCAAGAAGAGGATTTAAATTTTAGAAATTTAAATTGGGGCATTTTAGTTAATCTTACTT TTAAACACCAAACAGTGGCATCAATATTT | 1052 |

| Name | Sequence | SEQ ID NOs. |
|---|---|---|
| BCL2_chr18:60983884-00983984 | TGTCAACTTTGGTCAAATAAGATCAGATGTTCACATCAATCATCTACTTTTCTTGGCCTTTTCTCTATTTGG CCTCCTAGTATGAGCACACTTTGTAAAA | 1053 |
| BCL2_chr18:60983984-60984084 | TGTAATAAAAACATGTGGTGTGCTTCTTGACATCTAATCCACTTGCAGTAATTTCTAGGCTTTTTGCTCCT GTTAGGTCCTATAAAATAATGACATTAGT | 1054 |
| BCL2_chr18:60984454-60984554 | ATAGATACCTAGATGCAAATTTTTTTCAGCCGACCACAAAATTAGGTCCACTCTGAGTGGTGAAAAACAA AAGATTCTAACATTCTAGCAAACTGGTAAA | 1055 |
| BCL2_chr18:60984554-60984654 | CCATACACAAATTATAGAATACAAAGAATGCAGCCGATGCAAATTCTGTCACTGACAAGGTAGCAAAGCC ATAGCCTGATACTCCTCAGGACACCTCATC | 1056 |
| BCL2_chr18:60984654-60984754 | ACGCCCACTGGGAACATGGCACACACTGGAGATTCCAGTCCAAGGACTTTGGAATGTCAACTTAGCTCTT TACAAACACAACTAAGTTTTTCAGGGAAAA | 1057 |
| BCL2_chr18:60984754-60984854 | AGACTTACATTGGTTTTCCTCTTTTGGAAAATTTTACCGATTGATGATGCCCTTGGTCTTCTGTGGAGTCT ATTCTTCTAATCGGGTTGTTCTCCAATTT | 1058 |
| BCL2_chr18:60984854-60984954 | TAGTGTACAACGGGCTTGTTTCAGGGGAGCTTGTTTGGGATGCAGACTGTCAAGACCCAACCTGGTATCT GGTTCATAAGCAGTCCCTGAAACCTCCCTC | 1059 |
| BCL2_chr18:60984954-60985054 | CGGTTCCAACAAGCTGCTCAAGCCAGGAAACGGTGGTCCTGGGGACTCCTGGACCTTCAGCTTGAGAAAC ACTGAAGGGGTACCATTTACCACCACATCC | 1060 |
| BCL2_chr18:60985054-00985154 | TACTGGATTACAAACGCTAGATCTTTGGATCTCCACGACTAGCAAGCAAGTTAAAGACTTTTAGATGGCA GGCGTTATCGGTCAGGTTGGGAGTGAACGC | 1061 |
| BCL2_chr18:60985154-60985254 | TTTGTCCAGAGGAGGAGGTAGGGACGCCGGGAAGCAACAACTCTGATTTTATTTCGCCGGCTCCACAGCC TCCCATTGCCCCAGGAGCCCACCCGCACTC | 1062 |
| BCL2_chr18:60985254-60985354 | CAACCCCCGCATCTCGGACCTGTGGCCTCAGCCCAGACTCACATCACCAAGTGCACCTACCCAGCCTCCGT TATCCTGGATCCAGGTGTGCAGGTGCCGG | 1063 |
| BCL2_chr18:60985354-60985454 | TTCAGGTACTCAGTCATCCACAGGGCGATGTTGTCCACCAGGGGCGACATCTCCCGGTTGACGCTCTCCAC ACACATGACCCCACCGAACTCAAAGAAGG | 1064 |
| BCL2_chr18:60985454-60985554 | CCACAATCCTCCCCCAGTTCACCCCGTCCCTGAAGAGCTCCTCCACCACCGTGGCAAAGCGTCCCCGCGCG GTGAAGGGCGTCAGGTGCAGCTGGCTGGA | 1065 |

| Name | Sequence | SEQ ID NOs. |
|---|---|---|
| BCL2_chr18:60985554-60985654 | CATCTCGGCGAAGTCGCGGCGGTAGCGGCGGGAGAAGTCGTCGCCGGCCTGGCGGAGGGTCAGGTGGAC CACAGGTGGCACCGGGCTGAGCGCAGGCCCC | 1066 |
| BCL2_chr18:60985654-60985754 | GCGGCGGCGCCGGGGGCAGCCGGGGTCTGCAGCGGCGAGGTCCTGGCGACCGGGTCCCGGGATGCGGCT GGATGGGGCGTGTGCCCGGGCTGGGAGGAGA | 1067 |
| BCL2_chr18:60985754-60985854 | AGATGCCCGGTGCGGGGGCGGCCCCCGGGGGGCGCGGCGCCCACATCTCCCGCATCCCACTCGTAGCCCCT CTGCGACAGCTTATAATGGATGTACTTCAT | 1068 |
| BCL2_chr18:60985854-60985954 | CACTATCTCCCGGTTATCGTACCCTGTTCTCCCAGCGTGCGCCATCCTTCCCAGAGGAAAAGCAACGGGG GCCAACGGCACCTCTCGCCCCAGCTCCCAC | 1069 |
| BCL2_chr18:60985954-60986054 | CCCACGGCCCCCAGAGAAAGAAGAGGAGTTATAATCCAGCTATTTTATTGGATGTGCTTTGCATTCTTGG ACGAGGGGGTGTCTTCAATCACGCGGAACA | 1070 |
| BCL2_chr18:60986054-60986154 | CTTGATTCTGGTGTTTCCCCCTTGGCATGAGATGCAGGAAATTTTTATTCCAATTCCTTTCGGATCTTTATT TCATGAGGCACGTTATTATTAGTAAGTA | 1071 |
| BCL2_chr18:60986154-00986254 | TTGTTAATATCAGTCTACTTCCTCTGTGATGCTGAAAGGTTAAAGAAAAAACAAACTAATAAGTAAAAAA TCAGGTGCGTTTCCCTGTACACACTGAGTG | 1072 |
| BCL2_chr18:60986254-60986354 | AAAGCAGGGCATACACACTACAAGTAACACGGCTAAAAAGAATGTATTAAGCTGCCTGGAAATTAAATTT ACTCGAATGCACTTTAAGTAAAAAATCTCA | 1073 |
| BCL2_chr18:60986354-60986454 | AAGGTTTCCATTGAAAGTTACATTAAACCAATTTCCTGTGCAGAGAACTTACTTGTATTTTTTAAGTACAG CATGATCCTCTGTCAAGTTTCCTTTTTGT | 1074 |
| BCL2_chr18:60986454-60986554 | AAAACCAAAACAAATGCATAAGGCAACGATCCCATCAATCTTCAGCACTCTCCAGTTATAGCTGATTTGA AACTTCCCAATGAATCAGGAGTCGCGGGGA | 1075 |
| BCL2_chr18:60986554-60986654 | GAGGGAGTAAAAATTAGGAGGATTTCCAGATCGATTCCCAGACTTCTGCTTCACAGAAATGTCAATCCGC AGGAATCCCAACCGGAGATCTCAAGAGCTC | 1076 |
| BCL2_chr18:60986654-60986754 | GAGAAAAAAAAAAGGCAGCGGCGGCGGCAGATGAATTACAATTTTCAGTCCGGTATTCGCAGAAGTCCT GTGATGTTTTCCCCTTCTCGGCAATTTACAC | 1077 |
| BCL2_chr18:60986844-60986944 | TGAAGGAGCCGGGGACGGAGGCAGGAATCCTCTTCTGATTAAACTCCGAACAGCAAATGCATTTTCCGAA AAGCTGCTGGATAAATGAAGGCAGGACGCG | 1078 |

| Name | Sequence | SEQ ID NOs. |
|---|---|---|
| BCL2_chr18:60986944-60987044 | CCTGGCCCGCCGGTGCCGAGCGCTAGAAGCCCGCGCTGTGTGTGGTGCGGCGAGGGGTGGGGAGAAGGAGGTGGTGGGGGAGGGTTTTATTTTTTCCCTC | 1079 |
| BCL2_chr18:60987044-60987144 | TTTTCCTAAAAAGGATGACTGCTACGAAGTTCTCCCCCCTGGACCCCCTCTTCCGCTGCACCCCACCGGCGCACCCCGCCTCCGGGCTGCGCACCCTTTC | 1080 |
| BCL2_chr18:60987964-60988064 | GTGTGTGTCTCGCCTGGACCTTTTCTAGCCGTGTATGTGGGAGTGTGTGTGTCGCCTGGACCCTTTCTAGCCGTGTATGAGAGTGTGTACACGCGCCTAC | 1081 |
| BCL2_chr18:60988064-60988164 | ACACACACGTTGTGTTACCGGCGCTCGGCCGCCGGGGGAAGACCCAGGCCAATGCCGCCCCCCACCGCCCCCAGCAGTGGGACCTCAGCGCTGCCCTG | 1082 |
| BCL2_chr18:6098816440988264 | CTGTGAAGACAGGTGACTCTGCACGTTTTAAGCAATGTCTAGGGACGCCCCGAGCGTGGTGTTTACTTTCAAGTAGCTTCCTAGGTGTCCGCGCACTACA | 1083 |
| BCL2_chr18:60988264-60988364 | CACGCACGCGCATCCCCGCCCGTGTCCACCTGAACACCTAGTCCGTGGCCCAGGCCATGCAGAACTCAGCGCTCCAGGGAAGGGGTTTATCAAGGGCTTT | 1084 |
| BCL2_chr18:60988364-60988464 | ACGACAGTTTAAGTCAATGTTTTCCCTCTGTCCCTAACACCTTTTACACTGGTTTAGTGCTACACGATGAGGACTTCCATATAGTAACTTTCAGGCCCAC | 1085 |
| BCL2_chr18:60988464-60988564 | CGTCCTAACGCTGGGGTGGGTGGGCTCCTAAAGGTCTCCACCTTTGCCTCGTAGCCAATCCTAGTTGGCCGCACTTTCTCAAATGAGGTACATAGATACA | 1086 |
| S1PR2_chr19:10340823-10340923 | GTGTCTCCATGGAGATGGCAGCAGGACCCGACCCCGTGCTGGCCCGCACTCTCGGCCTCCTTATCTGGTTTAGGAATGCGCGGTATCCACGCTCGCTCGC | 1087 |
| S1PR2_chr19:10340923-10341023 | GCGGGAGCCACGCCTCCTCTCCCCCCCGCCCCCGAGACCGCCACACGCGCGGGGCCCCCACGTCTCCAAGCGGCACTGGAAGGATTCCTCTCCGTCCCGC | 1088 |
| S1PR2_chr19:10341023-10341123 | CAGGGGTCCCGCCTCGAGATTCTGGGAAGACTGGGGGTGGGGGACCAGATCGCAGCAGCAGCTGCACCGCGAGTTCCGCGCCTGGCCGTGTCGCCCCACG | 1089 |
| S1PR2_chr19:10341123-10341223 | AGGGGGACTGTGGGCTCAGCGCGTGGGGCCCGGAGCATCTGACAAGGACAGAGACAGAGGAGGGGGTGGAAATCCCCGGGTGAGTCAACCCGTGCCTGAG | 1090 |
| S1PR2_chr19:10341223-10341323 | AAGGGGGCGAGTTCCGACGCTCCGCCCGGCTCGGGGCCCACGCGAGGTCCGCGCCACGCGCGCCTTCACCCCACGACCCATCCCTGAGCCGGAGTTGAAAGA | 1091 |

EP 4 334 476 B1

293

| Name | Sequence | SEQ ID NOs. |
|---|---|---|
| S1PR2_chr19:10341323-10341423 | GGAGGCGTCTGAGCCACGCAGTCACTTTCTCTTTCCTTACAAAACAAAGCCACGCCCCCCGCCGGGGGAC CGGAGGAGGCAAACAACTTGGGGAAACCGA | 1092 |
| NCOA3_chr20:46131072-46131172 | CCCACTTTCCCCTTCTGTCCCTAAAGTTTTTTCTTCCTCTTGCCTCCCCCAGCCCTTTTGAAAGCTCCCCGC GTCGTCCTCCTGCTGCCCCGGCTCCTTA | 1093 |
| NCOA3_chr20:46131172-46131272 | GCAGCTTCTGGGACGCACGGGAGGGAAAAGCCGCGGGGACCCCCCCCCACCCCAGCCTCCCAGCCGGGTG AGATTTGGTTGCTGTGTTTCCTCCTCACTTG | 1094 |
| NCOA3_chr20:46131217-46131317 | CCACCCCAGCCTCCCAGCCGGGTGAGATTTGGTTGCTGTGTTTCCTCCTCACTTGGGCATTTAAAAAATAT TTTAACACGAATTGTCCGCGGAATTTTCA | 1095 |
| IGLV4-69_chr22:22380472-22380572 | CATGGCCTGGACCCCTCTCCTCCTCCAGCTTCTCACCCTCTGCTCAGGTGACTGCCTGTGGAATGCCAAAG TGATTATTGGGGACACATGGGATGACTTT | 1096 |
| IGLV4-69_chr22:22380572-22380672 | TCTCTTATATTTTAACATTGTGGGGTGGGTAGTGAACCCAGACTCACCTCTCTGTGCCTGCCTCCTCTGTT CCAGGGTCCTGGGCACAGTCTGCGCTGAC | 1097 |
| IGLV4-69_chr22:22380672-22380772 | CCAGGAAGCCTCGGTGTCAGGGACCGTGGGACAGAAGGTCACCCTCTCCTGTACTGGAAACAGCAACAAC GTTGGAAGTTATGCTGTGGGCTGGTACCAA | 1098 |
| IGLV4-69_chr22:22380772-22380872 | CAGATTTCTCACGGTGCTCCCAAAACTGTGATGTTTGGAAATTCTCTGCCCTCAGGGATCCCTGACCGCTT CTCTGGCTCAAAGTCTGGGACCACAGCCT | 1099 |
| IGLV4-69_chr22:22380872-22380972 | CCCTGACTATCTCGGGCCTCTAGCCTGAGGACGAGGCTGATTATTACTGTTCAACATGGGACTACAGCCTC AGTGCTCACACAGTGCTGCAGGCACATGG | 1100 |
| IGLV4-69_chr22:22380972-22381072 | GGAACCGAGACAAAAACCTGCCCTTGGCCTGTCCCGAGGCTGATCACTCCATACTTGCCTATGACAAACA AAGAGGGTGCCTGTGGCTGATCGTACAGTT | 1101 |
| IGLV4-60_chr22:22516707-22516807 | GAAATGTTGTTTGCTCTTGTCCTTCCTTCAGGCCATAATGAGCGTCTCTGTTTTCAGGGTCTCTCTCCCAGC CTGTGCTGACTCAATCATCCTCTGCCTC | 1102 |
| IGLV4-60_chr22:22516827-22516927 | TCAAGCTCACCTGCACTCTGAGCAGTGGGCACAGTAGCTACATCATCGCATGGCATCAGCAGCAGCCAGG GAAGGCCCCTCGGTACTTGATGAAGCTTGA | 1103 |
| IGLV4-60_chr22:22516927-22517027 | AGGTAGTGGAAGCTACAACAAGGGGAGCGGAGTTCCTGATCGCTTCTCAGGCTCCAGCTCTGGGGCTGAC CGCTACCTCACCATCTCCAACCTCCAGTTT | 1104 |

EP 4 334 476 B1

| Name | Sequence | SEQ ID NOs. |
|---|---|---|
| IGLV4-60_chr22:22517027-22517127 | GAGGATGAGGCTGATTATTACTGTGAGACCTGGGACAGTAACACTCACACAGTGATACAGGCAGATGAGGAAGTGGGACAAAATCCTCAACCTGCTGAGG | 1105 |
| IGLV1-51_chr22:22677077-22677177 | AAGGTCACCATCTCCTGCTCTGGAAGCAGCTCCAACATTGGGAATAATTATGTATCCTGGTACCAGCAGCTCCCAGGAACAGCCCCCAAACTCCTCATTT | 1106 |
| IGLV1-51_chr22:22677177-22677277 | ATGACAATAATAAGCGACCCTCAGGGATTCCTGACCGATTCTCTGGCTCCAAGTCTGGCACGTCAGCCACCCTGGGCATCACCGGACTCCAGACTGGGGA | 1107 |
| IGLV5-48 chr22:22707517-22707617 | TCAGCCAGACTCACCTGCACCTTGCGCAGTGGCATCAATCTTGGTAGCTACAGGATATTCTGGTACCAGCAGAAGCCAGAGAGCCCTCCCCGGTATCTCC | 1108 |
| IGLV5-48_chr22:22707617-22707717 | TGAGCTACTACTCAGACTCAAGTAAGCATCAGGGCTCTGGAGTCCCCAGCCGCTTCTCTGGATCCAAAGATGCTTCGAGCAATGCAGGGATTTTAGTCAT | 1109 |
| IGLV1-47_chr22:22712077-22712177 | AGAGATCTGGGGGAAGCTCAGCTTCAGCTGTGGTAGAGAAGACAGGATTCAGGACAATCTCCAGCATGGCCGGCTTCCCTCTCCTCCTCACCCTCCTCAC | 1110 |
| IGLV1-47_chr22:22712177-22712277 | TCACTGTGCAGGTGACAGGATGGGGACCAAGAGAGGGGCCCTGGGAAGCCCATGGGGCCCTGCTTTCTCCTCTTGTCTCCTTTCGTCTCTTGTCAATCAC | 1111 |
| IGLV1-47_chr22:22712277-22712377 | CATGTCTGTGTCTCTCTCACTTCCAGGGTCCTGGGCCCAGTCTGTGCTGACTCAGCCACCCTCAGCGTCTGGGACCCCCGGGCAGAGGGTCACCATCTCT | 1112 |
| IGLV1-47_chr22:22712377-22712477 | TGTTCTGGAAGCAGCTCCAACATCGGAAGTAATTATGTATACTGGTACCAGCAGCTCCCAGGAACGGCCCCCAAACTCCTCATCTATAGTAATAATCAGC | 1113 |
| IGLV1-47_chr22:22712477-22712577 | GGCCCTCAGGGGTCCCTGACCGATTCTCTGGCTCCAAGTCTGGCACCTCAGCCTCCCTGGCCATCAGTGGGCTCCGGTCCGAGGATGAGGCTGATTATTA | 1114 |
| IGLV7-46_chr22:22723897-22723997 | ATTTGCATAAAGCAGCACACAGCACACCCCCTCCGTGCGGAGAGCTCAATAGGAGATAAAGAGCCATCAGAATCCAGCCCCAGCTCTGGCACCAGGGGTC | 1115 |
| IGLV7-46_chr22:22723997-22724097 | CCTTCCAATATCAGCACCATGGCCTGGACTCCTCTCTTTCTGTTCCTCCTCACTTGCTGCCCAGGTTAAGAGAGATTTCAAATACCAGCCTTTGGAGGGA | 1116 |
| IGLV7-46_chr22:22724097-22724197 | TCCCTTTTTCTCCCTTTCTAATTCCTAATATATGTCTGTTTTTTTTTGTTTCAGGGTCCAATTCCCAGGCTGTGGTGACTCAGGAGCCCTCACTGACTGTG | 1117 |

EP 4 334 476 B1

| Name | Sequence | SEQ ID NOs. |
|---|---|---|
| IGLV7-46 chr22:22724207-22724307 | GGACAGTCACTCTCACCTGTGGCTCCAGCACTGGAGCTGTCACCAGTGGTCATTATCCCTACTGGTTCCAG CAGAAGCCTGGCCAAGCCCCCAGGACACT | 1118 |
| IGLV7-46_chr22:22724307-22724407 | GATTTATGATACAAGCAACAAACACTCCTGGACACCTGCCCGGTTCTCAGGCTCCCTCCTTGGGGGCAAA GCTGCCCTGACCCTTTTGGGTGCGCAGCCT | 1119 |
| IGLV7-46_chr22:22724407-22724507 | GAGGATGAGGCTGAGTATTACTGCTTGCTCTCCTATAGTGGTGCTCGGCACAGTGACAGACCCATGAGAG GAACCAAGACATAAACCTCCCTCGGCCCTT | 1120 |
| IGLV5-45_chr22:22730452-22730552 | GGTCAGCCACCCAGCCTGATTCTGACTCTTCTGGCAAAGATCCCTGAAAAACTTTACCCTGGTTTCTGCCT TAGCACCCATTAATGTCTGTGTTTCCAGG | 1121 |
| IGLV5-45_chr22:22730552-22730652 | TTCCCTCTCGCAGGCTGTGCTGACTCAGCCGTCTTCCCTCTCTGCATCTCCTGGAGCATCAGCCAGTCTCA CCTGCACCTTGCGCAGTGGCATCAATGTT | 1122 |
| IGLV5-45 chr22:22730607-22730707 | GCATCAGCCAGTCTCACCTGCACCTTGCGCAGTGGCATCAATGTTGGTACCTACAGGATATACTGGTACC AGCAGAAGCCAGGGAGTCCTCCCCAGTATC | 1123 |
| IGLV35-45_chr22:22730707-22730807 | TCCTGAGGTACAAATCAGACTCAGATAAGCAGCAGGGCTCTGGAGTCCCCAGCCGCTTCTCTGGATCCAA AGATGCTTCGGCCAATGCAGGGATTTTACT | 1124 |
| IGLV545_chr22:22730887-22730987 | ACAGATGGGGAAGTGGGACAAAAACCTCACCCTGCTCTGGGTCTTGCTCTGTACCAATTTTTAAATTTTAA AATAACTGGCCTAGGCACAAACTATATTT | 1125 |
| IGLV1-44_chr22:22735417-22735517 | GCCCAGTCTGTGCTGACTCAGCCACCCTCAGCGTCTGGGACCCCCGGGCAGAGGGTCACCATCTCTTGTTC TGGAAGCAGCTCCAACATCGGAAGTAATA | 1126 |
| IGLV1-44_chr22:22735517-22735617 | CTGTAAACTGGTACCAGCAGCTCCCAGGAACGGCCCCCAAACTCCTCATCTATAGTAATAATCAGCGGCC CTCAGGGGTCCCTGACCGATTCTCTGGCTC | 1127 |
| IGLV1-44_chr22:22735792-22735892 | TGCTGCTCAGGCCTGGCCTGTGGCTTCTGCTGCTGCAGCTTCCTTCATGGGTCCAGGGGCATCCAGGGCCC TGCCTGAGAGTGGAGGCTCCTCCTCCCCT | 1128 |
| IGLV7-43_chr22:22749602-22749702 | TCCAGCACTGGAGCAGTCACCAGTGGTTACTATCCAAACTGGTTCCAGCAGAAACCTGGACAAGCACCCA GGGCACTGATTTATAGTACAAGCAACAAAC | 1129 |
| IGLV7-43_chr22:22749732-22749832 | CCCTCCTTGGGGGCAAAGCTGCCCTGACACTGTCAGGTGTGCAGCCTGAGGACGAGGCTGAGTATTACTG CCTGCTCTACTATGGTGGTGCTCAGCACAG | 1130 |

EP 4 334 476 B1

| Name | Sequence | SEQ ID NOs. |
|---|---|---|
| IGLV7-43_chr22:22749832-22749932 | TGACAGACTCATAAGAGGAACCAAGACATAAACCTCCCTCGGCCCTTGTGATGTGGAGATTGTGTGATCA TACACACCAGCTCTCAAGACAGCCTACATG | 1131 |
| IGLV7-43_chr22:22749857-22749957 | ACATAAACCTCCCTCGGCCCTTGTGATGTGGAGATTGTGTGATCATACACACCAGCTCTCAAGACAGCCTA CATGTGGACCAGCCATAGAAAGGGGAAGG | 1132 |
| IGLV7-43_chr22:22749942-22750042 | ATAGAAAGGGGAAGGAAAGGGTCTGAATTGATTTCTATCCCTCCTTGTGCCCTGAAGTGGAGGAAATGTG AGAGTGATTTGCAGTAATTGAATGAGACAA | 1133 |
| IGLV7-43_chr22:22750042-22750142 | AGCAAAAGTTATTTGTTTTATATGAAAAAAAAAAACAGAAACAGCAGGATCAGATCTAAAGGCTGAGTCT AAATGCATTTCCTCCAGACAGAAGCTTCTT | 1134 |
| IGLV7-43_chr22:22750092-22750192 | CAGATCTAAAGGCTGAGTCTAAATGCATTTCCTCCAGACAGAAGCTTCTTCAAACGATGGGCTTTCTGAG CTAAGAGCAAAGAAAATAAACTCTCCACGG | 1135 |
| IGLV7-43_chr22:22750192-22750292 | GTATATTATTAAAGTTTATTTTATTGAGTTACTTTCAAAGCAATCCATGACTATTATATAAAGTCAGAAAG TATTAAAAATCACCAAGTTCTCTGCTAAG | 1136 |
| IGLV7-43_chr22:22750292-22750392 | CTACCTTATCCCATGCAATCAAAATAAGTACTTTTCTTCATTTGGATGCATTTTTTATTTCTGTTTTTAATA TTTCCACAATGGTGATTAAACCTGGTGC | 1137 |
| IGLV1-40_chr22:22758647-22758747 | ACAGGGTCAGGGGAGGGGTCCAGGAAGCCCATGAGGCCCTGCTTTCTCCTTCTCTCTCTAGACCAAGAAT CACCGTGTCTGTGTCTCTCCTGCTTCCACG | 1138 |
| IGLV1-40_chr22:22758747-22758847 | GTCCTGGGCCCAGTCTGTGTTGACGCAGCCGCCTTCAGTGTCTGCGGCCCCAGGACAGAAGGTCACCATC TCCTGCTCTGGAAGCAGCTCCGACATGGGG | 1139 |
| IGLV1-40_chr22:22758847-22758947 | AATTATGCGGTATCCTGGTACCAGCAGCTCCCAGGAACAGCCCCCAAACTCCTCATCTATGAAAATAATA AGCGACCCTCAGGGATTCCTGACCGATTCT | 1140 |
| IGLV1-40_chr22:22758947-22759047 | CTGGCTCCAAGTCTGGCACCTCAGCCACCCTGGGCATCACTGGCCTCTGGCCTGAGGACTAGGCCGATTA TTACTGCTTAGCATGGGATACCAGCCTGAG | 1141 |
| IGLV1-40_chr22:22759047-22759147 | AGCTTGCACAGTGCTCCAGGCCAATGGGGAACTGAGACAAGAACCCTCTTCCTCCTCCGCCAGGAGGGTG AGTGCCTGCAGCTGCTGCTCACACCTGACC | 1142 |
| IGLV1-40_chr22:22759147-22759247 | TGTAGCTTCTGCTGCTGTAGCTTCCCCCATGGGCCTCGGGGCATCCAGGGCCTTGCCTAGGAGTGGAGGC TCCACCACTTTTGTCCTCAGAGTCAGGAAC | 1143 |

EP 4 334 476 B1

| Name | Sequence | SEQ ID NOs. |
|---|---|---|
| IGLV1-40_chr22:22759247-22759347 | AGGGACCCCAGGAGACAGAATATCCTGCTCCTCAGCTTGGGACACAGGGTCTCTGCACTGAAATCGTGGG CTGAGGTGGCAGGTCCAACTGTGTCTTCAC | 1144 |
| IGLV1-40_chr22:22759297-22759397 | CTCTGCACTGAAATCGTGGGCTGAGGTGGCAGGTCCAACTGTGTCTTCACAGTCCTTCCTGTGCCTGCCCA TGGTGTGGGGACGGAGTGAGGAAGTGTGG | 1145 |
| IGLV1-40_chr22:22764167-22764267 | TCCTCACTCTCCTCGCTCACTGCACAGGTGACTGGATACAGGTCCAGGGGAGGGGCCCTGGGAAGCCTAT GGATTCTTGCTTTCTCCTGTTGTCTCTAGA | 1146 |
| IGLV1-40_chr22:22764267-22764367 | AGCCGAATAATGATGCCTGTGTCTCTCCCACTTCCAGGGTCCTGGGCCCAGTCTGTGCTGACGCAGCCGCC CTCAGTGTCTGGGGCCCCAGGGCAGAGGG | 1147 |
| IGLV1-40_chr22:22764367-22764467 | TCACCATCTCCTGCACTGGGAGCAGCTCCAACATCGGGGCAGGTTATGATGTACACTGGTACCAGCAGCT TCCAGGAACAGCCCCCAAACTCCTCATCTA | 1148 |
| IGLV1-40_chr22:22764552-22764652 | CTCCAGGCTGAGGATGAGGCTGATTATTACTGCCAGTCCTATGACAGCAGCCTGAGTGGTTCCACAGTGC TCCAGGCCCGGGGGGGAACTGAGACAAGAAC | 1149 |
| IGLV2-23_chr22:23040452-23040552 | GCTCCTCACTCTCCTCACTCAGGACACAGGTGACGCCTCCAGGGAAGGGGTCTTGGGGACCTCTGGGCTG ATCCTTGGTCTCCTGCTCCTCAGGCTCACC | 1150 |
| IGLV2-23_chr22:23040592-23040692 | TTCCAGGGTCCTGGGCCCAGTCTGCCCTGACTCAGCCTGCCTCCGTGTCTGGGTCTCCTGGACAGTCGATC ACCATCTCCTGCACTGGAACCAGCAGTGA | 1151 |
| IGLV2-23_chr22:23040692-23040792 | TGTTGGGAGTTATAACCTTGTCTCCTGGTACCAACAGCACCCAGGCAAAGCCCCCAAACTCATGATTTATG AGGGCAGTAAGCGGCCCTCAGGGGTTTCT | 1152 |
| IGLV2-23_chr22:23040792-23040892 | AATCGCTTCTCTGGCTCCAAGTCTGGCAACACGGCCTCCCTGACAATCTCTGGGCTCCAGGCTGAGGACG AGGCTGATTATTACTGCTGCTCATATGCAG | 1153 |
| IGLV2-23_chr22:23040852-23040952 | GCTGAGGACGAGGCTGATTATTACTGCTGCTCATATGCAGGTAGTAGCACTTTCCACAGTGGTCCAAGTT CATGGGGAACTGAGACCAAAACCTGCCCAG | 1154 |
| IGLV2-23_chr22:23040952-23041052 | GGCCTTCAGACTTCCTCCTTGCTCTGAAGATGCTTCCTCACCCGGTGCAAGAGGCTTGCTGCAGCGCGGCC TTGAGAATTCTTCTCTCAGCTCCTTCC | 1155 |
| IGLV2-23_chr22:23041052-23041152 | CTTTCCACCATGAATTCCAACAGGAAACCTGCCCTGTGGTTTCCCATCCAGGACAGGGACAGCTTCCTGAT GCTTGTGTGCTGTGGTCCCTGAATGTGCA | 1156 |

EP 4 334 476 B1

| Name | Sequence | SEQ ID NOs. |
|---|---|---|
| IGLV2-23_chr22:23041152-23041252 | ACTCTTCCCAGCTCTTCAAATGCAGGGACAGTGACAAGGAGCTGCCTGATTGGTGCAGTCACTGCTTTTTT CAGGGATGTCTTCACCCTACATGTATCAT | 1157 |
| IGLV2-23_chr22:23041252-23041352 | CATCCCCTACACTGTGGGTAGAATTTTAGCAACTACATTCTAATGGTTATCGCCACAACTTTGATCTTAGA AATAACAGTGCAGTGAACATCCCTATGCA | 1158 |
| IGLV2-23_chr22:23041352-23041452 | GGCTCCTTTGAGTTCCTGTGTGAATACGACCATAGGATTCATTTCTAAAAGTGAAATTGCGGGTCAGAAA GATGTGTGTTTGTGATTTTCACCCAATGTT | 1159 |
| IGLV3-21_chr22:23055497-23055597 | ACCAGCAGAAGCCAGGCCAGGCCCCTGTGCTGGTCGTCTATGATGATAGCGACCGGCCCTCAGGGATCCC TGAGCGATTCTCTGGCTCCAACTCTGGGAA | 1160 |
| IGLV3-21_chr22:23055727-23055827 | CCCAGCCTCGGTCACCCTCTTGCTCCAGCCCCGGGAAGCCTGTTGATAAAGCCATGAGTGAATCTGGCCC AGTTCACCTGGATCTGAGCCTTTCAGGTTG | 1161 |
| IGLV3-21_chr22:23055827-23055927 | CCCTTCCCTCCAGCCCCCTCCAGGAGTCTCTACAGAAGATACATCAGGCATAAATATGGCCTGGAAGGGC CAGAATCATCTGGTGACTTGGGGCTGTTGT | 1162 |
| IGLV2-14_chr22:23101392-23101492 | GGTCCTGGGCCCAGTCTGCCCTGACTCAGCCTGCCTCCGTGTCTGGGTCTCCTGGACAGTCGATCACCATC TCCTGCACTGGAACCAGCAGTGACGTTGG | 1163 |
| IGLV2-14_chr22:23101532-23101632 | AAAGCCCCCAAACTCATGATTTATGAGGTCAGTAATCGGCCCTCAGGGGTTTCTAATCGCTTCTCTGGCTC CAAGTCTGGCAACACGGCCTCCCTGACCA | 1164 |
| IGLV3-10_chr22:23154347-23154447 | AGGCTCAGTGCCCATAGACCCCAAGTTGGCCCTGCCCTGAACCCTGTGCAAAGCCCAGACACAGTCTTAG GGTAGGACCCCTGGGAATGGGCTCTTGATC | 1165 |
| IGLV3-10_chr22:23154447-23154547 | TTCAAGCCCCCTCTCCTGTTTTCCTTGCAGTCTCTGAGGCCTCCTATGAGCTGACACAGCCACCCTCGGTG TCAGTGTCCCCAGGACAAACGGCCAGGAT | 1166 |
| IGLV3-10 chr22:23154597-23154697 | AGAAGTCAGGCCAGGCCCCTGTGCTGGTCATCTATGAGGACAGCAAACGACCCTCCGGGATCCCTGAGAG ATTCTCTGGCTCCAGCTCAGGGACAATGGC | 1167 |
| IGLV3-10_chr22:23154697-23154797 | CACCTTGACTATCAGTGGGGCCCAGGTGGAGGATGAAGCTGACTACTACTGTTACTCAACAGACAGCAGT GGTAATCATAGCACAGTGACACTGGCAGAT | 1168 |
| IGLV3-10_chr22:23154797-23154897 | GGGGAAGTGAGACACAAACCCCTTCTTCATCTATTTTACCCTCTCCCTCCAGCCCCAGGACCGCTGTGGAC CAACCCATAAGCAGGTCTGGCAGAATTCA | 1169 |

EP 4 334 476 B1

| Name | Sequence | SEQ ID NOs. |
|---|---|---|
| IGLV2-8_chr22:23165422-23165522 | AGGCTCACCTGGGCCCAGCACTGACTCACTAGACTGTGTTTCTCCCTTTCCAGGGTCCTGGGCCCAGTCTG CCCTGACTCAGCCTCCCTCCGCGTCCGGG | 1170 |
| IGLV2-8_chr22:23165542-23165642 | CATCTCCTGCACTGGAACCAGCAGTGACGTTGGTGGTTATAACTATGTCTCCTGGTACCAACAGCACCCA GGCAAAGCCCCCAAACTCATGATTTATGAG | 1171 |
| IGLV2-8_chr22:23165642-23165742 | GTCAGTAAGCGGCCCTCAGGGGTCCCTGATCGCTTCTCTGGCTCCAAGTCTGGCAACACGGCCTCCCTGAC CGTCTCTGGGCTCCAGGCTGAGGATGAGG | 1172 |
| IGLV2-8_chr22:23165727-23165827 | AGGCTGAGGATGAGGCTGATTATTACTGCAGCTCATATGCAGGCAGCAACAATTTCCACAGTGTTTTAAG TCAATGAGGAAGTAAGATCAAAACCTGCCC | 1173 |
| IGLV4-3_chr22:23192412-23192512 | TCAGGCTCAGAACCCATAGGATCCTGAGCTGGGCCTGCCCAAACATGAGTTCATCCCAGGCACAACCTCA GGGTGGGACCCCCTGGGAACAGATTCATCA | 1174 |
| IGLV4-3 chr22:23192512-23192612 | TTTACAAGCCTCCTCTCCTGTCCTCTCTTGCAAGCTCCTATGAGCTTACACAGCCACCCTCAGTGTCAGTG TCACCAGGACAGGCAGCCATGATCACCTG | 1175 |
| IGLV4-3_chr22:23192612-23192712 | CTCTTGAGATAACCTCAAAGATGAGTATGTTTACTGGTTCTGGCAGAAGCCAGACCAGGCCCATACTGGT GATATATGAAGGCAGCAAGCGGCCCTCAGG | 1176 |
| IGLV4-3_chr22:23192712-23192812 | AATTTCTGATTTTCTGAGTCCAGCTCAGGGAACATGGCCACCCTGACCATCAGCAGGGCTCAGACTGAGG ACGAGGCTGACTATTACTGTCACAGGTACA | 1177 |
| IGLV4-3_chr22:23192812-23192912 | ATAGAAACAGTGATGAGCCCACAGTGACACAGGCAGATTAGGAAGTGAGACACAAACCCCTTCCCAATCT GTGTCACCCTCTTTCTCCAGCCCCAGGATG | 1178 |
| IGLV4-3_chr22:23197917-23198017 | GGGATGAGAAGGGACCAGGGGCCTGGGATTGAGCTGTGAAGGGAACCAAAAGGCAGGAGGGACAGGGC AGGGGCTGTCAGCTATGACTCAGGGGAGGTTC | 1179 |
| IGLV4-3_chr22:23198017-23198117 | CTGGGCCTCAGGATCCTCCCTCTGAGGCCACCAGGGGGCGGGGGTGGCACATGCCTGGACCTGGGAGGTC CCTGCTGGGCTTCACCCTGGGTGGGTCCTA | 1180 |
| IGLV4-3_chr22:23198067-23198167 | ATGCCTGGACCTGGGAGGTCCCTGCTGGGCTTCACCCTGGGTGGGTCCTAGGAGCTCCTTCCTCCTAAGTC CCCCTAAAGAGACAGAGGCATTCTGGGGT | 1181 |
| IGLV4-3_chr22:23198167-23198267 | CCTAAATCTGTCATGCCCCCATAAATGCATTTCTACGAGGGCCAATAAATGAACTCCAGGTTTATCCAAGC AGCAGCTTCAGGCGTCTGCAGACACAGAG | 1182 |

EP 4 334 476 B1

| Name | Sequence | SEQ ID NOs. |
|------|----------|-------------|
| IGLV4-3_chr22:23198267-23198367 | CGGGGAGGAATTAGCCAACCTGAGGCACCCTAGAAGGGGCTGAAGGGGGCTGAAGGGGACTGAAGGGTCC CTGTGGGGCCTGTGGTCCTGGGGAGGGGAGA | 1183 |
| IGLV4-3_chr22:23198367-23198467 | GCTGGGGTGTCTCCCAGCCACTCTGGGCCCTGTCCTGACACTTCTCCCACAAAGAAGGGAAGGGAAATCC TGGGACCCCACAGCCAGGACCAACCGTGAA | 1184 |
| IGLV4-3_chr22:23198467-23198567 | CCACAGGACAGGAAGGACAGGGACCCCCAAGGCTGGCTCCATTTCCCAGGCACTGTCATGGGCTGAGTCT CAGGAAATCCAAGTCAAGGAGTTTCAATCC | 1185 |
| IGLV4-3 chr22:23198587-23198687 | CCAAGGAAACAGAAGTCTACGGGCCCAGGCCCAGGTGAGGGTGGGGTAAGAAGAGGAGCTTAGGATGCA GATTTGCATGGAGGCCCCGCCCTCCTCTGAG | 1186 |
| IGLV4-3_chr22:23198687-23198787 | GCATCAGGGTAAGACAAGGCTGGGGGCAGGCCCAGTGCTGGGGTCTCAGGAGGCAGCGCTCTGGGGACG TCTCCACCATGGCCTGGGCTCTGCTCCTCCT | 1187 |
| IGLV4-3_chr22:23198797-23198897 | CTCAGGGCACAGGTGACGCCTCCAGGGAAGGGGCCTCGGGGACCCTTGGGCTGATCCTTGGTCTCCTGCT CCTCAGGCTCACCTGGGCCCAGCACTGACT | 1188 |
| IGLV4-3_chr22:23199022-23199122 | TTGGGAGTTATGACTATGTCTCCTGGTACCAACAGCACCCAGGCACAGTCCCCAAACCCATGATCTACAA TGTCAATACTCAGCCCTCAGGGGTCCCTGA | 1189 |
| IGLV4-3_chr22:23199122-23199222 | TCGTTTCTCTGGCTCCAAGTCTGGCAATACGGCCTCCATGACCATCTCTGGACTCCAGGCTGAGGACGAG GCTGATTATTAGTGCTGCTCATATACAAGC | 1190 |
| IGLV4-3_chr22:23199182-23199282 | TGAGGACGAGGCTGATTATTAGTGCTGCTCATATACAAGCAGTGCCACTTAACCACAGTGGTCCAAGTTC TTGGGGAACTGAGACGAAAACCTGCCCTGG | 1191 |
| IGLV4-3_chr22:23199277-23199377 | CCTGGGCTCTCAGGCTCCCTTTTTGCTCTGAAGATGTTTCCTCACCCAGTGCAACGGGCTTCCTGAAGCAC AGCCTTGAGAATTCTTCTCCCTCAGCAAC | 1192 |
| IGLV4-3_chr22:23199377-23199477 | TCTCTTTTCCCACCATGAAATCCAAAGGAAACCTGCTCTGTGGTTTCTCATCCAGGACAGGGACAGCTTCC TTTTGCTTGTGTGTTGTGGTCCCTGAGTG | 1193 |
| IGLV4-3_chr22:23199477-23199577 | GGTGCAACTCTTCCTAGCTTTTTAAATTATGGGAGGGTGACAATGAGCTCCCTGACTGGTGCAGTCCCTGC TGTTTTCAGGAACATCCTCATCCTAAATG | 1194 |
| IGLV4-3_chr22:23199577-23199677 | CATCTGAATCTCCCACTGTGTGCAGACCAATCTGGACAGATGTTATTAGGGGGAGTTTCCAGAAGCCACA TCTTACTCAACTCTGTATCCACCACACTCT | 1195 |

EP 4 334 476 B1

| Name | Sequence | SEQ ID NOs. |
|---|---|---|
| IGLV3-1_chr22:23222927-23223027 | TGCCTCAGCCATGGCATGGATCCCTCTCTTCCTCGGCGTCCTTGCTTACTGCACAGGTGCTGCCCCTAGGGTCCTAGCCACTGGTCCAGTCCCAGGGCTC | 1196 |
| IGLV3-1_chr22:23223027-23223127 | TGGGTCCAGCCTGGCCCTGACTCTGAGCTCAGCAGGGCCCCCGCCTGTGGTGGGCAGGATGCTCATGACCCTGCTGCAGGTGGATGGGCTCGGCGGGGCT | 1197 |
| IGLV3-1_chr22:23223077-23223177 | TGGGCAGGATGCTCATGACCCTGCTGCAGGTGGATGGGCTCGGCGGGGCTGAAATCCCCCCACACAGTGCTCATGTGCTCACACTGCCTTAGGGCTCTTT | 1198 |
| IGLV3-1_chr22:23223177-23223277 | CATCCCTGGATCTGTGTCCAGGCCAGGCACGTGGGAAGATTTACTTGGAGTTCAGCTCCTCAGTTTCAAGCCTTTTCTCTCCCGTTTTCTCTCCTGTAGG | 1199 |
| IGLV3-1_chr22:23223277-23223377 | ATCCGTGGCCTCCTATGAGCTGACTCAGCCACCCTCAGTGTCCGTGTCCCCAGGACAGACAGCCAGCATCACCTGCTCTGGAGATAAATTGGGGGATAAA | 1200 |
| IGLV3-1_chr22:23223327-23223427 | CAGGACAGACAGCCAGCATCACCTGCTCTGGAGATAAATTGGGGGATAAATATGCTTGCTGGTATCAGCAGAAGCCAGGCCAGTCCCCTGTGCTGGTCAT | 1201 |
| IGLV3-1_chr22:23223427-23223527 | CTATCAAGATAGCAAGCGGCCCTCAGGGATCCCTGAGCGATTCTCTGGCTCCAACTCTGGGAACACAGCCACTCTGACCATCAGCGGGACCCAGGCTATG | 1202 |
| IGLV3-1_chr22:23223527-23223627 | GATGAGGCTGACTATTACTGTCAGGCGTGGGACAGCAGCACTGCACACAGTGACACAGGCAGATGCGGAAGTGAGACAGAAACCAGCCACCTCGGCCTGG | 1203 |
| IGLV3-1_chr22:23223627-23223727 | CTCACAAGACCCTTCCCTCTCTCCTGCCCTGTCACACTGAGCAGGAGGGAGCCTTCCATGTGGAATGGAAGTTTCCAGTCCTATCCCTGCCCTTATGTTC | 1204 |
| IGLV3-1_chr22:23223727-23223827 | CTGAGAGACGGGAGCAAGTTCCTGCCCACCTCTAGGCTCAGCTTATCCCAGAATAAACTGAGCTAGTCATTTTGATGATCAAATGCCAGCTCCCAAAAGA | 1205 |
| IGLV3-1_chr22:23223827-23223927 | CCCCAGAAACCCTGATATCTAAGTAGCACCGACTCTATTAGTATCAAGGGAGACTAGCCCTAGGGTGGAATCATTTTAGTGTCTCAGAAGGCACAGGGCA | 1206 |
| IGLV3-1_chr22:23223927-23224027 | ATGGAAAGTGTTTATGAGGTTTCAGGATATGCACGTGAGCAGTTAAAGGCAGGTCTTACAAGGAAGGAACCTACTAGAATTGGGGCCCATCTGTGACATC | 1207 |
| IGLL5_chr22:23227062-23227162 | ACATCCCTCTGCTTTGGGAGAGAAGGGCCAGGGCGGGACCCAGAGAGCTCTGCAGAGGCACCACAGACCCTCAGCAGGGGGTCTGCCAAACAGGACAGCT | 1208 |

EP 4 334 476 B1

302

EP 4 334 476 B1

(continued)

| Name | Sequence | SEQID NOs. |
|---|---|---|
| IGLL5_chr22:23227162-23227262 | GGACTTGGCTGCTTCTGCCCAGGCCTGGATCCAGCCCTTGCACATCTCAGGGCAGGGGATAGGCCTGGGTGGCCAGAGCTGCAGCTGCACCTGCTGGGGA | 1209 |
| IGLL5_chr22:23227262-23227362 | GGCCTAGTCCAGTCCTCCAGGGTCCCAGACACAGACTCGGATTCCGACTGCAGCCACCATGGAAGGATGTGGTCTGCGGGTGACGATGTCATCCAGAGGC | 1210 |
| IGLL5_chr22:23227567-23227667 | CCGAATATCCAAGGAGCCCAAGATCAGAGGCAGGAATAGGCCAAGCTCCCCAGTGGAGAAGCTGTGCTGGACCAGGGGTTTCCCAGGCCCTCCCTTGTG | 1211 |
| IGLL5_chr22:23227667-23227767 | CCCTGAATGATGTCTGTTAGGGCACCTACACCCTGTTACTGCCTCAGTGCCTTGCCTATTTGAAGGACAGGGATGTGTGGGGTGATTATTGTATAAATCCAG | 1212 |
| IGLL5_chr22:23227767-23227867 | CCCCCAGCACCTGGTCCTCAAAAGTTACCCAAGCAATGTGTATAAAGATCCAGCCTGGAGATCTTTGAAAACCGATTCGATGAGTCGAACCATTAAGTCA | 1213 |
| IGLL5_chr22:23227867-23227967 | TGATCACCATCCTCAACTTCATCTCTTTCTTCCTCCTCCTCCTCATTATCATCACCTTCAAGAACTGTTAAGAGTCTGAGAC | 1214 |
| IGLL5_chr22:23227897-23227997 | TCCTCCTCCTCCTCATTATCATCACCTTCAAGAACTGTTAAGAGTCTGAGACTTCATCCTATTTGCAGACTAAAAAGTAAGCCTGCCACAGTGCCATGGA | 1215 |
| IGLL5_chr22:23227997-23228097 | TGCTGGCAGAAGATACAAGACTCCTGGGTCAGAGACAACGAATAATCTGTTTTCACAGCAATAGCAGTTGCCAAGGTATCAGCATTGTCTTGCACCAGT | 1216 |
| IGLL5_chr22:23228097-23228197 | TCCACAAGGTGATGCAAAGAGGGCCAGGTGACATCTGCATGCCAGAGCTCAGGGATCCCAAATATTTCATACTTGACAGTAAGCATATATCTGTGTTTTG | 1217 |
| IGLL5_chr22:23228197-23228297 | CTCCAAAGAGAGAGGCATTCTTCTGTACCTTCCGAGGTTGTTCACTCCACAAACACTCTTGAAAAGATAAATCCACAATCAGTGCCTTTGCCGAGACATGC | 1218 |
| IGLL5_chr22:23228297-23228397 | AGAAATGCAGAGATCCATAGTAGACCACTGTCTCCCAACAACCATCAACTTTATCAATGAAATGAAGTCTCAGGCTATTTGTCTGTTACCCATAGCCCACA | 1219 |
| IGLL5_chr22:23228397-23228497 | AAAATGTCTGGCTTGATTGTCACCAAATGTATCAAGGAAGTTAAGGAGTATCTGACACAAAATGTGAACCAAGCAATTCTCAAAGGAGCCTCCCAGGAAA | 1220 |
| IGLL5_chr22:23228497-23228597 | TTCACTTTAGGAAGTCCTAGGAGGCTCCTCTGAGAGTTGCTAAAACAAAACAAACATTGAGAGTCCTAGAGGGCTGCAGATCTGAACTTGAGCAGATATTTTTA | 1221 |

303

| Name | Sequence | SEQ ID NOs. |
|---|---|---|
| IGLL5_chr22:23228597-23228697 | AAGATTTTGTGGCAGAAAAAGAAACTGGAAAGCAAGAGGGCAGACCCTCATTGCAGTTCTGTAATGTAAGGGGGCAGAGCAGGGGCCTTTCTCACCAGAG | 1222 |
| IGLL5_chr22:23229332-23229432 | GATATTGGACCCTGCATTCATCTTCTCTGGATGGTAATTTTCTCACCTGTAAAACAGAGACACTGGCCCCAAGGACACCCCACAAGTAGTTGTGAATCCC | 1223 |
| IGLL5_chr22:23229432-23229532 | AAAGTAAGAGAAGAACAAAAAAAGAACCAGAATTTATTCAACACCCACTGAGTGCTTAGCAAACACATGGTTTCTTTAACTCTCATAAGCTTCATGCTGC | 1224 |
| IGLL5_chr22:23229532 -23229632 | AGAGGAACTCTCCCCATTTTACAGATAAGGAAACTGAGGCCCAGAGGTAACCTAGGTCTAGATAGACTCCACATTTATGACTTCACCACTCTTCCTTGCC | 1225 |
| IGLL5_chr22:23229562-23229662 | AAACTGAGGCCCAGAGGTAACCTAGGTCTAGATAGACTCCACATTTATGACTTCACCACTCTTCCTTGCCTGAAGGATATAGAATCACTCCCTGCAGGGC | 1226 |
| IGLL5_chr22:23229662-23229762 | TCTTGCCTGACTCAGGAAAGGGCCACAGGATAGCCAGCCAGGCTTAACCAACCCAGCCAAGAAAGGGCTGGTCCCAACTGGCTGGAGTGCAGTGTACAGG | 1227 |
| IGLL5_chr22:23230012-23230112 | GTTGGTAGATGCCCCTCTGGGAGAGATCCCCAGGGGTGACAGCCATGGACCCTGGAAGGGCCTGGGCTAGGGACAGGGACCAGAGCCAGTCCAGGGAGAG | 1228 |
| IGLL5_chr22:23230112-23230212 | GACAGAGCCAATGGACTGGGGTGTACTGTAACAGCCCTGCTGGCGAGAGGGACCAGGGCACCGTCCTCCAGGGAGCCCATGCTGCAAGTCGGGCCAGAGG | 1229 |
| IGLL5_chr22:23230212-23230312 | TGCCCCTGAACCTGAAGGCCAATGAGACCCAAGACAGGCCAAGTGGGTTGTGAGACCCCTGAGGAGCTGGGCCCTGGTCCCAGGCAGCGCTGGCCCCTGC | 1230 |
| IGLL5_chr22:23230312-23230412 | TGCTGCTGGGTCTGGCCATGGTCGCCCATGGCCTGCTGCGCCCAATGGTTGCACCGCAAAGCGGGGACCCAGACCCTGGAGCCTCAGTTGGAAGCAGCCG | 1231 |
| IGLL5_chr22:23230412-23230512 | ATCCAGCCTGCGGAGCCTGTGGGGCAGGTAAGGGGCAAGAGATTCCAGGGGATGTGGGGGTCCTGCAGCAGAGCTGGGAAAGGGTGACCAAGGGGAGACA | 1232 |
| IGLL5_chr22:23230512-23230612 | AGCCAGAGGAGTGAGGAGGAAGGTTAACCCCTAAGAGGGGCCTGGGCTGACACTGGCTTTAGTAATGGGTTGATATTTTGTCCATCACAGATTTGTTTGA | 1233 |
| IGLL5_chr22:23230612-23230712 | ATTACTGTTTTTAATATCATATTACGATATTATTTTTCTTGATTTCTGAGTTTTCTGGCGCCACTTAAATTTTCACCAGGGTCAGTGCCTCAATCACCTA | 1234 |

| Name | Sequence | SEQ ID NOs. |
|---|---|---|
| IGLL5_chr22:23230712-23230812 | GTCCTAGTCCTCTGGGTAGGGAAGGAACAGAGGCAGGGACAGGACATCCACAGGGGGTGGTGGCCACTG TCCCCACAGGGTGCCCAGGCCTGTTCCTCCC | 1235 |
| IGLL5_chr22:23230812-23230912 | CCTCCTCCTCTCTGCCCATGTGCCTCCTGCCCAGTGAGGGCAGGGGCCACTCCCTGGAGAAGGCAGCAAG GGCTTGGTTTGGTCTCCCCCAAGGCTGTCT | 1236 |
| IGLL5_chr22:23230912-23231012 | GTTCACCAACTTGCACATAAATGCTTACTGGGGCCAGGCTCAAGGACACAGGGAGGGTGGGATGAACCG AGGGGAGCTGTCCAGTCATTGGAACAGGCCC | 1237 |
| IGLL5_chr22:23231012-23231112 | ACGGCCCATGTTTGGAGCAATAAAGGGAGAGGGGATCTCCCTCTGGGATGATGCCCAGGCTGGTCTCACA GATCGAGGGGCACTGGCTGGTGATGGGTGC | 1238 |
| IGLL5_chr22:23231072-23231172 | TGGTCTCACAGATCGAGGGGCACTGGCTGGTGATGGGTGCCCCCAAAAGACAGAGCAGCGTCAGAGGAG AGGAGAGCACAGGATGAGGCTGGGAGCTCCT | 1239 |
| IGLL5_chr22:23231172-23231272 | GGGTGACTGGGAAGGGGAGGCAAGAAGACCATAGGGTCCGTGCACCATTCCCAGTCCAGGACGAGTCCT TGGATGGATTTAGGTAGATTGATTATCAGAG | 1240 |
| IGLL5_chr22:23231272-23231372 | TCAGATTTGTGTTTTTGGAAAAATCAGCACCGGATTGGAGGCTGATGCGACGCCCGATTAGAGGAGGGAG GAGAGGGGGTGATGGCCAAGTCCAGGGTAG | 1241 |
| IGLL5_chr22:23231372-23231472 | GTGGGGATCCTGGAGGAAGCCGTGCCTTGGGGATGGGGAGGACACTCAGATTCAGAGCACCCAGGGGCC CAGTTTCCTATGAAATGGGAGCATGAAGTTG | 1242 |
| IGLL5_chr22:23231472-23231572 | AAGTGAGGGCTGAGCAGAGGGGAGCAGACACGCTCGGGGACTGTCTATGGGCATTAAAAATGTATAACC ATTTTAGCAACAGGCGGCGAGTCAAAAAACA | 1243 |
| IGLL5_chr22:23231572-23231672 | AAGTGTGTTTATCTAAACTGGGCAATTCCACTTCTAGGAATTTATCCTAAGGGTTGGTTGGGGGAATAATC AAAGCTGTAACCAAATCTTTATAACAAGG | 1244 |
| IGLL5_chr22:23231672-23231772 | GTGGTTAGCTCAGCATTATTAGTGATGGGAGAAAACTGGAAAAAATCCAAATATCTACCAGAAAGGGTGT GAAAAAACACAATTGTATTTGGGGGACTGT | 1245 |
| IGLL5_chr22:23231927-23232027 | TGGCTAATTTTGATTAGGATTATTATTAGTTTAGAGACAGAGCCTCGCTATATTGCTCAGGCCTGTCTCAA ATTCCTAAGCTCAAGCAATCTTTCTGCCT | 1246 |
| IGLL5_chr22:23232062-23232162 | ACTGCACCTGACCCAACTGTGTTTTTAAAGTATATATGCATTTTCAAAAACCTGTCAGAAAATATAGAAAA ATGTCAATGGTGTGTCTGGCTGGCTGATG | 1247 |

305

EP 4 334 476 B1

| Name | Sequence | SEQ ID NOs. |
|---|---|---|
| IGLL5_chr22:23232162-23232262 | GGATTTCACCTAATTTTAATGTGGCTTTATAATTTTCTGGTTTTGTGAAGTTGTTCACAAAAAGAGACATT TCTTCTAATATAATTTTTAATACAACAGT | 1248 |
| IGLL5_chr22:23232262-23232362 | AATGTACTCATGTGCATTACTCTTTTTGTAATGAGTATATTACAAAATGTAATGACTTTTGTACATTACTCT TTTTTCTTGCCAAAAAAAAAAAAAGATTA | 1249 |
| IGLL5_chr22:23232362-23232462 | AGCAGAGAAGTATATAAAGTAAAAGCAAGTGCTTCTGCTTACCATCTCTCACCTCTTCCCAGAGATAGCC ACTGTCAGGTTGGTCAATATACTTCCAGAA | 1250 |
| IGLL5_chr22:23232462-23232562 | CTTTTCCTGTGTGTGTGTGTGTCCCTGAAAACACACACACACACACACACACACACACAGTTGGTGC TGGGATTTTATTTTGCAAAAGTAAGAGCC | 1251 |
| IGLL5_chr22:23232517-23232617 | CACACACAGTTGGTGCTGGGATTTTATTTTGCAAAAGTAAGAGCCATATTCTGCATATTACCAACTTTTAA TCTATTATTGACACTTCTGTATCAGTCC | 1252 |
| IGLL5_chr22:23232617-23232717 | ATATGGATTAACCACATTCATTGCTTATAAACTTTGTTTTATAAGCAAAGTTTAGATGAGCCAGAATTTAT TTCCACTAAAAAATCTAAATGACAAATGA | 1253 |
| IGLL5_chr22:23232717-23232817 | TGCTGCAGTGGAAATTTGTGTGTGTGTGTGTGTGTGTGTGTGTGTGTGTGTGTGTGTGTGTGTGTATGTGTAC AAAGTGCACTTATATATCTCCCCAGGATA | 1254 |
| IGLJ1_chr22:23234612-23234712 | TGACCTGGGTGTTTTTCTTTTTCTCTGTAGGATGTTAATAGTATCTTGTGTCATGCTAGGATGTCTAGGAC AGAGGGCAATACAATGAGGGGAAGGCATT | 1255 |
| IGLJ1_chr22:23234712-23234812 | CTGCGATGTCCCCAGGCCTCTGGCTTGAAGAGTAACTTGCTGAAGTGAGGACTCTGTGGAGGAGCAAGTT ATACAGAAGAAGTTTAGTTGTGATCTGTT | 1256 |
| IGLJ1_chr22:23234812-23234912 | GAGTTGGAGGTGTCTACAGGGCATCCAAGCAGACATAGGTTGAGGAGGCAGAATATATGTGAATCTGGA GCCAAGAAGAGAGGTAAGGGCTGGAAATAGG | 1257 |
| IGLJ1_chr22:23234912-23235012 | GATCTAAGACCCCTGGACAGTTGTGAGTGTGCACAATGAGGGTCAGATGCAGAGAAAATTAGGAGACTA CAGAGAGCAGAACCCAGGGTGGGGATCTGGG | 1258 |
| IGLJ1_chr22:23235012-23235112 | AGTCAGCAGTTGGGCATGGGCCTGGTAGAAAGGGAAGCCAAGGAGGAGGAGAGGGGGCAGTCTCGAC ACCAAGGAGGGGAGAGTGACTAGAAAGAAAC | 1259 |
| IGLJ1_chr22:23235112-23235212 | CTTCTTGCAGAGACATAGGGGATGGGGAAGAACTGCAGACTGAACTGGGGCAAAGGACTGTTGGCCTTA ACCAGAGAGATTTGAGGGAGAGATGAGGCTG | 1260 |

(continued)

| Name | Sequence | SEQ ID NOs. |
|---|---|---|
| IGLJ1_chr22:23235212-23235312 | AGAGCCAGGGGATCCTGCCATGTCCCAGCATAAAAACAGTACCTGACACAGATGGGTGCTTGGGAGCTGTTGTCGGATGAATGAGTGGACAGATGCATGG | 1261 |
| IGLJ1 chr22:23235312-23235412 | ATGGACGGATGGATGGAAGGATGATAGATTGATGGACAAACAGATGAACAGATGAATAGCTGGATGGACAACTGGATGGATGGGTAGACAGAATGATCTC | 1262 |
| IGLJ1_chr22:23235412-23235512 | AGAGATCAGAAAAAGCTTCATGCACTAAGTGGGACTGAACCGCGTCTCCATGGGTAGAAAGCAGAGGAATCTCCACTTGAGTCAGGAATGACCCAGTGCT | 1263 |
| IGLJ1_chr22:23235512-23235612 | CTCAATCCAGGGAGAAAGCCAGCCTGGCTTCACTGGGGACACTTGTGTGGGGGACTCAGAGGCCCTTTAAATGAGGCCAGACGAGGTTGGACAGGTCCAA | 1264 |
| IGLJ1_chr22:23235612-23235712 | GCCAACTCAGCACTCCTCTGCCACACTGCACAGGAGGGGATGTGTCACTCAGGGAGTTGCTGGGACCTATGGGTCCCAGTGTTGTCATCAGCACCGACAG | 1265 |
| IGLJ1_chr22:23235712-23235812 | CCTCAGAGAGGAAAGACACACTGGGGTAACTCCAAGGCTGTGTGTGGCACTTGCCTTGGACAGCAGACAGGCACAGGGACACCTCTAGGGGGCTGGCC | 1266 |
| IGLJ1_chr22:23235812-23235912 | ACCCCCCTGCCTCATGTCTAGGTCCCAGCCCCGCCCACTGCAACCCTGTGCCCGTCATGCCCAGCAGGCTCCTGCTCCAGCCCAGCCCCCAGAGAGCAGA | 1267 |
| IGLJ1_chr22:23235847-23235947 | CACTGCAACCCTGTGCCCGTCATGCCCAGCAGGCTCCTGCTCCAGCCCAGCCCCCAGAGAGCAGACCCCAGGTGCTGGCCCCGGGGGTTTTGGTCTGAGC | 1268 |
| IGLJ1_chr22:23235947-23236047 | CTCAGTCACTGTGTTATGTCTTCGGAACTGGGACCAAGGTCACCGTCCTAGGTAAGTGGCTCTCAACCTTTCCCAGCCTGTCTCACCCTCTGCTGTCCCT | 1269 |
| IGLJ1_chr22:23236047-23236147 | GGAAAATCTGTTTTCTCTCTCTGGGGCTTCCTCCCCTCTGTCCTCCCAGCCTTAAGCACTGACCCTTACCTTTCTCCATGGGGCCTGGAGGAGGTGCATT | 1270 |
| IGLJ1_chr22:23236147-23236247 | AGTCTCCGGGTAACCGGCAGGAAGGGCCTCCACAGTGGGAGCAGCCGGATGCAGCCTGGTCCCGGGGCCTGAGCTGGGATTGGGCAGGGTCAGGGCTCCT | 1271 |
| IGLJ1_chr22:23236247-23236347 | CCTCTCTTCCAGGGCAGATGTCTGAGTGAGGGACAGAGGCTGGTTCTGATGAGGGGCCCTGCAGTGTCCTTAGGGACATTGCCCAGTGACTCCTGGGGTC | 1272 |
| IGLJ1_chr22:23236277-23236377 | GGACAGAGGCTGGTTCTGATGAGGGGCCCTGCAGTGTCCTTAGGGACATTGCCCAGTGACTCCTGGGGTCAAGGACAGAGGCTGCTGGGGTGGGCCTGGG | 1273 |

| Name | Sequence | SEQ ID NOs. |
|---|---|---|
| IGLJ1_chr22:23236377-23236477 | AGCTGCTGAGTCTCATAGTCTAGGGGAGCAGCCCCAAGAACAGCTGAGGGTCTAGGCTGAGGACTGGAT GCCAATCCAGCCTGGGAGGGCCACACGGCCT | 1274 |
| IGLJ1_chr22:23236387-23236487 | TCTCATAGTCTAGGGGAGCAGCCCCAAGAACAGCTGAGGGTCTAGGCTGAGGACTGGATGCCAATCCAGC CTGGGAGGGCCACACGGCCTGGTGACACAG | 1275 |
| IGLJ1_chr22:23236487-23236587 | AGGTCACCCCAAGGGGAGACCAATGGAGGGCACAGAGAGGGCTCTGGGTCTAGGCTGCAGCTCTGTGGC CTGTGCTGGGTCATGAGGACATGGGGACACA | 1276 |
| IGLJ1_chr22:23236557-23236657 | TGTGCTGGGTCATGAGGACATGGGGACACAGAGGGACGGGTGAGACTGGGTGAGGTGCCAGAATCCAAC CCTCCCAGGACAGTCACCAGAAAGGAGACAG | 1277 |
| IGLJ1_chr22:23236657-23236757 | TCTCTTAGGGCAGAGATGTGTCTGTCCCTGGAGCCCCGTCACCTCTGGGGCCCAGTGTCTCTCTGTTCACG GATCGGCCTCCTGCCTTCCTCAAAGGGCA | 1278 |
| IGLC1_chr22:23236757-23236857 | TGTTAGACTCAGGAAATGACCAGAGGGGAGTGAATGAGGGGTGCAGAGAACTCCATGGCTACCAGGTGA AGTTTGGGGTCATCACAGGCTGCTGGGGTGG | 1279 |
| IGLC1_chr22:23236877-23236977 | CATAGTCTGTGGGAGCAGCCCCAGGAACAGCTGAGGTGAAGGGTTCTGTGGTCGGGCTTGTGGAGACAG GAAACATCTCAGAGCCTCAGAGGAGCCCTGA | 1280 |
| IGLC1_chr22:23236977-23237077 | GGCTTGTCTAGGTGGAGCCCACTCCTTGCCAGGAGAGCCAAGTGGGCTGGGCTGGGGCAGAGCCCGGTGC CTGTGAGGGATAGGAAGCTCCAGTTCAAAG | 1281 |
| IGLC1_chr22:23237077-23237177 | CAGGCTTGGGTCTCCCCACACACTGCCTGCCAGGACAGTCCTACAGGATGAGCAGGGGACCCACAGTTCA CGGAGGAGGCTCTAGGTCCTGGAAGAATAA | 1282 |
| IGLC1_chr22:23237177-23237277 | AGTGGGTGATGGAGGGGGGTATAGGGATGGAAATGAGGGATCCAGGGGTCAAGGCCAGATTCTAAACTC AGACTCCAGAGATCAGAGAAGAAGGAACACA | 1283 |
| IGLC1_chr22:23237277-23237377 | GCCTGCCCTGGGTATATGGAGAAATTGAGGCTGTAGAGGAGAGGGGCTGGGCCAGGACACCTGTGAAAG GTGACTTGGGAGGGCTCCTAGGAAGGCACAG | 1284 |
| IGLC2 _chr22:23242602-23242702 | TGAAAGCCCCACTGCTATGACCAGGTAGCCGGGACGTGGGGTGGATGCCAGAAAAGACTCCACGGAATA AGAGAGAGCCCAGGACAGCAGGCAGGCTCTC | 1285 |
| IGLC2 _chr22:23242702-23242802 | CGATCCCCCCAGGCCCTTGCCCCATACACGGGCTCCAGAACACACATTTGGCTGGAACAGCCTGAGGGAC CAAAAGGCCCCAGTATCCCACAGAGCTGAG | 1286 |

EP 4 334 476 B1

| Name | Sequence | SEQ ID NOs. |
|------|----------|-------------|
| IGLC2 _chr22:23242802-23242902 | GAGCCAGGCCAGAAAAGTAACCCCAGAGTTCGCTGTGCAGGGGAGACACAGAGCTCTCTTTATCTGTCAGGATGGCAGGAGGGGACAGGGTCAGGGCGCT | 1287 |
| IGLC2_chr22:23242902-23243002 | GAGGGTCAGATGTCGGTGTTGGGGGCCAAGGCCCCGAGAGATCTCAGGACAGGTGGTCAGGTGTCTAAGGTAAAACAGCTCCCCGTGCAGATCAGGGCAT | 1288 |
| IGLC2 chr22:23244157-23244257 | ATGCAGGACAGTCCGGAGAGGGAAATCAGGAGAAGTGAAGGGGTCTCTGGGGAGCCCAGATGTGGGCTAGAGGCAGAAGTAAGGGTGAAGAGCACCTATG | 1289 |
| IGLC2_chr22:23244257-23244357 | AGTCAATGTCATGGTCTCAGCAGGAACACAGTTGAAAATCCCCATTCCACACAAGACCGTTTAGCAGGAAAGGAGTCCATACTTGTGCTGCCACCAGGAT | 1290 |
| IGLC2_chr22:23244357-23244457 | GTCCTGAGAAGCCTTGGAGAATGAAACATACAGGTGCATTTCCTAGACTTGACAATGCACGTTAGCCAAGTAAAGGCAATGAAAAGTTCTCTACTAGGGA | 1291 |
| IGLJ3_chr22:23247257-23247357 | TTTGTTTGTTTCTGTATCTTGTCTCAACTTGTGGTCAGCCTTTCTCCCTGCATCCCAGGCCTGAGCAAGGACCTCTGCCCTCCCTGTTCAGACCCTTGCT | 1292 |
| IGLJ3_chr22:23247357-23247457 | TGCCTCAGCAGGTCACTACAACCACTTCACCTCTGACCGCAGGGGCAGGGGACTAGATAGAATGACCTACTGAGCCTCGTCTGTCTGTCTGTCTGTCTGT | 1293 |
| IGLJ3_chr22:23247467-23247567 | CTGTTTGTCTCTCTGTCTGTCTGACAGGCGCAGGCTGGGTCTCTAAGCCTTGTTCTGTTCTGGCCTCCTCAGTCTGGGTTCTTGTCGGAACAGCTTTGCC | 1294 |
| IGLJ3_chr22:23247567-23247667 | CTTGGGTTACCTGGGTTCCATCTCCTGGGGAATTGGGAACAAGGGGTCTGAGGGAGGCACCTCCTGGGAGACTTTAGAAGGACCCAGTGCCCTCGGGGCT | 1295 |
| IGLC3_chr22:23248182-23248282 | AGAGTTCGCTGTGCAGGGGAGACACAGAGCTCTCTTTATCTGTCAGGATGGCAGGAGGGGACAGGGTCAGGGCGCTGAGGGTCAGATGTCGGTGTTGGGG | 1296 |
| IGLC3 _chr22:23248282-23248382 | GCCAAGGCCCCGAGAGATCTCAGGACAGGTGGTCAGGTGTCTAAGGTAAAACAGCTCCCCGTGCAGATCAGGACATAGTGGAAAACACCCTGACCCCTCT | 1297 |
| IGLC3 _chr22:23248382-23248482 | GCCTGGCATAGACCTTCAGACACAGAGCCCCTGAACAAGGGCACCCCAACACCTCATCATATACTGAGGTCAGGGGCTCCCCAGGTGGACACCAGGACTC | 1298 |
| IGLJ7_chr22:23263872-23263972 | AGAATATTCCGTGAGAAGGTGGCCCCACAGCGCTGGGTCACACGCCATCCCCCAAGACAGGCAGGACACCACAGACAGGGTGGTGGGTCTCAGAAAACTC | 1299 |

EP 4 334 476 B1

| Name | Sequence | SEQ ID NOs. |
|---|---|---|
| IGLJ7 _chr22:23263972-23264072 | AGGCCCTAAACGTGGATGCTTACCAATTCCTCCACTGGAGGAAGACCTCAGAGCAGATGCCCAGGACAGGGACTTCTGGTAGGGACGGTGACTGGGACGG | 1300 |
| IGLJ7_chr22:23264072-23264172 | GTGCCTGTTTGTCAGGGAAAACCCACTGGAGAGTCAGATCCCCCAGATAACTTCTCACGACATGGAGACTCTTTCGAACAGACAAAGCTCCACGTTCAGC | 1301 |
| IGLC7 _chr22:23264172-23264272 | TCAGGGAGTAAAAAAAAAATGCCTCAAATGGAGGCCTTTGATCTACTGGAATCCAGCCCCCAGGACTGACACCCTGTCTCACCAGGCAGCCCAGAGGGGT | 1302 |
| IGLC7_chr22:23278157-23278257 | CAGGGTCCACCAGAAGGCATCTCAGAACCAGCCAGCAGTGGCCCTGATTGTCAGCAGGACCCCAGGGAGGGGGGTGGCCAGGACAGGGCTCTGAAGCCCC | 1303 |
| IGLC7_chr22:23278257-23278357 | CACCCCAGGACCTTCCCTGGGCAGAACGAGTTGGTGAGGGAGTGATGAGCAACCACAGGCCTCCTAACTTCCCAAGCTGGCGATTCTGAGAGGCCTCAAG | 1304 |
| IGLC7 _chr22:23278357-23278457 | GCTGAGACACGGTTCAGCCTTTTAGGCCCTCCTGAACGTGTCCCCTGTCTCCACAGCCTGGGAATGCACTCTCTTTTGACCCAGAAATCCTGCTCATAAG | 1305 |
| IGLC7_chr22:23282767-23282867 | CTGTCATTGTACAACACATCATTTCACTTTGTTTTTCAAACATAGTGAATTCTTTCCTAATTAAAGAAGAAAAGAGTATAAAGAGAAAGTTTCCAGTGCA | 1306 |
| IGLC7_chr22:23282842-23282942 | GTATAAAGAGAAAGTTTCCAGTGCAGCCTGGAGATCTGTACTGGTTGTATCTGGAATTCCAGACTCAGCCTTGCATTTCACATAGCAGATAGATGATGAT | 1307 |
| IGLC7_chr22:23282942-23283042 | GATGGAGAAGGAGAAGAAGAAGGAGGAGGAGGAGGAAAGAAGGAAGAAGAAGAAGAAGAGGAGGAGGAAGAAGAAGACGAAGGGAAGAAGAAGAAGGATG | 1308 |
| TBC1D22A_chr22:47570209-47570309 | TCCAGGTCTGCCAGGTGTAGGGGAGGTGTGACTGGTTCCATCATGGACCGGTTCCTCCATGGACCGGTTCCTCCGTGGACCGGTTCCGCCATGGACCGGT | 1309 |
| TBC1D22A_chr22:47570309-47570409 | TCCGCCATGGACCACTCCTGCCCTGGACCACTCCTGCCCTGGACCGGTTCTGCCGTGGACTGGTTCCCGCCGTGGACCAGTTCCCGCTGTATACTGGTTC | 1310 |
| TBC 1 D22A_chr22:47570409-47570509 | TGCCCTGGACTGGTTCCCGCTGTGGACTGGTTCCTTGGGGCTCTAAGTGCGGAAGGGCCCAGAGCTGGTCCCTGCCCAGCGCCCTGCTAGGGCTGTGTCC | 1311 |
| TMSB4X_chrX:12993264-12993364 | TCGTACTCGTGCGCCTCGCTTCGGTGAGCCCCAGGGCCCCTGCCTCCTTCCTCCTGCCGTCCTGCCTCCGTCCCCGCCCTTTCATCATCCGCGTCCCTGT | 1312 |

EP 4 334 476 B1

(continued)

| Name | Sequence | SEQID NOs. |
|---|---|---|
| TMSB4X_chrX:12993364-12993464 | GAAGGCATTCCCTAAATCCGAGCCCGAGTGGTTCTCCCCGGGAAGGCTACTTTGGGGAGCTGGGGGGATG CGAAACACCCTAGATACTGGATAATGGGGT | 1313 |
| TMSB4X_chrX:12993464-12993564 | GGGGAAATCGATGATTTAAGAACAACAAAACCGAAAAACTGGCGTTTTGCCGTGCCGCTCGGAGGGGACATT AAAAAATTTCTTAGTGTTTGCCCGCAAAGGT | 1314 |
| TMSB4X_chrX:12993544-12993644 | TAGTGTTTGCCCGCAAAGGTATTGTGCGTTGCCTTGGAGGCTGAGATATGGGGAATAGACAAGTCCTTT GTTCTTGAGGTTCATCTTCCGAGCCCGAGC | 1315 |
| TMSB4X_chrX:12993644-12993744 | CTCCTCCCAGCCTCGGACGGCTGCGCGGGCTGCATCTGTGCAGCCTGGCGCGGGGGCTGTGCTATGA CATCTTTACAGTCCTTCTTGCAGAGACATG | 1316 |
| TMSB4X_chrX:12993744-12993844 | TGTGCCAGGGGATGCCGAATTGCGGGAGAGCAGGCAAGACCGGCTTCGGGGCGCGGGCGGGCGCTTTG TGTGCGGGGCTGCATTGTGACGCGGGGCGATG | 1317 |
| TMSB4X_chrX:12993844-12993944 | AAGCCCGGTAGGGGCGGTGGTCGGAAGCTCCAGCCGCCGCCCTTTGTGAGAGGACTAGAAAGCCCG ATCCGGCCCGCATCCTTGCCGGAGAGGCCGCG | 1318 |
| TMSB4X_chrX:12993944-12994044 | GCTAGGAAAATGGAAACGCTTTTCCTACCTGGGCTCCCATTTTAGGAATTCTTGCCGATTTTTCCCACTTGAA TTTGGAAGTGGCTTTCCTCTTCTTTCCTT | 1319 |
| TMSB4X_chrX:12994044-12994144 | GTCCTAGCCAGCCTTTAATTTTAAACGCTGTAATTAACAATTCGCAGTGGTCAATTCCTTTATTCTGCAA GATTCGGCTTTGAGAGGCATCCGCCCTCT | 1320 |
| TMSB4X_chrX:12994144-12994244 | TTGGTCCACAGCGTTTTGAAATATGGGGAGGAGGGGCGCGGGGGTGTCGCCTCTCTTTTTCTGTAGAAAGA GGAAGCTCGTGAGCGCGGAACGGCAGCAGT | 1321 |
| TMSB4X_chrX:12994289-12994389 | AAGTGCGAGTTCCCAGCCCAGAGACAGCAGCGGGGCGGGGTGGCTCTTCCTCACGCTCGCTCTTGGCTTGCTCCCT GCAGCTTTTCCTCCGCAACCATGTCTGAC | 1322 |
| TMSB4X_chrX:12994389-12994489 | AAAACCCGATATGGCTGAGATCGAGAGAAATTCGATAAGTCGAAACTGAAGAAGACAGAGACGCAAGAGAAA AATCCACTGCCTTCCAAAGAAAGTGAGCTCC | 1323 |
| TMSB4X_chrX:12994444-12994544 | AGACGCAAGAGAGAAAAAATCCACTGCCTTCCAAAGAAAGTGAGCTCCGACCCAGCCCCCATCTTTAGAAAGGC TGGGTGGGAGCGGCCGGTGGGAGGGCGGGA | 1324 |
| DMD_chrX:33146106-33146206 | TTTATAGAAAGGCATATGGAACAGGAGTCATCCAAATATATCCCAGGGGTTGCAAATTGACCAAAAGAGT CACCTTTAGGGAAGCCTGCTTCTGAATGCT | 1325 |

| Name | Sequence | SEQ ID NOs. |
|---|---|---|
| DMD_chrX:33146206-33146306 | TGTGGAATTTATCATTCTTCTGAATGGCTGTTGCATTTATCTGCAGCTTTTACTCACCAGATGAGACCTCAGACATTTCAAATTCTGCGGAGGCTGGCTA | 1326 |
| DMD_chrX:33146306-33146406 | CACACCTTCATAGGAAAGCTTTTTGCTGATTTCCCTGTTGGTACTTTTCTCTTACACATTCTATGGGGTATGGTAAACCTGGAGGTAGAGTCATAGCCAA | 1327 |
| DMD_chrX:33146406-33146506 | GCACAGATAAAGCAGGCACAGAATCTCTGACCAGCCTCACAAAAGCAGACAAACACACAATCTTTTTGCACCTGTTTCTTCCACTCCGGTTGCCGTGAAT | 1328 |
| PABPC5_chrX:90026453-90026553 | TAGAAATGGTTCAACCAGTCCAATATCAATATAGCTGCTTATTACTCTATTCACTTACTTCAAAGTGGCATTTGTTTTGAGTAAGACTTTATTTAATTCT | 1329 |
| PABPC5_chrX:90026553-90026653 | TACCGTTAGCTTGAAACCATAGAGATCTTCTCTCTATTTGCCCTACTTCCTTCAAAAGTCAAATGACCTCCTACAAATAAAAGACGTTCTTATTTTCATT | 1330 |

312

EP 4 334 476 B1

**Claims**

1. A method for identifying sets of validated phased variants from a tumor sample of a subject, the method comprising:

   (a) obtaining, by a computer system, sequencing data from a tumor sample of a subject;
   (b) obtaining, by the computer system, sequencing data from a matched non-tumor sample of the subject;
   (c) analyzing, using the computer system, the sequencing data from the tumor sample and the sequencing data from the matched non-tumor sample to identify a plurality of regions of a genome of the subject that include a first putative phased variant and a second putative phased variant, wherein each of the plurality of regions is no more than 170 base pairs in length;
   (d) after (c), performing targeted sequencing on nucleic acids from the tumor sample of the subject that are from the plurality of regions identified in (c) to a depth of at least 250x;
   (e) identifying, using the computer system, sets of validated phased variants from the tumor sample of the subject based on sequencing data from the targeted sequencing in (d).

2. The method of claim 1, wherein the first putative phased variant and the second putative phased variant are separated by at least one nucleotide.

3. The method of claim 1 or 2, wherein (c) further comprises: determining a plurality of single nucleotide variants in the genome of the subject that are present in the tumor sample relative to the matched non-tumor sample; and from the identified plurality of single nucleotide variants, identifying the plurality of regions of the genome that include the first phased variant and the second phased variant based at least in part on one or more of (1) a proximity of two or more of the identified plurality of single nucleotide variants within a genome, and (2) a presence of two or more of the identified plurality of the single nucleotide variants on a single read from the sequence data from the tumor sample of the subject.

4. The method of claim 3, wherein the sequencing data from the tumor sample of the subject in (a) and (b) each comprises data for at least 50% of the genome of the subject.

5. The method of claim 4, wherein (c) further comprises aligning the sequencing data from the tumor sample and the sequencing data from the matched non-tumor sample to a genome.

6. The method of claim 5, wherein the sequencing data from the tumor sample and the sequencing data from the matched non-tumor sample are obtained at a depth of between 10x and 100x.

7. The method of claim 6, wherein the identified plurality of regions of the genome in (c) has at least 400 regions and no more than 15,000 regions.

8. The method of any one of claims 1 to 7, wherein identifying the plurality of regions of the genome in (c) comprises determining one or more of (i) a presence of phased variants in individual reads from the tumor sample of the subject, (ii) a presence or an absence of read support in the matched non-tumor sample, (iii) a presence of other non-reference bases on supporting reads, (iv) base quality, (v) mapping quality, and (vi) uniqueness of genomic positions.

9. The method of any one of claims 1 to 8, wherein performing the targeted sequencing of (d) comprises (1) performing hybridization capture of nucleic acids from the tumor sample of the subject that correspond to the identified plurality of regions of the genome from (c) to obtain captured nucleic acids, and (2) sequencing the captured nucleic acids from the tumor sample of the subject.

10. The method of any one of claims 1 to 9, wherein the number of sets of validated phased variants is less than 1000 and greater than 10.

11. The method of any one of claims 1 to 10, wherein the plurality of regions identified in (c) are non-overlapping regions.

12. The method of any one of claims 1 to 11, wherein the nucleic acids of (d) are DNA molecules.

13. The method of any one of claims 1 to 12, wherein the first phased variant and the second phased variant are each single nucleotide variants.

14. The method of any one of claims 1 to 13, wherein the sets of validated phased variants are found to have an allele fraction of greater than 5% and no read support in the sequencing data from the matched non-tumor sample.

15. The method of any one of claims 1 to 14, wherein (d) further comprises: after (c), performing targeted sequencing on nucleic acids from the matched non-tumor sample of the subject that are from the plurality of regions identified in (c) to a depth of at least 500x.

16. The method of any one of claims 1 to 15, wherein (d) further comprises: after (c), performing targeted sequencing on nucleic acids from the matched non-tumor sample of the subject that are from the plurality of regions identified in (c) to a depth of at least 250x.

17. The method of any one of claims 1 to 16, wherein the sets of validated phased variants have a background signal of less than one in a million.

18. The method of any one of claims 1 to 17, wherein the tumor sample is a solid tumor sample.

19. The method of any one of claims 1 to 18, wherein:

the sequencing data from the tumor sample is obtained at a depth of between 10x and 100x and comprises at least 50% of the genome of the subject;
the sequencing data from the matched non-tumor sample is obtained at a depth of between 10x and 100x and comprises at least 50% of the genome of the subject;
(c) further comprises:

aligning the sequencing data from the tumor sample and the sequencing data from the matched non-tumor sample to a genome;
determining a plurality of single nucleotide variants in the genome of the subject that are present in the tumor sample relative to the matched non-tumor sample; and
from the identified plurality of single nucleotide variants, identifying the plurality of regions of the genome that include the first phased variant and the second phased variant based at least in part on one or more of (1) a proximity of two or more of the identified plurality of single nucleotide variants within a genome, and (2) a presence of two or more of the identified plurality of the single nucleotide variants on a single read from the sequence data from the tumor sample of the subject;
wherein the plurality of regions identified in (c) are non-overlapping regions; performing the targeted sequencing of (d) comprises (1) performing hybridization capture of nucleic acids from the tumor sample of the subject that correspond to the identified plurality of regions of the genome from (c) to obtain captured nucleic acids, and (2) sequencing the captured nucleic acids from the tumor sample of the subject; and the first putative phased variant and the second putative phased variant are single nucleotide variants separated by at least one nucleotide.

20. The method of any one of claims 1 to 19, further comprising combining a set of nucleic acid probes with a plurality of cell-free nucleic acid molecules that are obtained or derived from a subject to form a mixture, wherein each nucleic acid probe of the set of nucleic acid probes is configured to hybridize to a cell-free nucleic acid molecule that comprises one or more of the identified sets of validated phased variants.

21. The method of claim 20, wherein each nucleic acid probe of the set of nucleic acid probes is configured to hybridize to a target nucleic acid molecule comprising a plurality of phased variants such that the nucleic acid probe is complementary to at least a region of the target nucleic acid molecule that extends from a first phased variant of the set of phased variants to a second phased variant of the set of phased variants.

22. The method of claim 20 or 21, wherein each nucleic acid probe of the set of nucleic acid probes comprises a biotin pull-down tag.

23. The method of any one of claims 20 to 22, further comprising:

separating target nucleic acid molecules that hybridize to at least one nucleic acid probe of the set of nucleic acid probes from nucleic acid molecules that do not hybridize to at least one nucleic acid probe of the set of nucleic acid probes, thereby capturing target nucleic acid molecules; and
sequencing the target nucleic acid molecules that hybridize to at least one of the set of nucleic acid probes.

24. The method of claim 23, further comprising identifying one or more cell-free nucleic, acid molecules as being a cancer-derived molecule with a limit of detection of less than about I out of 50,000 observations from sequencing data obtained from the sequencing of the cell-free nucleic acid molecules.

25. The method of claim 24, wherein the limit of detection of is less than 1 out of 1;000;000 observations from the sequencing data.

26. The method of any one of claims 23 to 25, wherein the sequencing does not comprise use of molecular barcodes.

27. The method of any one of claims 23 to 26, further comprising determining a condition of the subject.

28. The method of claim 27, wherein the condition is cancer.

29. The method of any one of claims 23 to 28, further comprising monitoring progression of a condition of the subject.

30. The method of claim 29, wherein the condition is cancer.

**Patentansprüche**

1. Verfahren zum Identifizieren von Sätzen validierter phasierter Varianten aus einer Tumorprobe eines Probanden, wobei das Verfahren umfasst:

   (a) Erhalten von Sequenzierungsdaten aus einer Tumorprobe eines Probanden durch ein Computersystem;
   (b) Erhalten von Sequenzierungsdaten aus einer passenden Nicht-Tumorprobe des Probanden durch das Computersystem;
   (c) Analysieren der Sequenzierungsdaten aus der Tumorprobe und der Sequenzierungsdaten aus der passenden Nicht-Tumorprobe unter Verwendung des Computersystems, um eine Vielzahl von Regionen eines Genoms des Probanden zu identifizieren, die eine erste mutmaßliche phasierte Variante und eine zweite mutmaßliche phasierte Variante umfassen, wobei jede der Vielzahl von Regionen nicht länger als 170 Basenpaare ist;
   (d) nach (c) Durchführen einer gezielten Sequenzierung von Nukleinsäuren aus der Tumorprobe des Probanden, die aus der Vielzahl von in (c) identifizierten Regionen stammen, bis zu einer Tiefe von mindestens 250x;
   (e) Identifizieren von Sätzen validierter phasierter Varianten aus der Tumorprobe des Probanden unter Verwendung des Computersystems auf der Grundlage der Sequenzierungsdaten aus der gezielten Sequenzierung in (d).

2. Verfahren nach Anspruch 1, wobei die erste mutmaßliche phasierte Variante und die zweite mutmaßliche phasierte Variante durch mindestens ein Nukleotid voneinander getrennt sind.

3. Verfahren nach Anspruch 1 oder 2, wobei (c) ferner umfasst: Bestimmen einer Vielzahl von Einzelnukleotidvarianten im Genom des Probanden, die in der Tumorprobe im Vergleich zur passenden Nicht-Tumorprobe vorhanden sind; und Identifizieren der Vielzahl von Regionen des Genoms, die die erste phasierte Variante und die zweite phasierte Variante umfassen, aus der identifizierten Vielzahl von Einzelnukleotidvarianten, zumindest teilweise basierend auf einem oder mehreren der folgenden Merkmale (1) einer Nähe von zwei oder mehr der identifizierten Vielzahl von Einzelnukleotidvarianten innerhalb eines Genoms und (2) einem Vorhandensein von zwei oder mehr der identifizierten Vielzahl von Einzelnukleotidvarianten in einem einzigen Read aus den Sequenzdaten aus der Tumorprobe des Probanden.

4. Verfahren nach Anspruch 3, wobei die Sequenzierungsdaten aus der Tumorprobe des Probanden in (a) und (b) jeweils Daten für mindestens 50 % des Genoms des Probanden umfassen.

5. Verfahren nach Anspruch 4, wobei (c) ferner das Ausrichten der Sequenzierungsdaten aus der Tumorprobe und der Sequenzierungsdaten aus der passenden Nicht-Tumorprobe an einem Genom umfasst.

6. Verfahren nach Anspruch 5, wobei die Sequenzierungsdaten aus der Tumorprobe und die Sequenzierungsdaten aus der passenden Nicht-Tumorprobe mit einer Tiefe zwischen 10x und 100x erhalten werden.

7. Verfahren nach Anspruch 6, wobei die identifizierte Vielzahl von Regionen des Genoms in (c) mindestens 400 Regionen und nicht mehr als 15.000 Regionen umfasst.

8. Verfahren nach einem der Ansprüche 1 bis 7, wobei das Identifizieren der mehreren Regionen des Genoms in (c) das Bestimmen eines oder mehrerer der folgenden Merkmale umfasst: (i) das Vorhandensein von phasierten Varianten in einzelnen Reads aus der Tumorprobe des Probanden, (ii) das Vorhandensein oder Fehlen von Read-Unterstützung in der passenden Nicht-Tumorprobe, (iii) das Vorhandensein anderer Nicht-Referenzbasen auf unterstützenden Reads, (iv) die Basenqualität, (v) die Mapping-Qualität und (vi) die Eindeutigkeit der Genompositionen umfasst.

9. Verfahren nach einem der Ansprüche 1 bis 8, wobei das Durchführen der gezielten Sequenzierung von (d) umfasst: (1) Durchführen einer Hybridisierungs-Capture von Nukleinsäuren aus der Tumorprobe des Probanden, die der identifizierten Vielzahl von Regionen des Genoms aus (c) entsprechen, um gefangene Nukleinsäuren zu erhalten, und (2) Sequenzieren der gefangenen Nukleinsäuren aus der Tumorprobe des Probanden.

10. Verfahren nach einem der Ansprüche 1 bis 9, wobei die Anzahl der Sätze validierter phasierter Varianten weniger als 1000 und mehr als 10 beträgt.

11. Verfahren nach einem der Ansprüche 1 bis 10, wobei die in (c) identifizierten mehreren Regionen nicht überlappende Regionen sind.

12. Verfahren nach einem der Ansprüche 1 bis 11, wobei die Nukleinsäuren von (d) DNA-Moleküle sind.

13. Verfahren nach einem der Ansprüche 1 bis 12, wobei die erste phasierte Variante und die zweite phasierte Variante jeweils Einzelnukleotidvarianten sind.

14. Verfahren nach einem der Ansprüche 1 bis 13, wobei festgestellt wird, dass die Sätze validierter phasierter Varianten eine Allelfraktion von mehr als 5 % und keine Read-Unterstützung in den Sequenzierungsdaten aus der passenden Nicht-Tumorprobe aufweisen.

15. Verfahren nach einem der Ansprüche 1 bis 14, wobei (d) ferner umfasst: nach (c) Durchführen einer gezielten Sequenzierung von Nukleinsäuren aus der passenden Nicht-Tumorprobe des Probanden, die aus der Vielzahl von in (c) identifizierten Regionen stammen, bis zu einer Tiefe von mindestens 500x.

16. Verfahren nach einem der Ansprüche 1 bis 15, wobei (d) ferner umfasst: nach (c) Durchführen einer gezielten Sequenzierung von Nukleinsäuren aus der passenden Nicht-Tumorprobe des Probanden, die aus der Vielzahl von in (c) identifizierten Regionen stammen, bis zu einer Tiefe von mindestens 250x.

17. Verfahren nach einem der Ansprüche 1 bis 16, wobei die Sätze validierter phasierter Varianten ein Hintergrundsignal von weniger als einem zu einer Million aufweisen.

18. Verfahren nach einem der Ansprüche 1 bis 17, wobei die Tumorprobe eine solide Tumorprobe ist.

19. Verfahren nach einem der Ansprüche 1 bis 18, wobei:

    die Sequenzierungsdaten aus der Tumorprobe mit einer Tiefe zwischen 10x und 100x erhalten werden und mindestens 50 % des Genoms des Probanden umfassen;
    die Sequenzierungsdaten aus der passenden Nicht-Tumorprobe mit einer Tiefe zwischen 10x und 100x erhalten werden und mindestens 50 % des Genoms des Probanden umfassen;
    (c) ferner umfasst:

    Ausrichten der Sequenzierungsdaten aus der Tumorprobe und der Sequenzierungsdaten aus der passenden Nicht-Tumorprobe an einem Genom;
    Bestimmen einer Vielzahl von Einzelnukleotidvarianten im Genom des Probanden, die in der Tumorprobe im Vergleich zur passenden Nicht-Tumorprobe vorhanden sind; und
    Identifizieren der Vielzahl von Regionen des Genoms, die die erste phasierte Variante und die zweite phasierte Variante umfassen, aus der identifizierten Vielzahl von Einzelnukleotidvarianten, zumindest teilweise basierend auf einem oder mehreren der folgenden Merkmale (1) einer Nähe von zwei oder mehr der identifizierten Vielzahl von Einzelnukleotidvarianten innerhalb eines Genoms und (2) einem Vorhandensein von zwei oder mehr der identifizierten Vielzahl von Einzelnukleotidvarianten in einem einzigen Read aus den Sequenzdaten aus der Tumorprobe des Probanden;

wobei die in (c) identifizierten mehreren Regionen nicht überlappende Regionen sind; das Durchführen der gezielten Sequenzierung von (d) umfasst (1) das Durchführen einer Hybridisierungs-Capture von Nukleinsäuren aus der Tumorprobe des Probanden, die den identifizierten mehreren Regionen des Genoms aus (c) entsprechen, um gefangene Nukleinsäuren zu erhalten, und (2) das Sequenzieren der gefangenen Nukleinsäuren aus der Tumorprobe des Probanden; und die erste mutmaßliche phasierte Variante und die zweite mutmaßliche phasierte Variante sind Einzelnukleotidvarianten, die durch mindestens ein Nukleotid voneinander getrennt sind.

20. Verfahren nach einem der Ansprüche 1 bis 19, das ferner das Kombinieren eines Satzes von Nukleinsäuresonden mit einer Vielzahl von zellfreien Nukleinsäuremolekülen umfasst, die von einem Probanden erhalten oder abgeleitet werden, um eine Mischung zu bilden, wobei jede Nukleinsäuresonde des Satzes von Nukleinsäuresonden so konfiguriert ist, dass sie mit einem zellfreien Nukleinsäuremolekül hybridisiert, das einen oder mehrere der identifizierten Sätze validierter phasierter Varianten umfasst.

21. Verfahren nach Anspruch 20, wobei jede Nukleinsäuresonde des Satzes von Nukleinsäuresonden so konfiguriert ist, dass sie mit einem Zielnukleinsäuremolekül hybridisiert, das eine Vielzahl von phasierten Varianten umfasst, sodass die Nukleinsäuresonde zu mindestens einem Bereich des Zielnukleinsäuremoleküls komplementär ist, der sich von einer ersten phasierten Variante des Satzes von phasierten Varianten zu einer zweiten phasierten Variante des Satzes von phasierten Varianten erstreckt.

22. Verfahren nach Anspruch 20 oder 21, wobei jede Nukleinsäuresonde des Satzes von Nukleinsäuresonden ein Biotin-Pull-down-Tag umfasst.

23. Verfahren nach einem der Ansprüche 20 bis 22, das ferner umfasst:

Trennen von Zielnukleinsäuremolekülen, die mit mindestens einer Nukleinsäuresonde des Satzes von Nukleinsäuresonden hybridisieren, von Nukleinsäuremolekülen, die nicht mit mindestens einer Nukleinsäuresonde des Satzes von Nukleinsäuresonden hybridisieren, wodurch Zielnukleinsäuremoleküle eingefangen werden; und

Sequenzieren der Zielnukleinsäuremoleküle, die mit mindestens einer der Nukleinsäuresonden des Satzes hybridisieren.

24. Verfahren nach Anspruch 23, das ferner umfasst: Identifizieren eines oder mehrerer zellfreier Nukleinsäuremoleküle als von Krebs stammende Moleküle mit einer Nachweisgrenze von weniger als etwa 1 von 50.000 Beobachtungen aus Sequenzierungsdaten, die aus der Sequenzierung der zellfreien Nukleinsäuremoleküle erhalten wurden.

25. Verfahren nach Anspruch 24, wobei die Nachweisgrenze weniger als 1 von 1.000.000 Beobachtungen aus den Sequenzierungsdaten beträgt.

26. Verfahren nach einem der Ansprüche 23 bis 25, wobei die Sequenzierung keine Verwendung von molekularen Barcodes umfasst.

27. Verfahren nach einem der Ansprüche 23 bis 26, das ferner das Bestimmen eines Zustands des Probanden umfasst.

28. Verfahren nach Anspruch 27, wobei der Zustand Krebs ist.

29. Verfahren nach einem der Ansprüche 23 bis 28, das ferner das Überwachen des Fortschreitens eines Zustands des Probanden umfasst.

30. Verfahren nach Anspruch 29, wobei der Zustand Krebs ist.

**Revendications**

1. Procédé permettant d'identifier des ensembles de variants phasés validés à partir d'un échantillon tumoral d'un sujet, le procédé comprenant :

(a) l'obtention, par un système informatique, de données de séquençage à partir d'un échantillon tumoral d'un sujet ;

(b) l'obtention, par le système informatique, de données de séquençage à partir d'un échantillon non tumoral apparié du sujet ;

(c) analyser, à l'aide du système informatique, les données de séquençage provenant de l'échantillon tumoral et les données de séquençage provenant de l'échantillon non tumoral apparié afin d'identifier une pluralité de régions d'un génome du sujet qui comprennent une première variante phasée putative et une deuxième variante phasée putative, chacune des régions de la pluralité n'ayant pas plus de 170 paires de bases de longueur ;

(d) après (c), effectuer un séquençage ciblé sur les acides nucléiques provenant de l'échantillon tumoral du sujet qui proviennent de la pluralité de régions identifiées en (c) à une profondeur d'au moins 250x ;

(e) identifier, à l'aide du système informatique, des ensembles de variants phasés validés provenant de l'échantillon tumoral du sujet sur la base des données de séquençage provenant du séquençage ciblé en (d).

2. Procédé selon la revendication 1, dans lequel le premier variant phasé putatif et le deuxième variant phasé putatif sont séparés par au moins un nucléotide.

3. Procédé selon la revendication 1 ou 2, dans lequel (c) comprend en outre : la détermination d'une pluralité de variants nucléotidiques simples dans le génome du sujet qui sont présents dans l'échantillon tumoral par rapport à l'échantillon non tumoral apparié ; et à partir de la pluralité identifiée de variants nucléotidiques simples, l'identification de la pluralité de régions du génome qui comprennent le premier variant phasé et le deuxième variant phasé sur la base d'au moins un ou plusieurs des éléments suivants (1) une proximité de deux ou plusieurs de la pluralité identifiée de variants nucléotidiques simples dans un génome, et (2) une présence de deux ou plusieurs de la pluralité identifiée de variants nucléotidiques simples sur une seule lecture à partir des données de séquence provenant de l'échantillon tumoral du sujet.

4. Procédé selon la revendication 3, dans lequel les données de séquençage provenant de l'échantillon tumoral du sujet dans (a) et (b) comprennent chacune des données pour au moins 50 % du génome du sujet.

5. Procédé selon la revendication 4, dans lequel (c) comprend en outre l'alignement des données de séquençage provenant de l'échantillon tumoral et des données de séquençage provenant de l'échantillon non tumoral apparié sur un génome.

6. Procédé selon la revendication 5, dans lequel les données de séquençage provenant de l'échantillon tumoral et les données de séquençage provenant de l'échantillon non tumoral apparié sont obtenues à une profondeur comprise entre 10x et 100x.

7. Procédé selon la revendication 6, dans lequel la pluralité de régions identifiées du génome dans (c) comprend au moins 400 régions et pas plus de 15 000 régions.

8. Procédé selon l'une quelconque des revendications 1 à 7, dans lequel l'identification de la pluralité de régions du génome dans (c) comprend la détermination d'un ou plusieurs des éléments suivants : (i) la présence de variants phasés dans des lectures individuelles provenant de l'échantillon tumoral du sujet, (ii) la présence ou l'absence de support de lecture dans l'échantillon non tumoral apparié, (iii) la présence d'autres bases non référencées sur les lectures de soutien, (iv) la qualité des bases, (v) la qualité du mappage et (vi) l'unicité des positions génomiques.

9. Procédé selon l'une quelconque des revendications 1 à 8, dans lequel la réalisation du séquençage ciblé de (d) comprend (1) la réalisation d'une capture par hybridation d'acides nucléiques provenant de l'échantillon tumoral du sujet qui correspondent à la pluralité de régions identifiées du génome de (c) afin d'obtenir des acides nucléiques capturés, et (2) le séquençage des acides nucléiques capturés provenant de l'échantillon tumoral du sujet.

10. Procédé selon l'une quelconque des revendications 1 à 9, dans lequel le nombre d'ensembles de variants phasés validés est inférieur à 1000 et supérieur à 10.

11. Procédé selon l'une quelconque des revendications 1 à 10, dans lequel la pluralité de régions identifiées en (c) sont des régions non chevauchantes.

12. Procédé selon l'une quelconque des revendications 1 à 11, dans lequel les acides nucléiques de (d) sont des molécules d'ADN.

**13.** Procédé selon l'une quelconque des revendications 1 à 12, dans lequel le premier variant phasé et le deuxième variant phasé sont chacun des variants à nucléotide unique.

**14.** Procédé selon l'une quelconque des revendications 1 à 13, dans lequel les ensembles de variants phasés validés présentent une fraction allélique supérieure à 5 % et aucun support de lecture dans les données de séquençage provenant de l'échantillon non tumoral apparié.

**15.** Procédé selon l'une quelconque des revendications 1 à 14, dans lequel (d) comprend en outre : après (c), la réalisation d'un séquençage ciblé sur les acides nucléiques provenant de l'échantillon non tumoral apparié du sujet qui proviennent de la pluralité de régions identifiées en (c) à une profondeur d'au moins 500x.

**16.** Procédé selon l'une quelconque des revendications 1 à 15, dans lequel (d) comprend en outre: après (c), la réalisation d'un séquençage ciblé sur les acides nucléiques provenant de l'échantillon non tumoral apparié du sujet qui proviennent de la pluralité de régions identifiées en (c) à une profondeur d'au moins 250x.

**17.** Procédé selon l'une quelconque des revendications 1 à 16, dans lequel les ensembles de variants phasés validés ont un signal de fond inférieur à un sur un million.

**18.** Procédé selon l'une quelconque des revendications 1 à 17, dans lequel l'échantillon tumoral est un échantillon tumoral solide.

**19.** Procédé selon l'une quelconque des revendications 1 à 18, dans lequel :

les données de séquençage provenant de l'échantillon tumoral sont obtenues à une profondeur comprise entre 10x et 100x et comprennent au moins 50 % du génome du sujet ;
les données de séquençage provenant de l'échantillon non tumoral apparié sont obtenues à une profondeur comprise entre 10x et 100x et comprennent au moins 50 % du génome du sujet ;
(c) comprend en outre :

l'alignement des données de séquençage provenant de l'échantillon tumoral et des données de séquençage provenant de l'échantillon non tumoral apparié sur un génome ;
la détermination d'une pluralité de variants nucléotidiques simples dans le génome du sujet qui sont présents dans l'échantillon tumoral par rapport à l'échantillon non tumoral apparié ; et
à partir de la pluralité identifiée de variants nucléotidiques simples, l'identification de la pluralité de régions du génome qui comprennent le premier variant phasé et le deuxième variant phasé sur la base d'au moins un ou plusieurs des éléments suivants (1) une proximité de deux ou plusieurs de la pluralité identifiée de variants nucléotidiques simples dans un génome, et (2) une présence de deux ou plusieurs de la pluralité identifiée de variants nucléotidiques simples sur une seule lecture à partir des données de séquençage provenant de l'échantillon tumoral du sujet ;
dans lequel la pluralité de régions identifiées en (c) sont des régions qui ne se chevauchent pas ; la réalisation du séquençage ciblé de (d) comprend (1) la réalisation d'une capture par hybridation d'acides nucléiques provenant de l'échantillon tumoral du sujet qui correspondent à la pluralité de régions identifiées du génome de (c) afin d'obtenir des acides nucléiques capturés, et (2) le séquençage des acides nucléiques capturés provenant de l'échantillon tumoral du sujet ; et le premier variant phasé putatif et le deuxième variant phasé putatif sont des variants nucléotidiques simples séparés par au moins un nucléotide.

**20.** Procédé selon l'une quelconque des revendications 1 à 19, comprenant en outre la combinaison d'un ensemble de sondes d'acide nucléique avec une pluralité de molécules d'acide nucléique acellulaires qui sont obtenues ou dérivées d'un sujet pour former un mélange, dans lequel chaque sonde d'acide nucléique de l'ensemble de sondes d'acide nucléique est configurée pour s'hybrider à une molécule d'acide nucléique acellulaire qui comprend un ou plusieurs des ensembles identifiés de variants phasés validés.

**21.** Procédé selon la revendication 20, dans lequel chaque sonde d'acide nucléique de l'ensemble de sondes d'acide nucléique est configurée pour s'hybrider à une molécule d'acide nucléique cible comprenant une pluralité de variants phasés de telle sorte que la sonde d'acide nucléique soit complémentaire d'au moins une région de la molécule d'acide nucléique cible qui s'étend d'un premier variant phasé de l'ensemble de variants phasés à un deuxième variant phasé de l'ensemble de variants phasés.

**22.** Procédé selon la revendication 20 ou 21, dans lequel chaque sonde d'acide nucléique de l'ensemble de sondes d'acide nucléique comprend une étiquette de pull-down à la biotine.

**23.** Procédé selon l'une quelconque des revendications 20 à 22, comprenant en outre :

la séparation des molécules d'acide nucléique cibles qui s'hybrident à au moins une sonde d'acide nucléique de l'ensemble de sondes d'acide nucléique des molécules d'acide nucléique qui ne s'hybrident pas à au moins une sonde d'acide nucléique de l'ensemble de sondes d'acide nucléique, capturant ainsi les molécules d'acide nucléique cibles ; et

le séquençage des molécules d'acide nucléique cibles qui s'hybrident à au moins une sonde de l'ensemble de sondes d'acide nucléique.

**24.** Procédé selon la revendication 23, comprenant en outre l'identification d'une ou plusieurs molécules d'acide nucléique acellulaire comme étant une molécule dérivée d'un cancer avec une limite de détection inférieure à environ 1 sur 50 000 observations à partir des données de séquençage obtenues à partir du séquençage des molécules d'acide nucléique acellulaire.

**25.** Procédé selon la revendication 24, dans lequel la limite de détection est inférieure à 1 sur 1 000 000 observations à partir des données de séquençage.

**26.** Procédé selon l'une quelconque des revendications 23 à 25, dans lequel le séquençage ne comprend pas l'utilisation de codes-barres moléculaires.

**27.** Procédé selon l'une quelconque des revendications 23 à 26, comprenant en outre la détermination d'un état du sujet.

**28.** Procédé selon la revendication 27, dans lequel l'état est un cancer.

**29.** Procédé selon l'une quelconque des revendications 23 à 28, comprenant en outre la surveillance de la progression d'un état du sujet.

**30.** Procédé selon la revendication 29, dans lequel l'état est un cancer.

Single Nucleotide Variant

cell-free DNA molecule

Phased Variants

cell-free DNA molecule

Plus Strand
Minus Strand
Mutation

FIG. 1A

Phased Variants / Total SNVs

0.00 0.03 0.06 0.09 0.12

(n)

| Cancer Type | (n) |
|---|---|
| FL-NHL | 74 |
| DLBCL-NHL | 68 |
| Skin-Melanoma | 107 |
| Burkitt-NHL | 36 |
| Lung-SCC | 48 |
| Lung-Adeno | 38 |
| Kidney-RCC | 144 |
| Bone-Osteosarc | 44 |
| Liver-HCC | 318 |
| Breast-Adeno | 198 |
| Panc-Adeno | 239 |
| Head-SCC | 57 |
| Ovary-Adeno | 113 |
| Eso-Adeno | 98 |
| Uterus-Adeno | 51 |
| Stomach-Adeno | 75 |
| CLL | 151 |
| ColoRect-Adeno | 60 |
| Prost-Adeno | 210 |
| CNS-GBM | 41 |
| Panc-Endocrine | 85 |
| Thy-Adeno | 48 |
| CNS-PiloAstro | 89 |
| CNS-Medullo | 146 |

FIG. 1B

FIG. 1C

EP 4 334 476 B1

FIG. 1D

Recovery of molecules by sequencing

Plus Strand
Minus Strand
Mutation

Sequencing library

Original cell-free DNA

Single nucleotide variant

Library prep PCR

x n copies

x m copies

Independent recovery of both plus and minus strands

Duplex sequencing (trans)

Phased variants

Library prep PCR

x n copies

x m copies

Recovery of either plus or minus strands

or

Phased variants (cis)

FIG. 1E

FIG. 2A

EP 4 334 476 B1

FIG. 2B

FIG. 2C

FIG. 2D

FIG. 2E

FIG. 2F

FIG. 3A

FIG. 3B

FIG. 3C

FIG. 3D

FIG. 3E

FIG. 3F

FIG. 3G

FIG. 3H

FIG. 3I

FIG. 4A

FIG. 4B

FIG. 4C

FIG. 4D

FIG. 4E

FIG. 4F

FIG. 4G

FIG. 5A

FIG. 5B

FIG. 5C

FIG. 6A

FIG. 6B

FIG. 6C

FIG. 6D

FIG. 6E

FIG. 6F

FIG. 6G

FIG. 6H

FIG. 6I

FIG. 6J

FIG. 6K

FIG. 6L

FIG. 6M

FIG. 6N

FIG. 6O

FIG. 6P

FIG. 6Q

FIG. 6R

FIG. 6S

FIG. 6T

FIG. 6U

FIG. 6V

FIG. 6W

FIG. 6X

FIG. 6Y

FIG. 6Z

FIG. 6AA

FIG. 6BB

FIG. 6CC

FIG. 6DD

FIG. 6EE

FIG. 6FF

FIG. 6GG

FIG. 6HH

FIG. 6II

FIG. 6JJ

FIG. 6KK

FIG. 6LL

FIG. 6MM

FIG. 6NN

FIG. 6OO

FIG. 6PP

FIG. 6QQ

FIG. 6RR

FIG. 6SS

FIG. 6TT

FIG. 6UU

FIG. 6VV

FIG. 6WW

FIG. 7

FIG. 7 (Cont.)

FIG. 8A

Phased Reporters Near *BCL2* locus

*BCL2*

FIG. 8B

**FIG. 8C**

**FIG. 8D**

FIG. 8E

FIG. 9A

FIG. 9B

FIG. 9C

FIG. 9D

FIG. 9E

FIG. 9F

Number of SNVs per case

FIG. 9G

Number of doublet PVs per case

FIG. 9H

Number of triplet PVs per case

FIG. 9I

Number of quadruplet PVs per case

FIG. 9J

Number of total PVs per case

FIG. 9K

FIG. 10A

FIG. 10B

FIG. 10C

FIG. 10D

FIG. 10E

FIG. 10F

FIG. 10G

FIG. 10H

FIG. 10I

Other Positions
IGLV3-1

GCB vs ABC: *IGLV3-1*
chr22:23222928-23223998

P < 0.0001

**FIG. 10J**

Other Positions
IGHG1

GCB vs ABC: *IGHG1*
chr14:106211961-106214011

P < 0.0001

**FIG. 10K**

Other Positions
IGHJ5

GCB vs ABC: *IGHJ5*
chr14:106329751-106330201

P < 0.0001

**FIG. 10L**

Other Positions
LPP

GCB vs ABC: *LPP*
chr3:187957433-188471931

P < 0.0001

**FIG. 10M**

Other Positions
IGKJ2

GCB vs ABC: *IGKJ2*
chr2:89160890-89161190

P < 0.0001

**FIG. 10N**

Other Positions
SGK1

GCB vs ABC: *SGK1*
chr6:134491383-134495968

P < 0.0001

**FIG. 10O**

Other Positions
PLEKHG1

GCB vs ABC: *PLEKHG1*
chr6:150954421-150954821

P < 0.0001

**FIG. 10P**

Other Positions
IGKV1-5

GCB vs ABC: *IGKV1-5*
chr2:89246682-89247982

P < 0.0001

**FIG. 10Q**

Other Positions
MYC

GCB vs ABC: *MYC*
chr8:128746808-128764273

P < 0.0001

**FIG. 10R**

FIG. 10S

FIG. 10T

FIG. 10U

FIG. 10V

FIG. 10W

FIG. 10X

FIG. 10Y

FIG. 11A — DLBCL vs PMBCL: *CIITA* — chr16:10971441-10974194 — P < 0.0001 — Other Positions, CIITA

FIG. 11B — DLBCL vs PMBCL: *BCL2* — chr18:60763830-60988465 — P < 0.0001 — Other Positions, BCL2

FIG. 11C — DLBCL vs PMBCL: *IGHG2* — chr14:106110676-106114376 — P < 0.0001 — Other Positions, IGHG2

FIG. 11D — DLBCL vs PMBCL: *IGHG3* — chr14:106239251-106241954 — P < 0.0001 — Other Positions, IGHG3

FIG. 11E — DLBCL vs PMBCL: *IGHG4* — chr14:106092381-106095531 — P < 0.0001 — Other Positions, IGHG4

FIG. 11F — DLBCL vs PMBCL: *IGHG1* — chr14:106211961-106214011 — P < 0.0001 — Other Positions, IGHG1

**Other Positions**
**RHOH**

DLBCL vs PMBCL: *RHOH*
chr4:40198811-40201559

**Other Positions**
**IGLV3-1**

DLBCL vs PMBCL: *IGLV3-1*
chr22:23222928-23223998

**Other Positions**
**IGHM**

DLBCL vs PMBCL: *IGHM*
chr14:106318101-106325773

$P < 0.0001$

$P < 0.0001$

$P < 0.0001$

FIG. 11G

FIG. 11H

FIG. 11I

FIG. 11J · FIG. 11K · FIG. 11L · FIG. 11M · FIG. 11N · FIG. 11O

Other Positions
ETS1

DLBCL vs PMBCL: *ETS1*
chr11:128391384-128392103

P < 0.0001

Fraction PMBCL

Fraction DLBCL

FIG. 11P

Other Positions
DUSP2

DLBCL vs PMBCL: *DUSP2*
chr2:96809890-96810360

P < 0.0001

Fraction PMBCL

Fraction DLBCL

FIG. 11Q

Other Positions
IGHV2-70

DLBCL vs PMBCL: *IGHV2-70*
chr14:1071178306-1071179990

P < 0.0001

Fraction PMBCL

Fraction DLBCL

FIG. 11R

FIG. 11S

FIG. 11T

FIG. 11U

FIG. 11V

FIG. 11W

FIG. 11X

FIG. 12A — DLBCL vs HL: *IGHG2* chr14:106110676-106114376

FIG. 12B — DLBCL vs HL: *IGLL5* chr22:23227063-23237340

FIG. 12C — DLBCL vs HL: *IGHG1* chr14:106211961-106214011

FIG. 12D — DLBCL vs HL: *IGHM* chr14:106318101-106325773

FIG. 12E — DLBCL vs HL: *IGHG4* chr14:106092381-106095531

FIG. 12F — DLBCL vs HL: *IGHG3* chr14:106239251-106241954

FIG. 12G

FIG. 12H

FIG. 12I

DLBCL vs HL: *CIITA*
chr16:10971441-10974194

FIG. 12J

DLBCL vs HL: *CXCR4*
chr2:136872526-136875621

FIG. 12K

DLBCL vs HL: *LRRN3*
chr7:110737412-110764944

FIG. 12L

DLBCL vs HL: *IGHE*
chr14:106068706-106071241

FIG. 12M

DLBCL vs HL: *ZFP36L1*
chr14:69258239-69259639

FIG. 12N

DLBCL vs HL: *PIM1*
chr6:37138285-37141880

FIG. 12O

DLBCL vs HL: *IGKJ5*
chr2:89158619-89160190

○ Other Positions
◉ IGKJ5

p < 0.0001

Fraction HL

Fraction DLBCL

**FIG. 12P**

DLBCL vs HL: *PTEN*
chr10:89624273-89720888

○ Other Positions
◉ PTEN

p < 0.0001

Fraction HL

Fraction DLBCL

**FIG. 12Q**

DLBCL vs HL: *DTX1*
chr12:113495365-113534745

○ Other Positions
◉ DTX1

p < 0.0001

Fraction HL

Fraction DLBCL

**FIG. 12R**

**DLBCL vs HL:** *IGHJ5*
**chr14:106329751-106330201**

FIG. 12S

**DLBCL vs HL:** *IGHV4-39*
**chr14:106877716-106878731**

FIG. 12T

**DLBCL vs HL:** *IGHV3-23*
**chr14:106725296-106726174**

FIG. 12U

**DLBCL vs HL:** *IGHD2-2*
**chr14:106381486-106383981**

FIG. 12V

**DLBCL vs HL:** *IGLV1-40*
**chr22:22758648-22764603**

FIG. 12W

**DLBCL vs HL:** *IGHV3-48*
**chr14:106994301-106994899**

FIG. 12X

DLBCL vs HL: *PIK3C2G*
chr12:18534683-18801013

DLBCL vs HL: *TNFAIP3*
chr6:138188484-138202489

DLBCL vs HL: *IGHV7-81*
chr14:107258911-107282996

FIG. 12Y

FIG. 12Z

FIG. 12AA

EP 4 334 476 B1

DLBCL vs HL: *IGHJ4*
chr14:106330251-106330601

**FIG. 12BB**

DLBCL vs HL: *NEDD4L*
chr18:56054916-56063816

**FIG. 12CC**

DLBCL vs HL: *DMD*
chrX:33146107-33146457

**FIG. 12DD**

DLBCL vs HL: *IGHV2-70*
chr14:107178306-107179990

**FIG. 12EE**

DLBCL vs HL: *BRCA2*
chr13:32907207 -32912827

**FIG. 12FF**

DLBCL vs HL: *IGKV1-5*
chr2:89246682-89247982

**FIG. 12GG**

EP 4 334 476 B1

DLBCL vs HL: *IGKV3-20*
chr2 :89442292-89443217

DLBCL vs HL: *IGKJ4*
chr2:89160240-89160540

DLBCL vs HL: *BCL6*
chr3:187440190-187661368

FIG. 12HH

FIG. 12II

FIG. 12JJ

FIG. 12KK

FIG. 12LL

FIG. 12MM

FIG. 12NN

FIG. 13

FIG. 14A

FIG. 14B

FIG. 14C

FIG. 14D

FIG. 14E

FIG. 15

FIG. 16A

FIG. 16B

FIG. 17A

FIG. 17B

FIG. 17C

FIG. 17D

FIG. 18A

FIG. 18B

FIG. 18C

FIG. 18D

FIG. 18E

FIG. 18F

FIG. 18G

FIG. 18H

FIG. 19

20M reads, 64ng

FIG. 20

FIG. 21A

Background signal rate in 24 healthy control cfDNA samples with and without molecular biology filter (i.e., with and without targeted resequencing)

## FIG. 21B

## FIG. 22A

## FIG. 22B

# Selection of validated phased variants from WGS data

| Case | viable PVs from WGS | | Candidate PVs<br>Targeted by Oligos<br>For Capture | | Validated PVs<br>In Targeted Resequencing |
|---|---|---|---|---|---|
| LUP502 | 5507 | Selection of candidate PVs to target using features such as: 1) presence in individual tumor reads as phased relationships, 2) absence of read support in matched normal, 3) presence of other non-reference bases on the supporting reads, 4) base quality, 5) mapping quality, and 6) uniqueness of genomic positions. | 670 | Filtering of final PVs by targeted resequencing of tumor and germline, considering only PVs present in the tumor at higher than 5% AF and had no read support in the matched germline DNA | 116 |
| LUP503 | 7063 | | 819 | | 223 |
| LUP814 | 5415 | | 1025 | | 622 |
| LUP831 | 3321 | | 466 | | 82 |

## FIG. 22C

FIG. 22D

FIG. 22E

| Case | Stage | No. of PVs Tracked |
|---|---|---|
| BRCA001 | IIB | 14 |
| LUP502 | IB | 116 |
| LUP503 | IA | 453 |
| LUP649 | IB | 248 |
| LUP831 | IIIB | 82 |
| LUP814 | IIIB | 622 |

Time of Diagnosis

Before Diagnosis | After Diagnosis

Patient Samples

Control Samples

□ not detected
□ false positive
▨ PhasED-Seq detected CAPP-Seq detected
▨ PhasED-Seq detected CAPP-Seq not detected

**FIG. 22F**

**FIG. 22G**

## FIG. 23A

## FIG. 23B

FIG. 24

**Process 2400**

Start

2401

Obtain, prepare, and sequence cell-free nucleic acids from a individual's biological sample, utilizing a capture sequencing approach across regions that have been shown to harbor a plurality of genetic variants occurring in phase

2403

Analyze the cell-free nucleic acid sequencing result to detect circulating-tumor nucleic acids, as determined by detection of genetic variants occurring in phase

2405

Perform a clinical intervention and/or treatment on the basis that the sequencing result indicates that the biological sample contains circulating-tumor nucleic acids

Complete

FIG. 25A

2510

Obtaining, by a computer system, sequencing data derived from a plurality of cell-free nucleic acid molecules from a subject. ⟋ 2512

Processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence, wherein at least about 10% of the one or more cell-free nucleic acid molecules comprises a first phased variant of the plurality of phased variants and a second phased variant of the plurality of phased variants that are separated by at least one nucleotide. ⟋ 2514

Analyzing, by the computer system, the identified one or more cell-free nucleic acid molecules to determine a condition of the subject. ⟋ 2516

**FIG. 25b**

**2520**

Obtaining, by a computer system, sequencing data derived from a plurality of cell-free nucleic acid molecules from a subject. — **2522**

Processing, by the computer system, the sequencing data to identify one or more cell-free nucleic acid molecules of the plurality of cell-free nucleic acid molecules, wherein each of the one or more cell-free nucleic acid molecules comprises a plurality of phased variants relative to a reference genomic sequence that are separated by at least one nucleotide. — **2524**

Analyzing, by the computer system, the identified one or more cell-free nucleic acid molecules to determine a condition of the subject. — **2526**

FIG. 25C

2530

Obtaining sequencing data derived from a plurality of cell-free nucleic acid molecules from a subject.  2532

Processing the sequencing data to identify one or more cell-free nucleic acid molecules of the plurality of cell-free nucleic acid molecules with a limit of detection being less than about 1 out of 50,000 observations from the sequencing data, wherein each of the one or more cell-free nucleic acid molecules comprises a plurality of phased variants relative to a reference genomic sequence.  2534

Analyzing the identified one or more cell-free nucleic acid molecules to determine a condition of the subject.  2536

FIG. 25D

2540

Identifying the subject for treatment of the condition, wherein the subject has been determined to have the condition based on identification of one or more cell-free nucleic acid molecules from a plurality of cell-free nucleic acid molecules from the subject,

wherein each of the one or more cell-free nucleic acid molecules identified comprises a plurality of phased variants relative to a reference genomic sequence that are separated by at least one nucleotide, and wherein a presence of the plurality of phased variants is indicative of the condition of the subject.

2542

Subjecting the subject to the treatment based on the identification.

2544

FIG. 25E

2550

Determining a first state of the condition of the subject based on identification of a first set of one or more cell-free nucleic acid molecules from a first plurality of cell-free nucleic acid molecules from the subject.

2552

Determining a second state of the condition of the subject based on identification of a second set of one or more cell-free nucleic acid molecules from a second plurality of cell-free nucleic acid molecules from the subject, wherein the second plurality of cell-free nucleic acid molecules are obtained from the subject subsequent to obtaining the first plurality of cell-free nucleic acid molecules from the subject.

2554

Determining the progress of the condition based on the first state of the condition and the second state of the condition, wherein each of the one or more cell-free nucleic acid molecules comprises a plurality of phased variants relative to a reference genomic sequence that are separated by at least one nucleotide.

2556

**FIG. 25F**

2560

Providing a mixture comprising (1) a set of nucleic acid probes and (2) a plurality of cell-free nucleic acid molecules from a subject,

wherein an individual nucleic acid probe of the set of nucleic acid probes is designed to hybridize to a target cell-free nucleic acid molecule comprising a plurality of phased variants relative to a reference genomic sequence that are separated by at least one nucleotide, and wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants.

2562

Detecting the reporter agent that is activated, to identify one or more cell-free nucleic acid molecules of the plurality of cell-free nucleic acid molecules, wherein each of the one or more cell-free nucleic acid molecules comprises the plurality of phased variants.

2564

Analyzing the identified one or more cell-free nucleic acid molecules to determine a condition of the subject.

2566

**FIG. 25G**

2570

Providing a mixture comprising (1) a set of nucleic acid probes and (2) a plurality of cell-free nucleic acid molecules from a subject,
wherein an individual nucleic acid probe of the set of nucleic acid probes is designed to hybridize to a target cell-free nucleic acid molecule comprising a plurality of phased variants relative to a reference genomic sequence, and
wherein the individual nucleic acid probe comprises an activatable reporter agent, activation of the activatable reporter agent being selected from the group consisting of: (i) hybridization of the individual nucleic acid probe to the plurality of phased variants and (ii) dehybridization of at least a portion of the individual nucleic acid probe that has been hybridized to the plurality of phased variants.

2572

Detecting the reporter agent that is activated, to identify one or more cell-free nucleic acid molecules of the plurality of cell-free nucleic acid molecules, wherein each of the one or more cell-free nucleic acid molecules comprises the plurality of phased variants, wherein a limit of detection of the identification step is less than about 1 out of 50,000 cell-free nucleic acid molecules of the plurality of cell-free nucleic acid molecules.

2574

Analyzing the identified one or more cell-free nucleic acid molecules to determine a condition of the subject.

2576

FIG. 26A

FIG. 26B

FIG. 27

2730

2701

2735

2740

2720

2705

2725

2715

2710

**FIG. 28**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63188410
- US 63224795
- US 30895821
- CA 233975
- CA 241076
- CA 188298
- US 2020131505 A
- US 5804396 A

### Non-patent literature cited in the description

- **GORELICK et al.** *Nature*, 2020
- **VAN DER AUWERA, G. A. et al.** From FastQ data to high-confidence variant calls: the Genome Analysis Toolkit best practices pipeline. *Curr. Protoc. Bioinformatics*, 2013, vol. 43, 11.10.1-11.10.33
- **KOBOLDT, D. C. et al.** VarScan 2: somatic mutation and copy number alteration discovery in cancer by exome sequencing. *Genome Res*, 2012, vol. 22, 568-576
- **CIBULSKIS, K. et al.** Sensitive detection of somatic point mutations in impure and heterogeneous cancer samples. *Nat. Biotechnol.*, 2013, vol. 31, 213-219
- **KIM, S. et al.** Strelka2: fast and accurate calling of germline and somatic variants. *Nat. Methods*, 2018, vol. 15, 591-594